# EUROPEAN PATENT APPLICATION

(11) **EP 4 574 829 A1**
(43) Date of publication of application: **25.06.2025**
(21) Application number: 23854513.1
(22) Date of filing: 18.08.2023
(51) Int. Cl.: C07D 498/04, C07D 493/00, C07D 491/00, A61K 31/553, A61K 31/423, A61P 35/00

(54) **COMPOUND CONTAINING CYCLOHEXYL**

(30) Priority: 19.08.2022 CN 202211000005; 16.01.2023 CN 202310206412; 08.08.2023 CN 202310996368
(71) Applicant: Chia Tai Tianqing Pharmaceutical Group Co., Ltd., Lianyungang, Jiangsu 222062 (CN)
(72) Inventor: REN, Jing, Lianyungang, Jiangsu 222062 (CN); XU, Sheng, Lianyungang, Jiangsu 222062 (CN); WEI, Jian, Lianyungang, Jiangsu 222062 (CN); ZHANG, Yinsheng, Lianyungang, Jiangsu 222062 (CN); DENG, Li, Lianyungang, Jiangsu 222062 (CN)
(74) Representative: v. Bezold & Partner Patentanwälte - PartG mbB
(86) International application number: PCT/CN2023/113691
(87) International publication number: WO 2024/037616

(57) **Abstract**

The present application relates to a compound containing cyclohexyl, in particular to a compound of formula (I-AA), a stereoisomer or pharmaceutically acceptable salt thereof, a preparation method therefor, a pharmaceutical composition containing the compound, and a use thereof in treating related diseases (such as cancer).

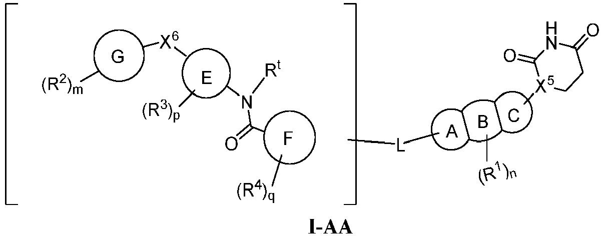

## Description

### CROSS-REFERENCE TO RELATED APPLICATIONS

The present application claims priority and benefits to the following Chinese patent applications filed with the China National Intellectual Property Administration, the contents of which are incorporated herein by reference in their entireties:
Chinese Patent Application No. 202211000005.5, filed on August 19, 2022;
Chinese Patent Application No. 202310206412.X, filed on January 16, 2023; and
Chinese Patent Application No. 202310996368.7, filed on August 08, 2023.

### TECHNICAL FIELD

The present application relates to a compound containing cyclohexyl, a preparation method therefor, a pharmaceutical composition containing the compound, and use thereof for treating a related disease (e.g., cancer).

### BACKGROUND

The androgen receptor (AR) is a steroid receptor in the nuclear receptor superfamily. When bound to androgens (such as testosterone and dihydrotestosterone), AR is released from the complex formed by heat shock proteins for a phosphorylation reaction to form a dimer. The dimer is transferred into the nucleus and binds to a DNA fragment associated there with, thereby stimulating transcription of its target gene. The transcriptional activity of androgen receptors activated by ligand binding is coordinated by co-activator proteins. AR antagonists have the main function of treating prostatic cancer by directly preventing the binding of testosterone or dihydrotestosterone to androgen receptors and thus blocking the action of androgens on cells, playing the roles of resisting androgens and inhibiting cell growth and finally resulting in apoptosis.

The proteolysis targeting chimera (PROTAC) molecule is a bifunctional compound capable of binding to a target protein and E3 ubiquitin ligase simultaneously. Such compounds can induce the target protein to be recognized by proteasomes of cells, cause the degradation of the target protein, and effectively reduce the content of the target protein in the cells. The introduction of ligands capable of binding to different target proteins into PROTAC molecules has made it possible to apply the PROTAC technology to the treatment of various diseases, and this technology has received much attention in recent years.

### SUMMARY

In one aspect, the present application relates to a compound of formula I-AA, a stereoisomer thereof, or a pharmaceutically acceptable salt thereof, 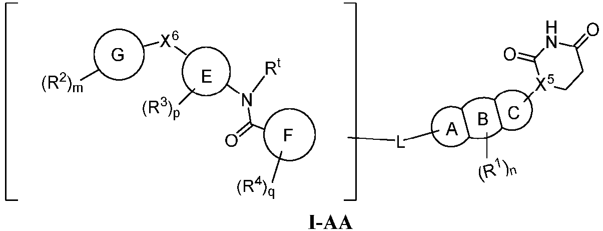 wherein,
ring A is absent or selected from the group consisting of C₅₋₁₅ cycloalkenyl, 5- to 15-membered heterocycloalkenyl, phenyl, and 5- to 6-membered heteroaryl;
ring B is selected from the group consisting of phenyl and 5- to 6-membered heteroaryl;
ring C is selected from 5- to 6-membered heteroaryl (e.g., isoxazolyl or furanyl);
each R¹ is independently selected from the group consisting of halogen, -OH, -NH₂, -CN, C₁₋₁₀ alkyl (e.g., C₁₋₆ alkyl), C₁₋₁₀ alkoxy (e.g., C₁₋₆ alkoxy), and halogenated C₁₋₁₀ alkyl (e.g., halogenated C₁₋₆ alkyl), wherein the -OH, -NH₂, C₁₋₁₀ alkyl (e.g., C₁₋₆ alkyl), C₁₋₁₀ alkoxy (e.g., C₁₋₆ alkoxy), or halogenated C₁₋₁₀ alkyl (e.g., halogenated C₁₋₆ alkyl) is optionally substituted with one or more substituents;
n is selected from the group consisting of 0, 1, 2, and 3;
L is selected from a connecting group;
X⁵ is selected from the group consisting of CH and N;
X⁶ is selected from the group consisting of -O-, -NH-, and -N(C₁₋₆ alkyl)-, wherein the -NH- or -N(C₁₋₆ alkyl)- is optionally substituted with one or more substituents;
each R², R³, and R⁴ is independently selected from the group consisting of halogen, -OH, -NH₂, -CN, C₁₋₁₀ alkyl (e.g., C₁₋₆ alkyl), C₁₋₁₀ alkoxy (e.g., C₁₋₆ alkoxy), and halogenated C₁₋₁₀ alkyl (e.g., halogenated C₁₋₆ alkyl), wherein the -OH, -NH₂, C₁₋₁₀ alkyl, C₁₋₁₀ alkoxy, or halogenated C₁₋₁₀ alkyl is optionally substituted with one or more substituents;
m, p, and q are each independently selected from the group consisting of 0, 1, 2, 3, and 4;
ring G is selected from the group consisting of C₆₋₁₀ aryl and 5- to 10-membered heteroaryl;
ring E is selected from the group consisting of C₃₋₁₀ cycloalkyl and 3- to 10-membered heterocycloalkyl;
ring F is selected from the group consisting of C₆₋₁₀ aryl and 5- to 10-membered heteroaryl;
R^{t} is selected from the group consisting of hydrogen, -OH, C₁₋₆ alkyl, C₃₋₁₀ cycloalkyl, and 3- to 10-membered heterocycloalkyl, wherein the C₁₋₆ alkyl, C₃₋₁₀ cycloalkyl, or 3- to 10-membered heterocycloalkyl is optionally substituted.

In some embodiments, ring A is absent or selected from the group consisting of C₅₋₁₀ cycloalkenyl, 5- to 10-membered heterocycloalkenyl, phenyl, and 5- to 6-membered heteroaryl.

In some embodiments, ring B is selected from the group consisting of phenyl and 6-membered heteroaryl.

In some embodiments, ring C is selected from 5-membered heteroaryl.

In some embodiments, ring G is selected from the group consisting of phenyl and 5- to 6-membered heteroaryl. In some embodiments, ring G is selected from the group consisting of phenyl and 6-membered heteroaryl. In some embodiments, ring G is phenyl.

In some embodiments, ring E is selected from the group consisting of C₃₋₉ cycloalkyl and 3- to 9-membered heterocycloalkyl. In some embodiments, ring E is selected from the group consisting of C₄₋₉ cycloalkyl and 4- to 9-membered heterocycloalkyl. In some embodiments, ring E is selected from the group consisting of C₄₋₇ cycloalkyl and 4- to 7-membered heterocycloalkyl. In some embodiments, ring E is selected from C₅₋₇ cycloalkyl. In some embodiments, ring E is selected from C₄₋₆ cycloalkyl. In some embodiments, ring E is cyclohexyl.

In some embodiments, ring F is selected from the group consisting of C₆₋₁₀ aryl and 5- to 7-membered heteroaryl. In some embodiments, ring F is selected from the group consisting of phenyl and 5- to 6-membered heteroaryl. In some embodiments, ring F is selected from the group consisting of phenyl and 6-membered heteroaryl. In some embodiments, ring F is selected from the group consisting of phenyl, pyridazinyl, pyrimidinyl, and pyrazinyl.

In some embodiments, R^{t} is selected from the group consisting of hydrogen, -OH, C₁₋₄ alkyl, C₃₋₆ cycloalkyl, and 3- to 6-membered heterocycloalkyl, wherein the C₁₋₄ alkyl, C₃₋₆ cycloalkyl, or 3- to 6-membered heterocycloalkyl is optionally substituted. In some embodiments, R^{t} is selected from the group consisting of hydrogen, -OH, C₁₋₃ alkyl, C₃₋₄ cycloalkyl, and 3- to 4-membered heterocycloalkyl, wherein the C₁₋₃ alkyl, C₃₋₄ cycloalkyl, or 3- to 4-membered heterocycloalkyl is optionally substituted. In some embodiments, R^{t} is selected from the group consisting of hydrogen and C₁₋₃ alkyl (e.g., methyl, ethyl, or propyl).

In some embodiments, the "optionally substituted" or "optionally substituted with one or more substituents" described above means being optionally substituted with one or more of the following groups: halogen (e.g., fluorine, chlorine, bromine, or iodine), CN, OH, and NH₂.

In one aspect, the present application relates to a compound of formula I-1, a stereoisomer thereof, or a pharmaceutically acceptable salt thereof, 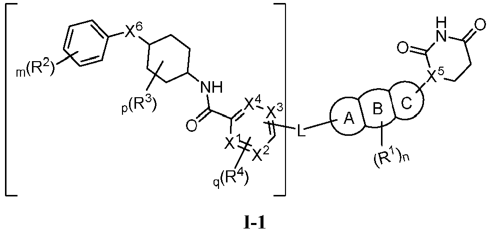 wherein,
ring A is absent or selected from the group consisting of C₅₋₁₀ cycloalkenyl, 5- to 10-membered heterocycloalkenyl, phenyl, and 5- to 6-membered heteroaryl;
ring B is selected from phenyl;
ring C is selected from the group consisting of isoxazolyl and furanyl;
each R¹ is independently selected from the group consisting of halogen, -OH, -NH₂, -CN, C₁₋₄ alkyl, C₁₋₄ alkoxy, and halogenated C₁₋₄ alkyl;
n is selected from the group consisting of 0, 1, 2, and 3;
L is selected from a connecting group;
X¹, X², X³, and X⁴ are each independently selected from the group consisting of N and CH;
X³ is selected from the group consisting of CH and N;
X⁶ is selected from the group consisting of -O-, -NH-, and -N(C₁₋₆ alkyl)-;
each R², R³, and R⁴ is independently selected from the group consisting of halogen, -OH, -NH₂, -CN, C₁₋₄ alkyl, C₁. ₄ alkoxy, and halogenated C₁₋₄ alkyl;
m, p, and q are each independently selected from the group consisting of 0, 1, 2, 3, and 4.

In one aspect, the present application relates to a compound of formula I, a stereoisomer thereof, or a pharmaceutically acceptable salt thereof, 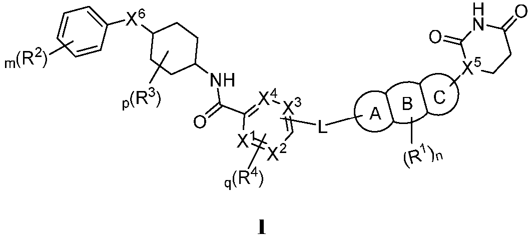 wherein,
ring A is absent or selected from the group consisting of C₅₋₁₀ cycloalkenyl, 5- to 10-membered heterocycloalkenyl, phenyl, and 5- to 6-membered heteroaryl;
ring B is selected from phenyl;
ring C is selected from the group consisting of isoxazolyl and furanyl;
each R¹ is independently selected from the group consisting of halogen, -OH, -NH₂, -CN, C₁₋₄ alkyl, C₁₋₄ alkoxy, and halogenated C₁₋₄ alkyl;
n is selected from the group consisting of 0, 1, 2, and 3;
L is selected from a connecting group;
X¹, X², X³, and X⁴ are each independently selected from the group consisting of N and CH;
X³ is selected from the group consisting of CH and N;
X⁶ is selected from the group consisting of -O-, -NH-, and -N(C₁₋₆ alkyl)-;
each R², R³, and R⁴ is independently selected from the group consisting of halogen, -OH, -NH₂, -CN, C₁₋₄ alkyl, C₁₋₄ alkoxy, and halogenated C₁₋₄ alkyl;
m, p, and q are each independently selected from the group consisting of 0, 1, 2, 3, and 4.

In some embodiments, ring A is absent or selected from the group consisting of C₅₋₇ cycloalkenyl, 5- to 9-membered heterocycloalkenyl, phenyl, and 5- to 6-membered heteroaryl.

In some embodiments, ring A is absent or selected from the group consisting of C₅₋₆ cycloalkenyl, 5- to 10-membered heterocycloalkenyl, phenyl, and 5- to 6-membered heteroaryl.

In some embodiments, ring A is absent or selected from the group consisting of C₅₋₆ cycloalkenyl, 5- to 9-membered heterocycloalkenyl, phenyl, and 5- to 6-membered heteroaryl.

In some embodiments, ring A is absent or selected from the group consisting of C₅₋₆ cycloalkenyl, 5- to 9-membered heterocycloalkenyl, phenyl, and 5-membered heteroaryl.

In some embodiments, ring A is absent or selected from the group consisting of C₅₋₆ cycloalkenyl, 5- to 9-membered heterocycloalkenyl, phenyl, pyrrolyl, pyrazolyl, furanyl, and oxazolyl. In some embodiments, ring A is absent or selected from the group consisting of C₅ cycloalkenyl, C₆ cycloalkenyl, 5-membered, 6-membered, 7-membered, 8-membered, and 9-membered heterocycloalkenyl, phenyl, pyrrolyl, pyrazolyl, furanyl, and oxazolyl.

In some embodiments, ring A is absent or selected from the group consisting of cyclopentenyl, monocyclohexenyl, bicyclohexenyl, dihydropyrrolyl, tetrahydropyridinyl, tetrahydroazepinyl, azaspirooctenyl, azaspirononenyl, phenyl, pyrrolyl, pyrazolyl, furanyl, oxazolyl, and dihydrooxazinyl.

In some embodiments, ring A is absent or selected from the group consisting of cyclopentenyl, bicyclohexenyl, dihydropyrrolyl, tetrahydropyridinyl, tetrahydroazepinyl, azaspirooctenyl, azaspirononenyl, phenyl, pyrrolyl, pyrazolyl, furanyl, oxazolyl, and dihydrooxazinyl. In some embodiments, ring A is selected from monocyclohexenyl.

In some specific embodiments, ring A is selected from the group consisting of C₅₋₆ cycloalkenyl, 5- to 10-membered heterocycloalkenyl, phenyl, and 5-membered heteroaryl. In some specific embodiments, ring A is selected from the group consisting of C₅₋₉ cycloalkenyl, 5- to 9-membered heterocycloalkenyl, phenyl, and 5-membered heteroaryl.

In some specific embodiments, ring A is selected from the group consisting of C₅₋₆ cycloalkenyl, 5- to 9-membered heterocycloalkenyl, phenyl, and 5-membered heteroaryl.

In some specific embodiments, ring A is selected from the group consisting of C₅₋₉ cycloalkenyl and 5- to 9-membered heterocycloalkenyl. In some specific embodiments, ring A is selected from the group consisting of C₅₋₆ cycloalkenyl and 5- to 9-membered heterocycloalkenyl. In some specific embodiments, ring A is selected from C₅₋₉ cycloalkenyl. In some specific embodiments, ring A is selected from C₅₋₆ cycloalkenyl. In some specific embodiments, ring A is selected from 5- to 9-membered heterocycloalkenyl.

In some specific embodiments, ring A is selected from the group consisting of cyclopentenyl, dihydropyrrolyl, tetrahydropyridinyl, tetrahydroazepinyl, azaspirononenyl, azaspirooctenyl, phenyl, pyrrolyl, and pyrazolyl.

In some more specific embodiments, ring A is selected from the group consisting of cyclopentenyl, dihydropyrrolyl, tetrahydropyridinyl, tetrahydroazepinyl, azaspirononenyl, and azaspirooctenyl.

In some more specific embodiments, ring A is selected from the group consisting of phenyl, pyrrolyl, and pyrazolyl. In some specific embodiments, ring C is isoxazolyl. In some specific embodiments, ring C is furanyl.

In some embodiments, the structural fragment 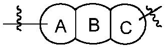 is selected from the group consisting of

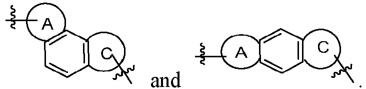

In some embodiments, the structural fragment 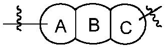 is selected from the group consisting of 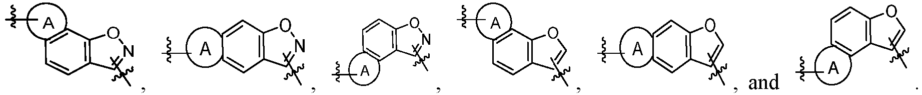 In some embodiments, the structural fragment 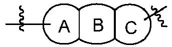 is selected from the group consisting of 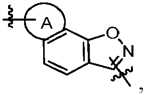 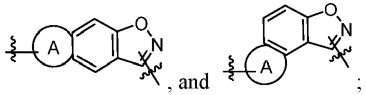 or the structural fragment 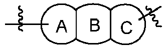 is selected from the group consisting of 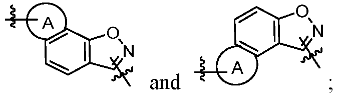 or the structural fragment 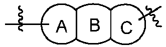 is selected from

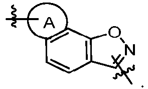

In some embodiments, the structural fragment 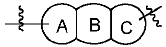 is selected from the group consisting of 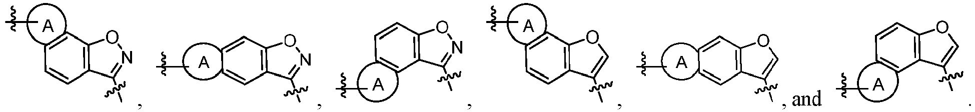 In some embodiments, the structural fragment 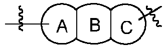 is selected from the group consisting of 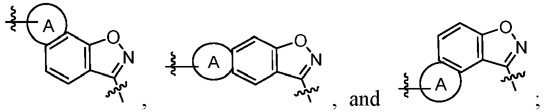 or the structural fragment 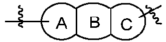 is selected from the group consisting of 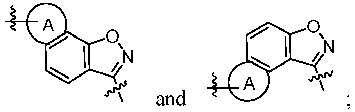 or the structural fragment 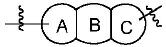 is selected from

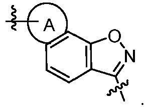

In some embodiments, the structural fragment 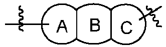 is selected from the group consisting of

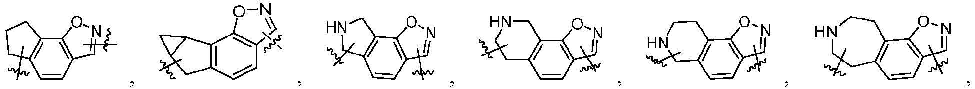

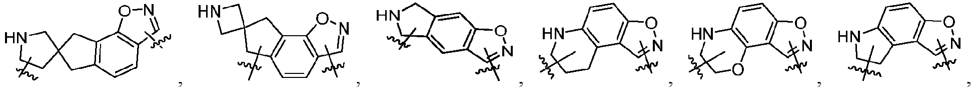

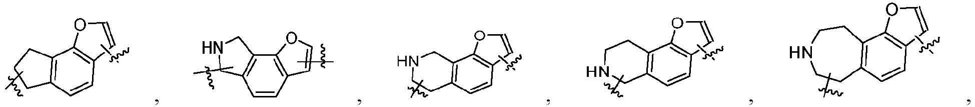

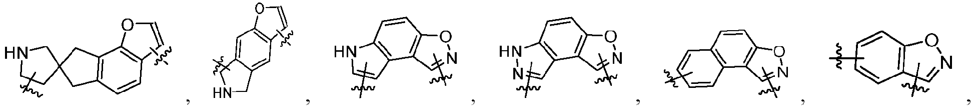

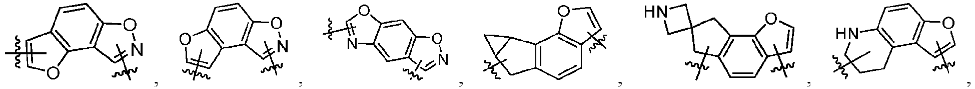

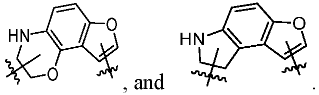

In some embodiments, the structural fragment 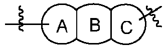 is selected from the group consisting of 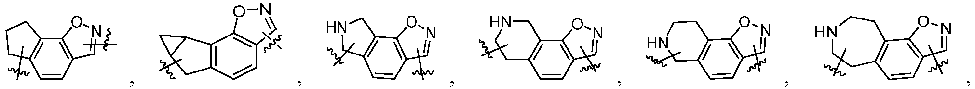 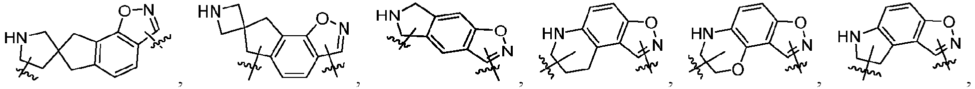 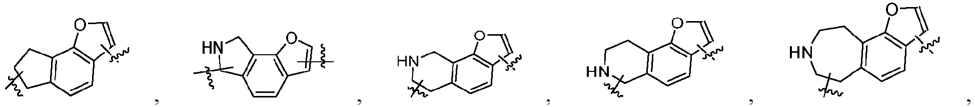 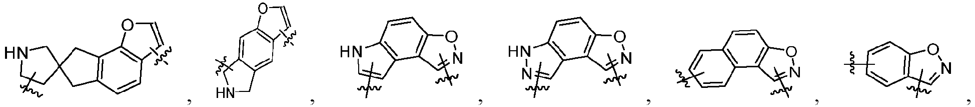 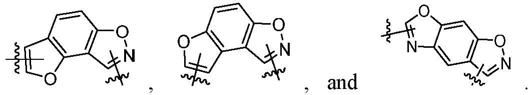 In some embodiments, the structural fragment 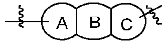 is selected from the group consisting of

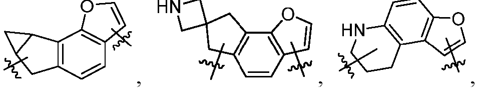

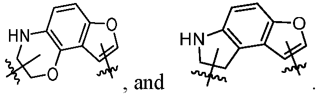

In some specific embodiments, the structural fragment 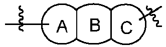 is selected from the group consisting of 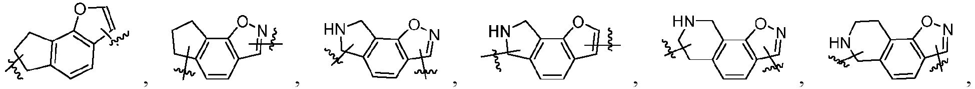 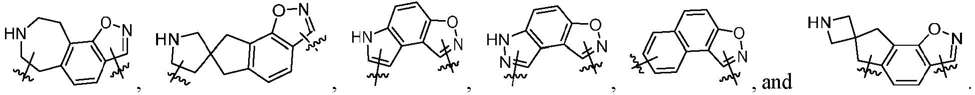 In some specific embodiments, the structural fragment 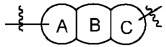 is selected from the group consisting of 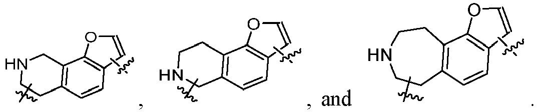 In some more specific embodiments, the structural fragment 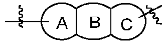 is selected from the group consisting of 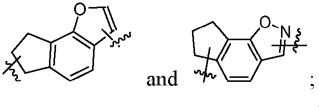 or the structural fragment 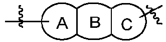 is selected from the group consisting of 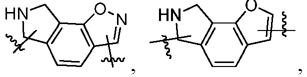 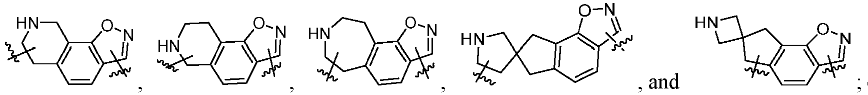 or the structural fragment 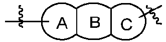 is selected from the group consisting of 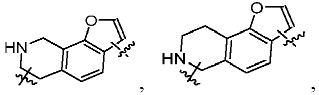 and 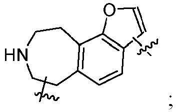 or the structural fragment 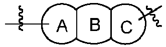 is selected from the group consisting of

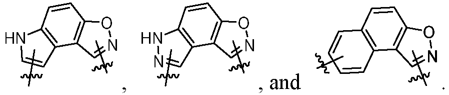

In some embodiments, the structural fragment 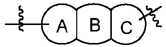 is selected from the group consisting of

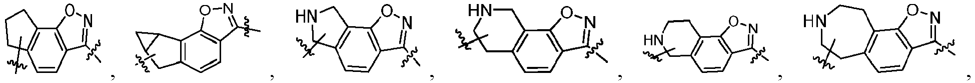

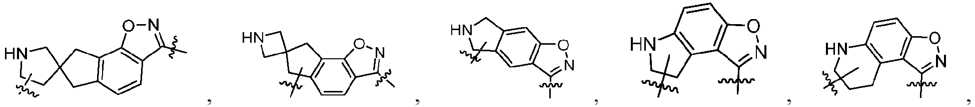

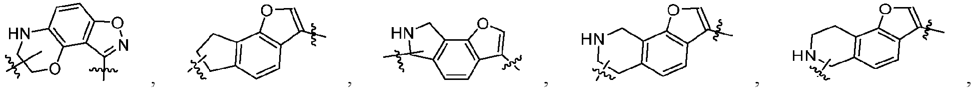

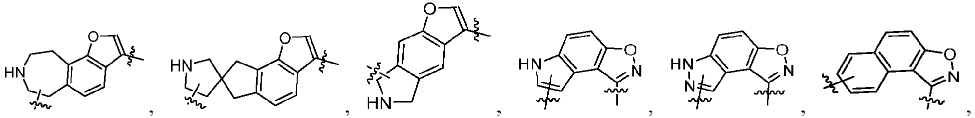

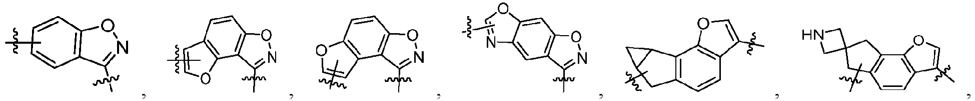

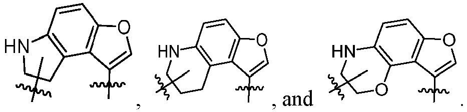

In some embodiments, the structural fragment 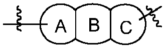 is selected from the group consisting of 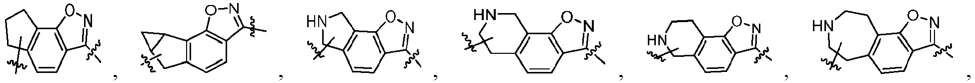 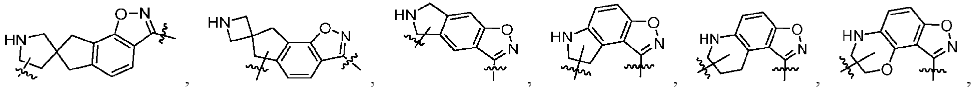 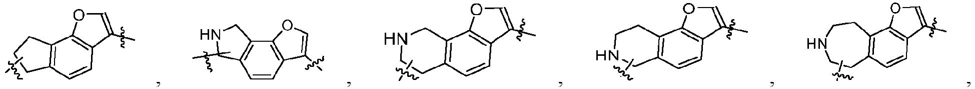 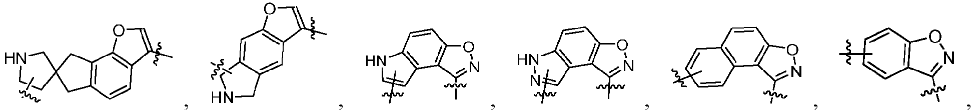 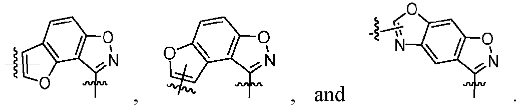 In some embodiments, the structural fragment 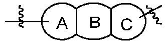 is selected from the group consisting of

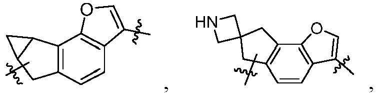

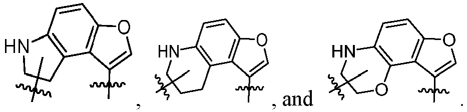

In some specific embodiments, the structural fragment 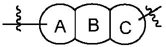 is selected from the group consisting of 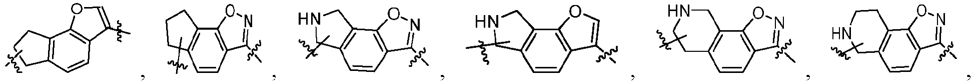 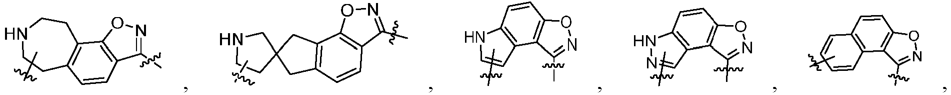 and 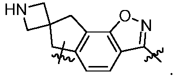 In some specific embodiments, the structural fragment 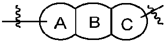 is selected from the group consisting of 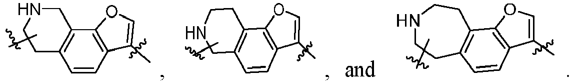 In some more specific embodiments, the structural fragment 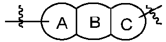 is selected from the group consisting of 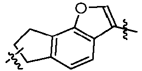 and 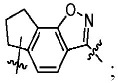 or the structural fragment 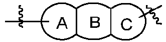 is selected from the group consisting of 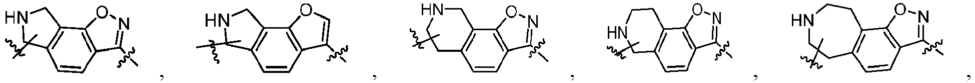 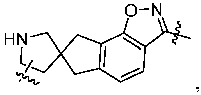 and 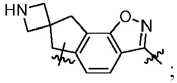 or the structural fragment 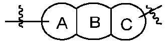 is selected from the group consisting of 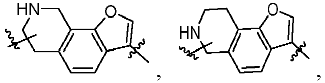 and 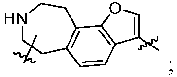 or the structural fragment 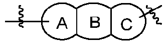 is selected from the group consisting of

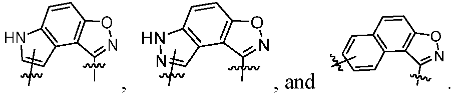

In some embodiments, the structural fragment 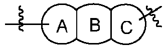 is selected from the group consisting of

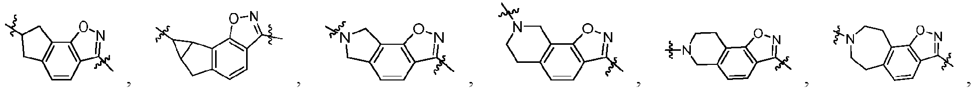

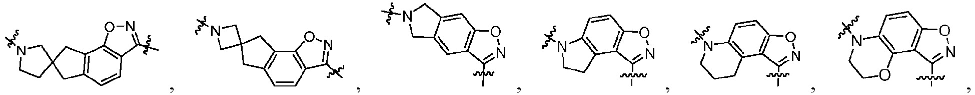

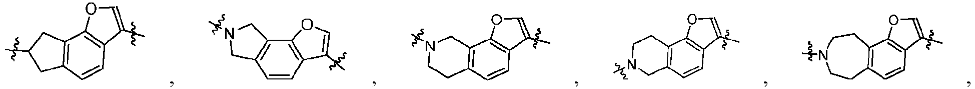

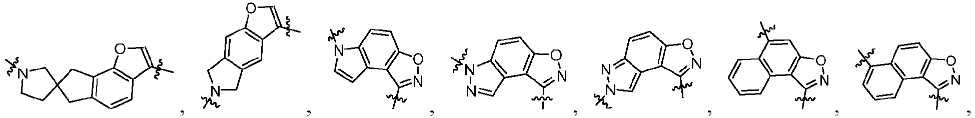

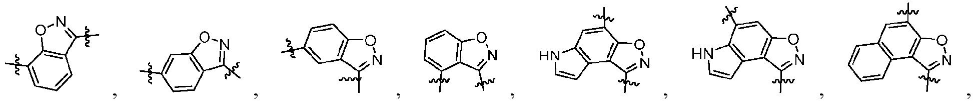

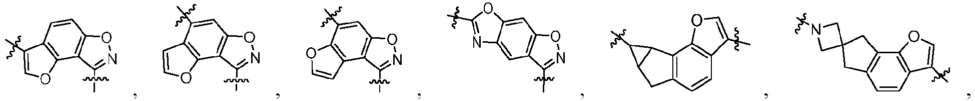

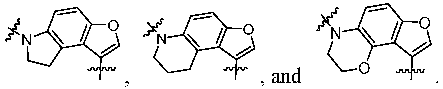

In some embodiments, the structural fragment 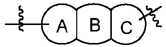 is selected from the group consisting of 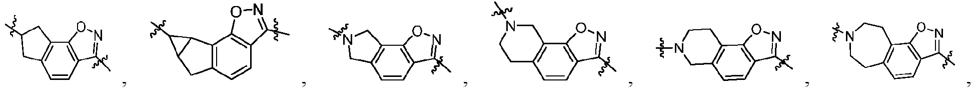 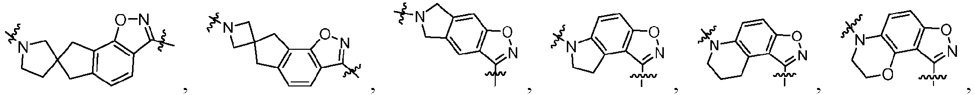 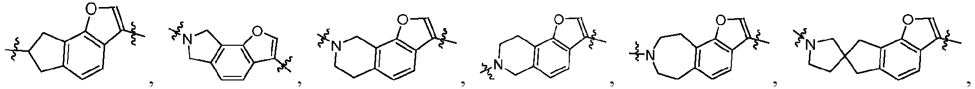 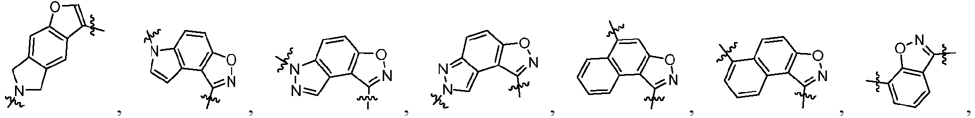 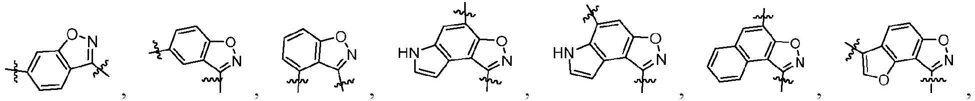 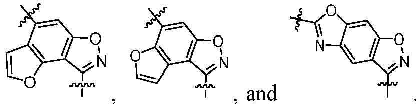 In some embodiments, the structural fragment 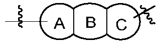 is selected from the group consisting of 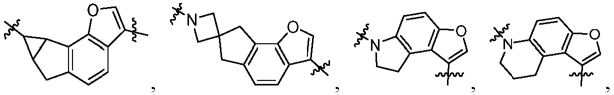 and

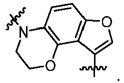

In some specific embodiments, the structural fragment 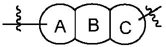 is selected from the group consisting of 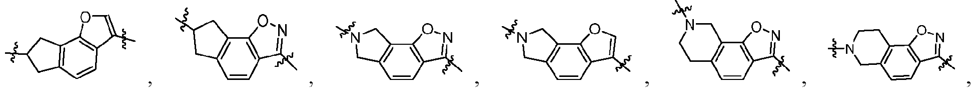 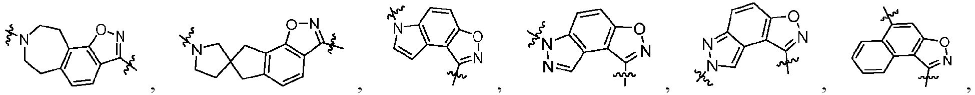 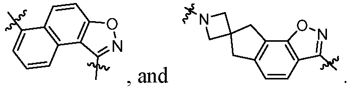 In some specific embodiments, the structural fragment 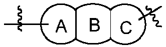 is selected from the group consisting of

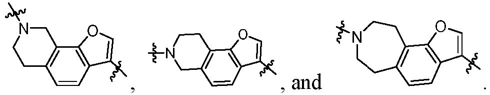

In some more specific embodiments, the structural fragment 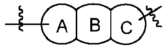 is selected from the group consisting of 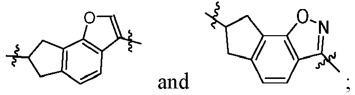 or the structural fragment 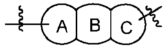 is selected from the group consisting of 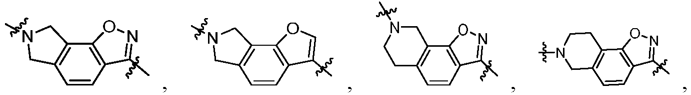 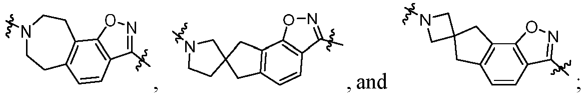 or the structural fragment 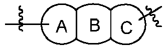 is selected from the group consisting of 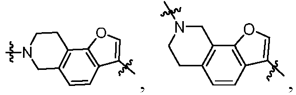 and 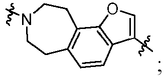 or the structural fragment 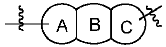 is selected from the group consisting of

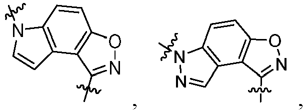

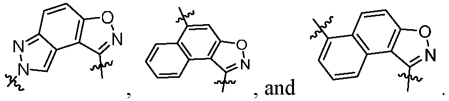

In some embodiments, each R¹ is independently selected from the group consisting of halogen, -OH, -NH₂, -CN, C₁₋₃ alkyl, C₁₋₃ alkoxy, and halogenated C₁₋₃ alkyl. In some embodiments, each R¹ is independently selected from the group consisting of fluorine, chlorine, bromine, -OH, -NH₂, and -CN. In some embodiments, each R¹ is independently selected from the group consisting of fluorine, chlorine, and bromine. In some embodiments, each R¹ is independently selected from fluorine.

In some embodiments, n is selected from the group consisting of 0, 1, and 2. In some embodiments, n is selected from the group consisting of 0 and 1.

In some specific embodiments, n is selected from 0.

In some embodiments, the structural fragment 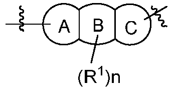 is selected from the group consisting of

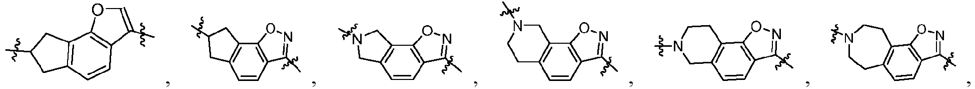

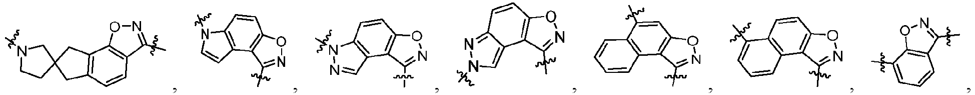

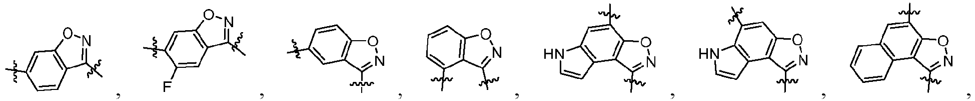

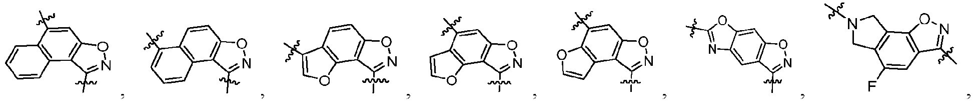

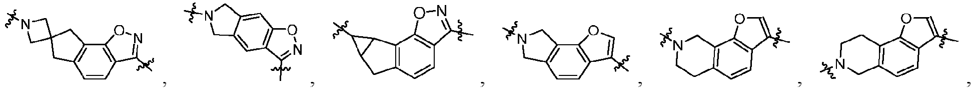

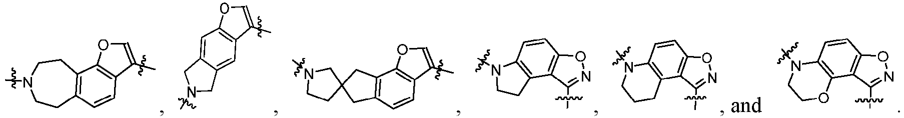

In some specific embodiments, the structural fragment 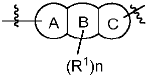 is selected from the group consisting of 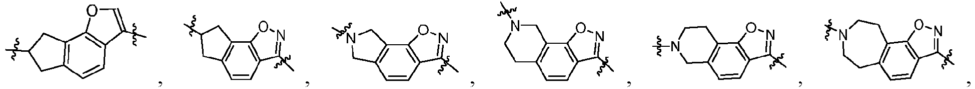 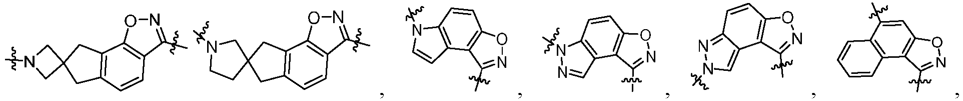 and 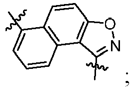 in some specific embodiments, the structural fragment 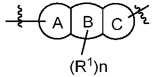 is selected from the group consisting of 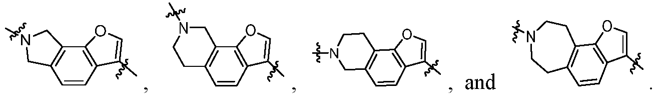 In some more specific embodiments, the structural fragment 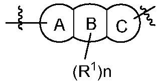 is selected from the group consisting of 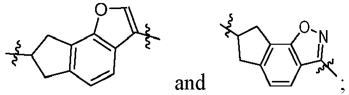 or the structural fragment 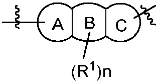 is selected from the group consisting of 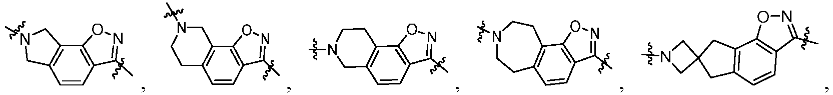 and 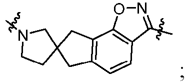 or the structural fragment 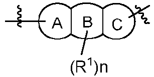 is selected from the group consisting of 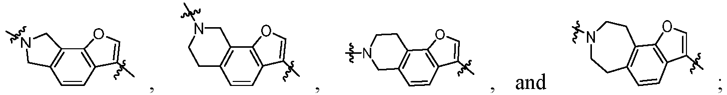 or the structural fragment 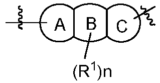 is selected from the group consisting of

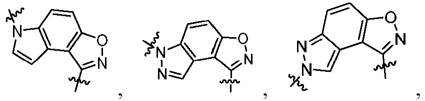

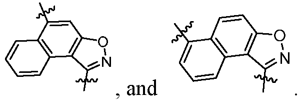

In some embodiments, L is selected from the group consisting of C₁₋₃₀ alkylene, C₂₋₃₀ alkenylene, and C₂₋₃₀ alkynylene, wherein one or more -CH₂- of the C₁₋₃₀ alkylene, C₂₋₃₀ alkenylene, or C₂₋₃₀ alkynylene are optionally substituted with -O-, C₃₋₁₂ cycloalkyl, 3- to 12-membered heterocycloalkyl, 4- to 12-membered heterocycloalkenyl, C₆₋₁₂ aryl, 5- to 12-membered heteroaryl, -NH-, -N(C₁₋₆ alkyl)-, or -S-, and the C₁₋₃₀ alkylene, C₂₋₃₀ alkenylene, or C₂₋₃₀ alkynylene is optionally substituted with one or more substituents.

In some embodiments, L is selected from the group consisting of C₁₋₂₀ alkylene, C₂₋₂₀ alkenylene, and C₂₋₂₀ alkynylene, wherein one or more -CH₂- of the C₁₋₂₀ alkylene, C₂₋₂₀ alkenylene, or C₂₋₂₀ alkynylene are optionally substituted with -O-, C₃₋₁₀ cycloalkyl, 3- to 10-membered heterocycloalkyl, 4- to 10-membered heterocycloalkenyl, C₆₋₁₀ aryl, 5- to 10-membered heteroaryl, -NH-, -N(C₁₋₆ alkyl)-, or -S-, and the C₁₋₂₀ alkylene, C₂₋₂₀ alkenylene, or C₂₋₂₀ alkynylene is optionally substituted with one or more substituents.

In some embodiments, L is selected from the group consisting of C₁₋₁₅ alkylene, C₂₋₁₅ alkenylene, and C₂₋₁₅ alkynylene, wherein one or more -CH₂- of the C₁₋₁₅ alkylene, C₂₋₁₅ alkenylene, or C₂₋₁₅ alkynylene are optionally substituted with -O-, C₃₋₈ cycloalkyl, 3- to 8-membered heterocycloalkyl, 4- to 8-membered heterocycloalkenyl, C₆₋₈ aryl, 5- to 8-membered heteroaryl, -NH-, -N(C₁₋₄ alkyl)-, or -S-, and the C₁₋₂₀ alkylene, C₂₋₁₅ alkenylene, or C₂₋₁₅ alkynylene is optionally substituted with one or more substituents.

In some embodiments, L is selected from the group consisting of C₁₋₁₀ alkylene, C₂₋₁₀ alkenylene, and C₂₋₁₀ alkynylene, wherein one or more -CH₂- of the C₁₋₁₀ alkylene, C₂₋₁₀ alkenylene, or C₂₋₁₀ alkynylene are optionally substituted with -O-, C₃₋₆ cycloalkyl, 3- to 6-membered heterocycloalkyl, 4- to 6-membered heterocycloalkenyl, C₆ aryl, 5- to 6-membered heteroaryl, -NH-, -N(C₁₋₃ alkyl)-, or -S-, and the C₁₋₁₀ alkylene, C₂₋₁₀ alkenylene, or C₂₋₁₀ alkynylene is optionally substituted with one or more substituents.

In some embodiments, L is selected from the group consisting of C₁₋₆ alkylene, C₂₋₆ alkenylene, and C₂₋₆ alkynylene, wherein one or more -CH₂- of the C₁₋₆ alkylene, C₂₋₆ alkenylene, or C₂₋₆ alkynylene are optionally substituted with -O-, C₃₋₆ cycloalkyl, 3- to 6-membered heterocycloalkyl, 4- to 6-membered heterocycloalkenyl, C₆ aryl, 5- to 6-membered heteroaryl, -NH-, -N(C₁₋₃ alkyl)-, or -S-, and the C₁₋₆ alkylene, C₂₋₆ alkenylene, or C₂₋₆ alkynylene is optionally substituted with one or more substituents.

In some embodiments, L is selected from the group consisting of C₁₋₄ alkylene, C₂₋₄ alkenylene, and C₂₋₄ alkynylene, wherein one or more (e.g., 1 or 2, 1 or 3, etc.) -CH₂- of the C₁₋₄ alkylene, C₂₋₄ alkenylene, or C₂₋₄ alkynylene are optionally substituted with -O-, C₄₋₆ cycloalkyl, 4- to 6-membered heterocycloalkyl, 4- to 6-membered heterocycloalkenyl, C₆ aryl, 5- to 6-membered heteroaryl, -NH-, -N(C₁₋₃ alkyl)-, or -S-, and the C₁₋₄ alkylene, C₂₋₄ alkenylene, or C₂₋₄ alkynylene is optionally substituted with one or more substituents.

In some embodiments, L is selected from the group consisting of C₁₋₆ alkylene, wherein one or more -CH₂- of the C₁₋₆ alkylene are optionally substituted with a group selected from the group consisting of -O-, C₃₋₁₀ cycloalkyl, 4-to 10-membered heterocycloalkyl, 4- to 10-membered heterocycloalkenyl, -NH-, -N(C₁₋₃ alkyl)-, and -S-, and the C₁₋₆ alkylene is optionally substituted with one or more substituents.

In some embodiments, in the definition of L, the substituent is selected from the group consisting of =O, OH, NH₂, halogen, CN, C₁₋₆ alkyl, and C₁₋₆ alkoxy. In some embodiments, in the definition of L, the substituent is selected from the group consisting of =O, OH, NH₂, halogen, and CN.

In some embodiments, L is selected from -LNK¹-Cy¹-LNK-Cy²-LNK²-, wherein,
Cy¹ is selected from the group consisting of a bond and the following groups optionally substituted with one or more R^{a}: C₃₋₁₂ cycloalkyl, 4- to 12-membered heterocycloalkyl, and 4- to 12-membered heterocycloalkenyl; LNK, LNK¹, and LNK² are each independently selected from the group consisting of a bond, C₁₋₁₂ alkylene, and C₁₋₁₂ heteroalkylene;
Cy² is selected from the group consisting of a bond and the following groups optionally substituted with one or more R^{b}: C₃₋₁₂ cycloalkyl, 4- to 12-membered heterocycloalkyl, and 4- to 12-membered heterocycloalkenyl;
each R^{a} and R^{b} is independently selected from the group consisting of halogen, -OH, -NH₂, -CN, C₁₋₄ alkyl, C₁₋₄ alkoxy, halogenated C₁₋₄ alkyl, C₁₋₄ alkylamino, di-C₁₋₄ alkylamino, C₃₋₁₂ cycloalkyl, and 4- to 12-membered heterocycloalkyl.

In some embodiments, L is selected from -LNK¹-Cy¹-LNK-Cy²-LNK²-, wherein,
Cy¹ is selected from the group consisting of a bond and the following groups optionally substituted with one or more R^{a}: C₃₋₁₂ cycloalkyl and 4- to 12-membered heterocycloalkyl;
LNK, LNK¹, and LNK² are each independently selected from the group consisting of a bond, C₁₋₁₂ alkylene, and C₁₋₁₂ heteroalkylene;
Cy² is selected from the group consisting of a bond and the following groups optionally substituted with one or more R^{b}: C₃₋₁₂ cycloalkyl and 4- to 12-membered heterocycloalkyl;
each R^{a} and R^{b} is independently selected from the group consisting of halogen, -OH, -NH₂, -CN, C₁₋₄ alkyl, C₁₋₄ alkoxy, halogenated C₁₋₄ alkyl, C₁₋₄ alkylamino, and di-C₁₋₄ alkylamino.

In some embodiments, L is selected from -LNK¹-Cy¹-LNK-Cy²-LNK²-, wherein Cy¹ and Cy² are not bonds at the same time.

In some embodiments, L is selected from the group consisting of -Cy¹-LNK-Cy²-LNK²-, -LNK¹-Cy¹-Cy²-LNK²-, - Cy¹-LNK-Cy²-, -Cy¹-Cy²-LNK²-, -LNK-Cy²-LNK²-, -Cy¹-LNK-, -Cy¹-Cy²-, and -Cy²-.

In some embodiments, L is selected from the group consisting of -Cy¹-LNK-Cy²- and -Cy¹-Cy²-LNK²-. In some embodiments, L is selected from -Cy¹-LNK-Cy²-. In some embodiments, L is selected from -Cy¹-Cy²-LNK²-. In some embodiments, L is selected from -Cy¹-LNK-Cy²-LNK²-.

In some embodiments, L or -Cy¹-LNK-Cy²- is selected from the group consisting of -Cy¹-, -Cy¹-LNK-, -Cy¹-Cy²-, -Cy¹-LNK-Cy²-, -Cy²-, and -LNK-Cy²-. In some embodiments, L or -Cy¹-LNK-Cy²- is selected from the group consisting of -Cy¹-, -Cy¹-Cy²-, and -Cy²-. In some specific embodiments, L or -Cy¹-LNK-Cy²- is selected from the group consisting of -Cy¹-LNK-, -Cy¹-LNK-Cy²-, and -LNK-Cy²-. In some specific embodiments, L or -Cy¹-LNK-Cy²- is selected from -Cy¹-LNK-. In some specific embodiments, L or -Cy¹-LNK-Cy²- is selected from -Cy¹-LNK-Cy²-. In some specific embodiments, L or -Cy¹-LNK-Cy²- is selected from -LNK-Cy²-. In some embodiments, Cy¹ is selected from the group consisting of the following groups optionally substituted with one or more R^{a}: C₃₋₁₂ cycloalkyl and 4- to 12-membered heterocycloalkyl; Cy² is selected from the group consisting of a bond and the following groups optionally substituted with one or more R^{b}: C₃₋₁₂ cycloalkyl and 4- to 12-membered heterocycloalkyl.

In some embodiments, LNK, LNK¹, and LNK² are selected from a bond. In some embodiments, LNK¹ is a bond. In some embodiments, LNK is selected from the group consisting of C₁₋₆ alkylene and C₁₋₆ heteroalkylene, and LNK¹ and LNK² are bonds.

In some embodiments, LNK² is selected from the group consisting of C₁₋₆ alkylene and C₁₋₆ heteroalkylene, and LNK and LNK¹ are bonds.

In some embodiments, LNK¹ and LNK² are selected from a bond, LNK is selected from the group consisting of a bond, C₁₋₆ alkylene, and C₁₋₆ heteroalkylene, Cy² is a bond, and Cy¹ is selected from the group consisting of the following groups optionally substituted with one or more R^{a}: C₄₋₁₁ cycloalkyl and 4- to 11-membered heterocycloalkyl.

In some embodiments, LNK is selected from the group consisting of C₁₋₆ alkylene and C₁₋₆ heteroalkylene, and LNK¹ and LNK² are bonds;
Cy² is a bond, and Cy¹ is selected from the group consisting of the following groups optionally substituted with one or more R^{a}: C₄₋₁₁ cycloalkyl and 4- to 11-membered heterocycloalkyl.

In some embodiments, LNK is selected from the group consisting of C₁₋₆ alkylene and C₁₋₆ heteroalkylene, and LNK¹ and LNK² are bonds;
Cy¹ is a bond, and Cy² is selected from the group consisting of the following groups optionally substituted with one or more R^{b}: C₄₋₁₁ cycloalkyl and 4- to 11-membered heterocycloalkyl.

In some embodiments, LNK is selected from the group consisting of C₁₋₆ alkylene and C₁₋₆ heteroalkylene, and LNK¹ and LNK² are bonds;
Cy¹ is selected from the group consisting of the following groups optionally substituted with one or more R^{a}: C₄₋₁₁ cycloalkyl and 4- to 11-membered heterocycloalkyl, and Cy² is selected from the group consisting of the following groups optionally substituted with one or more R^{b}: C₄₋₁₁ cycloalkyl and 4- to 11-membered heterocycloalkyl.

In some embodiments, Cy¹ is a bond or the following groups optionally substituted with one or more R^{a}: C₄₋₁₁ cycloalkyl, 4- to 11-membered heterocycloalkyl, and 4- to 11-membered heterocycloalkenyl.

In some embodiments, Cy¹ is a bond. In some embodiments, Cy¹ is selected from the group consisting of the following groups optionally substituted with one or more R^{a}: C₆₋₉ cycloalkyl, 4- to 11-membered heterocycloalkyl, or 4- to 9-membered heterocycloalkenyl (e.g., 5- to 7-membered heterocycloalkenyl).

In some embodiments, Cy¹ is selected from the group consisting of the following groups optionally substituted with one or more R^{a}: C₆ cycloalkyl, C₉ cycloalkyl, 4-membered, 5-membered, 6-membered, 7-membered, 8-membered, 9-membered, 10-membered, and 11-membered heterocycloalkyl, and 6-membered heterocycloalkenyl.

In some embodiments, Cy¹ is selected from the group consisting of the following groups optionally substituted with one or more R^{a}: C₆ cycloalkyl, C₉ cycloalkyl, 4-membered, 6-membered, and 8- to 11-membered heterocycloalkyl, and 5- to 6-membered heterocycloalkenyl. In some embodiments, Cy¹ is selected from the group consisting of the following groups optionally substituted with one or more R^{a}: C₆ cycloalkyl, C₉ cycloalkyl, 4-membered, 6-membered, and 8- to 11-membered heterocycloalkyl, and 6-membered heterocycloalkenyl.

In some embodiments, Cy¹ is selected from the group consisting of a bond and the following groups optionally substituted with one or more R^{a}: C₄₋₁₁ cycloalkyl and 4- to 11-membered heterocycloalkyl.

In some embodiments, Cy¹ is a bond. In some embodiments, Cy¹ is selected from the group consisting of the following groups optionally substituted with one or more R^{a}: C₆₋₉ cycloalkyl and 4- to 11-membered heterocycloalkyl.

In some embodiments, Cy¹ is selected from the group consisting of the following groups optionally substituted with one or more R^{a}: C₆ cycloalkyl, C₉ cycloalkyl, and 4-membered, 5-membered, 6-membered, 7-membered, 8-membered, 9-membered, 10-membered, and 11-membered heterocycloalkyl.

In some embodiments, Cy¹ is selected from the group consisting of the following groups optionally substituted with one or more R^{a}: C₆ cycloalkyl, C₉ cycloalkyl, and 4-membered, 6-membered, and 8- to 11-membered heterocycloalkyl. In some embodiments, Cy¹ is selected from the group consisting of the following groups optionally substituted with one or more R^{a}: 4-membered and 5-membered heterocycloalkyl. In some embodiments, Cy¹ is selected from the group consisting of the following groups optionally substituted with one or more R^{a}: 6- to 9-membered heterocycloalkyl. In some embodiments, Cy¹ is selected from the group consisting of the following groups optionally substituted with one or more R^{a}: 6-membered, 8-membered, and 9-membered heterocycloalkyl. In some specific embodiments, Cy¹ is selected from the group consisting of the following groups optionally substituted with one or more R^{a}: 6-membered heterocycloalkyl and 9-membered heterocycloalkyl.

In some embodiments, Cy¹ is selected from the group consisting of the following groups optionally substituted with one or more R^{a}: piperidinyl, diazaspirononanyl, piperazinyl, monoazaspirononanyl, cyclohexyl, spirononanyl, azetidinyl, pyrrolidinyl, octahydrocyclopentapyrrolyl, azabicyclononanyl, monoazaspiroundecanyl, diazaspiroundecanyl, and tetrahydropyridinyl.

In some embodiments, Cy¹ is selected from the group consisting of the following groups optionally substituted with one or more R^{a}: piperidinyl, diazaspirononanyl, piperazinyl, monoazaspirononanyl, cyclohexyl, spirononanyl, azetidinyl, octahydrocyclopentapyrrolyl, azabicyclononanyl, monoazaspiroundecanyl, and diazaspiroundecanyl. In some specific embodiments, Cy¹ is selected from the group consisting of the following groups optionally substituted with one or more R^{a}: piperidinyl, diazaspirononanyl, piperazinyl, and monoazaspirononanyl. In some specific embodiments, Cy¹ is selected from the group consisting of the following groups optionally substituted with one or more R^{a}: azetidinyl, pyrrolidinyl, and tetrahydropyridinyl.

In some embodiments, Cy¹ is selected from the group consisting of the following groups optionally substituted with one or more R^{a}:

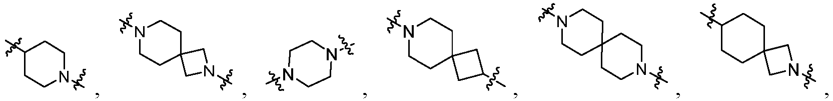

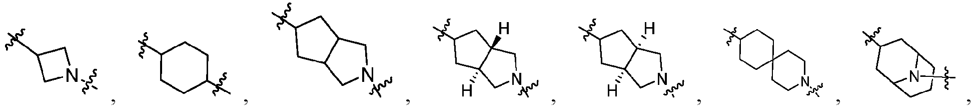

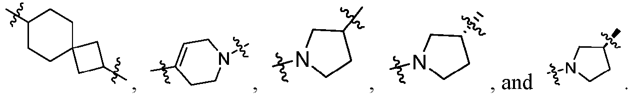

In some other embodiments, Cy¹ is selected from the group consisting of the following groups optionally substituted with one or more R^{a}: 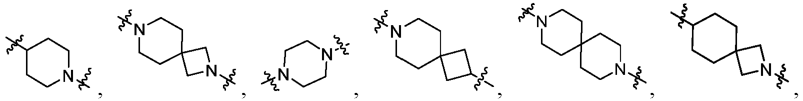 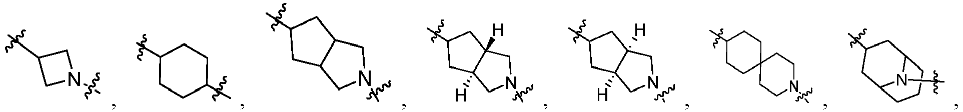 and

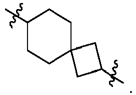

In some specific embodiments, Cy¹ is selected from the group consisting of the following groups optionally substituted with one or more R^{a}: 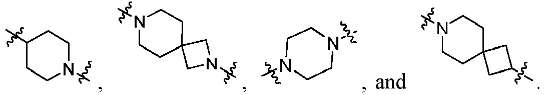 In some specific embodiments, Cy¹ is selected from the group consisting of the following groups optionally substituted with one or more R^{a}:

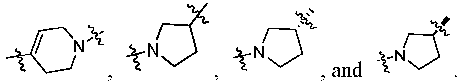

**In** some embodiments, LNK, LNK¹, and LNK² are each independently selected from the group consisting of a bond, C₁₋₆ alkylene, and C₁₋₆ heteroalkylene.

In some embodiments, LNK, LNK¹, and LNK² are each independently selected from the group consisting of a bond, C₁₋₃ alkylene, and C₁₋₃ heteroalkylene.

In some embodiments, LNK, LNK¹, and LNK² are each independently selected from the group consisting of a bond and C₁₋₄ alkylene.

In some embodiments, LNK, LNK¹, and LNK² are each independently selected from the group consisting of a bond and C₁₋₃ alkylene.

In some embodiments, LNK, LNK¹, and LNK² are each independently selected from the group consisting of a bond and -CH₂-. In some specific embodiments, LNK is a bond. In some specific embodiments, LNK, LNK¹, and LNK² are each independently -CH₂-.

In some embodiments, Cy² is selected from the group consisting of a bond and the following groups optionally substituted with one or more R^{b}: C₄₋₁₁ cycloalkyl and 4- to 11-membered heterocycloalkyl. In some specific embodiments, Cy² is a bond. In some specific embodiments, Cy² is selected from the group consisting of the following groups optionally substituted with one or more R^{b}: C₄₋₁₁ cycloalkyl and 4- to 11-membered heterocycloalkyl.

In some embodiments, Cy² is selected from the group consisting of a bond and the following groups optionally substituted with one or more R^{b}: C₄₋₆ cycloalkyl and 4- to 6-membered heterocycloalkyl. In some embodiments, Cy² is selected from the group consisting of the following groups optionally substituted with one or more R^{b}: C₄₋₆ cycloalkyl and 4- to 6-membered heterocycloalkyl.

In some embodiments, Cy² is selected from the group consisting of a bond and the following groups optionally substituted with one or more R^{b}: cyclobutyl, cyclopentyl, cyclohexyl, azetidinyl, pyrrolidinyl, and piperidinyl. In some specific embodiments, Cy² is selected from the group consisting of a bond and the following groups optionally substituted with one or more R^{b}: cyclobutyl, cyclopentyl, cyclohexyl, azetidinyl, and piperidinyl.

In some embodiments, Cy² is selected from the group consisting of a bond, 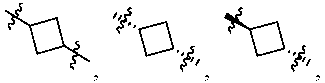 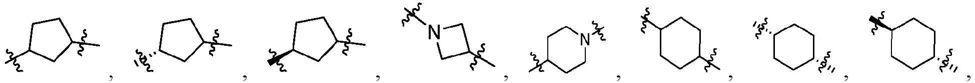 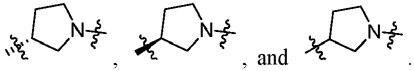 In some specific embodiments, Cy² is selected from the group consisting of a bond,

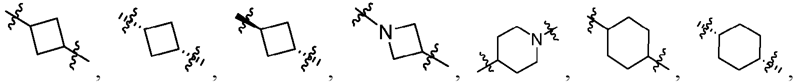

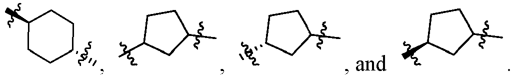

In some embodiments, R^{a} and R^{b} are each independently selected from the group consisting of halogen, -OH, -NH₂, -CN, C₁₋₃ alkyl, C₁₋₃ alkoxy, halogenated C₁₋₃ alkyl, C₁₋₃ alkylamino, and di-C₁₋₃ alkylamino.

In some embodiments, R^{a} and R^{b} are each independently selected from the group consisting of halogen, -OH, -NH₂, -CN, and C₁₋₃ alkyl.

In some embodiments, R^{a} and R^{b} are each independently selected from the group consisting of halogen, -OH, -NH₂, and -CN.

In some embodiments, the structural fragment -Cy¹-LNK- is selected from the group consisting of

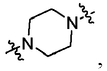

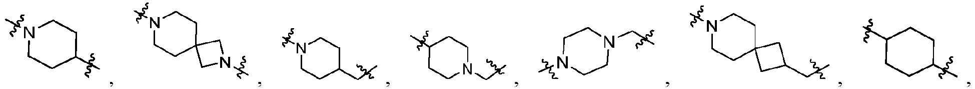

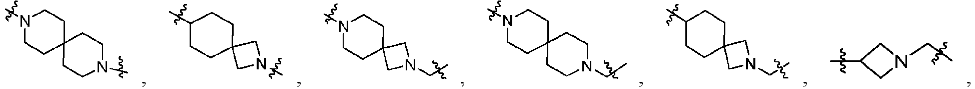

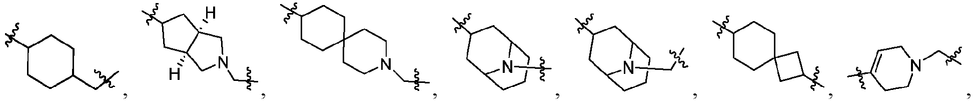

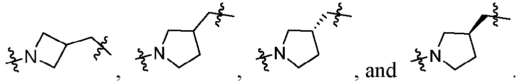

In some other embodiments, the structural fragment -Cy¹-LNK- is selected from the group consisting of

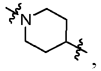

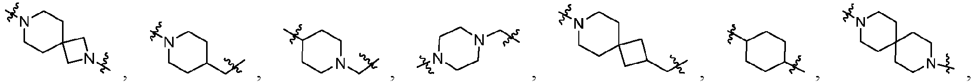

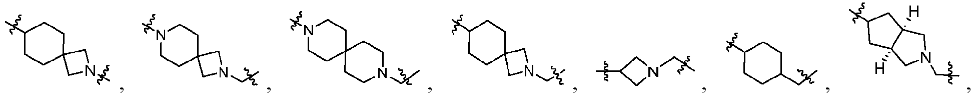

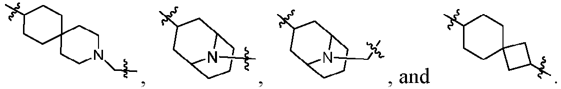

In some specific embodiments, the structural fragment -Cy¹-LNK- is selected from the group consisting of 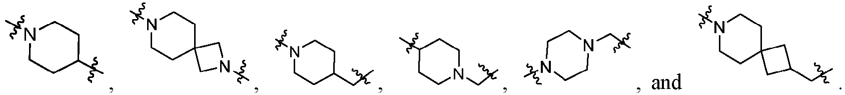 In some specific embodiments, the structural fragment -Cy¹-LNK- is selected from the group consisting of

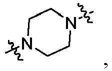

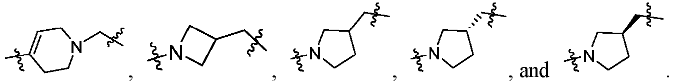

In some other embodiments, the structural fragment -LNK-Cy²- is selected from the group consisting of a bond, - CH₂-,

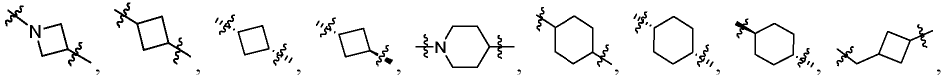

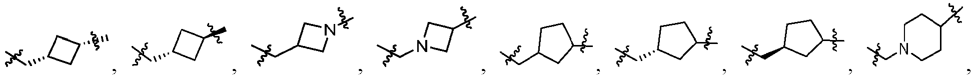

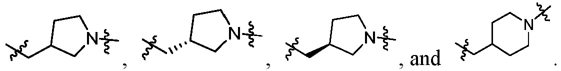

In some specific embodiments, the structural fragment -LNK-Cy²- is selected from the group consisting of a bond, -CH₂-,

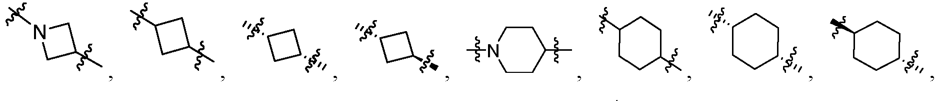

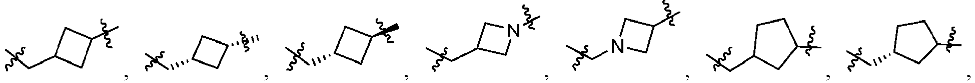

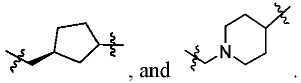

In some embodiments, the structural fragment -Cy¹-Cy²- is selected from the group consisting of

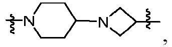

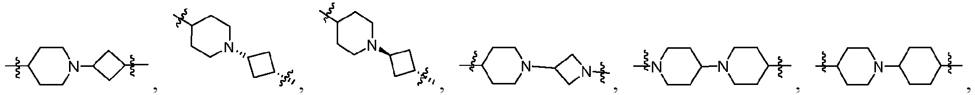

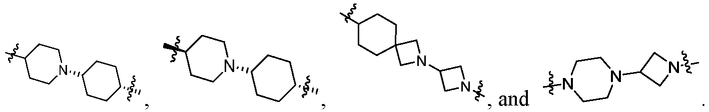

In some other embodiments, the structural fragment -Cy¹-Cy²- is selected from the group consisting of

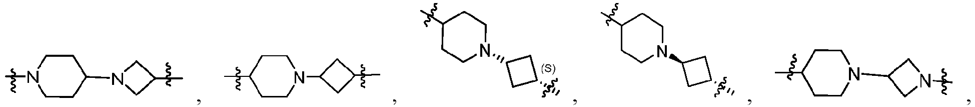

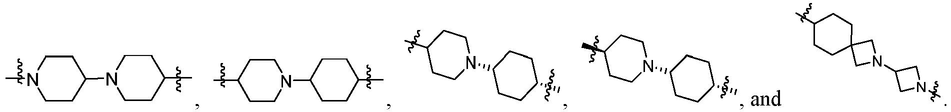

In some specific embodiments, the structural fragment -Cy¹-Cy²- is selected from the group consisting of

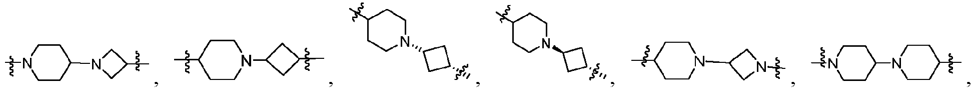

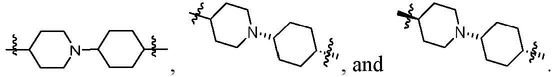

In some embodiments, the structural fragment -Cy¹-Cy²-LNK²- is selected from the group consisting of

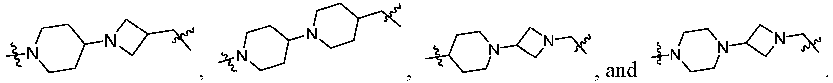

In some embodiments, the structural fragment or -Cy¹-Cy²-LNK²- is selected from the group consisting of

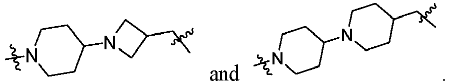

In some embodiments, the structural fragment -L- or -LNK¹-Cy¹-LNK-Cy²-LNK²- is selected from the group consisting of

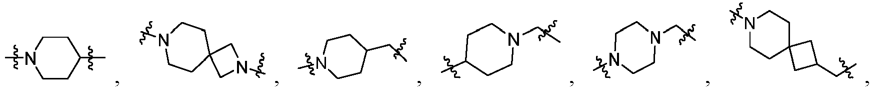

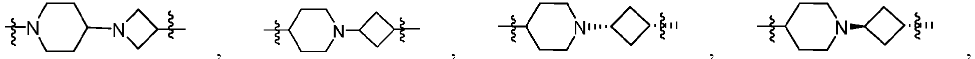

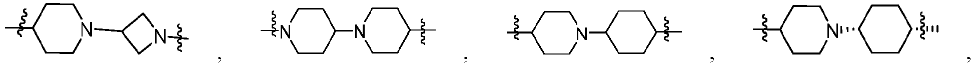

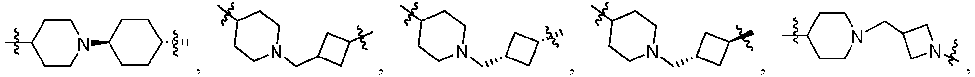

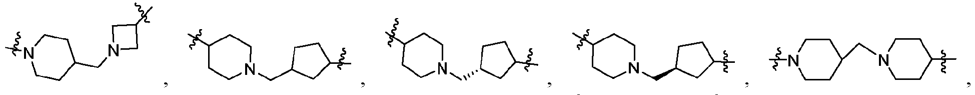

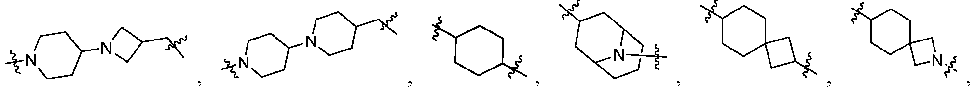

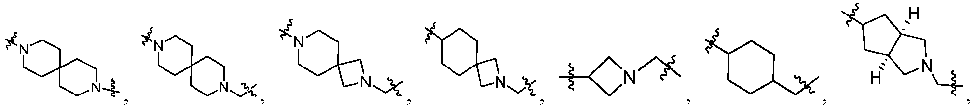

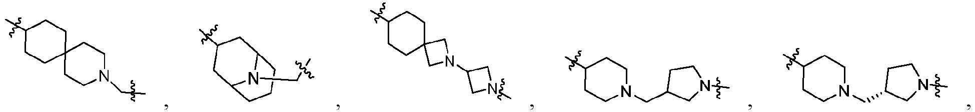

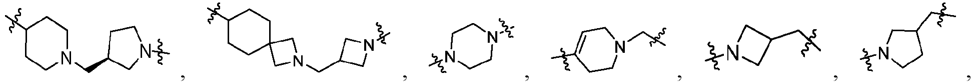

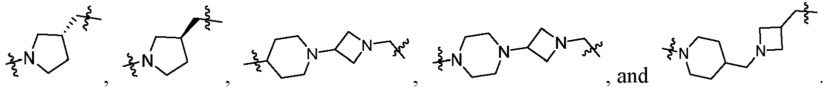

In some other embodiments, the structural fragment -L- or -LNK¹-Cy¹-LNK-Cy²-LNK²- is selected from the group consisting of

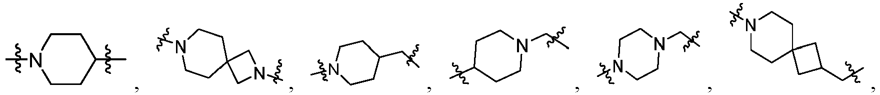

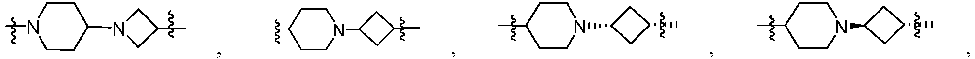

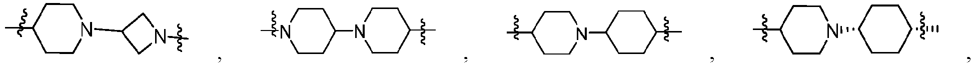

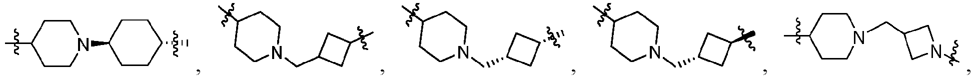

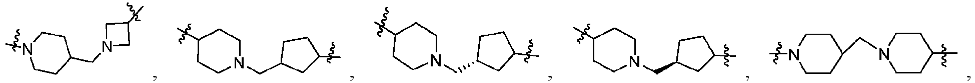

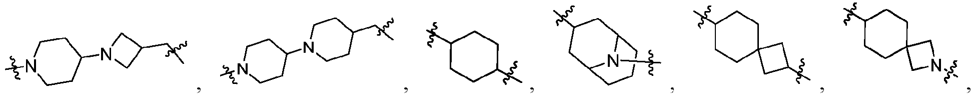

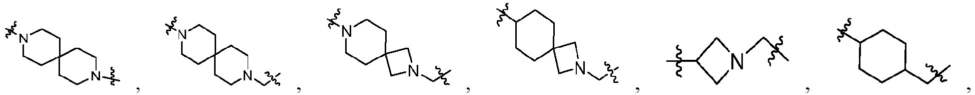

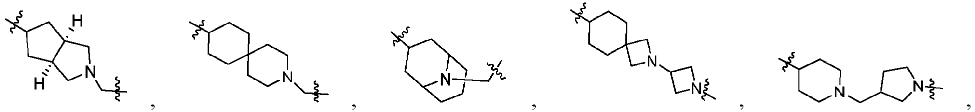

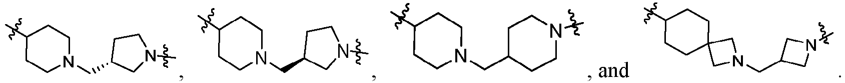

In some specific embodiments, the structural fragment -L- or -LNK¹-Cy¹-LNK-Cy²-LNK²- is selected from the group consisting of

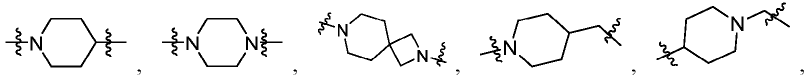

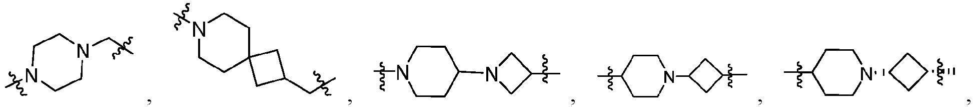

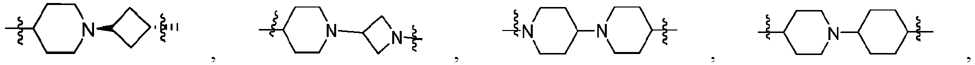

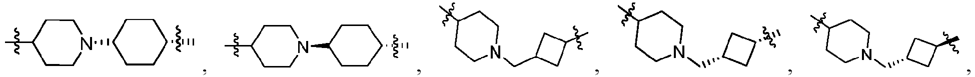

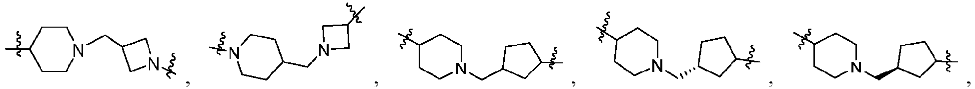

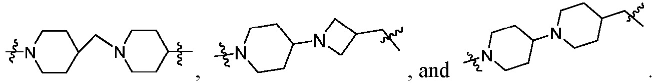

In some embodiments, the structural fragment 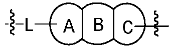 is selected from the group consisting of

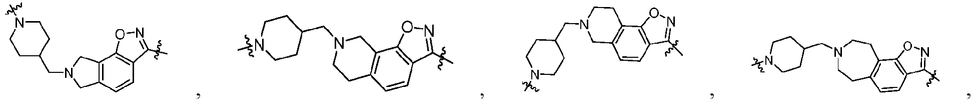

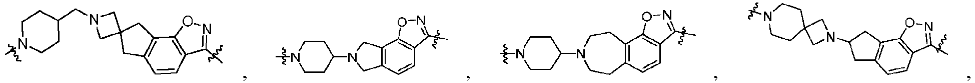

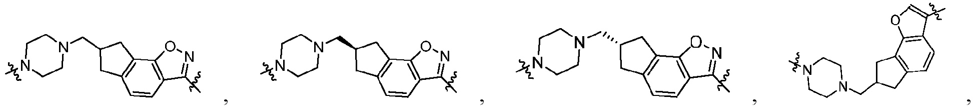

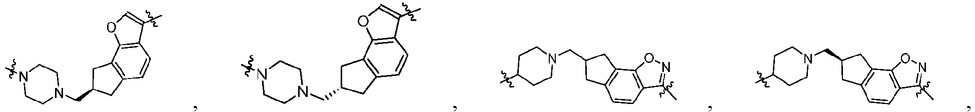

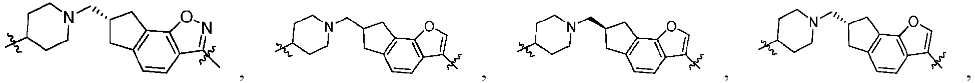

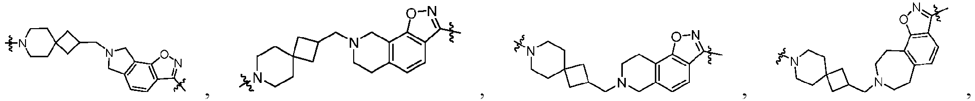

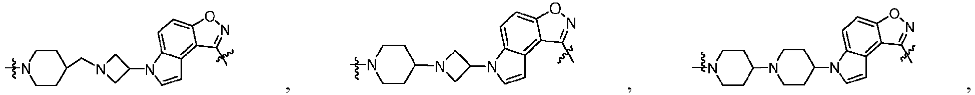

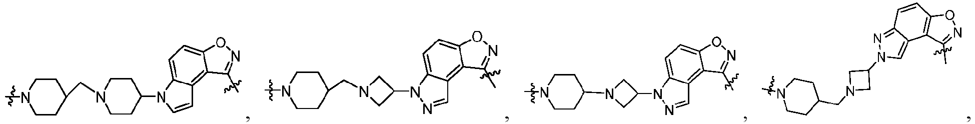

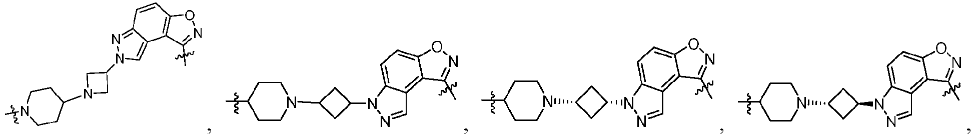

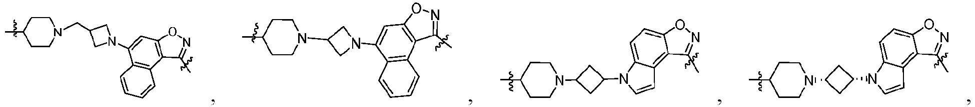

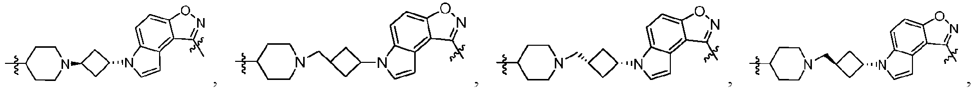

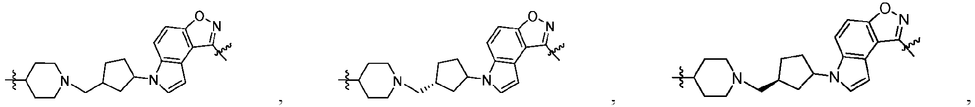

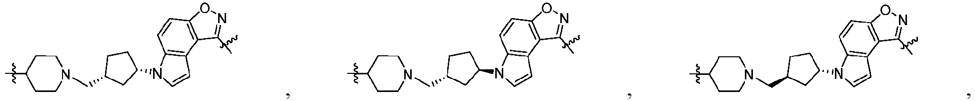

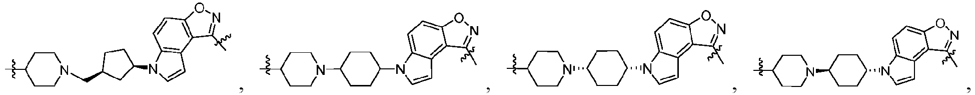

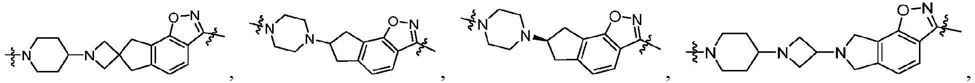

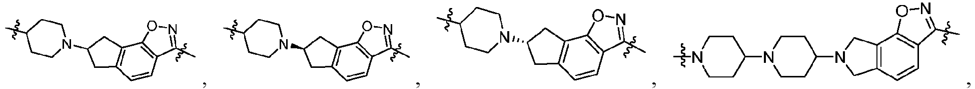

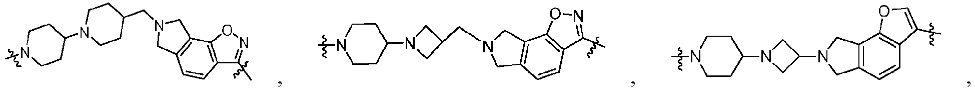

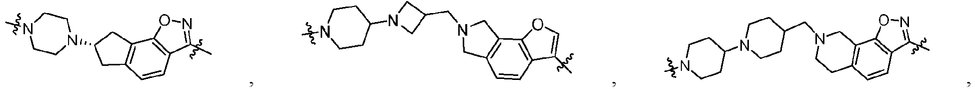

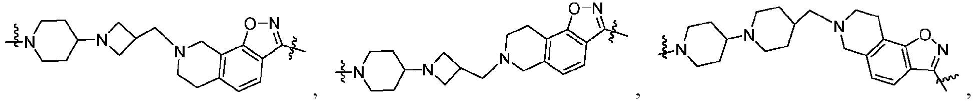

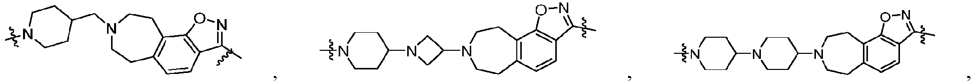

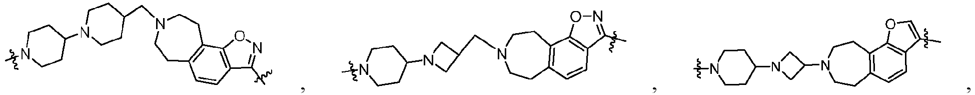

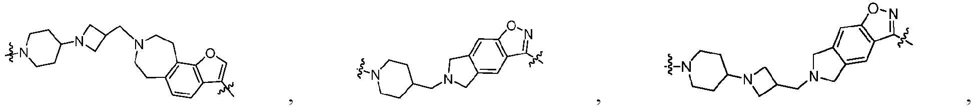

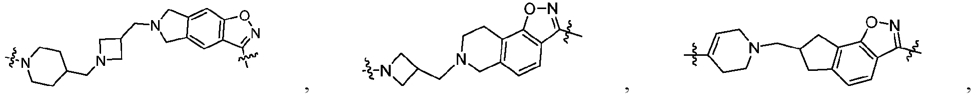

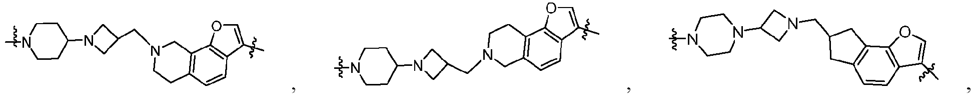

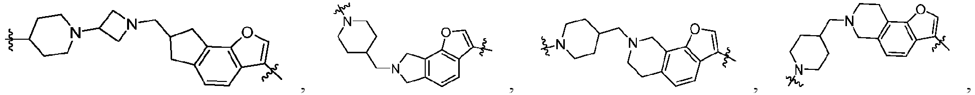

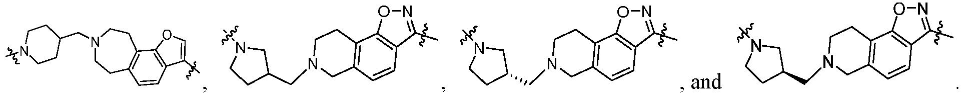

In some embodiments, the structural fragment 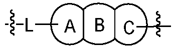 is selected from the group consisting of 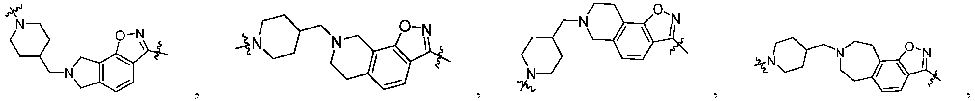 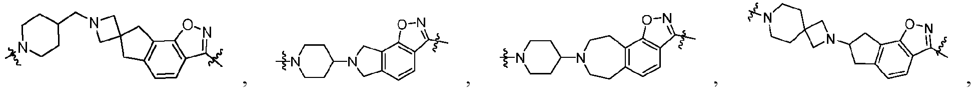 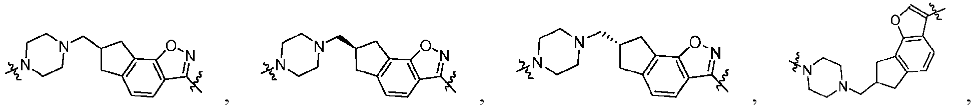 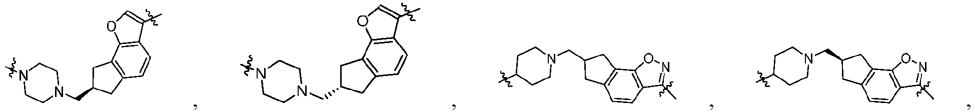 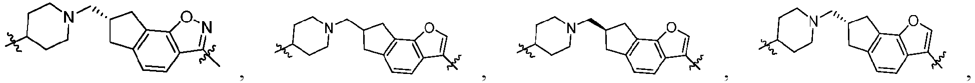 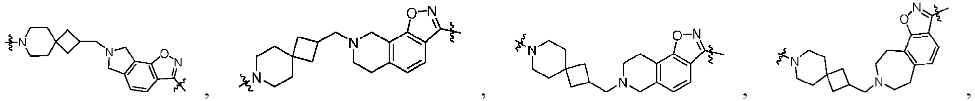 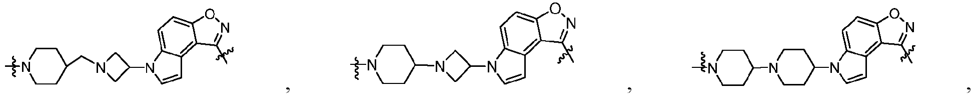 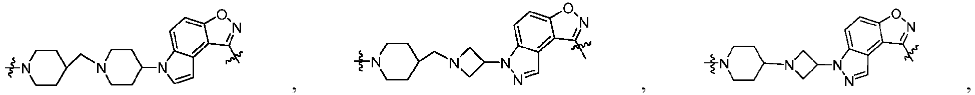 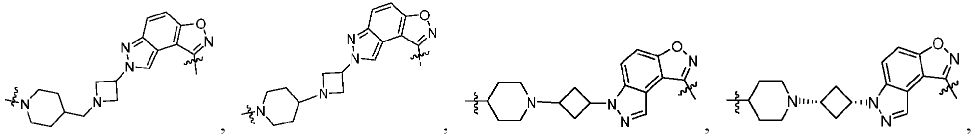 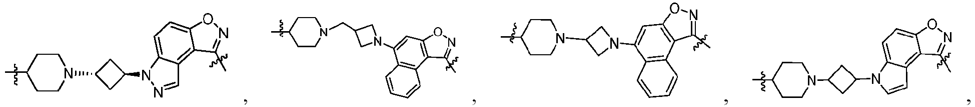 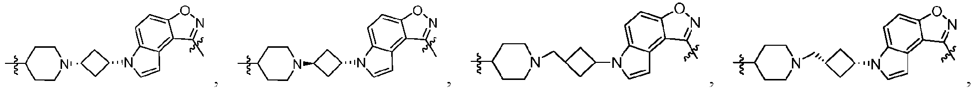 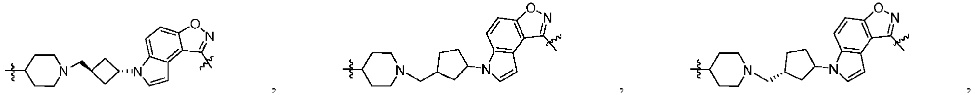 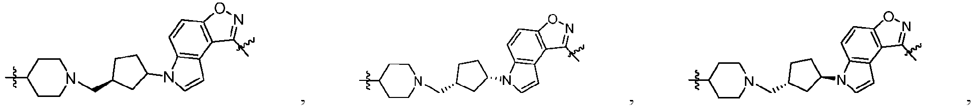 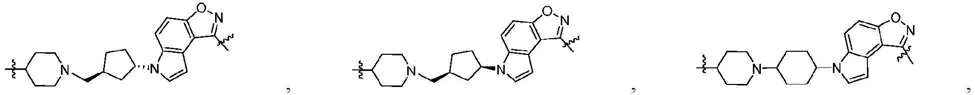 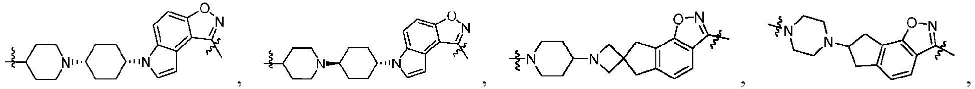 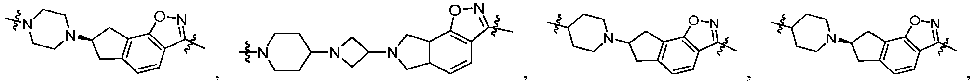 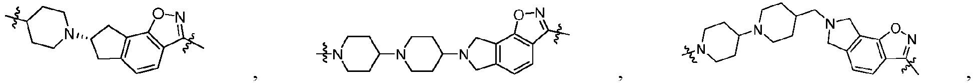 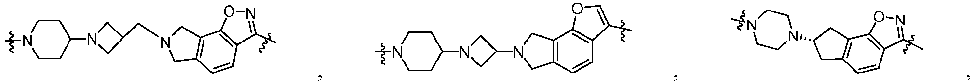 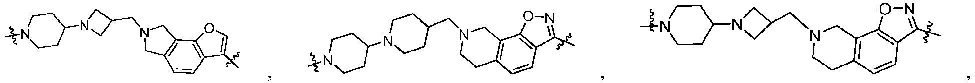 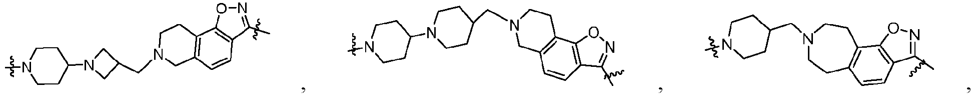 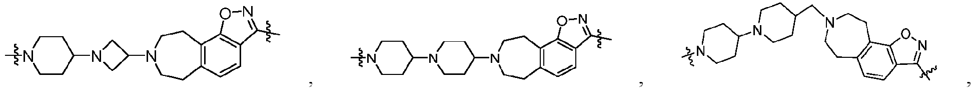 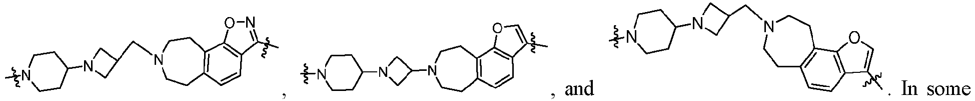 embodiments, the structural fragment 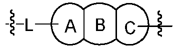 is selected from the group consisting of

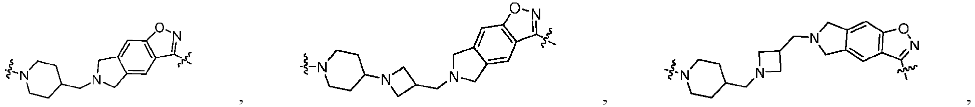

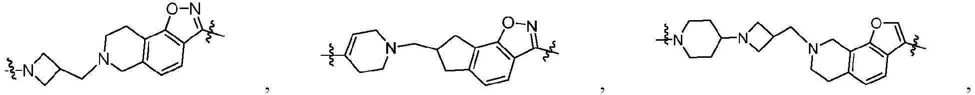

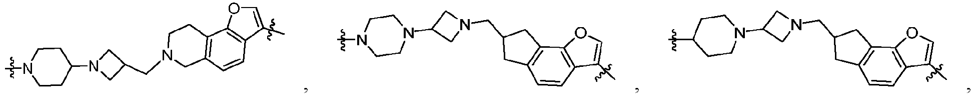

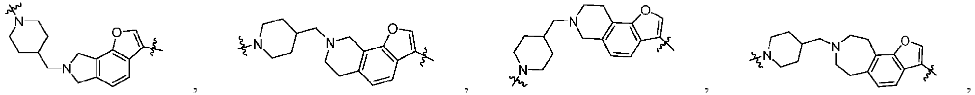

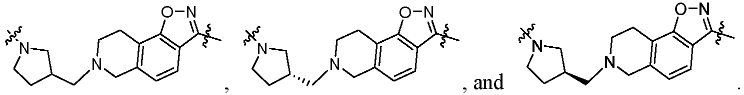

In some embodiments, X¹, X², and X³ are each independently selected from the group consisting of N and CH, and X⁴ is CH. In some embodiments, X¹ and X² are each independently selected from the group consisting of N and CH, and X³ and X⁴ are CH. In some embodiments, X¹ and X² are each independently N, and X³ and X⁴ are CH. In some embodiments, X¹, X², X³, and X⁴ are CH. In some embodiments, X¹ and X⁴ are CH, and X² and X³ are N. In some embodiments, X² and X³ are each independently CH, and X¹ and X⁴ are N. In some embodiments, X¹ and X³ are each independently CH, and X² and X⁴ are N. In some embodiments, X² and X³ are each independently selected from the group consisting of N and CH, and X¹ and X⁴ are CH. In some embodiments, X¹ and X³ are each independently selected from the group consisting of N and CH, X² is N, and X⁴ is CH.

In some embodiments, X⁵ is CH.

In some embodiments, X⁶ is selected from the group consisting of -O-, -NH-, and -N(C₁₋₃ alkyl)-. In some embodiments, X⁶ is selected from the group consisting of -O- and -N(CH₃)-.

In some embodiments, each R² is independently selected from the group consisting of halogen, -OH, -NH₂, -CN, C₁₋₃ alkyl, C₁₋₃ alkoxy, and C₁₋₃ alkyl halide. In some embodiments, each R² is independently selected from the group consisting of halogen, -OH, -NH₂, -CN, and C₁₋₃ alkyl. In some embodiments, each R² is independently selected from the group consisting of fluorine, chlorine, bromine, -OH, -NH₂, and -CN. In some embodiments, each R² is independently selected from the group consisting of fluorine, chlorine, bromine, and -CN. In some embodiments, each R² is independently selected from the group consisting of chlorine and -CN.

In some embodiments, each R⁴ is independently selected from the group consisting of halogen, -OH, -NH₂, -CN, C₁₋₃ alkyl, C₁₋₃ alkoxy, and C₁₋₃ alkyl halide. In some embodiments, each R⁴ is independently selected from the group consisting of halogen, -OH, -NH₂, -CN, and C₁₋₃ alkyl. In some embodiments, each R⁴ is independently selected from the group consisting of fluorine, chlorine, bromine, -OH, -NH₂, and -CN.

In some embodiments, m is selected from the group consisting of 0, 1, 2, and 3. In some embodiments, m is selected from the group consisting of 1, 2, and 3. In some embodiments, m is 2. In some embodiments, q is selected from the group consisting of 0, 1, 2, and 3. In some embodiments, q is selected from the group consisting of 0 and 1. In some embodiments, q is 0.

In some embodiments, the structural fragment

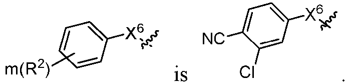

In some embodiments, each R³ and R⁴ is independently selected from the group consisting of halogen, -OH, -NH₂, -CN, C₁₋₃ alkyl, C₁₋₃ alkoxy, and C₁₋₃ alkyl halide. In some embodiments, each R³ and R⁴ is independently selected from the group consisting of halogen, -OH, -NH₂, -CN, and C₁₋₃ alkyl. In some embodiments, each R³ and R⁴ is independently selected from the group consisting of fluorine, chlorine, bromine, -OH, -NH₂, and -CN.

In some embodiments, p and q are each independently selected from the group consisting of 0, 1, 2, and 3. In some embodiments, p and q are each independently selected from the group consisting of 0 and 1. In some embodiments, p and q are 0.

In some embodiments, the structural fragment 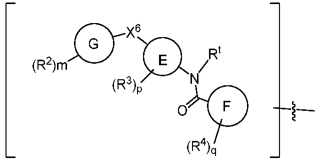 is selected from the group consisting of 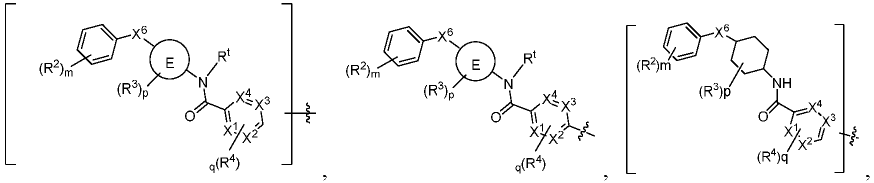 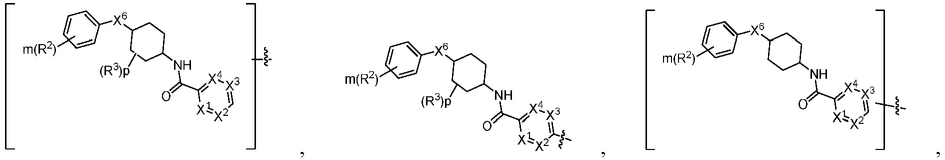 and 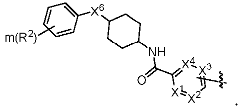 In some embodiments, the structural fragment 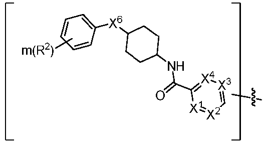 is selected from the group consisting of 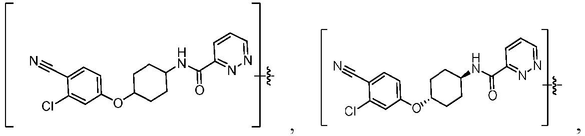 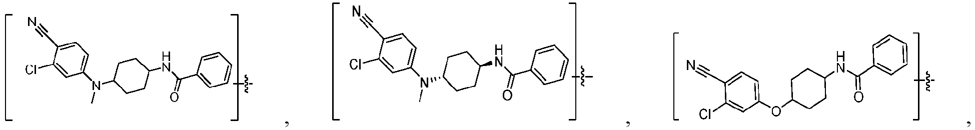 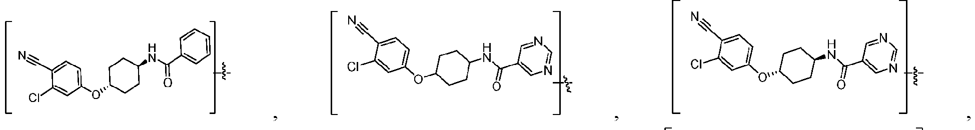 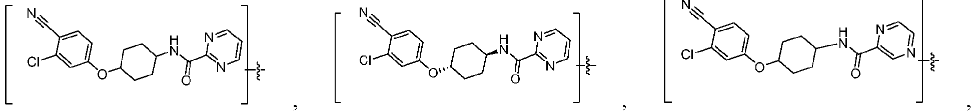 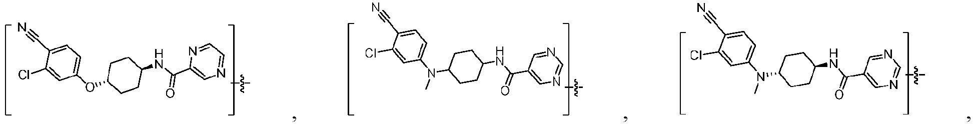 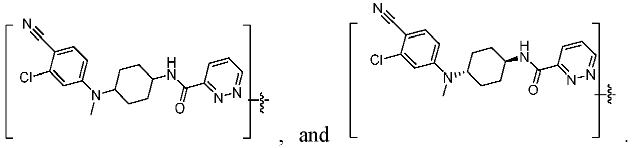 Optionally, in the structural fragment, 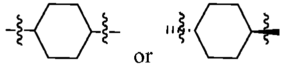 is substituted with p R³, wherein p and R³ are as defined herein.

In some embodiments, the structural fragment 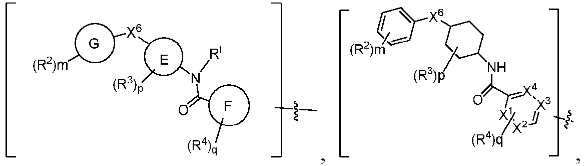 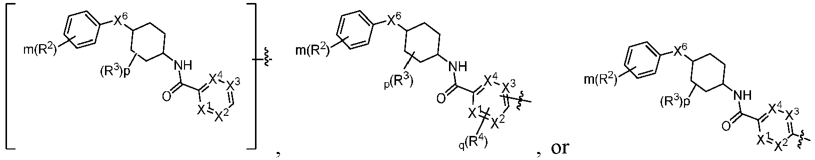 is selected from the group consisting of 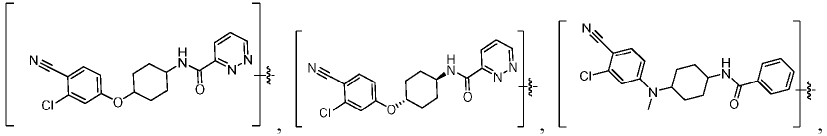 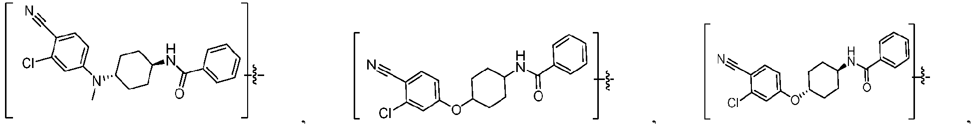 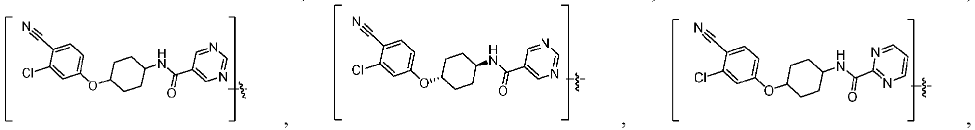 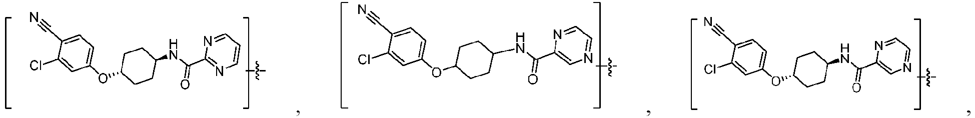 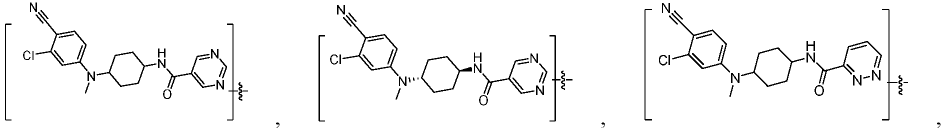 and 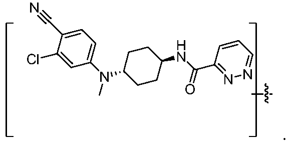 Optionally, in the structural fragment, 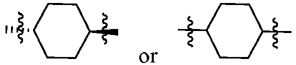 is substituted with p R³, wherein p and R³ are as defined herein.

In some embodiments, the structural fragment 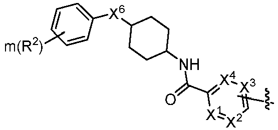 is selected from the group consisting of 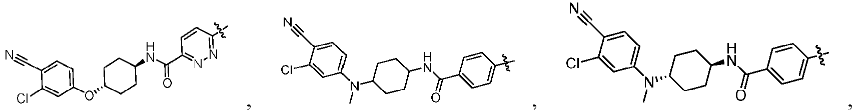 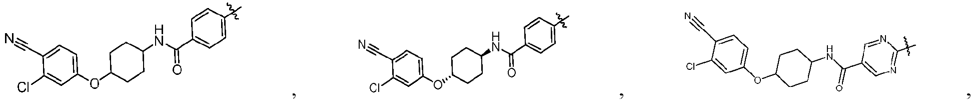 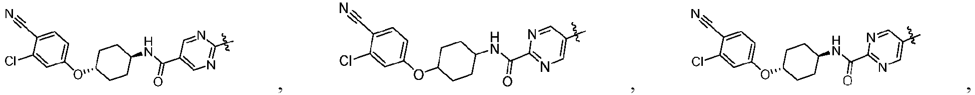 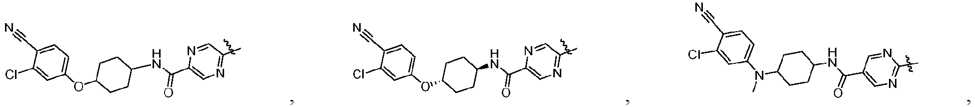 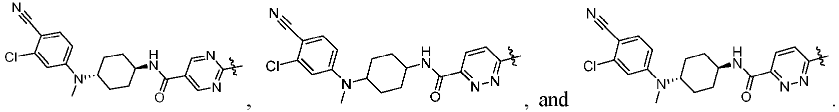 Optionally, in the structural fragment, 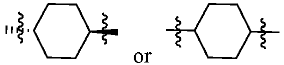 is substituted with p R³, wherein p and R³ are as defined herein.

In some embodiments, the structural fragment 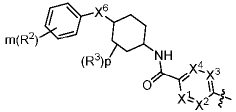 is selected from the group consisting of 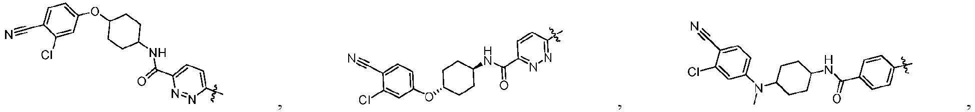 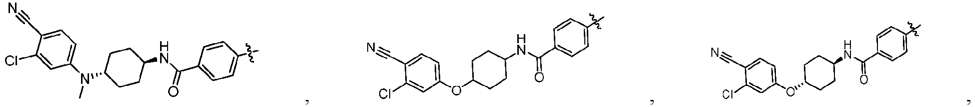 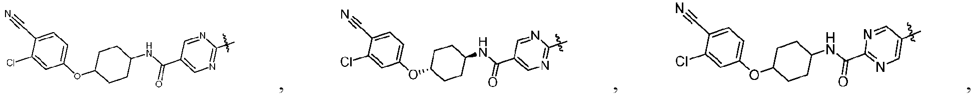 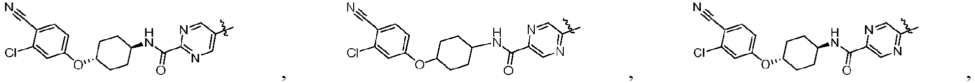 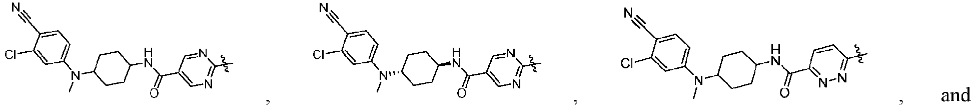 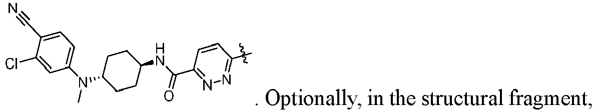 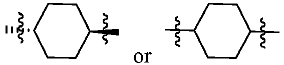 is substituted with p R³, wherein p and R³ are as defined herein.

In some specific embodiments, the structural fragment 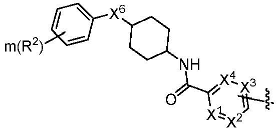 is selected from the group consisting of 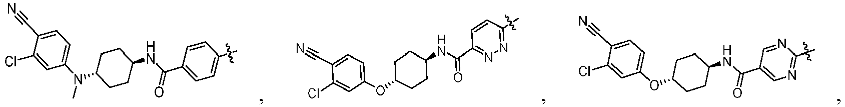 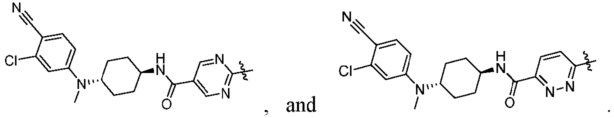 Optionally, in the structural fragment, 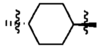 is substituted with p R³, wherein p and R³ are as defined herein.

In some specific embodiments, the structural fragment 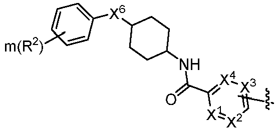 is selected from the group consisting of 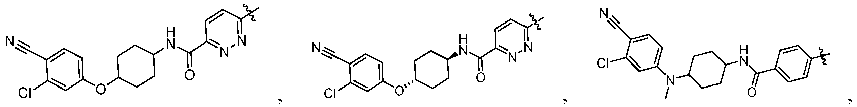 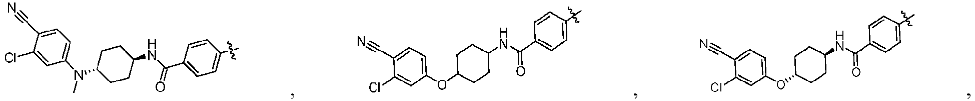 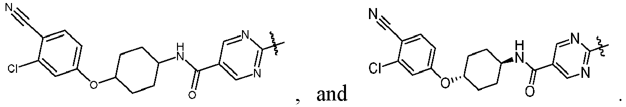 Optionally, in the structural fragment, 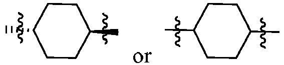 is substituted with p R³, wherein p and R³ are as defined herein.

In some specific embodiments, the structural fragment 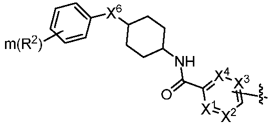 is selected from the group consisting of 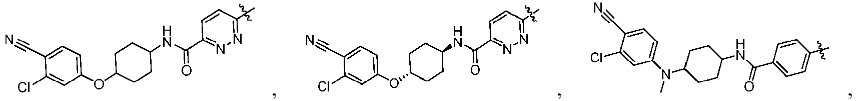 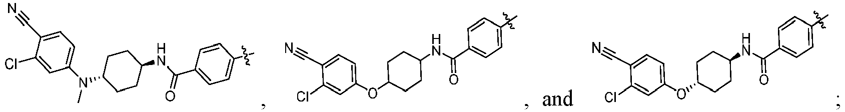 or the structural fragment 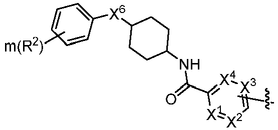 is selected from the group consisting of 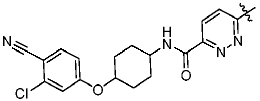 and 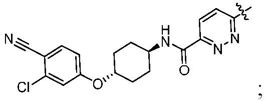 or the structural fragment 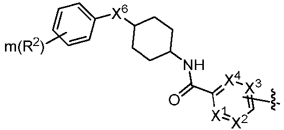 is selected from 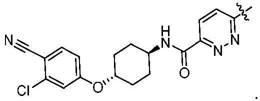 Optionally, in the structural fragment, 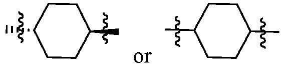 is substituted with p R³, wherein p and R³ are as defined herein.

In some embodiments, the heterocycloalkenyl, heteroaryl, heterocycloalkyl, or heteroalkylene described above comprises one or more heteroatoms or heteroatom groups independently selected from the group consisting of -O-, -NH-, -N-, -S-, C=O, -C(=O)NH-, -C(=O)O-, -S(=O)-, and -S(=O)₂-; in some embodiments, the heterocycloalkenyl, heteroaryl, heterocycloalkyl, or heteroalkylene described above comprises one or more heteroatoms or heteroatom groups independently selected from the group consisting of -O-, -NH-, -N-, and -S-; in some embodiments, the heterocycloalkenyl, heteroaryl, heterocycloalkyl, or heteroalkylene described above comprises one or more heteroatoms or heteroatom groups independently selected from the group consisting of -O-, -NH-, and -N-. In some embodiments, the number of the heteroatoms or heteroatom groups is independently selected from the group consisting of 1, 2, 3, 4, 5, and 6, or is selected from the group consisting of 1, 2, 3, and 4, or is selected from the group consisting of 1, 2, and 3, or is selected from the group consisting of 1 and 2.

In some embodiments, the heteroatom in the heterocycloalkenyl described above is selected from the group consisting of N, NH, O, and S. In some embodiments, the heteroatom in the heterocycloalkenyl described above is selected from the group consisting of N, O, and S. In some specific embodiments, the heteroatom in the heterocycloalkenyl described above is selected from the group consisting of N and O. In some embodiments, the number of the heteroatoms in the heterocycloalkenyl described above is selected from the group consisting of 1, 2, 3, 4, 5, and 6. In some embodiments, the number of the heteroatoms in the heterocycloalkenyl described above is selected from the group consisting of 1, 2, 3, and 4. In some embodiments, the number of the heteroatoms in the heterocycloalkenyl described above is selected from the group consisting of 1, 2, and 3. In some specific embodiments, the number of the heteroatoms in the heterocycloalkenyl described above is selected from the group consisting of 1 and 2.

In some specific embodiments, the heterocycloalkenyl contains 1-3 (e.g., 1-2) heteroatoms selected from the group consisting of -O-, -NH-, -N-, and -S-. In some specific embodiments, the heteroaryl contains 1-3 (e.g., 1-2) heteroatoms selected from the group consisting of -O-, -NH-, -N-, and -S-. In some specific embodiments, the heterocycloalkyl contains 1-3 (e.g., 1-2) heteroatoms selected from the group consisting of -O-, -NH-, -N-, and -S-. In some specific embodiments, the heteroalkylene contains 1-3 (e.g., 1-2) heteroatoms selected from the group consisting of -O-, -NH-, -N-, and -S-.

It should be understood that any embodiment of the compounds of the present application as described above, and any specific substituents set forth herein with respect to particular X¹, X², X³, X⁴, X⁵, X⁶, ring A, ring B, ring C, ring E, ring F, ring G, R¹, R², R³, R⁴, R^{t}, and L substituents in the compounds of the present application as described above, may be independently combined with other embodiments of the present application and/or substituents of the compounds to form embodiments of the present application not specifically set forth above. Further, when a range of substituents is disclosed in specific embodiments and/or claims with respect to any particular X¹, X², X³, X⁴, X⁵, X⁶, ring A, ring B, ring C, ring E, ring F, ring G, R¹, R², R³, R⁴, R^{t}, and L substituents, it should be understood that one or more substituents may be deleted from the range and the remaining range of substituents should also be considered as an embodiment of the present application.

The compound of formula I-AA, the compound of formula I-1 or the compound of formula I, the stereoisomer thereof, or the pharmaceutically acceptable salt thereof is selected from a compound of formula I-A1 or a compound of formula I-A, a stereoisomer thereof, or a pharmaceutically acceptable salt thereof, 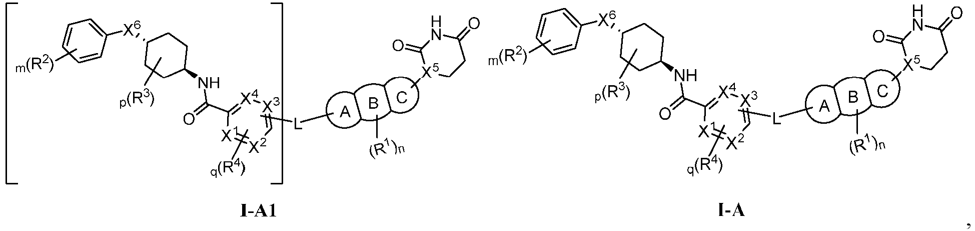 wherein ring A, ring B, ring C, R¹, n, L, X¹, X², X³, X⁴, X⁵, X⁶, R², R³, R⁴, m, p, and q are as described herein.

In some embodiments, the structural fragments 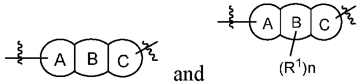 are as described herein. The compound of formula I-AA, the compound of formula I-1 or the compound of formula I, the stereoisomer thereof, or the pharmaceutically acceptable salt thereof is selected from a compound of formula I-1A, formula I-2A, formula I-3A, formula I-4A, formula I-5A, formula I-6A, formula I-7A, formula I-8A, formula I-9A, formula I-10A, formula I-11A, formula I-12A, formula I-13A, formula I-14A, formula I-15A, formula I-16A or formula I-17A, a stereoisomer thereof, or a pharmaceutically acceptable salt thereof, 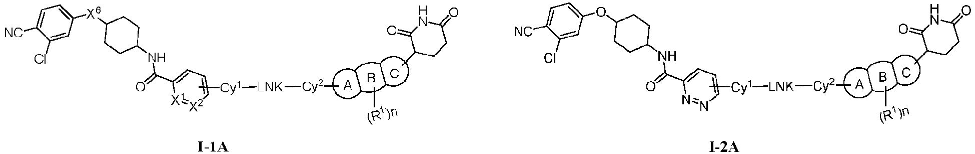 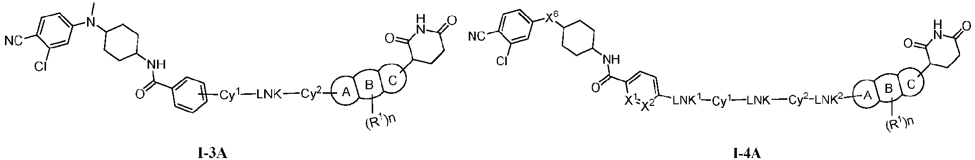 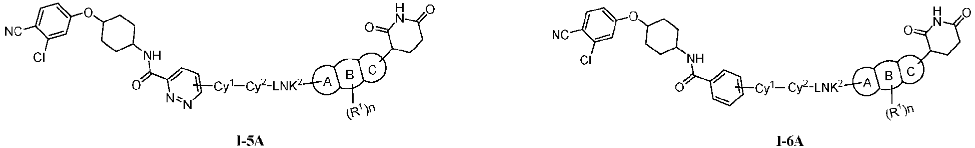 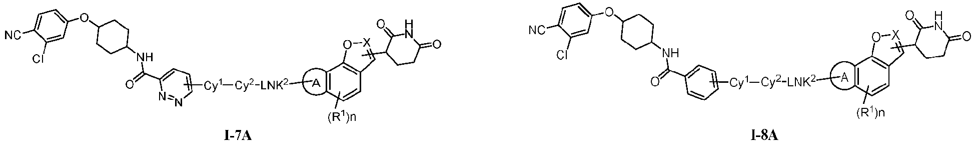 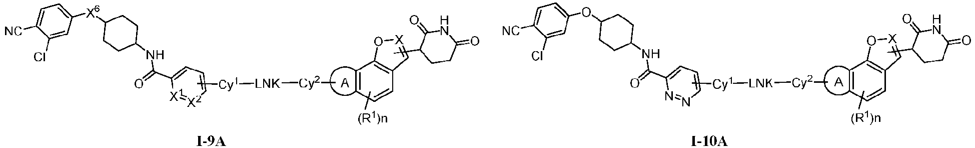 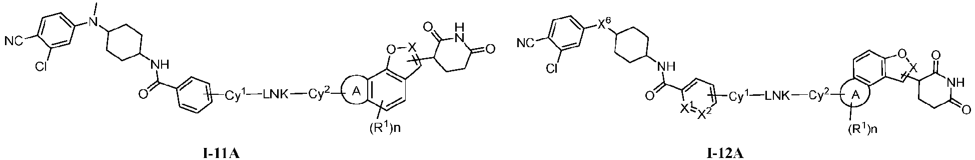 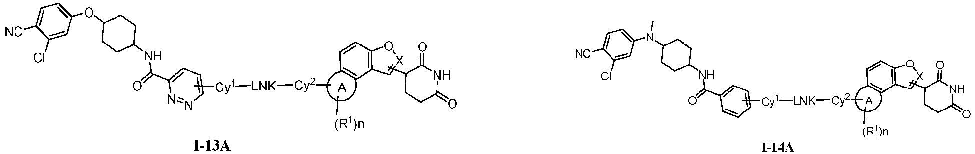 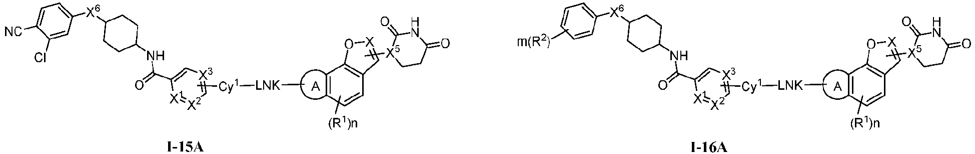 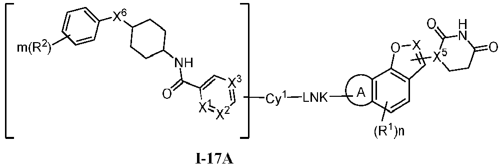
wherein ring A, ring B, ring C, R¹, n, R², m, X¹, X², X³, X⁵, X⁶, Cy¹, Cy², LNK, LNK¹, and LNK² are as defined herein;
X is selected from the group consisting of CH and N.

The compound of formula I-AA, the compound of formula I-1, the compound of formula I or the compound of formula I-A, the stereoisomer thereof or the pharmaceutically acceptable salt thereof is selected from a compound of formula I-1A-1, formula I-2A-1, formula I-3A-1, formula I-4A-1, formula I-5A-1, formula I-6A-1, formula I-7A-1, formula I-8A-1, formula I-9A-1, formula I-10A-1, formula I-11A-1, formula I-12A-1, formula I-13A-1, formula I-14A-1, formula I-15A-1, formula I-16A-1 or formula I-17A-1, a stereoisomer thereof, or a pharmaceutically acceptable salt thereof, 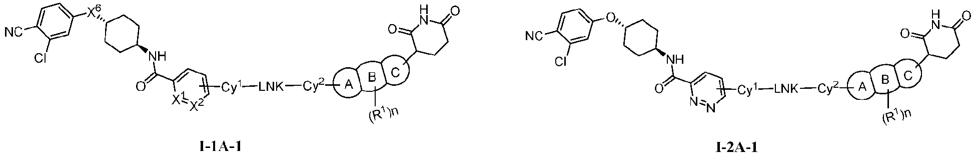 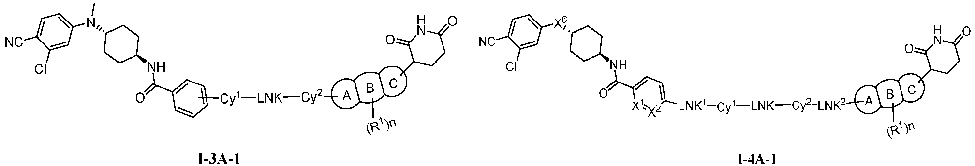 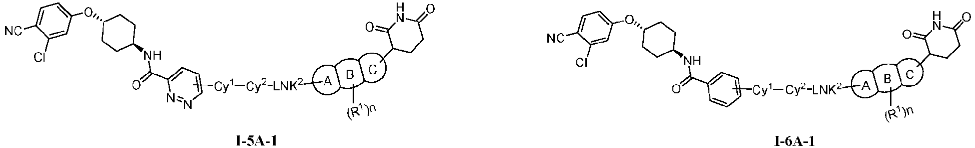 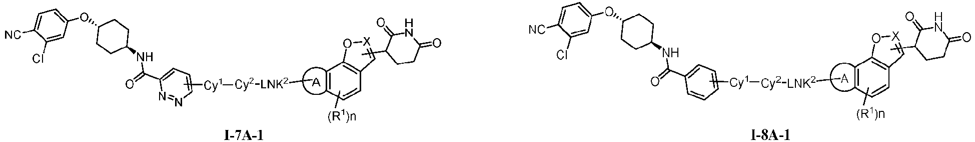 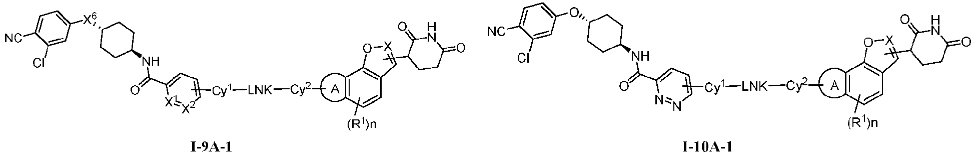 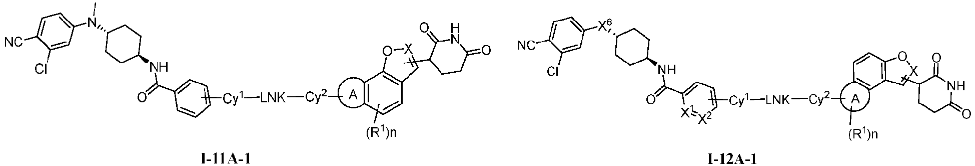 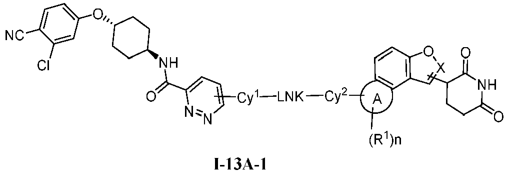 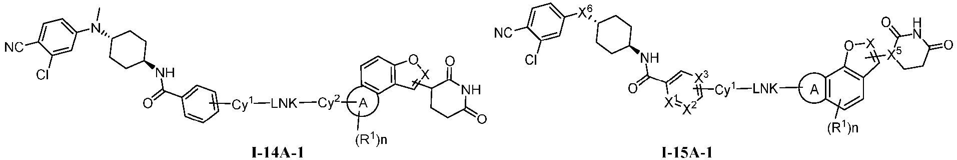 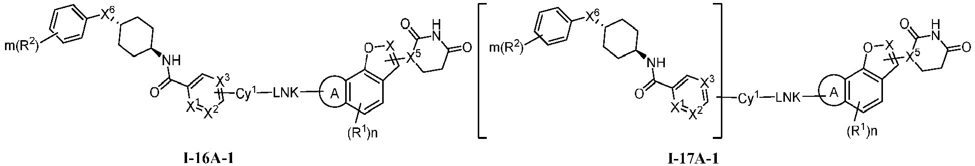
wherein ring A, ring B, ring C, R¹, R², m, n, X¹, X², X³, X⁵, X⁶, Cy¹, Cy², LNK, LNK¹, and LNK² are as defined herein;
X is selected from the group consisting of CH and N.

In some embodiments, X¹ and X² are CH, and X³ is CH. In some embodiments, X¹ and X² are N, and X³ is CH. In some embodiments, X² and X³ are N, and X¹ is CH.

In some embodiments, X¹ and X² are CH. In some embodiments, X¹ and X² are N.

In some embodiments, the structural fragment 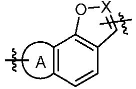 is selected from the group consisting of 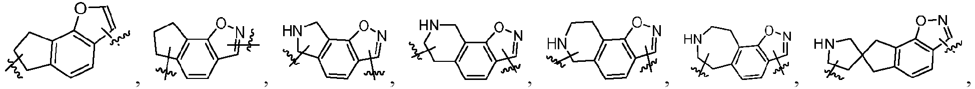 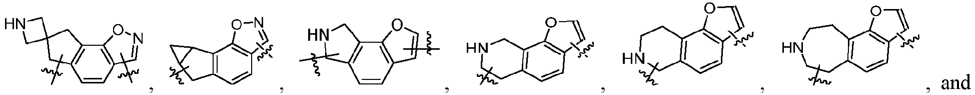 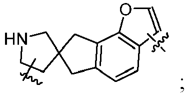 or the structural fragment 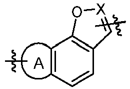 is selected from the group consisting of 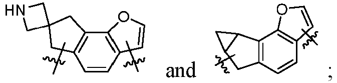 or the structural fragment 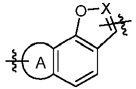 is selected from the group consisting of 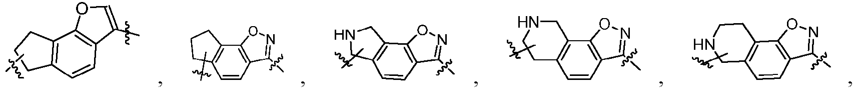 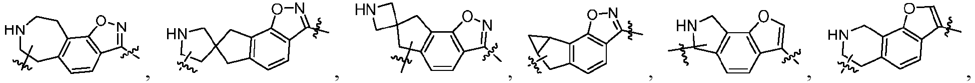 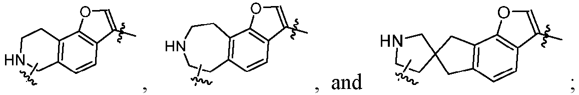 or the structural fragment 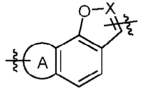 is selected from the group consisting of 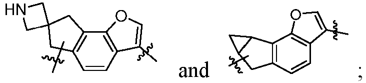 or the structural fragment 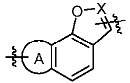 is selected from the group consisting of 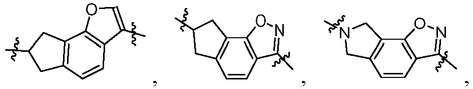 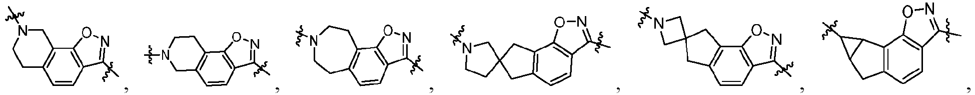 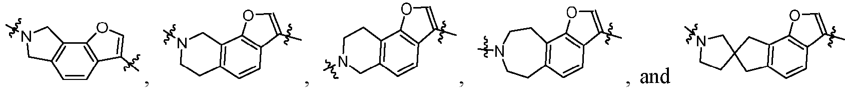 ; or the structural fragment 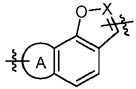 is selected from the group consisting of

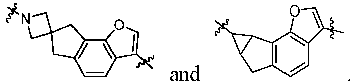

In some embodiments, the structural fragment 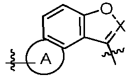 is selected from the group consisting of 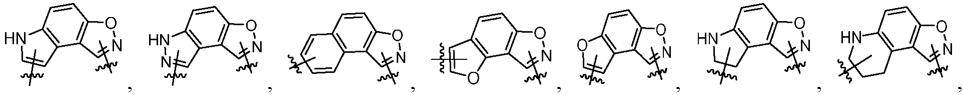 and 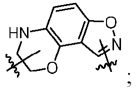 or the structural fragment is selected from the group consisting of 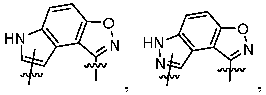 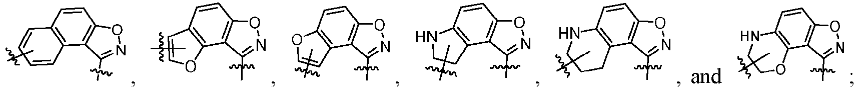 or the structural fragment is selected from the group consisting of

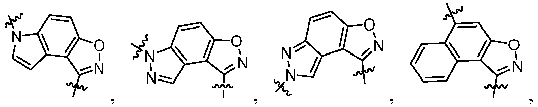

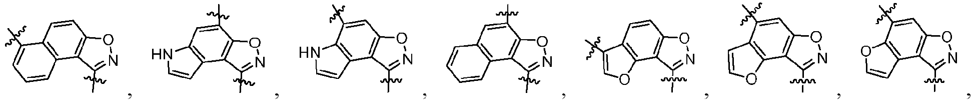

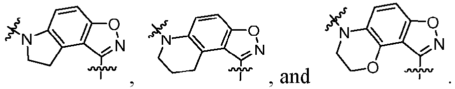

In some embodiments, the structural fragment 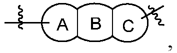 -Cy¹-LNK-, -LNK-Cy²-, -Cy¹-Cy²-, or -Cy¹-LNK-Cy²- is as described above.

In some embodiments, optionally, the compound of the present application is not the following compound:

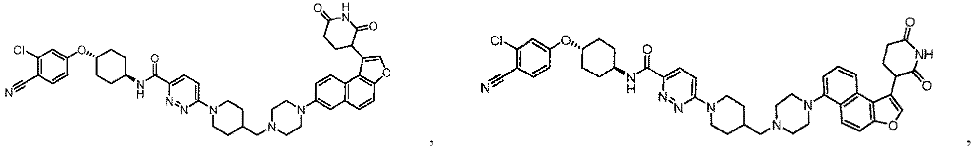

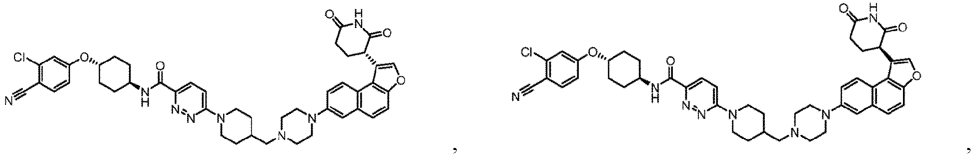

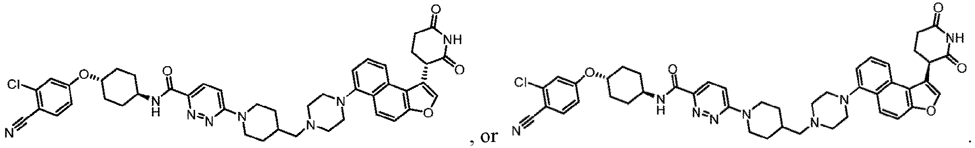

In some embodiments, optionally, the compound of the present application is not the following compound:

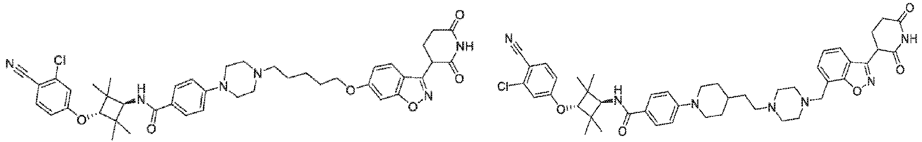

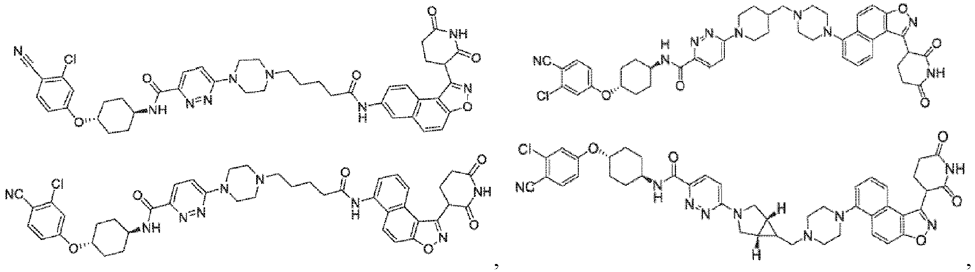

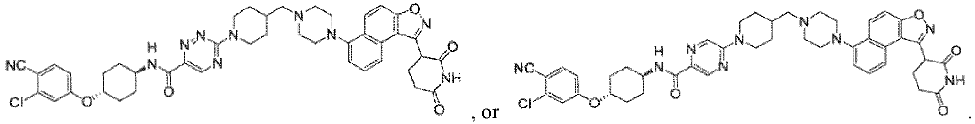

In some embodiments, the structural moiety 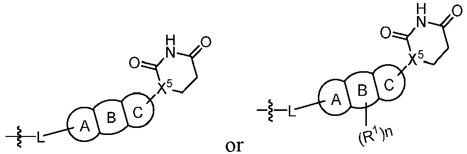 is not selected from the group consisting of

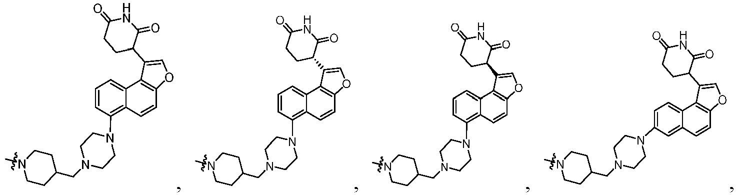

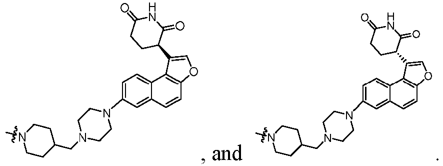

In some embodiments, the structural moiety 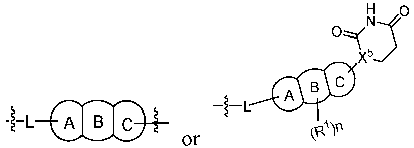 is not selected from the group consisting of 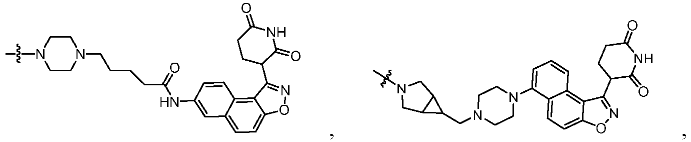 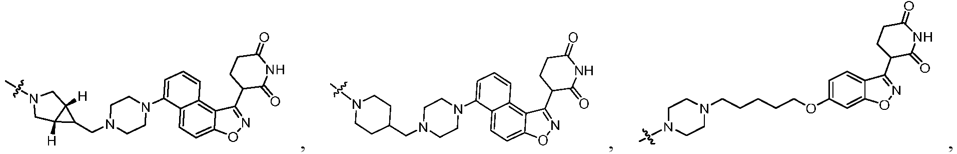 and

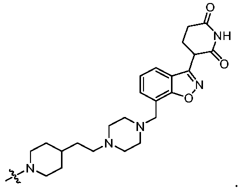

In some embodiments, the structural moiety 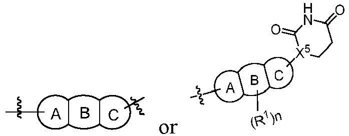 is not selected from the group consisting of 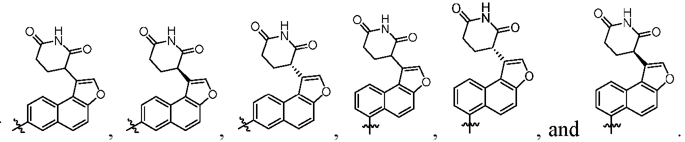 In some embodiments, the structural moiety 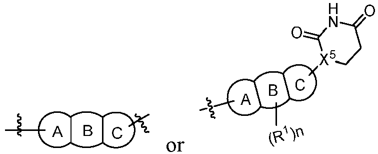 is not selected from the group consisting of

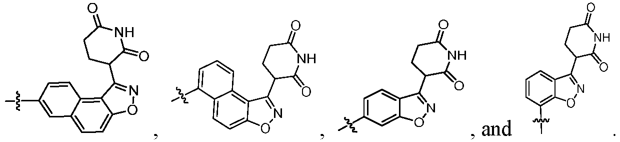

In some embodiments, the structural fragment 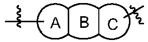 is not selected from the group consisting of 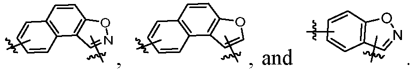 In some embodiments, the structural fragment 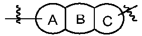 is not selected from the group consisting of 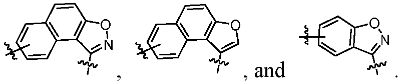 In some embodiments, the structural fragment 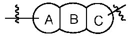 is not selected from the group consisting of

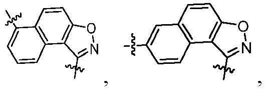

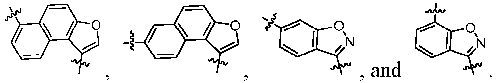

In another aspect, the present application relates to a compound of formula I' or I", a moiety, a stereoisomer thereof, a derivative (such as a Protac molecule), or a pharmaceutically acceptable salt thereof, 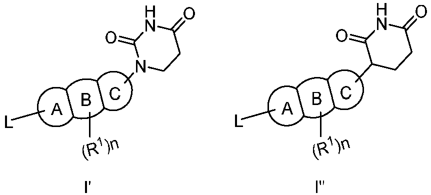
wherein ring A is absent or selected from the group consisting of C₅₋₁₀ cycloalkenyl, 5- to 10-membered heterocycloalkenyl, phenyl, and 5- to 6-membered heteroaryl;
ring B is selected from phenyl;
ring C is selected from the group consisting of isoxazolyl and furanyl;
L is selected from a connecting group; optionally, L is as defined herein; optionally, R¹ and n are as defined herein.

In some embodiments, the structural moiety 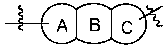 is as described herein.

In another aspect, the present application relates to a compound of formula I'-a or I"-a, a moiety, a stereoisomer thereof, a derivative (such as a Protac molecule), or a pharmaceutically acceptable salt thereof, 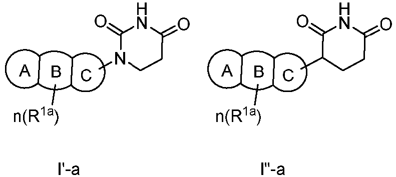
wherein ring A is absent or selected from the group consisting of C₅₋₁₀ cycloalkenyl, 5- to 10-membered heterocycloalkenyl, phenyl, and 5- to 6-membered heteroaryl;
ring B is selected from phenyl;
ring C is selected from the group consisting of isoxazolyl and furanyl;
each R^{1a} is independently selected from the group consisting of halogen, -OH, -NH₂, -CN, =O, -CHO, C₁₋₄ alkyl, C₁₋₄ alkoxy, C₁₋₆ alkyl OC(O)-, C₃₋₁₂ cycloalkyl, and 4- to 12-membered heterocycloalkyl, wherein the C₁₋₄ alkyl, C₁₋₄ alkoxy, C₃₋₁₂ cycloalkyl, or 4- to 12-membered heterocycloalkyl is optionally substituted with one or more of the following groups: halogen, =O, -OH, -NH₂, -CN, CHO, COOH, -C₁₋₄ alkyl-OH, C₁₋₆ alkyl OC(O)-, and 4- to 10-membered heterocycloalkyl optionally substituted with C₁₋₆ alkyl COC(O)-;
n is selected from the group consisting of 0, 1, 2, and 3.

In some embodiments, ring A is absent or selected from the group consisting of C₅₋₇ cycloalkenyl, 5- to 9-membered heterocycloalkenyl, phenyl, and 5- to 6-membered heteroaryl.

In some embodiments, ring A is absent or selected from the group consisting of C₅₋₆ cycloalkenyl, 5- to 10-membered heterocycloalkenyl, phenyl, and 5- to 6-membered heteroaryl.

In some embodiments, ring A is absent or selected from the group consisting of C₅₋₆ cycloalkenyl, 5- to 9-membered heterocycloalkenyl, phenyl, and 5- to 6-membered heteroaryl.

In some embodiments, ring A is absent or selected from the group consisting of C₅₋₆ cycloalkenyl, 5- to 9-membered heterocycloalkenyl, phenyl, and 5-membered heteroaryl.

In some embodiments, ring A is absent or selected from the group consisting of C₅₋₆ cycloalkenyl, 5- to 9-membered heterocycloalkenyl, phenyl, pyrrolyl, pyrazolyl, furanyl, and oxazolyl.

In some embodiments, ring A is absent or selected from the group consisting of C₅ cycloalkenyl, C₆ cycloalkenyl, 5-membered, 6-membered, 7-membered, 8-membered, and 9-membered heterocycloalkenyl, phenyl, pyrrolyl, pyrazolyl, furanyl, and oxazolyl.

In some embodiments, ring A is absent or selected from the group consisting of cyclopentenyl, monocyclohexenyl, bicyclohexenyl, dihydropyrrolyl, tetrahydropyridinyl, tetrahydroazepinyl, azaspirooctenyl, azaspirononenyl, phenyl, pyrrolyl, pyrazolyl, furanyl, oxazolyl, and dihydrooxazinyl.

In some specific embodiments, ring A is selected from the group consisting of C₅₋₉ cycloalkenyl, 5- to 9-membered heterocycloalkenyl, phenyl, and 5-membered heteroaryl.

In some specific embodiments, ring A is selected from the group consisting of C₅₋₆ cycloalkenyl, 5- to 9-membered heterocycloalkenyl, phenyl, and 5-membered heteroaryl.

In some specific embodiments, ring A is selected from the group consisting of C₅₋₉ cycloalkenyl and 5- to 9-membered heterocycloalkenyl. In some specific embodiments, ring A is selected from the group consisting of C₅₋₆ cycloalkenyl and 5- to 9-membered heterocycloalkenyl.

In some specific embodiments, ring A is selected from C₅₋₉ cycloalkenyl. In some specific embodiments, ring A is selected from C₅₋₆ cycloalkenyl. In some specific embodiments, ring A is selected from 5- to 9-membered heterocycloalkenyl.

In some specific embodiments, ring A is selected from the group consisting of cyclopentenyl, dihydropyrrolyl, tetrahydropyridinyl, tetrahydroazepinyl, azaspirononenyl, azaspirooctenyl, phenyl, pyrrolyl, and pyrazolyl.

In some specific embodiments, ring A is selected from the group consisting of cyclopentenyl, dihydropyrrolyl, tetrahydropyridinyl, tetrahydroazepinyl, azaspirononenyl, and azaspirooctenyl. In some specific embodiments, ring A is selected from the group consisting of cyclopentenyl, dihydropyrrolyl, tetrahydropyridinyl, tetrahydroazepinyl, and azaspirooctenyl.

In some specific embodiments, ring A is selected from cyclopentenyl. In some specific embodiments, ring A is selected from the group consisting of dihydropyrrolyl, tetrahydropyridinyl, tetrahydroazepinyl, and azaspirooctenyl. In some specific embodiments, ring A is selected from the group consisting of phenyl, pyrrolyl, and pyrazolyl.

In some embodiments, ring A is selected from the group consisting of

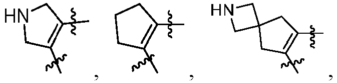

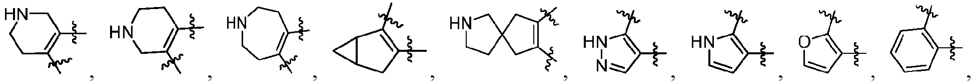

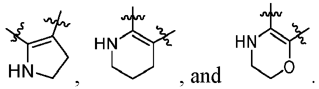

In some specific embodiments, ring C is isoxazolyl. In some specific embodiments, ring C is furanyl.

In some embodiments, the structural moiety 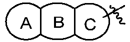 is selected from the group consisting of 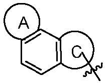 and

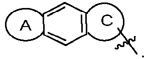

In some embodiments, the structural fragment 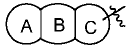 is selected from the group consisting of 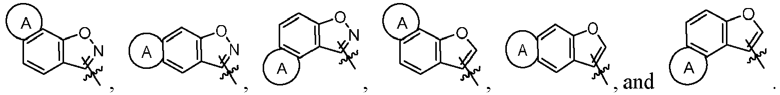 In some embodiments, the structural fragment 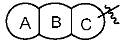 is selected from the group consisting of 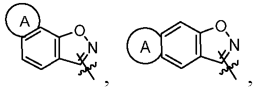 and 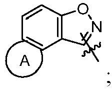 or the structural fragment 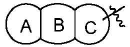 is selected from the group consisting of 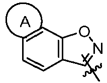 and 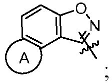 or the structural fragment 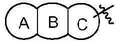 is selected from

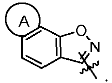

In some embodiments, the structural fragment 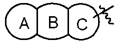 is selected from the group consisting of 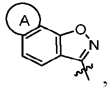 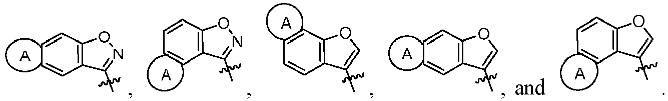 In some embodiments, the structural fragment 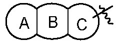 is selected from the group consisting of 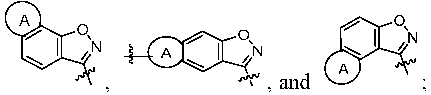 or the structural fragment 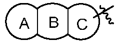 is selected from the group consisting of 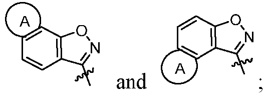 or the structural fragment 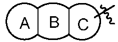 is selected from

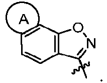

In some embodiments, the structural fragment 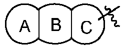 is selected from the group consisting of

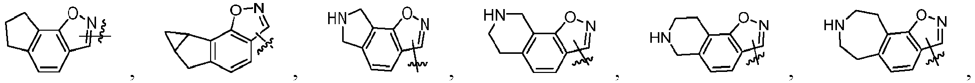

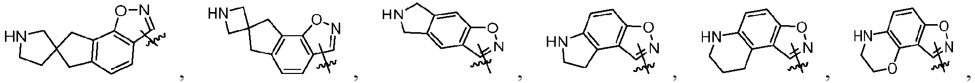

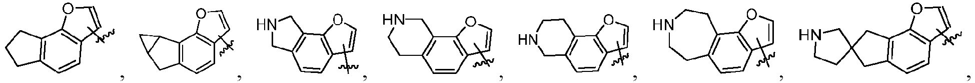

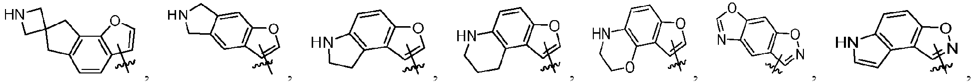

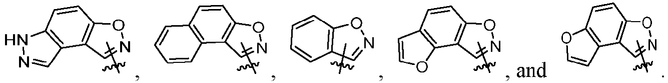

In some specific embodiments, the structural fragment 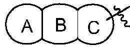 is selected from the group consisting of 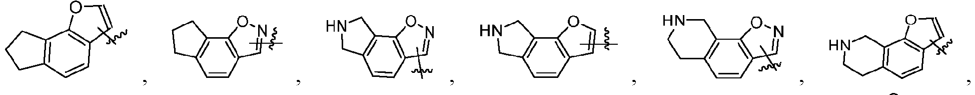 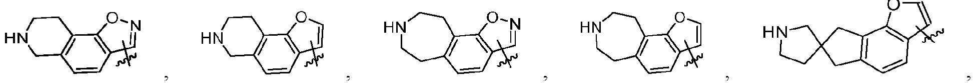 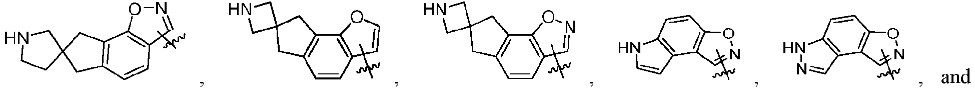 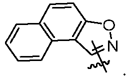 In some more specific embodiments, the structural fragment 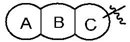 is selected from the group consisting of 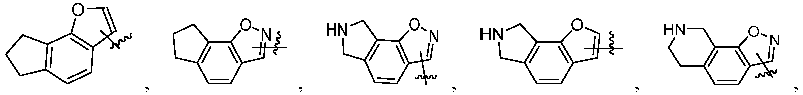 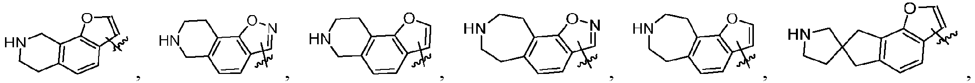 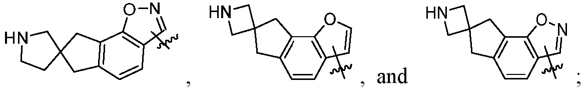 or the structural fragment 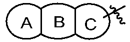 is selected from the group consisting of

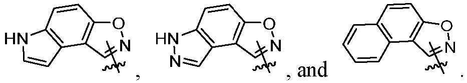

In some embodiments, the structural fragment 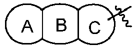 is selected from the group consisting of

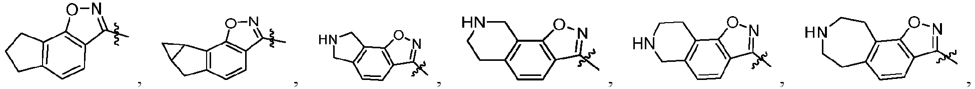

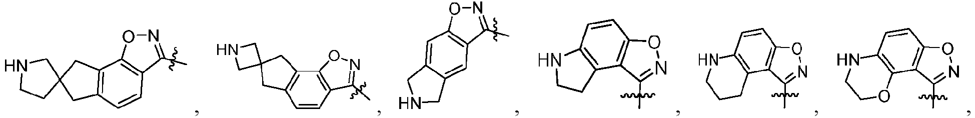

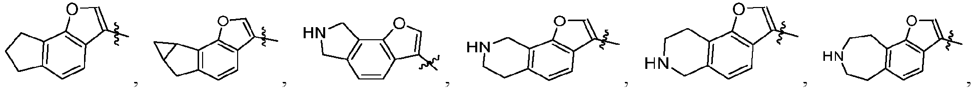

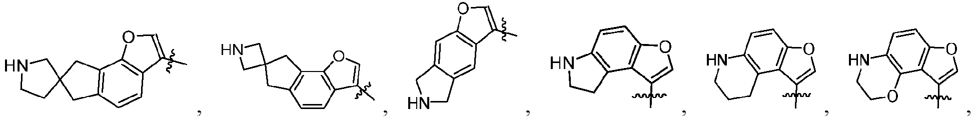

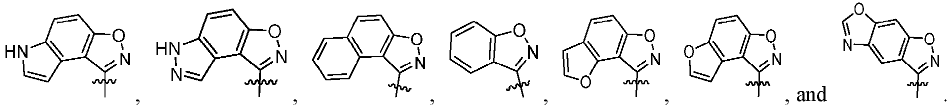

In some specific embodiments, the structural fragment 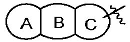 is selected from the group consisting of 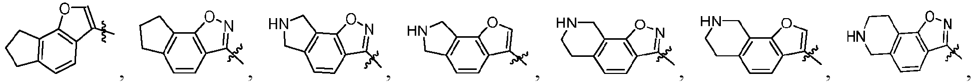 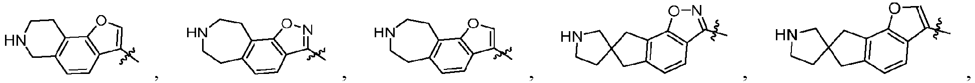 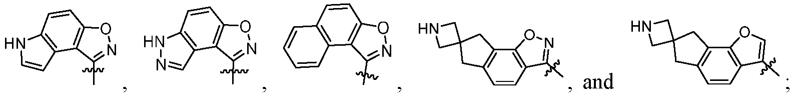 in some more specific embodiments, the structural fragment 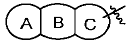 is selected from the group consisting of 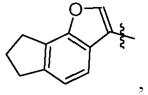 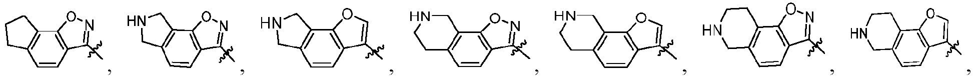 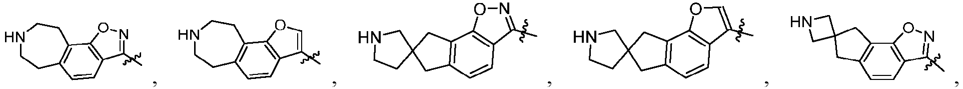 and 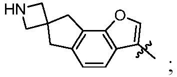 or the structural fragment 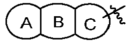 is selected from the group consisting of

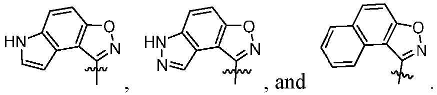

In some embodiments, each R^{1a} is independently selected from the group consisting of halogen, -OH, -NH₂, -CN,-CHO, C₁₋₆ alkyl OC(O)-, C₁₋₄ alkyl, C₁₋₄ alkoxy, C₃₋₁₀ cycloalkyl, and 4- to 10-membered heterocycloalkyl, wherein the C₁₋₄ alkyl, C₁₋₄ alkoxy, C₃₋₁₀ cycloalkyl, or 4- to 10-membered heterocycloalkyl is optionally substituted with one or more of the following groups: halogen, =O, -OH, -NH₂, -CN, CHO, COOH, -C₁₋₄ alkyl-OH, C₁₋₆ alkyl OC(O)-, and 4- to 10-membered heterocycloalkyl optionally substituted with C₁₋₆ alkyl COC(O)-.

In some embodiments, each R^{1a} is independently selected from the group consisting of halogen, -OH, -NH₂, -CN, - CHO, C₁₋₄ alkyl OC(O)-, C₁₋₃ alkyl, C₁₋₃ alkoxy, C₃₋₉ cycloalkyl, and 4- to 9-membered heterocycloalkyl, wherein the C₁₋₃ alkyl, C₁₋₃ alkoxy, C₃₋₉ cycloalkyl, or 4- to 9-membered heterocycloalkyl is optionally substituted with one or more of the following groups: halogen, -OH, =O, -NH₂, -CN, CHO, COOH, -C₁₋₄ alkyl-OH, C₁₋₄ alkyl OC(O)-, and 4- to 9-membered heterocycloalkyl optionally substituted with C₁₋₄ alkyl COC(O)-.

In some embodiments, each R^{1a} is independently selected from the group consisting of halogen, -OH, -NH₂, -CN, - CHO, C₁₋₆ alkyl OC(O)-, C₁₋₃ alkyl, C₁₋₃ alkoxy, C₃₋₆ cycloalkyl, and 4- to 6-membered heterocycloalkyl, wherein the C₁₋₃ alkyl, C₁₋₃ alkoxy, C₄₋₆ cycloalkyl, or 4- to 6-membered heterocycloalkyl is optionally substituted with one or more of the following groups: halogen, -OH, =O, -NH₂, -CN, CHO, COOH, -C₁₋₄ alkyl-OH, C₁₋₆ alkyl OC(O)-, and 3- to 6-membered heterocycloalkyl optionally substituted with C₁₋₆ alkyl COC(O)-.

In some embodiments, each R^{1a} is independently selected from the group consisting of halogen, -OH, -NH₂, -CN, - CHO, C₁₋₄ alkyl OC(O)-, C₁₋₃ alkyl, and 4- to 6-membered heterocycloalkyl, wherein the C₁₋₃ alkyl or 4- to 6-membered heterocycloalkyl is optionally substituted with one or more of the following groups: -OH, =O, -NH₂, - CN, CHO, COOH, C₁₋₄ alkyl OC(O)-, and 4- to 6-membered heterocycloalkyl optionally substituted with C₁₋₄ alkyl OC(O)-.

In some embodiments, each R^{1a} is independently selected from the group consisting of halogen, -OH, -CHO, (CH₃)₃COC(O)-, C₁₋₃ alkyl, cyclobutyl, and piperidinyl, wherein the C₁₋₃ alkyl, cyclobutyl, or piperidinyl is optionally substituted with one or more of the following groups: -OH, (CH₃)₃COC(O)-, and cyclobutyl optionally substituted with (CH₃)₃COC(O)-.

In some embodiments, each R^{1a} is independently selected from the group consisting of F, -OH, -CHO, (CH₃)₃COC(O)-, -CH₂OH,

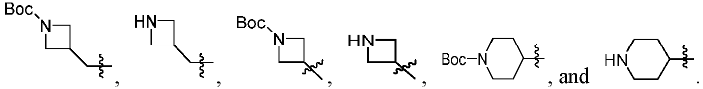

In some embodiments, n is selected from the group consisting of 0, 1, and 2. In some embodiments, n is selected from the group consisting of 0 and 1. In some embodiments, n is 0.

The present application relates to the following compound, a moiety, a stereoisomer thereof, a derivative (such as a Protac molecule), or a pharmaceutically acceptable salt thereof, 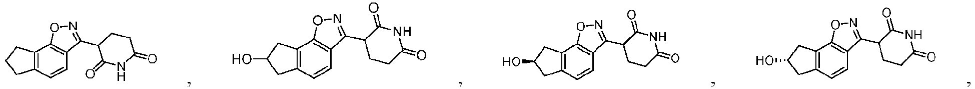 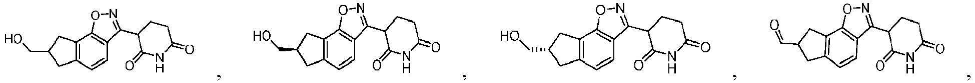 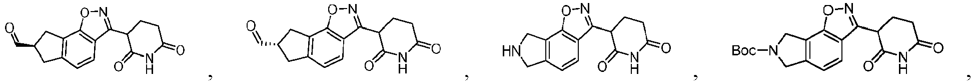 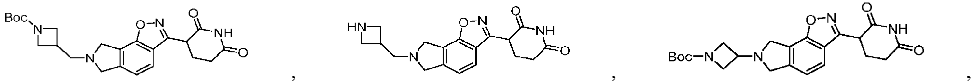 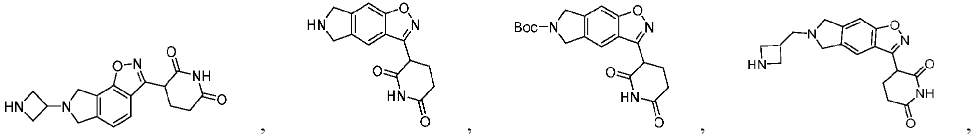 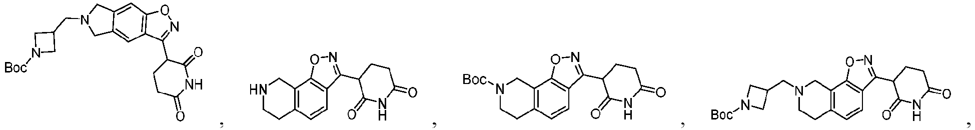 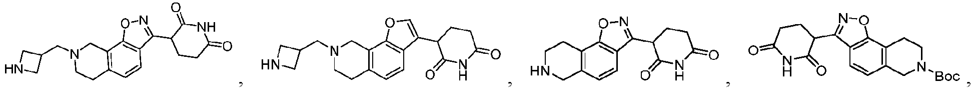 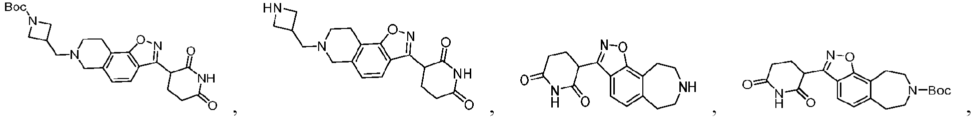 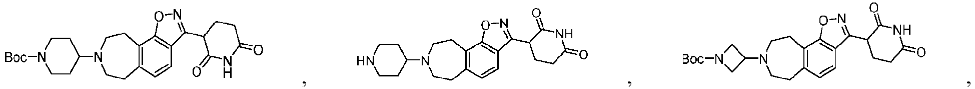 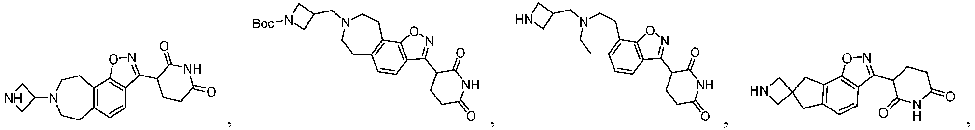 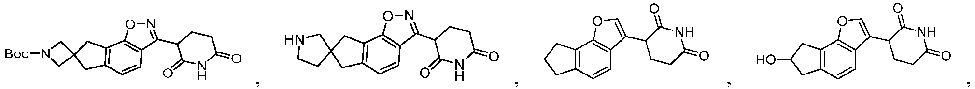 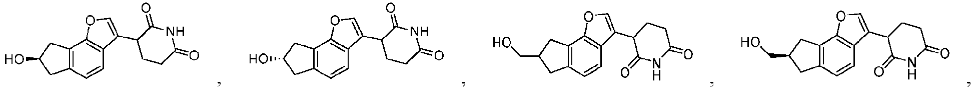 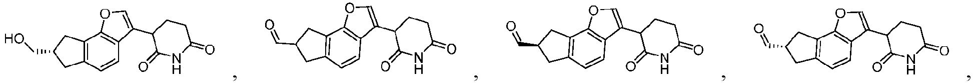 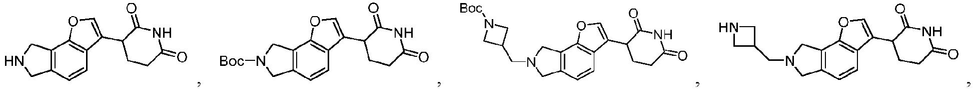 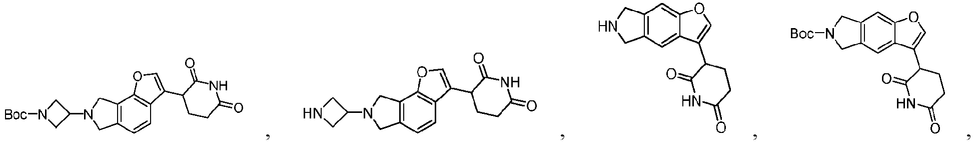 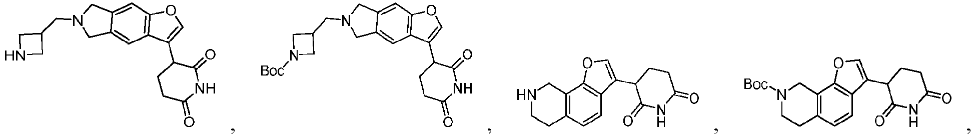 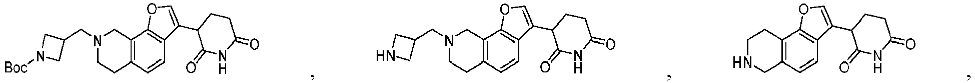 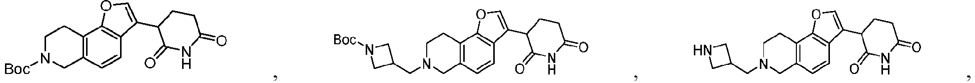 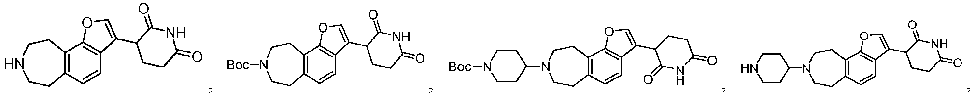 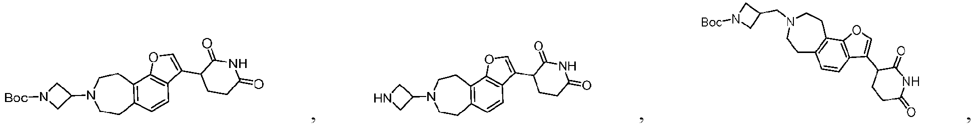 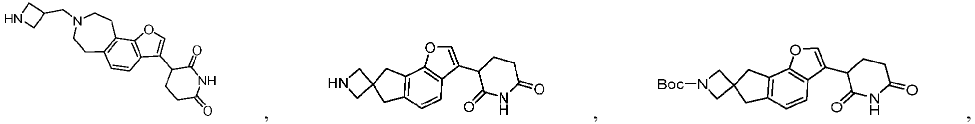 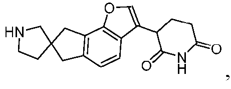 or,

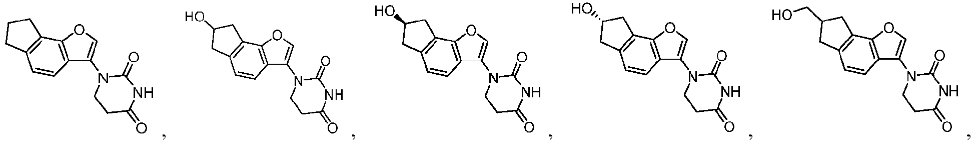

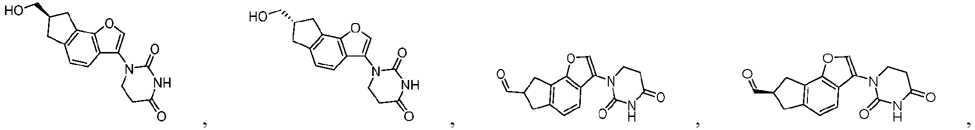

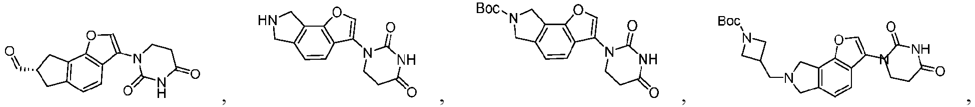

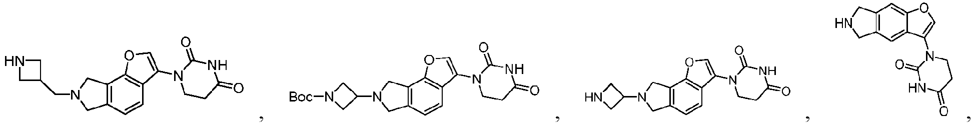

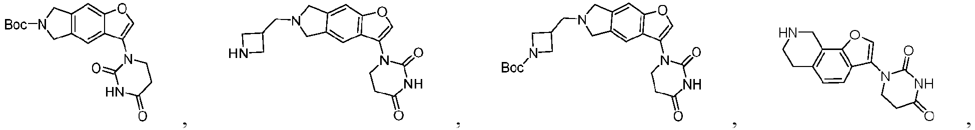

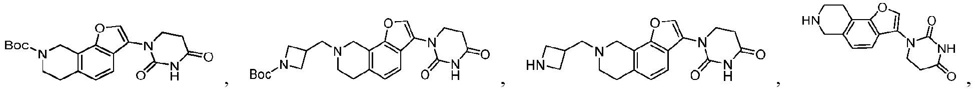

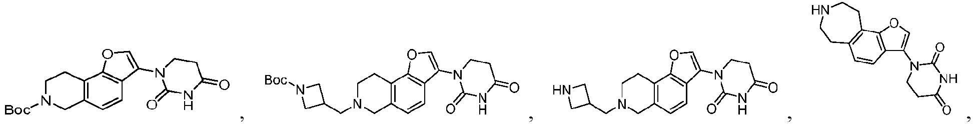

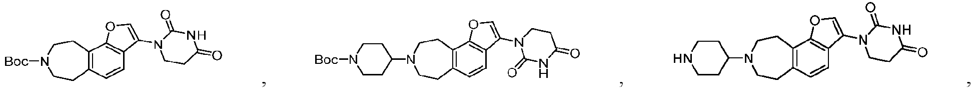

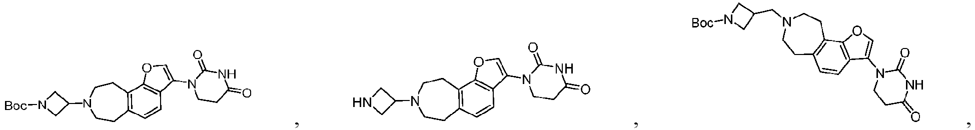

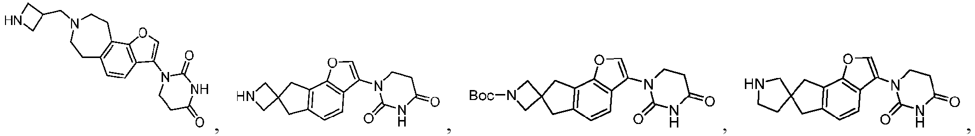

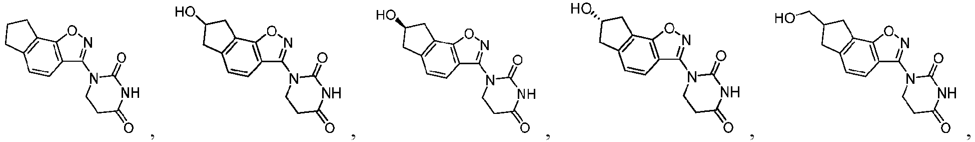

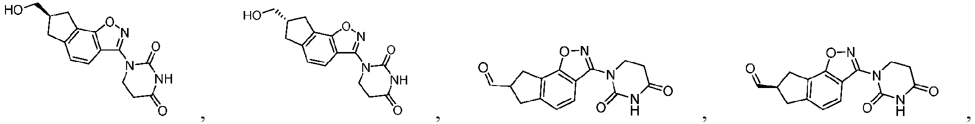

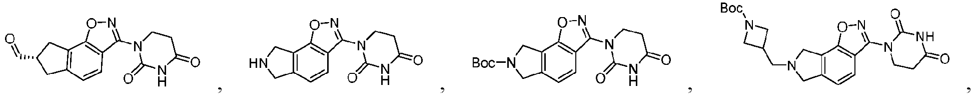

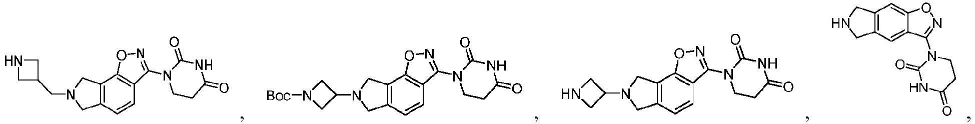

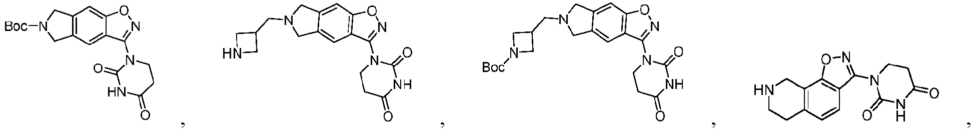

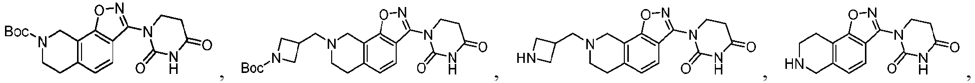

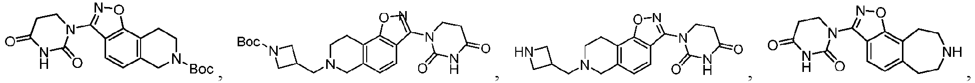

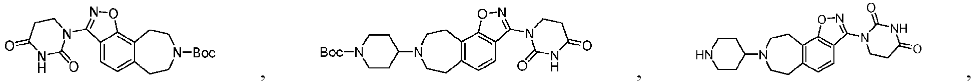

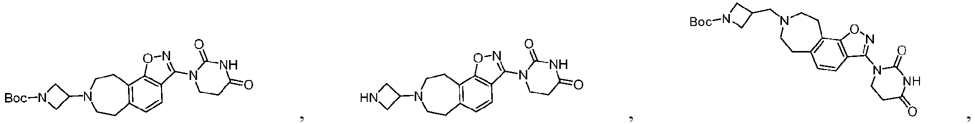

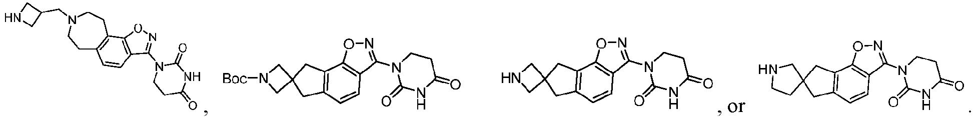

In another aspect, the present application relates to use of the compound (e.g., formula I' or formula I", formula I'-a or formula I"-a, or a specific compound), the moiety, the isomer (e.g., stereoisomer) thereof, or the derivative thereof in a Protac molecule. In another aspect, the present application relates to use of the compound (e.g., formula I' or formula I", formula I'-a or formula I"-a, or a specific compound), the moiety, the isomer (e.g., stereoisomer) thereof, or the derivative thereof for constituting part of a Protac molecule. In another aspect, the present application relates to the compound (e.g., formula I' or formula I", formula I'-a or formula I"-a, or a specific compound), the moiety, the isomer (e.g., stereoisomer) thereof, or the derivative thereof present in the form of a Protac molecule. In another aspect, the present application relates to use of the compound (e.g., formula I' or formula I", formula I'-a or formula I"-a, or a specific compound), the moiety, the isomer (e.g., stereoisomer) thereof, or the derivative thereof for degrading a protein. For example, the compound (e.g., formula I' or formula I", formula I'-a or formula I"-a, or a specific compound), the moiety, the isomer (e.g., stereoisomer) thereof, or the derivative thereof degrades the protein in the form of a Protac molecule. In another aspect, the present application relates to use of the compound (e.g., formula I' or formula I", formula I'-a or formula I"-a, or a specific compound), the moiety, the isomer (e.g., stereoisomer) thereof, or the derivative thereof for degrading a protein in the form of a Protac molecule. The present application relates to use of the compound (e.g., formula I' or formula I", formula I'-a or formula I"-a, or a specific compound), the moiety, the isomer (e.g., stereoisomer) thereof, or the derivative thereof (e.g., as a preparation intermediate) for preparing a Protac molecule. The present application relates to use of the compound (e.g., formula I' or formula I", formula I'-a or formula I"-a, or a specific compound), the moiety, the isomer (e.g., stereoisomer) thereof, or the derivative thereof (e.g., as a preparation intermediate) for preparing a protein degrader.

In some embodiments, the present application relates to a compound of formula I-AA, a compound of formula I, or a compound of formula I-1, a stereoisomer thereof, or a pharmaceutically acceptable salt thereof, wherein,
ring A is selected from the group consisting of C₅₋₆ cycloalkenyl, 5- to 8-membered heterocycloalkenyl containing 1-3 heteroatoms selected from the group consisting of N, O, and S (e.g., 1-2 heteroatoms selected from the group consisting of N and O), phenyl, and 5- to 6-membered heteroaryl containing 1-3 heteroatoms selected from the group consisting of N, O, and S (e.g., 1-2 heteroatoms selected from the group consisting of N and O);
ring B is phenyl;
ring C is selected from the group consisting of isoxazolyl and furanyl;
each R¹ is independently selected from the group consisting of halogen, -OH, -NH₂, -CN, and C₁₋₃ alkyl (e.g., methyl, ethyl, or propyl);
n is selected from the group consisting of 0 and 1;
L is selected from LNK¹-Cy¹-LNK-Cy²-LNK²-, wherein LNK, LNK¹, and LNK² are each independently selected from the group consisting of a bond and C₁₋₃ alkylene, Cy¹ is selected from the group consisting of a bond, C₃₋₇ cycloalkyl, 4- to 7-membered heterocycloalkyl, and 5- to 7-membered heterocycloalkenyl, Cy² is selected from the group consisting of a bond, C₃₋₇ cycloalkyl, 4- to 7-membered heterocycloalkyl, and 5- to 7-membered heterocycloalkenyl, and Cy¹ and Cy² are not bonds at the same time;
X¹, X², X³, and X⁴ are each independently selected from the group consisting of N and CH;
X³ is selected from the group consisting of CH and N;
X⁶ is selected from the group consisting of -O-, -NH-, and -N(C₁₋₆ alkyl)-;
each R², R³, and R⁴ is independently selected from the group consisting of halogen, -OH, -NH₂, -CN, and C₁₋₃ alkyl (e.g., methyl, ethyl, or propyl);
m is selected from the group consisting of 1 and 2;
p and q are each independently selected from the group consisting of 0 and 1.
In some embodiments, the present application relates to a compound of formula I-AA, a compound of formula I, or a compound of formula I-1, a stereoisomer thereof, or a pharmaceutically acceptable salt thereof, wherein,
ring A is selected from the group consisting of cyclopentenyl, dihydropyrrolyl, tetrahydropyridinyl, tetrahydroazepinyl, azaspirooctenyl, phenyl, pyrrolyl, and pyrazolyl;
ring B is phenyl;
ring C is selected from the group consisting of isoxazolyl and furanyl;
n is 0;
L is selected from the group consisting of piperidinyl, diazaspirononanyl, piperazinyl, monoazaspirononanyl, 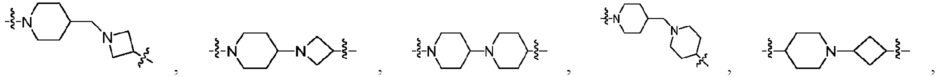 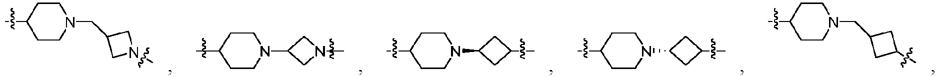 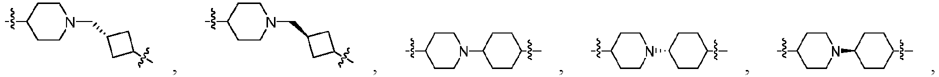 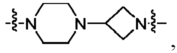 azetidinyl, and tetrahydropyridinyl, which can be optionally attached to C₁₋₃ alkylene;
X¹, X², X³, and X⁴ are all CH, or two of X¹, X², X³, and X⁴ are N and the other two are CH;
X⁵ is selected from the group consisting of CH and N;
X⁶ is selected from the group consisting of -O-, -NH-, and -N(C₁₋₄ alkyl)-;
R² is selected from the group consisting of halogen and -CN;
m is 2;
p and q are each independently 0.

The present application also relates to the following compound, a stereoisomer thereof, or a pharmaceutically acceptable salt thereof: 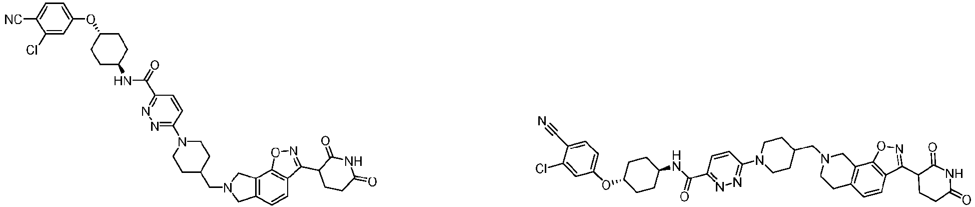 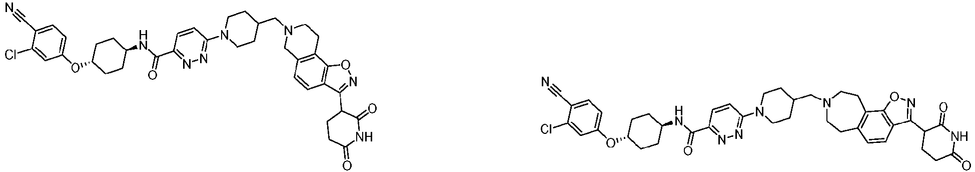 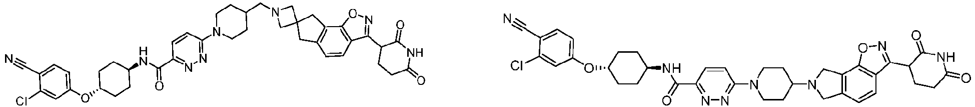 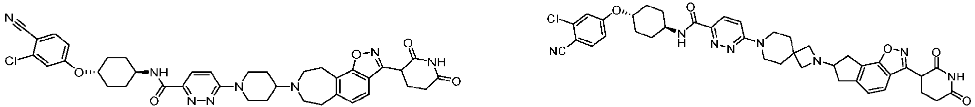 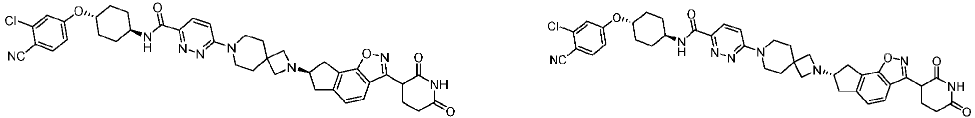 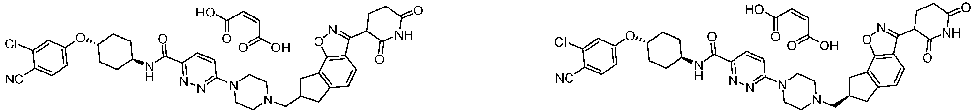 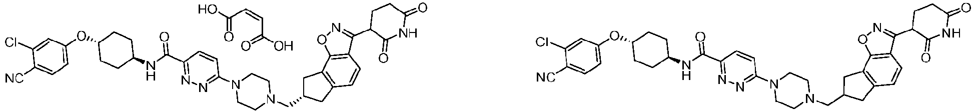 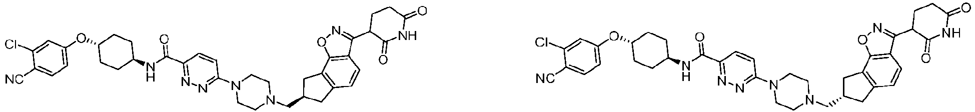 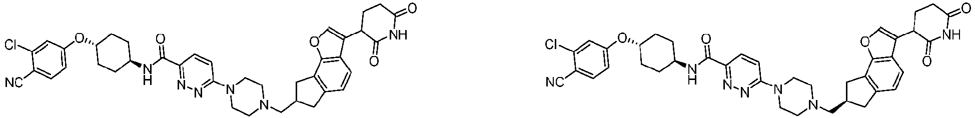 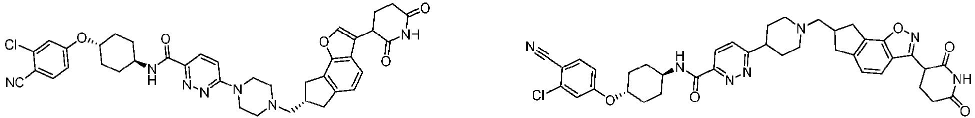 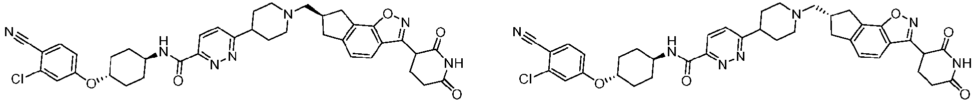 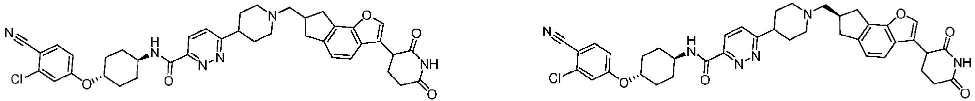 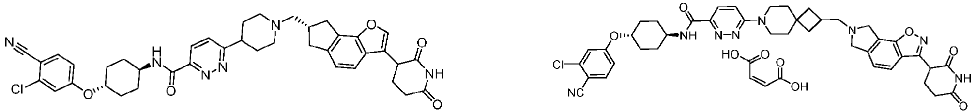 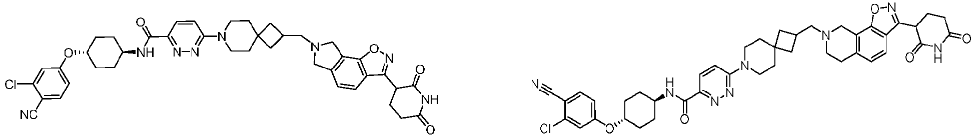 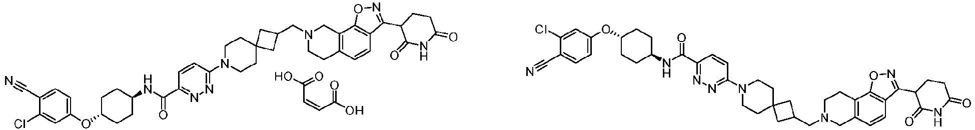 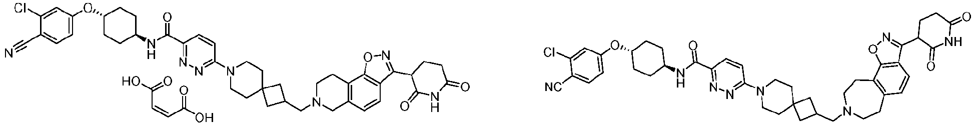 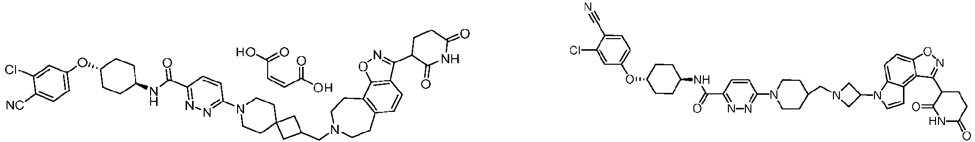 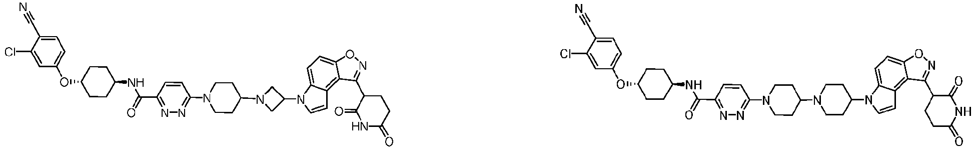 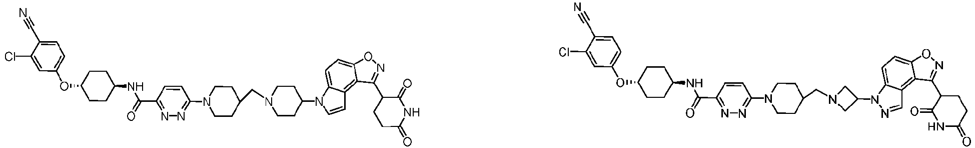 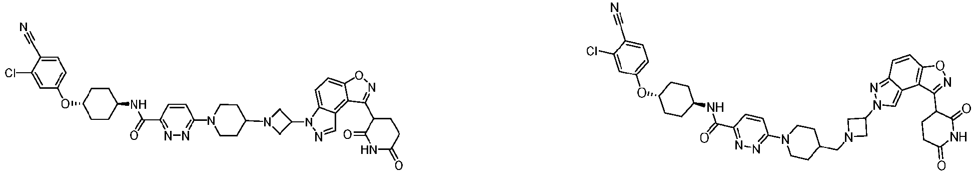 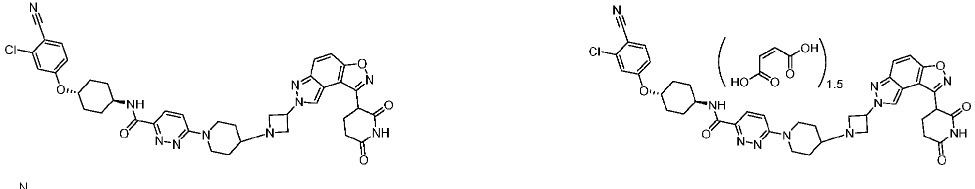 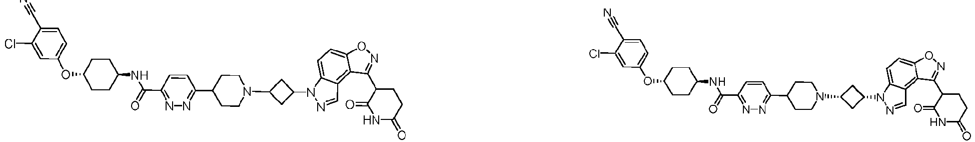 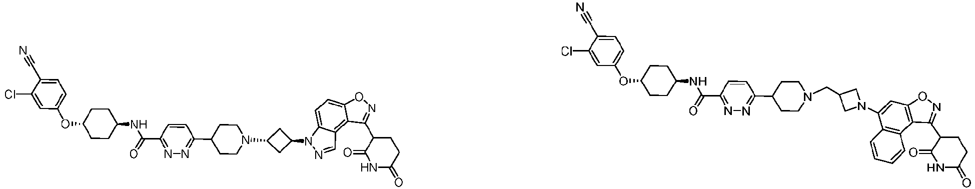 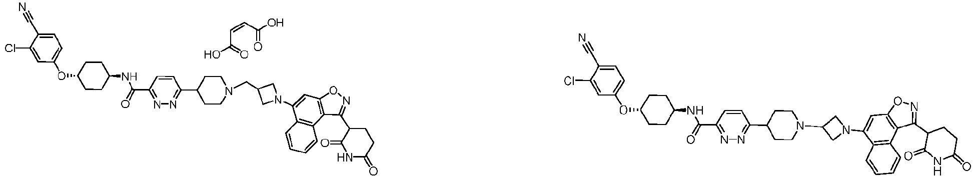 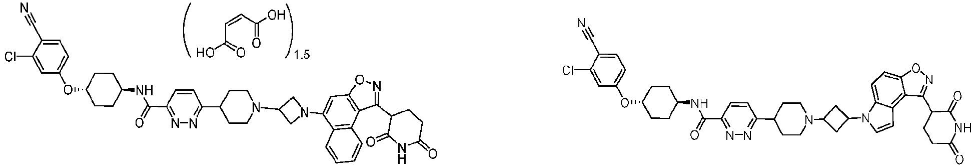 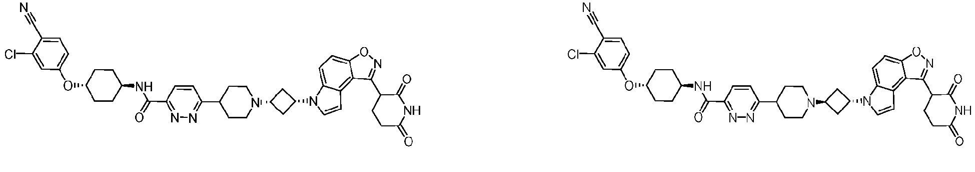 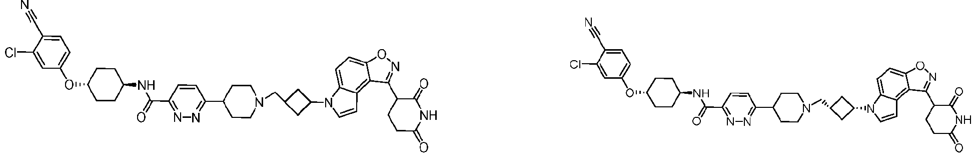 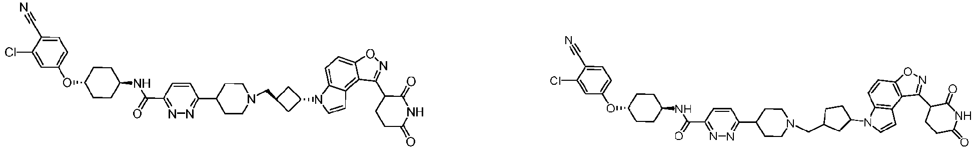 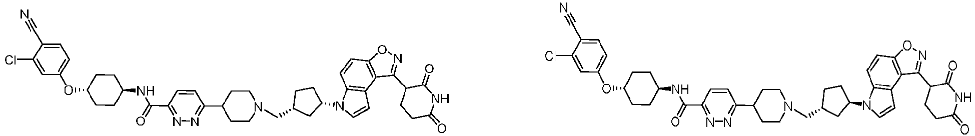 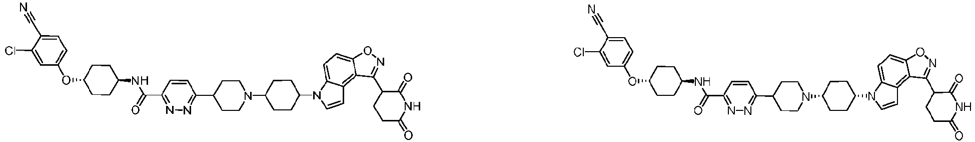 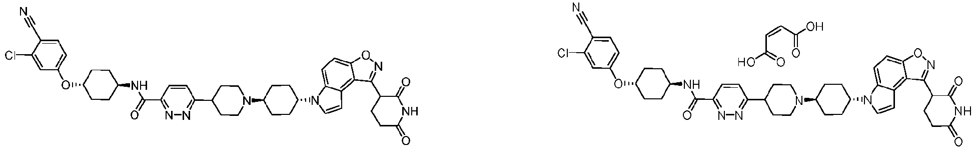 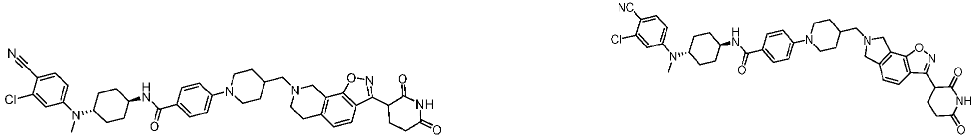 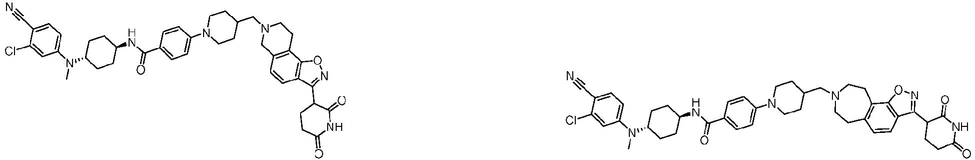 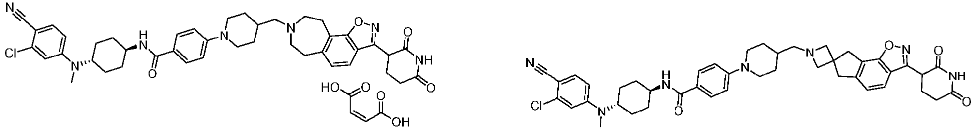 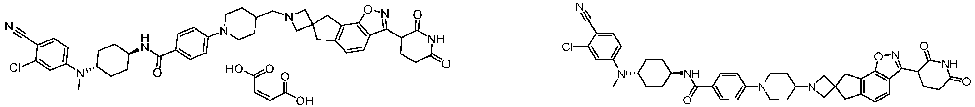 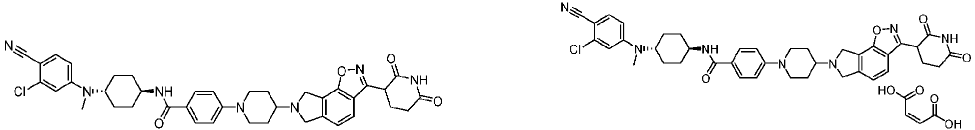 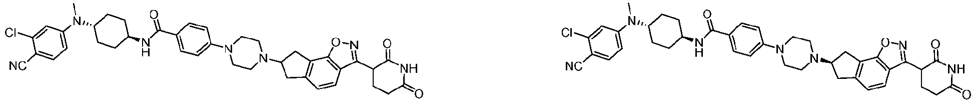 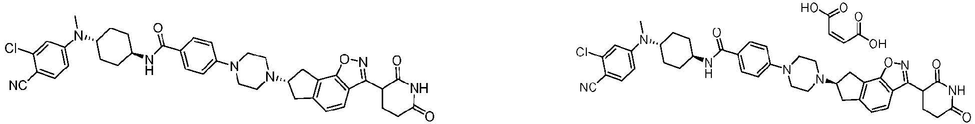 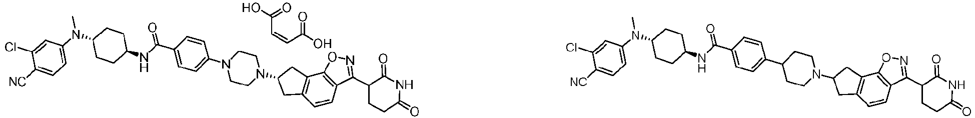 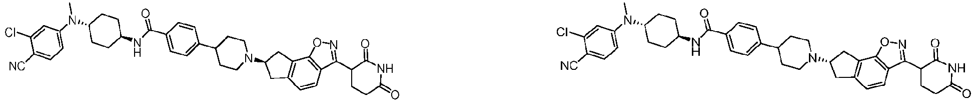 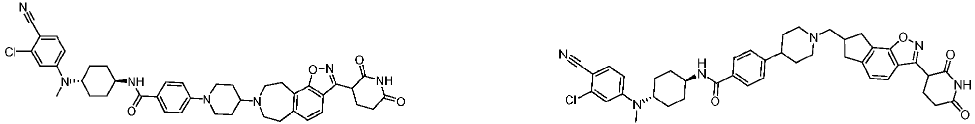 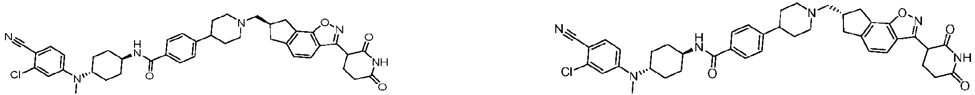 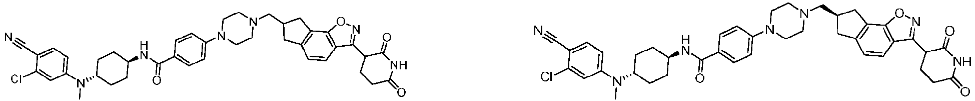 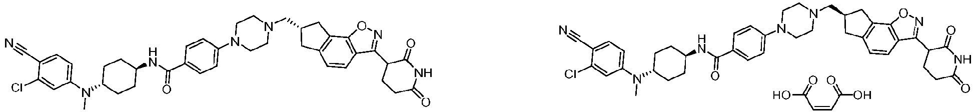 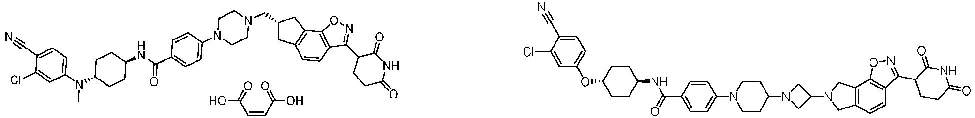 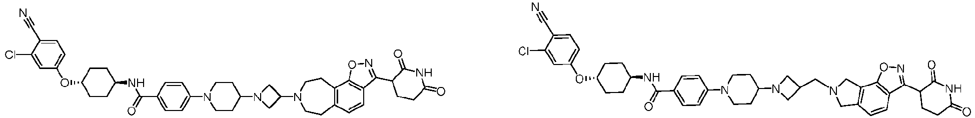 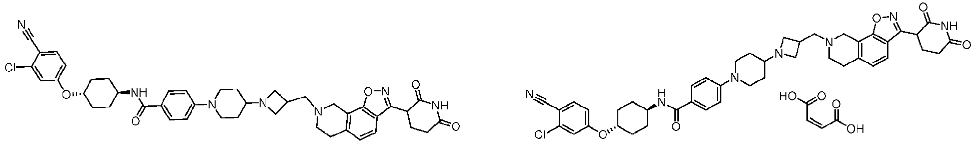 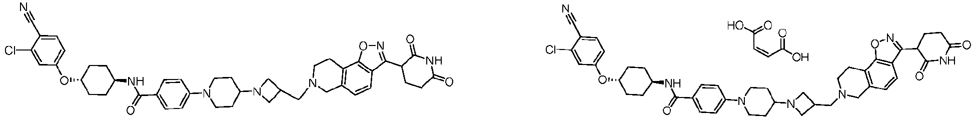 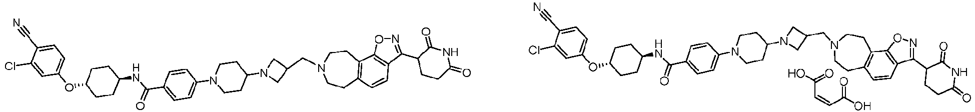 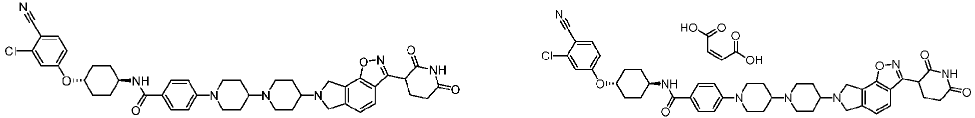 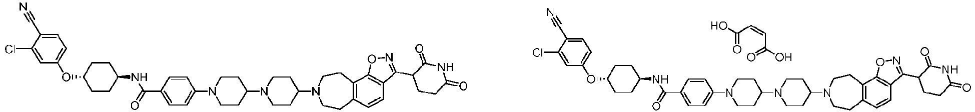 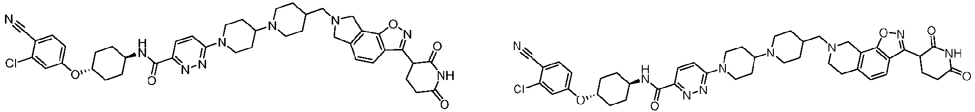 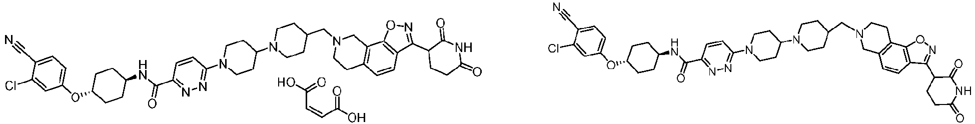 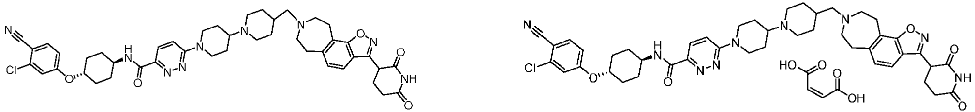 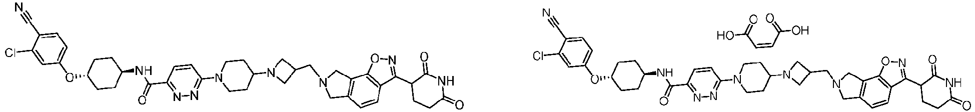 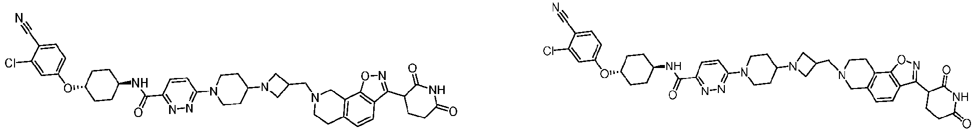 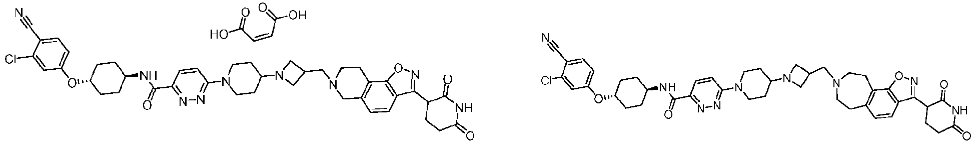 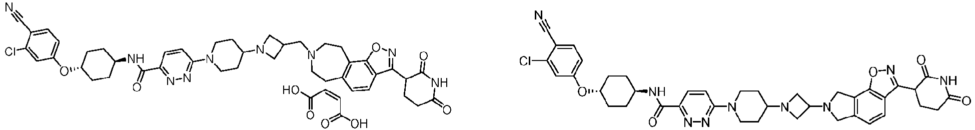 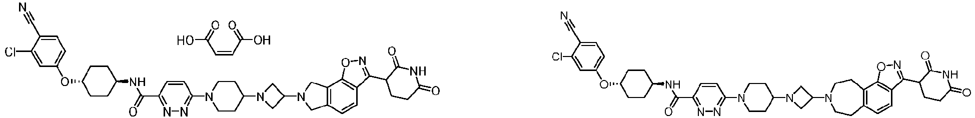 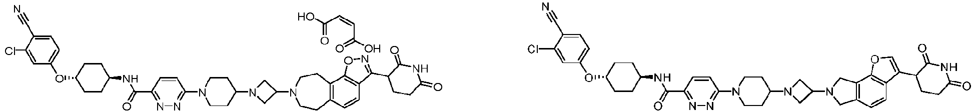 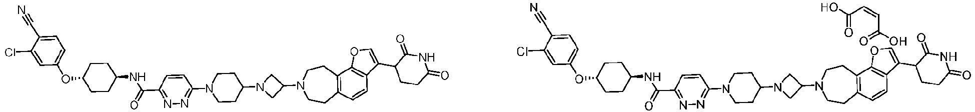 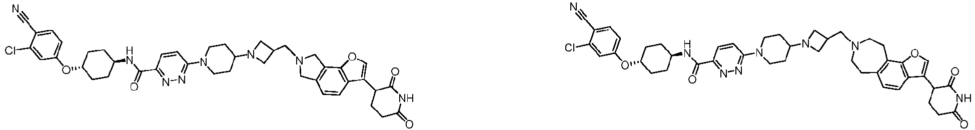 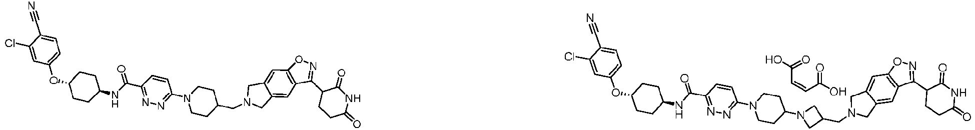 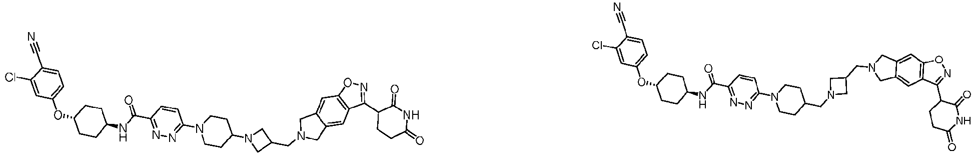 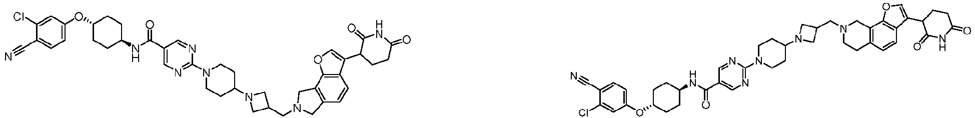 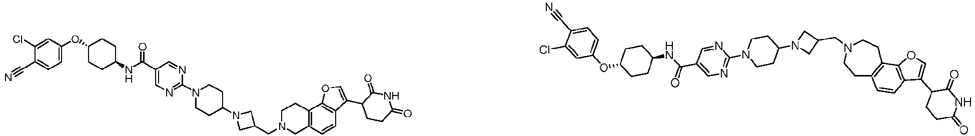 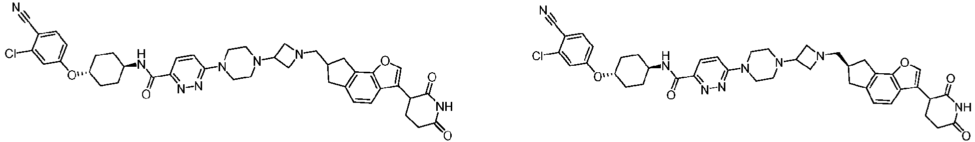 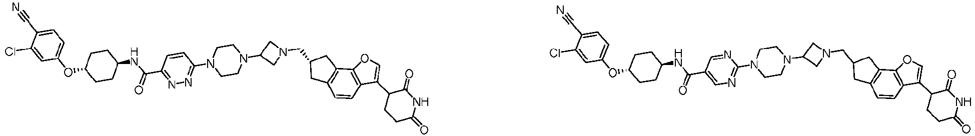 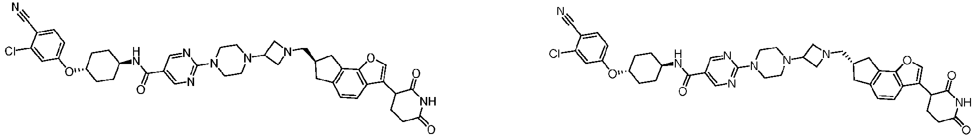 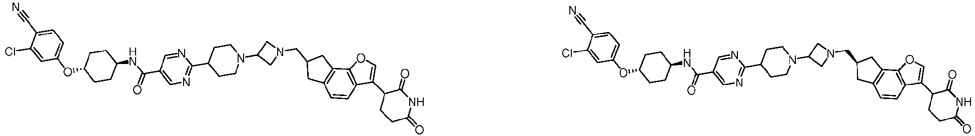 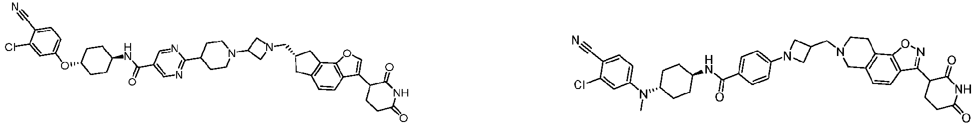 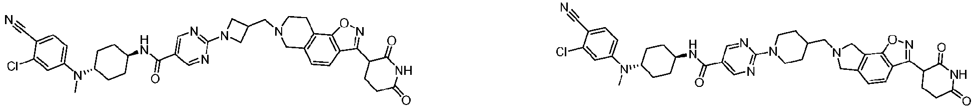 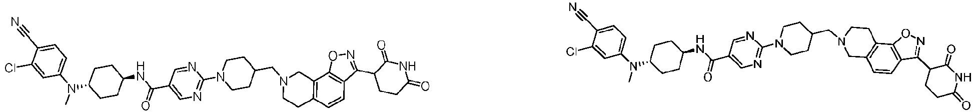 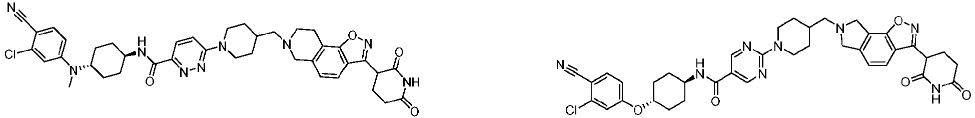 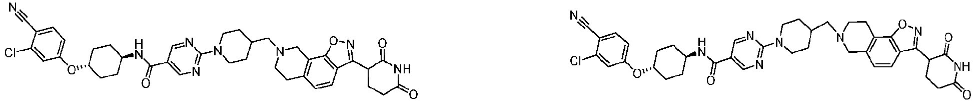 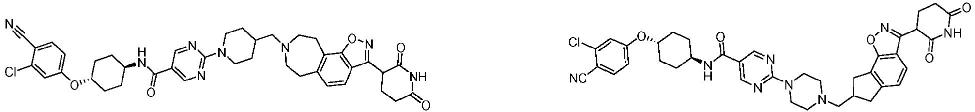 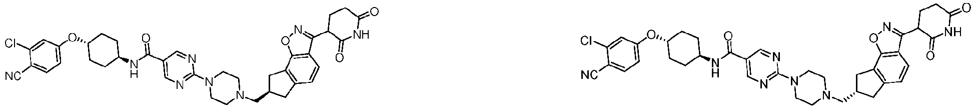 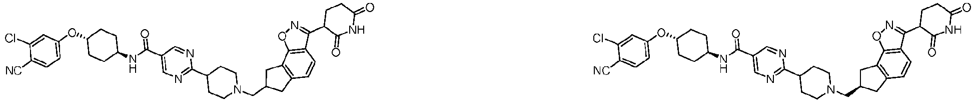 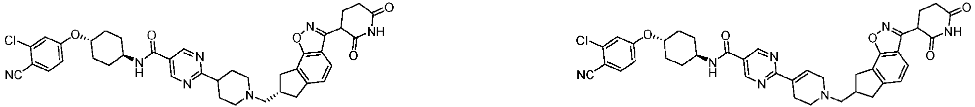 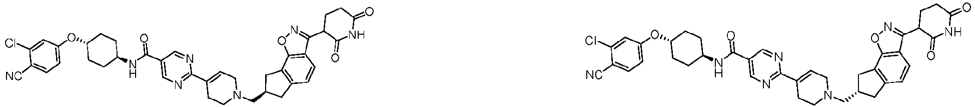 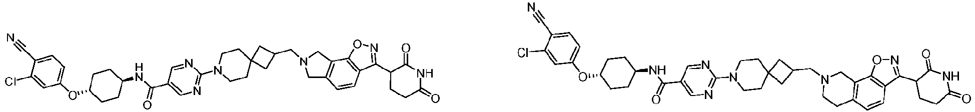 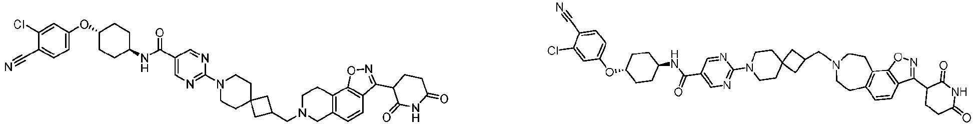 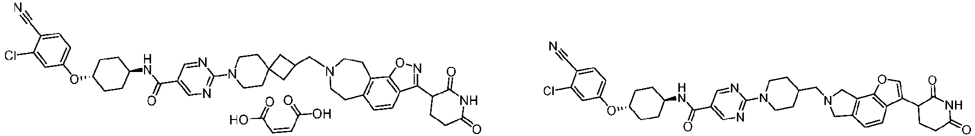 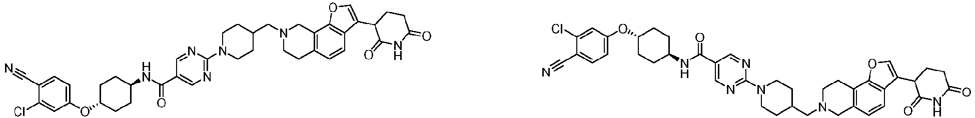 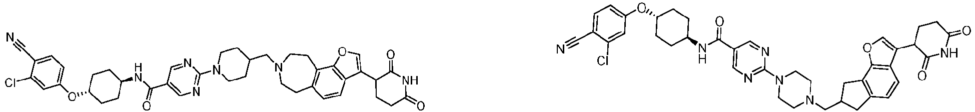 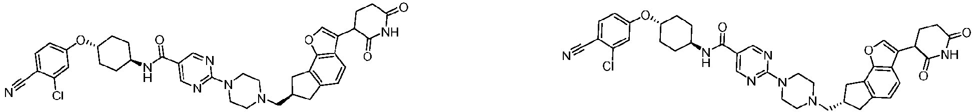 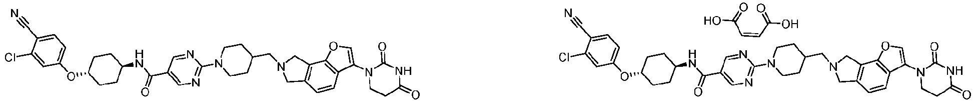 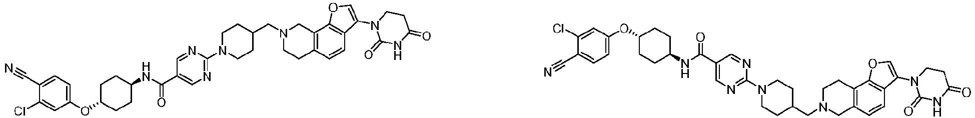 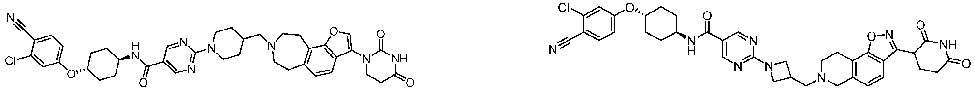 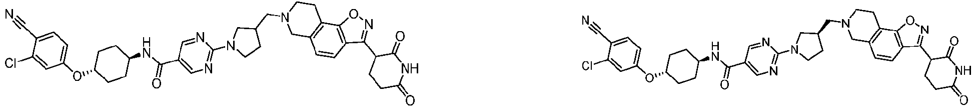 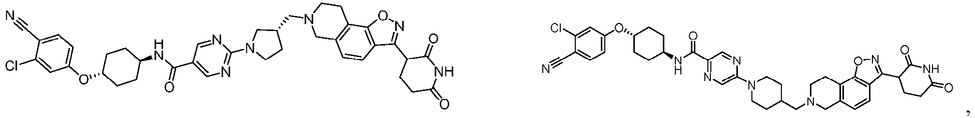 or

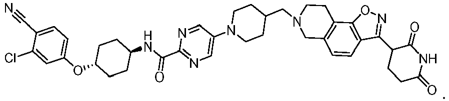

In some embodiments, the pharmaceutically acceptable salt is selected from maleate.

The present application also covers solutions obtained by any combination, deletion, or exchange of the embodiments described above.

In another aspect, the present application relates to a pharmaceutical composition comprising the compound, the stereoisomer thereof, or the pharmaceutically acceptable salt thereof of the present application described above. The pharmaceutical composition of the present application also comprises a pharmaceutically acceptable excipient.

In another aspect, the present application relates to use of the compound, the stereoisomer thereof, or the pharmaceutically acceptable salt thereof, or the pharmaceutical composition thereof described above for preparing a medicament for preventing or treating a disorder treated by degrading a target protein (e.g., androgen receptor, AR) that binds to a targeting ligand.

In another aspect, the present application relates to use of the compound, the stereoisomer thereof, or the pharmaceutically acceptable salt thereof, or the pharmaceutical composition thereof described above for preparing a medicament for preventing or treating a disorder treated by binding to cereblon protein *in vivo.*

In another aspect, the present application relates to use of the compound, the stereoisomer thereof, or the pharmaceutically acceptable salt thereof, or the pharmaceutical composition thereof described above for preparing a medicament for preventing or treating an AR-related disease.

The present application relates to a method for treating or preventing a disorder treated by degrading a target protein (e.g., androgen receptor, AR) that binds to a targeting ligand in a mammal, which comprises administering to a mammal (preferably a human) in need of such treatment a therapeutically effective amount of the compound, the stereoisomer thereof, or the pharmaceutically acceptable salt thereof, or the pharmaceutical composition thereof of the present application described above.

The present application relates to a method for treating or preventing a disorder treated by binding to cereblon protein *in vivo,* which comprises administering to a mammal (preferably a human) in need of such treatment a therapeutically effective amount of the compound, the stereoisomer thereof, or the pharmaceutically acceptable salt thereof, or the pharmaceutical composition thereof of the present application described above.

In another aspect, the present application relates to a method for treating an AR-related disease in a mammal, which comprises administering to a mammal (preferably a human) in need of such treatment a therapeutically effective amount of the compound, the stereoisomer thereof, or the pharmaceutically acceptable salt thereof, or the pharmaceutical composition thereof of the present application described above.

In another aspect, the present application relates to the compound, the stereoisomer thereof, or the pharmaceutically acceptable salt thereof, or the pharmaceutical composition thereof described above for use in preventing or treating a disorder treated by degrading a target protein (e.g., androgen receptor, AR) that binds to a targeting ligand.

In another aspect, the present application relates to the compound, the stereoisomer thereof, or the pharmaceutically acceptable salt thereof, or the pharmaceutical composition thereof described above for use in preventing or treating a disorder treated by binding to cereblon protein *in vivo.*

In another aspect, the present application relates to the compound, the stereoisomer thereof, or the pharmaceutically acceptable salt thereof, or the pharmaceutical composition thereof described above for use in preventing or treating an AR-related disease.

In another aspect, the present application relates to use of the compound, the stereoisomer thereof, or the pharmaceutically acceptable salt thereof, or the pharmaceutical composition thereof described above for preventing or treating a disorder treated by degrading a target protein (e.g., androgen receptor, AR) that binds to a targeting ligand.

In another aspect, the present application relates to use of the compound, the stereoisomer thereof, or the pharmaceutically acceptable salt thereof, or the pharmaceutical composition thereof described above for preventing or treating a disorder treated by binding to cereblon protein *in vivo.*

In another aspect, the present application relates to use of the compound, the stereoisomer thereof, or the pharmaceutically acceptable salt thereof, or the pharmaceutical composition thereof described above for preventing or treating an AR-related disease.

In some specific embodiments, the AR-related disease described above is selected from the group consisting of disorders treated by degrading and/or inhibiting a protein (androgen receptor, AR) that binds to an AR target protein ligand; in some specific embodiments, the AR-related disease described above is selected from the group consisting of disorders treated by binding to cereblon protein *in vivo*; in some embodiments, the disease or disorder described above is selected from the group consisting of cancers, e.g., prostatic cancer.

In some specific embodiments, the disorder treated by binding to cereblon protein *in vivo* and/or by degrading a target protein that binds to a targeting ligand described above is selected from the group consisting of AR-related diseases; in some specific embodiments, the AR-related disease described above is selected from the group consisting of cancers, e.g., prostatic cancer.

"One or more" used herein refers to an integer ranging from one to ten. For example, "one or more" refers to one, two, three, four, five, six, seven, eight, nine, or ten; in some embodiments, the "one or more" is selected from the group consisting of one, two, three, four, five, and six. In some embodiments, the "one or more" is selected from the group consisting of one, two, and three. In some embodiments, the "one or more" is selected from the group consisting of one and two.

In some embodiments, the present application encompasses the variables defined above and embodiments thereof, as well as any combination thereof.

### Technical effects

The compounds of the present application have degradation activity against VCaP cell ARs and LNCaP cell ARs and have inhibitory activity against the proliferation of VCaP cells and LNCaP cells. In addition, the compounds of the present application also have good *in vitro* liver microsome stability and good *in vivo* pharmacokinetic properties (such as AUC and other parameters) in mammals (such as mice), can inhibit the growth of tumors *in vivo,* and show good druggability.

### Definitions

Unless otherwise stated, the following terms used in the present application shall have the following meanings. A certain term, unless otherwise specifically defined, should not be considered uncertain or unclear, but construed according to its common meaning in the art. When referring to a trade name, it is intended to refer to its corresponding commercial product or its active ingredient.

The term "substituted" means that any one or more hydrogen atoms on a specific atom are substituted with substituents, as long as the valence of the specific atom is normal and the resulting compound is stable. When the substituent is oxo (i.e., =O), it means that two hydrogen atoms are substituted, and oxo is not possible in an aromatic group.

The term "optional" or "optionally" means that the subsequently described event or circumstance may, but does not necessarily, occur. The description includes instances where the event or circumstance occurs and instances where it does not. "Optionally substituted" includes unsubstituted and substituted. For example, ethyl being "optionally" substituted with halogen means that the ethyl may be unsubstituted (CH₂CH₃), monosubstituted (for example, CH₂CH₂F), polysubstituted (for example, CHFCH₂F and CH₂CHF₂), or fully substituted (CF₂CF₃). It can be understood by those skilled in the art that for any groups comprising one or more substituents, no substitutions or substitution modes that are impossible to spatially exist and/or synthesize will be introduced.

Cₘ₋ₙ used herein means that the moiety has an integer number of carbon atoms in the given range. For example, "C₁₋₆" means that the group may have 1 carbon atom, 2 carbon atoms, 3 carbon atoms, 4 carbon atoms, 5 carbon atoms, or 6 carbon atoms.

When any variable (e.g., R) occurs once or more times in the constitution or structure of a compound, the definition of the variable in each case is independent. For example, if a group comprises 2 R, the definition of each R is independent.

When a bond is crosslinked to two atoms of a ring (including monocyclic, fused, or spiro ring), the bond may be bonded to any atom on the ring (including monocyclic, fused, or spiro ring). For example, the structural unit 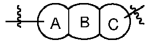 indicates that the bonds on two sides may be linked to any two different atoms on ring A, ring B, or ring C; for another example, 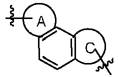 indicates that the bonds on two sides may be linked to any two different atoms on ring A, the middle benzene ring, or ring C; for further example, 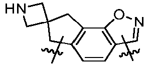 indicates that the bonds on two sides may be linked to any two different atoms of the four rings in the system.

The term "halo-" or "halogen" refers to fluorine, chlorine, bromine, and iodine.

The term "hydroxy" refers to -OH group.

The term "amino" refers to -NH₂ group.

The term "cyano" refers to -CN group.

The term "alkyl" refers to hydrocarbyl with a general formula of CₙH₂ₙ₊₁. The alkyl may be linear or branched. For example, the term "C₁-₆ alkyl" refers to alkyl containing 1 to 6 carbon atoms (for example, methyl, ethyl, n-propyl, isopropyl, n-butyl, isobutyl, sec-butyl, *tert*-butyl, n-pentyl, 1-methylbutyl, 2-methylbutyl, 3-methylbutyl, neopentyl, hexyl, and 2-methylpentyl). Similarly, the alkyl moieties (i.e., alkyl) of alkoxy, alkylamino, dialkylamino, alkylsulfonyl, and alkylthio have the same definition as those described above.

The term "alkylene" refers to a divalent group formed by the removal of one hydrogen at any position of alkyl. For example, the term "C₁-₆ alkyl" refers to alkylene containing 1 to 6 carbon atoms; the term "C₁-₄ alkyl" refers to alkylene containing 1 to 4 carbon atoms, including but not limited to -CH₂-, -CH₂CH₂-, -CH₂CH₂CH₂-, or - CH₂CH₂CH₂CH₂-.

The term "alkenylene" refers to a divalent group formed by the removal of one hydrogen at any position of alkenyl. For example, the term "C₂-₆ alkenyl" refers to alkenylene containing 2 to 6 carbon atoms; the term "C₂-₄ alkenyl" refers to alkenylene containing 2 to 4 carbon atoms, including but not limited to -CH₂CH=CH-, -CH₂CH₂CH=CH-, or -CH₂CH=CHCH₂-.

The term "alkynylene" refers to a divalent group formed by the removal of one hydrogen at any position of alkynyl. For example, the term "C₂-₆ alkynyl" refers to alkynylene containing 2 to 6 carbon atoms; the term "C₂-₄ alkynyl" refers to alkynylene containing 2 to 4 carbon atoms, including but not limited to -C≡C-, -H₂C-C≡C-, -H₂C-H₂C-C≡C-, or -H₂C-C≡C-CH₂-.

The term "heteroalkyl" is linear or branched alkyl consisting of a certain number of carbon atoms and at least one heteroatom; it has preferably 1 to 14 carbons, more preferably 1 to 10 carbons, even more preferably 1 to 6 carbons, and most preferably 1 to 3 carbons in the chain, and preferably has 1, 2, or 3 heteroatoms selected from the group consisting of S, O, and N. For example, Cₘ heteroalkyl represents alkyl consisting of m carbon atoms and at least one heteroatom (e.g., 1-3 heteroatoms selected from the group consisting of S, O, and N) located between any two carbon atoms or attached to the endmost carbon atom and having a heteroatom inserted in the chain. The nitrogen and sulfur atoms are optionally oxidized and the nitrogen atom is optionally quaternized. The heteroatom or heteroatom group may be located at any internal position of heterohydrocarbyl, including the position where the hydrocarbyl is attached to the rest of the molecule. Exemplary heteroalkyl includes alkyl ether, secondary alkylamine and tertiary alkylamine, amide, sulfide, and the like, including alkoxy, alkylthio, and alkylamino. Unless otherwise specified, C₁₋₆ heteroalkyl includes C₁, C₂, C₃, C₄, C₅, and C₆ heteroalkyl, e.g., C₁₋₆ alkoxy, C₁₋₆ alkylthio, or C₁₋₆ alkylamino.

The term "heteroalkylene" refers to a divalent group formed by the removal of one hydrogen at any position of heteroalkyl.

The term "alkoxy" refers to -O- alkyl.

The term "alkenyl" refers to linear or branched unsaturated aliphatic hydrocarbyl consisting of carbon atoms and hydrogen atoms and having at least one double bond. Non-limiting examples of the alkenyl include, but are not limited to, ethenyl, 1-propenyl, 2-propenyl, 1-butenyl, isobutenyl, 1,3-butadienyl, and the like.

The term "cycloalkenyl" refers to a non-aromatic carbocyclic ring that is not fully saturated and may exist as a monocyclic ring, a bicyclic bridged ring, or a spiro ring. Unless otherwise specified, the carbocyclic ring is usually a 4- to 16-membered ring, a 4- to 12-membered ring, a 4- to 10-membered ring, or a 4- to 8-membered ring. Non-limiting examples of cycloalkenyl include, but are not limited to, cyclopentenyl, cyclopentadienyl, cyclohexenyl, cyclohexadienyl, cycloheptenyl, cycloheptadienyl, and the like.

The term "cycloalkyl" refers to a carbocyclic ring that is fully saturated and may exist as a monocyclic ring, a bridged ring, or a spiro ring. Unless otherwise specified, the carbocyclic ring is usually a 3- to 16-membered ring (e.g., a 3- to 10-membered ring or a 5- to 8-membered ring). Non-limiting examples of the cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, and the like.

The term "heterocycloalkyl" refers to a cyclic group that is fully saturated and may exist as a monocyclic ring, a bridged ring, or a spiro ring. Unless otherwise specified, the heterocyclyl is usually a 3- to 16-membered, 3- to 11-membered, 3- to 10-membered, 3- to 7-membered, 3- to 6-membered, or 3- to 5-membered ring containing 1 to 3 heteroatoms (preferably 1 or 2 heteroatoms) independently selected from the group consisting of sulfur, oxygen, and nitrogen. Examples of 3-membered heterocycloalkyl include, but are not limited to, oxiranyl, thiiranyl, and aziranyl; non-limiting examples of 4-membered heterocycloalkyl include, but are not limited to, azetidinyl, oxetanyl, and thietanyl; examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocycloalkyl include, but are not limited to, piperidinyl, tetrahydropyranyl, tetrahydrothiopyranyl, morpholinyl, piperazinyl, 1,4-oxathianyl, 1,4-dioxanyl, thiomorpholinyl, 1,3-dithianyl, and 1,4-dithianyl; examples of 7-membered heterocycloalkyl include, but are not limited to, azepanyl, oxepanyl, and thiepanyl. Monocyclic heterocycloalkyl having 5 or 6 ring atoms is preferred.

The term "spiro ring" refers to a fully saturated or partially unsaturated polycyclic ring system in which monocyclic rings share one carbon atom (referred to as a spiro atom), including carbocyclic rings and heterocyclic rings. Unless otherwise specified, the spiro ring is 5- to 20-membered, preferably 6- to 14-membered, and more preferably 8- to 12-membered. When the spiro ring is a heterocyclic ring, one or more ring atoms in the polycyclic ring are heteroatoms (preferably 1 or 2 heteroatoms) selected from the group consisting of N, O, S(O)ₙ, and P(O)ₙ (wherein n is 0, 1, or 2), and the remaining ring atoms are carbon atoms. The term "spirocycloalkyl" refers to a fully saturated all-carbon polycyclic ring in which monocyclic rings share one carbon atom (referred to as a spiro atom). Unless otherwise specified, the spirocycloalkyl is 5- to 20-membered, preferably 6- to 14-membered, and more preferably 8- to 12-membered. According to the number of spiro atoms shared among the rings, the spirocycloalkyl is monospirocycloalkyl, bispirocycloalkyl, or polyspirocycloalkyl, preferably monospirocycloalkyl or bispirocycloalkyl, and more preferably 4-membered/4-membered, 4-membered/5-membered, 4-membered/6-membered, 5-membered/5-membered, or 5-membered/6-membered monospirocycloalkyl. Non-limiting examples of the spirocycloalkyl include 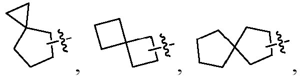 and

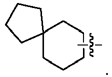

The term "spiro-heterocycloalkyl" refers to a fully saturated polycyclic ring in which monocyclic rings share one carbon atom (referred to as a spiro atom), wherein one or more ring atoms in the polycyclic ring are heteroatoms (preferably 1 or 2 heteroatoms) selected from the group consisting of N, O, S(O)ₙ, and P(O)ₙ (wherein n is 0, 1, or 2), and the remaining ring atoms are carbon atoms. Unless otherwise specified, the spiro-heterocycloalkyl is 5- to 20-membered, preferably 6- to 14-membered, and more preferably 6- to 10-membered. According to the number of spiro atoms shared among the rings, the spiro heterocyclic ring is a monospiro heterocyclic ring, a bispiro heterocyclic ring, or a polyspiro heterocyclic ring, preferably a monospiro heterocyclic ring or a bispiro heterocyclic ring, and more preferably a 4-membered/4-membered, 4-membered/5-membered, 4-membered/6-membered, 5-membered/5-membered, or 5-membered/6-membered monospiro heterocyclic ring. Non-limiting examples of the spiro-heterocycloalkyl include 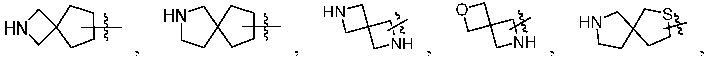 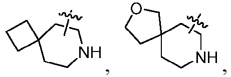 or the like.

The term "heterocycloalkenyl" includes cycloalkenyl in which one or more (e.g., 1-5, 1-4, 1-3, or 1-2) carbon atoms are substituted with a heteroatom, such as, specifically, cycloalkenyl in which up to 3 carbon atoms, in one embodiment up to 2 carbon atoms, and in another embodiment 1 carbon atom, are each independently replaced by O, S, S(O), or N, provided that at least one cycloalkenyl carbon-carbon double bond is preserved. The heterocycloalkenyl may be a cyclic group that exists as a monocyclic ring, a bridged ring, or a spiro ring and may be a 3- to 16-membered ring (for example, a 3- to 12-membered or 5- to 8-membered ring, specifically, for example, a 5-membered, 6-membered, 7-membered, 8-membered, 9-membered, 10-membered, or 11-membered ring). Examples of heterocycloalkenyl include, but are not limited to, dihydropyrrolyl, tetrahydropyridinyl, tetrahydroazepinyl, or azaspirooctenyl.

The term "aryl" refers to an all-carbon aromatic monocyclic or fused polycyclic group having a conjugated π-electron system. For example, aryl may have 6-20 carbon atoms, 6-14 carbon atoms, or 6-12 carbon atoms. Non-limiting examples of the aryl include, but are not limited to, phenyl, naphthyl, anthryl, 1,2,3,4-tetrahydronaphthalene, and the like.

The term "heteroaryl" refers to a monocyclic or fused polycyclic system that comprises at least one (e.g., 1-5, 1-4, 1-3, or 1-2) ring atom selected from the group consisting of N, O, and S, with the remaining ring atoms being C, and that has at least one aromatic ring. Preferably, the heteroaryl has a single 4- to 8-membered ring, in particular a 5- to 8-membered ring (e.g., 5-membered, 6-membered, 7-membered, or 8-membered), or has a plurality of fused rings comprising 6-14 ring atoms, in particular 6-10 (e.g., 6, 7, 8, 9, or 10) ring atoms. Non-limiting examples of the heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like.

Unless otherwise specified, the term "hetero" refers to a heteroatom or a heteroatom group (i.e., a heteroatom-containing group), including atoms other than carbon (C) and hydrogen (H) and groups containing such heteroatoms, including, for example, oxygen (O), nitrogen (N), sulfur (S), phosphorus (P), silicon (Si), germanium (Ge), aluminum (Al), boron (B), -O-, -S-, =O, =S, -P(=O)-, -P(=O)₂-, -P(=O)O-, -P(=O)₂O-, -C(=O)O-, -C(=O)-, - C(=S)-, -S(=O)-, -S(=O)₂-, and optionally substituted -C(=O)N(H)-, -N(H)-, -C(=NH)-, -S(=O)₂N(H)-, or - S(=O)N(H)-.

The term "derivative" refers to a new compound or a group of new compounds that are produced through one or more chemical reactions or structural evolutions. The derivative retains the basic structure of the parent compound, with changes or modifications occurring in the side chains, functional groups, or substituents only.

The term "substituent", "optionally substituted with one or more substituents", or "optionally substituted" includes all substituents and substitutions with substituents mentioned in the text, and may be, e.g., the terms "halogen", "deuterium", "O 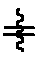 ", "-NH₂", "-NH(C₁₋₄ alkyl)", "-N(C₁₋₄ alkyl)₂", "-OH", "-OC₁₋₄ alkyl", "-CN", "C₁₋₄ alkyl", and "3- to 6-membered heterocycloalkyl", and the corresponding non-limiting or exemplary groups mentioned below, wherein some non-limiting examples of the "substituent" include sulfydryl, nitro, nitroso, cyano, azido, a sulfoxide group, a sulfone group, a sulfonamide group, carboxyl, an aldehyde group, an imine group, alkyl, halogenated alkyl, cycloalkyl, halogenated cycloalkyl, alkenyl, halogenated alkenyl, cycloalkenyl, halogenated cycloalkenyl, alkynyl, halogenated alkynyl, cycloalkynyl, halogenated cycloalkynyl, heteroalkyl, halogenated heteroalkyl, alkoxy, alkylthio, aryl, aryloxy, arylthio, arylalkylene, arylalkoxy, arylalkylthio, heteroaryl, heteroaryloxy, heteroarylthio, heteroarylalkylene, heteroarylalkoxy, heteroarylalkylthio, heterocyclyl, heterocyclyloxy, heterocyclylthio, heterocyclylalkylene, heterocyclylalkoxy, heterocyclylalkylthio, acyl, acyloxy, a carbamate group, amido, ureido, an epoxy group, an ester group, oxo, and the like, wherein these substituents are optionally substituted with one or more substituents selected from the group consisting of the following substituents: oxo, hydroxy, amino, nitro, halogen, cyano, alkyl, alkenyl, alkynyl, alkoxy, halogenated alkoxy, alkylamino, dialkylamino, halogenated alkylamino, halogenated dialkylamino, carboxyl, -C(O)O-alkyl, -OC(O)-alkyl, -C(O)NH₂, -C(O)NH-alkyl, - C(O)N(alkyl)₂, -NHC(O)-alkyl, -C(O)-alkyl, -S(O)-alkyl, -S(O)₂-alkyl, -S(O)₂NH₂, -S(O)₂NH-alkyl, - S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

In some embodiments herein, the "substituent" is selected from the group consisting of deuterium, tritium, hydroxyl, sulfydryl, halogen, amino, nitro, nitroso, cyano, azido, a sulfoxide group, a sulfone group, a sulfonamide group, carboxyl, an aldehyde group, an imine group, C₁₋₁₂ alkyl, halogenated C₁₋₁₂ alkyl, 3- to 12-membered cycloalkyl, halogenated 3- to 12-membered cycloalkyl, C₂₋₁₂ alkenyl, halogenated C₂₋₁₂ alkenyl, 3- to 12-membered cycloalkenyl, halogenated 3- to 12-membered cycloalkenyl, C₂₋₁₂ alkynyl, halogenated C₂₋₁₂ alkynyl, 8- to 12-membered cycloalkynyl, halogenated 8- to 12-membered cycloalkynyl, C₁₋₁₂ heteroalkyl, halogenated C₁₋₁₂ heteroalkyl, C₁₋₁₂ alkoxy, C₁₋₁₂ alkylthio, 6- to 10-membered aryl, 6- to 10-membered aryloxy, 6- to 10-membered arylthio, 6- to 10-membered aryl C₁₋₁₂ alkylene, 6- to 10-membered aryl C₁₋₁₂ alkoxy, 6- to 10-membered aryl C₁₋₁₂ alkylthio, 5- to 10-membered heteroaryl, 5- to 10-membered heteroaryloxy, 5- to 10-membered heteroarylthio, 5-to 10-membered heteroarylalkylene, 5- to 10-membered heteroarylalkoxy, 5- to 10-membered heteroarylalkylthio, 3- to 12-membered heterocyclyl, 3- to 12-membered heterocyclyloxy, 3- to 12-membered heterocyclylthio, 3- to 12-membered heterocyclyl C₁₋₁₂ alkylene, 3-to 12-membered heterocyclyl C₁₋₁₂ alkoxy, 3- to 12-membered heterocyclyl C₁₋₁₂ alkylthio, C₁₋₁₂ acyl, C₁₋₁₂ acyloxy, a carbamate group, C₁₋₁₂ amido, ureido, an epoxy group, a C₂₋₁₂ ester group, and oxo, wherein these substituents are optionally substituted with one or more substituents selected from the group consisting of the following substituents: oxo, hydroxy, amino, nitro, halogen, cyano, C₁₋₁₂ alkyl, C₂₋₁₂ alkenyl, C₂₋₁₂ alkynyl, C₁₋₁₂ alkoxy, halogenated C₁₋₁₂ alkoxy, C₁₋₁₂ alkylamino, di-C₁₋₁₂ alkylamino, halogenated C₁₋₁₂ alkylamino, halogenated di-C₁₋₁₂ alkylamino, carboxyl, -C(O)O-C₁₋₁₂ alkyl, -OC(O)-C₁₋₁₂ alkyl, -C(O)NH₂, - C(O)NH-C₁₋₁₂ alkyl, -C(O)N(C₁₋₁₂ alkyl)₂, -NHC(O)-C₁₋₁₂ alkyl, -C(O)-C₁₋₁₂ alkyl, -S(O)-C₁₋₁₂ alkyl, -S(O)₂-C₁₋₁₂ alkyl, -S(O)₂NH₂, -S(O)₂NH-C₁₋₁₂ alkyl, -S(O)₂N(C₁₋₁₂ alkyl)₂, 3- to 12-membered cycloalkyl, 3- to 12-membered cycloalkyl C₁₋₁₂ alkylene, 3- to 12-membered cycloalkyloxy, 3- to 12-membered heterocyclyl, 3- to 12-membered heterocyclyl C₁₋₁₂ alkylene, 3- to 12-membered heterocyclyloxy, 3- to 12-membered heterocycloalkyl, 3- to 12-membered heterocycloalkyl C₁₋₁₂ alkylene, 3- to 12-membered heterocycloalkyloxy, 5- to 10-membered heteroaryl, 5- to 10-membered heteroaryl C₁₋₁₂ alkylene, 5- to 10-membered heteroaryloxy, 6- to 10-membered aryl, 6- to 10-membered aryl C₁₋₁₂ alkylene, and 6- to 10-membered aryloxy.

Unless otherwise specified, the term "hetero" refers to a heteroatom or a heteroatom group (i.e., a heteroatom-containing group), including atoms other than carbon (C) and hydrogen (H) and groups containing such heteroatoms. For example, heteroatoms include, but are not limited to, oxygen (O), nitrogen (N), sulfur (S), phosphorus (P), silicon (Si), germanium (Ge), aluminum (Al), and boron (B); specific heteroatoms or heteroatom groups are, e.g., -O-, -S-, -N=, =O, =S, -P(=O)-, -P(=O)₂-, -P(=O)O-, -P(=O)₂O-, -C(=O)O-, -C(=O)-, -C(=S)-, - S(=O), -S(=O)₂-, and optionally substituted -C(=O)N(H)-, -N(H)-, -C(=NH)-, -S(=O)₂N(H)-, or -S(=O)N(H)-. Preferably, the term "hetero" means that a heteroatom or a heteroatom of a heteroatom group (i.e., a heteroatom-containing group) is selected from the group consisting of oxygen, nitrogen, and sulphur.

The term "derivative" refers to a new compound or a group of new compounds that are produced through one or more chemical reactions or structural evolutions. The derivative retains the basic structure of the parent compound, with changes or modifications occurring in the side chains, functional groups, or substituents only.In the present application, one of the absolute configurations (e.g., one of 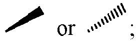 specifically 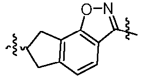 represents 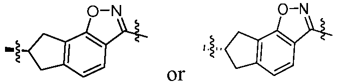 ) or one of the relative configurations (e.g., 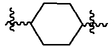 represents 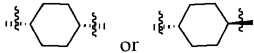 ) of a stereogenic center is represented by a wavy line ( 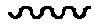 ). Unless otherwise specified, the compounds disclosed herein include both E and Z geometric isomers when they contain olefinic double bonds or other centers of geometric asymmetry. Likewise, all tautomeric forms are included within the scope of the present application.

The group or structural fragment in the present application, such as -LNK¹-Cy¹-LNK-Cy²-LNK²-, -Cy¹-Cy²-LNK²-, LNK, Cy¹, Cy², -Cy¹-LNK-Cy²-, -Cy¹-LNK-, or -LNK-Cy²-, and specific options thereof, optionally can be linked to the left-side group and the right-side group of the group or fragment respectively in the general formula in a left-to-right reading order. For example, in the case that L is selected from -Cy¹-LNK-Cy²-, when Cy¹ is selected from 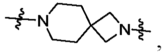 according to a left-to-right reading order, the left side of Cy¹ is linked to the fragment 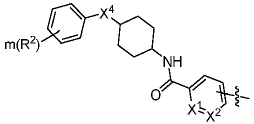 on the corresponding left side in the general formula, and the right side is linked to the fragment 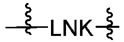 on the right side, resulting in a fragment 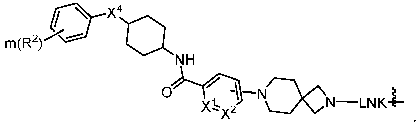 Optionally, the group or structural fragment in the present application, such as -LNK¹-Cy¹-LNK-Cy²-LNK²-, -Cy¹-Cy²-LNK²-, LNK, Cy¹, Cy², -Cy¹-LNK-Cy²-, -Cy¹-LNK-, or -LNK-Cy²-, and specific options thereof, can be linked to the left-side group and the right-side group of the group or fragment respectively in the general formula in a right-to-left reading order. For example, in the case that L is selected from -Cy¹-LNK-Cy², when Cy¹ is selected from 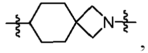 according to a right-to-left reading order, the right side of Cy¹ is linked to the fragment 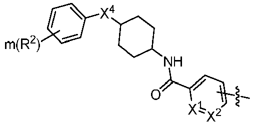 on the corresponding left side in the general formula, and the left side is linked to the fragment 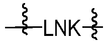 on the right side in the general formula, resulting in a fragment 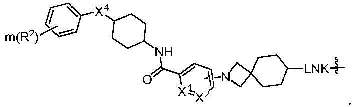 The other groups are as described above.

The term "treat", "treating", or "treatment" means administering the compound or formulation of the present application to ameliorate or eliminate a disease or one or more symptoms associated with the disease, and includes: (i) inhibiting the disease or disease state, i.e., arresting its progression; and (ii) relieving the disease or disease state, i.e., causing regression of the disease or disease state.

The term "prevent", "preventing", or "prevention" means administering the compound or formulation of the present application to prevent a disease or one or more symptoms associated with the disease, and includes: preventing the occurrence of the disease or disease state in a mammal, particularly when such a mammal is predisposed to the disease state but has not yet been diagnosed with it.

The term "therapeutically effective amount" refers to an amount of the compound of the present application for (i) treating or preventing a specific disease, condition, or disorder; (ii) alleviating, ameliorating, or eliminating one or more symptoms of a specific disease, condition, or disorder, or (iii) preventing or delaying onset of one or more symptoms of a specific disease, condition, or disorder described herein. The amount of the compound of the present application composing the "therapeutically effective amount" varies depending on the compound, the disease state and its severity, the mode of administration, and the age of the mammal to be treated, but may be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may be a metal salt, an ammonium salt, a salt formed with an organic base, a salt formed with an inorganic acid, a salt formed with an organic acid, and a salt formed with a basic or acidic amino acid.

The term "pharmaceutical composition" refers to a mixture consisting of one or more of the compounds or the salts thereof of the present application and a pharmaceutically acceptable excipient. The pharmaceutical composition is intended to facilitate the administration of the compound of the present application to an organic entity.

The term "pharmaceutically acceptable excipient" refers to those that do not have a significant irritating effect on an organic entity and do not impair the biological activity and properties of the active compound. Suitable excipients are well known to those skilled in the art, such as carbohydrate, wax, water-soluble and/or water-swellable polymers, hydrophilic or hydrophobic material, gelatin, oil, solvent, and water.

The word "comprise" and variations thereof such as "comprises" or "comprising" will be understood in an open, non-exclusive sense, i.e., "including but not limited to".

Unless otherwise specified clearly in the context, singular terms herein encompass plural referents, and vice versa. Similarly, unless otherwise specified clearly in the context, the word "or" herein is intended to include "and". Unless otherwise stated, all numbers expressing the amounts of ingredients, measurements, or reaction conditions used herein are to be understood as being modified in all instances by the term "about". The term "about" when connected to a percentage may mean, for example, ±1%, preferably, ±0.5%, and more preferably, ±0.1%.

The compounds and intermediates of the present application may also exist in different tautomeric forms, and all such forms are included within the scope of the present application. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerization and imine-enamine isomerization. A specific example of a proton tautomer is an imidazole moiety where a proton can transfer between two ring nitrogen atoms. A valence tautomer includes the interconversion via recombination of some bonding electrons. Specifically, any of the compounds of the present disclosure in which, for example, pyrazolyl is independent or as part of heterocyclyl, may exist in the form of any two tautomers or a mixture of two tautomers in any amount, i.e., 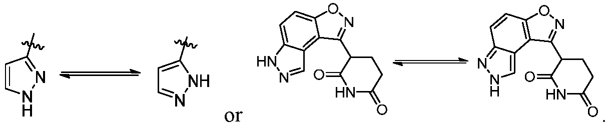 The present disclosure includes all possible tautomers of the compounds of the present disclosure, either as a single tautomer or any mixture of the tautomers in any ratio.

The present application also includes isotopically labeled compounds of the present application which are identical to those recited herein but have one or more atoms replaced by an atom having an atomic mass or mass number different from the atomic mass or mass number generally found in nature. Examples of isotopes that can be incorporated into the compounds of the present application include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Certain isotopically labeled compounds of the present application (e.g., those labeled with ³H and ¹⁴C) can be used to analyze compounds and/or substrate tissue distribution. Tritiated (i.e., ³H) and carbon-14 (i.e., ¹⁴C) isotopes are particularly preferred for their ease of preparation and detectability. Positron emitting isotopes, such as ¹⁵O, ¹³N, ¹¹C, and ¹⁸F, can be used in positron emission tomography (PET) studies to determine substrate occupancy. Isotopically labeled compounds of the present application can generally be prepared by following procedures analogous to those disclosed in the schemes and/or examples below while substituting a non-isotopically labeled reagent with an isotopically labeled reagent.

Furthermore, substitution with heavier isotopes such as deuterium (i.e., ²H) may provide certain therapeutic advantages (e.g., increased *in vivo* half-life or reduced dose) resulting from greater metabolic stability and hence may be preferred in some circumstances in which deuterium substitution may be partial or complete, wherein partial deuterium substitution refers to substitution of at least one hydrogen with at least one deuterium.

The compound of the present application may be asymmetrical, for example, having one or more stereoisomers. Unless otherwise stated, all stereoisomers include, for example, enantiomers and diastereoisomers. The compound of the present application containing asymmetrical carbon atoms may be separated in an optically pure form or in a racemic form. The optically pure form may be separated from a racemic mixture or may be synthesized using a chiral raw material or a chiral reagent.

The pharmaceutical composition of the present application may be prepared by combining the compound of the present application with a suitable pharmaceutically acceptable excipient, and may be formulated, for example, into a solid, semisolid, liquid, or gaseous formulation such as tablet, pill, capsule, powder, granule, ointment, emulsion, suspension, suppository, injection, inhalant, gel, microsphere, and aerosol.

Typical routes of administration of the compound or the pharmaceutically acceptable salt thereof or the pharmaceutical composition thereof of the present application include, but are not limited to, oral, rectal, local, inhalation, parenteral, sublingual, intravaginal, intranasal, intraocular, intraperitoneal, intramuscular, subcutaneous, and intravenous administration.

The pharmaceutical composition of the present application may be manufactured using methods well known in the art, such as conventional mixing, dissolving, granulating, dragee-making, levigating, emulsifying, and lyophilizing. In some embodiments, the pharmaceutical composition is in an oral form. For oral administration, the pharmaceutical composition can be formulated by mixing the active compounds with pharmaceutically acceptable excipients well known in the art. These excipients enable the compounds of the present application to be formulated into tablets, pills, pastilles, dragees, capsules, liquids, gels, slurries, suspensions, and the like for oral administration to a patient.

A solid oral composition can be prepared by conventional mixing, filling, or tableting. For example, it can be obtained by the following method: mixing the active compounds with solid excipients, optionally grinding the resulting mixture, adding additional suitable excipients if desired, and processing the mixture into granules to get the core parts of tablets or dragees. Suitable excipients include, but are not limited to: binders, diluents, disintegrants, lubricants, glidants, sweeteners, flavoring agents, or the like.

The pharmaceutical composition may also be suitable for parenteral administration, such as a sterile solution, a suspension, or a lyophilized product in a suitable unit dosage form.

In all of the administration methods of the compound of general formula I described herein, the daily dose administered is from 0.01 mg/kg to 200 mg/kg of body weight, given in individual or separated doses.

The compounds of the present application can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application.

The chemical reactions in the specific embodiments of the present application are conducted in a proper solvent that must be suitable for the chemical changes in the present application and the reagents and materials required. In order to obtain the compounds of the present application, it is sometimes necessary for those skilled in the art to modify or select a synthesis procedure or a reaction process based on the existing embodiments.

An important consideration in synthetic route planning in the art is the selection of suitable protective groups for reactive functional groups (e.g., amino in the present application). For example, reference may be made to Greene's Protective Groups in Organic Synthesis (4th Ed.) Hoboken, New Jersey: John Wiley & Sons, Inc.

In some embodiments, the compound of formula I of the present application can be prepared by one skilled in the art of organic synthesis through the following routes:

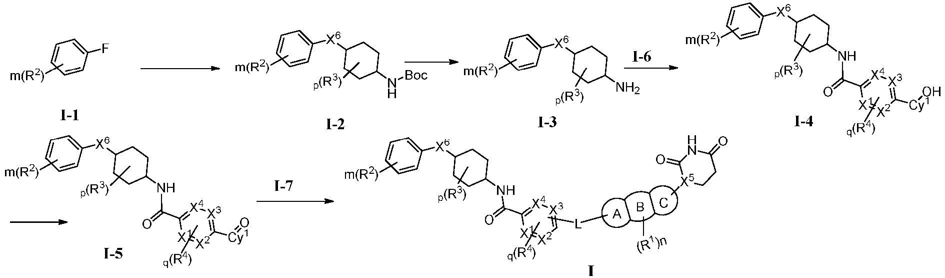

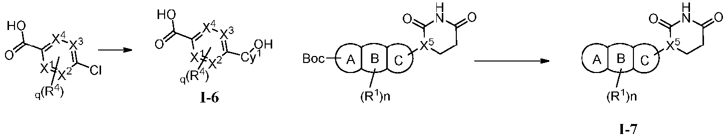

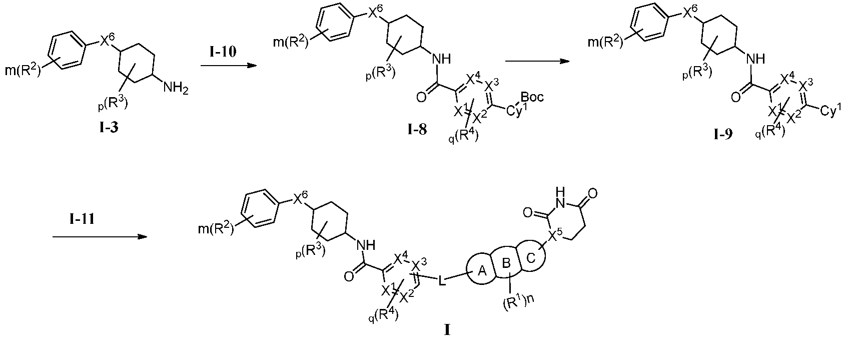

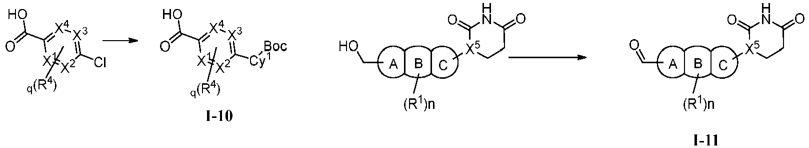

A compound of general formula **I-1** is subjected to a substitution reaction to obtain a compound of general formula **1-2,** the compound of general formula **I-2** is subjected to a deprotection reaction to obtain a compound of general formula **I-3**, then a condensation reaction is carried out to obtain a compound of general formula **I-4**, and the compound of general formula **I-4** is subjected to an oxidation reaction to obtain a compound of general formula **I-5;** the chloro-substituted aromatic ring-containing carboxylic acid is subjected to a substitution reaction with hydroxyl-substituted Cy¹ to obtain a compound of general formula **I-6**; a compound of general formula **I-7** is obtained by a deprotection reaction; finally, the compound of general formula **I-5** and the compound of general formula **I-7** are subjected to a reductive amination reaction to obtain a compound of general formula **I.**

A compound of general formula **I-3** and a compound of general formula **I-10** are subjected to a condensation reaction to obtain a compound of general formula **I-8**, and the compound of general formula **I-8** is subjected to a deprotection reaction to obtain a compound of general formula **I-9**; the chloro-substituted aromatic ring-containing carboxylic acid is subjected to a substitution reaction with Boc-protected Cy¹ to obtain a compound of general formula **I-10**; a compound of general formula **I-11** is obtained by an oxidation reaction; the compound of general formula **I-9** and the compound of general formula **I-11** are subjected to a reductive amination reaction to obtain a compound of the general formula **I.**

The following abbreviations are used in the present application:
Boc represents *tert*-butyloxycarbonyl; Et represents ethyl; EA represents ethyl acetate; DMSO represents dimethyl sulfoxide; DMF represents *N,N*-dimethylformamide; BINAP represents 2,2'-bis(diphenylphosphino)-1,1'-binaphthyl; DCM represents dichloromethane; Pd₂(dba)₃ represents tris(dibenzylideneacetone)dipalladium(0); THF represents tetrahydrofuran; PMB represents *p*-methoxybenzyl; MeOH represents methanol; PE represents petroleum ether; IBX represents 2-iodoxybenzoic acid; DIPEA represents *N,N'*-diisopropylethylamine; DIBAL-H represents diisobutylaluminum hydride; NIS represents *N*-iodosuccinimide; NBS represents *N*-bromosuccinimide; Tf represents -OSO₂CF₃; HATU represents *O*-(7-azabenzotriazol-1-yl)-*N,N,N',N'*-tetramethyluronium hexafluorophosphate; xantphos represents 4,5-bis(diphenylphosphino)-9,9-dimethylxanthene; DCE represents dichloroethane; DMA or DMAC represents *N,N*-dimethylacetamide; AIBN represents azobisisobutyronitrile; DMAP represents 4-dimethylaminopyridine; CCl₄ represents carbon tetrachloride; and Ruphos represents 2-dicyclohexylphosphino-2',6'-diisopropoxybiphenyl.

For clarity, the present application is further described with the following examples, which are, however, not intended to limit the scope of the present application. All reagents used in the present application are commercially available and can be used without further purification.

### DETAILED DESCRIPTION

### Example 1: Synthesis of Intermediate 1

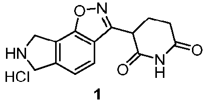

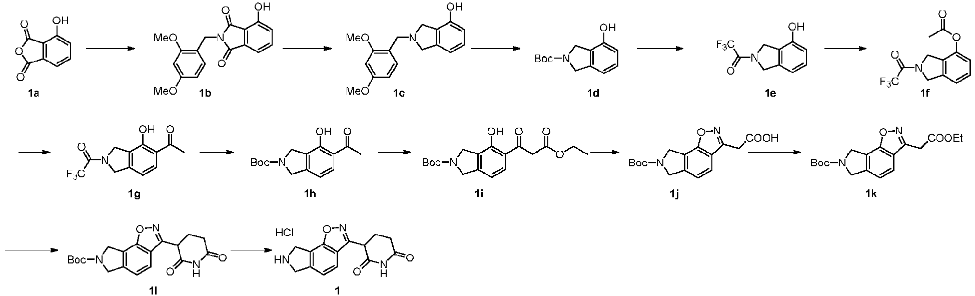

### Step 1: Preparation of intermediate 1b

At 10 °C, **1a** (81 g), 2,4-dimethoxybenzylamine (83 g), and acetic acid (500 mL) were added to a reaction flask in sequence, and the mixture was warmed to 80 °C and reacted. After the reaction was completed as monitored, water (800 mL) was added to the reaction solution, and the mixture was filtered under vacuum. The filter cake was washed with water and dried to give intermediate **1b** (95.78 g).

MS (ESI, [M-H]⁻) *m*/*z*: 312.02*.*

¹H NMR (500 MHz, DMSO-*d*₆) δ 11.03 (s, 1H), 7.62 (dd, *J* = 8.4, 7.1 Hz, 1H), 7.29 (d, *J* = 7.1 Hz, 1H), 7.22 (d, *J* = 8.4 Hz, 1H), 6.90 (d, *J* = 8.4 Hz, 1H), 6.56 (d, *J=* 2.4 Hz, 1H), 6.43 (dd, *J* = 8.4, 2.4 Hz, 1H), 4.60 (s, 2H), 3.80 (s, 3H), 3.73 (s, 3H).

### Step 2: Preparation of intermediate 1c

At 10 °C, a 2.5 M solution of lithium aluminum hydride in tetrahydrofuran (227 mL) was slowly added dropwise to a solution of **1b** (96.00 g) in tetrahydrofuran (1000 mL), and the mixture was reacted at 80 °C. A 15% sodium hydroxide aqueous solution (30.5 g) and water (100 mL) were added to the reaction solution, and the mixture was filtered under vacuum. The filter cake was washed with a solution of dichloromethane/MeOH = 1/1, and the filtrate was concentrated. The crude product was separated and purified by silica gel column chromatography to give intermediate **1c** (55.87 g).

MS (ESI, [M+H]⁺) *m*/*z*: 286.01
¹H NMR (500 MHz, DMSO-*d*₆) δ 9.28 (s, 1H), 7.24 (d, *J* = 8.3 Hz, 1H), 6.97 (t, *J* = 7.7 Hz, 1H), 6.64 (d, *J* = 7.4 Hz, 1H), 6.59 (d, *J=* 8.0 Hz, 1H), 6.55 (d, *J=* 2.4 Hz, 1H), 6.51 (dd, *J* = 8.3, 2.4 Hz, 1H), 3.81 - 3.71 (m, 12H).

### Step 3: Preparation of intermediate 1d

**1c** (48.00 g), methanol (350 mL), palladium hydroxide (4.8 g), and di-tert-butyl dicarbonate (41.4 g) were added to a reaction flask in sequence, and the mixture was reacted at 25 °C under hydrogen atmosphere. The reaction solution was filtered under vacuum. The filtrate was concentrated, extracted with water (200 mL) and ethyl acetate, washed with saturated brine, dried over anhydrous sodium sulfate, and filtered. The filtrate was concentrated, and the crude product was separated and purified by silica gel column chromatography to give intermediate **1d** (34.43 g).

¹H NMR (500 MHz, DMSO-*d*₆) δ 7.07 (t, *J =* 7.7 Hz, 1H), 6.68 (ddd, *J =* 18.9, 7.8, 2.7 Hz, 2H), 4.56 - 4.50 (m, 2H), 4.48 - 4.42 (m, 2H), 1.45 (s, 9H).

### Step 4: Preparation of intermediate 1e

**1d** (35.00 g), methanol (250 mL), and a 4 M solution of hydrochloric acid in 1,4-dioxane (123 mL) were added to a reaction flask in sequence, and the mixture was reacted at 25 °C for 3 h. The reaction solution was concentrated, pyridine (200 mL) and trifluoroacetic anhydride (25.20 g) were added, and the mixture was stirred at 25 °C for 40 h. The reaction solution was poured into a 3 M hydrochloric acid aqueous solution, and the mixture was vigorously stirred, extracted with ethyl acetate, washed with saturated brine, dried over anhydrous sodium sulfate, and filtered. The filtrate was concentrated to give intermediate **1e** (32.48 g).

MS (ESI, [M-H]⁻) *m*/*z:* 229.99.

¹H NMR (500 MHz, DMSO-*d*₆)δ 9.82 (d, *J* = 24.4 Hz, 1H), 7.16 (td, *J* = 7.7, 3.7 Hz, 1H), 6.81 (dd, *J* = 11.5, 7.5 Hz, 1H), 6.74 (d, *J* = 8.0 Hz, 1H), 4.99 (s, 1H), 4.89 (s, 1H), 4.80 (s, 1H), 4.70 (s, 1H).

### Step 5: Preparation of intermediate 1f

**1e** (32.48 g), dichloromethane (300 mL), triethylamine (28.40 g), 4-dimethylaminopyridine (1.72 g), and acetic anhydride (15.78 g) were added to a reaction flask in sequence, and the mixture was reacted at 25 °C. After the reaction was completed, the reaction solution was poured into water, and the mixture was vigorously stirred, extracted with dichloromethane, washed with saturated brine, dried over anhydrous sodium sulfate, and filtered. The filtrate was concentrated, and the crude product was separated by silica gel column chromatography to give intermediate **1f** (33.82 g).

¹H NMR (500 MHz, DMSO-*d*₆)δ 7.41 (td, *J* = 7.8, 2.3 Hz, 1H), 7.30 (t, *J* = 8.5 Hz, 1H), 7.12 (dd, *J* = 7.9, 2.9 Hz, 1H), 5.09 (s, 1H), 4.90 (d, *J* = 12.3 Hz, 2H), 4.72 (s, 1H), 2.31 (d, *J* = 3.4 Hz, 3H).

### Step 6: Preparation of intermediate 1g

**1f** (30.00 g), aluminum trichloride (29.30 g), and orthodichlorobenzene (200 mL) were added to a reaction flask in sequence, and the mixture was reacted at 150 °C for 1 h. The reaction solution was then poured into an citric acid aqueous solution, and the mixture was vigorously stirred, extracted with ethyl acetate, washed with saturated brine, dried over anhydrous sodium sulfate, and filtered. The filtrate was concentrated, and the crude product was separated by silica gel column chromatography to give intermediate **1g** (18.19 g).

¹H NMR (500 MHz, DMSO-*d*₆) δ 12.38 (s, 1H), 7.94 (dd, *J* = 8.2, 4.7 Hz, 1H), 7.01 (dd, *J* = 12.7, 8.1 Hz, 1H), 5.07 (s, 1H), 4.94 (s, 1H), 4.86 (s, 1H), 4.74 *(d, J* = 1.6 Hz, 1H), 2.66 (d, *J* = 1.0 Hz, 3H).

### Step 7: Preparation of intermediate 1h

**1g** (18.19 g), sodium hydroxide (7.56 g), methanol (150 mL), and water (150 mL) were added to a reaction flask in sequence, and the mixture was reacted at 25 °C. The reaction solution was concentrated to remove the methanol, 1,4-dioxane (150 mL) and di-*tert*-butyl dicarbonate (15.14 g) were added to the residue, and the mixture was stirred at 25 °C. After the reaction was completed, the reaction solution was poured into water, and the mixture was vigorously stirred, extracted with ethyl acetate, washed with saturated brine, dried over anhydrous sodium sulfate, and filtered. The filtrate was concentrated, and the crude product was separated by silica gel column chromatography to give intermediate **1h** (14.10 g).

MS (ESI, [M-H]⁻) *m*/*z:* 276.07.

¹H NMR (500 MHz, DMSO-*d*₆) δ 12.36 (s, 1H), 7.89 (dd, J = 8.1, 2.9 Hz, 1H), 6.95 (t, J = 8.0 Hz, 1H), 4.62 (dt, J = 13.4, 2.2 Hz, 2H), 4.51 (dt, J = 13.9, 2.3 Hz, 2H), 2.65 (s, 3H), 1.46 (s, 9H).

### Step 8: Preparation of intermediate 1i

**1h** (14.10 g), diethyl carbonate (27.00 g), and toluene (200 mL) were added to a reaction flask in sequence, and the mixture was cooled to 0 °C. After 60 wt% sodium hydride (9.16 g) was added, the mixture was warmed to 120 °C and stirred. After the reaction was completed, the reaction solution was poured into a 3 M hydrochloric acid aqueous solution, and the mixture was vigorously stirred, extracted with ethyl acetate, washed with saturated brine, dried over anhydrous sodium sulfate, and filtered. The filtrate was concentrated, and the crude product was separated by silica gel column chromatography to give intermediate **1i** (14.96 g).

¹H NMR (500 MHz, DMSO-*d*₆) δ 11.72 (d, *J* = 9.6 Hz, 1H), 7.82 (dd, *J* = 8.2, 4.7 Hz, 1H), 6.97 (t, *J* = 8.4 Hz, 1H), 4.63 (dt, *J=* 12.4, 2.1 Hz, 2H), 4.53 (dt, *J* = 13.7, 2.1 Hz, 2H), 4.22 (d, *J* = 2.5 Hz, 2H), 4.13 (q, *J* = 7.1 Hz, 2H), 1.46 *(d, J=* 1.9 Hz, 9H), 1.19 (t, *J* = 7.1 Hz, 3H).

### Step 9: Preparation of intermediate 1j

**1i** (14.96 g), a 50% hydroxylamine aqueous solution (6.36 g), and ethanol (150 mL) were added to a reaction flask in sequence, and the mixture was stirred at 85 °C for 3 h. The reaction solution was concentrated and extracted with water and ethyl acetate, followed by liquid separation, and the organic phase was washed with saturated brine, dried over anhydrous sodium sulfate, and filtered. The filtrate was concentrated to give a crude product of intermediate **1j** (10.13 g).

MS (ESI, [M-H]⁻) *m*/*z*: 317.20.

¹H NMR (500 MHz, DMSO-*d*₆) δ 12.59 (s, 1H), 7.78 (d, *J* = 8.2 Hz, 1H), 7.38 (t, *J* = 7.6 Hz, 1H), 4.85 (dt, *J* = 13.5, 2.5 Hz, 2H), 4.75 (dt, *J* = 12.2, 2.4 Hz, 2H), 4.11 (s, 2H), 1.49 (d, *J* = 2.1 Hz, 9H).

### Step 10: Preparation of intermediate 1k

**1j** (10.13 g), potassium carbonate (12.55 g), *N,N*-dimethylacetamide (150 mL), and iodoethane (7.08 g) were added to a reaction flask in sequence, and the mixture was stirred at 80 °C for 1 h. The reaction solution was poured into water, and the mixture was vigorously stirred, extracted with ethyl acetate, washed with saturated brine, dried over anhydrous sodium sulfate, and filtered. The filtrate was concentrated, and the residue was separated by silica gel column chromatography to give intermediate **1k** (11.22 g).

MS (ESI, [M+H]⁺) *m*/*z:* 347.16.

¹H NMR (500 MHz, DMSO-*d*₆)δ 7.78 (d, *J* = 8.0 Hz, 1H), 7.39 (t, *J* = 7.5 Hz, 1H), 4.89 - 4.82 (m, 2H), 4.75 (dd, *J=* 11.8, 2.8 Hz, 2H), 4.22 (d, *J=* 4.3 Hz, 2H), 4.13 (p, *J* = 7.2 Hz, 2H), 1.48 (d, *J=* 2.1 Hz, 9H), 1.21 - 1.17 (m, 3H).

### Step 11: Preparation of intermediate 1l

Acrylamide (1.32 g) was slowly added to a stirred solution of **1k** (10.72 g) in tetrahydrofuran (50 mL) at 0 °C under N₂ atmosphere, a 1 M solution of potassium *tert*-butoxide in tetrahydrofuran (18.63 mL) was then added dropwise, and the mixture was stirred at 0 °C for 3 h. The reaction solution was poured into an ammonium chloride aqueous solution, and the mixture was vigorously stirred, extracted with ethyl acetate, washed with saturated brine, dried over anhydrous sodium sulfate, and filtered. The filtrate was concentrated, and the crude product was separated by silica gel column chromatography to give intermediate **1l** (7.52 g).

MS (ESI, [M+H]⁺) *m*/*z:* 372.02.

¹H NMR (500 MHz, DMSO-*d*₆) δ 11.11 (s, 1H), 7.80 (dd, *J* = 8.1, 2.7 Hz, 1H), 7.37 (t, *J* = 7.9 Hz, 1H), 4.85 (dd, J = 12.2, 2.6 Hz, 2H), 4.75 (dt, *J* = 12.0, 2.3 Hz, 2H), 4.63 (dd, *J=* 12.1, 4.9 Hz, 1H), 2.78 (ddd, *J* = 17.3, 12.1, 5.3 Hz, 1H), 2.67 - 2.51 (m, 2H), 2.20 (dq, *J* = 13.5, 4.7 Hz, 1H), 1.49 (d, *J* = 2.6 Hz, 9H).

### Step 12: Preparation of intermediate 1

**1l** (1.00 g), a 4 M solution of hydrochloric acid in 1,4-dioxane (10 mL), and ethyl acetate (50 mL) were added to a reaction flask in sequence, and the mixture was reacted at 25 °C for 8 h. The reaction solution was then directly filtered, and the filter cake was washed with ethyl acetate and then dried to give intermediate 1 (0.83 g).

MS (ESI, [M+H]⁺) *m*/*z*: 272.11.

¹H NMR (500 MHz, DMSO-*d*₆) δ 11.13 (s, 1H), 10.36 (s, 2H), 7.90 (d, *J* = 8.1 Hz, 1H), 7.45 (d, *J* = 8.2 Hz, 1H), 4.81 (s, 2H), 4.70 - 4.63 (m, 3H), 2.79 (ddd, *J* = 17.4, 12.2, 5.3 Hz, 1H), 2.62 (dt, *J* = 17.3, 4.0 Hz, 1H), 2.59 - 2.52 (m, 1H), 2.21 (ddt, *J* = 13.2, 5.1, 2.5 Hz, 1H).

### Example 2: Preparation of Intermediate 2

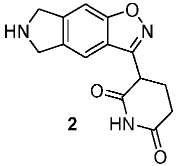

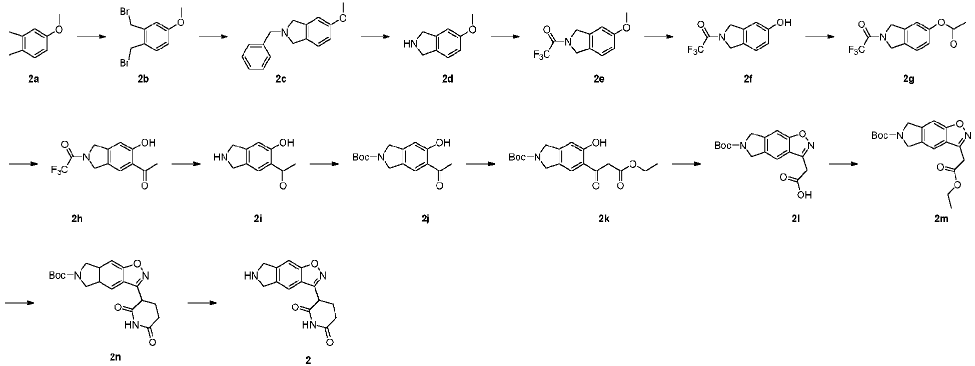

### Step 1: Preparation of intermediate 2b

**2a** (18 g), AIBN (0.738 g), carbon tetrachloride (500 mL), and NBS (47.8 g) were added to a reaction flask in sequence, and the mixture was warmed to 60 °C and reacted. The reaction solution was then cooled to room temperature and concentrated by evaporation under reduced pressure to remove the solvent, and dichloromethane (200 mL) was added to the residue. The mixture was washed with saturated brine, dried over anhydrous sodium sulfate, and filtered. The filtrate was concentrated by evaporation under reduced pressure to remove the solvent, and the residue was purified by silica gel column chromatography to give intermediate **2b** (18.7 g).

### Step 2: Preparation of intermediate 2c

**2b** (18.7 g), benzylamine (1.78 mL), *N,N*-diisopropylethylamine (7.13 mL), and toluene (50 mL) were added to a reaction flask in sequence, and the mixture was warmed to 50 °C and reacted. The reaction solution was then cooled to room temperature. After the reaction was completed, the reaction solution was extracted with ethyl acetate (200 mL) and an icy HCl aqueous solution (1 M, 200 mL). The aqueous phase was collected, adjusted to about pH 8 with a sodium bicarbonate solid, and extracted with ethyl acetate. The organic phase was collected, dried over anhydrous sodium sulfate, filtered, and concentrated by evaporation under reduced pressure to remove the solvent, thus giving intermediate **2c** (9.6 g).

MS(ESI, [M+H]⁺) *m*/*z*: 240.1.

### Step 3: Preparation of intermediate 2d

**2c** (9.6 g), methanol (200 mL), 10% palladium on carbon (5 g), and toluene (50 mL) were added to a reaction flask in sequence, and the mixture was purged three times with hydrogen and reacted at room temperature under hydrogen atmosphere. The palladium on carbon was filtered out, and the filter cake was washed with methanol. The filtrate was collected and concentrated by evaporation under reduced pressure to remove the solvent, thus giving **2d** (4.5 g).

MS(ESI, [M+H]⁺) *m*/*z*: 149.9.

### Step 4: Preparation of intermediate 2e

**2d** (4 g), tetrahydrofuran (50 mL), and trifluoroacetic anhydride (5.63 g) were added to a reaction flask in sequence, and the mixture was reacted at room temperature. An aqueous solution (200 mL) was added to the reaction solution to quench the reaction, and ethyl acetate was then added for extraction. The organic phase was separated, dried over anhydrous sodium sulfate, and purified by silica gel column chromatography to give **2e** (4.58 g).

¹H NMR (500 MHz, DMSO-d6) δ 7.29 (t, *J* = 9.3 Hz, 1H), 6.98 (d, *J* = 8.6 Hz, 1H), 6.93 - 6.86 (m, 1H), 4.97 (d,*J* = 21.7 Hz, 2H), 4.77 (dd, *J* = 21.4, 5.2 Hz, 2H), 3.76 (dd, *J* = 3.6, 1.6 Hz, 3H).

### Step 5: Preparation of intermediate 2f

**2e** (4.6 g), dichloromethane (200 mL), and boron tribromide (1 M, 18.76 mL) were added to a reaction flask in sequence, and the mixture was reacted at room temperature. An aqueous solution (200 mL) was added to the reaction solution under an ice bath to quench the reaction. The organic phase was separated, and the aqueous phase was extracted with dichloromethane. The organic phases were combined, dried over anhydrous sodium sulfate, and concentrated by evaporation under reduced pressure to remove the solvent, thus giving **2f** (4.2 g).

¹H NMR (500 MHz, DMSO-d6) δ 7.16 (t, *J* = 8.8 Hz, 1H), 6.87 - 6.59 (m, 2H), 4.92 (d, *J* = 20.8 Hz, 2H), 4.72 (d, *J* = 19.9 Hz, 2H).

### Step 6: Preparation of intermediate 2g

**2f** (6.5 g), dichloromethane (60 mL), triethylamine (7.8 mL), and acetic anhydride (2.94 mL) were added to a reaction flask in sequence, and the mixture was reacted at room temperature. The organic solvent dichloromethane (200 mL) and water (300 mL) were added to the reaction solution. The organic phase was separated, washed with saturated brine, dried over anhydrous sodium sulfate, filtered, and concentrated by evaporation under reduced pressure to remove the solvent, thus giving **2g** (7 g).

¹H NMR (500 MHz, DMSO-d6) δ 7.42 (dd, *J* = 10.0, 8.3 Hz, 1H), 7.17 (dd, *J* = 14.0, 2.1 Hz, 1H), 7.09 (d, *J* = 8.2 Hz, 1H), 5.03 (d, *J* = 6.6 Hz, 2H), 4.83 (d, *J* = 6.9 Hz, 2H), 2.27 (s, 3H).

### Step 7: Preparation of intermediate 2h

**2g** (6 g) and aluminum trichloride (4.39 g) were added to a reaction flask in sequence, and the mixture was gradually heated from room temperature to 150 °C and reacted. The reaction solution was then cooled to room temperature, and water (500 mL) and a 3 M hydrochloric acid aqueous solution (100 mL) were added to the residue. The organic phase was separated, washed with saturated brine (100 mL), dried over anhydrous sodium sulfate, and filtered. The filtrate was concentrated under reduced pressure to remove the solvent, and the residue was purified by silica gel column chromatography to give **2h** (3.78 g).

MS(ESI, [M-H]⁻) *m*/*z*: 271.9*.*

### Step 7: Preparation of intermediate 2j

**2h** (550 mg), MeOH (5.00 mL), and a solution of sodium hydroxide (242 mg) in water (5.00 mL) were added to a reaction flask in sequence, and the mixture was reacted at room temperature. The reaction solution was concentrated to remove the methanol, and the aqueous phase was retained, thus giving **2i.** 1,4-Dioxane (5 mL) and Boc anhydride (439 mg, 0.462 mL) were added to the system, and the mixture was reacted at room temperature. The reaction solution was extracted with ethyl acetate (200 mL) and saturated brine (200 mL), and the organic phase was dried over anhydrous sodium sulfate, filtered, and concentrated to give **2j** (200 mg).

¹H NMR (500 MHz, DMSO-d6) δ 12.11 (d, *J* = 19.3 Hz, 1H), 7.88 (d, *J* = 9.5 Hz, 1H), 6.74 - 6.56 (m, 1H), 4.60 - 4.50 (m, 4H), 2.63 (d, *J* = 6.5 Hz, 3H), 1.45 (s, 9H).

### Step 8: Preparation of intermediate 2k

**2j** (3.5 g) and THF (300 mL) were added to a reaction flask in sequence, and diethyl carbonate (14.91 g, 15.29 mL) was added. The mixture was cooled to about 0 °C, and 60 wt% sodium hydride (5.05 g, 126 mmol) was added in portions. The mixture was heated to 85 °C and reacted. The reaction solution was cooled to room temperature and slowly poured into ice water (500 mL). The mixture was extracted with ethyl acetate, and the organic phase was discarded. The aqueous phase was adjusted to pH = 1-2 with 3 M hydrochloric acid, then extracted with ethyl acetate, dried over anhydrous sodium sulfate, filtered, and concentrated by evaporation under reduced pressure to remove the solvent, thus giving **2k** (7.0 g).

MS(ESI, [M-H]⁻) *m*/*z*: 348.3*.*

### Step 9: Preparation of intermediate 2l

**2k** (4.4 g), an hydroxylamine aqueous solution (4.16 g, 63.0 mmol), and ethanol (50 mL) were added to a reaction flask in sequence, and the mixture was reacted at 85 °C. The reaction solution was then cooled to room temperature, ethyl acetate (200 mL) and a saturated sodium carbonate aqueous solution (100 mL) were added to the residue for extraction, and the organic phase was discarded. The aqueous phase was adjusted to pH = 2-3 with a 1 M HCl aqueous solution, extracted with ethyl acetate, dried over anhydrous sodium sulfate, filtered, and concentrated by evaporation under reduced pressure to remove the solvent, thus giving **2l** (3.25 g).

¹H NMR (500 MHz, DMSO-*d*₆) δ 12.83 (s, 1H), 7.74 (d, *J* = 13.0 Hz, 1H), 7.68 (d, *J* = 3.5 Hz, 1H), 4.71 (d, *J* = 13.6 Hz, 2H), 4.66 (d, *J* = 11.4 Hz, 2H), 4.07 (s, 2H), 1.47 (s, 9H).

### Step 10: Preparation of intermediate 2m

**2l** (3.14 g), potassium carbonate (1.500 g), DMA (5 mL), and iodoethane (2.308 g, 1.183 mL) were added to a reaction flask in sequence, and the mixture was heated to 80 °C and reacted. The reaction solution was then cooled to room temperature and poured into a mixed solution of ethyl acetate (100 mL) and water (200 mL). The organic phase was separated, dried over anhydrous sodium sulfate, and filtered. The filtrate was concentrated by evaporation under reduced pressure to remove the solvent, thus giving **2m** (2.47 g).

¹H NMR (500 MHz, DMSO-*d*₆) δ 7.73 (d, *J* = 15.9 Hz, 1H), 7.69 (d, *J* = 3.5 Hz, 1H), 4.69 (dd, *J* = 23.8, 12.4 Hz, 4H), 4.19 - 4.11 (m, 4H), 1.47 (s, 9H), 1.20 (t, *J* = 7.1 Hz, 3H).

### Step 11: Preparation of intermediate 2n

**2m** (1.5 g) and THF (75 mL) were added to a reaction flask in sequence, acrylamide (0.215 g) was added, and the mixture was cooled to about -15 °C. A 1 M solution of potassium tert-butoxide in tetrahydrofuran (2.60 mL) was added, and the system was warmed to 0 °C and reacted for 1.5 h. The mixture was added dropwise to an ammonium chloride solution (200 mL) to quench the reaction and extracted with ethyl acetate. The organic phase was separated, dried over anhydrous sodium sulfate, and filtered, and the filtrate was concentrated and then purified by silica gel column chromatography to give **2n** (0.88 g).

¹H NMR (500 MHz, DMSO-*d*₆) δ 11.11 (d, *J* = 3.4 Hz, 1H), 7.78 (d, *J* = 12.4 Hz, 1H), 7.70 (s, 1H), 4.68 (dd, *J* = 29.4, 13.3 Hz, 4H), 4.58 (dd, *J* = 12.0, 4.9 Hz, 1H), 2.79 (ddd, *J* = 17.3, 12.1, 5.3 Hz, 1H), 2.62 (dt, *J* = 17.3, 4.1 Hz, 1H), 2.56 - 2.50 (m, 1H), 2.31 - 2.14 (m, 1H), 1.47 (d, *J* = 1.5 Hz, 9H).

### Step 12: Preparation of intermediate 2

**2n** (0.428 g) and dichloromethane (10.00 mL) were added to a reaction flask in sequence, then trifluoroacetic acid (3.29 g, 2.211 mL) was added, and the mixture was reacted at room temperature for 1 h. Water (80 mL) was added to the reaction solution, and the mixture was adjusted to pH 7-8 with saturated sodium bicarbonate and extracted with dichloromethane. The organic phases were combined, dried over anhydrous sodium sulfate, and concentrated by rotary evaporation to remove the solvent, thus giving intermediate 2 (0.439 g).

MS(ESI, [M+H]⁺) *m*/*z*: 272.24*.*

¹H NMR (500 MHz, DMSO-d6) δ 7.81 - 7.77 (m, 1H), 7.74 (s, 1H), 4.59 (dd, *J* = 12.1, 5.0 Hz, 1H), 4.49 (s, 2H), 4.43 (s, 2H), 2.79 (ddd, *J* = 17.4, 12.2, 5.3 Hz, 1H), 2.62 (dt, *J* = 17.3, 4.0 Hz, 1H), 2.47 (dd, *J* = 12.4, 4.4 Hz, 1H), 2.20 (ddt, *J* = 13.3, 5.2, 2.6 Hz, 1H).

### Example 3: Synthesis of Intermediate 3

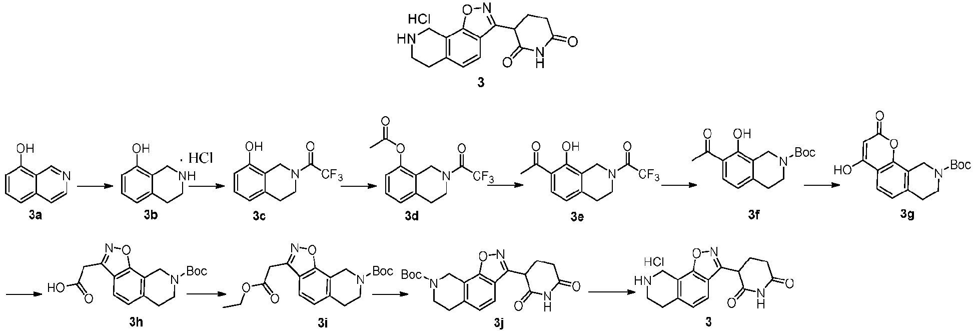

### Step 1: Preparation of intermediate 3b

Intermediate **3a** (25 g) was completely dissolved in methanol (1000 mL) and acetic acid (103 g, 99 mL, 1722 mmol) and then injected into a flow hydrogenation reactor at a pressure of 3 MPa, a temperature of 110 °C, and a flow rate of 3 mL/min. After the reaction was completed, the reaction solution was concentrated by evaporation under reduced pressure to remove the solvent, and a solution of hydrochloric acid in 1,4-dioxane (4 mol/L, 100 mL) was added to the residue. The mixture was concentrated by evaporation under reduced pressure to remove the solvent, and the residue was slurried with ethyl acetate and filtered. The filter cake was collected to give the target intermediate **3b** (28.97 g).

MS(ESI, [M+H]⁺) m/z: 150.0.

¹H NMR (500 MHz, DMSO-d6) δ 10.01 (s, 1H), 7.06 (t, J = 7.8 Hz, 1H), 6.76 (d, J = 8.0 Hz, 1H), 6.64 (d, J = 7.6 Hz, 1H), 4.01 (t, J = 4.9 Hz, 2H), 3.33 - 3.25 (m, 2H), 2.94 (t, J = 6.2 Hz, 2H).

### Step 2: Preparation of intermediate 3c

Intermediate **3b** (28.97 g) and tetrahydrofuran (300 mL) were added to a reaction flask in sequence, and trifluoroacetic anhydride (27.0 mL) was added under an ice bath. The mixture was reacted at room temperature. After the reaction was completed, the reaction solution was extracted with ethyl acetate (500 mL) and water (1000 mL). The organic phase was separated, and the aqueous phase was extracted with ethyl acetate. The organic phases were combined, washed with saturated brine, dried over anhydrous sodium sulfate, filtered, and concentrated by evaporation under reduced pressure to remove the solvent. The filter cake was collected by filtration to give the target intermediate **3c** (22.67 g).

MS(ESI, [M-H]⁻) *m*/*z:* 244.0.

¹H NMR (500 MHz, DMSO-d6) δ 7.03 (q, J = 7.9 Hz, 1H), 6.74 - 6.68 (m, 1H), 6.64 (t, J = 6.9 Hz, 1H), 4.61 (d, J = 23.5 Hz, 2H), 3.78 (td, J = 6.0, 3.7 Hz, 2H), 2.84 (dt, J = 16.8, 5.9 Hz, 2H).

### Step 3: Preparation of intermediate 3d

Intermediate **3c** (22.67 g), dichloromethane (200 mL), triethylamine (28.1 g, 38.6 mL), and DMAP (0.282 g) were added to a reaction flask in sequence, and acetic anhydride (10.38 g, 9.68 mL) was added under an ice bath. The mixture was warmed to room temperature and reacted. After the reaction was completed, the reaction solution was concentrated by evaporation under reduced pressure to remove the solvent, and ethyl acetate (500 mL) and water (1000 mL) were added to the residue for extraction. The organic phase was separated, washed with a saturated ammonium chloride solution and saturated brine separately, dried over anhydrous sodium sulfate, and filtered, and the filtrate was concentrated by evaporation under reduced pressure to remove the solvent, thus giving the target intermediate **3d** (21.94 g).

¹H NMR (500 MHz, DMSO-d6) δ 7.30 (dt, J = 11.1, 7.8 Hz, 1H), 7.18 - 7.11 (m, 1H), 7.05 (dt, J = 8.0, 2.2 Hz, 1H), 4.59 (s, 2H), 3.81 (q, J = 6.1 Hz, 2H), 2.95 (dt, J = 10.1, 6.0 Hz, 2H), 2.33 (d, J = 9.5 Hz, 3H).

### Step 4: Preparation of intermediate 3e

Intermediate **3d** (21 g) and aluminum trichloride (14.62 g) were added to a reaction flask in sequence, and the mixture was heated to 170 °C and reacted under N₂ atmosphere. After the reaction was completed, the reaction solution was cooled to room temperature, water (300 mL) was added to quench the reaction, and then dichloromethane was added for extraction. The organic phases were combined, washed with saturated brine (500 mL), dried over anhydrous sodium sulfate, and filtered, and the filtrate was purified by a silica gel column to give the target intermediate **3e** (10.16 g).

MS(ESI, [M-H]⁻) *m*/*z*: 286.0*.*

¹H NMR (500 MHz, DMSO-d6) δ 12.76 (d, J = 8.4 Hz, 1H), 7.83 (t, J = 8.8 Hz, 1H), 6.86 (dd, J = 8.3, 5.7 Hz, 1H), 4.67 (d, J = 25.1 Hz, 2H), 3.86 - 3.78 (m, 2H), 2.94 (dt, J = 13.3, 5.9 Hz, 2H), 2.64 (d, J = 1.2 Hz, 3H).

### Step 5: Preparation of intermediate 3f

Intermediate **3e** (10.16 g) and methanol (100 mL) were added to a reaction flask in sequence, and a solution of sodium hydroxide (4.24 g) in water (100 mL) was added dropwise under an ice bath. The mixture was warmed to room temperature and reacted. After the reaction was completed, the reaction solution was concentrated by evaporation under reduced pressure to remove the methanol, and 1,4-dioxane (100 mL) and di-*tert*-butyl dicarbonate (8.49 g, 9.03 mL) were added. The mixture was reacted at room temperature. After the reaction was completed, the reaction solution was extracted with ethyl acetate (500 mL) and water (800 mL), dried over anhydrous sodium sulfate, and filtered, and the filtrate was concentrated by evaporation under reduced pressure to remove the solvent, thus giving the target intermediate **3f** (8.96 g).

MS(ESI, [M+H]⁺) m/z: 292.0.

¹H NMR (500 MHz, DMSO-d6) δ 12.72 (s, 1H), 7.76 (d, J = 8.2 Hz, 1H), 6.80 (d, J = 8.2 Hz, 1H), 4.41 (s, 2H), 3.55 (t, J = 5.8 Hz, 2H), 2.80 (t, J = 5.8 Hz, 2H), 2.63 (s, 3H), 1.43 (s, 9H).

### Step 6: Preparation of intermediate 3g

Intermediate **3f** (8.76 g), diethyl carbonate (17.76 g, 18.21 mL), and toluene (90 mL) were added to a reaction flask in sequence, and 60 wt% sodium hydride (6.01 g) was added in portions under an ice bath. The mixture was heated to 120 °C and reacted. After the reaction was completed, the reaction solution was cooled to room temperature and poured into ice water to quench the reaction. A 1 M hydrochloric acid solution was added to adjust the pH to 1-2, and ethyl acetate (300 mL) was added for extraction. The organic phase was separated, dried over anhydrous sodium sulfate, and filtered, and the filtrate was concentrated by evaporation under reduced pressure to remove the solvent, thus giving the target intermediate **3g** (12.03 g).

### Step 7: Preparation of intermediate 3h

Intermediate **3g** (9.54 g), EtOH (100 mL), and an hydroxylamine aqueous solution (9.93 g, 9.21 mL) were added to a reaction flask in sequence, and the mixture was heated to 85 °C and reacted. After the reaction was completed, the reaction solution was adjusted to pH 9-10 with a saturated sodium carbonate solution (200 mL) and extracted with ethyl acetate. The organic phase was separated and extracted twice with water. The aqueous phases were then combined, adjusted to pH 3 with 1 M hydrochloric acid, extracted with ethyl acetate (200 mL), dried over anhydrous sodium sulfate, and filtered, and the filtrate was concentrated by evaporation under reduced pressure to remove the solvent, thus giving the target intermediate **3h** (9.01 g).

MS(ESI, [M-H]⁻) *m*/*z*: 331.0*.*

### Step 8: Preparation of intermediate 3i

Intermediate **3h** (9.01 g), potassium carbonate (11.24 g), DMA (90 mL), and iodoethane (5.07 g, 2.63 mL) were added to a reaction flask in sequence, and the mixture was heated to 80 °C and reacted under N₂ atmosphere. After the reaction was completed, the reaction solution was cooled to room temperature and extracted with ethyl acetate (200 mL) and water (1000 mL). The organic phase was separated, dried over anhydrous sodium sulfate, and filtered, and the filtrate was purified by a silica gel column to give the target intermediate **3i** (7.33 g).

MS(ESI, [M+H]⁺) m/z: 361.0.

¹H NMR (500 MHz, DMSO-d6) δ 7.63 (d, J = 8.1 Hz, 1H), 7.21 (d, J = 8.2 Hz, 1H), 4.76 (s, 2H), 4.18 (s, 2H), 4.13 (q, J = 7.1 Hz, 2H), 3.66 (t, J = 5.8 Hz, 2H), 2.93 (t, J = 5.8 Hz, 2H), 1.45 (s, 9H), 1.19 (t, J = 7.1 Hz, 3H).

### Step 9: Preparation of intermediate 3j

Intermediate **3i** (7.3 g), tetrahydrofuran (80 mL), and acrylamide (0.864 g) were added to a reaction flask in sequence, the mixture was cooled to -15 °C under N₂ atmosphere, and then a solution of potassium tert-butoxide in tetrahydrofuran (1 mol/L, 11.14 mL) was added dropwise. After the dropwise addition, the mixture was warmed to 0 °C and reacted. After the reaction was completed, the resulting reaction system was added to a saturated ammonium chloride solution (200 mL), and ethyl acetate was added for extraction. The organic phase was separated, dried over anhydrous sodium sulfate, and filtered, and the filtrate was purified by a silica gel column to give the target intermediate **3j** (4.54 g).

MS(ESI, [M-H]⁻) *m*/*z*: 384.3*.*

¹H NMR (500 MHz, DMSO-d6) δ 11.09 (s, 1H), 7.65 (d, J = 8.1 Hz, 1H), 7.20 (d, J = 8.2 Hz, 1H), 4.76 (s, 2H), 4.58 (dd, J = 12.0, 5.0 Hz, 1H), 3.66 (t, J = 5.8 Hz, 2H), 2.93 (t, J = 5.8 Hz, 2H), 2.77 (ddd, J = 17.3, 12.1, 5.3 Hz, 1H), 2.61 (dt, J = 17.3, 4.1 Hz, 1H), 2.54 (d, J = 4.5 Hz, 1H), 2.18 (dtd, J = 13.5, 5.2, 3.6 Hz, 1H), 1.45 (s, 9H).

### Step 10: Preparation of intermediate 3

Intermediate **3j** (300 mg) and ethyl acetate (5 mL) were added to a reaction flask in sequence, a solution of hydrochloric acid in 1,4-dioxane (4 mol/L, 3.89 mL) was added, and the mixture was reacted at room temperature. After the reaction was completed, the reaction solution was concentrated to give intermediate 3 (235 mg). MS(ESI, [M+H]⁺) m/z: 286.10.

¹H NMR (500 MHz, DMSO-d6) δ 11.08 (s, 1H), 7.55 (d, J = 8.1 Hz, 1H), 7.10 (d, J = 8.2 Hz, 1H), 4.55 (dd, J = 11.9, 5.0 Hz, 1H), 4.07 (s, 2H), 3.00 (t, J = 5.7 Hz, 2H), 2.81 (t, J = 5.7 Hz, 2H), 2.78 - 2.71 (m, 1H), 2.60 (dt, J = 17.3, 4.2 Hz, 1H), 2.46 (dd, J = 12.2, 4.5 Hz, 1H), 2.18 (dq, J = 13.6, 4.9 Hz, 1H).

### Example 4: Synthesis of Intermediate 4

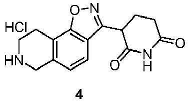

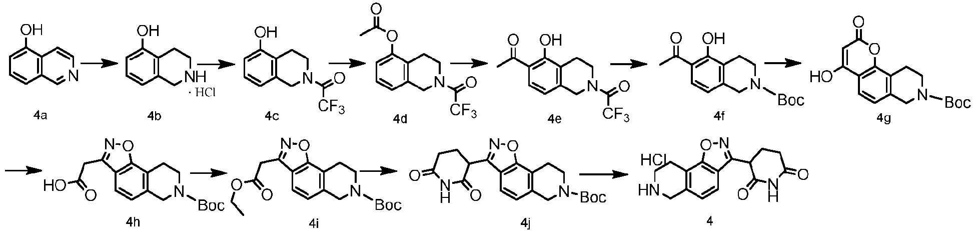

### Step 1: Preparation of intermediate 4b

Intermediate **4a** was completely dissolved in methanol (1680 mL) and acetic acid (166 mL) and then injected into a flow hydrogenation reactor at a pressure of 3 MPa, a temperature of 110 °C, and a flow rate of 3 mL/min. After the reaction was completed, the reaction solution was concentrated by evaporation under reduced pressure to remove the solvent, and a solution of hydrochloric acid in 1,4-dioxane (4 mol/L, 200 mL) was added to the residue. The mixture was concentrated by evaporation under reduced pressure to remove the solvent, and the residue was slurried with ethyl acetate (100 mL) and filtered. The filter cake was collected to give the target intermediate **4b** (46.96 g). MS(ESI, [M+H]⁺) m/z: 150.0.

¹H NMR (500 MHz, DMSO-d6) δ 9.81 (s, 1H), 7.03 (t, J = 7.7 Hz, 1H), 6.78 (d, J = 7.8 Hz, 1H), 6.62 (d, J = 7.6 Hz, 1H), 4.15 (s, 2H), 3.35 (s, 2H), 2.79 (s, 2H).

### Step 2: Preparation of intermediate 4c

Intermediate **4b** (40 g) and tetrahydrofuran (400 mL) were added to a reaction flask in sequence, and trifluoroacetic anhydride (61.9 g, 41.0 mL) was added under an ice bath. The mixture was reacted at room temperature. After the reaction was completed, the reaction solution was extracted with ethyl acetate (500 mL) and water (1000 mL). The organic phase was separated, dried over anhydrous sodium sulfate, and filtered, and the filtrate was concentrated by evaporation under reduced pressure to remove the solvent, thus giving the target intermediate **4c** (81 g).

MS(ESI, [M-H]⁻) *m*/*z:* 244.04.

### Step 3: Preparation of intermediate 4d

Intermediate **4c** (65.3 g), dichloromethane (650 mL), triethylamine (81 g, 111 mL), and DMAP (0.813 g) were added to a reaction flask in sequence, and acetic anhydride (29.9 g, 27.9 mL) was added under an ice bath. The mixture was reacted at room temperature. After the reaction was completed, the reaction solution was concentrated by evaporation under reduced pressure to remove the solvent, and ethyl acetate (500 mL) and water (1000 mL) were added to the residue for extraction. The organic phase was separated, washed with a saturated ammonium chloride solution and saturated brine separately, dried over anhydrous sodium sulfate, and filtered, and the filtrate was concentrated by evaporation under reduced pressure to remove the solvent, thus giving the target intermediate 4d (55.6 g).

¹H NMR (500 MHz, Chloroform-d) δ 7.28 (d, J = 7.9 Hz, 1H), 7.10 - 7.02 (m, 1H), 7.02 - 6.95 (m, 1H), 4.79 (d, J = 27.0 Hz, 2H), 3.92 - 3.78 (m, 2H), 2.82 - 2.72 (m, 2H), 2.33 (d, J = 2.0 Hz, 3H).

### Step 4: Preparation of intermediate 4e

Intermediate **4d** (30.73 g) and aluminum trichloride (21.40 g) were added to a reaction flask in sequence, and the mixture was heated to 170 °C and reacted under N₂ atmosphere. After the reaction was completed, the reaction solution was cooled to room temperature, water (300 mL) was added to quench the reaction, and the mixture was extracted with dichloromethane. The organic phase was separated, dried over anhydrous sodium sulfate, and filtered, and the filtrate was purified by a silica gel column to give the target intermediate **4e** (16.21 g).

MS(ESI, [M-H]⁻) *m*/*z*: 286.0*.*

¹H NMR (500 MHz, DMSO-d6) δ 12.74 (d, J = 11.3 Hz, 1H), 7.82 (d, J = 8.3 Hz, 1H), 6.91 (dd, J = 14.9, 8.3 Hz, 1H), 4.80 (d, J = 9.9 Hz, 2H), 3.86 (dt, J = 8.2, 5.9 Hz, 2H), 2.77 (dt, J = 17.7, 6.1 Hz, 2H), 2.64 (s, 3H).

### Step 5: Preparation of intermediate 4f

Intermediate **4e** (15.7 g) and methanol (160 mL) were added to a reaction flask in sequence, and a solution of sodium hydroxide (6.56 g) in water (160 mL) was added dropwise under an ice bath. The mixture was warmed to room temperature and reacted. After the reaction was completed, the reaction solution was concentrated by evaporation under reduced pressure to remove the methanol, and 1,4-dioxane (160 mL) and di-tert-butyl dicarbonate (13.12 g, 13.96 mL) were added. The mixture was reacted at room temperature for 1 h. After the reaction was completed, the reaction solution was extracted with ethyl acetate (500 mL) and water (800 mL). The organic phase was separated, dried over anhydrous sodium sulfate, and filtered, and the filtrate was concentrated by evaporation under reduced pressure to remove the solvent, thus giving the target intermediate **4f** (19.83 g).

MS(ESI, [M+H]⁺) m/z: 292.5.

¹H NMR (500 MHz, DMSO-d6) δ 12.76 (s, 1H), 7.76 (d, J = 8.3 Hz, 1H), 6.80 (d, J = 8.3 Hz, 1H), 4.52 (s, 2H), 3.57 (d, J = 1.8 Hz, 2H), 2.64 (d, J = 6.1 Hz, 5H), 1.43 (s, 9H).

### Step 6: Preparation of intermediate 4g

Intermediate **4f** (15.92 g), diethyl carbonate (32.3 g, 33.1 mL), and toluene (200 mL) were added to a reaction flask in sequence, and 60 wt% sodium hydride (10.93 g) was added in portions under an ice bath. The mixture was heated to 120 °C and reacted. After the reaction was completed, the reaction solution was cooled to room temperature and poured into ice water to quench the reaction. A 1 M hydrochloric acid solution was added to adjust the pH to 1-2, and ethyl acetate was added for extraction. The organic phases were separated, combined, washed with saturated brine, dried over anhydrous sodium sulfate, and filtered, and the filtrate was concentrated by evaporation under reduced pressure to remove the solvent. The residue was slurried with petroleum ether (100 mL) and filtered, and the filter cake was collected to give the target intermediate **4g** (12 g).

¹H NMR (500 MHz, DMSO-d6) δ 12.46 (s, 1H), 7.64 (d, J = 8.1 Hz, 1H), 7.17 (d, J = 8.2 Hz, 1H), 5.56 (s, 1H), 4.60 (s, 2H), 3.62 (t, J = 6.0 Hz, 2H), 2.83 (t, J = 5.8 Hz, 2H), 1.44 (s, 9H).

### Step 7: Preparation of intermediate 4h

Intermediate **4g** (12 g), ethanol (120 mL), and an hydroxylamine aqueous solution (12.49 g, 11.59 mL) were added to a reaction flask in sequence, and the mixture was heated to 85 °C and reacted. After the reaction was completed, a saturated sodium bicarbonate solution (200 mL) was added to the reaction solution, and ethyl acetate (100 mL) was added for extraction. The organic phase was separated, and the aqueous phase was adjusted to pH 3 with 1 M hydrochloric acid and extracted with ethyl acetate (200 mL). The organic phases were combined, washed with saturated brine, dried over anhydrous sodium sulfate, and filtered, and the filtrate was concentrated by evaporation under reduced pressure to remove the solvent, thus giving the target intermediate **4h** (10.44 g).

MS(ESI, [M-H]⁻) *m*/*z*: 331.0.

¹H NMR (500 MHz, DMSO-d6) δ 12.85 (s, 1H), 7.63 (d, J = 8.1 Hz, 1H), 7.21 (d, J = 8.2 Hz, 1H), 4.67 (s, 2H), 4.07 (s, 2H), 3.69 (t, J = 5.9 Hz, 2H), 2.98 (t, J = 5.9 Hz, 2H), 1.44 (s, 9H).

### Step 8: Preparation of intermediate 4i

Intermediate **4h** (10.44 g), potassium carbonate (13.02 g), DMA (110 mL), and iodoethane (5.88 g, 3.05 mL) were added to a reaction flask in sequence, and the mixture was heated to 80 °C and reacted under N₂ atmosphere. After the reaction was completed, the reaction solution was cooled to room temperature and extracted with ethyl acetate (200 mL) and water (1000 mL). The organic phase was separated, dried over anhydrous sodium sulfate, and filtered, and the filtrate was purified by a silica gel column to give the target intermediate **4i** (8.31 g).

MS(ESI, [M+H]⁺) m/z: 361.2.

### Step 9: Preparation of intermediate 4j

Intermediate **4i** (5.5 g), tetrahydrofuran (50 mL), and acrylamide (0.759 g) were added to a reaction flask in sequence, the mixture was cooled to -15 °C under N₂ atmosphere, and then a solution of potassium *tert*-butoxide in tetrahydrofuran (1 mol/L, 9.92 mL) was added dropwise. After the dropwise addition, the mixture was warmed to 0 °C and reacted. After the reaction was completed, the resulting reaction system was added to a saturated ammonium chloride solution (200 mL), and ethyl acetate was added for extraction. The organic phase was separated, dried over anhydrous sodium sulfate, and filtered, and the filtrate was purified by a silica gel column to give the target intermediate **4j** (2.81 g).

MS(ESI, [M-H]⁻) *m*/*z*: 384.34*.*

¹H NMR (500 MHz, DMSO-d6) δ 11.09 (s, 1H), 7.64 (d, J = 8.2 Hz, 1H), 7.20 (d, J = 8.3 Hz, 1H), 4.67 (s, 2H), 4.57 (dd, J = 12.0, 5.0 Hz, 1H), 3.69 (t, J = 5.9 Hz, 2H), 2.98 (t, J = 5.9 Hz, 2H), 2.77 (ddd, J = 17.3, 12.1, 5.3 Hz, 1H), 2.61 (dt, J = 17.3, 4.1 Hz, 1H), 2.54 (d, J = 4.5 Hz, 1H), 2.18 (dtd, J = 13.5, 5.2, 3.7 Hz, 1H), 1.44 (s, 9H).

### Step 10: Preparation of intermediate 4

Intermediate **4j** (850 mg) and ethyl acetate (20 mL) were added to a reaction flask in sequence, a solution of hydrochloric acid in 1,4-dioxane (4 mol/L, 11.03 mL) was added, and the mixture was reacted at room temperature. After the reaction was completed, the reaction solution was concentrated to give intermediate **4** (760 mg). MS(ESI, [M+H]⁺) m/z: 286.12.

¹H NMR (500 MHz, DMSO-d6) δ 11.08 (s, 1H), 7.54 (d, J = 8.1 Hz, 1H), 7.05 (d, J = 8.2 Hz, 1H), 4.54 (dd, J = 11.8, 5.0 Hz, 1H), 3.97 (s, 2H), 3.03 (t, J = 5.8 Hz, 2H), 2.86 (t, J = 5.8 Hz, 2H), 2.76 (td, J = 12.0, 5.9 Hz, 1H), 2.60 (dt, J = 17.3, 4.2 Hz, 1H), 2.46 (dd, J = 12.2, 4.4 Hz, 1H), 2.18 (dq, J = 13.5, 4.8 Hz, 1H).

### Example 5: Synthesis of Intermediate 5

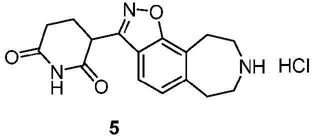

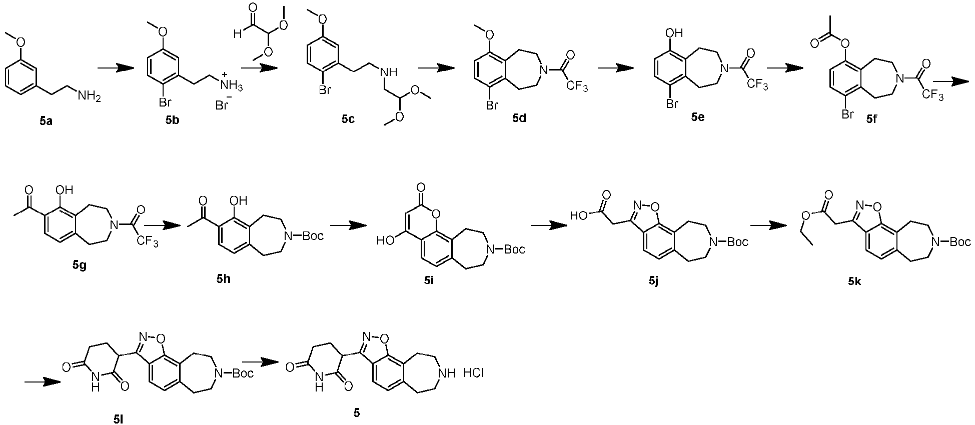

### Step 1: Preparation of intermediate 5b

Liquid bromine (55.5 g) was added dropwise to a solution of **5a** (50 g) in acetic acid (180 mL) at 15 °C. After the dropwise addition, the mixture was allowed to react at room temperature for 1 h. Methyl *tert-butyl* ether (800 mL) was added dropwise to the reaction solution, and the mixture was filtered. The filter cake was collected and dried to give intermediate **5b** (95 g).

MS(ESI, [M+H]⁺) *m*/*z*: 230.1.

### Step 2: Preparation of intermediate 5c

**5b** (80 g), glyoxal dimethyl acetal (66.9 g), triethylamine (27.3 g), anhydrous sodium sulfate (80 g), and methanol (600 mL) were added to a reaction flask in sequence, and the mixture was reacted at room temperature overnight. The reaction solution was cooled to -15 °C, and sodium borohydride (14.6 g) was added in portions. After the addition, the mixture was reacted at room temperature. The reaction solution was concentrated, and dichloromethane (400 mL) and water (700 mL) were added to the concentrate. The organic phase was separated, washed with a saturated sodium chloride solution, dried over anhydrous sodium sulfate, filtered, and concentrated. The concentrate was separated and purified by silica gel column chromatography to give intermediate **5c** (60 g).

MS(ESI, [M+H]⁺) *m*/*z*: 318.1.

### Step 3: Preparation of intermediate 5d

**5c** (47 g) was added dropwise to trifluoroacetic anhydride (148 g) at 0 °C under nitrogen atmosphere. After the dropwise addition, the mixture was allowed to react at room temperature. Trifluoroacetic acid (87 g) was added dropwise, and the mixture was warmed to 40 °C and reacted. Triethylsilane (68 g) was added dropwise, and the mixture was warmed to 60 °C and reacted. Ethyl acetate (400 mL) and water (600 mL) were added to the reaction solution. The organic phase was separated, washed with a saturated sodium chloride solution, dried over anhydrous sodium sulfate, filtered, and concentrated. The concentrate was separated and purified by silica gel column chromatography to give intermediate **5d** (20.5 g).

¹H NMR (500 MHz, DMSO-d6) δ 7.46 (dd, *J* = 8.9, 1.7 Hz, 1H), 6.87 (d, *J* = 8.9 Hz, 1H), 3.77 (d, *J* = 2.8 Hz, 3H), 3.67 (ddt, *J* = 14.4, 5.7, 3.4 Hz, 4H), 3.22 (ddd, *J* = 11.9, 6.4, 4.6 Hz, 2H), 3.16 - 3.06 (m, 2H).

### Step 4: Preparation of intermediate 5e

A solution of boron tribromide in dichloromethane (146 mL, 1 M) was slowly added dropwise to a stirred solution of **5d** (20.5 g) in dichloroethane (200 mL) at 0 °C under nitrogen atmosphere. After the dropwise addition, the mixture was allowed to react at room temperature. After the reaction was completed, the reaction solution was slowly poured into ice water (400 mL), and the mixture was stirred and filtered. The filter cake was collected and dried to give **5e** (18.5 g).

MS(ESI, [M-H] ⁻) *m*/*z:* 336.1.

¹H NMR (500 MHz, DMSO-d6) δ 9.74 (s, 1H), 7.26 (dd, *J* = 8.7, 1.3 Hz, 1H), 6.68 (dd, *J* = 8.7, 3.1 Hz, 1H), 3.73 - 3.61 (m, 4H), 3.23 - 3.13 (m, 2H), 3.12 - 3.02 (m, 2H).

### Step 5: Preparation of intermediate 5f

Acetic anhydride (5.65 g) was slowly added dropwise to a stirred solution of **5e** (17.0 g) and triethylamine (7.63 g) in dichloromethane (200 mL) at 0 °C under nitrogen atmosphere. After the dropwise addition, the mixture was allowed to react at room temperature. After the reaction was completed, the reaction solution was slowly poured into water (200 mL). The organic phase was separated, washed with a saturated sodium chloride solution, dried over anhydrous sodium sulfate, filtered, and concentrated to give **5f** (19.8 g).

MS(ESI, [M+H]⁺) *m*/*z*: 380.1.

### Step 6: Preparation of intermediate 5g

**5f** (19.5 g), aluminum trichloride (18.7 g), and orthodichlorobenzene (80 mL) were added to a reaction flask in sequence, and the mixture was warmed to 150 °C and reacted. After the reaction was completed, the reaction solution was cooled to room temperature, and 3 N diluted hydrochloric acid (250 mL) was added, followed by extraction with ethyl acetate. The organic phases were combined, washed with saturated brine, dried over anhydrous sodium sulfate, and filtered, and the concentrate was separated and purified by silica gel column chromatography to give intermediate **5g** (11.2 g).

MS(ESI, [M-H]⁻) *m*/*z:* 300.0.

¹H NMR (500 MHz, DMSO-d6) δ 12.83 (d, *J* = 4.8 Hz, 1H), 7.78 (dd, *J* = 8.1, 3.5 Hz, 1H), 6.83 (t, *J* = 8.3 Hz, 1H), 3.69 (ddd, *J* = 12.9, 9.6, 5.9 Hz, 4H), 3.11 - 3.00 (m, 4H), 2.64 (s, 3H).

### Step 7: Preparation of intermediate 5h

**5g** (9.5 g), methanol (100 mL), water (20 mL), and sodium hydroxide (1.9 g) were added to a reaction flask in sequence, and the mixture was reacted at room temperature. Then, di-tert-butyl dicarbonate (8.2 g) was added. After the reaction was completed, ethyl acetate (200 mL) and water (400 mL) were added to the reaction solution. The organic phase was separated, washed with saturated brine, dried over anhydrous sodium sulfate, and filtered, and the concentrate was separated and purified by silica gel column chromatography to give intermediate **5h** (8.5 g).

¹H NMR (500 MHz, DMSO-*d*₆) δ 12.80 (s, 1H), 7.74 (d, *J =* 8.1 Hz, 1H), 6.79 (d, *J* = 8.2 Hz, 1H), 3.45 (dt, *J* = 11.6, 5.0 Hz, 4H), 2.92 (q, *J* = 5.0 Hz, 4H), 2.63 (s, 3H), 1.38 (s, 9H).

### Step 8: Preparation of intermediate 5i

**5h** (8.5 g), diethyl carbonate (16.4 g), and toluene (100 mL) were added to a reaction flask in sequence, and 60 wt% sodium hydride (5.57 g) was added in portions. The reaction solution was warmed to 115 °C and reacted. After the reaction was completed, the reaction solution was cooled to room temperature, and ethyl acetate (200 mL) and water (300 mL) were added to the reaction solution. The organic phase was separated, washed with saturated brine, dried over anhydrous sodium sulfate, and filtered, and the concentrate was separated and purified by silica gel column chromatography to give intermediate **5i** (9.0 g).

MS(ESI, [M-H]⁻) *m*/*z*: 330.1.

### Step 9: Preparation of intermediate 5j

**5i** (9.0 g), an hydroxylamine aqueous solution (8.7 g), and ethanol (100 mL) were added to a reaction flask in sequence, and the reaction solution was warmed to 80 °C and reacted. After the reaction was completed, the reaction solution was cooled to room temperature, and ethyl acetate (200 mL) and water (300 mL) were added to the reaction solution. The organic phase was separated, washed with saturated brine, dried over anhydrous sodium sulfate, and filtered, and the concentrate was separated and purified by silica gel column chromatography to give intermediate **5j** (8.5 g).

MS(ESI, [M-H]⁻) *m*/*z*: 345.4.

¹H NMR (500 MHz, DMSO-*d*₆) δ 7.53 (d, *J* = 8.0 Hz, 1H), 7.12 (d, *J* = 8.0 Hz, 1H), 3.73 (*q*, *J* = 13.9, 11.5 Hz, 2H), 3.59 - 3.54 (m, 2H), 3.52 - 3.47 (m, 2H), 3.13 (t, *J* = 5.2 Hz, 2H), 3.07 - 2.98 (m, 2H), 1.40 (s, 9H).

### Step 10: Preparation of intermediate 5k

**5j** (8.5 g), potassium carbonate (3.3 g), iodoethane (5.1 g), and DMA (70 mL) were added to a reaction flask in sequence, and the reaction solution was warmed to 80 °C and reacted. The reaction solution was cooled to room temperature, and ethyl acetate (200 mL) and water (300 mL) were added to the reaction solution. The organic phase was separated, washed with saturated brine, dried over anhydrous sodium sulfate, and filtered, and the concentrate was separated and purified by silica gel column chromatography to give intermediate **5k** (6.5 g).

MS(ESI, [M-H]⁻) *m*/*z*: 373.1.

¹H NMR (500 MHz, DMSO-*d*₆) δ 7.56 (d, *J* = 8.0 Hz, 1H), 7.22 (d, *J* = 8.0 Hz, 1H), 4.13 (dd, *J* = 13.7, 6.6 Hz, 4H), 3.55 (dt, *J* = 28.2, 5.0 Hz, 4H), 3.17 (s, 2H), 3.05 (t, *J* = 5.2 Hz, 2H), 1.38 (dd, *J* = 9.3, 4.4 Hz, 9H), 1.19 (t, *J=* 6.5 Hz, 3H).

### Step 11: Preparation of intermediate 5l

A solution of sodium tert-butoxide in tetrahydrofuran (14 mL, 1 M) was slowly added dropwise to a stirred solution of **5k** (5.6 g) in tetrahydrofuran (70 mL) at -10 °C under nitrogen atmosphere. After the dropwise addition, the mixture was reacted for 30 min with the temperature maintained. Acrylamide (0.71 g) was weighed out and dissolved in tetrahydrofuran (5 mL), and the resulting solution was added dropwise to the reaction solution. The mixture was reacted for 2 h with the temperature maintained. The reaction solution was slowly poured into saturated ammonium chloride solution (200 mL), and ethyl acetate (200 mL) was added. The organic phase was separated, washed with a saturated sodium chloride solution, dried over anhydrous sodium sulfate, and filtered, and the concentrate was separated and purified by silica gel column chromatography to give intermediate **5l** (2.5 g).

MS(ESI, [M-H]⁻) *m*/*z:* 397.9.

¹H NMR (500 MHz, DMSO-*d*₆) δ 11.08 (s, 1H), 7.58 (d, *J* = 8.0 Hz, 1H), 7.21 (d, *J* = 8.1 Hz, 1H), 4.55 (dd, *J* = 12.0, 4.9 Hz, 1H), 3.55 (dt, *J* = 31.4, 5.0 Hz, 4H), 3.22 - 3.00 (m, 4H), 2.77 (ddd, *J* = 17.3, 12.0, 5.3 Hz, 1H), 2.60 (dt, *J* = 17.3, 4.1 Hz, 1H), 2.46 (dd, *J* = 12.2, 4.4 Hz, 1H), 2.20 - 2.12 (m, 1H), 1.38 (d, *J* = 6.3 Hz, 9H).

### Step 12: Preparation of intermediate 5

**51** (2.5 g), ethyl acetate (30 mL), and a solution of hydrochloric acid in 1,4-dioxane (15 mL,4 M) were added to a reaction flask in sequence, and the mixture was reacted at room temperature. After the reaction was completed, the mixture was filtered, and the filter cake was collected and dried to give intermediate **5** (18.5 g).

MS(ESI, [M+H]⁺) *m*/*z*: 300.2*.*

¹H NMR (500 MHz, DMSO-d6) δ 11.10 (s, 1H), 9.53 (s, 2H), 7.65 (d, *J* = 8.1 Hz, 1H), 7.27 (d, *J =* 8.1 Hz, 1H), 4.59 (dd, *J* = 12.1, 4.9 Hz, 1H), 3.43 (dd, *J* = 7.0, 3.3 Hz, 2H), 3.37 - 3.19 (m, 6H), 2.78 (ddd, *J* = 17.3, 12.1, 5.3 Hz, 1H), 2.61 (dt, *J=* 17.3, 4.1 Hz, 1H), 2.17 (dtd, *J* = 13.4, 5.2, 3.6 Hz, 1H).

### Example 6: Synthesis of Intermediate 6

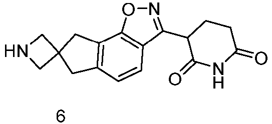

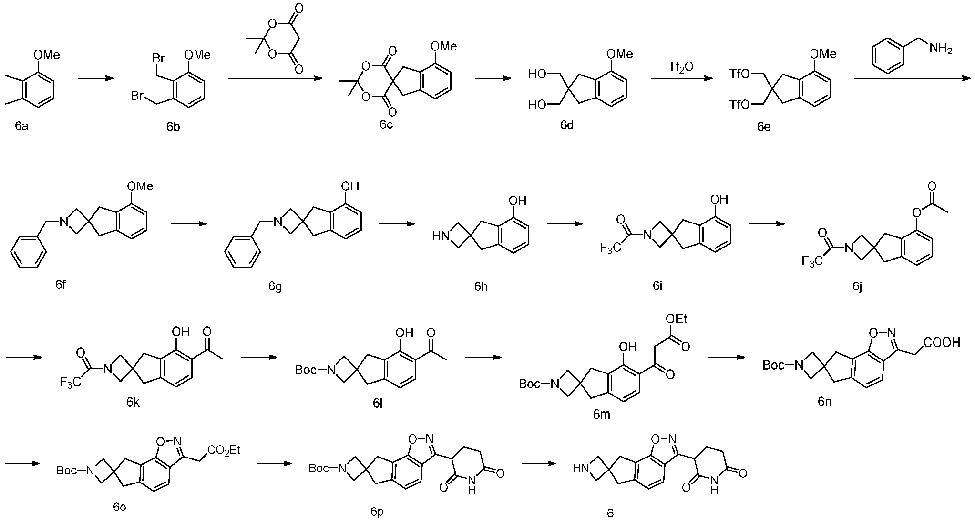

### Step 1: Preparation of intermediate 6b

Carbon tetrachloride (1500 mL), **6a** (100 g), 2,2-azobisisobutyronitrile (4.1 g), and N-bromosuccinimide (265 g) were added to a reaction flask in sequence, and the mixture was heated to 80 °C and reacted. After the reaction was completed, the mixture was filtered under vacuum, and the mother liquor was concentrated to dryness. The residue was slurried with petroleum ether and filtered under vacuum. The filter cake was collected and dried to give intermediate **6b** (154 g).

¹H NMR (500 MHz, DMSO-d6) δ 7.36-7.33 (m, 1H), 7.09-7.04 (m, 2H), 4.78 (s, 2H), 4.76 (s, 2H), 3.87 (s, 3H).

### Step 2: Preparation of intermediate 6c

2,2-Dimethyl-1,3-dioxane-4,6-dione (150 g), DMSO (500 mL), triethylamine (255 mL), and intermediate **6b** (120 g) were added to a reaction flask in sequence, and the mixture was reacted at room temperature. After the reaction was completed, the reaction solution was poured into water, extracted with EA, washed with a saturated citric acid aqueous solution, a saturated sodium bicarbonate aqueous solution, and saturated brine in sequence, dried over anhydrous sodium sulfate, filtered, and concentrated. The concentrate was dissolved in THF with heating. A solid was precipitated at room temperature, and the mixture was filtered under vacuum. The filter cake was collected and dried to give intermediate **6c** (45 g).

¹H NMR (500 MHz, DMSO-d6) δ 7.21 (t, *J* = 7.8 Hz, 1H), 6.83 (t, *J* = 7.0 Hz, 2H), 3.79 (s, 3H), 3.63 (s, 2H), 3.49 (s, 2H), 1.77 (s, 6H).

### Step 3: Preparation of intermediate 6d

A solution of lithium aluminum hydride in tetrahydrofuran (1 M, 163 mL) was slowly added dropwise to a stirred solution of intermediate **6c** (45 g) in THF (500 mL) at 0 °C under nitrogen atmosphere, and the temperature was controlled below 5 °C. The mixture was reacted at 0 °C for 0.5 h and then warmed to room temperature. After the reaction was completed, a saturated ammonium chloride solution (200 mL) was added to quench the reaction, and the mixture was extracted with EA, washed with saturated brine, dried over anhydrous sodium sulfate, filtered, and concentrated to give intermediate **6d** (40.5 g).

¹H NMR (500 MHz, DMSO-d6) δ 7.07 (t, *J* = 7.8 Hz, 1H), 6.74 (d, *J* = 7.4 Hz, 1H), 6.70 (d, *J* = 8.1 Hz, 1H), 4.59 (t, *J* = 5.3 Hz, 2H), 3.73 (s, 3H), 3.34 (d, *J* = 5.4 Hz, 4H), 2.68 (s, 2H), 2.58 (s, 2H).

### Step 4: Preparation of intermediate 6c

Trifluoroacetic anhydride (122 g) was slowly added dropwise to a stirred solution of intermediate **6d** (40.5 g) and DIPEA (76 g) in DCM (500 mL) at -20 °C under nitrogen atmosphere, and the mixture was stirred at -5 °C for 0.5 h, then warmed to room temperature, and reacted. After the reaction was completed, water (100 mL) was added to quench the reaction, and the mixture was extracted with DCM, washed with a 10% citric acid aqueous solution, a saturated sodium bicarbonate aqueous solution, and a saturated sodium chloride aqueous solution in sequence, dried over anhydrous sodium sulfate, filtered, and concentrated. The residue was subjected to silica gel column chromatography to give intermediate **6e** (64.2 g).

¹H NMR (500 MHz, DMSO-d6) δ 7.20 - 7.16 (m, 1H), 6.84 - 6.80 (m, 2H), 4.25 (s, 4H), 3.77 (s, 3H), 2.87 (s, 2H), 2.77 (s, 2H).

### Step 5: Preparation of intermediate 6f

Intermediate **6e** (63.4 g), benzylamine (11.51 g), DIPEA (41.6 g), and acetonitrile (500 mL) were added to a reaction flask in sequence at room temperature under nitrogen atmosphere, and the mixture was reacted at room temperature overnight. After the reaction was completed, the mixture was subjected to silica gel column chromatography to give intermediate **6f** (31.5 g).

¹H NMR (500 MHz, DMSO-d6) δ 7.33 - 7.24 (m, 4H), 7.24 - 7.18 (m, 1H), 7.09 (t, *J* = 7.8 Hz, 1H), 6.78 (d, *J* = 7.4 Hz, 1H), 6.73 (d, *J* = 8.1 Hz, 1H), 3.74 (s, 3H), 3.56 (s, 2H), 3.15 - 3.08 (m, 4H), 3.06 (s, 2H), 2.96 (s, 2H).

### Step 6: Preparation of intermediate 6g

A solution of hydrochloric acid in 1,4-dioxane (4 M, 79 mL) was added to a solution of intermediate 6f (25.2 g) in DCM (500 mL) at 0 °C, and the mixture was reacted for 5 min and concentrated to dryness under reduced pressure. DCM (500 mL) was then added, the mixture was cooled to -78 °C, and a solution of boron tribromide in dichloromethane (1 M, 271 mL) was slowly added dropwise. After the dropwise addition, the mixture was warmed to room temperature and reacted overnight. After the reaction was completed, the reaction solution was cooled under an ice bath. Methanol was added to quench the reaction, and a saturated sodium bicarbonate solution was added to adjust the pH to neutral. The mixture was extracted with DCM, washed with saturated brine, dried over anhydrous sodium sulfate, filtered, and concentrated to give intermediate **6g** (29.6 g).

MS(ESI, [M+H]⁺) m/z: 266.1.

¹H NMR (500 MHz, DMSO-d6) δ 9.26 (s, 1H), 7.47 - 7.31 (m, 5H), 6.94 (t, *J* = 7.7 Hz, 1H), 6.64 (d, *J* = 7.3 Hz, 1H), 6.58 (d, *J* = 8.0 Hz, 1H), 4.42 - 3.41 (m, 6H), 3.13 (s, 2H), 3.06 (s, 2H).

### Step 7: Preparation of intermediate 6h

Intermediate **6g** (29.60 g), palladium on carbon (10%, 29.6 g), and MeOH (600 mL) were added to a reaction flask in sequence, and the mixture was purged with hydrogen and then reacted at 40 °C under hydrogen atmosphere overnight. After the reaction was completed, the mixture was filtered under vacuum and concentrated to give intermediate **6h** (23.1 g).

MS(ESI, [M+H]+) m/z: 176.1.

### Step 8: Preparation of intermediate 6i

Intermediate **6h** (23.5 g), THF (500 mL), and trifluoroacetic anhydride (28.4 g) were added to a reaction flask in sequence at room temperature, and the mixture was reacted at room temperature. After the reaction was completed, saturated sodium bicarbonate (200 mL) was added to quench the reaction, and the mixture was extracted with EA, washed with saturated brine, dried over anhydrous sodium sulfate, filtered, and concentrated. The residue was subjected to silica gel column chromatography to give intermediate **6i** (25.92 g).

MS(ESI, [M-H]⁻) m/z: 270.1.

### Step 9: Preparation of intermediate 6j

Intermediate **6i** (23.5 g), DCM (500 mL), triethylamine (17.53 g), DMAP (1.058 g), and acetic anhydride (9.73 g) were added to a reaction flask in sequence at room temperature, and the mixture was reacted at room temperature. After the reaction was completed, water was added to quench the reaction, and the mixture was extracted with DCM. The organic phase was collected, washed with a 5% citric acid aqueous solution, a saturated sodium bicarbonate aqueous solution, and a saturated sodium chloride aqueous solution in sequence, dried over anhydrous sodium sulfate, filtered, and concentrated to give intermediate **6j** (18.58 g).

¹H NMR (500 MHz, DMSO-*d*6) δ 7.20 (t, *J* = 7.7 Hz, 1H), 7.16 - 7.10 (m, 1H), 6.91 (dd, *J* = 8.0, 0.9 Hz, 1H), 4.43 - 4.31 (m, 2H), 4.10 - 3.98 (m, 2H), 3.25 (s, 2H), 3.07 (s, 2H), 2.28 (s, 3H).

### Step 10: Preparation of intermediate 6k

Intermediate **6j** (18.4 g), aluminum trichloride (15.66 g), and orthodichlorobenzene(200 mL) were added to a reaction flask in sequence, and the mixture was gradually warmed from 70 °C to 150 °C and reacted. After the reaction was completed, the mixture was cooled to room temperature, and EA (1000 mL), 3 M hydrochloric acid (60 mL), and water (200 mL) were added in sequence for dissolution. The mixture was extracted with EA, washed with saturated brine, dried over anhydrous sodium sulfate, filtered, and concentrated. The residue was subjected to silica gel column chromatography to give intermediate **6k** (15.11 g).

MS(ESI, [M-H]⁻) m/z: 312.1.

¹H NMR (500 MHz, DMSO-d6) δ 12.35 (s, 1H), 7.78 (d, *J* = 8.0 Hz, 1H), 6.87 (d, *J* = 8.0 Hz, 1H), 4.41 (d, *J* = 5.9 Hz, 2H), 4.10 - 4.06 (m, 2H), 3.27 (s, 2H), 3.17 (s, 2H), 2.62 (s, 3H).

### Step 11: Preparation of intermediate 6l

An sodium hydroxide aqueous solution (1 M, 97 mL) was added to a solution of intermediate **6k** (15.11 g) in MeOH (150 mL) at 0 °C, and the mixture was reacted at room temperature. After the reaction was completed, the mixture was concentrated to remove the methanol, and water was retained. Dioxane (150 mL) and Boc anhydride (11.58 g) were added to the residue, and the mixture was reacted at room temperature. After the reaction was completed, 10% citric acid was added to adjust the pH to 5-6, and the mixture was extracted with EA, then washed with a saturated sodium bicarbonate aqueous solution and a saturated sodium chloride aqueous solution in sequence, dried over anhydrous sodium sulfate, filtered, and concentrated. The residue was subjected to silica gel column chromatography to give intermediate **6l** (11.40 g).

MS(ESI, [M-H]⁻) m/z: 316.1.

¹H NMR (500 MHz, DMSO-d6) δ 12.34 (s, 1H), 7.77 (d, *J* = 8.0 Hz, 1H), 6.85 (d, *J* = 8.0 Hz, 1H), 3.81 (s, 4H), 3.18 (s, 2H), 3.06 (s, 2H), 2.61 (s, 3H), 1.38 (s, 9H).

### Step 12: Preparation of intermediate 6m

Sodium hydride (60 wt%, 6.93 g) was added in portions to a solution of intermediate **6l** (11 g) and diethyl carbonate (20.47 g) in a mixed solvent of toluene (200 mL) and THF (100 mL) at 0 °C, and the mixture was warmed to 100 °C and reacted. After the reaction was completed, the reaction solution was cooled to room temperature, water was added to quench the reaction, and the mixture was extracted with EA. The organic phases were combined, washed with saturated brine, dried over anhydrous sodium sulfate, filtered, and concentrated. The residue was subjected to silica gel column chromatography to give intermediate **6m** (12.62 g).

MS(ESI, [M-H]⁻) m/z: 388.2.

¹H NMR (500 MHz, DMSO-d6) δ 11.74 (s, 1H), 7.70 (d, *J* = 8.1 Hz, 1H), 6.87 (d, *J =* 8.1 Hz, 1H), 4.19 (s, 2H), 4.12 (q, *J* = 7.1 Hz, 2H), 3.82 (s, 4H), 3.19 (s, 2H), 3.08 (s, 2H), 1.38 (s, 9H), 1.18 (t, *J* = 7.1 Hz, 3H).

### Step 13: Preparation of intermediate 6n

Intermediate **6m** (12.5 g), hydroxylamine (5.30 g), and ethanol (200 mL) were added to a reaction flask in sequence, and the mixture was reacted at 85 °C. After the reaction was completed, the reaction solution was cooled to room temperature and concentrated by evaporation under reduced pressure to remove the solvent. EA (500 mL), water (200 mL), and a saturated sodium carbonate aqueous solution (150 mL) were added to the residue, and the aqueous phase was collected, then adjusted to about pH 4 with 1 M diluted hydrochloric acid, and extracted with EA. The organic phases were combined, washed with saturated brine (200 mL), dried over anhydrous sodium sulfate, filtered, and concentrated to give intermediate **6n** (10.12 g).

¹H NMR (500 MHz, DMSO-d6) δ 12.86 (s, 1H), 7.62 (d, *J* = 8.0 Hz, 1H), 7.27 (d, *J* = 8.1 Hz, 1H), 4.05 (s, 2H), 3.95 - 3.79 (m, 4H), 3.38 (s, 2H), 3.30 (s, 2H), 1.39 (s, 9H).

### Step 14: Preparation of intermediate 6o

Intermediate **6n** (9.6 g), potassium carbonate (11.11 g), DMA (150 mL), and iodoethane (6.27 g) were added to a reaction flask in sequence, and the mixture was reacted at 85 °C. After the reaction was completed, the reaction solution was cooled to room temperature, water (600 mL) was added, and the mixture was extracted with EA, washed with saturated brine, dried over anhydrous sodium sulfate, filtered, and concentrated. The residue was subjected to silica gel column chromatography to give intermediate **6o** (11.02 g).

MS(ESI, [M+H]⁺) m/z: 387.2.

### Step 15: Preparation of intermediate 6p

A solution of potassium *tert*-butoxide in tetrahydrofuran (1 M, 15.52 mL) was slowly added dropwise to a stirred solution of intermediate **6o** (10 g) and acrylamide (1.104 g) in THF (300 mL) at -5 °C under nitrogen atmosphere. After 10 min, the dropwise addition was completed, and then the mixture was stirred at -5 °C. A saturated ammonium chloride solution (200 mL) was added to quench the reaction, and the mixture was extracted with DCM (300 mL × 2). The organic phases were combined, washed with saturated brine, dried over anhydrous sodium sulfate, filtered, and concentrated. The residue was subjected to silica gel column chromatography to give intermediate **6p** (4.32 g). MS(ESI, [M+H]⁺) m/z: 412.2.

¹H NMR (500 MHz, DMSO-*d*₆) δ 11.08 (s, 1H), 7.64 (d, *J* = 8.1 Hz, 1H), 7.26 (d, *J* = 8.1 Hz, 1H), 4.57 (dd, *J* = 12.0, 4.9 Hz, 1H), 3.98 - 3.80 (m, 4H), 3.38 (s, 2H), 3.30 (s, 2H), 2.82 - 2.71 (m, 1H), 2.65 - 2.56 (m, 1H), 2.49 - 2.43 (m, 1H), 2.22 - 2.14 (m, 1H), 1.39 (s, 9H).

### Step 16: Preparation of intermediate 6

**6p** (50 mg), DCM (5.00 mL), and EA (5 mL) were added to a reaction flask in sequence. After the reaction solution achieved complete dissolution, trifluoroacetic acid (1 mL) was added. The mixture was stirred at 25 °C overnight.

After the reaction was completed, the reaction solution was concentrated to dryness, and the residue was dissolved in DMSO, purified by reversed-phase chromatography, and lyophilized to give intermediate **6** (25 mg).

MS(ESI, [M+H]⁺) m/z: 312.2.

¹H NMR (500 MHz, DMSO-d6) δ 7.63 (d, *J* = 8.0 Hz, 1H), 7.27 (d, *J* = 8.1 Hz, 1H), 4.56 (dd, *J =* 11.9, 5.0 Hz, 1H), 3.61 (q, *J* = 8.2 Hz, 4H), 3.36 (s, 2H), 3.28 (s, 2H), 2.82 - 2.71 (m, 1H), 2.60 (dt, *J* = 17.3, 4.1 Hz, 1H), 2.49 - 2.43 (m, 1H), 2.22 - 2.12 (m, 1H).

### Example 7: Synthesis of Intermediate 7

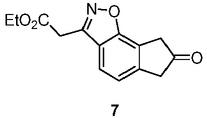

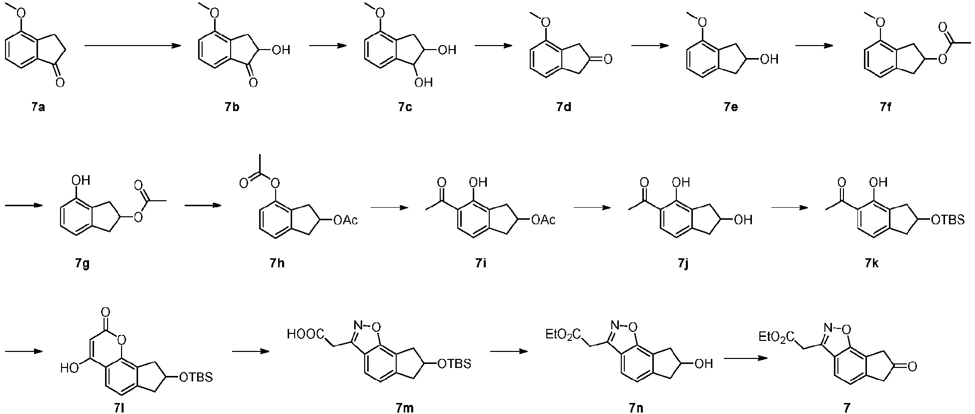

### Step 1: Preparation of compound 7b

**7a** (33.3 g), MeOH (500 mL), iodobenzene diacetate (82 g, 246 mmol), and potassium hydroxide (127 g) were added to a reaction flask in sequence under an ice bath, and the mixture was reacted at room temperature for 3 h. The reaction solution was concentrated by evaporation under reduced pressure to remove the solvent, and ethyl acetate (500 mL) and a sodium bicarbonate solution (1000 mL) were added to the residue for extraction. The organic phase was separated, dried over anhydrous sodium sulfate, filtered, and concentrated by evaporation under reduced pressure to remove the solvent. The residue was dissolved in THF (500 mL), and then hydrochloric acid (6 M, 68.4 mL) was added. The mixture was reacted at room temperature. After the reaction was completed, the reaction solution was adjusted to pH 8 with a saturated sodium bicarbonate solution, extracted with ethyl acetate, dried over anhydrous sodium sulfate, concentrated, and purified by silica gel column chromatography to give **7b** (16 g).

MS(ESI, [M+H]⁺) *m*/*z*: 178.9.

### Step 2: Preparation of compound 7c

**7b** (80 g) and MeOH (1000 mL) were added to a reaction flask in sequence, and after complete dissolution, sodium borohydride (17.83 g, 471 mmol) was added. The mixture was reacted at room temperature. After the reaction was completed, a saturated ammonium chloride solution (500 mL) was added dropwise to the reaction solution to quench the reaction, and ethyl acetate was added for extraction. The organic phase was dried over anhydrous sodium sulfate, filtered, and concentrated by evaporation under reduced pressure to remove the solvent, thus giving **7c** (82 g).

¹H NMR (500 MHz, DMSO-d6) δ 7.17 (t, J = 7.8 Hz, 1H), 6.87 (d, J = 7.4 Hz, 1H), 6.80 (d, J = 8.1 Hz, 1H), 5.34 (d, J = 6.3 Hz, 1H), 5.13 (d, J = 5.0 Hz, 1H), 4.67 (t, J = 5.8 Hz, 1H), 4.06 (ddd, J = 12.0, 6.8, 5.1 Hz, 1H), 3.75 (s, 3H), 3.03 (dd, J = 15.8, 7.1 Hz, 1H), 2.43 (dd, J = 15.8, 6.5 Hz, 1H).

### Step 3: Preparation of compound 7d

**7c** (35 g), toluene (300 mL), and *p*-toluenesulfonic acid (66.9 g) were added to a reaction flask in sequence, and the mixture was heated to 120 °C and reacted. After the reaction was completed, the reaction solution was cooled to room temperature, and the organic solvent ethyl acetate (200 mL) and water (500 mL) were added to quench the reaction. The organic phase was separated, dried over anhydrous sodium sulfate, filtered, and concentrated by evaporation under reduced pressure to remove the solvent, thus giving **7d** (35.5 g).

¹H NMR (500 MHz, DMSO-d6) δ 7.26 - 7.22 (m, 1H), 6.93 - 6.87 (m, 2H), 3.79 (s, 3H), 3.53 (s, 2H), 3.37 (s, 2H). Step 4: Preparation of compound **7e**

**7d** (35 g), MeOH (400 mL), and sodium borohydride (5.83 g) were added to a reaction flask in sequence, and the mixture was reacted at room temperature. After the reaction was completed, a saturated ammonium chloride solution (500 mL) was added dropwise to the reaction solution to quench the reaction, and ethyl acetate was added for extraction. The organic phase was dried over anhydrous sodium sulfate, filtered, and concentrated by evaporation under reduced pressure to remove the solvent, thus giving **7e** (18 g).

¹H NMR (500 MHz, DMSO-d6) δ 7.13 - 7.07 (m, 1H), 6.83 - 6.78 (m, 1H), 6.74 (d, J = 8.1 Hz, 1H), 4.81 (d, J = 3.8 Hz, 1H), 4.49 (tq, J = 6.5, 3.4 Hz, 1H), 3.75 (s, 3H), 3.04 (dd, J = 16.1, 6.1 Hz, 1H), 2.94 (dd, J = 16.3, 6.2 Hz, 1H), 2.79 - 2.61 (m, 2H).

### Step 5: Preparation of compound 7f

**7e** (60 g), dichloromethane (500 mL), triethylamine (111 g, 152 mL), and acetic anhydride (41.0 g, 38.2 mL) were added to a reaction flask in sequence, and the mixture was reacted at room temperature. After the reaction was completed, the reaction solution was washed with a saturated ammonium chloride solution (500 mL) and saturated brine (500 mL) separately, dried over anhydrous sodium sulfate, filtered, and concentrated under reduced pressure to give **7f** (37.2 g).

¹H NMR (500 MHz, DMSO-d6) δ 7.22 - 7.06 (m, 1H), 6.82 (dd, J = 27.3, 8.3 Hz, 2H), 5.41 (s, 1H), 3.78 (d, J = 13.2 Hz, 3H), 3.32 - 3.20 (m, 1H), 3.18 - 3.09 (m, 1H), 2.85 (dd, J = 34.6, 17.1 Hz, 2H), 1.97 (d, J = 15.3 Hz, 3H).

### Step 6: Preparation of compound 7g

Boron trichloride (19.22 g, 164 mL) was slowly added dropwise to a stirred solution of **7f** (17 g) in dichloromethane (500 mL) under an ice bath. After the dropwise addition, the mixture was naturally warmed to room temperature and reacted. After the reaction was completed, 1 M HCl (160 mL) and an aqueous solution (200 mL) were added to the reaction solution to quench the reaction, and then dichloromethane was added for extraction. The organic phase was separated, dried over anhydrous sodium sulfate, and concentrated by evaporation under reduced pressure to remove the solvent, thus giving **7g** (15 g).

MS(ESI, [M-H]⁻) *m*/*z:* 190.9.

### Step 7: Preparation of compound 7h

**7g** (16 g), dichloromethane (200 mL), triethylamine (9.50 g, 13.01 mL), and acetic anhydride (5.27 g, 4.91 mL) were added to a reaction flask in sequence, and the mixture was reacted at room temperature. After the reaction was completed, the reaction solution was washed with a saturated ammonium chloride solution (500 mL) and saturated brine (500 mL) separately, dried over anhydrous sodium sulfate, filtered, and concentrated under reduced pressure to give **7h** (14.8 g).

MS(ESI, [M-H]⁻) *m*/*z*: 233.01*.*

### Step 8: Preparation of compound 7i

**7h** (11.6 g), dichloromethane (300 mL), and zirconium tetrachloride (46.2 g) were added to a reaction flask in sequence, and the mixture was reacted at 50 °C. After the reaction was completed, the reaction solution was cooled to room temperature, and a 3 M hydrochloric acid aqueous solution (200 mL) was added to the residue, followed by the addition of water (100 mL) and dichloromethane (100 mL). The organic phase was separated, dried over anhydrous sodium sulfate, filtered, and concentrated by evaporation under reduced pressure to remove the solvent, thus giving **7i** (11.4 g).

¹H NMR (500 MHz, DMSO-d6) δ 12.34 (s, 1H), 7.80 (d, *J* = 8.0 Hz, 1H), 6.91 (d, *J* = 8.0 Hz, 1H), 5.45 (tt, *J* = 6.3, 2.2 Hz, 1H), 3.37 - 3.33 (m, 1H), 3.19 (dd, *J* = 17.3, 6.3 Hz, 1H), 2.95 (dd, *J* = 17.9, 2.2 Hz, 1H), 2.86 (dd, *J* = 17.3, 2.1 Hz, 1H), 2.63 (s, 3H), 1.97 (s, 3H).

### Step 9: Preparation of compound 7k

**7i** (15.4 g), ethanol (200 mL), and a solution of sodium hydroxide (2.63 g) in water (10.00 mL) were added to a reaction flask in sequence, and the mixture was reacted at room temperature. After the reaction was completed, the reaction solution was adjusted to pH 2-3 with a 2 M HCl aqueous solution, extracted with ethyl acetate (100 mL) and water (200 mL), dried over anhydrous sodium sulfate, filtered, and concentrated by evaporation under reduced pressure to remove the solvent, thus giving a crude product of intermediate **7j.** Dichloroethane (200 mL), imidazole (17.90 g), and TBSCl (39.6 g) were added, and the mixture was reacted under reflux overnight. The reaction solution was cooled to room temperature, and dichloromethane (100 mL) and water (300 mL) were added for extraction. The organic phase was dried over anhydrous sodium sulfate, filtered, and concentrated by evaporation under reduced pressure to remove the solvent, thus giving **7k** (15.4 g).

¹H NMR (500 MHz, DMSO-d6) δ 12.25 (s, 1H), 7.69 (d, *J =* 8.0 Hz, 1H), 6.79 (d, *J* = 8.0 Hz, 1H), 4.64 (dq, *J* = 6.2, 3.2 Hz, 1H), 3.05 (ddd, *J* = 56.3, 16.6, 6.2 Hz, 2H), 2.71 (dd, *J* = 17.0, 3.6 Hz, 1H), 2.61 (dd, *J* = 16.3, 3.5 Hz, 1H), 2.51 (s, 3H), 0.78 (s, 9H), 0.00 (s, 6H).

### Step 10: Preparation of compound 7l

**7k** (8.4 g) and THF (300 mL) were added to a reaction flask in sequence, and diethyl carbonate (16.19 g, 16.52 mL) was added. The mixture was cooled to about 0 °C, and 60 wt% sodium hydride (5.48 g, 137 mmol) was added in portions. The mixture was heated to 85 °C and reacted. After the reaction was completed, the reaction solution was cooled to room temperature and slowly poured into ice water (500 mL). The mixture was extracted with ethyl acetate (200 mL), and the organic phase was discarded. The aqueous phase was adjusted to pH = 1-2 with 3 M hydrochloric acid, then extracted with ethyl acetate, dried over anhydrous sodium sulfate, filtered, and concentrated by evaporation under reduced pressure to remove the solvent, thus giving **7l** (10 g).

MS(ESI, [M-H]⁻) m/z: 331.2.

### Step 11: Preparation of intermediate 7m

7**l** (10 g), an hydroxylamine aqueous solution (9.93 g, 9.93 mL), and ethanol (100 mL) were added to a reaction flask in sequence, and the mixture was reacted at 85 °C. After the reaction was completed, the reaction solution was cooled to room temperature, ethyl acetate (200 mL) and a saturated sodium carbonate aqueous solution (100 mL) were added to the residue for extraction, and the organic phase was discarded. The aqueous phase was adjusted to pH = 2-3 with a 1 M HCl aqueous solution, extracted with ethyl acetate, dried over anhydrous sodium sulfate, filtered, and concentrated by evaporation under reduced pressure to remove the solvent, thus giving **7m** (11 g).

MS(ESI, [M-H]⁻) m/z: 346.2.

### Step 12: Preparation of intermediate 7n

**7m** (10 g), ethanol (150 mL), and sulfuric acid (14.40 g, 7.83 mL) were added to a reaction flask in sequence, and the mixture was reacted at 85 °C. After the reaction was completed, the reaction solution was cooled to room temperature and adjusted to pH = 7 with dichloromethane (200 mL) and a saturated sodium bicarbonate aqueous solution. The organic phase was separated, dried over anhydrous sodium sulfate, filtered, and concentrated by evaporation under reduced pressure to remove the solvent, thus giving **7n** (5.8 g).

MS(ESI, [M+H]⁺) m/z: 261.97.

¹H NMR (500 MHz, DMSO-d6) δ 7.65 (d, *J* = 8.0 Hz, 1H), 7.33 (d, *J* = 8.0 Hz, 1H), 5.09 (d, *J* = 4.0 Hz, 1H), 4.71 (dt, *J* = 6.4, 3.1 Hz, 1H), 4.26 - 4.12 (m, 4H), 3.30 (ddd, *J* = 31.0, 16.5, 6.0 Hz, 2H), 2.98 (ddd, *J* = 31.6, 16.5, 3.0 Hz, 2H), 1.22 (t, *J* = 7.1 Hz, 3H).

### Step 13: Preparation of intermediate 7

Compound **7n** (300 mg), dichloromethane (10 mL), and Dess-Martin periodinane (974 mg) were added to a reaction flask in sequence, and the mixture was stirred at room temperature. After the reaction was completed, the reaction solution was poured into a saturated sodium sulfite solution to quench the reaction, and then ethyl acetate (100 mL) was added for extraction. The organic phase was separated, and the aqueous phase was extracted with ethyl acetate. The organic phases were then combined, washed with a saturated sodium bicarbonate solution and saturated brine separately, dried over anhydrous sodium sulfate, and filtered, and the filtrate was concentrated by evaporation under reduced pressure to remove the solvent, thus giving intermediate **7** (320 mg).

MS(ESI, [M+H]⁺) m/z: 260.0.

¹H NMR (500 MHz, DMSO-d6) δ 7.74 (d, J = 8.1 Hz, 1H), 7.38 (d, J = 8.1 Hz, 1H), 4.21 (s, 2H), 4.14 (q, J = 7.1 Hz, 2H), 3.79 (s, 2H), 3.72 (s, 2H), 1.19 (t, J = 7.1 Hz, 3H).

### Examples 8 and 9: Synthesis of Intermediates 8 and 9

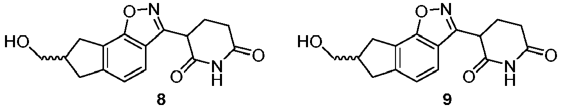

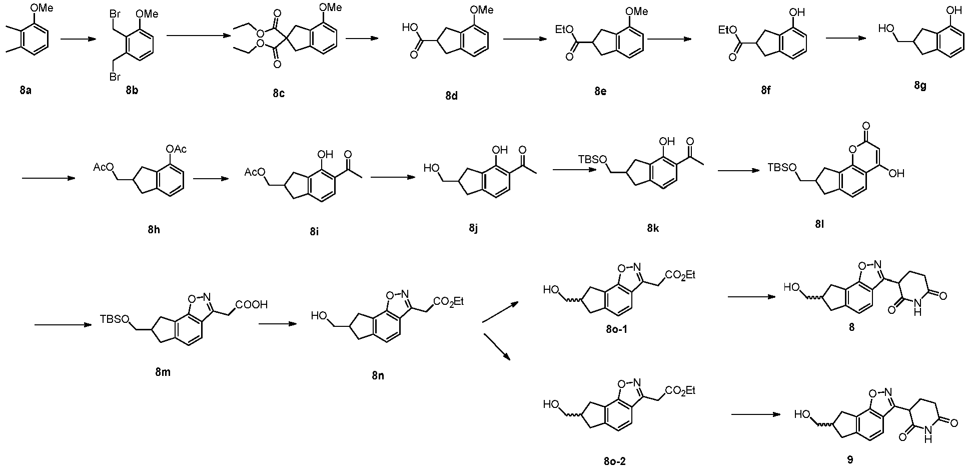

### Step 1: Preparation of intermediate 8b

CCl₄ (6750 mL), **8a** (450 g), 2,2-azobisisobutyronitrile (18.45 g), and N-bromosuccinimide (1194 g) were added to a reaction flask in sequence. The mixture was heated to 80 °C and reacted. After the reaction was completed, the reaction solution was filtered, and the filtrate was concentrated by evaporation under reduced pressure to remove the solvent. The residue was slurried with petroleum ether and filtered. The filter cake was collected to give intermediate **8b** (833 g).

¹H NMR (500 MHz, DMSO-d6) δ 7.34 (d, J = 8.1 Hz, 1H), 7.06 (ddd, J = 17.8, 8.1, 1.1 Hz, 2H), 4.77 (d, J = 9.5 Hz, 4H), 3.87 (s, 3H).

### Step 2: Preparation of intermediate 8c

60 wt% NaH (187 g) and THF (2000 mL) were added to a reaction flask in sequence, and diethyl malonate (300 g, 284 mL) was added under an ice bath. The mixture was stirred at room temperature for 30 min, and then **8b** (606 g) was added. The mixture was then stirred at room temperature for 2 h. After the reaction was completed, the reaction solution was slowly added dropwise to a saturated ammonium chloride solution to quench the reaction, and the mixture was extracted with petroleum ether (2000 mL) and water (2000 mL), then dried over anhydrous sodium sulfate, and filtered. The filtrate was concentrated by evaporation under reduced pressure to remove the solvent, and the residue was purified by silica gel column chromatography to give intermediate **8c** (262 g).

MS(ESI, [M-H]⁻) m/z: 291.2

¹H NMR (500 MHz, DMSO-d6) δ 7.16 (t, J = 7.8 Hz, 1H), 6.80 (dd, J = 15.3, 7.8 Hz, 2H), 4.14 (q, J = 7.1 Hz, 4H), 3.77 (s, 3H), 3.48 (s, 2H), 3.38 (s, 2H), 1.17 (t, J = 7.0 Hz, 6H).

### Step 3: Preparation of intermediate 8d

**8c** (130 g), DMSO (1000 mL), H₂O (300 mL), and lithium chloride (42.6 g) were added to a reaction flask, and the mixture was stirred at 180 °C. After the reaction was completed, the reaction solution was poured into ice water (1000 mL) to quench the reaction, and 1 M hydrochloric acid was added to adjust the pH to 2-3. The mixture was extracted with ethyl acetate, dried over anhydrous sodium sulfate, and filtered, and the filtrate was concentrated by evaporation under reduced pressure to remove the solvent, thus giving intermediate **8d** (191 g).

MS(ESI, [M+H]+) m/z: 193.05.

### Step 4: Preparation of intermediate 8e

**8d** (85 g), ethanol (1000 mL), and concentrated sulfuric acid (44 g) were added to a reaction flask in sequence, and the mixture was heated to 70 °C and reacted. After the reaction was completed, the reaction solution was cooled to room temperature and concentrated by evaporation under reduced pressure to remove the solvent. The residue was poured into ice water, and a saturated sodium bicarbonate aqueous solution was added for neutralization. Petroleum ether (1000 mL) was then added for extraction, and the organic phase was separated, dried over anhydrous sodium sulfate, and filtered. The filtrate was concentrated by evaporation under reduced pressure to remove the solvent, thus giving intermediate **8e** (99 g).

MS(ESI, [M+H]+) m/z: 221.1.

¹H NMR (500 MHz, DMSO-d6) δ 7.13 (t, J = 7.8 Hz, 1H), 6.81 (d, J = 7.5 Hz, 1H), 6.76 (d, J = 8.2 Hz, 1H), 4.09 (q, J = 7.1 Hz, 2H), 3.76 (s, 3H), 3.36 - 3.31 (m, 1H), 3.20 - 3.10 (m, 2H), 3.10 - 2.96 (m, 2H), 1.20 (t, J = 7.1 Hz, 3H).

### Step 5: Preparation of intermediate 8f

In a reaction flask, boron tribromide (415 g, 1657 mL) was added dropwise to a stirred solution of **8e** (150 g) in dichloromethane (750 mL) under N₂ atmosphere, and the mixture was reacted at 0 °C. After the reaction was completed, MeOH (500 mL) was added, and the mixture was then gradually warmed to room temperature and stirred. The reaction solution was then poured into a mixed solvent of ice water (1000 mL) and dichloromethane (1000 mL), and the mixture was stirred, separated by a separating funnel, washed with saturated brine, dried over anhydrous sodium sulfate, and filtered. The filtrate was concentrated under reduced pressure to remove the solvent, thus giving intermediate **8f** (122 g).

¹H NMR (500 MHz, DMSO-d6) δ 9.24 (s, 1H), 6.95 (t, J = 7.7 Hz, 1H), 6.70 - 6.60 (m, 1H), 6.62 - 6.52 (m, 1H), 4.10 (q, J = 7.1 Hz, 2H), 3.33 - 3.27 (m, 1H), 3.13 - 3.01 (m, 3H), 2.96 (dd, J = 16.1, 7.1 Hz, 1H), 1.20 (t, J = 7.1 Hz, 3H).

### Step 6: Synthesis of intermediate 8g

**8f** (130 g) and tetrahydrofuran (2000 mL) were added to a reaction flask in sequence at 0 °C under N₂ atmosphere, and a solution of lithium aluminum hydride in tetrahydrofuran (1 M, 438 mL) was slowly added dropwise. The mixture was reacted under an ice-water bath. After the reaction was completed, water (3 L) was slowly added dropwise to quench the reaction, and the mixture was adjusted to pH 1-2 with concentrated hydrochloric acid and extracted with ethyl acetate. The organic layer was dried over anhydrous sodium sulfate and filtered, and the filtrate was concentrated under reduced pressure to remove the solvent, thus giving intermediate **8g** (129 g).

¹H NMR (500 MHz, DMSO-d6) δ 6.88 (t, J = 7.7 Hz, 1H), 6.58 (d, J = 7.3 Hz, 1H), 6.52 (d, J = 7.9 Hz, 1H), 3.38 - 3.32 (m, 2H), 2.84 (ddd, J = 33.5, 16.2, 8.3 Hz, 2H), 2.60 (dq, J = 13.9, 8.1, 6.6 Hz, 1H), 2.55 - 2.49 (m, 3H).

### Step 7: Preparation of intermediate 8h

**8g** (120 g), 4-dimethylaminopyridine (7.14 g), dichloromethane (2000 mL), and triethylamine (177 g, 244 mL) were added to a reaction flask in sequence, and acetyl chloride (101 g, 91 mL) was slowly added dropwise at 0 °C. The mixture was reacted at room temperature. After the reaction was completed, the reaction solution was poured into a mixed solvent of dichloromethane (1000 mL) and water (1000 mL). The organic phase was separated, dried over anhydrous sodium sulfate, and filtered, and the filtrate was purified by silica gel column chromatography to give intermediate **8h** (127 g).

MS(ESI, [M+H]+) m/z: 249.3.

¹H NMR (500 MHz, DMSO-d6) δ 7.18 (t, J = 7.7 Hz, 1H), 7.11 (d, J = 7.4 Hz, 1H), 6.88 (d, J = 7.9 Hz, 1H), 4.07 - 3.96 (m, 2H), 3.11 - 3.00 (m, 1H), 2.87 (dd, J = 15.9, 7.8 Hz, 1H), 2.81 - 2.69 (m, 2H), 2.56 - 2.50 (m, 1H), 2.27 (s, 3H), 2.02 (s, 3H).

### Step 8: Preparation of intermediate 8i

**8h** (91 g), dichloromethane (2000 mL), and zirconium tetrachloride (342 g) were added to a reaction flask in sequence, and the mixture was stirred at 50 °C under N₂ atmosphere overnight. After the reaction was completed, the reaction solution was cooled to room temperature and then poured into a mixed solvent of ice water (1000 mL) and dichloromethane (1000 mL). The organic phase was separated, and the aqueous phase was extracted with dichloromethane. The organic phases were combined, dried over anhydrous sodium sulfate, and filtered, and the filtrate was purified by silica gel column chromatography to give intermediate **8i** (90 g).

MS(ESI, [M-H]⁻) m/z: 247.2.

### Step 9: Preparation of intermediate 8j

**8i** (95 g) and ethanol (900 mL) were added to a reaction flask in sequence, and a solution of sodium hydroxide (77 g) in H₂O (800 mL) was added dropwise under an ice bath. The mixture was reacted at room temperature under N₂ atmosphere. After the reaction was completed, the reaction solution was diluted with ethyl acetate (2 L) and water (1 L), and 3 M hydrochloric acid was slowly added to adjust the pH to 3, followed by liquid separation. The aqueous layer was extracted with ethyl acetate. The organic phases were combined, washed with saturated brine, dried over anhydrous sodium sulfate, and filtered, and the filtrate was concentrated by evaporation under reduced pressure to remove the solvent, thus giving intermediate **8j** (86 g).

MS(ESI, [M-H]⁻) m/z: 205.1.

¹H NMR (500 MHz, DMSO-d6) δ 12.34 (s, 1H), 7.73 (d, J = 8.0 Hz, 1H), 6.84 (d, J = 8.0 Hz, 1H), 4.68 (t, J = 5.3 Hz, 1H), 3.36 (ddd, J = 7.0, 5.2, 2.0 Hz, 2H), 2.98 (dd, J = 17.0, 8.2 Hz, 1H), 2.92 - 2.82 (m, 1H), 2.72 (dd, J = 16.9, 5.6 Hz, 1H), 2.61 (s, 3H), 2.61 - 2.53 (m, 2H).

### Step 10: Preparation of intermediate 8k

**8j** (37 g), 1,2-dichloroethane (700 mL), imidazole (36.6 g), and tert-butyldimethylsilyl chloride (29.7 g) were added to a reaction flask in sequence, and the mixture was reacted at 75 °C. After the reaction was completed, the reaction solution was cooled to room temperature, and dichloromethane (1000 mL) and water (1000 mL) were added. The organic phase was separated, washed with saturated brine, dried over anhydrous sodium sulfate, and filtered, and the filtrate was concentrated by evaporation under reduced pressure to remove the solvent, thus giving intermediate **8k** (60 g).

¹H NMR (500 MHz, DMSO-d6) δ 12.31 (s, 1H), 7.71 (d, J = 8.0 Hz, 1H), 6.82 (d, J = 8.0 Hz, 1H), 3.52 (d, J = 6.4 Hz, 2H), 2.97 (dd, J = 16.9, 8.0 Hz, 1H), 2.85 (dd, J = 15.4, 7.5 Hz, 1H), 2.69 (dd, J = 16.9, 5.6 Hz, 1H), 2.64 - 2.59 (m, 1H), 2.58 (s, 3H), 2.55 (d, J = 5.6 Hz, 1H), 0.82 (s, 9H), 0.00 (s, 6H).

### Step 11: Preparation of intermediate 8l

**8k** (55 g), diethyl carbonate (101 g, 103 mL), and toluene (1000 mL) were added to a reaction flask in sequence. The reaction solution was cooled to 0 °C, and 60 wt% sodium hydride (34.3 g, 858 mmol) was added in portions. After the addition, the mixture was slowly heated to 120 °C and reacted. After the reaction was completed, the reaction solution was slowly poured into ice water (2000 mL), and the mixture was extracted with ethyl acetate. The aqueous phase was adjusted to pH = 3 with 3 N hydrochloric acid and extracted with ethyl acetate, and the organic phases were combined, dried over anhydrous sodium sulfate, filtered, and concentrated by evaporation under reduced pressure to remove the solvent, thus giving intermediate **8l** (53.6 g).

MS(ESI, [M-H]⁻) m/z: 345.1.

¹H NMR (500 MHz, DMSO-d6) δ 12.31 (s, 1H), 7.57 (d, J = 7.9 Hz, 1H), 7.16 (d, J = 7.9 Hz, 1H), 5.49 (s, 1H), 3.56 (d, J = 6.4 Hz, 2H), 3.04 (ddd, J = 16.0, 13.3, 8.1 Hz, 2H), 2.75 (td, J = 14.8, 13.3, 4.4 Hz, 2H), 2.71 - 2.63 (m, 1H), 0.81 (s, 9H), 0.00 (s, 6H).

### Step 12: Preparation of intermediate 8m

**8l** (51 g), hydroxylamine hydrochloride (61.4 g), sodium ethoxide (61.1 g), and ethanol (2000 mL) were added to a reaction flask in sequence, and the mixture was heated to 85 °C and reacted under N₂ atmosphere. After the reaction was completed, the reaction solution was concentrated under reduced pressure to remove the solvent, and water (2 L) was added. The mixture was adjusted to pH = 8-9 with a saturated sodium carbonate solution and extracted with ethyl acetate. The aqueous phase was collected, adjusted to pH = 6-7 with 1 M hydrochloric acid, and extracted with ethyl acetate. The organic phase was collected, washed with saturated brine, dried over anhydrous sodium sulfate, and filtered, and the filtrate was concentrated under reduced pressure to remove the solvent, thus giving intermediate **8m** (47 g).

MS(ESI, [M-H]⁻) m/z: 360.2.

¹H NMR (500 MHz, DMSO-d6) δ 7.55 (d, J = 8.0 Hz, 1H), 7.22 (d, J = 8.0 Hz, 1H), 3.97 (s, 2H), 3.57 (d, J = 6.6 Hz, 2H), 3.16 - 3.07 (m, 2H), 2.88 - 2.72 (m, 3H), 0.81 (s, 9H), 0.00 (s, 6H).

### Step 13: Preparation of intermediate 8n

**8m** (47 g), ethanol (1500 mL), and concentrated sulfuric acid (65.1 g, 35.4 mL) were added to a reaction flask in sequence, and the mixture was heated to 85 °C and reacted under N₂ atmosphere. The reaction solution was cooled to room temperature and concentrated by rotary evaporation to remove the solvent. Dichloromethane (1000 mL) was added, and a saturated sodium bicarbonate aqueous solution was added dropwise for neutralization. The mixture was washed with saturated brine, dried over anhydrous sodium sulfate, and filtered, and the filtrate was concentrated by evaporation under reduced pressure to remove the solvent, thus giving intermediate **8n** (43 g).

MS (ESI, [M+H]⁺) m/z: 276.1.

¹H NMR (500 MHz, DMSO-d6) δ 7.58 (d, J = 8.0 Hz, 1H), 7.27 (d, J = 8.0 Hz, 1H), 4.74 (q, J = 4.9 Hz, 1H), 4.16 (s, 2H), 4.12 (t, J = 7.1 Hz, 2H), 3.43 (dd, J = 6.8, 5.3 Hz, 2H), 3.15 (ddd, J = 29.7, 16.4, 8.3 Hz, 2H), 2.93 - 2.81 (m, 2H), 2.79 - 2.70 (m, 1H), 1.19 (t, J = 7.1 Hz, 3H).

### Step 14: Preparation of intermediates 8o-1 and 8o-2

Preparative resolution: Intermediate **8n** (43 g) was dissolved in a solution of dichloromethane in ethanol (430 mL) to achieve a concentration of about 100.0 mg/mL. The mixture was filtered through a 0.45 µm organic filter membrane, and the filtrate was collected. Instrument: YMC high pressure preparative chromatograph; chromatographic column: CHIRALPAK IG (Innovation 036#, 30 × 250 mm, S-10 µm); mobile phases: A: ethanol, B: *n*-hexane. The prepeak gave intermediate **8o-1** (9.057 g) and the postpeak gave intermediate **8o-2** (8.833 g).

**80-1**:MS(ESI, [M+H]⁺) m/z: 276.1.

**8o-2:**MS(ESI, [M+H]⁺) m/z: 276.1.

### Step 15: Preparation of intermediate 8

**8o-1** (11.93 g), THF (200 mL), and acrylamide (3.39 g) were added to a reaction flask in sequence under N₂ atmosphere. After the mixture was cooled to 0 °C, a solution of potassium *tert*-butoxide in tetrahydrofuran (1 M, 34.7 mL) was added. The mixture was reacted at 0 °C. After the reaction was completed, the reaction solution was added dropwise to an icy saturated ammonium chloride aqueous solution, and ethyl acetate (1000 mL) was added for extraction. The aqueous phase was extracted with ethyl acetate (500 mL). The organic phases were then combined, washed with saturated brine, dried over anhydrous sodium sulfate, and filtered, and the filtrate was concentrated by evaporation under reduced pressure to remove the solvent and filtered. The filter cake was collected to give intermediate **8** (6.77 g).

¹H NMR (500 MHz, DMSO-d6) δ 11.08 (s, 1H), 7.60 (d, J = 8.0 Hz, 1H), 7.25 (d, J = 8.1 Hz, 1H), 4.74 (td, J = 5.3, 2.2 Hz, 1H), 4.56 (dd, J = 11.8, 5.0 Hz, 1H), 3.43 (dd, J = 6.8, 5.3 Hz, 2H), 3.22 - 3.09 (m, 2H), 2.94 - 2.83 (m, 2H), 2.76 (dq, J = 16.9, 6.3 Hz, 2H), 2.60 (dt, J = 17.3, 4.2 Hz, 1H), 2.50 - 2.44 (m, 1H), 2.23 - 2.13 (m, 1H). Step 16: Synthesis of intermediate **9**

**8o-2** (12.83 g), THF (200 mL), and acrylamide (3.64 g) were added to a reaction flask in sequence. After the mixture was cooled to 0 °C, a solution of potassium *tert*-butoxide in tetrahydrofuran (1 M, 37.3 mL) was added. The mixture was reacted at 0 °C under N₂ atmosphere. After the reaction was completed, the reaction solution was added dropwise to an icy saturated ammonium chloride aqueous solution, and ethyl acetate was added for extraction. The aqueous phase was extracted with ethyl acetate. The organic phases were then combined, washed with saturated brine, dried over anhydrous sodium sulfate, and filtered, and the filtrate was concentrated by evaporation under reduced pressure to remove the solvent and filtered. The filter cake was collected to give intermediate 9 (7.454 g).

¹H NMR (500 MHz, DMSO-d6) δ 11.08 (s, 1H), 7.60 (d, J = 8.2 Hz, 1H), 7.25 (d, J = 8.1 Hz, 1H), 4.74 (td, J = 5.3, 2.2 Hz, 1H), 4.56 (dd, J = 11.8, 5.0 Hz, 1H), 3.43 (dd, J = 6.8, 5.2 Hz, 2H), 3.21 - 3.09 (m, 2H), 2.94 - 2.83 (m, 2H), 2.80 - 2.71 (m, 2H), 2.60 (dt, J = 17.3, 4.2 Hz, 1H), 2.46 (dd, J = 12.1, 4.5 Hz, 1H), 2.23 - 2.15 (m, 1H).

### Example 10: Synthesis of Intermediate 10

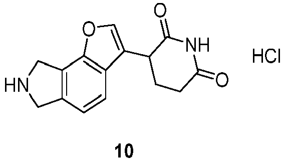

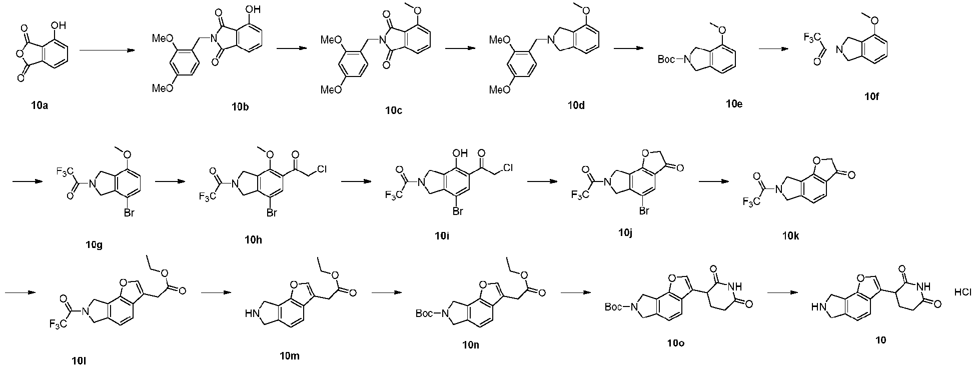

### Step 1: Preparation of intermediate 10b

**10a** (100 g), 2,4-dimethoxybenzylamine (102 g), and acetic acid (600 mL) were added to a reaction flask in sequence, and the mixture was reacted at 80 °C. After the reaction was completed, water was added to the reaction solution, and the mixture was filtered under vacuum. The filter cake was washed with water and dried to give intermediate 10b (95.7 g).

MS (ESI, [M-H]⁻) *m*/*z*: 312.02*.*

¹H NMR (500 MHz, DMSO-*d*₆) δ 11.03 (s, 1H), 7.62 (dd, *J* = 8.4, 7.1 Hz, 1H), 7.29 (d, *J* = 7.1 Hz, 1H), 7.22 (d, *J* = 8.4 Hz, 1H), 6.90 (d, *J* = 8.4 Hz, 1H), 6.56 (d, *J* = 2.4 Hz, 1H), 6.43 (dd, *J* = 8.4, 2.4 Hz, 1H), 4.60 (s, 2H), 3.80 (s, 3H), 3.73 (s, 3H).

### Step 2: Preparation of intermediate 10c

Intermediate **10b** (130 g), potassium carbonate (97 g), iodomethane (65 mL), and N,N-dimethylformamide (1 L) were added to a reaction flask in sequence, and the mixture was reacted at 80 °C. After the reaction was completed, the reaction solution was cooled to room temperature, and water was added thereto. The mixture was filtered under vacuum, and the filter cake was washed with water and dried to give intermediate **10c** (126 g).

MS (ESI, [M+H]⁺) *m*/*z*: 327.99.

¹H NMR (500 MHz, DMSO-*d*6) δ 7.80 (dd, *J* = 8.5, 7.2 Hz, 1H), 7.48 (d, *J* = 8.4 Hz, 1H), 7.45 - 7.39 (m, 1H), 6.91 (d, *J =* 8.5 Hz, 1H), 6.56 (d, *J* = 2.4 Hz, 1H), 6.43 (dd, *J* = 8.4, 2.4 Hz, 1H), 4.60 (s, 2H), 3.95 (s, 3H), 3.79 (s, 3H), 3.72 (s, 3H).

### Step 3: Preparation of intermediate 10d

Intermediate **10c** (128 g) and tetrahydrofuran (800 mL) were added to a reaction flask in sequence, and lithium aluminum hydride (89 g) was slowly added at 0 °C under N₂ atmosphere. The mixture was warmed to 80 °C and reacted. After the reaction was completed, water (89 mL) was slowly added dropwise to the reaction solution under an ice bath, and a 15% NaOH aqueous solution (267 mL) was then added, followed by the addition of water (89 mL). The mixture was stirred for 0.5 h and filtered through diatomite under vacuum, and the filter cake was washed with ethyl acetate. The filtrate was collected, and the organic layer was separated, dried over anhydrous sodium sulfate, and filtered. The filtrate was concentrated to give intermediate **10d** (86.4 g).

MS (ESI, [M+H]⁺) *m*/*z*: 300.20*.*

¹H NMR (500 MHz, DMSO-d6) δ 7.26 (d, *J* = 8.3 Hz, 1H), 7.18 (t, *J* = 7.8 Hz, 1H), 6.90 - 6.77 (m, 2H), 6.57 (d, *J* = 2.4 Hz, 1H), 6.52 (dd, *J* = 8.3, 2.4 Hz, 1H), 3.92 (s, 2H), 3.89 - 3.80 (m, 4H), 3.78 (s, 3H), 3.76 (d, *J* = 3.6 Hz, 6H).

### Step 4: Preparation of intermediate 10e

Intermediate **10d** (86 g), 10% palladium on carbon (15 g), methanol (500 mL), and di-*tert*-butyl dicarbonate (63.3 g) were added to a reaction flask in sequence, and the mixture was reacted at 0 °C under hydrogen atmosphere at room temperature. After the reaction was completed, the reaction solution was filtered, and the filtrate was concentrated to give intermediate **10e** (100 g).

¹H NMR (500 MHz, DMSO) δ 7.27 (t, *J* = 7.9 Hz, 1H), 6.93 - 6.86 (m, 2H), 4.60 - 4.53 (m, 2H), 4.50 - 4.43 (m, 2H), 3.80 (d, *J* = 1.4 Hz, 3H), 1.45 (s, 9H).

### Step 5: Preparation of intermediate 10f

Intermediate **10e** (100 g), dichloromethane (1000 mL), and trifluoroacetic acid (457 g) were added to a reaction flask in sequence, and the mixture was reacted at room temperature for 2 h. After the reaction was completed, the reaction solution was directly concentrated, and the concentrate was dissolved in tetrahydrofuran (1000 mL). Trifluoroacetic anhydride (84 g) was then added under an ice-water bath, and the mixture was reacted at room temperature. After the reaction was completed, the reaction solution was slowly added to a saturated sodium bicarbonate solution to quench the reaction, and then ethyl acetate was added for extraction. The organic phases were combined, dried over anhydrous sodium sulfate, and filtered, and the filtrate was concentrated to give intermediate **10f** (57.4 g).

### Step 6: Preparation of intermediate 10g

Intermediate **10f** (40 g) and acetonitrile (500 mL) were added to a reaction flask in sequence. After dissolution, N-bromosuccinimide (30.5 g) was added, and the mixture was reacted at 75 °C. After the reaction was completed, ice water was added to the reaction solution to quench the reaction, and the mixture was filtered under vacuum. The filter cake was washed with water and dried to give intermediate **10g** (60 g).

¹H NMR (500 MHz, DMSO-d6) δ 7.51 (dd, *J =* 8.7, 1.5 Hz, 1H), 6.96 (d, *J =* 8.6 Hz, 1H), 4.95 (d, *J =* 29.1 Hz, 2H), 4.76 (d, *J* = 29.7 Hz, 2H), 3.83 (d, *J* = 2.4 Hz, 3H).

### Step 7: Preparation of intermediate 10h

**10g** (40 g) and chloroacetyl chloride (30 mL) were added to a reaction flask in sequence, and trifluoromethanesulfonic acid (120 mL) was slowly added dropwise under an ice-water bath. The mixture was reacted at 50 °C. After the reaction was completed, the reaction solution was slowly added dropwise to ice water, and then the mixture was extracted twice with dichloromethane. The organic phases were combined and then washed twice with a saturated sodium bicarbonate aqueous solution. The organic layer was separated, dried over anhydrous sodium sulfate, and filtered. The filtrate was concentrated, and then the residue was purified by silica gel column chromatography to give the target intermediate **10h** (20 g).

¹H NMR (500 MHz, DMSO-d6) δ 7.84 (d, *J* = 8.8 Hz, 1H), 5.31 (s, 1H), 5.15 (s, 1H), 5.01 (d, *J* = 2.4 Hz, 2H), 4.99 (s, 1H), 4.79 (s, 1H), 3.93 (d, *J* = 19.4 Hz, 3H).

### Step 8: Preparation of intermediate 10i

**10h** (20 g) and dichloromethane (300 mL) were added to a reaction flask in sequence, and boron trichloride (149 mL) was slowly added dropwise under an ice-water bath. The mixture was reacted at room temperature. After the reaction was completed, the reaction solution was slowly added to ice water, and then the mixture was extracted twice with dichloromethane. The organic phases were combined, dried over anhydrous sodium sulfate, and filtered, and the filtrate was concentrated to give the target intermediate **10i** (20 g).

MS (ESI, [M+H]⁺) *m*/*z*: 386.00*.*

¹H NMR (500 MHz, DMSO-d6) δ 11.37 (s, 1H), 7.98 (d, *J =* 2.5 Hz, 1H),5.18 (s, 2H),5.09 (s, 1H), 5.00 (s, 1H), 4.89 (s, 1H),4.80 (s, 1H).

### Step 9: Preparation of intermediate 10j

**10i** (19.5 g), sodium bicarbonate (12.7 g), and acetonitrile (900 mL) were added to a reaction flask in sequence, and the mixture was reacted at 80 °C. After the reaction was completed, water and ethyl acetate were added. The organic layer was collected, dried over anhydrous sodium sulfate, and filtered, and the filtrate was concentrated to give the target intermediate **10j** (24 g).

¹H NMR (500 MHz, DMSO-d6) δ 7.87 (s, 1H), 5.21 (d, *J=* 2.4 Hz, 1H), 5.06 (s, 1H), 4.99 (d, *J=* 2.1 Hz, 1H), 4.97 (d, *J* = 3.1 Hz, 2H), 4.85 (s, 1H).

### Step 10: Preparation of intermediate 10k

**10j** (23.4 g), 10% palladium on carbon (1.4 g), sodium bicarbonate (5.61 g), and ethanol (400 mL) were added to a reaction flask in sequence, and the mixture was reacted at room temperature under hydrogen atmosphere. After the reaction was completed, the reaction solution was filtered, and the filtrate was concentrated by evaporation under reduced pressure to remove the solvent. Ethyl acetate and water were added to the residue, and the organic layer was separated, dried over anhydrous sodium sulfate, and filtered. The filtrate was concentrated, and then the residue was purified by silica gel column chromatography to give the target intermediate **10k** (12 g).

MS (ESI, [M-H]⁻) *m*/*z*: 270.00*.*

¹H NMR (500 MHz, DMSO-*d*6) δ 7.65 (dd, *J* = 7.9, 2.5 Hz, 1H), 7.20 (dd, *J* = 12.0, 7.9 Hz, 1H), 5.14 (s, 1H), 5.10 (s, 1H), 4.94 (s, 1H), 4.92 - 4.88 (m, 3H).

### Step 11: Preparation of intermediate 10l

**10k** (11.5 g), (carbethoxymethylene)triphenylphosphorane (22.2 g), and toluene (30 mL) were added to a reaction flask in sequence, and the mixture was reacted at 120 °C under N₂ atmosphere. After the reaction was completed, ethyl acetate and water were added to the reaction system. The organic layer was separated, dried over anhydrous sodium sulfate, and filtered. The filtrate was concentrated, and then the residue was purified by silica gel column chromatography to give the target intermediate **10l** (5.3 g).

¹H NMR (500 MHz, DMSO-d6) δ 7.96 (d, *J* = 2.9 Hz, 1H), 7.60 (dd, *J* = 8.0, 3.3 Hz, 1H), 7.33 - 7.27 (m, 1H), 5.27 (s, 1H), 5.15 (s, 1H), 5.07 (s, 1H), 4.96 (s, 1H), 4.11 (q, *J* = 7.1 Hz, 2H), 3.81 (d, *J* = 1.0 Hz, 2H), 1.19 (t, *J* = 7.1 Hz, 3H).

### Step 12: Preparation of intermediate 10m

**10l** (5.3 g), potassium carbonate (6.4 g), and ethanol (50 mL) were added to a reaction flask in sequence, and the mixture was reacted at room temperature. After the reaction was completed, ethyl acetate and water were added to the reaction system. The organic layer was separated, dried over anhydrous sodium sulfate, and filtered, and the filtrate was concentrated to give the target intermediate **10m** (4.3 g).

MS(ESI, [M+H]⁺) m/z: 246.10.

### Step 13: Preparation of intermediate 10n

**10m** (4.3 g), triethylamine (3.47 g), and dichloromethane (50 mL) were added to a reaction flask in sequence, then di-*tert*-butyl dicarbonate (4.11 g) was added, and the mixture was reacted at room temperature. After the reaction was completed, water was added to the reaction system. The organic layer was separated, dried over anhydrous sodium sulfate, and filtered. The filtrate was concentrated, and then the residue was purified by silica gel column chromatography to give the target intermediate **10n** (3.7 g).

¹H NMR (500 MHz, DMSO-d6) δ7.93 (s, 1H), 7.53 (d, *J* = 7.9 Hz, 1H), 7.23 (t, *J* = 7.6 Hz, 1H), 4.80 (dd, *J* = 12.9, 2.4 Hz, 2H), 4.73 - 4.66 (m, 2H), 4.11 (q, *J* = 7.1 Hz, 2H), 3.79 (s, 2H), 1.48 (s, 9H), 1.19 (t, *J* = 7.1 Hz, 3H). Step 14: Preparation of intermediate **10o**

**10n** (3.7 g), acrylamide (0.84 g), and *N,N-*dimethylformamide (50 mL) were added to a reaction flask in sequence, and the mixture was cooled to 0 °C. Potassium *tert*-butoxide (8.6 mL, 1 M) was slowly added dropwise, and the mixture was reacted at 0 °C. After the reaction was completed, the reaction solution was added dropwise to an icy ammonium chloride aqueous solution, and ethyl acetate was added for extraction. The organic layer was separated, dried over anhydrous sodium sulfate, and filtered. The filtrate was concentrated, and then the residue was purified by silica gel column chromatography to give the target intermediate **10o** (2.6 g).

¹H NMR (500 MHz, DMSO-d6) δ10.90 (s, 1H), 7.91 (s, 1H), 7.53 (dd, *J =* 8.0, 1.9 Hz, 1H), 7.22 (t, *J =* 7.9 Hz, 1H), 4.86 - 4.77 (m, 2H), 4.69 (d, *J* = 11.8 Hz, 2H), 4.15 (dd, *J* = 12.1, 5.0 Hz, 1H), 2.77 - 2.69 (m, 1H), 2.58 (dt, *J=* 17.4, 4.0 Hz, 1H), 2.33 (qd, *J* = 12.5, 4.4 Hz, 1H), 2.15 - 2.07 (m, 1H), 1.48 (d, *J* = 2.6 Hz, 9H).

### Step 15: Preparation of intermediate 10

**10o** (2.4 g) and dioxane (15 mL) were added to a reaction flask in sequence, then a solution of hydrochloric acid in dioxane (15 mL, 4 M) was added, and the mixture was reacted at room temperature. After the reaction was completed, methyl *tert-*butyl ether was added to the reaction system, and the mixture was filtered under vacuum. The filter cake was washed with methyl *tert-*butyl ether, collected, and dried to give the target intermediate **10** (2.0 g).

MS(ESI, [M+H]⁺) m/z: 271.15.

¹H NMR (500 MHz, DMSO-*d*6) δ10.92 (s, 1H), 10.16 (s, 2H), 7.99 (s, 1H), 7.61 (d, *J* = 8.0 Hz, 1H), 7.29 (d, *J* = 8.1 Hz, 1H), 4.75 (d, *J* = 5.0 Hz, 2H), 4.61 (d, *J* = 5.1 Hz, 2H), 4.18 (dd, *J* = 12.2, 4.9 Hz, 1H), 2.80 - 2.71 (m, 1H), 2.58 (dt, *J* = 17.3, 4.0 Hz, 1H), 2.34 (qd, *J* = 12.6, 4.4 Hz, 1H), 2.11 (dtd, *J* = 13.2, 5.2, 3.4 Hz, 1H).

### Example 11: Synthesis of Intermediate 11

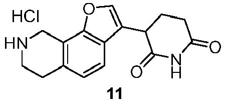

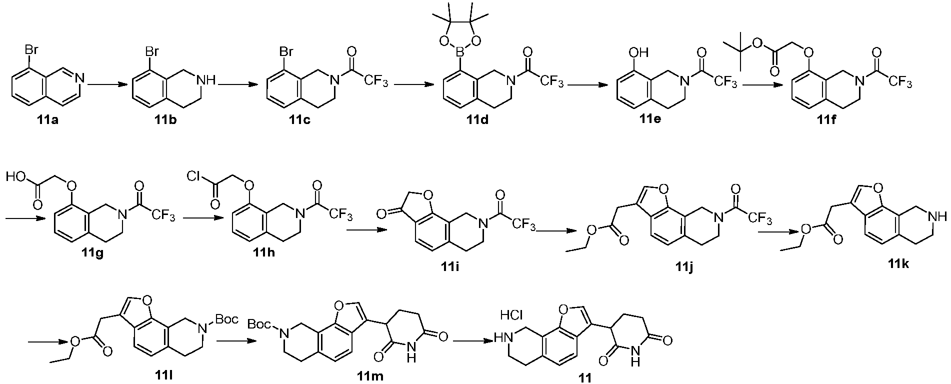

### Step 1: Preparation of intermediate 11b

Intermediate **11a** and methanol (1500 mL) were added to a reaction flask in sequence, then sodium cyanoborohydride (148 g) was added at 0 °C, and the mixture was reacted at 0 °C for 10 min. Boron trifluoride diethyl etherate (334 g) was added dropwise at 0 °C, and the mixture was warmed to 75 °C and reacted. After the reaction was completed, a saturated sodium bicarbonate solution (50 mL) was added to the reaction solution. The mixture was concentrated by evaporation under reduced pressure to remove the solvent, and dichloromethane and water were added for extraction. The organic phase was separated, dried over anhydrous sodium sulfate, and filtered, and the filtrate was concentrated by evaporation under reduced pressure to remove the solvent, thus giving the target intermediate **11b** (102.65 g).

MS(ESI, [M+H]⁺) m/z: 212.2.

¹H NMR (500 MHz, DMSO-d6) δ 7.38 (dd, J = 7.7, 1.4 Hz, 1H), 7.08 (dd, J = 16.0, 7.5 Hz, 2H), 3.74 (s, 2H), 2.89 (t, J = 5.8 Hz, 2H), 2.68 (t, J = 5.8 Hz, 2H).

### Step 2: Preparation of intermediate 11c

Intermediate **11b** (100.06 g) and tetrahydrofuran (1000 mL) were added to a reaction flask in sequence, and trifluoroacetic anhydride (95 g, 63.2 mL) was added under an ice bath. The mixture was warmed to room temperature and reacted. After the reaction was completed, the reaction solution was extracted with ethyl acetate (1000 mL) and water (2000 mL). The organic phase was separated, dried over anhydrous sodium sulfate, and filtered, and the filtrate was concentrated by evaporation under reduced pressure to remove the solvent, thus giving the target intermediate **11c** (137.6 g).

¹H NMR (500 MHz, DMSO-d6) δ 7.54 (dd, J = 7.9, 1.4 Hz, 1H), 7.26 (q, J = 7.0, 5.9 Hz, 1H), 7.23 - 7.18 (m, 1H), 4.71 (d, J = 27.7 Hz, 2H), 3.82 (t, J = 6.0 Hz, 2H), 2.95 (dt, J = 13.6, 6.0 Hz, 2H).

### Step 3: Preparation of intermediate 11d

Intermediate **11c** (137.6 g), [1,1'-bis(diphenylphosphino)ferrocene]palladium dichloride dichloromethane complex (36.5 g), bis(pinacolato)diboron (136 g), potassium acetate (131 g), and dioxane (1500 mL) were added to a reaction flask in sequence, and the mixture was heated to 85 °C and reacted under N₂ atmosphere. After the reaction was completed, the reaction solution was filtered, and the filtrate was concentrated by evaporation under reduced pressure to remove the solvent. The residue was slurried with petroleum ether and filtered, and the filtrate was extracted with water (2 L). The organic phase was dried over anhydrous sodium sulfate and filtered, and the filtrate was concentrated by evaporation under reduced pressure to remove the solvent, thus giving the target intermediate **11d** (165.4 g).

MS(ESI, [M+H]⁺) m/z: 356.2.

¹H NMR (500 MHz, DMSO-d6) δ 7.59 (ddd, J = 7.4, 3.7, 1.4 Hz, 1H), 7.33 (ddd, J = 17.9, 7.6, 1.4 Hz, 1H), 7.29 - 7.22 (m, 1H), 5.03 (d, J = 38.6 Hz, 2H), 3.80 (t, J = 6.2 Hz, 1H), 3.75 (t, J = 6.4 Hz, 1H), 2.94 (t, J = 6.3 Hz, 2H), 1.31 (d, J = 4.3 Hz, 12H).

### Step 4: Preparation of intermediate 11e

Intermediate **11d** (165.4 g), tetrahydrofuran (1000 mL), and acetic acid (98 g, 93 mL) were added to a reaction flask in sequence, and 30% hydrogen peroxide (185 g, 166 mL) was added under an ice bath. The mixture was warmed to room temperature and reacted. After the reaction was completed, the reaction solution was poured into an icy saturated sodium thiosulfate solution to quench the reaction, the mixture was adjusted to pH 8 with a saturated sodium bicarbonate solution, and ethyl acetate was added for extraction. The organic phase was separated, dried over anhydrous sodium sulfate, and filtered, and the filtrate was concentrated by evaporation under reduced pressure to remove the solvent. The residue was slurried with petroleum ether/methyl *tert-*butyl ether (200 mL) and filtered, and the filter cake was collected to give the target intermediate **11e** (85.3 g).

MS(ESI, [M-H]⁻) m/z: 244.2.

¹H NMR (500 MHz, DMSO-d6) δ 9.80 (d, J = 25.4 Hz, 1H), 7.03 (q, J = 7.9 Hz, 1H), 6.71 (dd, J = 8.0, 3.1 Hz, 1H), 6.64 (t, J = 6.9 Hz, 1H), 4.61 (d, J = 23.4 Hz, 2H), 3.81 - 3.75 (m, 2H), 2.84 (dt, J = 16.8, 5.9 Hz, 2H).

### Step 5: Preparation of intermediate 11f

Intermediate **11e** (30 g), acetonitrile (300 mL), potassium carbonate (33.8 g), and *tert-*butyl bromoacetate (26.3 g, 19.89 mL) were added to a reaction flask in sequence, and the mixture was heated to 80 °C and reacted. After the reaction was completed, the reaction solution was extracted with ethyl acetate (500 mL) and water (1000 mL). The organic phase was separated, dried over anhydrous sodium sulfate, filtered, and concentrated by evaporation under reduced pressure to remove the solvent, thus giving the target intermediate **11f** (47.4 g).

¹H NMR (500 MHz, DMSO-d6) δ 7.18 (q, J = 8.2 Hz, 1H), 6.83 (t, J = 6.9 Hz, 1H), 6.78 (dd, J = 8.3, 2.8 Hz, 1H), 4.75 - 4.66 (m, 4H), 3.83 - 3.77 (m, 2H), 2.89 (dt, J = 17.6, 6.0 Hz, 2H), 1.43 - 1.41 (m, 9H).

### Step 6: Preparation of intermediate 11g

Intermediate **11f** (47.4 g), dichloromethane (500 mL), and trifluoroacetic acid (207 g, 140 mL) were added to a reaction flask in sequence, and the mixture was reacted at room temperature for 1 h. After the reaction was completed, the reaction solution was concentrated by evaporation under reduced pressure to remove the solvent, and dichloromethane was added to the residue. The mixture was then concentrated by evaporation under reduced pressure to remove the solvent, thus giving the target intermediate **11g** (34.2 g).

MS(ESI, [M-H]⁻) m/z: 302.0.

¹H NMR (500 MHz, DMSO-d6) δ 13.04 (s, 1H), 7.18 (q, J = 7.9 Hz, 1H), 6.85 - 6.75 (m, 2H), 4.78 - 4.66 (m, 4H), 3.81 (q, J = 5.6 Hz, 2H), 2.88 (dt, J = 18.5, 5.9 Hz, 2H).

### Step 7: Preparation of intermediates 11h and 11i

Intermediate **11g** (33.7 g), tetrahydrofuran (350 mL), and thionyl chloride (39.7 g) were added to a reaction flask in sequence, and the mixture was heated to 75 °C and reacted. After the reaction was completed, the reaction solution was cooled to room temperature and concentrated by evaporation under reduced pressure to remove the solvent, thus giving the target intermediate **11h.** Intermediate **11h** was added to dichloromethane (350 mL) and aluminum trichloride (39.7 g), and the mixture was stirred at room temperature. After the reaction was completed, the reaction solution was poured into ice water (500 mL) to quench the reaction, and the mixture was filtered through diatomite.

The filtrate was extracted with dichloromethane (100 mL) and water (100 mL), and the organic phase was separated, dried over anhydrous sodium sulfate, filtered, and concentrated by evaporation under reduced pressure to remove the solvent, thus giving the target intermediate **11i** (32.5 g).

¹H NMR (500 MHz, DMSO-d6) δ 7.50 (dd, J = 9.6, 7.9 Hz, 1H), 7.02 (dd, J = 8.0, 5.7 Hz, 1H), 4.87 (s, 2H), 4.78 (d, J = 21.1 Hz, 2H), 3.88 (td, J = 5.9, 2.5 Hz, 2H), 3.02 (dt, J = 13.1, 5.8 Hz, 2H).

### Step 8: Preparation of intermediate 11j

Intermediate **11i** (32.5 g), (carbethoxymethylene)triphenylphosphorane (54.7 g), and toluene (350 mL) were added to a reaction flask in sequence, and the mixture was heated to 120 °C and reacted under N₂ atmosphere. After the reaction was completed, the reaction solution was extracted with ethyl acetate (300 mL) and water (800 mL). The organic phase was dried over anhydrous sodium sulfate and filtered, and the filtrate was purified by a silica gel column to give the target intermediate **11j** (19.37 g).

¹H NMR (500 MHz, DMSO-d6) δ 7.92 (s, 1H), 7.47 (t, J = 8.2 Hz, 1H), 7.12 (dd, J = 8.0, 5.9 Hz, 1H), 4.99 (d, J = 22.7 Hz, 2H), 4.11 (q, J = 7.1 Hz, 2H), 3.93 - 3.87 (m, 2H), 3.78 (d, J = 1.0 Hz, 2H), 3.02 (dt, J = 13.4, 5.9 Hz, 2H), 1.19 (td, J = 7.1, 1.3 Hz, 3H).

### Step 9: Preparation of intermediate 11k

Intermediate **11j,** potassium carbonate (22.60 g, 164 mmol), and ethanol (200 mL) were added to a reaction flask in sequence, and the mixture was reacted at room temperature. After the reaction was completed, the reaction solution was extracted with ethyl acetate (300 mL) and water (500 mL). The organic phase was separated, and the aqueous phase was extracted with ethyl acetate. The organic phases were combined, washed with saturated brine, dried over anhydrous sodium sulfate, and filtered, and the filtrate was concentrated by evaporation under reduced pressure to remove the solvent, thus giving the target intermediate **11k** (13.12 g).

MS(ESI, [M+H]⁺) m/z: 260.1.

¹H NMR (500 MHz, DMSO-d6) δ 7.81 (s, 1H), 7.32 (d, J = 8.0 Hz, 1H), 6.97 (d, J = 7.9 Hz, 1H), 4.10 (q, J = 7.1 Hz, 2H), 4.05 (s, 2H), 3.73 (d, J = 1.0 Hz, 2H), 2.98 (t, J = 5.8 Hz, 2H), 2.76 (t, J = 5.7 Hz, 2H), 1.18 (t, J = 7.1 Hz, 3H).

### Step 10: Preparation of intermediate 11l

Intermediate **11k** (13.12 g), dichloromethane (130 mL), triethylamine (10.24 g, 14.10 mL), and di-*tert-*butyl dicarbonate (12.15 g, 12.92 mL) were added to a reaction flask in sequence, and the mixture was reacted at room temperature. After the reaction was completed, the reaction solution was concentrated by evaporation under reduced pressure to remove the solvent, and ethyl acetate (300 mL) and water (500 mL) were added to the residue for extraction. The organic phase was separated, dried over anhydrous sodium sulfate, and filtered, and the filtrate was purified by a silica gel column to give the target intermediate **11l** (16.32 g).

MS(ESI, [M+H]⁺) m/z: 360.2.

¹H NMR (500 MHz, DMSO-d6) δ 7.88 (s, 1H), 7.40 (d, J = 7.9 Hz, 1H), 7.06 (d, J = 7.9 Hz, 1H), 4.73 (s, 2H), 4.10 (q, J = 7.1 Hz, 2H), 3.78 - 3.75 (m, 2H), 3.63 (t, J = 5.8 Hz, 2H), 2.87 (t, J = 5.8 Hz, 2H), 1.45 (s, 9H), 1.19 (t, J = 6.6 Hz, 3H).

### Step 11: Preparation of intermediate 11m

Intermediate **11l** (16.32 g), *N,N-*dimethylformamide (160 mL), and acrylamide (3.55 g) were added to a reaction flask in sequence. The mixture was cooled to 0 °C under N₂ atmosphere, then a solution of potassium *tert*-butoxide in tetrahydrofuran (1 mol/L, 40.9 mL) was added, and the mixture was reacted at 0 °C. After the reaction was completed, the reaction solution was added dropwise to an icy saturated ammonium chloride aqueous solution, and ethyl acetate was added for extraction. The organic phase was separated, dried over anhydrous sodium sulfate, filtered, and concentrated by evaporation under reduced pressure to remove the solvent. The residue was slurried with petroleum ether/ethyl acetate (200 mL) and filtered, and the filter cake was collected to give the target intermediate **11m** (14.5 g).

MS(ESI, [M+H]⁺) m/z: 385.1.

¹H NMR (500 MHz, DMSO-d6) δ 10.89 (s, 1H), 7.88 (s, 1H), 7.39 (d, J = 8.0 Hz, 1H), 7.05 (d, J = 8.0 Hz, 1H), 4.73 (s, 2H), 4.12 (dd, J = 12.0, 4.9 Hz, 1H), 3.63 (t, J = 5.8 Hz, 2H), 2.87 (t, J = 5.8 Hz, 2H), 2.73 (td, J = 12.2, 6.0 Hz, 1H), 2.59 - 2.54 (m, 1H), 2.31 (qd, J = 12.5, 4.4 Hz, 1H), 2.10 (ddt, J = 9.9, 5.2, 2.7 Hz, 1H), 1.44 (s, 9H).

### Step 12: Preparation of intermediate 11

Intermediate **11m** (14.5 g), dichloromethane (150 mL), and a solution of hydrochloric acid in 1,4-dioxane (4 mol/L, 141 mL) were added to a reaction flask in sequence, and the mixture was reacted at room temperature. The reaction solution was concentrated by evaporation under reduced pressure to remove the solvent. The residue was slurried with methyl *tert-*butyl ether and filtered, and the filter cake was collected to give intermediate **11** (12.6 g).

MS(ESI, [M+H]⁺) m/z: 285.1.

¹H NMR (500 MHz, DMSO-d6) δ 10.90 (s, 1H), 9.87 (s, 2H), 7.93 (s, 1H), 7.49 (d, J = 8.0 Hz, 1H), 7.10 (d, J = 8.1 Hz, 1H), 4.45 (s, 2H), 4.15 (dd, J = 12.1, 4.8 Hz, 1H), 3.40 (t, J = 6.1 Hz, 2H), 3.12 (t, J = 6.1 Hz, 2H), 2.75 (ddd, J = 17.4, 12.3, 5.3 Hz, 1H), 2.57 (dt, J = 17.3, 4.0 Hz, 1H), 2.32 (qd, J = 12.6, 4.5 Hz, 1H), 2.10 (ddt, J = 9.9, 5.2, 2.6 Hz, 1H).

### Example 12: Synthesis of Intermediate 12

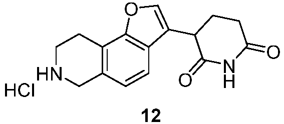

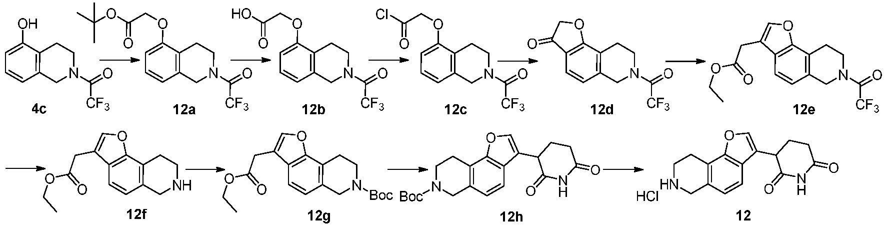

### Step 1: Preparation of intermediate 12a

Intermediate **4c,** acetonitrile (130 mL), potassium carbonate (14.09 g), and *tert-butyl* bromoacetate (10.94 g, 8.29 mL) were added to a reaction flask in sequence, and the mixture was heated to 80 °C and reacted. After the reaction was completed, ethyl acetate (200 mL) and water (500 mL) were added for extraction. The organic phase was separated, and the aqueous phase was extracted with ethyl acetate. The organic phases were then combined, washed with saturated brine, dried over anhydrous sodium sulfate, filtered, and concentrated by evaporation under reduced pressure to remove the solvent, thus giving the target intermediate **12a** (20.33 g).

¹H NMR (500 MHz, DMSO-d6) δ 7.17 (t, J = 8.0 Hz, 1H), 6.87 (t, J = 7.8 Hz, 1H), 6.76 (t, J = 8.0 Hz, 1H), 4.75 (d, J = 4.8 Hz, 2H), 4.70 (d, J = 4.6 Hz, 2H), 3.84 (t, J = 6.1 Hz, 2H), 2.81 (dt, J = 16.3, 6.1 Hz, 2H), 1.41 (d, J = 2.4 Hz, 9H).

### Step 2: Preparation of intermediate 12b

Intermediate **12a** (20.33 g), dichloromethane (100 mL), and trifluoroacetic acid (29.0 g, 19.61 mL) were added to a reaction flask in sequence, and the mixture was reacted at room temperature. After the reaction was completed, the reaction solution was concentrated by evaporation under reduced pressure to remove the solvent, and dichloromethane was added to the residue. The mixture was then concentrated by evaporation under reduced pressure to remove the solvent, thus giving the target intermediate **12b** (16.2 g).

MS(ESI, [M-H]⁻) m/z: 301.9.

¹H NMR (500 MHz, DMSO-d6) δ 13.00 (s, 1H), 7.17 (t, J = 7.9 Hz, 1H), 6.87 (t, J = 8.0 Hz, 1H), 6.77 (t, J = 7.6 Hz, 1H), 4.74 (d, J = 4.7 Hz, 2H), 4.72 (d, J = 4.1 Hz, 2H), 3.84 (t, J = 6.1 Hz, 2H), 2.81 (dt, J = 16.2, 6.1 Hz, 2H).

### Step 3: Preparation of intermediates 12c and 12d

Intermediate **12b** (16.2 g), tetrahydrofuran (160 mL), and thionyl chloride (30.0 g, 18.29 mL) were added to a reaction flask in sequence, and the mixture was heated to 75 °C and reacted for 2 h. After the reaction was completed, the reaction solution was concentrated by evaporation under reduced pressure to remove the solvent, thus giving the target intermediate **12c.** Trifluoromethanesulfonic acid (100 mL) was added dropwise to intermediate **12c** under an ice bath, and the mixture was stirred at room temperature. After the reaction was completed, the reaction solution was poured into an icy aqueous solution (2000 mL), and ethyl acetate (500 mL) was added. The organic phase was separated, and a saturated sodium bicarbonate solution was added to adjust the pH to 8. The organic phase was separated, dried over anhydrous sodium sulfate, and filtered, and the filtrate was purified by a silica gel column to give the target intermediate **12d** (3.27 g).

¹H NMR (500 MHz, DMSO-d6) δ 7.49 (d, J = 8.0 Hz, 1H), 7.08 (dd, J = 13.3, 8.0 Hz, 1H), 4.89 - 4.84 (m, 4H), 3.90 (dt, J = 10.0, 6.1 Hz, 2H), 2.88 (dt, J = 17.6, 6.0 Hz, 2H).

### Step 4: Preparation of intermediate 12e

Intermediate **12d** (4.59 g), (carbethoxymethylene)triphenylphosphorane (8.41 g), and toluene (100 mL) were added to a reaction flask in sequence, and the mixture was heated to 130 °C and reacted under N₂ atmosphere. After the reaction was completed, ethyl acetate (200 mL) and water (300 mL) were added for extraction. The organic phase was separated, and the aqueous phase was extracted with ethyl acetate. The organic phases were combined, washed with saturated brine, dried over anhydrous sodium sulfate, and filtered, and the filtrate was purified by a silica gel column to give the target intermediate **12e** (3.93 g).

¹H NMR (500 MHz, DMSO-d6) δ 7.92 (d, J = 2.7 Hz, 1H), 7.46 (dd, J = 8.1, 3.2 Hz, 1H), 7.16 (t, J = 8.4 Hz, 1H), 4.88 (d, J = 7.3 Hz, 2H), 4.10 (q, J = 7.1 Hz, 2H), 3.97 - 3.91 (m, 2H), 3.77 (d, J = 1.0 Hz, 2H), 3.09 (dt, J = 15.5, 6.0 Hz, 2H), 1.19 (td, J = 7.0, 0.8 Hz, 3H).

### Step 5: Preparation of intermediate 12f

Intermediate **12e** (3.93 g), potassium carbonate (4.36 g), and ethanol (50 mL) were added to a reaction flask in sequence, and the mixture was reacted at room temperature. After the reaction was completed, ethyl acetate (200 mL) and water (300 mL) were added for extraction. The organic phase was separated, and the aqueous phase was extracted with ethyl acetate. The organic phases were combined, washed with saturated brine, dried over anhydrous sodium sulfate, filtered, and concentrated by evaporation under reduced pressure to remove the solvent, thus giving the target intermediate **12f** (2.63 g).

MS(ESI, [M+H]⁺) m/z: 260.1.

¹H NMR (500 MHz, DMSO-d6) δ 7.83 (s, 1H), 7.31 (d, J = 7.9 Hz, 1H), 6.92 (d, J = 7.9 Hz, 1H), 4.09 (t, J = 7.1 Hz, 2H), 3.92 (s, 2H), 3.74 (d, J = 1.0 Hz, 2H), 3.02 (t, J = 5.9 Hz, 2H), 2.83 (t, J = 5.9 Hz, 2H), 1.18 (t, J = 7.1 Hz, 3H).

### Step 6: Preparation of intermediate 12g

Intermediate **12f** (2.63 g), dichloromethane (30 mL), triethylamine (2.053 g, 2.83 mL), and di-*tert*-butyl dicarbonate (2.435 g, 2.59 mL) were added to a reaction flask in sequence, and the mixture was reacted at room temperature. After the reaction was completed, the reaction solution was concentrated by evaporation under reduced pressure to remove the solvent, and ethyl acetate (200 mL) and water (200 mL) were added to the residue for extraction. The organic phase was separated, and the aqueous phase was extracted with ethyl acetate. The organic phases were then combined, washed with saturated brine, dried over anhydrous sodium sulfate, and filtered, and the filtrate was purified by a silica gel column to give the target intermediate **12g** (3.53 g).

MS(ESI, [M+H]⁺) m/z: 360.1.

¹H NMR (500 MHz, DMSO-d6) δ 7.88 (d, J = 1.1 Hz, 1H), 7.40 (d, J = 8.0 Hz, 1H), 7.06 (d, J = 8.0 Hz, 1H), 4.61 (s, 2H), 4.10 (q, J = 7.1 Hz, 2H), 3.76 (d, J = 1.1 Hz, 2H), 3.66 (t, J = 5.9 Hz, 2H), 2.94 (t, J = 5.9 Hz, 2H), 1.43 (s, 9H), 1.18 (t, J = 7.1 Hz, 3H).

### Step 7: Preparation of intermediate 12h

Intermediate **12g** (3.71 g), *N,N-*dimethylformamide (12 mL), and acrylamide (0.807 g) were added to a reaction flask in sequence. The mixture was cooled to 0 °C under N₂ atmosphere, then a solution of potassium *tert*-butoxide in tetrahydrofuran (1 mol/L, 8.26 mL) was added, and the mixture was reacted at 0 °C for 1 h. After the reaction was completed, the reaction solution was added dropwise to an icy saturated ammonium chloride aqueous solution, and ethyl acetate was added for extraction. The organic phase was separated, and the aqueous phase was extracted with ethyl acetate. The organic phases were then combined, washed with saturated brine, dried over anhydrous sodium sulfate, and filtered, and the filtrate was purified by a silica gel column to give the target intermediate **12h** (2.57 g).

MS(ESI, [M+H]⁺) m/z: 385.2.

¹H NMR (500 MHz, DMSO-d6) δ 10.89 (s, 1H), 7.87 (s, 1H), 7.40 (d, J = 8.0 Hz, 1H), 7.05 (d, J = 8.1 Hz, 1H), 4.61 (s, 2H), 4.11 (dd, J = 12.0, 4.9 Hz, 1H), 3.66 (t, J = 6.0 Hz, 2H), 2.95 (t, J = 5.9 Hz, 2H), 2.74 (ddd, J = 17.3, 12.2, 5.3 Hz, 1H), 2.57 (dt, J = 17.3, 4.1 Hz, 1H), 2.35 - 2.27 (m, 1H), 2.10 (dtd, J = 13.5, 5.2, 3.7 Hz, 1H), 1.43 (s, 9H).

### Step 8: Preparation of intermediate 12

Intermediate **12h** (2.57 g), dichloromethane (25 mL), and a solution of hydrochloric acid in 1,4-dioxane (4 mol/L, 25.10 mL) were added to a reaction flask in sequence, and the mixture was reacted at room temperature. After the reaction was completed, the reaction solution was concentrated by evaporation under reduced pressure to remove the solvent. The residue was slurried with methyl *tert-*butyl ether and filtered, and the filter cake was collected to give intermediate **12** (2.09 g).

MS(ESI, [M+H]⁺) m/z: 285.1.

¹H NMR (500 MHz, DMSO-d6) δ 10.90 (s, 1H), 9.72 (s, 2H), 7.94 (s, 1H), 7.48 (d, J = 8.1 Hz, 1H), 7.10 (d, J = 8.2 Hz, 1H), 4.35 (d, J = 4.1 Hz, 2H), 4.14 (dd, J = 12.1, 4.9 Hz, 1H), 3.38 (s, 2H), 3.18 (t, J = 6.2 Hz, 2H), 2.75 (ddd, J = 17.4, 12.3, 5.4 Hz, 1H), 2.57 (dt, J = 17.3, 4.0 Hz, 1H), 2.32 (qd, J = 12.6, 4.4 Hz, 1H), 2.10 (dtd, J = 13.4, 5.2, 3.5 Hz, 1H).

### Example 13: Synthesis of Intermediate 13

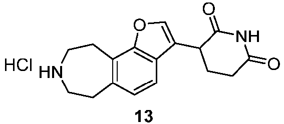

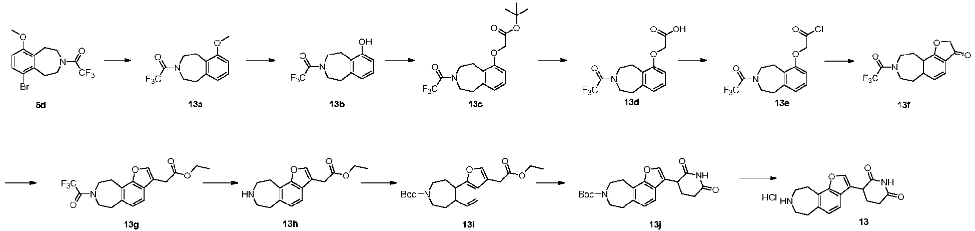

### Step 1: Preparation of intermediate 13a

**5d** (32.0 g), 10% palladium on carbon (6.1 g), and methanol (250 mL) were added to a reaction flask in sequence, and the mixture was purged three times with hydrogen and reacted overnight. After the reaction was completed, the mixture was filtered through diatomite, the filtrate was concentrated to dryness by rotary evaporation, and ethyl acetate (200 mL) and water (300 mL) were added. The organic phase was separated, washed with saturated brine, dried over anhydrous sodium sulfate, filtered, and concentrated to give intermediate **13a** (22.7 g).

MS(ESI, [M+H]⁺) *m*/*z:* 274.0.

### Step 2: Preparation of intermediate 13b

A solution of boron tribromide in dichloromethane (125 mL, 1 M) was slowly added dropwise to a solution of **13a** (22.7 g) in dichloromethane (200 mL) at 0 °C under nitrogen atmosphere. After the dropwise addition, the mixture was warmed to room temperature and reacted. After the reaction was completed, the reaction solution was slowly poured into ice water (350 mL), and the mixture was stirred for 10 min, concentrated to dryness by rotary evaporation to remove the dichloromethane, and filtered. The filter cake was collected and dried to give **13b** (21.1 g).

MS(ESI, [M-H]⁻) *m*/*z*: 258.1*.*

¹H NMR (500 MHz, DMSO-d6) δ 9.39 (s, 1H), 6.93 (td, *J* = 7.7, 3.0 Hz, 1H), 6.71 (ddd, *J* = 8.1, 4.7, 1.1 Hz, 1H), 6.61 (t, *J* = 7.2 Hz, 1H), 3.72 - 3.57 (m, 4H), 3.07 - 2.86 (m, 4H).

### Step 3: Preparation of intermediate 13c

**13b** (20.5 g), *tert*-butyl bromoacetate (18.5 g), potassium carbonate (27.3 g), and DMF (100 mL) were added to a reaction flask in sequence, and the reaction solution was warmed to 80 °C and reacted. The reaction solution was cooled to room temperature, and ethyl acetate (200 mL) and water (300 mL) were added to the reaction solution. The organic phase was separated, washed with saturated brine, dried over anhydrous sodium sulfate, filtered, and concentrated to give **13c** (34.5 g).

MS(ESI, [M-H]⁻) *m*/*z*: 372.1*.*

### Step 4: Preparation of intermediate 13d

**13c** (29.5 g), trifluoroacetic acid (90.2 g), and dichloromethane (200 mL) were added to a reaction flask in sequence, and the mixture was reacted at room temperature. After the reaction was completed, the reaction solution was concentrated to dryness by rotary evaporation to remove the dichloromethane, and water (300 mL) was added. The mixture was stirred for 10 min and filtered, and the filter cake was collected and dried to give **13d** (23.5 g).

MS(ESI, [M-H]⁻) *m*/*z*: 316.0*.*

### Step 5: Preparation of intermediate 13f

**13d** (21.2 g), thionyl chloride (79.5 g), and tetrahydrofuran (200 mL) were added to a reaction flask in sequence, and the reaction solution was warmed to 80 °C and reacted. The reaction solution was concentrated by rotary evaporation to remove the solvent, thus giving **13e,** followed by the addition of dichloromethane (200 mL). The reaction solution was cooled to 0 °C, and trifluoromethanesulfonic acid (49.7 g) was slowly added dropwise. After the dropwise addition, the mixture was allowed to react at room temperature. The reaction solution was slowly poured into ice water (400 mL), and dichloromethane was added. The organic phase was separated, washed with a saturated sodium chloride solution, dried over anhydrous sodium sulfate, and filtered, and the concentrate was separated and purified by silica gel column chromatography to give intermediate **13f** (8.8 g).

MS(ESI, [M+H]⁺) *m*/*z*: 300.1.

¹H NMR (500 MHz, DMSO-*d*₆) δ 7.44 (dd, *J* = 7.8, 1.9 Hz, 1H), 7.01 (t, *J* = 8.4 Hz, 1H), 4.82 (d, *J* = 2.0 Hz, 2H), 3.73 (dq, *J* = 9.9, 5.8 Hz, 4H), 3.16 - 3.06 (m, 4H).

### Step 6: Preparation of intermediate 13g

**13f** (8.8 g), (carbethoxymethylene)triphenylphosphorane (14.2 g), and toluene (50 mL) were added to a reaction flask in sequence, and the reaction solution was warmed to 130 °C and reacted overnight. The reaction solution was cooled to room temperature, and ethyl acetate (50 mL) and water (60 mL) were added. The organic phase was separated, washed with a saturated sodium chloride solution, dried over anhydrous sodium sulfate, and filtered, and the concentrate was separated and purified by silica gel column chromatography to give intermediate **13g** (6.8 g).

MS(ESI, [M+H]⁺) *m*/*z*: 370.1

¹H NMR (500 MHz, DMSO-d6) δ 7.92 - 7.84 (m, 1H), 7.36 (dd, *J* = 7.8, 3.1 Hz, 1H), 7.10 (t, *J* = 7.9 Hz, 1H), 4.10 (q, *J* = 7.1 Hz, 2H), 3.81 - 3.70 (m, 6H), 3.30 - 3.20 (m, 2H), 3.16 - 3.06 (m, 2H), 1.19 (t, *J* = 7.1 Hz, 3H).

### Step 7: Preparation of intermediate 13h

**13g** (6.8 g), potassium carbonate (7.6 g), and ethanol (100 mL) were added to a reaction flask in sequence, and the reaction solution was warmed to 50 °C and reacted overnight. The reaction solution was cooled to room temperature, and ethyl acetate (70 mL) and water (150 mL) were added. The organic phase was separated, washed with a saturated sodium chloride solution, dried over anhydrous sodium sulfate, and filtered, and the concentrate was separated and purified by silica gel column chromatography to give intermediate **13h** (3.8 g).

MS(ESI, [M+H]⁺) *m*/*z*: 274.2

### Step 8: Preparation of intermediate 13i

**13h** (3.8 g), triethylamine (2.8 g), and dichloromethane (50 mL) were added to a reaction flask in sequence, Boc anhydride (3.64 g) was added under stirring, and the mixture was reacted at room temperature. Dichloromethane (70 mL) and water (100 mL) were added. The organic phase was separated, washed with a saturated sodium chloride solution, dried over anhydrous sodium sulfate, and filtered, and the concentrate was separated and purified by silica gel column chromatography to give intermediate **13i** (3.9 g).

### Step 9: Preparation of intermediate 13j

A solution of potassium *tert*-butoxide in tetrahydrofuran (7.7 mL, 1 M) was slowly added dropwise to a solution of **13i** (3.7 g) and acrylamide (0.7 g) in DMF (30 mL) at 0 °C under nitrogen atmosphere. After the dropwise addition, the mixture was reacted with the temperature maintained. After the reaction was completed, the reaction solution was slowly poured into saturated ammonium chloride solution (100 mL), and the mixture was stirred for 10 min and filtered. The filter cake was collected, slurried with ethyl acetate, filtered, and dried to give **13j** (2.1 g). MS(ESI, [M-H]⁻) *m*/*z:* 397.0.

¹H NMR (500 MHz, DMSO-d6) δ 10.88 (s, 1H), 7.85 (s, 1H), 7.30 (d, *J* = 7.9 Hz, 1H), 7.05 (d, *J* = 7.9 Hz, 1H), 4.09 (dd, *J* = 11.9, 4.9 Hz, 1H), 3.52 (dt, *J* = 28.7, 4.3 Hz, 4H), 3.11 (d, *J* = 6.2 Hz, 2H), 3.02 - 2.92 (m, 2H), 2.73 (ddd, *J* = 17.2, 12.1, 5.3 Hz, 1H), 2.56 (dt, *J* = 17.3, 4.1 Hz, 1H), 2.30 (qd, *J=* 12.3, 4.4 Hz, 1H), 2.14 - 2.04 (m, 1H), 1.40 (s, 9H).

### Step 10: Preparation of intermediate 13

**13j** (2.1 g), a solution of hydrochloric acid in 1,4-dioxane (10 mL), and dichloromethane (10 mL) were added to a reaction flask in sequence, and the mixture was reacted at room temperature. After the reaction was completed, the mixture was filtered, and the filter cake was rinsed with a small amount of methyl *tert-*butyl ether and dried to give intermediate **13** (1.6 g).

MS(ESI, [M+H]⁺) *m*/*z:* 299.1

¹H NMR (500 MHz, DMSO-*d*₆) δ 10.89 (s, 1H), 9.57 (s, 2H), 7.91 (s, 1H), 7.37 (d, *J* = 7.8 Hz, 1H), 7.11 (d, *J* = 8.0 Hz, 1H), 4.12 (dd, *J* = 12.1, 4.9 Hz, 1H), 3.38 (s, 2H), 3.26 (td, *J* = 8.1, 7.3, 3.8 Hz, 4H), 3.20 (dt, *J* = 8.6, 3.9 Hz, 2H), 2.75 (ddd, *J =* 17.4, 12.3, 5.4 Hz, 1H), 2.57 (dt, *J* = 17.2, 4.1 Hz, 1H), 2.32 (qd, *J =* 12.6, 4.4 Hz, 1H), 2.09 (dq, *J* = 13.5, 4.7 Hz, 1H).

### Example 14: Synthesis of Intermediate 14

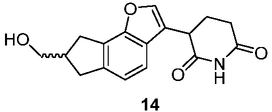

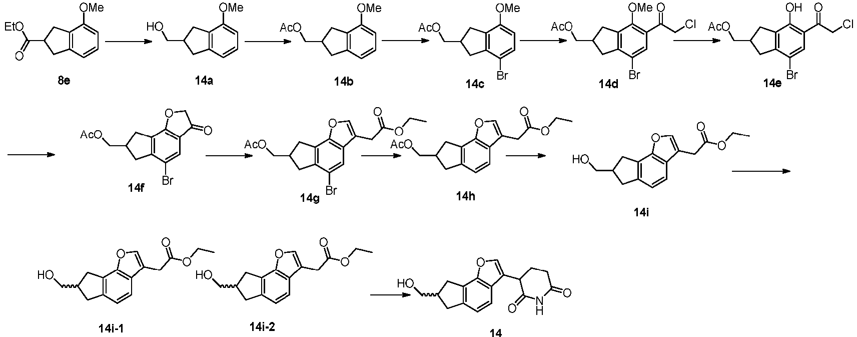

### Step 1: Preparation of intermediate 14a

Intermediate **8e** (14.95 g) and THF (200 mL) were added to a reaction flask in sequence. The mixture was cooled to 0 °C under N₂ atmosphere, and then a solution of lithium aluminum hydride in tetrahydrofuran (5.67 g, 59.8 mL) was added. After the dropwise addition, the mixture was reacted at 0 °C for 2 h. After the reaction was completed, water was slowly added dropwise to the reaction solution at 0 °C to quench the reaction, and anhydrous sodium sulfate was added. The mixture was filtered, and the filtrate was concentrated by evaporation under reduced pressure to remove the solvent, thus giving the target intermediate **14a** (11.15 g).

¹H NMR (500 MHz, DMSO-d6) δ 7.08 (t, J = 7.7 Hz, 1H), 6.81 - 6.76 (m, 1H), 6.71 (d, J = 8.2 Hz, 1H), 4.62 (t, J = 5.3 Hz, 1H), 3.74 (s, 3H), 3.35 (dd, J = 6.8, 5.3 Hz, 2H), 2.92 (dd, J = 16.0, 8.2 Hz, 1H), 2.88 - 2.79 (m, 1H), 2.65 (dd, J = 16.1, 5.7 Hz, 1H), 2.60 - 2.51 (m, 2H).

### Step 2: Preparation of intermediate 14b

Intermediate **14a,** dichloromethane (100 mL), triethylamine (18.99 g), and 4-dimethylaminopyridine (0.191 g) were added to a reaction flask in sequence, acetic anhydride (7.03 g, 6.55 mL) was added under an ice bath, and the mixture was warmed to room temperature and reacted for 1 h. After the reaction was completed, the reaction solution was concentrated by evaporation under reduced pressure to remove the solvent, and ethyl acetate (300 mL) and water (500 mL) were added to the residue for extraction. The organic phase was separated, washed with a saturated ammonium chloride solution and saturated brine separately, dried over anhydrous sodium sulfate, and filtered, and the filtrate was concentrated by evaporation under reduced pressure to remove the solvent, thus giving the target intermediate **14b** (12.97 g).

¹H NMR (500 MHz, DMSO-d6) δ 7.13 - 7.09 (m, 1H), 6.80 (d, J = 7.2 Hz, 1H), 6.74 (d, J = 8.0 Hz, 1H), 4.00 (d, J = 7.1 Hz, 2H), 3.75 (s, 3H), 2.99 (dd, J = 15.6, 7.9 Hz, 1H), 2.92 (dd, J = 16.1, 8.1 Hz, 1H), 2.77 - 2.69 (m, 1H), 2.66 (dd, J = 15.6, 6.4 Hz, 1H), 2.55 (dd, J = 16.2, 6.2 Hz, 1H), 2.02 (s, 3H).

### Step 3: Preparation of intermediate 14c

Intermediate **14b** (10.15 g), *N*-bromosuccinimide (9.02 g), and acetonitrile (100 mL) were added to a reaction flask in sequence, and the mixture was heated to 75 °C and reacted for 1 h. After the reaction was completed, ethyl acetate (200 mL) and water (500 mL) were added for extraction. The organic phase was separated, and the aqueous phase was extracted with ethyl acetate. The organic phases were then combined, washed with saturated brine, dried over anhydrous sodium sulfate, filtered, and concentrated by evaporation under reduced pressure to remove the solvent, thus giving the target intermediate **14c** (14.11 g).

¹H NMR (500 MHz, DMSO-d6) δ 7.31 (d, J = 8.7 Hz, 1H), 6.76 (d, J = 8.6 Hz, 1H), 4.06 - 4.00 (m, 2H), 3.76 (s, 3H), 3.02 (ddd, J = 16.3, 14.7, 8.3 Hz, 2H), 2.81 - 2.73 (m, 1H), 2.70 - 2.62 (m, 2H), 2.03 (s, 3H).

### Step 4: Preparation of intermediate 14d

Trifluoromethanesulfonic acid (40 mL) was slowly added dropwise to a stirred solution of intermediate **14c** (14.11 g) and chloroacetyl chloride (5.13 g, 3.61 mL) under an ice bath. After the addition, the mixture was stirred at room temperature for 1 h. After the reaction was completed, the reaction solution was poured into an icy aqueous solution (500 mL), and the mixture was extracted with ethyl acetate, dried over anhydrous sodium sulfate, filtered, and concentrated by evaporation under reduced pressure to remove the solvent, thus giving the target intermediate **14d** (15.23 g).

¹H NMR (500 MHz, DMSO-d6) δ 7.67 (s, 1H), 4.99 (s, 2H), 4.07 (dd, J = 6.9, 4.6 Hz, 2H), 3.86 (s, 3H), 3.28 (dd, J = 16.2, 8.2 Hz, 1H), 3.05 (dd, J = 16.9, 8.2 Hz, 1H), 2.94 (dd, J = 16.2, 6.7 Hz, 1H), 2.87 - 2.81 (m, 1H), 2.72 (dd, J = 16.9, 6.7 Hz, 1H), 2.03 (s, 3H).

### Step 5: Preparation of intermediate 14e

A solution of boron trichloride in dichloromethane (1 mol/L, 73.0 mL) was slowly added dropwise to a stirred solution of intermediate **14d** (15.23 g) in dichloromethane (450 mL) at -35 °C under N₂ atmosphere. After the dropwise addition, the mixture was stirred at -30 °C. After the reaction was completed, an hydrochloric acid aqueous solution (1 M, 100 mL) was added to the reaction solution at -30 °C to quench the reaction. The mixture was warmed to room temperature, and the organic phase was separated, washed with saturated brine, dried over anhydrous sodium sulfate, filtered, and concentrated by evaporation under reduced pressure to remove the solvent, thus giving the target intermediate **14e** (15.24 g).

MS(ESI, [M+H]⁺) m/z: 361.1.

¹H NMR (500 MHz, DMSO-d6) δ 11.26 (s, 1H), 7.87 (s, 1H), 5.19 (s, 2H), 4.05 (dd, J = 7.0, 2.5 Hz, 2H), 3.13 - 3.06 (m, 2H), 2.84 (ddd, J = 8.4, 5.1, 1.8 Hz, 1H), 2.76 - 2.71 (m, 2H), 2.03 (s, 3H).

### Step 6: Preparation of intermediate 14f

Intermediate **14e** (15.24 g), acetonitrile (150 mL), and sodium carbonate (4.41 g) were added to a reaction flask in sequence, and the mixture was heated to 75 °C and reacted. After the reaction was completed, ethyl acetate (200 mL) and water (500 mL) were added for extraction. The organic phase was separated, and the aqueous phase was extracted with ethyl acetate. The organic phases were then combined, washed with saturated brine, dried over anhydrous sodium sulfate, filtered, and concentrated by evaporation under reduced pressure to remove the solvent, thus giving the target intermediate **14f** (13.97 g).

¹H NMR (500 MHz, DMSO-d6) δ 7.64 (s, 1H), 4.86 (s, 2H), 4.08 (t, J = 6.4 Hz, 2H), 3.20 - 3.11 (m, 2H), 2.94 - 2.90 (m, 1H), 2.85 - 2.77 (m, 2H), 2.03 (s, 3H).

### Step 7: Preparation of intermediate 14g

Intermediate **14f** (6.85 g), (carbethoxymethylene)triphenylphosphorane (11.01 g), and toluene (80 mL) were added to a reaction flask in sequence, and the mixture was heated to 130 °C and reacted under N₂ atmosphere. After the reaction was completed, ethyl acetate (200 mL) and water (300 mL) were added for extraction. The organic phase was separated, and the aqueous phase was extracted with ethyl acetate. The organic phases were combined, washed with saturated brine, dried over anhydrous sodium sulfate, and filtered, and the filtrate was purified by a silica gel column to give the target intermediate 14g (4.09 g).

¹H NMR (500 MHz, DMSO-d6) δ 7.90 (s, 1H), 7.65 (s, 1H), 4.13 - 4.08 (m, 4H), 3.77 (s, 2H), 3.16 - 3.11 (m, 1H), 3.01 - 2.91 (m, 2H), 2.76 (ddd, J = 23.5, 16.3, 6.3 Hz, 2H), 2.04 (s, 3H), 1.19 (t, J = 7.1 Hz, 3H).

### Step 8: Preparation of intermediate 14h

Intermediate **14g** (1.2 g), 10% palladium on carbon (1.2 g), ethanol (40 mL), and dichloromethane (20 mL) were added to a reaction flask in sequence, and the mixture was purged with H₂ and then reacted at room temperature. After the reaction was completed, the reaction mixture was filtered through diatomite. The filtrate was adjusted to pH 8 with a saturated sodium bicarbonate solution, and ethyl acetate (200 mL) and water (200 mL) were added for extraction. The organic phase was separated, and the aqueous phase was extracted with ethyl acetate. The organic phases were combined, washed with saturated brine (300 mL), dried over anhydrous sodium sulfate, filtered, and concentrated by evaporation under reduced pressure to remove the solvent, thus giving the target intermediate **14h** (1.01 g).

### Step 9: Preparation of intermediates 14i-1 and 14i-2

Intermediate **14h** (0.98 g), potassium carbonate (1.713 g), and ethanol (15 mL) were added to a reaction flask in sequence, and the mixture was reacted at room temperature for 18 h. After the reaction was completed, ethyl acetate (100 mL) and water (100 mL) were added for extraction. The organic phase was separated, dried over anhydrous sodium sulfate, filtered, and concentrated by evaporation under reduced pressure to remove the solvent, thus giving the target intermediate **14i** (0.9 g). Intermediate **14i** was separated by preparative high performance liquid chromatography to give intermediates **14i-1** (0.36 g) and **14i-2** (0.42 g). The conditions for the preparative chromatography were as follows:
Instrument and preparative column: YMC K-prep Lab100g high pressure preparative chromatograph was used, and the preparative column was CHIRALART Amylose-SA (5 µm, 30 × 250 mm). Mobile phase system: n-hexane/ethanol, isocratic elution: n-hexane/ethanol = 90/10.

The data of **14i-1** were as follows:
MS(ESI, [M+H]+) m/z: 275.2.

¹H NMR (500 MHz, DMSO-d6) δ 7.81 (s, 1H), 7.34 (d, J = 7.8 Hz, 1H), 7.11 (d, J = 7.9 Hz, 1H), 4.69 (t, J = 5.3 Hz, 1H), 4.10 (q, J = 7.1 Hz, 2H), 3.76 - 3.72 (m, 2H), 3.41 (ddd, J = 6.8, 5.2, 1.4 Hz, 2H), 3.12 (dd, J = 16.1, 8.3 Hz, 1H), 3.05 (dd, J = 15.9, 8.2 Hz, 1H), 2.86 (dd, J = 16.2, 5.7 Hz, 1H), 2.78 (dd, J = 15.9, 5.7 Hz, 1H), 2.68 (ddd, J = 13.6, 8.0, 5.8 Hz, 1H), 1.19 (t, J = 7.1 Hz, 3H).

### Step 10: Preparation of intermediate 14

Intermediate **14i-1** (350 mg), *N*,*N*-dimethylformamide (10 mL), and acrylamide (100 mg) were added to a reaction flask in sequence. The mixture was cooled to 0 °C under N₂ atmosphere, then a solution of potassium *tert*-butoxide in tetrahydrofuran (1 mol/L, 1.025 mL) was added, and the mixture was reacted at 0 °C for 1 h. After the reaction was completed, the reaction solution was added dropwise to an icy saturated ammonium chloride aqueous solution, and ethyl acetate was added for extraction. The organic phase was separated, and the aqueous phase was extracted with ethyl acetate. The organic phases were then combined, washed with saturated brine, dried over anhydrous sodium sulfate, and filtered, and the filtrate was purified by a silica gel column to give intermediate 14 (0.204 g).

MS(ESI, [M+H]⁺) m/z: 300.1.

¹H NMR (500 MHz, DMSO-d6) δ 10.87 (s, 1H), 7.80 (s, 1H), 7.33 (d, J = 7.8 Hz, 1H), 7.09 (d, J = 7.9 Hz, 1H), 4.69 (t, J = 5.2 Hz, 1H), 4.10 (dd, J = 11.9, 4.9 Hz, 1H), 3.41 (t, J = 6.1 Hz, 2H), 3.18 - 3.03 (m, 2H), 2.86 (dd, J = 16.2, 5.6 Hz, 1H), 2.81 - 2.76 (m, 1H), 2.74 - 2.63 (m, 2H), 2.56 (dt, J = 17.3, 4.2 Hz, 1H), 2.30 (qd, J = 12.2, 4.4 Hz, 1H), 2.13 - 2.06 (m, 1H).

### Example 15: Synthesis of Intermediate 15

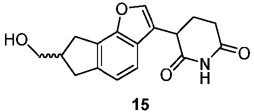

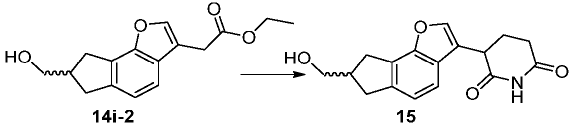

Intermediate **14i-2** (420 mg), N,N-dimethylformamide (10 mL), and acrylamide (120 mg) were added to a reaction flask in sequence. The mixture was cooled to 0 °C under N₂ atmosphere, then potassium tert-butoxide (1 mol/L, 1.221 mL) was added, and the mixture was reacted at 0 °C. After the reaction was completed, the reaction solution was added dropwise to an icy saturated ammonium chloride aqueous solution, and ethyl acetate was added for extraction. The organic phase was separated, and the aqueous phase was extracted with ethyl acetate. The organic phases were then combined, washed with saturated brine, dried over anhydrous sodium sulfate, and filtered, and the filtrate was purified by a silica gel column to give intermediate **15** (0.237 g).

MS(ESI, [M+H]⁺) m/z: 300.1.

¹H NMR (500 MHz, DMSO-d6) δ 10.87 (s, 1H), 7.80 (s, 1H), 7.33 (d, J = 7.8 Hz, 1H), 7.09 (d, J = 7.9 Hz, 1H), 4.69 (t, J = 5.2 Hz, 1H), 4.10 (dd, J = 11.8, 4.9 Hz, 1H), 3.41 (t, J = 6.1 Hz, 2H), 3.18 - 3.04 (m, 2H), 2.86 (dd, J = 16.2, 5.6 Hz, 1H), 2.78 (dd, J = 15.9, 5.7 Hz, 1H), 2.72 (dd, J = 11.3, 6.0 Hz, 1H), 2.70 - 2.63 (m, 1H), 2.56 (dt, J = 17.3, 4.2 Hz, 1H), 2.30 (qd, J = 12.2, 4.4 Hz, 1H), 2.14 - 2.06 (m, 1H).

### Example 16: Synthesis of Intermediate 16

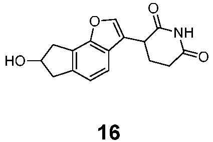

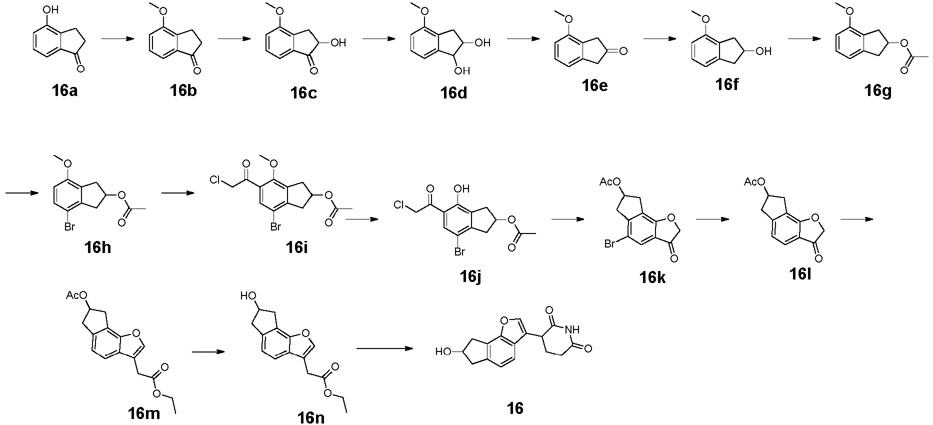

### Step 1: Preparation of intermediate 16b

**16a** (60 g), potassium carbonate (1.315 g), *N*,*N*-dimethylformamide (500 mL), and iodomethane (172 g) were added to a reaction flask in sequence, and the mixture was heated to 80 °C and reacted under N₂ atmosphere. After the reaction was completed, the reaction solution was cooled to room temperature, diluted with ethyl acetate, and washed with a saturated sodium chloride solution. The organic layer was collected, dried over anhydrous sodium sulfate, filtered, and concentrated to give the target intermediate **16b** (64.5 g).

MS(ESI, [M+H]⁺) m/z: 163.10.

### Step 2: Preparation of intermediate 16c

Potassium hydroxide (152 g) and methanol (600 mL) were added to a reaction flask in sequence, and the mixture was stirred for 20 min under an ice bath. Intermediate **16b** (40 g) was then added, and the mixture was stirred for another 20 min, followed by the addition of iodobenzene diacetate (98 g). The mixture was reacted at room temperature under N₂ atmosphere for 1 h. After the reaction was completed, the reaction solution was concentrated by evaporation under reduced pressure to remove the solvent, and ethyl acetate and a saturated sodium bicarbonate solution were added to the residue. The organic phase was separated, dried over anhydrous sodium sulfate, filtered, and concentrated by evaporation under reduced pressure to remove the solvent. The concentrate was dissolved in tetrahydrofuran (600 mL), then hydrochloric acid (82 mL, 6 M) was added, and the mixture was reacted at room temperature for 0.5 h. After the reaction was completed, the reaction solution was adjusted to pH 8-9 with a saturated sodium bicarbonate solution and extracted with ethyl acetate. The organic phases were combined, dried over anhydrous sodium sulfate, and filtered. The filtrate was concentrated, and then the residue was purified by silica gel column chromatography to give the target intermediate **16c** (35.1 g).

¹H NMR (500 MHz, Chloroform-d) δ 7.42 - 7.31 (m, 2H), 7.08 (dd, *J* = 7.2, 1.7 Hz, 1H), 4.51 (dd, *J* = 7.8, 4.7 Hz, 1H), 3.91 (s, 3H), 3.57 (dd, *J =* 17.0, 7.8 Hz, 1H), 3.02 (s, 1H), 2.84 (dd, *J =* 17.0, 4.7 Hz, 1H).

### Step 3: Preparation of intermediate 16d

Intermediate **16c** (37 g) and methanol (500 mL) were added to a reaction flask in sequence, then sodium borohydride (8.25 g) was added, and the mixture was reacted at room temperature for 1.5 h. After the reaction was completed, a saturated ammonium chloride solution was added dropwise to the reaction solution to quench the reaction, and ethyl acetate was added for extraction. The organic phases were combined, dried over anhydrous sodium sulfate, and filtered, and the filtrate was concentrated to give intermediate **16d** (50 g).

### Step 4: Preparation of intermediate 16e

Intermediate **16d** (35 g), toluene (300 mL), and p-toluenesulfonic acid (66.9 g) were added to a reaction flask in sequence, and the mixture was reacted at 120 °C under N₂ atmosphere. After the reaction was completed, the reaction solution was cooled to room temperature and concentrated by evaporation under reduced pressure to remove the solvent, and ethyl acetate and water were added to the residue. The organic layer was separated, and the aqueous layer was extracted with ethyl acetate. The organic layers were combined, dried over anhydrous sodium sulfate, and filtered. The filtrate was concentrated, and then the residue was purified by silica gel column chromatography to give the target intermediate **16e** (35.5 g).

¹H NMR (500 MHz, DMSO-d6) δ 7.26 - 7.22 (m, 1H), 6.93 - 6.87 (m, 2H), 3.79 (s, 3H), 3.53 (s, 2H), 3.37 (s, 2H).

### Step 5: Preparation of intermediate 16f

Intermediate **16e** (35 g) and methanol (400 mL) were added to a reaction flask in sequence at 0 °C, and sodium borohydride (5.83 g) was added in portions. The mixture was reacted at room temperature. After the reaction was completed, a saturated ammonium chloride solution was added dropwise to the reaction solution to quench the reaction, and water and ethyl acetate were added. The organic phase was separated, dried over anhydrous sodium sulfate, and filtered. The filtrate was concentrated, and then the residue was purified by silica gel column chromatography to give the target intermediate **16f** (18 g).

¹H NMR (500 MHz, DMSO-d6) δ 7.10 (td, *J=* 7.9, 7.4, 0.9 Hz, 1H), 6.83 - 6.78 (m, 1H), 6.74 *(d, J=* 8.1 Hz, 1H), 4.81 (d, *J=* 3.8 Hz, 1H), 4.49 (tq, *J =* 6.5, 3.4 Hz, 1H), 3.75 (s, 3H), 3.04 (dd, *J =* 16.1, 6.1 Hz, 1H), 2.94 (dd, *J*= 16.3, 6.2 Hz, 1H), 2.76 - 2.69 (m, 1H), 2.65 (dd, *J =* 16.4, 3.4 Hz, 1H).

### Step 6: Preparation of intermediate 16g

**16f** (15 g), dichloromethane (150 mL), triethylamine (27.8 g), and 4-dimethylaminopyridine (0.28 g) were added to a reaction flask in sequence, and acetic anhydride (10.2 g) was added under an ice bath. The mixture was reacted at room temperature. After the reaction was completed, the reaction solution was concentrated by evaporation under reduced pressure to remove the solvent, and ethyl acetate and water were added to the residue. The organic layer was separated, and the aqueous layer was extracted with ethyl acetate. The organic layers were combined, dried over anhydrous sodium sulfate, and filtered. The filtrate was concentrated, and then the residue was purified by silica gel column chromatography to give the target intermediate **16g** (9.3 g).

MS(ESI, [M+H]⁺) m/z: 207.10.

¹H NMR (500 MHz, DMSO-d6) δ 7.22 - 7.06 (m, 1H), 6.82 (dd, *J=* 27.3, 8.3 Hz, 2H), 5.41 (s, 1H), 3.78 (d, *J* = 13.2 Hz, 3H), 3.32 - 3.20 (m, 1H), 3.18 - 3.09 (m, 1H), 2.85 (dd, *J=* 34.6, 17.1 Hz, 2H), 1.97 (d, *J=* 15.3 Hz, 3H). Step 7: Preparation of intermediate **16h**

**16g** (5.6 g), N-bromosuccinimide (5.32 g), and acetonitrile (50 mL) were added to a reaction flask in sequence, and the mixture was reacted at 70 °C. After the reaction was completed, the reaction solution was cooled to room temperature, and water and ethyl acetate were added. The organic phase was separated, dried over anhydrous sodium sulfate, and filtered. The filtrate was concentrated, and then the residue was purified by silica gel column chromatography to give the target intermediate **16h** (6.4 g).

¹H NMR (500 MHz, DMSO-d6) δ 7.36 (d, *J =* 8.6 Hz, 1H), 6.81 (d, *J =* 8.7 Hz, 1H), 5.41 (tt, *J* = 6.4, 2.0 Hz, 1H), 3.77 (s, 3H), 3.31 - 3.23 (m, 2H), 2.92 (dd, *J=* 17.5, 2.0 Hz, 1H), 2.85 (dd, *J* = 17.4, 2.0 Hz, 1H), 1.97 (s, 3H). Step 8: Preparation of intermediate **16i**

**16h** (6.3 g) and chloroacetyl chloride (7.49 g) were added to a reaction flask in sequence, and trifluoromethanesulfonic acid (60 mL) was slowly added dropwise under an ice-water bath. The mixture was reacted at room temperature. After the reaction was completed, the reaction solution was slowly added dropwise to ice water, and the mixture was extracted with dichloromethane. The organic phases were combined and washed with a saturated sodium bicarbonate aqueous solution. The organic layer was separated, dried over anhydrous sodium sulfate, and filtered. The filtrate was concentrated, and then the residue was purified by silica gel column chromatography to give the target intermediate **16i** (7.3 g).

¹H NMR (500 MHz, DMSO-d6) δ 7.71 (s, 1H), 5.46 (tq, *J =* 7.5, 3.4, 2.7 Hz, 1H), 5.00 (s, 2H), 3.87 (d, *J =* 1.9 Hz, 3H), 3.57 (dd, *J =* 17.3, 6.2 Hz, 1H), 3.39 - 3.33 (m, 1H), 3.18 (dd, *J* = 17.4, 2.0 Hz, 1H), 2.92 (dd, *J =* 18.0, 1.9 Hz, 1H), 1.99 (s, 3H).

### Step 9: Preparation of intermediate 16j

**16i** (7.0 g) and dichloromethane (70 mL) were added to a reaction flask in sequence, and boron trichloride (58 mL) was slowly added dropwise under an ice-water bath. The mixture was reacted at room temperature. After the reaction was completed, the reaction solution was slowly added dropwise to ice water, and the mixture was extracted with dichloromethane. The organic phases were combined, dried over anhydrous sodium sulfate, and filtered. The filtrate was concentrated, and then the residue was purified by silica gel column chromatography to give the target intermediate **16j** (5.0 g).

MS(ESI, [M-H]⁻) m/z: 347.00.

¹H NMR (500 MHz, DMSO-d6) δ 11.24 (s, 1H), 7.90 (s, 1H), 5.44 (tq, *J* = 8.1, 3.5, 2.8 Hz, 1H), 5.19 (s, 2H), 3.38 (d, *J =* 6.3 Hz, 1H), 3.31 (t, *J =* 6.8 Hz, 1H), 3.03 (dd, *J =* 17.6, 1.8 Hz, 1H), 2.92 (dd, *J =* 18.2, 1.9 Hz, 1H), 1.98 (s, 3H).

### Step 10: Preparation of intermediate 16k

**16j** (4.9 g), sodium carbonate (2.99 g), and acetonitrile (120 mL) were added to a reaction flask in sequence, and the mixture was reacted at 50 °C. After the reaction was completed, water and ethyl acetate were added. The organic layer was collected, dried over anhydrous sodium sulfate, and filtered. The filtrate was concentrated, and then the residue was purified by silica gel column chromatography to give the target intermediate **16k** (3.7 g).

¹H NMR (500 MHz, DMSO-d6) δ 7.69 (s, 1H), 5.51 (tt, *J =* 6.4, 1.9 Hz, 1H), 4.88 (s, 2H), 3.49 - 3.36 (m, 2H), 3.08 (dd, *J =* 17.5, 1.8 Hz, 1H), 2.97 (dd, *J =* 18.3, 1.8 Hz, 1H), 1.99 (s, 3H).

### Step 11: Preparation of intermediate 16l

**16k** (3.0 g), 10% palladium on carbon (0.257 g), sodium bicarbonate (0.81 g), and ethanol (90 mL) were added to a reaction flask in sequence, and the mixture was reacted at room temperature under hydrogen atmosphere. After the reaction was completed, the reaction solution was filtered, and the filtrate was concentrated by evaporation under reduced pressure to remove the solvent. Ethyl acetate and water were added to the residue, and the organic layer was separated, dried over anhydrous sodium sulfate, and filtered. The filtrate was concentrated to give the target intermediate **161** (2.4 g).

¹H NMR (500 MHz, DMSO-d6) δ7.90 (d, *J* = 8.5 Hz, 1H), 6.95 (d, *J* = 8.6 Hz, 1H), 5.42 (s, 1H), 3.87 (s, 2H), 3.44 (d, *J=* 6.3 Hz, 1H), 3.27 (dd, *J* = 18.6, 1.9 Hz, 1H), 3.09 (d, *J* = 6.5 Hz, 1H), 2.80 (dd, *J* = 17.6, 1.8 Hz, 1H), 1.94 (s, 3H).

### Step 12: Preparation of intermediate 16m

**16l** (2.4 g), (carbethoxymethylene)triphenylphosphorane (5.4 g), and toluene (30 mL) were added to a reaction flask in sequence, and the mixture was reacted at 120 °C under N₂ atmosphere. After the reaction was completed, ethyl acetate and water were added to the reaction system. The organic layer was separated, dried over anhydrous sodium sulfate, and filtered. The filtrate was concentrated, and then the residue was purified by silica gel column chromatography to give the target intermediate **16m** (0.58 g).

¹H NMR (500 MHz, DMSO-d6) δ 7.85 (s, 1H), 7.42 (d, *J =* 7.9 Hz, 1H), 7.18 *(d, J =* 7.9 Hz, 1H), 5.54 (tt, *J* = 6.3, 2.3 Hz, 1H), 4.10 (q, *J =* 7.1 Hz, 2H), 3.77 (s, 2H), 3.46 (dd, *J* = 17.0, 6.2 Hz, 1H), 3.40 (dd, *J* = 17.0, 6.4 Hz, 1H), 3.11 (dd, *J =* 17.1, 2.2 Hz, 1H), 3.02 (dd, *J =* 17.0, 2.4 Hz, 1H), 1.97(s, 3H), 1.19 (t, *J* = 7.1 Hz, 3H).

### Step 13: Preparation of intermediate 16n

**16m** (2.4 g), potassium carbonate (0.79 g), and ethanol (30 mL) were added to a reaction flask in sequence, and the mixture was reacted at room temperature. After the reaction was completed, ethyl acetate and water were added to the reaction system. The organic layer was separated, dried over anhydrous sodium sulfate, and filtered. The filtrate was concentrated, and then the residue was purified by silica gel column chromatography to give the target intermediate **16n** (0.42 g).

MS(ESI, [M+H]⁺) m/z: 261.10.

¹H NMR (500 MHz, DMSO-d6) δ7.89 (s, 1H), 7.36 (d, *J* = 7.8 Hz, 1H), 7.13 (d, *J =* 7.8 Hz, 1H), 4.96 (d, *J* = 4.1 Hz, 1H), 4.62 (td, *J* = 6.2, 2.8 Hz, 1H), 4.10 (q, *J =* 7.1 Hz, 2H), 3.75 (d, *J =* 1.0 Hz, 2H), 3.24 (dd, *J* = 16.2, 6.1 Hz, 1H), 3.18 (dd, *J =* 16.0, 6.0 Hz, 1H), 2.92 (dd, *J =* 16.2, 3.3 Hz, 1H), 2.85 (dd, *J =* 16.0, 3.3 Hz, 1H), 1.18 (t, *J* = 7.1 Hz, 3H).

### Step 14: Preparation of intermediate 16

**16n** (90 mg), acrylamide (27 mg), and *N*,*N*-dimethylformamide (4 mL) were added to a reaction flask in sequence, and the mixture was cooled to 0 °C. Potassium tert-butoxide (0.28 mL, 1 M) was slowly added dropwise, and the mixture was reacted at 0 °C. After the reaction was completed, the reaction solution was added dropwise to an icy ammonium chloride aqueous solution, and ethyl acetate was added for extraction. The organic layer was separated, dried over anhydrous sodium sulfate, and filtered. The filtrate was concentrated, and then the residue was purified by silica gel column chromatography to give the target intermediate **16** (20 mg).

¹H NMR (500 MHz, DMSO-d6) δ 10.88 (s, 1H), 7.80 (s, 1H), 7.35 (d, *J* = 7.8 Hz, 1H), 7.11 (d, *J =* 7.9 Hz, 1H), 4.62 (tt, *J =* 6.8, 3.4 Hz, 1H), 4.11 (dd, *J =* 11.8, 4.9 Hz, 1H), 3.24 (dd, *J =* 16.2, 6.1 Hz, 1H), 3.17 (dd, *J =* 16.0, 6.0 Hz, 1H), 2.92 (dd, *J =* 16.2, 3.3 Hz, 1H), 2.85 (dd, *J =* 16.1, 3.3 Hz, 1H), 2.73 (ddd, *J =* 17.3, 12.0, 5.3 Hz, 1H), 2.56 (dt, *J =* 17.4, 4.2 Hz, 1H), 2.37 - 2.24 (m, 1H), 2.10 (dq, *J* = 13.7, 4.7 Hz, 1H).

### Example 17: Synthesis of Compound 17

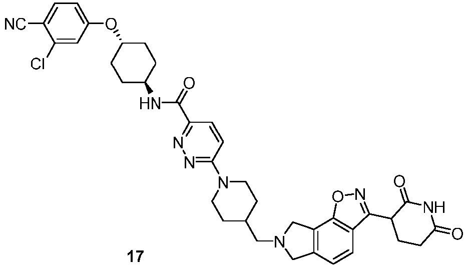

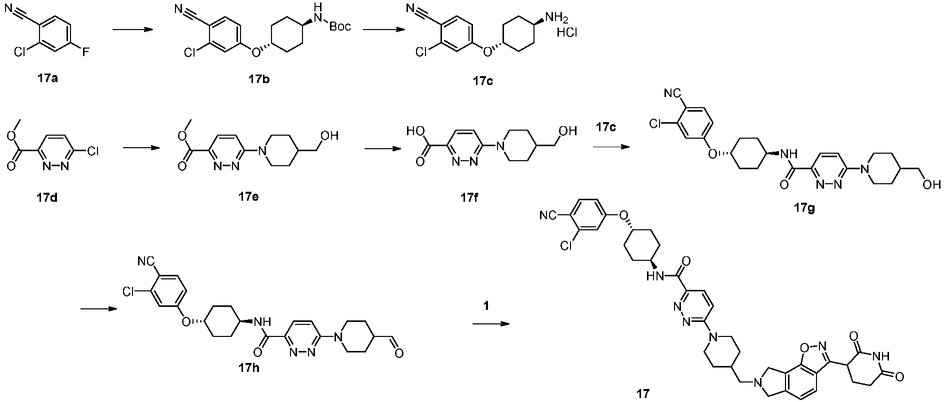

### Step 1: Preparation of intermediate 17b

Sodium hydride (60 wt%, 7.7 g) was added in portions to a solution of **17a** (20.0 g) and *trans-*4-Boc-aminocyclohexanol (27.7 g) in DMF (100 mL) at 0 °C under nitrogen atmosphere. After the addition, the mixture was reacted at room temperature. After the reaction was completed, the reaction solution was slowly poured into ice water (400 mL), and the mixture was stirred for 10 min and filtered. The filter cake was collected and dried to give **17b** (39.1 g).

MS(ESI, [M+H]⁺) *m*/*z:* 351.1.

¹H NMR (500 MHz, DMSO-d6) δ 7.84 (d, *J =* 8.7 Hz, 1H), 7.37 *J =* 2.4 Hz, 1H), 7.11 (dd, *J* = 8.8, 2.4 Hz, 1H), 6.90 - 6.80 (m, 1H), 4.49 (ddt, *J =* 14.2, 9.8, 4.1 Hz, 1H), 3.29 (t, *J =* 8.4 Hz, 1H), 2.09 - 1.99 (m, 2H), 1.87 - 1.74 (m, 2H), 1.38 (s, 13H).

### Step 2: Preparation of intermediate 17c

**17b** (20.5 g) and a solution of hydrochloric acid in 1,4-dioxane (100 mL) were added to a reaction flask in sequence, and the mixture was reacted at room temperature. The reaction solution was concentrated by rotary evaporation to remove the solvent, and the residue was slurried with n-hexane at room temperature and filtered. The filter cake was collected and dried to give **17c** (18.3 g).

MS(ESI, [M+H]⁺) *m*/*z:* 251.1.

### Step 3: Preparation of intermediate 17e

**17d** (10.0 g), 4-piperidinemethanol (8.1 g), DIPEA (11.2 g), and DMSO (90 mL) were added to a reaction flask in sequence, and the reaction solution was warmed to 90 °C and reacted. After the reaction was completed, the reaction solution was poured into water (300 mL), and the mixture was extracted with DCM. The organic phase was dried over anhydrous sodium sulfate, filtered, and concentrated to give intermediate **17e** (12.0 g).

MS(ESI, [M+H]⁺) *m*/*z:* 252.0.

¹H NMR (500 MHz, DMSO-d6) δ 7.80 (d, *J* = 9.7 Hz, 1H), 7.27 (d, *J=* 9.7 Hz, 1H), 4.58 - 4.46 (m, 3H), 3.86 (s, 3H), 3.28 (t, *J=* 5.6 Hz, 2H), 3.00 (td, *J=* 12.8, 2.6 Hz, 2H), 1.84 - 1.67 (m, 3H), 1.21 - 1.07 (m, 2H).

### Step 3: Preparation of intermediate 17f

**17e** (10.1 g), sodium hydroxide (2.4 g), methanol (100 mL), and water (10 mL) were added to a reaction flask in sequence, and the mixture was reacted at room temperature for 1 h. After the reaction was completed, the reaction solution was adjusted to pH 3-4 with concentrated hydrochloric acid and concentrated by rotary evaporation to remove the solvent. The residue was slurried with DCM:MEOH and filtered, and the filtrate was concentrated to dryness to give intermediate **17f** (1.2 g).

MS(ESI, [M-H]⁻) *m*/*z:* 236.2.

¹H NMR (500 MHz, DMSO-d6) δ 7.91 (ddd, *J =* 9.8, 5.4, 2.9 Hz, 1H), 7.57 (d, *J =* 9.3 Hz, 1H), 4.51 (d, *J =* 13.4 Hz, 2H), 3.40 - 3.24 (m, 2H), 3.20 - 3.16 (m, 2H), 3.11 (t, *J =* 12.8 Hz, 2H), 1.78 (t, *J =* 14.5 Hz, 3H), 1.29 - 1.14 (m, 2H).

### Step 4: Preparation of intermediate 17g

**17f**(1.1 g), **17c** (1.1 g), HATU (2.2 g), DIPEA (1.5 g), and DMF (20 mL) were added to a reaction flask in sequence, and the mixture was reacted at room temperature. After the reaction was completed, ethyl acetate (70 mL) and water (150 mL) were added. The organic phase was separated, washed with a saturated sodium chloride solution, dried over anhydrous sodium sulfate, and filtered, and the concentrate was separated and purified by silica gel column chromatography to give intermediate **17g** (1.2 g).

MS(ESI, [M+H]⁺) *m*/*z:* 470.4.

¹H NMR (500 MHz, DMSO-d6) δ 8.63 - 8.50 (m, 1H), 7.88 - 7.71 (m, 2H), 7.43 - 7.24 (m, 2H), 7.12 (tq, *J =* 7.7, 4.7, 3.7 Hz, 1H), 4.50 (dtt, *J =* 20.4, 10.3, 4.6 Hz, 4H), 3.90 - 3.76 (m, 1H), 2.97 (q, *J =* 13.1 Hz, 2H), 2.87 (dd, *J* = 14.4, 6.2 Hz, 1H), 2.71 (dd, *J =* 14.3, 6.2 Hz, 1H), 2.07 (d, *J =* 13.1 Hz, 2H), 1.87 (d, *J* = 12.8 Hz, 2H), 1.79 - 1.67 (m, 3H), 1.61 (q, *J =* 12.5 Hz, 2H), 1.57 - 1.44 (m, 2H), 1.20 - 1.04 (m, 2H).

### Step 5: Preparation of intermediate 17h

**17g** (0.5 g), Dess-Martin periodinane (1.3 g), and dichloromethane (30 mL) were added to a reaction flask in sequence, and the mixture was reacted at room temperature. After the reaction was completed, dichloromethane (70 mL) and water (100 mL) were added. The organic phase was separated, washed with a saturated sodium chloride solution, dried over anhydrous sodium sulfate, filtered, and concentrated to give intermediate **17h** (0.6 g).

### Step 6: Preparation of compound 17

**17h** (90 mg), intermediate **1** (60 mg), sodium acetate (16 mg), and DCE/isopropanol (5:1, 20 mL) were added to a reaction flask in sequence, and the mixture was reacted at room temperature for 30 min. Sodium cyanoborohydride (24 mg) was then added, and the mixture was reacted at room temperature. After the reaction was completed, the reaction solution was concentrated by rotary evaporation to remove the solvent, and the crude product was separated and purified by silica gel column chromatography to give compound **17** (30 mg).

MS(ESI, [M+H]⁺) *m*/*z:* 723.4.

¹H NMR (500 MHz, DMSO-*d₆*) δ 11.09 (s, 1H), 8.58 (d, *J* = 8.2 Hz, 1H), 7.85 (d, *J =* 8.8 Hz, 1H), 7.81 (d, *J* = 9.5 Hz, 1H), 7.72 (d, *J* = 8.1 Hz, 1H), 7.41 - 7.28 (m, 3H), 7.14 (dd, *J* = 8.7, 2.4 Hz, 1H), 4.60 (dd, *J =* 11.9, 5.0 Hz, 1H), 4.57 - 4.46 (m, 3H), 4.16 (d, *J* = 2.4 Hz, 2H), 4.05 (t, *J =* 2.3 Hz, 2H), 3.86 (tdt, *J =* 11.4, 8.1, 4.0 Hz, 1H), 3.11 - 3.01 (m, 2H), 2.77 (ddd, *J* = 17.2, 11.9, 5.3 Hz, 1H), 2.69 - 2.57 (m, 3H), 2.20 (dq, *J* = 13.5, 4.8 Hz, 1H), 2.16 - 2.06 (m, 2H), 1.91 (d, *J* = 13.7 Hz, 4H), 1.58 (ddt, *J* = 63.2, 13.6, 10.8 Hz, 5H), 1.19 (d, *J* = 13.4 Hz, 3H).

### Example 18: Synthesis of Compound 18

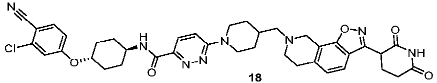

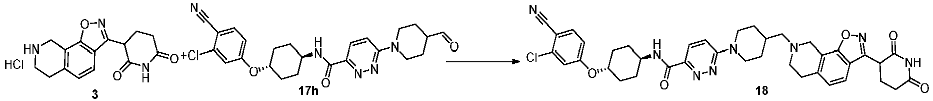

Intermediate **3** (70 mg), intermediate **17h** (102 mg), sodium acetate (17.85 mg), 1,2-dichloroethane (5 mL), isopropanol (1 mL), and sodium cyanoborohydride (41.0 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by a silica gel column to give compound **18** (42 mg).

MS(ESI, [M+H]⁺) m/z: 737.3.

¹H NMR (500 MHz, DMSO-d6) δ 11.09 (s, 1H), 8.59 (d, J = 8.2 Hz, 1H), 7.85 (d, J = 8.8 Hz, 1H), 7.80 (d, J = 9.5 Hz, 1H), 7.60 (d, J = 8.1 Hz, 1H), 7.39 (d, J = 2.4 Hz, 1H), 7.34 (d, J = 9.6 Hz, 1H), 7.14 (t, J = 7.6 Hz, 2H), 4.55 (td, J = 10.9, 10.0, 4.8 Hz, 2H), 4.49 (d, J = 13.4 Hz, 2H), 3.86 (dd, J = 9.5, 5.0 Hz, 1H), 3.82 (s, 2H), 3.06 (t, J = 12.6 Hz, 2H), 2.97 (t, J = 5.7 Hz, 2H), 2.76 (p, J = 6.2, 5.6 Hz, 3H), 2.60 (dt, J = 17.4, 4.3 Hz, 1H), 2.48 (s, 1H), 2.44 (d, J = 7.3 Hz, 2H), 2.18 (dq, J = 13.2, 4.5 Hz, 1H), 2.13 - 2.04 (m, 3H), 1.88 (t, J = 14.1 Hz, 4H), 1.64 (q, J = 12.2 Hz, 2H), 1.55 - 1.47 (m, 2H), 1.15 (dd, J = 18.0, 7.8 Hz, 2H).

### Example 19: Synthesis of Compound 19

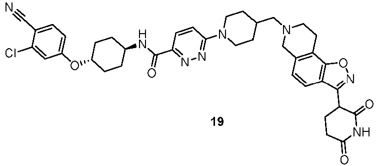

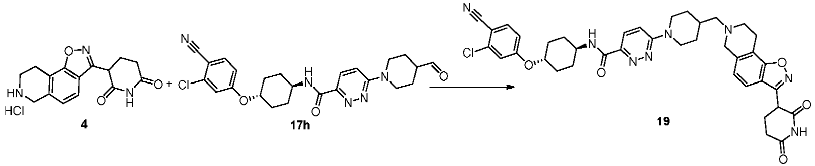

Intermediate 4 (70 mg), intermediate **17h** (102 mg), sodium acetate (17.85 mg), 1,2-dichloroethane (5 mL), isopropanol (1 mL), and sodium cyanoborohydride (41.0 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were then combined, washed with saturated brine, dried over anhydrous sodium sulfate, and filtered, and the filtrate was purified by a silica gel column to give compound **19** (48 mg).

MS(ESI, [M+H]⁺) m/z: 737.3.

¹H NMR (500 MHz, DMSO-d6) δ 11.09 (s, 1H), 8.59 (d, J = 8.1 Hz, 1H), 7.85 (d, J = 8.8 Hz, 1H), 7.80 (d, J = 9.5 Hz, 1H), 7.58 (d, J = 8.1 Hz, 1H), 7.39 (d, J = 2.4 Hz, 1H), 7.34 (d, J = 9.6 Hz, 1H), 7.16 - 7.08 (m, 2H), 4.56 (dt, J = 10.5, 5.2 Hz, 2H), 4.49 (d, J = 13.1 Hz, 2H), 3.90 - 3.82 (m, 1H), 3.71 (s, 2H), 3.05 (d, J = 12.6 Hz, 2H), 3.00 (t, J = 6.1 Hz, 2H), 2.76 (dt, J = 17.3, 5.6 Hz, 3H), 2.61 (dt, J = 17.5, 4.3 Hz, 1H), 2.47 (s, 1H), 2.39 (d, J = 7.2 Hz, 2H), 2.19 (dq, J = 13.3, 4.5 Hz, 1H), 2.10 (d, J = 12.0 Hz, 2H), 2.04 (s, 1H), 1.88 (t, J = 16.2 Hz, 4H), 1.64 (q, J = 12.5 Hz, 2H), 1.51 (q, J = 12.0 Hz, 2H), 1.16 (d, J = 12.4 Hz, 2H).

### Example 20: Synthesis of Compound 20

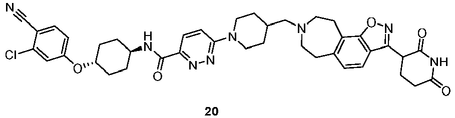

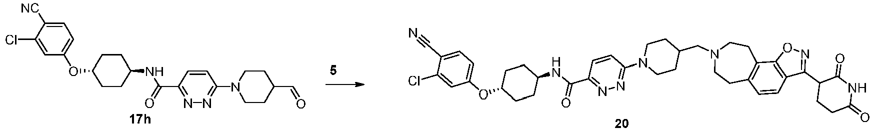

### Step 1: Preparation of compound 20

**17h** (95 mg), intermediate **5** (60 mg), sodium acetate (18 mg), and DCE/isopropanol (5:1, 20 mL) were added to a reaction flask in sequence, and the mixture was reacted at room temperature for 30 min. Sodium cyanoborohydride (26 mg) was then added, and the mixture was reacted at room temperature. The reaction solution was concentrated by rotary evaporation to remove the solvent, and the crude product was separated and purified by silica gel column chromatography to give compound **20** (22 mg).

MS(ESI, [M+H]⁺) *m*/*z:* 751.6.

¹H NMR (500 MHz, DMSO-*d₆*) δ 11.08 (s, 1H), 8.58 *(d, J=* 8.2 Hz, 1H), 7.83 (dd, *J=* 24.4, 9.1 Hz, 2H), 7.54 (d, *J= 8.0* Hz, 1H), 7.41 - 7.30 (m, 2H), 7.23 - 7.10 (m, 2H), 4.61 - 4.44 (m, 4H), 3.93 - 3.80 (m, 1H), 3.20 - 3.10 (m, 2H), 3.09 - 2.98 (m, 4H), 2.77 (ddd, *J* = 17.2, 12.0, 5.3 Hz, 1H), 2.69 - 2.65 (m, 2H), 2.65 - 2.61 (m, 2H), 2.34 (d, *J=* 7.0 Hz, 2H), 2.18 (dq, *J* = 8.8, 4.3 Hz, 1H), 2.14 - 2.06 (m, 2H), 1.89 *(t, J=* 15.3 Hz, 5H), 1.64 (dt, *J* = 13.7, 11.0 Hz, 2H), 1.59 - 1.45 (m, 3H), 1.17 *(t, J=* 11.3 Hz, 3H).

### Example 21: Synthesis of Compound 21

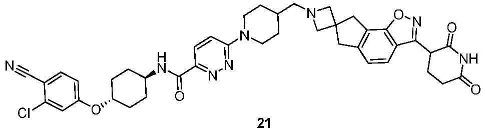

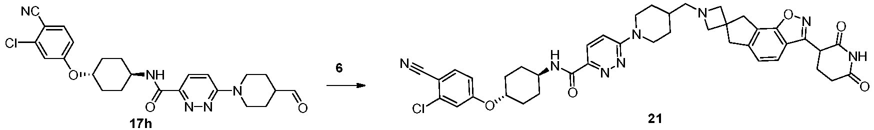

### Step 1: Preparation of compound 21

**17h** (85 mg), intermediate **6** (66 mg), sodium acetate (20 mg), and DCE/isopropanol (5:1, 20 mL) were added to a reaction flask in sequence, and the mixture was reacted at room temperature for 30 min. Sodium cyanoborohydride

(28 mg) was then added, and the mixture was reacted at room temperature. After the reaction was completed, the reaction solution was concentrated by rotary evaporation to remove the solvent, and the crude product was separated and purified by silica gel column chromatography to give compound **21** (42 mg).

MS(ESI, [M+H]⁺) *m*/*z:* 763.5.

¹H NMR (500 MHz, DMSO-d6) δ 11.08 (s, 1H), 8.58 (d, J = 8.2 Hz, 1H), 7.86 (d, J = 8.8 Hz, 1H), 7.79 (d, J = 9.6 Hz, 1H), 7.62 (d, J = 8.0 Hz, 1H), 7.39 (d, J = 2.4 Hz, 1H), 7.32 (d, J = 9.6 Hz, 1H), 7.26 (d, J = 8.2 Hz, 1H), 7.14 (dd, J = 8.8, 2.4 Hz, 1H), 4.55 (ddd, J = 16.3, 11.2, 5.4 Hz, 2H), 4.46 (d, J = 13.2 Hz, 2H), 3.91 - 3.81 (m, 1H), 2.98 (t, J = 12.5 Hz, 2H), 2.77 (ddd, J = 17.2, 12.0, 5.3 Hz, 1H), 2.60 (dt, J = 17.4, 4.2 Hz, 1H), 2.32 (s, 2H), 2.17 (dq, J = 13.4, 4.7 Hz, 1H), 2.10 (d, J = 11.7 Hz, 2H), 1.93 - 1.86 (m, 2H), 1.84 - 1.76 (m, 2H), 1.69 - 1.59 (m, 3H), 1.58 - 1.45 (m, 3H), 1.18 - 1.09 (m, 3H).

### Example 22: Synthesis of Compound 22

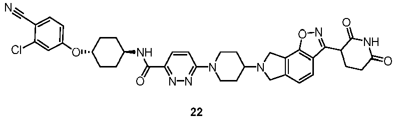

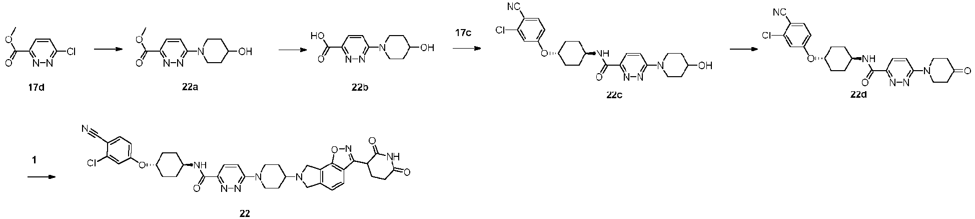

### Step 1: Preparation of intermediate 22a

**17d** (10.0 g), 4-hydroxypiperidine (7.1 g), DIPEA (11.2 g), and DMSO (90 mL) were added to a reaction flask, and the reaction solution was warmed to 90 °C and reacted for 2 h. After the reaction was completed, the reaction solution was poured into water (300 mL), and the mixture was filtered. The filter cake was collected and dried to give intermediate **22a** (8.2 g).

MS(ESI, [M+H]⁺) *m*/*z:* 238.1.

¹H NMR (500 MHz, DMSO-d6) δ 7.80 (d, *J=* 9.6 Hz, 1H), 7.29 (d, *J=* 9.7 Hz, 1H), 4.79 (d, *J=* 4.2 Hz, 1H), 4.16 (dt, *J=* 13.6, 4.8 Hz, 2H), 3.87 (s, 3H), 3.79 (tq, *J* = 8.2, 4.0 Hz, 1H), 3.38 (ddd, *J* = 13.2, 9.6, 3.3 Hz, 2H), 1.87 - 1.79 (m, 2H), 1.45 - 1.35 (m, 2H).

### Step 2: Preparation of intermediate 22b

**22a** (8.3 g), sodium hydroxide (2.8 g), methanol (70 mL), and water (10 mL) were added to a reaction flask in sequence, and the mixture was reacted at room temperature. After the reaction was completed, the reaction solution was adjusted to pH 3-4 with concentrated hydrochloric acid and concentrated by rotary evaporation to remove the solvent. The residue was slurried with DCM:MeOH and filtered, and the filtrate was concentrated to dryness to give intermediate **22b** (1.2 g).

MS(ESI, [M-H]⁻) *m*/*z:* 222.1.

¹H NMR (500 MHz, DMSO-d6) δ 7.80 *(d, J=* 9.5 Hz, 1H), 7.27 *(d, J=* 9.6 Hz, 1H), 4.81 (s, 1H), 4.14 (dt, *J* = 13.5, 4.7 Hz, 2H), 3.77 (tt, *J=* 8.4, 3.9 Hz, 1H), 3.34 - 3.30 (m, 3H), 1.82 (ddd, *J* = 13.1, 5.8, 3.3 Hz, 2H), 1.39 (ddt, *J*= 13.4, 9.1, 4.6 Hz, 2H).

### Step 3: Preparation of intermediate 22c

**22b** (1.5 g), **17c** (1.3 g), HATU (3.0 g), DIPEA(2.1 g), and DMF (30 mL) were added to a reaction flask in sequence, and the mixture was reacted at room temperature. After the reaction was completed, ethyl acetate (70 mL) and water (150 mL) were added. The organic phase was separated, washed with a saturated sodium chloride solution, dried over anhydrous sodium sulfate, and filtered, and the concentrate was separated and purified by silica gel column chromatography to give intermediate **22c** (1.4 g).

MS(ESI, [M+H]⁺) *m*/*z:* 456.3.

¹H NMR (500 MHz, DMSO-d6) δ 8.58 (d, *J* = 8.2 Hz, 1H), 7.83 (dd, *J* = 25.8, 9.1 Hz, 2H), 7.41 - 7.32 (m, 2H), 7.14 (dd, *J =* 8.8, 2.4 Hz, 1H), 4.77 (d, *J =* 4.2 Hz, 1H), 4.54 (tt, *J =* 10.3, 4.2 Hz, 1H), 4.14 (dt, *J =* 13.5, 4.7 Hz, 2H), 3.83 (ddtd, *J=* 36.7, 12.6, 8.5, 8.0, 4.0 Hz, 2H), 3.35 (td, *J =* 9.8, 4.7 Hz, 2H), 2.14 - 2.07 (m, 2H), 1.90 (dd, *J=* 13.1, 3.8 Hz, 2H), 1.85 - 1.77 (m, 2H), 1.69 - 1.58 (m, 2H), 1.57 - 1.46 (m, 2H), 1.39 (dtd, *J=* 12.9, 9.1, 3.8 Hz, 2H).

### Step 4: Preparation of intermediate 22d

**22c** (0.5 g), Dess-Martin periodinane (1.4 g), and dichloromethane (30 mL) were added to a reaction flask in sequence, and the mixture was reacted at room temperature for 1 h. After the reaction was completed, dichloromethane (70 mL) and water (100 mL) were added. The organic phase was separated, washed with a saturated sodium chloride solution, dried over anhydrous sodium sulfate, filtered, and concentrated to give intermediate **22d** (0.6 g).

### Step 5: Preparation of compound 22

**22d** (88 mg), intermediate **1** (69 mg), sodium acetate (25 mg), and DMA (20 mL) were added to a reaction flask in sequence, and the mixture was reacted at room temperature for 30 min. Sodium cyanoborohydride (31 mg) was then added, and the mixture was reacted at room temperature for 2 h. The reaction solution was concentrated by rotary evaporation to remove the solvent, and the crude product was separated and purified by silica gel column chromatography to give compound **22** (32 mg).

MS(ESI, [M+H]⁺) *m*/*z:* 709.3.

¹H NMR (500 MHz, DMSO-d6) δ 11.10 (s, 1H), 8.61 (d, *J* = 8.2 Hz, 1H), 7.84 (dd, *J =* 14.1, 9.1 Hz, 2H), 7.72 (d, *J =* 8.0 Hz, 1H), 7.43 - 7.37 (m, 2H), 7.32 (d, *J =* 8.2 Hz, 1H), 7.14 (dd, *J =* 8.8, 2.4 Hz, 1H), 4.60 (dd, *J =* 11.9, 5.0 Hz, 1H), 4.54 (tt, *J* = 9.7, 4.0 Hz, 1H), 4.40 - 4.29 (m, 2H), 4.23 (s, 2H), 4.12 (s, 2H), 3.91 - 3.81 (m, 1H), 3.29 (d, *J =* 10.7 Hz, 2H), 2.86 (d, *J* = 10.1 Hz, 1H), 2.77 (ddd, *J* = 17.2, 11.9, 5.3 Hz, 1H), 2.61 (dt, *J* = 17.3, 4.3 Hz, 1H), 2.21 (dq, *J=* 8.6, 4.5, 3.9 Hz, 1H), 2.14 - 2.08 (m, 2H), 2.08 - 2.00 (m, 2H), 1.94 - 1.85 (m, 2H), 1.72 - 1.59 (m, 3H), 1.59 - 1.45 (m, 4H).

### Example 23: Synthesis of Compound 23

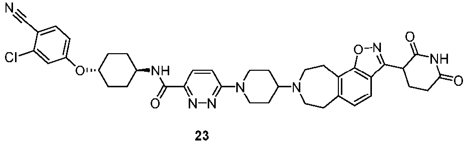

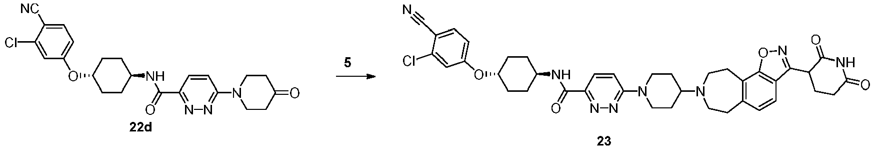

### Step 1: Preparation of compound 23

**22d** (108 mg), intermediate **5** (79 mg), sodium acetate (28 mg), and DMA (20 mL) were added to a reaction flask in sequence, and the mixture was reacted at room temperature for 30 min. Sodium cyanoborohydride (33 mg) was then added, and the mixture was reacted at room temperature. The reaction solution was concentrated by rotary evaporation to remove the solvent, and the crude product was separated and purified by silica gel column chromatography to give compound **23** (27 mg).

MS(ESI, [M+H]⁺) *m*/*z:* 737.4.

¹H NMR (500 MHz, DMSO-d6) δ 11.07 (s, 1H), 8.59 (d, *J* = 8.2 Hz, 1H), 7.83 (dd, *J* = 24.2, 9.2 Hz, 2H), 7.53 (d, *J =* 8.0 Hz, 1H), 7.43 - 7.30 (m, 2H), 7.23 - 7.10 (m, 2H), 4.54 (td, *J =* 11.0, 9.6, 5.9 Hz, 4H), 3.86 (dtd, *J* = 11.3, 7.6, 4.1 Hz, 1H), 3.13 (t, *J =* 4.9 Hz, 2H), 2.98 (dt, *J =* 31.3, 12.8 Hz, 5H), 2.82 - 2.64 (m, 5H), 2.60 (dt, *J* = 17.3, 4.2 Hz, 1H), 2.46 (dd, *J=* 12.1, 4.5 Hz, 1H), 2.18 (dq, *J=* 8.5, 4.4 Hz, 1H), 2.14 - 2.05 (m, 2H), 1.90 (d, *J=* 13.6 Hz, 2H), 1.80 (d, *J=* 12.2 Hz, 2H), 1.70 - 1.58 (m, 2H), 1.51 (qd, *J=* 12.4, 6.0 Hz, 4H).

### Examples 24 and 25: Synthesis of Compounds 24 and 25

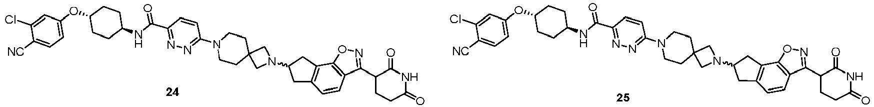

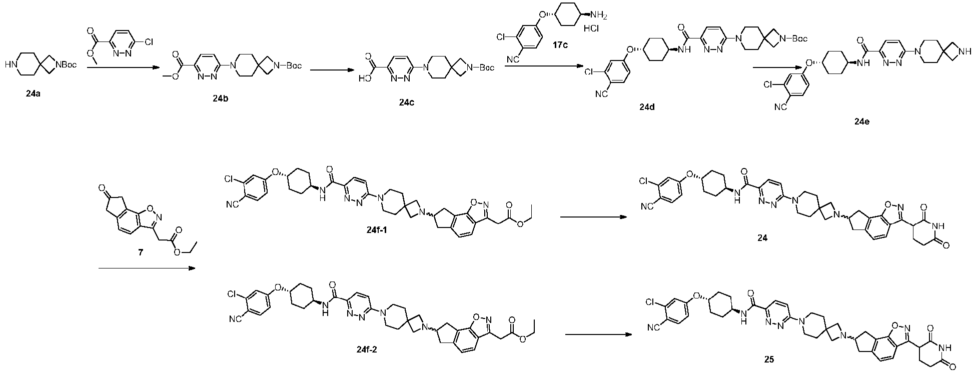

### Step 1: Preparation of intermediate 24b

24a (1.443 g), DMSO (10 mL), DIPEA (2.247 g, 3.08 mL), and methyl 6-chloropyridazine-3-carboxylate (1 g) were added to a reaction flask in sequence, and the mixture was reacted at 90 °C. After the reaction was completed, the mixture was poured into ice water, and the mixture was filtered under vacuum. The filter cake was dried to give 24b (2.7 g).

MS(ESI, [M+H]⁺) m/z: 363.16.

¹H NMR (500 MHz, DMSO-d6) δ 7.81 (d, *J=* 9.6 Hz, 1H), 7.32 (d, *J=* 9.7 Hz, 1H), 3.86 (s, 3H), 3.71 (s, 8H), 1.74 (t, *J=* 5.6 Hz, 4H), 1.39 (s, 9H).

### Step 2: Preparation of intermediate 24c

**24b** (2.6 g), MeOH (30 mL), and water (3 mL) were added to a reaction flask in sequence, sodium hydroxide (1.43

g) was then added, and the mixture was reacted at room temperature. After the reaction was completed, the system was adjusted to pH 4-5 with 6 M hydrochloric acid, concentrated to remove the solvent, and then slurried with

MeOH/DCM (v:v = 1/10) (about 100 mL). The solid was removed by filtration under vacuum, and the mother liquor was concentrated to give **24c** (1.9 g).

MS(ESI, [M+H]⁺) m/z: 349.25.

### Step 3: Preparation of intermediate 24d

**17c** (0.85 g), **24c** (1.031 g), DCM (10 mL), HATU (1.68 g), and DIPEA (1.148 g, 1.551 mL) were added to a reaction flask in sequence, and the mixture was reacted at room temperature. After the reaction was completed, the reaction solution was diluted with ethyl acetate (100 mL), washed with a 10% citric acid aqueous solution (100 mL), and then washed with a saturated sodium bicarbonate solution (100 mL). The organic phase was dried over anhydrous sodium sulfate, filtered, and concentrated to give **24d** (1.61 g).

MS(ESI, [M+H]⁺) m/z: 581.28.

### Step 4: Preparation of intermediate 24e

**24d** (1.61 g), DCM (20 mL), and trifluoroacetic acid (5 mL) were added to a reaction flask in sequence, and the mixture was reacted at room temperature. After the reaction was completed, the mixture was added to a saturated sodium bicarbonate solution (200 mL), and DCM (200 mL) was added for extraction. The organic phase was dried over anhydrous sodium sulfate, filtered, and concentrated to give **24e** (1.3 g).

MS(ESI, [M+H]⁺) m/z: 481.22.

¹H NMR (500 MHz, DMSO-d6) δ 8.59 (dd, *J=* 8.2, 5.4 Hz, 1H), 7.84 (dd, *J* = 20.6, 9.1 Hz, 2H), 7.41 - 7.33 (m, 2H), 7.13 (dd, *J=* 8.8, 2.4 Hz, 1H), 4.53 (tt, *J=* 9.9, 4.2 Hz, 1H), 3.85 (dtd, *J=* 15.2, 7.8, 3.9 Hz, 1H), 3.68 (t, *J*= 5.7 Hz, 4H), 3.57 (s, 4H), 2.16 - 2.05 (m, 2H), 1.94 - 1.85 (m, 2H), 1.84 - 1.76 (m, 3H), 1.74 - 1.44 (m, 5H). Step 5: Preparation of intermediates **24f-1 and 24f-2**

**24e** (350 mg), 1,2-dichloroethane (10 mL), isopropanol (3 mL), intermediate **7** (208 mg), and one drop of acetic acid were added to a reaction flask in sequence, then sodium cyanoborohydride (137 mg) was added, and the mixture was reacted at room temperature overnight. After the reaction was completed, a saturated sodium bicarbonate solution (20 mL) and water (50 mL) were added to the reaction solution, and then dichloromethane was added for extraction. The organic phase was separated, dried over anhydrous sodium sulfate, filtered, and concentrated, and the residue was purified by silica gel column chromatography to give a crude product. The crude product was separated by preparative high performance liquid chromatography to give prepeak intermediate **24f-1** (95 mg) and postpeak intermediate **24f-2** (85 mg) in sequence.

The conditions for the preparative chromatography were as follows: Instrument and preparative column: YMC high pressure preparative chromatograph was used, and the preparative column was CHIRALART Cellose-SB. Mobile phase system: ethanol:dichloromethane (1:1)/n-hexane, isocratic elution: ethanol:dichloromethane (1:1)/n-hexane = 50/50.

The characterization data of **24f-1** were as follows:
MS(ESI, [M+H]⁺) m/z: 724.30.

¹H NMR (500 MHz, DMSO-d6) δ 8.58 (d, *J=* 8.2 Hz, 1H), 7.85 (d, *J=* 8.7 Hz, 1H), 7.79 (d, *J=* 9.6 Hz, 1H), 7.61 *(d, J=* 8.0 Hz, 1H), 7.41 - 7.32 (m, 2H), 7.27 *(d, J=* 8.1 Hz, 1H), 7.13 (dd, *J* = 8.7, 2.4 Hz, 1H), 4.54 (dq, *J* = 10.5, 5.8, 5.1 Hz, 1H), 4.23 - 4.08 (m, 4H), 3.90 - 3.80 (m, 1H), 3.67 (t, *J=* 5.6 Hz, 4H), 3.46 (s, 1H), 3.13 (d, *J* = 24.8 Hz, 5H), 2.84 (ddd, *J=* 31.4, 16.6, 3.1 Hz, 2H), 2.16 - 2.05 (m, 2H), 1.89 (d, *J=* 9.9 Hz, 3H), 1.71 (t, *J* = 5.6 Hz, 4H), 1.68 - 1.58 (m, 2H), 1.56 - 1.45 (m, 2H), 1.19 (t, *J* = 7.0 Hz, 3H).

### Step 6: Preparation of compound 24

**24f-1** (91 mg), acrylamide (8.93 mg), and THF (10 mL) were added to a reaction flask in sequence, and a 1 M solution of potassium tert-butoxide in tetrahydrofuran (0.088 mL) was added under an ice bath. The mixture was reacted at 0 °C. After the reaction was completed, the reaction solution was added dropwise to an icy saturated ammonium chloride aqueous solution, and ethyl acetate was added for extraction. The organic phase was separated, dried over anhydrous sodium sulfate, filtered, and concentrated, and the residue was purified by silica gel column chromatography to give compound **24** (17 mg).

MS(ESI, [M+H]⁺) m/z: 749.30.

¹H NMR (500 MHz, DMSO-d6) δ 11.09 (s, 1H), 8.57 (d, *J =* 8.2 Hz, 1H), 7.85 (d, *J =* 8.7 Hz, 1H), 7.79 (d, *J =* 9.5 Hz, 1H), 7.62 (d, *J =* 8.1 Hz, 1H), 7.42 - 7.31 (m, 2H), 7.26 (d, *J =* 8.1 Hz, 1H), 7.13 (dd, *J =* 8.8, 2.4 Hz, 1H), 4.60 - 4.49 (m, 2H), 3.85 (d, *J* = 10.3 Hz, 1H), 3.67 (t, *J* = 5.4 Hz, 4H), 3.60 (d, *J* = 6.1 Hz, 1H), 3.06 (s, 5H), 2.89 - 2.74 (m, 3H), 2.66 - 2.55 (m, 2H), 2.18 (dd, *J =* 13.1, 5.2 Hz, 1H), 2.10 (d, *J =* 11.8 Hz, 2H), 2.00 (q, *J =* 7.7 Hz, 1H), 1.93 - 1.85 (m, 2H), 1.74 (d, *J* = 21.8 Hz, 4H), 1.63 (d, *J =* 12.4 Hz, 2H), 1.51 (d, *J* = 12.0 Hz, 2H).

### Step 7: Preparation of compound 25

**24f-2** (80 mg), acrylamide (7.93 mg), and THF (10 mL) were added to a reaction flask in sequence, and a 1 M solution of potassium *tert*-butoxide in tetrahydrofuran (0.078 mL) was added under an ice bath. The mixture was reacted at 0 °C. After the reaction was completed, the reaction solution was added dropwise to an icy saturated ammonium chloride aqueous solution, and ethyl acetate was added for extraction. The organic phase was separated, dried over anhydrous sodium sulfate, filtered, and concentrated, and the residue was purified by silica gel column chromatography to give compound **25** (15 mg).

MS(ESI, [M+H]⁺) m/z: 749.52.

¹H NMR (500 MHz, DMSO-d6) δ 11.09 (s, 1H), 8.57 *(d, J=* 8.2 Hz, 1H), 7.85 (d, *J=* 8.7 Hz, 1H), 7.79 (d, *J* = 9.5 Hz, 1H), 7.62 (d, *J =* 8.1 Hz, 1H), 7.42 - 7.31 (m, 2H), 7.26 (d, *J* = 8.1 Hz, 1H), 7.13 (dd, *J* = 8.8, 2.4 Hz, 1H), 4.60 - 4.49 (m, 2H), 3.85 (d, *J* = 10.3 Hz, 1H), 3.67 (t, *J* = 5.4 Hz, 4H), 3.60 (d, *J* = 6.1 Hz, 1H), 3.06 (s, 5H), 2.89 - 2.74 (m, 3H), 2.66 - 2.55 (m, 2H), 2.18 (dd, *J =* 13.1, 5.2 Hz, 1H), 2.10 (d, *J* = 11.8 Hz, 2H), 2.00 (q, *J* = 7.7 Hz, 1H), 1.93 - 1.85 (m, 2H), 1.74 (d, *J=* 21.8 Hz, 4H), 1.63 (d, *J =* 12.4 Hz, 2H), 1.51 (d, *J* = 12.0 Hz, 2H).

### Example 26: Synthesis of Compound 26

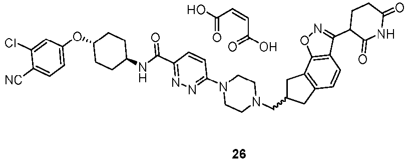

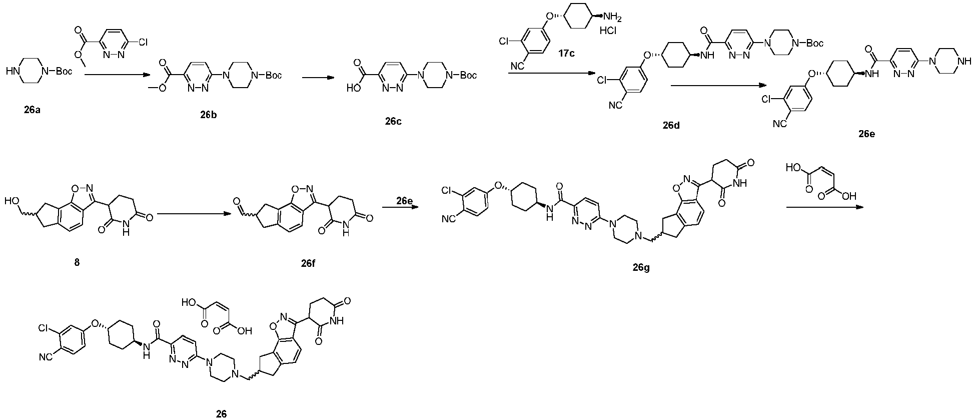

### Step 1: Preparation of intermediate 26b

**26a** (5.94 g), DMSO (30 mL), DIPEA (1.23 g, 15.39 mL), and methyl 6-chloropyridazine-3-carboxylate (5 g) were added to a reaction flask in sequence, and the mixture was reacted at 90 °C. After the reaction was completed, the mixture was poured into ice water, and the mixture was filtered under vacuum. The filter cake was dried to give **26b** (11.3 g).

MS(ESI, [M+H]⁺) m/z: 323.07.

¹H NMR (500 MHz, DMSO-d6) δ 7.87 (d, *J* = 9.6 Hz, 1H), 7.29 (d, *J=* 9.7 Hz, 1H), 3.87 (s, 3H), 3.80 - 3.69 (m, 4H), 3.47 (dd, *J* = 6.3, 4.0 Hz, 4H), 1.43 (s, 9H).

### Step 2: Preparation of intermediate 26c

**26b** (11.3 g), MeOH (100 mL), and water (30 mL) were added to a reaction flask in sequence, sodium hydroxide (7.01 g) was then added, and the mixture was reacted at room temperature. After the reaction was completed, the system was adjusted to pH 4-5 with 6 M hydrochloric acid, concentrated to remove the solvent, and then slurried with MeOH/DCM (v:v = 1/10) (about 100 mL). The solid was removed by filtration under vacuum, and the mother liquor was concentrated to give **26c** (9.8 g).

MS(ESI, [M+H]⁺) m/z: 308.99.

### Step 3: Preparation of intermediate 26d

**17c** (2.5 g), **26c** (2.68 g), DCM (30 mL), HATU (4.30 g), and DIPEA (5.63 g, 7.60 mL) were added to a reaction flask in sequence, and the mixture was reacted at room temperature. After the reaction was completed, the system was diluted with ethyl acetate (100 mL), washed with a 10% citric acid aqueous solution (100 mL), and then washed with a saturated sodium bicarbonate solution. The organic phase was dried over anhydrous sodium sulfate, filtered, and concentrated to give **26d** (3.2 g).

MS(ESI, [M+H]⁺) m/z: 541.21.

### Step 4: Preparation of intermediate 26e

**26d** (3.2 g), DCM (20 mL), and trifluoroacetic acid (5 mL) were added to a reaction flask in sequence, and the mixture was reacted at room temperature. After the reaction was completed, the mixture was added to a saturated sodium bicarbonate solution (200 mL) and extracted with DCM. The organic phase was dried over anhydrous sodium sulfate, filtered, and concentrated to give **26e** (2.56 g).

MS(ESI, [M+H]⁺) m/z: 441.20.

¹H NMR (500 MHz, DMSO-d6) δ 8.62 (d, *J=* 8.2 Hz, 1H), 7.85 (t, *J* = 9.3 Hz, 2H), 7.39 (d, *J=* 2.4 Hz, 1H), 7.34 (d, *J* = 9.6 Hz, 1H), 7.14 (dd, *J* = 8.8, 2.4 Hz, 1H), 4.53 (tt, *J=* 10.3, 4.2 Hz, 1H), 3.86 (tdt, *J* = 11.8, 8.2, 4.0 Hz, 1H), 3.77 - 3.61 (m, 4H), 2.99 - 2.80 (m, 4H), 2.16 - 2.05 (m, 2H), 1.95 - 1.84 (m, 2H), 1.64 (qd, *J* = 13.1, 3.1 Hz, 2H), 1.58 - 1.45 (m, 2H).

### Step 5: Preparation of intermediate 26f

Intermediate **8** (50 mg), Dess-Martin periodinane (141 mg), dichloromethane (5 mL), and DMF (1 mL) were added to a reaction flask in sequence, and the mixture was reacted at room temperature. After the reaction was completed, the reaction solution was extracted with water (50 mL) and ethyl acetate (20 mL). The organic phases were combined, washed with a saturated sodium chloride solution, dried over anhydrous sodium sulfate, and filtered, and the filtrate was concentrated by evaporation under reduced pressure to remove the solvent, thus giving the target intermediate **26f** (50 mg).

MS(ESI, [M+H]⁺) m/z: 299.51.

### Step 6: Preparation of intermediate 26g

Intermediate **26f** (50 mg), 1,2-dichloroethane (6 mL), isopropanol (2 mL), intermediate **26e** (88 mg), acetic acid (5.00 mg), and sodium cyanoborohydride (20.93 mg) were added to a reaction flask in sequence, and the mixture was stirred at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, filtered, and concentrated, and the residue was purified by a silica gel column to give compound **26g** (30 mg).

MS(ESI, [M+H]⁺) m/z: 723.36.

### Step 7: Preparation of compound 26

**26g** (30 mg), maleic acid (4.81 mg), and MeOH/DCM (v:v = 1/10, 5 mL) were added to a reaction flask in sequence, and after complete dissolution, the reaction solution was concentrated. The reaction solution was slurried with petroleum ether (10 mL) and filtered under vacuum to give **26** (32 mg).

MS(ESI, [M+H]⁺) m/z: 723.43.

¹H NMR (500 MHz, DMSO-d6) δ 11.09 (s, 1H), 8.66 *(d, J=* 8.2 Hz, 1H), 7.93 *(d, J=* 9.4 Hz, 1H), 7.86 *(d, J=* 8.7 Hz, 1H), 7.66 (d, *J=* 8.2 Hz, 1H), 7.54 - 7.42 (m, 1H), 7.39 (d, *J=* 2.5 Hz, 1H), 7.30 (d, *J=* 8.1 Hz, 1H), 7.13 (dd, *J=* 8.9, 2.5 Hz, 1H), 6.11 (s, 2H), 4.56 (dq, *J=* 22.4, 6.0, 5.1 Hz, 2H), 3.93 - 3.83 (m, 1H), 3.17 (s, 13H), 3.09 (s, 1H), 3.01 *(d, J=* 16.1 Hz, 1H), 2.90 (dd, *J* = 16.4, 6.1 Hz, 1H), 2.77 (td, *J* = 12.0, 5.8 Hz, 1H), 2.61 *(d, J=* 18.1 Hz, 1H), 2.25 - 2.05 (m, 3H), 1.96 - 1.83 (m, 2H), 1.65 (q, *J =* 12.4 Hz, 2H), 1.59 - 1.42 (m, 2H).

### Example 27: Synthesis of Compound 27

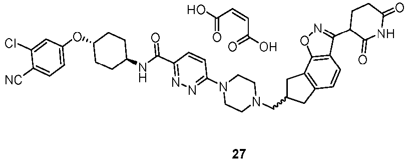

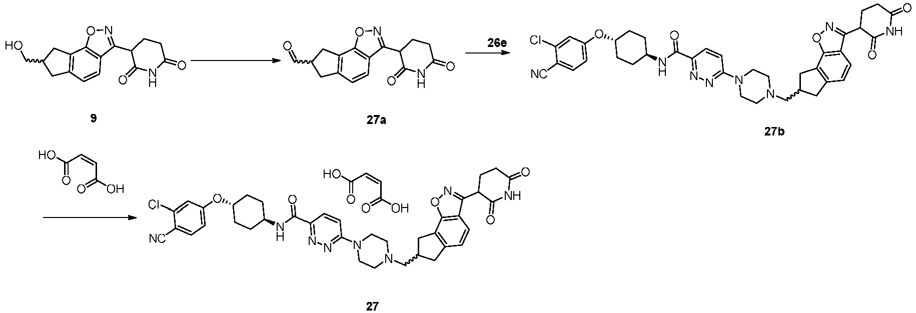

### Step 1: Preparation of intermediate 27a

Intermediate **9** (50 mg), Dess-Martin periodinane (141 mg), dichloromethane (5 mL), and DMF (1 mL) were added to a reaction flask in sequence, and the mixture was reacted at room temperature. After the reaction was completed, the reaction solution was extracted with water (50 mL) and ethyl acetate (20 mL). The organic phases were combined, washed with a saturated sodium chloride solution, dried over anhydrous sodium sulfate, and filtered, and the filtrate was concentrated by evaporation under reduced pressure to remove the solvent, thus giving the target intermediate **27a** (50 mg).

MS(ESI, [M+H]⁺) m/z: 299.31.

### Step 6: Preparation of intermediate 27b

Intermediate **27a** (50 mg), 1,2-dichloroethane (6 mL), isopropanol (2 mL), intermediate **26e** (88 mg), acetic acid (5.00 mg), and sodium cyanoborohydride (20.93 mg) were added to a reaction flask in sequence, and the mixture was stirred at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, filtered, and concentrated, and the residue was purified by a silica gel column to give compound **27b** (53 mg).

MS(ESI, [M+H]⁺) m/z: 723.42.

### Step 2: Preparation of compound 27

**27b** (53 mg), maleic acid (8.51 mg), and MeOH/DCM (v:v = 1/10, 5 mL) were added to a reaction flask in sequence, and after complete dissolution, the reaction solution was concentrated. The concentrate was slurried with petroleum ether (10 mL) and filtered under vacuum to give **27** (58 mg).

MS(ESI, [M+H]⁺) m/z: 723.52.

¹H NMR (500 MHz, DMSO-d6) δ 11.09 (s, 1H), 8.66 *(d, J* = 8.2 Hz, 1H), 7.93 *(d, J* = 9.4 Hz, 1H), 7.86 (d, *J* = 8.7 Hz, 1H), 7.66 (d, *J =* 8.2 Hz, 1H), 7.54 - 7.42 (m, 1H), 7.39 (d, *J=* 2.5 Hz, 1H), 7.30 (d, *J=* 8.1 Hz, 1H), 7.13 (dd, *J =* 8.9, 2.5 Hz, 1H), 6.11 (s, 2H), 4.56 (dq, *J =* 22.4, 6.0, 5.1 Hz, 2H), 3.93 - 3.83 (m, 1H), 3.17 (s, 13H), 3.09 (s, 1H), 3.01 (d, *J* = 16.1 Hz, 1H), 2.90 (dd, *J* = 16.4, 6.1 Hz, 1H), 2.77 (td, *J* = 12.0, 5.8 Hz, 1H), 2.61 *(d, J=* 18.1 Hz, 1H), 2.25 - 2.05 (m, 3H), 1.96 - 1.83 (m, 2H), 1.65 (q, *J =* 12.4 Hz, 2H), 1.59 - 1.42 (m, 2H).

### Example 28: Synthesis of Compound 28

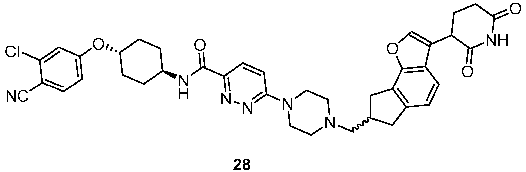

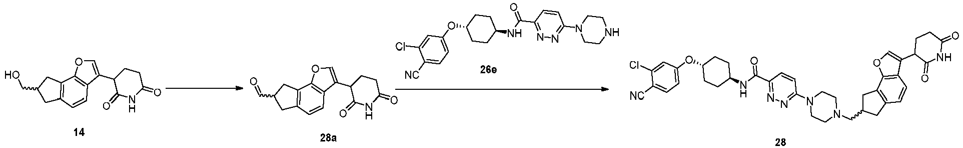

### Step 1: Preparation of intermediate 28a

Intermediate **14** (50 mg), IBX (94 mg), and dimethyl sulfoxide (5 mL) were added to a reaction flask in sequence, and the mixture was reacted at room temperature. After the reaction was completed, the reaction solution was extracted with a sodium bicarbonate solution (100 mL) and ethyl acetate (100 mL). The organic phase was washed with a saturated sodium chloride solution, dried over anhydrous sodium sulfate, filtered, and concentrated to give the target intermediate **28a** (50 mg).

MS(ESI, [M+H]⁺) m/z: 298.41.

### Step 2: Preparation of compound 28

Intermediate **28a** (50 mg), 1,2-dichloroethane (6 mL), isopropanol (2 mL), intermediate **26e** (90 mg), acetic acid (5.00 mg), and sodium cyanoborohydride (20 mg) were added to a reaction flask in sequence, and the mixture was stirred at room temperature for 1 h. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, filtered, and concentrated, and the residue was purified by a silica gel column to give compound **28** (72 mg). MS(ESI, [M+H]⁺) m/z: 722.43.

¹H NMR (500 MHz, DMSO-d6) δ 10.87 (d, *J* = 2.1 Hz, 1H), 8.61 (d, *J* = 8.2 Hz, 1H), 7.84 (dd, *J* = 18.0, 8.6 Hz, 3H), 7.44 - 7.28 (m, 3H), 7.20 - 7.04 (m, 2H), 4.53 (dq, *J =* 10.0, 5.4, 4.3 Hz, 1H), 4.11 (dd, *J =* 11.8, 4.9 Hz, 1H), 3.93 - 3.81 (m, 1H), 3.73 (t, *J =* 4.8 Hz, 4H), 3.17 (ddd, *J =* 29.9, 16.6, 8.4 Hz, 2H), 2.98 - 2.85 (m, 2H), 2.84 - 2.67 (m, 2H), 2.65 - 2.52 (m, 5H), 2.40 (d, *J* = 7.4 Hz, 2H), 2.31 (qd, *J* = 12.4, 4.4 Hz, 1H), 2.16 - 2.05 (m, 3H), 1.95 - 1.85 (m, 2H), 1.72 - 1.58 (m, 2H), 1.51 (q, *J=* 11.8, 11.3 Hz, 2H).

### Example 29: Synthesis of Compound 29

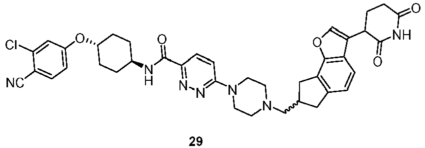

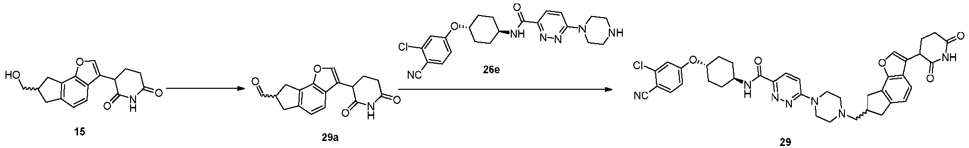

### Step 1: Preparation of intermediate 29a

Intermediate **15** (50 mg), IBX (94 mg), and dimethyl sulfoxide (5 mL) were added to a reaction flask in sequence, and the mixture was reacted at room temperature. After the reaction was completed, the reaction solution was extracted with a sodium bicarbonate solution (100 mL) and ethyl acetate (100 mL). The organic phase was washed with a saturated sodium chloride solution, dried over anhydrous sodium sulfate, filtered, and concentrated to give the target intermediate **29a** (50 mg).

MS(ESI, [M+H]⁺) m/z: 298.35.

### Step 2: Preparation of compound 29

Intermediate **29a** (50 mg), 1,2-dichloroethane (6 mL), isopropanol (2 mL), intermediate **26e** (90 mg), acetic acid (5.00 mg), and sodium cyanoborohydride (20 mg) were added to a reaction flask in sequence, and the mixture was stirred at room temperature for 1 h. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, filtered, and concentrated, and the residue was purified by a silica gel column to give compound 29 (93 mg). MS(ESI, [M+H]⁺) m/z: 722.47.

¹H NMR (500 MHz, DMSO-d6) δ 10.87 (d, *J* = 2.1 Hz, 1H), 8.61 (d, *J* = 8.2 Hz, 1H), 7.84 (dd, *J* = 18.0, 8.6 Hz, 3H), 7.44 - 7.28 (m, 3H), 7.20 - 7.04 (m, 2H), 4.53 (dq, *J =* 10.0, 5.4, 4.3 Hz, 1H), 4.11 (dd, *J =* 11.8, 4.9 Hz, 1H), 3.93 - 3.81 (m, 1H), 3.73 (t, *J =* 4.8 Hz, 4H), 3.17 (ddd, *J =* 29.9, 16.6, 8.4 Hz, 2H), 2.98 - 2.85 (m, 2H), 2.84 - 2.67 (m, 2H), 2.65 - 2.52 (m, 5H), 2.40 (d, *J* = 7.4 Hz, 2H), 2.31 (qd, *J* = 12.4, 4.4 Hz, 1H), 2.16 - 2.05 (m, 3H), 1.95 - 1.85 (m, 2H), 1.72 - 1.58 (m, 2H), 1.51 (q, *J=* 11.8, 11.3 Hz, 2H).

### Example 30: Synthesis of Compound 30

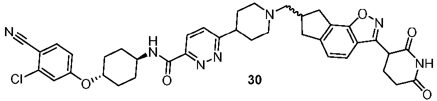

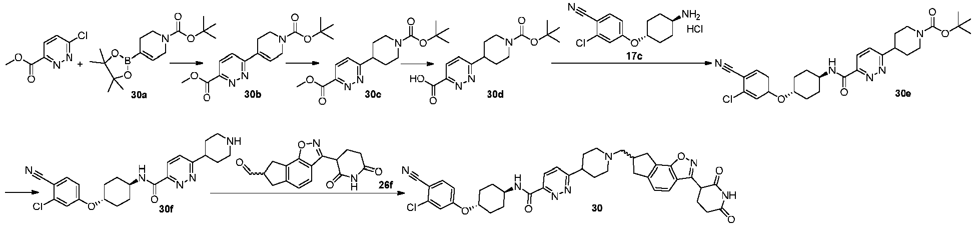

### Step 1: Preparation of intermediate 30b

Methyl 6-chloropyridazine-3-carboxylate (10 g), intermediate **30a** (26.9 g), [1,1'-bis(diphenylphosphino)ferrocene]palladium dichloride dichloromethane complex (4.73 g), sodium carbonate (18.43 g), dioxane (200 mL), and water (7 mL) were added to a reaction flask in sequence, and the mixture was heated to 85 °C and reacted under N₂ atmosphere. After the reaction was completed, ethyl acetate (200 mL) and water (200 mL) were added for extraction. The organic phase was separated, dried over anhydrous sodium sulfate, and filtered, and the filtrate was purified by a silica gel column to give the target intermediate **30b** (8.92 g).

MS(ESI, [M+H]+) m/z: 320.0.

¹H NMR (500 MHz, DMSO-d6) δ 8.17 (d, J = 9.0 Hz, 1H), 8.08 (d, J = 8.9 Hz, 1H), 6.98 (s, 1H), 4.18 - 4.10 (m, 2H), 3.96 (s, 3H), 3.59 (t, J = 5.7 Hz, 2H), 2.72 (tt, J = 6.0, 2.1 Hz, 2H), 1.44 (s, 9H).

### Step 2: Preparation of intermediate 30c

Intermediate **30b** (8.92 g), methanol (210 mL), dichloromethane (30 mL), and 10% palladium on carbon (2.23 g) were added to a reaction flask in sequence. The mixture was purged with H₂ and reacted at room temperature for 5 h. After the reaction was completed, the mixture was filtered, and the filtrate was concentrated by evaporation under reduced pressure to remove the solvent. The residue was added with dimethyl sulfoxide (20 mL) and filtered, and the filter cake was collected, slurried with water (50 mL), and filtered. The filter cake was collected to give the target intermediate **30c** (2.66 g).

MS(ESI, [M+H]⁺) m/z: 322.1.

¹H NMR (500 MHz, DMSO-d6) δ 8.15 (d, J = 8.7 Hz, 1H), 7.85 (d, J = 8.7 Hz, 1H), 4.16 - 4.05 (m, 2H), 3.95 (s, 3H), 3.21 (ddt, J = 15.2, 11.5, 5.8 Hz, 1H), 2.91 (d, J = 3.3 Hz, 2H), 1.94 - 1.88 (m, 2H), 1.67 (qd, J = 12.5, 4.3 Hz, 2H), 1.42 (s, 9H).

### Step 3: Preparation of intermediate 30d

Intermediate **30c** (1.7 g), sodium hydroxide (0.423 g), methanol (20 mL), and water (1 mL) were added to a reaction flask in sequence, and the mixture was reacted at room temperature. After the reaction was completed, a saturated citric acid solution was added to adjust the pH to 2-3, and dichloromethane was added. The organic phase was washed with saturated brine, dried over anhydrous sodium sulfate, filtered, and concentrated by evaporation under reduced pressure to remove the solvent, thus giving the target intermediate **30d** (1.63 g).

MS(ESI, [M-H]⁻) m/z: 306.2.

¹H NMR (500 MHz, DMSO-d6) δ 13.75 (s, 1H), 8.12 (d, J = 8.6 Hz, 1H), 7.83 (d, J = 8.7 Hz, 1H), 4.10 (d, J = 12.9 Hz, 2H), 3.21 (tt, J = 12.0, 3.6 Hz, 1H), 2.90 (s, 2H), 1.93 - 1.88 (m, 2H), 1.68 (qd, J = 12.5, 4.3 Hz, 2H), 1.42 (s, 9H).

### Step 4: Preparation of intermediate 30e

Intermediate **30d** (1.637 g), dichloromethane (30 mL), HATU (2.430 g), N,N-diisopropylethylamine (2.75 g, 3.72 mL), and intermediate **17c** (1.335 g) were added to a reaction flask in sequence, and the mixture was reacted at room temperature. After the reaction was completed, ethyl acetate (200 mL) and a saturated citric acid solution (300 mL) were added. The organic phase was separated, washed with a saturated sodium bicarbonate solution and saturated brine separately, dried over anhydrous sodium sulfate, filtered, and concentrated by evaporation under reduced pressure to remove the solvent, thus giving the target intermediate **30e** (2.52 g).

MS(ESI, [M+H]⁺) m/z: 540.3.

### Step 5: Preparation of intermediate 30f

Intermediate **30e** (2.52 g), dichloromethane (30 mL), and trifluoroacetic acid (37.0 g, 25 mL) were added to a reaction flask in sequence, and the mixture was reacted at room temperature. After the reaction was completed, the reaction solution was concentrated by evaporation under reduced pressure to remove the solvent, and the residue was added with dichloromethane/methanol (v:v = 9/1) (200 mL) and adjusted to pH of strong alkalinity with a 20% sodium hydroxide solution. The organic phase was separated, and the aqueous phase was extracted with dichloromethane/methanol (v:v = 9/1) (100 mL). The organic phases were combined, washed with saturated brine, dried over anhydrous sodium sulfate, filtered, and concentrated by evaporation under reduced pressure to remove the solvent, thus giving the target intermediate **30f** (1.88 g).

MS(ESI, [M+H]⁺) m/z: 440.3.

¹H NMR (500 MHz, DMSO-d6) δ 9.04 (d, J = 8.1 Hz, 1H), 8.13 (d, J = 8.7 Hz, 1H), 7.83 (dd, J = 31.5, 8.8 Hz, 2H), 7.40 (s, 1H), 7.15 (d, J = 9.0 Hz, 1H), 4.54 (d, J = 11.6 Hz, 1H), 3.92 (q, J = 11.0, 10.4 Hz, 1H), 3.19 (d, J = 12.6 Hz, 3H), 2.81 (t, J = 12.3 Hz, 2H), 2.69 (s, 1H), 2.12 (d, J = 12.2 Hz, 2H), 1.88 (dq, J = 24.6, 12.6, 12.0 Hz, 6H), 1.69 (q, J = 12.6 Hz, 2H), 1.52 (q, J = 13.6, 12.7 Hz, 2H).

### Step 6: Preparation of compound 30

Intermediate **26f** (60 mg), 1,2-dichloroethane (5 mL), isopropanol (1 mL), intermediate **30f** (88 mg), and sodium cyanoborohydride (37.7 mg) were added to a reaction flask in sequence, and the mixture was stirred at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, and filtered, and the filtrate was purified by a silica gel column to give compound **30** (77 mg).

MS(ESI, [M+H]⁺) m/z: 722.5.

¹H NMR (500 MHz, DMSO-d6) δ 11.09 (s, 1H), 9.03 (d, J = 8.2 Hz, 1H), 8.11 (d, J = 8.6 Hz, 1H), 7.85 (dd, J = 8.7, 5.0 Hz, 2H), 7.62 (d, J = 8.1 Hz, 1H), 7.40 (d, J = 2.5 Hz, 1H), 7.28 (d, J = 8.1 Hz, 1H), 7.14 (dd, J = 8.8, 2.5 Hz, 1H), 4.60 - 4.52 (m, 2H), 3.97 - 3.87 (m, 1H), 3.28 - 3.22 (m, 1H), 3.22 - 3.16 (m, 1H), 3.06 (d, J = 10.5 Hz, 2H), 3.00 (t, J = 5.1 Hz, 1H), 2.93 (d, J = 13.1 Hz, 2H), 2.85 (dd, J = 16.1, 4.8 Hz, 1H), 2.77 (td, J = 11.8, 5.8 Hz, 1H), 2.64 - 2.58 (m, 1H), 2.46 (s, 1H), 2.39 (d, J = 7.3 Hz, 2H), 2.20 (dq, J = 9.2, 4.3 Hz, 1H), 2.12 (d, J = 11.8 Hz, 4H), 1.94 - 1.84 (m, 6H), 1.70 (q, J = 12.4 Hz, 2H), 1.53 (q, J = 11.3 Hz, 2H).

### Example 31: Synthesis of Compound 31

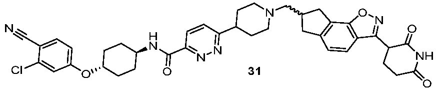

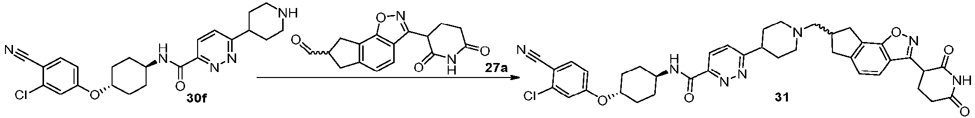

Intermediate **27a** (60 mg), 1,2-dichloroethane (5 mL), isopropanol (1 mL), intermediate **30f** (88 mg), and sodium cyanoborohydride (37.7 mg) were added to a reaction flask in sequence, and the mixture was stirred at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, and filtered, and the filtrate was purified by a silica gel column to give compound **31** (48 mg).

MS(ESI, [M+H]⁺) m/z: 722.5.

¹H NMR (500 MHz, DMSO-d6) δ 11.09 (s, 1H), 9.03 (d, J = 8.2 Hz, 1H), 8.11 (d, J = 8.6 Hz, 1H), 7.86 (dd, J = 8.8, 5.0 Hz, 2H), 7.62 (d, J = 8.1 Hz, 1H), 7.40 (d, J = 2.4 Hz, 1H), 7.28 (d, J = 8.1 Hz, 1H), 7.14 (dd, J = 8.6, 2.5 Hz, 1H), 4.55 (tt, J = 11.0, 5.5 Hz, 2H), 3.92 (dtd, J = 11.7, 7.7, 4.1 Hz, 1H), 3.28 - 3.22 (m, 1H), 3.18 (dd, J = 16.8, 8.1 Hz, 1H), 3.06 (d, J = 10.7 Hz, 2H), 3.00 (p, J = 6.2 Hz, 1H), 2.93 (d, J = 13.0 Hz, 2H), 2.85 (dd, J = 16.2, 4.8 Hz, 1H), 2.77 (ddd, J = 17.2, 11.8, 5.3 Hz, 1H), 2.61 (dt, J = 17.8, 4.5 Hz, 1H), 2.47 (s, 1H), 2.39 (d, J = 7.3 Hz, 2H), 2.20 (dq, J = 8.9, 4.3 Hz, 1H), 2.12 (d, J = 12.0 Hz, 4H), 1.92 (d, J = 12.2 Hz, 5H), 1.85 (d, J = 11.8 Hz, 1H), 1.70 (q, J = 11.8, 11.4 Hz, 2H), 1.53 (td, J = 13.5, 6.9 Hz, 2H).

### Example 32: Synthesis of Compound 32

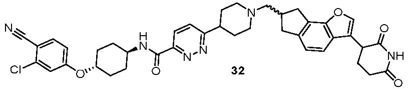

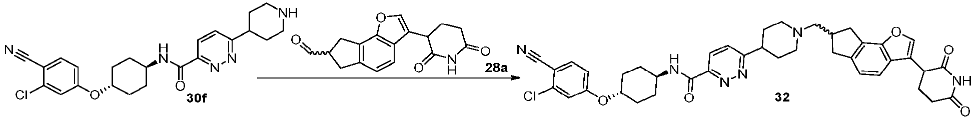

Intermediate **28a** (50 mg), 1,2-dichloroethane (5 mL), isopropanol (1 mL), intermediate **30f** (73.5 mg), and sodium cyanoborohydride (31.5 mg) were added to a reaction flask in sequence, and the mixture was stirred at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, and filtered, and the filtrate was purified by a silica gel column to give compound **32** (44 mg).

MS(ESI, [M+H]⁺) m/z: 721.6.

¹H NMR (500 MHz, DMSO-d6) δ 10.89 (s, 1H), 9.04 (d, J = 8.2 Hz, 1H), 8.11 (d, J = 8.6 Hz, 1H), 7.86 (dd, J = 8.8, 5.5 Hz, 2H), 7.81 (s, 1H), 7.40 (s, 1H), 7.34 (d, J = 7.8 Hz, 1H), 7.17 - 7.10 (m, 2H), 4.58 - 4.51 (m, 1H), 4.11 (dd, J = 12.0, 4.9 Hz, 1H), 3.92 (d, J = 9.3 Hz, 1H), 3.20 - 3.10 (m, 2H), 3.02 (d, J = 31.3 Hz, 3H), 2.88 (d, J = 14.3 Hz, 2H), 2.81 - 2.70 (m, 2H), 2.57 (d, J = 18.2 Hz, 1H), 2.42 - 2.34 (m, 2H), 2.30 (dt, J = 12.6, 6.3 Hz, 1H), 2.13 (d, J = 13.3 Hz, 5H), 1.92 (d, J = 12.5 Hz, 6H), 1.70 (q, J = 12.4 Hz, 2H), 1.53 (q, J = 12.1 Hz, 2H).

### Example 33: Synthesis of Compound 33

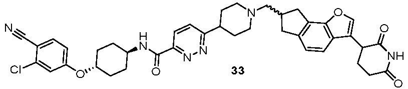

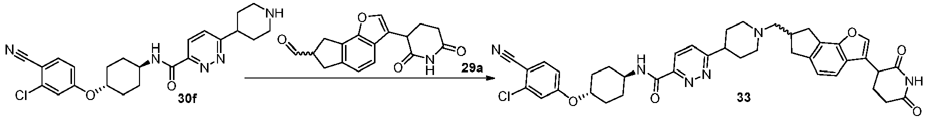

Intermediate **29a** (50 mg), 1,2-dichloroethane (5 mL), isopropanol (1 mL), intermediate **30f** (73.5 mg), and sodium cyanoborohydride (31.5 mg) were added to a reaction flask in sequence, and the mixture was stirred at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, and filtered, and the filtrate was purified by a silica gel column to give compound **33** (37 mg).

MS(ESI, [M+H]⁺) m/z: 721.6.

¹H NMR (500 MHz, DMSO-d6) δ 10.88 (s, 1H), 9.03 (d, J = 8.2 Hz, 1H), 8.11 (d, J = 8.7 Hz, 1H), 7.86 (dd, J = 8.8, 5.2 Hz, 2H), 7.81 (s, 1H), 7.40 (d, J = 2.4 Hz, 1H), 7.34 (d, J = 7.9 Hz, 1H), 7.17 - 7.10 (m, 2H), 4.55 (dq, J = 10.9, 6.3, 5.4 Hz, 1H), 4.11 (dd, J = 11.8, 4.9 Hz, 1H), 3.95 - 3.88 (m, 1H), 3.15 (dq, J = 22.0, 7.4, 6.4 Hz, 2H), 3.02 (d, J = 30.2 Hz, 3H), 2.88 (d, J = 13.2 Hz, 2H), 2.81 - 2.77 (m, 1H), 2.75 - 2.68 (m, 1H), 2.57 (d, J = 18.0 Hz, 1H), 2.38 (d, J = 7.3 Hz, 2H), 2.30 (td, J = 12.3, 4.2 Hz, 1H), 2.12 (d, J = 12.9 Hz, 5H), 1.92 (d, J = 12.3 Hz, 6H), 1.70 (q, J = 12.4 Hz, 2H), 1.53 (q, J = 12.1 Hz, 2H).

### Example 34: Synthesis of Compound 34

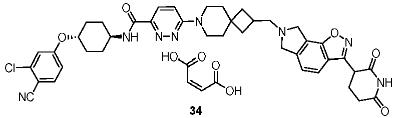

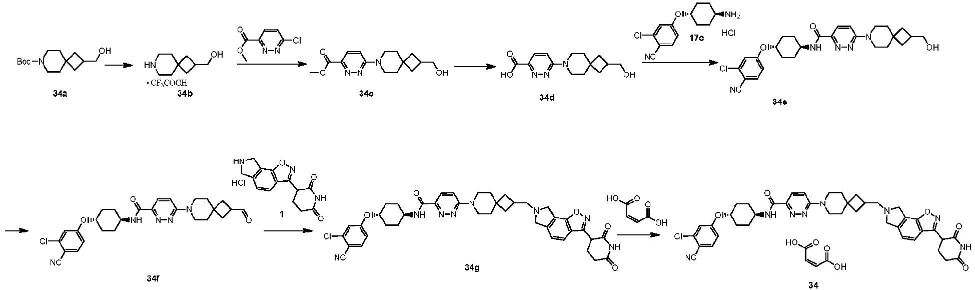

### Step 1: Preparation of intermediate 34b

**34a** (3 g), DCM (30 mL), and trifluoroacetic acid (5 mL) were added to a reaction flask in sequence and reacted at room temperature. After the reaction was completed, the reaction solution was concentrated to give **34b** (2.67 g). Step 2: Preparation of intermediate **34c**

**34b** (2.67 g), DMSO (30 mL), DIPEA (15 mL), and methyl 6-chloropyridazine-3-carboxylate (2 g) were added to a reaction flask in sequence, and the mixture was reacted at 120 °C. After the reaction was completed, the reaction solution was diluted with ethyl acetate and extracted with a saturated potassium carbonate solution. The organic phase was dried over anhydrous sodium sulfate, filtered, and concentrated to give **34c** (3.1 g).

MS(ESI, [M+H]⁺) m/z: 292.18.

### Step 3: Preparation of intermediate 34d

**34c** (3 g), MeOH (100 mL), and water (30 mL) were added to a reaction flask in sequence, sodium hydroxide (2.88 g) was then added, and the mixture was reacted at room temperature. After the reaction was completed, the system was adjusted to pH 4-5 with 6 M hydrochloric acid, concentrated to remove the solvent, and slurried with MeOH/DCM. The solid was removed by filtration under vacuum, and the mother liquor was concentrated to give **34d** (3.1 g).

MS(ESI, [M+H]⁺) m/z: 278.14.

### Step 4: Preparation of intermediate 34e

**17c** (1 g), **34d** (1.48 g), DCM (10 mL), HATU (1.58 g), and DIPEA( 2.43 mL) were added to a reaction flask in sequence, and the mixture was reacted at room temperature. After the reaction was completed, the reaction solution was diluted with ethyl acetate (100 mL), extracted with a 10% citric acid aqueous solution (100 mL), and then extracted with a saturated sodium bicarbonate solution (100 mL). The organic phase was dried over anhydrous sodium sulfate, filtered, and concentrated to give **34e** (1.1 g).

MS(ESI, [M+H]⁺) m/z: 510.21.

¹H NMR (500 MHz, DMSO-d6) δ 8.56 (d, *J=* 8.2 Hz, 1H), 7.85 (d, *J=* 8.7 Hz, 1H), 7.79 (d, *J* = 9.5 Hz, 1H), 7.38 (d, *J=* 2.4 Hz, 1H), 7.33 (d, *J=* 9.6 Hz, 1H), 7.13 (dd, *J=* 8.8, 2.4 Hz, 1H), 4.57 - 4.50 (m, 1H), 4.45 (t, *J=* 5.3 Hz, 1H), 3.85 (ddd, *J* = 11.3, 6.6, 2.9 Hz, 1H), 3.72 - 3.66 (m, 2H), 3.60 (dd, *J* = 6.8, 4.4 Hz, 2H), 3.38 (t, *J* = 5.8 Hz, 2H), 2.36 (p, *J* = 7.4 Hz, 1H), 2.14 - 2.07 (m, 2H), 1.92 - 1.82 (m, 4H), 1.67 - 1.60 (m, 4H), 1.57 - 1.48 (m, 6H). Step 5: Preparation of intermediate **34f**

**34e** (600 mg), DCM (10 mL), and Dess-Martin periodinane (926 mg) were added to a reaction flask in sequence, and the mixture was reacted at room temperature. After the reaction was completed, the reaction solution was quenched with a saturated sodium thiosulfate solution and a sodium bicarbonate solution and extracted with dichloromethane. The organic phase was separated, collected, dried over anhydrous sodium sulfate, filtered, and concentrated, and the residue was purified by silica gel column chromatography to give 34f (490 mg).

¹H NMR (500 MHz, DMSO-d6) δ 9.71 (d, *J* = 1.6 Hz, 1H), 8.57 (d, *J* = 8.2 Hz, 1H), 7.85 (d, *J* = 8.7 Hz, 1H), 7.80 (d, *J=* 9.6 Hz, 1H), 7.38 (d, *J=* 2.4 Hz, 1H), 7.35 (d, *J* = 9.7 Hz, 1H), 7.13 (dd, *J* = 8.8, 2.4 Hz, 1H), 4.57 - 4.50 (m, 1H), 3.86 (dtd, *J =* 11.3, 7.5, 4.0 Hz, 1H), 3.73 - 3.67 (m, 2H), 3.65 - 3.59 (m, 2H), 3.23 (dtd, *J* = 9.3, 7.6, 6.1 Hz, 1H), 2.14 - 2.07 (m, 2H), 2.04 - 1.97 (m, 4H), 1.92 - 1.87 (m, 2H), 1.71 - 1.59 (m, 4H), 1.51 (ddt, *J* = 12.8, 10.7, 4.0 Hz, 4H).

### Step 6: Preparation of intermediate 34g

Intermediate **34f** (111 mg), 1,2-dichloroethane (6 mL), isopropanol (2 mL), intermediate **1** (70 mg), sodium acetate (17 mg), and sodium cyanoborohydride (27 mg) were added to a reaction flask in sequence, and the mixture was stirred at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, filtered, and concentrated, and the residue was purified by a silica gel column to give compound **34g** (40 mg).

MS(ESI, [M+H]⁺) m/z: 763.18.

### Step 7: Preparation of compound 34

**34g** (40 mg), maleic acid (6 mg), and MeOH/DCM (1/10, 5 mL) were added to a reaction flask in sequence, and after complete dissolution, the reaction solution was concentrated. The reaction solution was slurried with petroleum ether and filtered under vacuum to give compound **34** (43 mg).

MS(ESI, [M+H]⁺) m/z: 763.58.

¹H NMR (500 MHz, DMSO-*d₆*) δ 11.12 (s, 1H), 8.56 (d, *J* = 8.2 Hz, 1H), 7.87 (dd, *J=* 13.6, 8.4 Hz, 2H), 7.81 (d, *J=9.5* Hz, 1H), 7.43 (d, *J* = 8.2 Hz, 1H), 7.41 - 7.31 (m, 2H), 7.13 (dd, *J* = 8.8, 2.5 Hz, 1H), 6.07 (s, 2H), 5.02 - 4.32 (m, 6H), 3.86 (tdt, *J=* 11.6, 8.1, 3.8 Hz, 1H), 3.73 (t, *J=* 5.3 Hz, 2H), 3.69 - 3.62 (m, 2H), 3.32 (s, 2H), 2.78 (dtd, *J =* 18.5, 13.4, 12.8, 6.7 Hz, 2H), 2.63 (dt, *J =* 17.2, 4.1 Hz, 1H), 2.58 - 2.53 (m, 1H), 2.21 (dq, *J =* 13.5, 4.6 Hz, 1H), 2.18 - 2.02 (m, 4H), 1.96 - 1.85 (m, 2H), 1.61 (dddd, *J =* 71.5, 36.0, 12.6, 7.2 Hz, 10H).

### Example 35: Synthesis of Compound 35

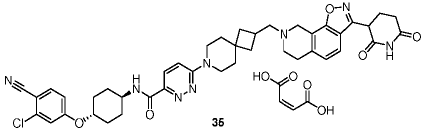

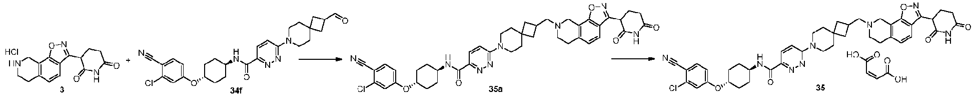

### Step 1: Preparation of intermediate 35a

Intermediate **3** (70 mg), intermediate **34f** (111 mg), sodium acetate (17.85 mg), 1,2-dichloroethane (5 mL), isopropanol (1 mL), and sodium cyanoborohydride (27.3 mg) were added to a reaction flask in sequence, and the mixture was stirred at room temperature for 1 h. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, and filtered, and the filtrate was purified by a silica gel column to give intermediate **35a** (92 mg).

MS(ESI, [M+H]⁺) m/z: 777.5.

### Step 2: Preparation of compound 35

Intermediate **35a** (92 mg), dichloromethane (10 mL), methanol (5 mL), and maleic acid (15.11 mg) were added to a reaction flask in sequence, and the mixture was reacted at room temperature. After the reaction was completed, the mixture was concentrated by evaporation under reduced pressure to remove the solvent. The residue was slurried with petroleum ether and filtered, and the filter cake was collected to give compound **35** (63 mg).

MS(ESI, [M+H]⁺) m/z: 777.5.

¹H NMR (500 MHz, DMSO-d₆) δ 11.10 (s, 1H), 9.97 (s, 1H), 8.56 (d, J = 8.2 Hz, 1H), 7.86 (d, J = 8.8 Hz, 1H), 7.81 (d, J = 9.4 Hz, 1H), 7.77 (d, J = 8.2 Hz, 1H), 7.40 - 7.34 (m, 2H), 7.28 (d, J = 8.2 Hz, 1H), 7.13 (dd, J = 8.8, 2.4 Hz, 1H), 6.08 (s, 2H), 4.61 (dd, J = 12.2, 5.0 Hz, 2H), 4.54 (dq, J = 10.5, 5.4 Hz, 2H), 3.88 - 3.83 (m, 1H), 3.73 (t, J = 5.4 Hz, 2H), 3.64 (t, J = 5.3 Hz, 2H), 3.23 (s, 6H), 2.88 (q, J = 8.0 Hz, 1H), 2.82 - 2.75 (m, 1H), 2.62 (dt, J = 17.5, 4.1 Hz, 1H), 2.54 (d, J = 16.2 Hz, 1H), 2.21 - 2.09 (m, 5H), 1.94 - 1.87 (m, 2H), 1.73 - 1.51 (m, 10H).

### Example 36: Synthesis of Compound 36

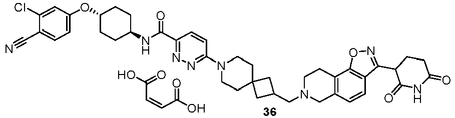

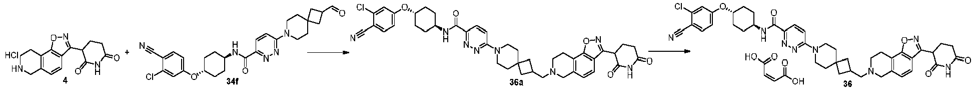

### Step 1: Preparation of intermediate 36a

Intermediate **4** (70 mg), intermediate **34f** (111 mg), sodium acetate (17.85 mg), 1,2-dichloroethane (5 mL), isopropanol (1 mL), and sodium cyanoborohydride (27.3 mg) were added to a reaction flask in sequence, and the mixture was stirred at room temperature for 1 h. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, and filtered, and the filtrate was purified by a silica gel column to give the target intermediate **36a** (97 mg).

MS(ESI, [M+H]⁺) m/z: 777.5.

### Step 2: Preparation of compound 36

Intermediate **36a** (97 mg), dichloromethane (10 mL), methanol (5 mL), and maleic acid (15.93 mg) were added to a reaction flask in sequence, and the mixture was reacted at room temperature. After the reaction was completed, the mixture was concentrated by evaporation under reduced pressure to remove the solvent. The residue was slurried with petroleum ether and filtered, and the filter cake was collected to give compound **36** (100 mg).

MS(ESI, [M+H]⁺) m/z: 777.5.

¹H NMR (500 MHz, DMSO-d6) δ 11.10 (s, 1H), 9.94 (s, 1H), 8.55 (d, J = 8.2 Hz, 1H), 7.86 (d, J = 8.7 Hz, 1H), 7.81 (d, J = 9.6 Hz, 1H), 7.76 (d, J = 8.2 Hz, 1H), 7.42 - 7.33 (m, 2H), 7.24 (d, J = 8.3 Hz, 1H), 7.13 (dd, J = 8.8, 2.4 Hz, 1H), 6.09 (s, 2H), 4.68 - 4.36 (m, 4H), 3.86 (dtd, J = 11.5, 7.6, 4.0 Hz, 1H), 3.76 - 3.70 (m, 2H), 3.65 - 3.60 (m, 2H), 3.18 (d, J = 5.7 Hz, 6H), 2.80 (ddt, J = 17.3, 12.1, 6.8 Hz, 2H), 2.62 (dt, J = 17.5, 4.1 Hz, 1H), 2.55 (d, J = 13.0 Hz, 1H), 2.23 - 2.07 (m, 5H), 1.94 - 1.86 (m, 2H), 1.60 (dddd, J = 58.7, 33.4, 13.0, 7.2 Hz, 10H).

### Example 37: Synthesis of Compound 37

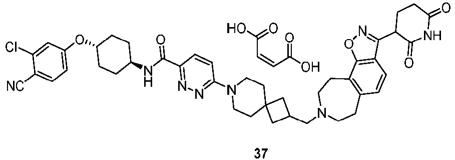

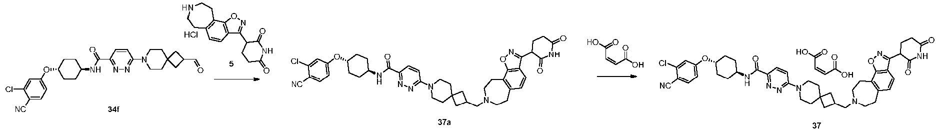

### Step 1: Preparation of intermediate 37a

Intermediate **34f** (106 mg), 1,2-dichloroethane (6 mL), isopropanol (2 mL), intermediate **5** (70 mg), sodium acetate (17 mg), and sodium cyanoborohydride (26 mg) were added to a reaction flask in sequence, and the mixture was stirred at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, filtered, and concentrated, and the residue was purified by a silica gel column to give compound **37a** (70 mg).

MS(ESI, [M+H]⁺) m/z: 791.23.

### Step 7: Preparation of compound 37

**37a** (70 mg), maleic acid (10 mg), and MeOH/DCM (1/10, 5 mL) were added to a reaction flask in sequence, and after complete dissolution, the reaction solution was concentrated. The reaction solution was slurried with petroleum ether and filtered under vacuum to give compound **37** (73 mg).

MS(ESI, [M+H]⁺) m/z: 791.35.

¹H NMR (500 MHz, DMSO-*d*₆) δ 11.09 (s, 1H), 9.67 (s, 1H), 8.55 (d, *J =* 8.2 Hz, 1H), 7.83 (dd, *J =* 25.1, 9.1 Hz, 2H), 7.67 (d, *J =* 8.0 Hz, 1H), 7.45 - 7.33 (m, 2H), 7.29 (d, *J* = 8.1 Hz, 1H), 7.13 (dd, *J* = 8.7, 2.5 Hz, 1H), 6.10 (s, 2H), 4.68 - 4.46 (m, 2H), 3.89 - 3.80 (m, 1H), 3.66 (dt, *J* = 47.6, 5.3 Hz, 7H), 3.52 - 3.31 (m, 7H), 2.79 (ddd, *J* = 17.0, 11.6, 5.2 Hz, 2H), 2.67 - 2.58 (m, 1H), 2.56 (m, 1H), 2.22 - 2.04 (m, 5H), 1.95 - 1.83 (m, 2H), 1.73 - 1.44 (m, 10H).

### Example 38: Synthesis of Compound 38

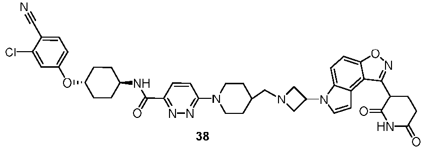

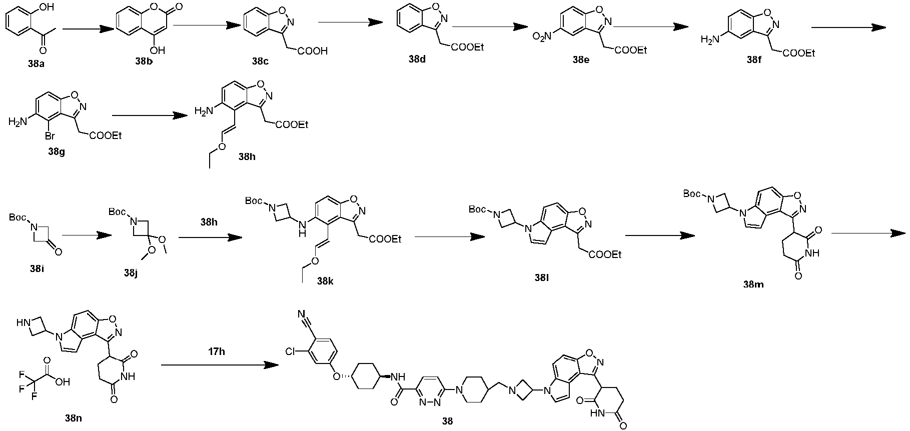

### Step 1: Preparation of intermediate 38b

**38a** (50 g), diethyl carbonate (216.9 g), and toluene (500 mL) were added to a reaction flask in sequence. The reaction solution was cooled to 0 °C, and 60 wt% sodium hydride (44.06 g) was added in portions. The mixture was first warmed to 70 °C and reacted for about 10 min, and then heated to 120 °C and reacted for 5 h. The reaction solution was cooled to room temperature and slowly poured into stirred ice water. The mixture was extracted with ethyl acetate, and the organic phase was discarded. The aqueous phase was adjusted to pH = 3 with 3 N hydrochloric acid and extracted with ethyl acetate, and the organic phases were combined, dried over anhydrous sodium sulfate, filtered, and concentrated to give intermediate **38b** (55 g).

¹H NMR (500 MHz, DMSO-*d*₆) δ 12.52 (s, 1H), 7.83 (dd, *J* = 7.8, 1.7 Hz, 1H), 7.65 (ddd, *J* = 8.6, 7.2, 1.7 Hz, 1H), 7.43 - 7.32 (m, 2H), 5.61 (s, 1H).

### Step 2: Preparation of intermediate 38c

Intermediate **38b** (55 g), methanol (500 mL), hydroxylamine hydrochloride (63.5 g), and sodium ethoxide (80.8 g) were added to a reaction flask in sequence, and the mixture was heated to 80 °C and reacted overnight. The reaction solution was cooled to room temperature, added with 3 N hydrochloric acid to adjust the pH to 5, concentrated, and added with water (2 L). The reaction flask was placed under an ice-water bath to cool, and meanwhile the pH was adjusted to 3 with 3 N hydrochloric acid. The mixture was stirred for 30 min and filtered. The filter cake was collected and dried to give intermediate **38c** (54.5 g).

¹H NMR (500 MHz, DMSO-*d₆*) δ 12.90 (s, 1H), 7.86 (dt, *J =* 7.9, 1.0 Hz, 1H), 7.74 (d, *J* = 8.4 Hz, 1H), 7.66 (ddd, *J =* 8.3, 7.0, 1.2 Hz, 1H), 7.40 (td, *J =* 7.4, 7.0, 0.9 Hz, 1H), 4.11 (s, 2H).

### Step 3: Preparation of intermediate 38d

Intermediate **38c** (54 g), ethanol (400 mL), and sulfuric acid (106 g) were added to a reaction flask in sequence, and the mixture was heated to 90 °C and reacted for 2 h. The reaction solution was cooled to room temperature and concentrated by evaporation under reduced pressure to remove the solvent, and ethyl acetate (1 L) and water (1 L) were added to the residue for dilution. A saturated sodium bicarbonate solution was added to adjust the pH to 7, and the organic phase was separated. The aqueous phase was extracted with ethyl acetate, and the organic phases were combined, dried over anhydrous sodium sulfate, filtered, and concentrated to give intermediate **38d** (62 g). MS(ESI, [M+H]⁺) *m*/*z:* 206.1.

### Step 4: Preparation of intermediate 38e

Intermediate **38d** (30 g) and sulfuric acid (200 mL) were added to a reaction flask in sequence, and a mixed solution of nitric acid (11.05 g) in sulfuric acid (4 mL) was slowly added under an ice bath. After the dropwise addition, the mixture was slowly warmed to room temperature and reacted for 1 h. The reaction solution was slowly poured into ice water (2 L) and extracted with ethyl acetate. The organic phase was collected, and the aqueous phase was extracted with ethyl acetate. The organic phases were combined, dried over anhydrous sodium sulfate, filtered, and concentrated, and the concentrate was separated and purified by silica gel column chromatography to give intermediate **38e** (29.3 g).

MS(ESI, [M+H]⁺) *m*/*z:* 251.1.

### Step 5: Preparation of intermediate 38f

Intermediate **38e** (29 g), ethanol (300 mL), and stannous chloride dihydrate (130.76 g) were added to a reaction flask in sequence, and the mixture was stirred at room temperature for 4 h. The reaction solution was concentrated, and dichloromethane (1 L) and water (0.5 L) were added to the residue. A saturated sodium bicarbonate solution was slowly added under an ice bath to adjust the pH to 9-10. The mixture was filtered, and the organic phase of the filtrate was separated, dried over anhydrous sodium sulfate, filtered, and concentrated. The concentrate was separated and purified by silica gel column chromatography to give intermediate **38f** (24.3 g).

MS(ESI, [M-H]⁻) *m*/*z:* 219.1.

### Step 6: Preparation of intermediate 38g

Intermediate **38f** (24 g) and DCM (200 mL) were added to a reaction flask in sequence, NBS (21.34 g) was added at 0 °C, and the mixture was reacted at room temperature for 1 h. DCM (500 mL) and water (500 mL) were added to the reaction solution. The organic phase was separated, washed with a saturated sodium chloride solution, dried over anhydrous sodium sulfate, filtered, and concentrated, and the concentrate was separated and purified by silica gel column chromatography to give intermediate **38g** (22.4 g).

MS(ESI, [M+H]⁺) *m*/*z:* 299.2.

¹H NMR (500 MHz, DMSO-*d₆*) δ 7.52 (d, *J* = 8.9 Hz, 1H), 7.17 (d, *J* = 8.9 Hz, 1H), 4.87 (s, 2H), 4.19 - 4.11 (m, 4H), 1.20 (t, *J* = 7.1 Hz, 3H).

### Step 7: Preparation of intermediate 38h

Intermediate **38g** (30.0 g), ethoxyvinyl borate (24.83 g), cesium carbonate (94.25 g), palladium acetate (2.16 g), Ruphos (9.00 g), and 1,4-dioxane (500 mL) were added to a reaction flask, and the mixture was stirred at 100 °C. After the reaction was completed, the reaction solution was added with water (1000 mL) and extracted with ethyl acetate. The organic layers were combined, dried over anhydrous sodium sulfate, filtered under vacuum, and concentrated, and the residue was purified by silica gel column chromatography to give intermediate **38h** (14.50 g). MS(ESI, [M+H]⁺) m/z: 291.11.

### Step 8: Preparation of intermediate 38j

Intermediate **38i** (20 g), MeOH (500 mL), trimethyl orthoformate (61.99 g), and *p*-toluenesulfonic acid (2.01 g) were added to a reaction flask, and the mixture was stirred at room temperature overnight. After the reaction was completed as confirmed, the reaction solution was added with a saturated NaHCO₃ solution (100 mL) and subjected to rotary evaporation. The residue was added with water (200 mL) and extracted with DCM, and the organic layers were combined, dried over anhydrous sodium sulfate, filtered under vacuum, and concentrated to give intermediate **38j** (21.65 g), which was directly used in the next step.

### Step 9: Preparation of intermediate 38k

Intermediate **38h** (7.5 g), intermediate **38j** (16.84 g), acetonitrile (150 mL), iodine (656 mg), and triethylsilane (6.01 g) were added to a reaction flask, and the mixture was stirred at room temperature overnight. After the reaction was completed, the reaction solution was added with a sodium thiosulfate solution (100 mL) and subjected to rotary evaporation, and the residue was added with water (200 mL) and extracted with DCM. The organic layers were combined, dried over anhydrous sodium sulfate, filtered under vacuum, and concentrated, and the residue was purified by silica gel column chromatography to give intermediate **38k** (5.05 g).

MS(ESI, [M+H]+) m/z: 446.16.

### Step 10: Preparation of intermediate 381

Intermediate **38k** (5.00 g) and DCM (100 mL) were added to a reaction flask, a solution of hydrochloric acid in 1,4-dioxane (4 M, 4.21 mL) was added dropwise under stirring, and the mixture was stirred at room temperature for 1 h. After the reaction was completed, the reaction solution was added with water (200 mL), oscillated, left to stand, and separated. The aqueous layer was extracted with DCM. The organic layers were combined, dried over anhydrous sodium sulfate, filtered under vacuum, and concentrated, and the residue was purified by silica gel column chromatography to give intermediate **381** (1.35 g).

MS(ESI, [M+H]+) m/z: 400.15.

### Step 11: Preparation of intermediate 38m

Intermediate **381** (1.30 g), acrylamide (254 mg), and anhydrous THF (30 mL) were added to a reaction flask, and a solution of potassium tert-butoxide in THF (4.88 mL, 4.88 mmol) was slowly added dropwise at -5 °C. After the dropwise addition, the mixture was reacted at room temperature. After the reaction was completed, the reaction solution was added dropwise to a mixed solution of a saturated NH₄Cl solution (100 mL) and ethyl acetate (50 mL) under stirring, and the mixture was oscillated, left to stand, and separated. The aqueous layer was extracted with EA (50 mL). The organic layers were combined, dried over anhydrous sodium sulfate, filtered under vacuum, and concentrated, and the residue was purified by silica gel column chromatography to give intermediate **38m** (400 mg). MS(ESI, [M+H]+) m/z: 425.1.

### Step 12: Preparation of intermediate 38n

Intermediate **38m** (390 mg) and DCM (5 mL) were added to a reaction flask, trifluoroacetic acid (3.45 mL) was added dropwise under stirring, and the mixture was stirred at room temperature for 1 h. After the reaction was completed, the reaction solution was directly subjected to rotary evaporation to dryness to give intermediate **38n** (451 mg).

MS(ESI, [M+H]+) m/z: 325.13.

### Step 13: Preparation of compound 38

Intermediate **17h** (100 mg), intermediate **38n** (101 mg), 1.2-dichloroethane (20 mL), and isopropanol (4 mL) were added to a reaction flask, and the mixture was stirred at room temperature for 5 min. Sodium triacetoxyborohydride (138 mg) was then added, and the mixture was stirred at room temperature. After the reaction was completed, the reaction solution was added with a saturated NaHCO₃ solution (1 mL), and the mixture was purified by a silica gel column and a reversed-phase column in sequence to give compound **38** (28 mg).

Q-TOF (ESI, [M+H]⁺) *m*/*z:* 776.3078.

¹H NMR (500 MHz, DMSO-*d*₆) δ 11.17 (s, 1H), 8.57 (d, *J=* 8.2 Hz, 1H), 7.92 (d, *J* = 9.1 Hz, 1H), 7.89 - 7.83 (m, 2H), 7.80 (d, *J* = 9.5 Hz, 1H), 7.50 (d, *J* = 9.1 Hz, 1H), 7.41 - 7.31 (m, 2H), 7.13 (dd, *J* = 8.8, 2.5 Hz, 1H), 6.65 (d, *J=* 3.1 Hz, 1H), 5.29 (p, *J=* 6.7 Hz, 1H), 4.70 (dd, *J* = 11.8, 5.1 Hz, 1H), 4.59 - 4.43 (m, 3H), 3.85 (t, *J=* 7.4 Hz, 3H), 3.37 (t, *J=* 6.6 Hz, 2H), 3.01 (td, *J=* 13.1, 2.6 Hz, 2H), 2.85 (ddd, *J* = 17.3, 12.1, 5.4 Hz, 1H), 2.64 (dt, *J* = 17.1, 4.3 Hz, 1H), 2.44 (dd, *J* = 14.5, 5.4 Hz, 3H), 2.24 (dt, *J* = 13.6, 4.6 Hz, 1H), 2.11 (d, *J* = 11.8 Hz, 2H), 1.93 - 1.87 (m, 2H), 1.87 - 1.80 (m, 2H), 1.69 (d, *J=* 14.8 Hz, 1H), 1.67 - 1.58 (m, 2H), 1.51 (dt, *J* = 13.2, 9.8 Hz, 2H), 1.18 (qd, *J =* 12.6, 4.1 Hz, 2H).

### Example 39: Synthesis of Compound 39

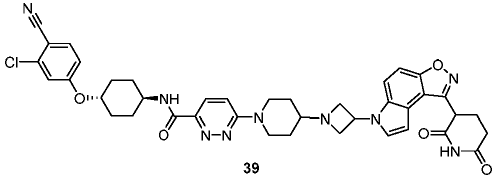

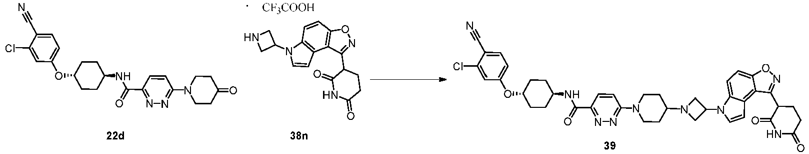

### Step 1: Preparation of compound 39

Intermediate **22d** (100 mg), intermediate **38n** (101 mg), 1.2-dichloroethane (20 mL), and isopropanol (4 mL) were added to a reaction flask, and the mixture was stirred at room temperature for 5 min. Sodium triacetoxyborohydride (138 mg) was then added, and the mixture was stirred at room temperature. After the reaction was completed, the reaction solution was added with a saturated NaHCO₃ solution (1 mL), and the mixture was purified by a silica gel column and a reversed-phase column in sequence to give compound **39** (28 mg).

Q-TOF (ESI, [M+H]⁺) *m*/*z:* 762.3078.

¹H NMR (500 MHz, DMSO-*d*₆) δ 11.18 (s, 1H), 8.60 (d, J = 8.2 Hz, 1H), 7.93 (d, J = 9.2 Hz, 1H), 7.91 - 7.79 (m, 3H), 7.51 (d, J = 9.1 Hz, 1H), 7.41 - 7.34 (m, 2H), 7.14 (dd, J = 8.8, 2.5 Hz, 1H), 6.66 (d, J = 3.1 Hz, 1H), 5.31 (p, J = 6.7 Hz, 1H), 4.71 (dd, J = 11.9, 5.1 Hz, 1H), 4.54 (p, J = 5.7 Hz, 1H), 4.19 (d, J = 13.3 Hz, 2H), 3.88 (t, J = 7.2 Hz, 3H), 3.45 - 3.36 (m, 5H), 2.85 (ddd, J = 17.3, 11.9, 5.3 Hz, 1H), 2.64 (dt, J = 17.0, 4.1 Hz, 1H), 2.43 (td, J = 12.3, 4.2 Hz, 1H), 2.28 - 2.20 (m, 1H), 2.14 - 2.07 (m, 2H), 1.94 - 1.87 (m, 2H), 1.80 (d, J = 12.8 Hz, 2H), 1.64 (q, J = 11.9 Hz, 2H), 1.57 - 1.46 (m, 2H), 1.36 - 1.27 (m, 2H).

### Example 40: Synthesis of Compound 40

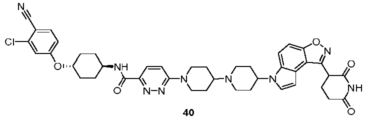

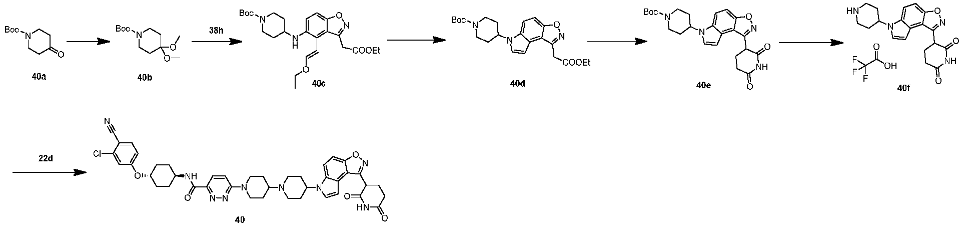

### Step 1: Preparation of intermediate 40b

Intermediate **40a** (10 g), MeOH (100 mL), trimethyl orthoformate (10.65 g), and p-toluenesulfonic acid (0.432 g) were added to a reaction flask, and the mixture was stirred at room temperature overnight. After the reaction was completed, the reaction solution was added with a saturated NaHCO₃ solution (100 mL) and subjected to rotary evaporation. The residue was added with water (200 mL) and extracted twice with DCM, and the organic layers were combined, dried over anhydrous sodium sulfate, filtered under vacuum, and concentrated to give intermediate **40b** (12.2 g).

¹H NMR (500 MHz, DMSO-*d*₆) δ 3.28 (t, *J =* 5.7 Hz, 4H), 3.09 (s, 6H), 1.59 (dd, *J =* 6.9, 4.8 Hz, 4H), 1.39 (s, 9H).

### Step 2: Preparation of intermediate 40c

Intermediate **38h** (12.0 g), intermediate **40b** (12.17 g), acetonitrile (120 mL), iodine (1.05 g), and triethylsilane (9.61 g) were added to a reaction flask, and the mixture was stirred at room temperature overnight. After the reaction was completed, the reaction solution was added with a sodium thiosulfate solution (100 mL) and subjected to rotary evaporation, and the residue was added with water (200 mL) and extracted with DCM. The organic layers were combined, dried over anhydrous sodium sulfate, filtered under vacuum, and concentrated, and the residue was purified by silica gel column chromatography to give intermediate **40c** (9.45 g).

MS(ESI, [M+H]⁺) *m*/*z:* 474.21.

### Step 3: Preparation of intermediate 40d

Intermediate **40c** (9.40 g) and DCM (100 mL) were added to a reaction flask, a solution of hydrochloric acid in 1,4-dioxane (4 M, 7.84 mL) was added dropwise under stirring, and the mixture was stirred at room temperature for 1 h. After the reaction was completed, the reaction solution was added with water (200 mL), oscillated, left to stand, and separated. The aqueous layer was extracted once with DCM. The organic layers were combined, dried over anhydrous sodium sulfate, filtered under vacuum, and concentrated, and the residue was purified by silica gel column chromatography to give intermediate **40d** (7.09 g).

MS(ESI, [M+H]⁺) *m*/*z:* 428.20.

### Step 4: Preparation of intermediate 40e

Intermediate **40d** (7.05 g), acrylamide (1.17 g), and anhydrous THF (50 mL) were added to a reaction flask, and a solution of potassium tert-butoxide in THF (20.49 mL) was slowly added dropwise at -5 °C. After the dropwise addition, the mixture was reacted at room temperature. After the reaction was completed, the reaction solution was added dropwise to a mixed solution of a saturated NH₄Cl solution (150 mL) and ethyl acetate (100 mL) under stirring, and the mixture was oscillated, left to stand, and separated. The aqueous layer was extracted with EA. The organic layers were combined, dried over anhydrous sodium sulfate, filtered under vacuum, and concentrated, and the residue was purified by silica gel column chromatography to give intermediate **40e** (7.55 g).

MS(ESI, [M+H]⁺) *m*/*z:* 453.11.

### Step 5: Preparation of intermediate 40f

Intermediate **40e** (7.50 g) and DCM (30 mL) were added to a reaction flask, trifluoroacetic acid (54.85 mL) was added dropwise under stirring, and the mixture was stirred at room temperature for 1 h. After the reaction was completed, the reaction solution was directly subjected to rotary evaporation to dryness to give intermediate **40f** (8.45 g).

MS(ESI, [M+H]⁺) *m*/*z:* 353.16*.*

### Step 6: Preparation of compound 40

Intermediate **22d** (130 mg), intermediate **40f** (100 mg), dichloroethane (15 mL), isopropanol (3 mL), and glacial acetic acid (17 mg) were added to a reaction flask, and the mixture was stirred at room temperature for 5 min. Sodium cyanoborohydride (36 mg) was then added, and the mixture was warmed to 80 °C and reacted for 12 h. After the reaction was completed, the reaction solution was added with water (100 mL) and extracted with dichloromethane (100 mL). The organic phases were combined, dried over anhydrous sodium sulfate, filtered under vacuum, and concentrated, and the residue was purified by silica gel column chromatography to give compound **40** (73 mg).

Q-TOF (ESI, [M+H]⁺) *m*/*z:* 790.3046.

¹H NMR (500 MHz, DMSO-*d₆*) δ 11.18 (s, 1H), 8.60 (d, *J* = 8.2 Hz, 1H), 7.93 (d, *J =* 9.2 Hz, 1H), 7.84 (dd, *J* = 16.9, 9.2 Hz, 2H), 7.75 *(d, J=* 3.2 Hz, 1H), 7.48 *(d, J=* 9.1 Hz, 1H), 7.41 - 7.35 (m, 2H), 7.14 (dd, *J* = 8.7, 2.4 Hz, 1H), 6.56 (d, *J =* 3.1 Hz, 1H), 4.68 (dd, *J =* 11.8, 5.2 Hz, 1H), 4.59 - 4.48 (m, 4H), 3.86 (dtd, *J =* 11.5, 7.8, 4.3 Hz, 1H), 3.04 (d, *J =* 11.4 Hz, 4H), 2.85 (ddd, *J =* 17.4, 12.1, 5.4 Hz, 1H), 2.73 (t, *J* = 11.6 Hz, 1H), 2.63 (dt, *J* = 17.4, 4.3 Hz, 1H), 2.42 (td, *J =* 12.4, 4.1 Hz, 3H), 2.21 (dq, *J =* 14.0, 5.0 Hz, 1H), 2.14 - 2.07 (m, 2H), 1.98 (s, 4H), 1.94 - 1.87 (m, 4H), 1.64 (q, *J* = 12.1 Hz, 2H), 1.51 (qd, *J* = 13.3, 12.6, 5.6 Hz, 4H).

### Example 41: Synthesis of Compound 41

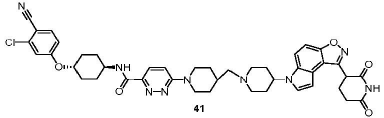

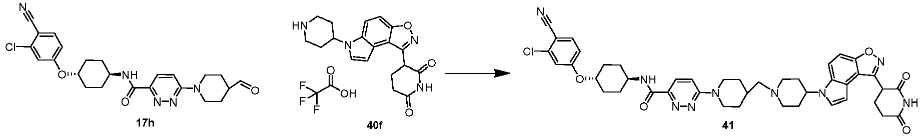

### Step 1: Preparation of compound 41

Intermediate **17h** (100 mg), intermediate **40f** (119 mg), 1.2-dichloroethane (20 mL), and isopropanol (4 mL) were added to a reaction flask, and the mixture was stirred at room temperature for 5 min. Sodium triacetoxyborohydride (162 mg) was then added, and the mixture was stirred at room temperature. After the reaction was completed, the reaction solution was added with a saturated NaHCO₃ solution (1 mL), and the mixture was directly subjected to rotary evaporation and purified by a silica gel column and a reversed-phase column in sequence to give compound **41** (31 mg).

Q-TOF (ESI, [M+H]⁺) *m*/*z:* 804.3393.

¹H NMR (500 MHz, DMSO-*d₆*) δ 11.17 (s, 1H), 8.57 (d, *J* = 8.2 Hz, 1H), 7.94 (d, *J =* 9.2 Hz, 1H), 7.83 (dd, *J* = 23.1, 9.2 Hz, 2H), 7.75 (d, *J =* 3.2 Hz, 1H), 7.49 *(d, J=* 9.1 Hz, 1H), 7.41 - 7.31 (m, 2H), 7.13 (dd, *J* = 8.8, 2.4 Hz, 1H), 6.57 (d, *J =* 3.0 Hz, 1H), 4.68 (dd, *J=* 11.9, 5.2 Hz, 1H), 4.59 - 4.46 (m, 4H), 3.86 (tdd, *J =* 11.3, 9.5, 7.5, 4.0 Hz, 1H), 3.03 (t, *J* = 12.7 Hz, 3H), 2.85 (ddd, *J* = 17.3, 12.0, 5.4 Hz, 1H), 2.63 (dt, *J* = 17.2, 4.2 Hz, 1H), 2.43 (td, *J=* 12.6, 4.4 Hz, 1H), 2.27 - 2.16 (m, 5H), 2.14 - 2.07 (m, 2H), 2.06 - 1.96 (m, 4H), 1.88 (dd, *J* = 20.7, 12.9 Hz, 5H), 1.70 - 1.58 (m, 2H), 1.57 - 1.46 (m, 3H), 1.20 - 1.09 (m, 2H).

### Example 42: Synthesis of Compound 42

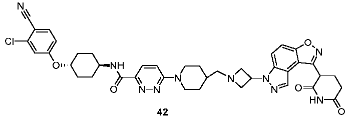

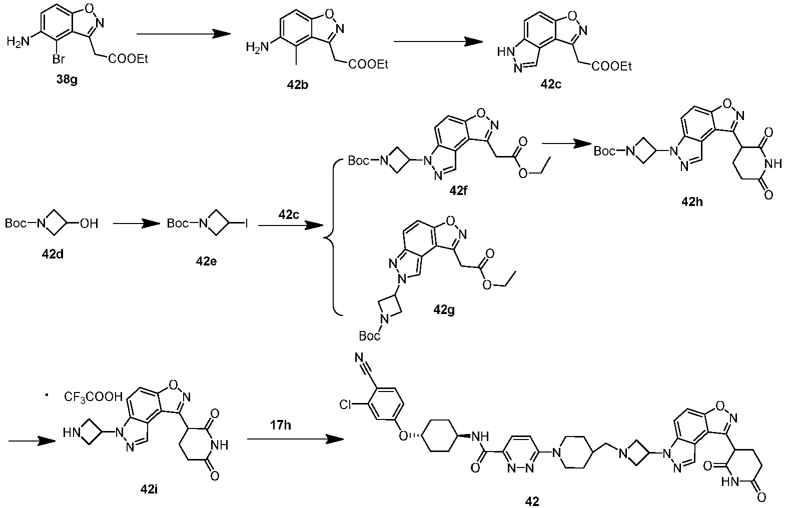

### Step 1: Preparation of intermediate 42b

Intermediate **38g** (1 g), methylboronic acid (0.6 g), Pd(dppf)₂Cl₂ (0.245 g), cesium fluoride (1.625 g), and 1,4-dioxane (20 mL) were added to a reaction flask in sequence, and the mixture was heated to 85 °C and reacted under N₂ atmosphere for 4 h. After the reaction was completed, the reaction solution was cooled to room temperature, and ethyl acetate (100 mL) and water (200 mL) were added to the system. The organic phase was separated, and the aqueous phase was then extracted with ethyl acetate. The organic phases were combined, dried over anhydrous sodium sulfate, filtered, and concentrated, and the concentrate was separated and purified by silica gel column chromatography to give intermediate **42b** (0.64 g).

MS(ESI, [M+H]⁺) *m*/*z:* 235.1.

¹H NMR (500 MHz, DMSO-*d*₆) δ 7.26 (d, *J =* 8.8 Hz, 1H), 7.01 (d, *J =* 8.8 Hz, 1H), 4.89 (s, 2H), 4.18 (s, 2H), 4.15 (q, *J=* 7.1 Hz, 2H), 2.18 (s, 3H), 1.20 (t, *J* = 7.1 Hz, 3H).

### Step 2: Preparation of intermediate 42c

Intermediate **42b** (0.42 g), potassium acetate (0.563 g), and toluene (20 mL) were added to a reaction flask in sequence, acetic anhydride (0.366 g) was added at 0 °C, and the mixture was reacted at room temperature for 2 h. The reaction solution was warmed to 75 °C, *tert*-butyl nitrite (0.555 g) was then added, and the mixture was reacted at 75 °C overnight. The reaction solution was cooled to room temperature, and DCM (100 mL) and water (100 mL) were added to the system. The organic phase was separated, dried over anhydrous sodium sulfate, filtered, and concentrated, and the concentrate was dissolved in EtOH (20 mL). Hydrochloric acid (0.327 g) was added, and the mixture was reacted at room temperature for 2 h. The reaction solution was concentrated, and DCM (100 mL) and a saturated NaHCO₃ solution (100 mL) were then added. The organic phase was separated, washed with a saturated sodium chloride solution, dried over anhydrous sodium sulfate, filtered, and concentrated to give intermediate **42c** (0.48 g).

MS(ESI, [M+H]⁺) *m*/*z:* 246.1.

¹H NMR (500 MHz, DMSO-*d*₆) δ 13.67 (s, 1H), 8.24 (s, 1H), 7.88 (dd, *J* = 9.2, 1.0 Hz, 1H), 7.76 (d, *J* = 9.2 Hz, 1H), 4.35 (s, 2H), 4.15 (q, *J =* 7.1 Hz, 2H), 1.17 (t, *J =* 7.1 Hz, 3H).

### Step 3: Preparation of intermediate 42e

Iodine (40 g), triphenylphosphine (30.3 g), imidazole (11.79 g), and toluene (100 mL) were added to a reaction flask, intermediate **42d** (10 g) was then added, and the mixture was warmed to 100 °C and reacted. The reaction was stopped, a saturated sodium bicarbonate solution (400 mL) and EA (400 mL) were added to the reaction solution, and the organic phase was separated and washed with saturated sodium sulfite. After washing, the organic phase was dried over anhydrous sodium sulfate, filtered, and concentrated, and the concentrate was separated and purified by silica gel column chromatography to give intermediate **42e** (14.2 g).

¹H NMR (500 MHz, Chloroform-d) δ 4.64 (ddd, *J* = 9.3, 7.8, 1.3 Hz, 2H), 4.47 (tt, *J* = 7.7, 5.1 Hz, 1H), 4.29 (ddd, *J=* 9.8, 5.1, 1.3 Hz, 2H), 1.44 (s, 9H).

### Step 4: Preparation of intermediates 42f and 42g

Intermediate **42c** (2.5 g), intermediate **42e** (3.46 g), cesium carbonate (6.64 g), and DMF (20 mL) were added to a reaction flask in sequence, and the mixture was warmed to 60 °C and reacted for 12 h. The reaction solution was cooled to room temperature, and EA (200 mL) and water (200 mL) were added to the system. The organic phase was separated, dried over anhydrous sodium sulfate, filtered, and concentrated, and the concentrate was separated and purified by silica gel column chromatography to give intermediate **42f** (1.28 g) and intermediate **42g** (1.37 g). Intermediate **42f:**

MS(ESI, [M+H]⁺) *m*/*z:* 401.1.

¹H NMR (500 MHz, DMSO-*d*₆) δ 8.38 (s, 1H), 8.03 (d, *J=* 9.3 Hz, 1H), 7.86 (d, *J=* 9.3 Hz, 1H), 5.85 (tt, *J=* 7.9, 5.3 Hz, 1H), 4.42 (t, *J=* 8.2 Hz, 2H), 4.37 (s, 2H), 4.29 (s, 2H), 4.16 (q, *J* = 7.2 Hz, 2H), 1.44 (s, 9H), 1.19 (t, *J* = 7.1 Hz, 3H).

### Intermediate 42g:

MS(ESI, [M+H]⁺) *m*/*z:* 401.2.

¹H NMR (500 MHz, DMSO-*d*₆) δ 8.72 (s, 1H), 7.96 (d, *J=* 9.4 Hz, 1H), 7.73 (d, *J* = 9.5 Hz, 1H), 5.61 (tt, *J* = 7.9, 5.0 Hz, 1H), 4.45 *(t, J=* 8.3 Hz, 2H), 4.34 - 4.25 (m, 4H), 4.15 *(q, J=* 7.1 Hz, 2H), 1.44 (s, 9H), 1.18 *(t, J=* 7.1 Hz, 3H).

### Step 5: Preparation of intermediate 42h

Intermediate **42f** (1.2 g), acrylamide (0.234 g), and tetrahydrofuran (40 mL) were added to a reaction flask in sequence. Potassium tert-butoxide (0.471 g) was added at -15 °C under N₂ atmosphere, and the mixture was reacted at room temperature for 3 h. After the reaction was completed, a saturated ammonium chloride aqueous solution was added dropwise to the reaction solution to quench the reaction, and the mixture was extracted with ethyl acetate. The organic phase was separated, and the aqueous phase was extracted with ethyl acetate. The organic phases were combined, dried over anhydrous sodium sulfate, filtered, and concentrated, and the concentrate was separated and purified by silica gel column chromatography to give intermediate **42h** (0.952 g).

MS(ESI, [M+H]⁺) *m*/*z:* 426.2.

¹H NMR (500 MHz, DMSO-*d*₆) δ 11.18 (s, 1H), 8.35 (s, 1H), 8.04 (d, *J* = 9.3 Hz, 1H), 7.88 (d, *J=* 9.3 Hz, 1H), 5.90 - 5.81 (m, 1H), 4.80 (dd, *J* = 12.3, 4.9 Hz, 1H), 4.42 (t, *J* = 8.4 Hz, 2H), 4.28 (s, 2H), 2.83 (ddd, *J* = 17.4, 12.2, 5.3 Hz, 1H), 2.67 (dt, *J* = 17.3, 4.1 Hz, 1H), 2.55 (d*, J* = 12.8 Hz, 1H), 2.32 - 2.23 (m, 1H), 1.43 (s, 9H).

### Step 6: Preparation of intermediate 42i

Intermediate **42h** (0.95 g), DCM (30 mL), and trifluoroacetic acid (5 mL) were added to a reaction flask in sequence, and the mixture was reacted at room temperature for 3 h. After the reaction was completed, the reaction solution was concentrated to dryness to give intermediate **42i** (0.71 g).

MS(ESI, [M+H]⁺) *m*/*z:* 326.2.

### Step 7: Preparation of compound 42

Intermediate **17h** (90 mg), intermediate **42i** (85 mg), dichloroethane (10 mL), isopropanol (2 mL), and glacial acetic acid (13 mg) were added to a reaction flask, and the mixture was stirred at room temperature for 5 min. Sodium cyanoborohydride (32 mg) was then added, and the mixture was reacted at room temperature for 4 h. After the reaction was completed, the reaction solution was added with water (100 mL) and extracted with dichloromethane (100 mL). The organic phases were combined, dried over anhydrous sodium sulfate, filtered under vacuum, and concentrated, and the residue was purified by silica gel column chromatography to give compound **42** (42 mg).

Q-TOF (ESI, [M+H]⁺) *m*/*z:* 777.3045.

¹H NMR (500 MHz, DMSO-*d₆*) δ 11.18 (s, 1H), 8.59 (d, *J* = 8.2 Hz, 1H), 8.27 (s, 1H), 8.15 (d, *J* = 9.3 Hz, 1H), 7.88 - 7.77 (m, 3H), 7.39 (s, 1H), 7.34 (d, *J =* 9.6 Hz, 1H), 7.14 (d, *J =* 8.9 Hz, 1H), 5.65 - 5.58 (m, 1H), 4.79 (dd, *J =* 12.3, 5.0 Hz, 1H), 4.51 (t, *J* = 17.0 Hz, 3H), 3.88 (t, *J =* 7.4 Hz, 3H), 3.54 (d, *J =* 7.0 Hz, 2H), 3.01 (t, *J =* 12.5 Hz, 2H), 2.84 (ddd, *J =* 17.3, 12.3, 5.4 Hz, 1H), 2.67 (d, *J* = 17.7 Hz, 1H), 2.47 (s, 2H), 2.33 - 2.25 (m, 1H), 2.16 - 2.08 (m, 2H), 1.93 - 1.86 (m, 2H), 1.83 (d, *J* = 12.7 Hz, 2H), 1.72 (s, 1H), 1.64 (q, *J=* 12.5, 12.0 Hz, 2H), 1.51 (q, *J=* 11.9 Hz, 2H), 1.19 (tt, *J* = 16.5, 9.6 Hz, 3H).

### Example 43: Synthesis of Compound 43

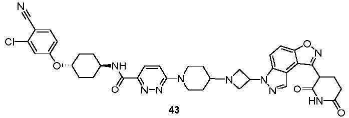

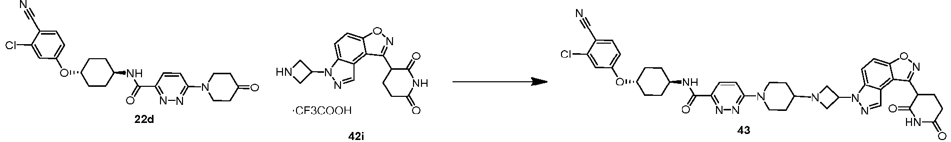

### Step 1: Preparation of compound 40

Intermediate **22d** (100 mg), intermediate **42i** (96 mg), dichloroethane (15 mL), isopropanol (3 mL), and glacial acetic acid (17 mg) were added to a reaction flask, and the mixture was stirred at room temperature for 5 min. Sodium cyanoborohydride (34 mg) was then added, and the mixture was reacted at room temperature for 4 h. After the reaction was completed, the reaction solution was added with water (100 mL) and extracted with dichloromethane (100 mL). The organic phases were combined, dried over anhydrous sodium sulfate, filtered under vacuum, and concentrated, and the residue was purified by silica gel column chromatography to give compound **43** (28 mg).

Q-TOF (ESI, [M+H]⁺) *m*/*z:* 763.2878.

¹H NMR (500 MHz, DMSO-*d*₆) δ 11.17 (s, 1H), 8.61 (d, *J* = 8.2 Hz, 1H), 8.28 (s, 1H), 8.15 (d, *J* = 9.3 Hz, 1H), 7.84 (dd, *J* = 18.2, 9.3 Hz, 3H), 7.43 - 7.34 (m, 2H), 7.14 (dd, *J* = 8.8, 2.4 Hz, 1H), 5.63 (p, *J* = 7.1 Hz, 1H), 4.80 (dd, *J* = 12.2, 5.0 Hz, 1H), 4.54 (tt, *J* = 10.1, 4.2 Hz, 1H), 4.20 (dt, *J* = 13.9, 4.5 Hz, 2H), 3.89 (dd, *J* = 13.0, 5.8 Hz, 3H), 3.59 (td, *J* = 7.0, 4.0 Hz, 2H), 3.36 (dd, *J* = 11.6, 7.9 Hz, 2H), 2.83 (ddd, *J* = 17.4, 12.3, 5.4 Hz, 1H), 2.67 (dt, *J* = 17.4, 4.2 Hz, 1H), 2.58 (tt, *J* = 8.7, 4.0 Hz, 1H), 2.28 (dq, *J* = 13.5, 4.4 Hz, 1H), 2.15 - 2.07 (m, 2H), 1.94 - 1.87 (m, 2H), 1.85 - 1.78 (m, 2H), 1.70 - 1.59 (m, 2H), 1.52 (td, *J* = 13.9, 13.4, 6.8 Hz, 2H), 1.31 (qd, *J* = 8.8, 4.3 Hz, 2H).

### Example 44: Synthesis of Compound 44

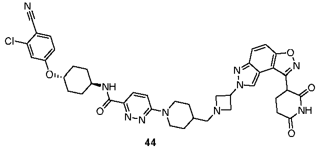

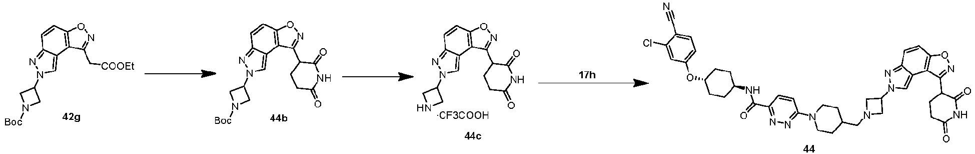

### Step 1: Preparation of intermediate 44b

Intermediate **42g** (1.0 g), acrylamide (0.234 g), and tetrahydrofuran (40 mL) were added to a reaction flask in sequence. Potassium tert-butoxide (0.471 g) was added at -15 °C under N₂ atmosphere, and the mixture was reacted at room temperature for 3 h. After the reaction was completed, a saturated ammonium chloride aqueous solution was added dropwise to the reaction solution to quench the reaction, and the mixture was extracted with ethyl acetate. The organic phase was separated, and the aqueous phase was extracted with ethyl acetate. The organic phases were combined, dried over anhydrous sodium sulfate, filtered, and concentrated, and the concentrate was separated and purified by silica gel column chromatography to give intermediate **44b** (0.63 g).

MS(ESI, [M+H]⁺) *m*/*z:* 426.2.

¹H NMR (500 MHz, DMSO-*d*₆) δ 11.12 (s, 1H), 8.67 (s, 1H), 7.97 (d, *J =* 9.4 Hz, 1H), 7.74 (d, *J* = 9.4 Hz, 1H), 5.57 (td, *J* = 7.8, 4.1 Hz, 1H), 4.68 (dd, *J=* 12.3, 4.9 Hz, 1H), 4.44 (d, *J* = 8.7 Hz, 2H), 4.28 (s, 2H), 2.81 (ddd, *J* = 17.4, 12.1, 5.4 Hz, 1H), 2.65 (dt, *J* = 17.3, 4.0 Hz, 1H), 2.47 (dd, *J* = 12.5, 4.4 Hz, 1H), 2.27 - 2.18 (m, 1H), 1.43 (s, 9H).

### Step 2: Preparation of intermediate 44c

**Intermediate 44b** (0.63 g), DCM (30 mL), and trifluoroacetic acid (5 mL) were added to a reaction flask in sequence, and the mixture was reacted at room temperature for 3 h. After the reaction was completed, the reaction solution was concentrated to dryness to give intermediate **44c** (0.6 g).

MS(ESI, [M+H]⁺) *m*/*z:* 326.2.

### Step 3: Preparation of compound 44

Intermediate **17h** (100 mg), intermediate **44c** (85 mg), dichloroethane (10 mL), isopropanol (2 mL), and glacial acetic acid (14 mg) were added to a reaction flask, and the mixture was stirred at room temperature for 5 min. Sodium cyanoborohydride (33 mg) was then added, and the mixture was reacted at room temperature for 4 h. After the reaction was completed, the reaction solution was added with water (100 mL) and extracted with dichloromethane. The organic phases were combined, dried over anhydrous sodium sulfate, filtered under vacuum, and concentrated, and the residue was purified by silica gel column chromatography to give compound **44** (55 mg). Q-TOF (ESI, [M+H]⁺) *m*/*z:* 777.3040.

¹H NMR (500 MHz, DMSO-*d*₆) δ 11.13 (s, 1H), 8.66 - 8.53 (m, 2H), 7.95 (d, *J* = 9.4 Hz, 1H), 7.86 *(d, J =* 8.6 Hz, 1H), 7.80 (d, *J =* 9.4 Hz, 1H), 7.72 (d, *J=* 9.4 Hz, 1H), 7.39 (d, *J =* 2.4 Hz, 1H), 7.34 (d, *J* = 9.6 Hz, 1H), 7.14 (dd, *J =* 8.9, 2.5 Hz, 1H), 5.35 (q, *J =* 6.9 Hz, 1H), 4.70 (dd, *J =* 12.2, 5.0 Hz, 1H), 4.55 (d, *J =* 10.1 Hz, 1H), 4.49 (d, *J* = 13.5 Hz, 2H), 3.90 (s, 1H), 3.86 (d, *J=* 8.2 Hz, 3H), 3.57 (d, *J=* 6.5 Hz, 2H), 3.00 (t, *J* = 12.6 Hz, 2H), 2.82 (td, *J =* 14.2, 11.8, 5.3 Hz, 1H), 2.71 - 2.62 (m, 1H), 2.47 *(d, J =* 5.9 Hz, 2H), 2.29 - 2.22 (m, 1H), 2.14 - 2.07 (m, 2H), 1.90 (d, *J =* 12.0 Hz, 2H), 1.83 (d, *J =* 12.7 Hz, 2H), 1.64 (q, *J* = 12.6, 11.9 Hz, 3H), 1.57 - 1.47 (m, 2H), 1.17 (d, *J* = 12.7 Hz, 2H).

### Example 45: Synthesis of Compound 45

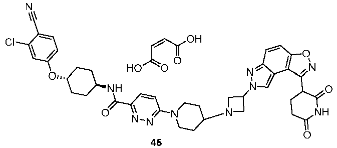

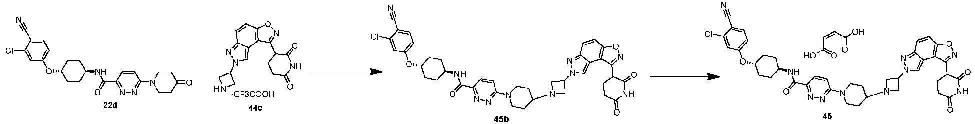

### Step 1: Preparation of compound 45

Intermediate **22d** (100 mg), intermediate **44c** (90 mg), dichloroethane (10 mL), isopropanol (2 mL), and glacial acetic acid (14 mg) were added to a reaction flask, and the mixture was stirred at room temperature for 5 min. Sodium cyanoborohydride (33 mg) was then added, and the mixture was reacted at room temperature for 4 h. After the reaction was completed, the reaction solution was added with water (100 mL) and extracted with dichloromethane (100 mL). The organic phases were combined, dried over anhydrous sodium sulfate, filtered under vacuum, and concentrated, and the residue was purified by silica gel column chromatography to give compound **45b** (31 mg). Compound **45b** (31 mg) was dissolved in MeOH (5 mL), maleic acid (4.7 mg) was then added, and the mixture was stirred at room temperature for 0.5 h and then concentrated to dryness. *n*-Hexane was added, and the mixture was stirred, filtered under vacuum, and dried to give compound **45** (27 mg).

MS (ESI, [M+H]⁺) *m*/*z:* 763.30.

¹H NMR (500 MHz, DMSO-*d*₆) δ 11.12 (s, 1H), 8.72 - 8.60 (m, 2H), 7.99 (d, *J* = 9.4 Hz, 1H), 7.87 (t, *J =* 8.8 Hz, 2H), 7.81 (d, *J* = 9.5 Hz, 1H), 7.46 (d*, J* = 9.6 Hz, 1H), 7.39 (d, *J* = 2.4 Hz, 1H), 7.14 (dd, *J* = 8.9, 2.4 Hz, 1H), 6.13 (s, 2H), 5.70 (s, 1H), 4.69 (dd, *J =* 12.3, 5.0 Hz, 2H), 4.65 - 4.51 (m, 5H), 3.92 - 3.84 (m, 3H), 3.16 - 3.00 (m, 5H), 2.85 (ddd, *J =* 17.5, 12.0, 5.4 Hz, 1H), 2.72 - 2.63 (m, 1H), 2.29 - 2.21 (m, 1H), 2.17 - 2.06 (m, 4H), 1.91 (d, *J* = 12.0 Hz, 2H), 1.65 *(q, J=* 12.2, 11.5 Hz, 2H), 1.58 - 1.48 (m, 2H), 1.43 (s, 2H).

### Example 46: Synthesis of Compound 46

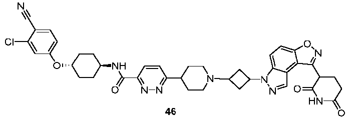

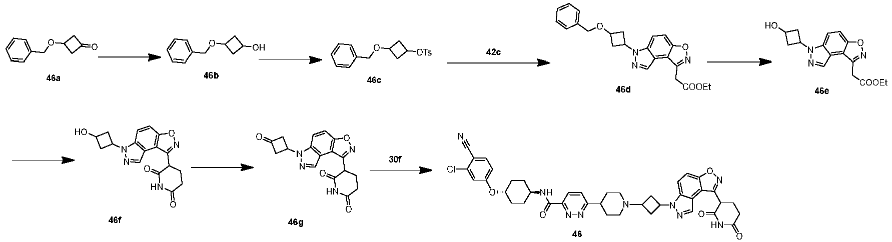

### Step 1: Preparation of intermediate 46b

Intermediate **46a** (3.7 g) and MeOH (30 mL) were added to a reaction flask in sequence, sodium borohydride (1.192 g) was added under an ice bath under N₂ atmosphere, and the mixture was reacted at room temperature for 3 h. After the reaction was completed, a saturated ammonium chloride aqueous solution was added dropwise to the reaction solution to quench the reaction, and ethyl acetate (200 mL) and water (200 mL) were then added for extraction. The organic phase was separated, dried over anhydrous sodium sulfate, filtered, and concentrated to give intermediate **46b** (3.81 g).

¹H NMR (500 MHz, Chloroform-d) δ 7.38 - 7.26 (m, 5H), 4.42 (s, 2H), 3.91 (p, *J=* 7.2 Hz, 1H), 3.69 - 3.58 (m, 2H), 2.79 - 2.65 (m, 2H), 1.93 (dtd, *J* = 9.5, 7.6, 2.9 Hz, 2H).

### Step 2: Preparation of intermediate 46c

Intermediate **46b** (0.2 g), DCM (5 mL), and triethylamine (0.341 g) were added to a reaction flask in sequence, p-toluenesulfonic acid (0.257 g) was then added, and the mixture was reacted at room temperature for 3 h. After the reaction was completed, DCM (50 mL) and water (50 mL) were added for extraction, the organic phase was separated, dried over anhydrous sodium sulfate, filtered, and concentrated, and the residue was purified by silica gel column chromatography to give intermediate **46c** (0.135 g).

¹H NMR (500 MHz, DMSO-*d*₆) δ 7.77 (d, *J* = 7.8 Hz, 2H), 7.47 (d, *J* = 7.9 Hz, 2H), 7.36 - 7.23 (m, 5H), 4.48 (p, *J=* 7.2 Hz, 1H), 4.31 (s, 2H), 3.63 (p, *J=* 6.8 Hz, 1H), 2.54 (dd, *J=* 7.2, 3.9 Hz, 2H), 2.42 (s, 3H), 1.93 (h, *J* = 6.4 Hz, 2H).

### Step 3: Preparation of intermediate 46d

Intermediate **42c** (1.3 g), intermediate **46c** (2.11 g), potassium carbonate (1.46 g), and DMF (20 mL) were added to a reaction flask in sequence, and the mixture was warmed to 80 °C and reacted for 12 h. The reaction solution was cooled to room temperature, and EA (200 mL) and water (200 mL) were added to the system. The organic phase was separated, dried over anhydrous sodium sulfate, filtered, and concentrated, and the concentrate was separated and purified by silica gel column chromatography to give intermediate **46d** (0.28 g).

MS(ESI, [M+H]⁺) *m*/*z:* 406.2.

¹H NMR (500 MHz, DMSO-*d*₆) δ 8.28 (s, 1H), 8.04 (dd, *J* = 9.3, 0.9 Hz, 1H), 7.80 (d, *J =* 9.3 Hz, 1H), 7.41 - 7.29 (m, 5H), 5.59 (tt, *J =* 8.5, 5.5 Hz, 1H), 4.48 (s, 2H), 4.36 (s, 2H), 4.16 *(q, J =* 7.0 Hz, 2H), 2.84 - 2.73 (m, 2H), 2.67 (ddd, *J* = 13.0, 8.3, 4.0 Hz, 3H), 1.18 (t, *J =* 7.1 Hz, 3H).

### Step 4: Preparation of intermediate 46e

**Intermediate 46d** (0.28 g), a 10% palladium on carbon catalyst (0.14 g), MeOH (10 mL), and hydrochloric acid (0.074 mL, 4 mol/L) were added to a reaction flask in sequence, and the mixture was reacted at room temperature under H₂ atmosphere for 4 h. The reaction solution was filtered under vacuum, and the filtrate was concentrated and then separated and purified by silica gel column chromatography to give intermediate **46e** (0.19 g).

MS(ESI, [M+H]⁺) *m*/*z:* 316.2*.*

¹H NMR (500 MHz, DMSO-*d*₆) δ 8.29 - 8.24 (m, 1H), 8.00 (dd, *J=* 9.4, 5.5 Hz, 1H), 7.79 (dd, *J* = 9.3, 4.4 Hz, 1H), 5.52 (tt, *J =* 8.8, 5.2 Hz, 1H), 4.63 - 4.54 (m, 2H), 4.16 (q, *J=* 7.1 Hz, 2H), 3.69 (s, 1H), 2.82 - 2.70 (m, 3H), 1.18 *(t, J=* 7.1 Hz, 2H), 1.06 *(t, J=* 7.0 Hz, 3H).

### Step 5: Preparation of intermediate 46f

Intermediate **46e** (0.16 g), acrylamide (0.04 g), and tetrahydrofuran (10 mL) were added to a reaction flask in sequence. Potassium tert-butoxide (0.08 g) was added at -15 °C under N₂ atmosphere, and the mixture was reacted at room temperature for 3 h. After the reaction was completed, a saturated ammonium chloride aqueous solution was added dropwise to the reaction solution to quench the reaction, and the mixture was extracted with ethyl acetate. The organic phase was separated, dried over anhydrous sodium sulfate, filtered, and concentrated, and the concentrate was separated and purified by silica gel column chromatography to give intermediate **46f** (0.12 g).

MS(ESI, [M+H]⁺) *m*/*z:* 341.2.

### Step 6: Preparation of intermediate 46g

Intermediate **46f** (0.1 g), acetonitrile (10 mL), and an IBX oxidant (0.11 g) were added to a reaction flask in sequence, and the mixture was reacted at 80 °C for 1 h. The reaction solution was filtered, and the filtrate was concentrated to give intermediate **46g** (0.09 g).

MS(ESI, [M+H]⁺) *m*/*z:* 339.2.

### Step 7: Preparation of compound 46

Intermediate **30f** (60 mg), intermediate **46g** (85 mg), dichloroethane (10 mL), isopropanol (2 mL), and glacial acetic acid (10 mg) were added to a reaction flask, and the mixture was stirred at room temperature for 5 min. Sodium cyanoborohydride (25 mg) was then added, and the mixture was reacted at room temperature for 4 h. After the reaction was completed, the reaction solution was added with water (100 mL) and extracted with dichloromethane. The organic phases were combined, dried over anhydrous sodium sulfate, filtered under vacuum, and concentrated, and the residue was purified by silica gel column chromatography to give compound **46** (15 mg).

Q-TOF (ESI, [M+H]⁺) *m*/*z:* 762.2929.

¹H NMR (500 MHz, DMSO-*d*₆) δ 11.19 (s, 1H), 9.05 (d, *J* = 8.2 Hz, 1H), 8.26 (s, 1H), 8.10 (dd, *J* = 15.1, 9.0 Hz, 2H), 7.85 (dt, *J =* 7.0, 3.5 Hz, 3H), 7.40 (d, *J =* 2.4 Hz, 1H), 7.14 (dd, *J =* 8.8, 2.4 Hz, 1H), 5.21 (s, 1H), 4.79 (dd, *J* = 12.2, 5.0 Hz, 1H), 4.59 - 4.50 (m, 1H), 3.97 - 3.86 (m, 1H), 3.02 (s, 3H), 2.84 (ddd, *J=* 17.4, 12.2, 5.4 Hz, 1H), 2.78 - 2.62 (m, 5H), 2.32 - 2.25 (m, 3H), 2.16 - 2.09 (m, 2H), 1.99 (d, *J* = 12.3 Hz, 2H), 1.91 (dt, *J* = 24.5, 12.0 Hz, 6H), 1.75 - 1.65 (m, 2H), 1.58 - 1.48 (m, 2H).

### Example 47: Synthesis of Compound 47

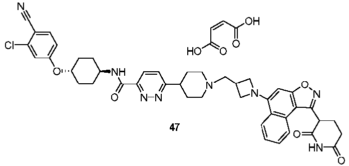

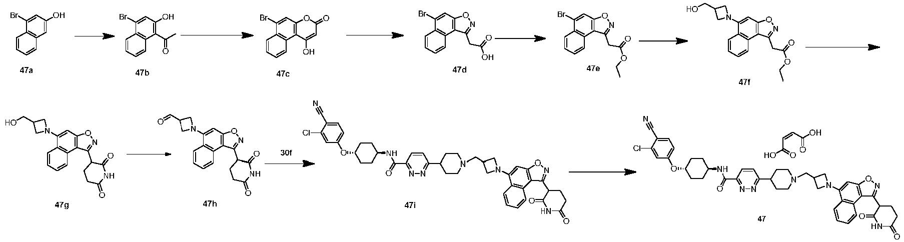

### Step 1: Preparation of intermediate 47b

Intermediate **47a** (20 g), 1,2-dichloroethane (200 mL), acetyl chloride (7.04 g), and aluminum trichloride (17.93 g) were added to a reaction flask in sequence, and the mixture was heated to 85 °C and reacted 2 h. After the reaction was completed, the reaction solution was cooled to room temperature, slowly added to 1 M diluted hydrochloric acid with ice, and extracted with dichloromethane. The organic phase was separated, and the aqueous phase was then extracted with dichloromethane. The organic phases were combined, dried over anhydrous sodium sulfate, and filtered, and the filtrate was concentrated by evaporation under reduced pressure to remove the solvent. The residue was separated by silica gel column chromatography to give intermediate **47b** (21.1 g).

MS(ESI, [M-H]⁻) *m*/*z:* 262.9.

¹H NMR (500 MHz, DMSO-*d₆*) *δ* 10.81 (s, 1H), 8.10 - 8.04 (m, 1H), 7.73 (dt, J = 8.5, 1.0 Hz, 1H), 7.62 (s, 1H), 7.56 (ddd, J = 8.4, 6.7, 1.4 Hz, 1H), 7.49 (ddd, J = 8.1, 6.8, 1.2 Hz, 1H), 2.61 (s, 3H).

### Step 2: Preparation of intermediate 47c

Intermediate **47b** (21 g), diethyl carbonate (46.4 g), and toluene (200 mL) were added to a reaction flask in sequence. The reaction solution was cooled to 0 °C, and 60 wt% sodium hydride (9.43 g) was added in portions. The mixture was heated to 120 °C and reacted for 4 h. After the reaction was completed, the reaction solution was cooled to room temperature and slowly poured into stirred ice water. The mixture was extracted with ethyl acetate, and the organic phase was discarded. The aqueous phase was adjusted to pH = 3 with 3 N hydrochloric acid and extracted with ethyl acetate, and the organic phases were combined, dried over anhydrous sodium sulfate, filtered, and concentrated by evaporation under reduced pressure to remove the solvent, thus giving intermediate **47c** (20.2 g). MS(ESI, [M-H]⁻) *m*/*z:* 288.9.

¹H NMR (500 MHz, DMSO-*d₆*) *δ* 8.07 (dd, J = 8.4, 1.2 Hz, 1H), 7.85 - 7.81 (m, 1H), 7.62 - 7.56 (m, 2H), 7.50 (ddd, J = 8.2, 6.9, 1.3 Hz, 1H), 4.08 (s, 2H).

### Step 3: Preparation of intermediate 47d

Intermediate **47c** (20 g), an hydroxylamine aqueous solution (21.05 mL), and ethanol (200 mL) were added to a reaction flask in sequence, and the mixture was reacted at 85 °C for 16 h. The reaction solution was cooled to room temperature and concentrated by evaporation under reduced pressure to remove the solvent, and ethyl acetate (200 mL) and a saturated sodium carbonate aqueous solution (200 mL) were added to the residue for extraction. The organic phase was separated, and the aqueous phase was collected, adjusted to pH 2-3 with a 3 M HCl aqueous solution, and extracted with ethyl acetate. The organic phase was collected, dried over anhydrous sodium sulfate, filtered, and concentrated by evaporation under reduced pressure to remove the solvent, thus giving intermediate **47d** (18.5 g).

MS(ESI, [M-H]⁻) *m*/*z:* 303.9.

¹H NMR (500 MHz, DMSO-*d*₆) *δ* 13.09 (s, 1H), 8.51 (s, 1H), 8.37 (dd, J = 8.4, 1.2 Hz, 1H), 8.28 - 8.21 (m, 1H), 7.87 (ddd, J = 8.3, 7.0, 1.3 Hz, 1H), 7.79 (ddd, J = 8.3, 7.0, 1.3 Hz, 1H), 4.43 (s, 2H).

### Step 4: Preparation of intermediate 47e

Intermediate **47d** (18 g), ethanol (150 mL), and sulfuric acid (3.13 mL) were added to a reaction flask in sequence, and the mixture was heated to 85 °C and reacted for 2 h. After the reaction was completed, the reaction solution was cooled to room temperature and concentrated by evaporation under reduced pressure to remove the solvent. Ethyl acetate (300 mL) and water (300 mL) were added to the residue for dilution, and a saturated sodium bicarbonate aqueous solution was added to adjust the pH = 7. The organic phase was separated, and the aqueous phase was extracted with ethyl acetate. The organic phases were combined, dried over anhydrous sodium sulfate, filtered, and concentrated by evaporation under reduced pressure to remove the solvent, thus giving intermediate **47e** (17.42 g). MS(ESI, [M+H]⁺) *m*/*z:* 334.12.

¹H NMR (500 MHz, DMSO-*d₆*) *δ* 8.52 (s, 1H), 8.38 (dd, J = 8.4, 1.2 Hz, 1H), 8.19 (dd, J = 8.1, 1.0 Hz, 1H), 7.86 (ddd, J = 8.2, 7.0, 1.3 Hz, 1H), 7.79 (ddd, J = 8.2, 7.0, 1.2 Hz, 1H), 4.54 (s, 2H), 4.15 (q, J = 7.1 Hz, 2H), 1.16 (t, J = 7.1 Hz, 3H).

### Step 5: Preparation of intermediate 47f

Intermediate **47e** (3.0 g), azetidin-3-ylmethanol (1.173 g), potassium phosphate (5.72 g), palladium acetate (0.302 g), xantphos (1.558 g), and 1,4-dioxane (50 mL) were added to a reaction flask, and the mixture was heated at 100 °C under nitrogen atmosphere. After the reaction was completed, the reaction solution was added with water (10 mL) and extracted with ethyl acetate. The organic layers were combined, washed with saturated brine, dried over anhydrous sodium sulfate, filtered, and subjected to silica gel column chromatography to give intermediate **47f** (3.0 g).

MS(ESI, [M+H]⁺) *m*/*z:* 341.30.

¹H NMR (500 MHz, DMSO-*d*₆) δ 8.13 (dd, *J* = 8.6, 1.2 Hz, 1H), 8.01 (dd, *J =* 8.3, 1.2 Hz, 1H), 7.66 (ddd, *J* = 8.2, 5.7, 1.2 Hz, 1H), 7.50 (ddd, *J* = 8.4, 6.9, 1.3 Hz, 1H), 7.35 - 7.25 (m, 1H), 6.70 (s, 1H), 4.83 (t, *J* = 5.3 Hz, 1H), 4.31 (t, *J =* 8.0 Hz, 4H), 4.14 (q, *J=* 7.1 Hz, 2H), 4.01 (dd, *J* = 7.8, 5.5 Hz, 2H), 3.68 - 3.60 (m, 1H), 2.85 (tt, *J* = 8.1, 5.7 Hz, 1H), 1.17 (t, *J =* 7.1 Hz, 3H).

### Step 6: Preparation of intermediate 47g

Intermediate **47f** (1.5 g) and acrylamide (0.275 g) were added to a reaction flask. The reaction apparatus was sealed, then anhydrous tetrahydrofuran (30 mL) was added, and a solution of potassium tert-butoxide in THF was slowly added dropwise (1 M, 3.22 mL). After the dropwise addition, the temperature was maintained at 0 °C and the mixture was reacted for 30 min, and then the mixture was reacted at room temperature for 30 min. The reaction solution was added dropwise to a mixed system of a saturated ammonium chloride solution-ethyl acetate under stirring. The organic layer was separated, dried over anhydrous sodium sulfate, filtered, mixed with silica gel, and purified by column chromatography to give intermediate **47g** (581 mg).

MS(ESI, [M+H]⁺) *m*/*z:* 366.30.

¹H NMR (500 MHz, DMSO-*d₆*) δ 11.09 (s, 1H), 8.16 - 8.11 (m, 1H), 7.65 (ddd, *J* = 8.2, 6.9, 1.2 Hz, 1H), 7.54 - 7.47 (m, 2H), 6.73 (s, 1H), 4.90 (dd, *J=* 10.9, 4.9 Hz, 1H), 4.82 (t, *J=* 5.4 Hz, 1H), 4.31 *(q, J=* 7.9 Hz, 2H), 4.01 (td, *J=* 7.9, 5.4 Hz, 2H), 3.65 (t, *J=* 5.8 Hz, 2H), 2.89 - 2.75 (m, 2H), 2.59 (dt, *J=* 17.3, 4.6 Hz, 1H), 2.46 (dd, *J* = 10.9, 4.3 Hz, 1H), 2.33 (dq, *J* = 13.8, 5.0 Hz, 1H).

### Step 7: Preparation of intermediate 47h

Intermediate **47g** (280 mg), Dess-Martin periodinane (584 mg), and dichloromethane (20 mL) were added to a reaction flask, and the mixture was stirred at room temperature. After the reaction was completed, the reaction solution was filtered under vacuum, and the filter cake was washed with dichloromethane. The filtrate and the washing solution were combined and subjected to rotary evaporation to dryness, thus giving intermediate **47h** (300 mg).

MS(ESI, [M+H]⁺) *m*/*z:* 364.2.

### Step 8: Preparation of compound 47

Intermediate **30f** (100 mg), intermediate **47h** (103 mg), dichloroethane (10 mL), isopropanol (2 mL), and glacial acetic acid (14 mg) were added to a reaction flask, and the mixture was stirred at room temperature for 5 min. Sodium cyanoborohydride (96 mg) was then added, and the mixture was reacted at room temperature for 4 h. After the reaction was completed, the reaction solution was added with water (100 mL) and extracted with dichloromethane (100 mL). The organic layers were combined, dried over anhydrous sodium sulfate, filtered under vacuum, and concentrated, and the residue was purified by silica gel column chromatography to give compound **47i** (65 mg). Compound **47i** (65 mg) was dissolved in MeOH (5 mL), maleic acid (9 mg) was then added, and the mixture was stirred at room temperature for 0.5 h and then concentrated to dryness. Methanol was added, and the mixture was stirred, filtered under vacuum, and dried to give compound **47** (55 mg).

Q-TOF (ESI, [M+H]⁺) *m*/*z:* 787.3132.

¹H NMR (500 MHz, DMSO-*d₆*) δ 11.11 (s, 1H), 9.04 (d, *J* = 8.2 Hz, 1H), 8.15 (dd, *J =* 24.5, 8.3 Hz, 3H), 7.86 (t, *J* = 8.1 Hz, 2H), 7.68 (t, *J =* 7.6 Hz, 1H), 7.54 (t, *J =* 7.7 Hz, 1H), 7.40 (d, *J* = 2.4 Hz, 1H), 7.14 (dd, *J =* 8.8, 2.4 Hz, 1H), 6.83 (s, 1H), 6.05 (s, 2H), 4.93 (dd, *J =* 11.1, 4.9 Hz, 1H), 4.52 (ddt, *J =* 22.8, 15.3, 6.8 Hz, 3H), 4.09 (q, *J* = 7.9, 7.5 Hz, 2H), 3.93 (tdt, *J* = 11.8, 8.1, 4.0 Hz, 1H), 3.68 - 3.58 (m, 2H), 3.54 (s, 3H), 3.16 (s, 3H), 2.82 (ddd, *J* = 17.1, 11.5, 5.5 Hz, 1H), 2.61 (dt, *J* = 17.3, 4.6 Hz, 1H), 2.56 - 2.51 (m, 1H), 2.35 (dt, *J =* 13.9, 4.7 Hz, 1H), 2.22 (d, *J=* 13.4 Hz, 3H), 2.17 - 2.08 (m, 3H), 1.96 - 1.88 (m, 2H), 1.76 - 1.64 (m, 2H), 1.59 - 1.49 (m, 2H).

### Example 48: Synthesis of Compound 48

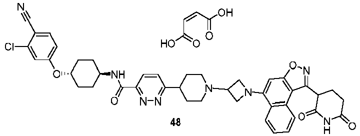

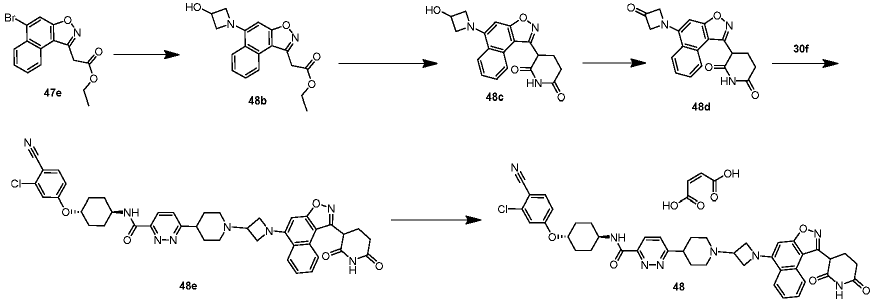

### Step 1: Preparation of intermediate 48b

Intermediate **47e** (4 g), azetidin-3-ol (1.050 g), palladium acetate (0.537 g), xantphos (2.77 g), potassium phosphate (7.62 g), and 1,4-dioxane (30 mL) were added to a reaction flask in sequence, and the mixture was heated to 100 °C and reacted under N₂ atmosphere for 3 h. After the reaction was completed, the reaction solution was cooled to room temperature, and ethyl acetate (100 mL) and water (200 mL) were added to the system. The organic phase was separated, and the aqueous phase was then extracted with ethyl acetate. The organic phases were combined, dried over anhydrous sodium sulfate, and filtered, and the filtrate was concentrated by evaporation under reduced pressure to remove the solvent. The crude product was separated by silica gel column chromatography to give intermediate **48b** (3.03 g).

MS(ESI, [M+H]⁺) *m*/*z:* 327.28.

### Step 2: Preparation of intermediate 48c

Intermediate **48b** (580 mg), acrylamide (126 mg), and tetrahydrofuran (10 mL) were added to a reaction flask in sequence. The mixture was purged three times with nitrogen, and potassium tert-butoxide (299 mg) was added at - 15 °C. The mixture was reacted at room temperature for 3 h. After the reaction was completed, a saturated ammonium chloride aqueous solution was added dropwise to the reaction solution to quench the reaction, and the mixture was extracted with ethyl acetate. The organic phase was separated, and the aqueous phase was extracted with ethyl acetate. The organic phases were combined, dried over anhydrous sodium sulfate, filtered, and concentrated by evaporation under reduced pressure to remove the solvent, and the residue was separated by silica gel column chromatography to give intermediate **48c** (0.41 g).

MS(ESI, [M-H]⁻) *m*/*z:* 350.12.

### Step 3: Preparation of intermediate 48d

Intermediate **48c** (410 mg), acetonitrile (10 mL), and an IBX oxidant (980 mg) were added to a reaction flask in sequence, and the mixture was heated to 85 °C and reacted under N₂ atmosphere for 2 h. After the reaction was completed, the reaction solution was cooled to room temperature and filtered, and the filtrate was concentrated by evaporation under reduced pressure to remove the solvent. The crude product was separated by silica gel column chromatography to give intermediate **48d** (0.39 g).

MS(ESI, [M-H]⁻) *m*/*z:* 348.21.

### Step 4: Preparation of compound 48

Intermediate **30f** (100 mg), intermediate **48d** (99 mg), dichloroethane (10 mL), isopropanol (2 mL), and glacial acetic acid (14 mg) were added to a reaction flask, and the mixture was stirred at room temperature for 5 min. Sodium cyanoborohydride (96 mg) was then added, and the mixture was reacted at room temperature for 4 h. After the reaction was completed, the reaction solution was added with water (100 mL) and extracted with dichloromethane (100 mL). The organic layers were combined, dried over anhydrous sodium sulfate, filtered under vacuum, and concentrated, and the residue was purified by silica gel column chromatography to give compound **48e** (22 mg). Compound **48e** (22 mg) was dissolved in MeOH (5 mL), maleic acid (3 mg) was then added, and the mixture was stirred at room temperature for 0.5 h and then concentrated to dryness. *n*-Hexane was added, and the mixture was stirred, filtered under vacuum, and dried to give compound **48** (20 mg).

Q-TOF (ESI, [M+H]⁺) *m*/*z:* 773.2977.

¹H NMR (500 MHz, DMSO-*d₆*) δ 11.11 (s, 1H), 9.04 (d, *J* = 8.2 Hz, 1H), 8.15 (dd, *J* = 15.0, 8.5 Hz, 3H), 7.86 (d, *J =* 8.8 Hz, 2H), 7.71 (t, *J =* 7.7 Hz, 1H), 7.57 (t, *J* = 7.7 Hz, 1H), 7.40 (s, 1H), 7.14 (d, *J* = 8.8 Hz, 1H), 6.96 (s, 1H), 6.13 (s, 3H), 4.95 (dd, *J =* 11.1, 4.9 Hz, 1H), 4.56 (dd, *J=* 18.2, 9.2 Hz, 3H), 4.46 (s, 2H), 4.18 (s, 1H), 3.95 - 3.89 (m, 2H), 3.64 (s, 3H), 2.87 - 2.79 (m, 2H), 2.63 (s, 1H), 2.35 (d, *J =* 12.6 Hz, 1H), 2.24 (s, 2H), 2.16 - 2.00 (m, 5H), 1.92 (d, *J =* 13.6 Hz, 2H), 1.69 (q, *J* = 12.4 Hz, 2H), 1.53 (q, *J* = 12.3 Hz, 2H).

### Examples 49 and 50: Synthesis of Compounds 49 and 50

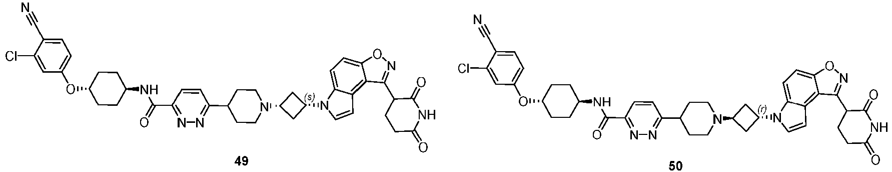

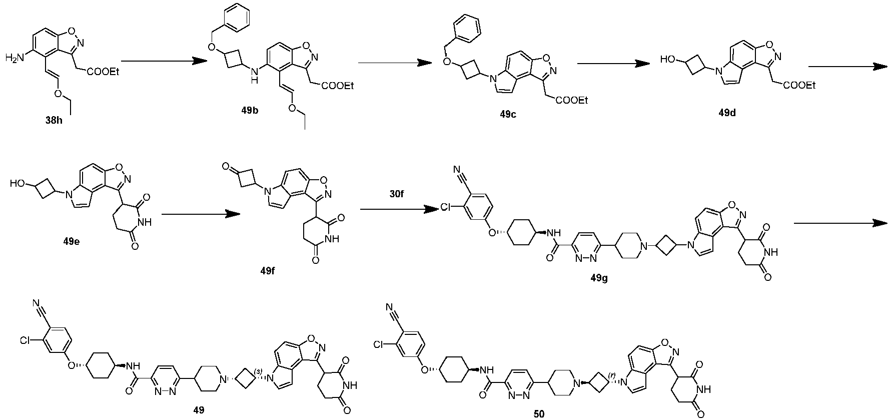

### Step 1: Preparation of intermediate 49b

Intermediate **38h** (5.3 g), 3-(benzyloxy)-1-cyclobutanone (3.38 g), methanol (50 mL), and glacial acetic acid (0.384 g) were added to a reaction flask, and the mixture was stirred at room temperature for 5 min. Sodium cyanoborohydride (1.606 g) was then added, and the mixture was stirred at room temperature overnight. After the reaction was completed, the reaction solution was added with water (300 mL) and extracted with dichloromethane (300 mL). The organic layers were combined, dried over anhydrous sodium sulfate, filtered under vacuum, and concentrated, and the residue was purified by silica gel column chromatography to give intermediate **49b** (3.95 g).

MS(ESI, [M+H]+) m/z: 451.38.

¹H NMR (500 MHz, DMSO-*d₆*) δ 7.41 - 7.37 (m, 1H), 7.35 - 7.32 (m, 4H), 7.30 - 7.28 (m, 1H), 6.91 - 6.76 (m, 1H), 6.67 (t, *J* = 13.3 Hz, 1H), 5.65 (dd, *J =* 13.0, 7.8 Hz, 1H), 4.92 (dd, *J =* 10.5, 6.5 Hz, 1H), 4.42 - 4.37 (m, 2H), 4.21 - 4.11 (m, 2H), 4.10 - 4.04 (m, 4H), 3.94 (q, *J* = 7.0 Hz, 2H), 2.73 (dtdd, *J* = 9.2, 6.7, 4.8, 2.9 Hz, 2H), 2.37 (tt, *J=* 10.7, 4.9 Hz, 1H), 1.85 (qd, *J=* 8.5, 2.8 Hz, 1H), 1.30 (td, *J=* 7.0, 1.6 Hz, 3H), 1.20 - 1.17 (m, 3H).

### Step 2: Preparation of intermediate 49c

Intermediate **49b** (3.95 g), dichloromethane (40 mL), and hydrochloric acid (6.58 mL, 4 mol/L) were added to a reaction flask in sequence, and the mixture was reacted at room temperature for 4 h. An NaHCO₃ aqueous solution (200 mL) and dichloromethane (200 mL) were added to the reaction solution. The organic phase was separated, dried over anhydrous sodium sulfate, filtered, and concentrated, and the concentrate was separated and purified by silica gel column chromatography to give intermediate **49c** (2.51 g).

MS(ESI, [M+H]⁺) *m*/*z:* 405.3.

¹H NMR (500 MHz, DMSO-*d*₆) δ 7.87 - 7.75 (m, 2H), 7.47 (dd, *J* = 9.1, 7.2 Hz, 1H), 7.41 - 7.27 (m, 6H), 6.69 (t, *J* = 3.6 Hz, 1H), 4.48 (s, 2H), 4.42 - 4.33 (m, 1H), 4.26 (d, *J=* 1.4 Hz, 2H), 4.14 (qd, *J* = 7.1, 2.3 Hz, 2H), 4.05 - 3.96 (m, 1H), 2.97 (dtd, *J* = 9.1, 6.7, 2.9 Hz, 1H), 2.69 (dt, *J* = 8.5, 5.4 Hz, 1H), 2.34 (ddd, *J=* 11.7, 9.1, 7.4 Hz, 1H), 1.18 (td, *J* = 7.1, 3.0 Hz, 3H).

### Step 3: Preparation of intermediate 49d

Intermediate **49c** (1.5 g), a 10% palladium on carbon catalyst (0.75 g), MeOH (30 mL), and hydrochloric acid (0.926 mL, 4 mol/L) were added to a reaction flask in sequence, and the mixture was reacted at room temperature under H₂ atmosphere for 4 h. The reaction solution was filtered under vacuum, and the filtrate was concentrated and then separated and purified by silica gel column chromatography to give intermediate **49d** (0.765 g).

MS(ESI, [M+H]⁺) *m*/*z:* 315.2.

¹H NMR (500 MHz, DMSO-*d*₆) δ 7.83 - 7.77 (m, 1H), 7.77 - 7.71 (m, 1H), 7.46 (dd, *J* = 9.1, 4.1 Hz, 1H), 6.67 (t, *J =* 2.8 Hz, 1H), 5.25 (tt, *J =* 8.2, 5.8 Hz, 1H), 4.61 (tt, *J =* 9.3, 7.2 Hz, 1H), 4.31 - 4.24 (m, 2H), 4.12 (dq, *J =* 13.5, 7.1 Hz, 2H), 4.07 - 4.00 (m, 1H), 2.93 (dtd, *J* = 9.7, 6.9, 2.9 Hz, 1H), 2.71 - 2.59 (m, 1H), 2.53 (dd, *J* = 8.2, 4.2 Hz, 1H), 2.29 - 2.22 (m, 1H), 1.18 (td, *J* = 7.1, 1.5 Hz, 3H).

### Step 4: Preparation of intermediate 49e

Intermediate **49d** (1.7 g), acrylamide (0.461 g), and anhydrous tetrahydrofuran (30 mL) were added to a reaction flask in sequence. Potassium *tert*-butoxide (0.91 g) was slowly added under an ice bath, and the mixture was reacted for 1 h. A saturated ammonium chloride aqueous solution (100 mL) was added to the reaction solution to quench the reaction, and the mixture was extracted with dichloromethane. The organic phase was separated, dried over anhydrous sodium sulfate, filtered, and concentrated, and the concentrate was separated and purified by silica gel column chromatography to give intermediate **49e** (0.84 g).

MS(ESI, [M+H]⁺) *m*/*z:* 340.2.

### Step 5: Preparation of intermediate 49f

Intermediate **49e** (400 mg), acetonitrile (10.00 mL), and an IBX oxidant (353 mg) were added to a reaction flask in sequence, and the mixture was reacted at 80 °C for 1 h. The reaction solution was directly filtered to remove solids, and the filtrate was concentrated to give intermediate **49f** (410 mg).

MS(ESI, [M+H]⁺) *m*/*z:* 338.3.

### Step 6: Preparation of compound 49 and compound 50

Intermediate **30f** (200 mg), intermediate **49f** (177 mg), dichloroethane (20 mL), isopropanol (4 mL), and glacial acetic acid (21 mg) were added to a reaction flask, and the mixture was stirred at room temperature for 15 min. Sodium cyanoborohydride (54.9 mg) was then added, and the mixture was stirred at room temperature overnight. After the reaction was completed, the reaction solution was added with water (100 mL) and extracted with dichloromethane (100 mL). The organic phases were combined, dried over anhydrous sodium sulfate, filtered under vacuum, and concentrated, and the residue was purified by silica gel column chromatography to give compound **49g** (128 mg). The compound was separated by preparative high performance liquid chromatography to give compound **49** (84 mg) and compound **50** (11 mg) separately. The conditions for the preparative chromatography were as follows:
Instrument and preparative column: Shimadzu LC-20AD high performance liquid chromatograph was used, and the preparative column was Uitimate XB-Phenyl (4.6×250 mm, 10 µm). Mobile phase system: acetonitrile/10 mM ammonium acetate, isocratic elution: acetonitrile/10 mM ammonium acetate = 60/40.

### Compound 49:

MS(ESI, [M+H]+) m/z: 761.45.

¹H NMR (500 MHz, DMSO-*d*₆) δ 11.20 (s, 1H), 9.05 (d, *J* = 8.2 Hz, 1H), 8.12 (d, *J =* 8.6 Hz, 1H), 7.91 - 7.84 (m, 3H), 7.80 (d, *J =* 9.1 Hz, 1H), 7.49 (d, *J =* 9.0 Hz, 1H), 7.40 (d, *J=* 2.4 Hz, 1H), 7.15 (dd, *J =* 8.9, 2.4 Hz, 1H), 6.63 (d, *J =* 3.0 Hz, 1H), 5.18 (s, 1H), 4.71 (dd, *J=* 11.8, 5.1 Hz, 1H), 4.55 (tt, *J* = 10.1, 4.3 Hz, 1H), 3.92 (ddp, *J* = 11.7, 8.1, 4.1 Hz, 1H), 3.21 - 2.98 (m, 4H), 2.87 (ddd, *J* = 17.4, 12.1, 5.4 Hz, 1H), 2.72 - 2.60 (m, 3H), 2.54 (t,*J* = 7.0 Hz, 3H), 2.44 (td, *J* = 12.4, 4.3 Hz, 1H), 2.25 (dt, *J* = 13.6, 4.7 Hz, 1H), 2.16 - 2.09 (m, 2H), 2.03 - 1.85 (m, 4H), 1.70 (td, *J* = 14.2, 7.1 Hz, 2H), 1.53 (td, *J =* 13.8, 7.0 Hz, 2H), 1.32 - 1.18 (m, 3H).

### Compound 50:

MS(ESI, [M+H]+) m/z: 761.51.

¹H NMR (500 MHz, DMSO-*d₆*) δ 11.19 (s, 1H), 9.03 (d, *J =* 8.2 Hz, 1H), 8.11 (d, *J* = 8.7 Hz, 1H), 7.86 (td, *J* = 9.6, 7.7 Hz, 3H), 7.78 (d, *J=* 3.2 Hz, 1H), 7.51 (d, *J=* 9.1 Hz, 1H), 7.40 (d, *J=* 2.4 Hz, 1H), 7.14 (dd, *J* = 8.8, 2.4 Hz, 1H), 6.61 (d, *J* = 3.1 Hz, 1H), 4.87 (p, *J* = 8.6 Hz, 1H), 4.70 (dd, *J =* 11.9, 5.1 Hz, 1H), 4.54 (tt, *J* = 9.9, 4.1 Hz, 1H), 3.91 (dtt, *J =* 11.5, 8.0, 4.0 Hz, 1H), 3.03 (d, *J* = 11.0 Hz, 3H), 2.85 (ddd, *J =* 26.9, 13.5, 7.1 Hz, 3H), 2.75 - 2.68 (m, 1H), 2.64 (dt, *J=* 17.2, 4.2 Hz, 1H), 2.44 (qd, *J* = 12.3, 4.4 Hz, 4H), 2.28 - 2.19 (m, 3H), 2.16 - 2.08 (m, 2H), 2.04 - 1.79 (m, 5H), 1.69 (qd, *J=* 13.2, 3.2 Hz, 2H), 1.58 - 1.46 (m, 2H).

### Examples 51 and 52: Synthesis of Compounds 51 and 52

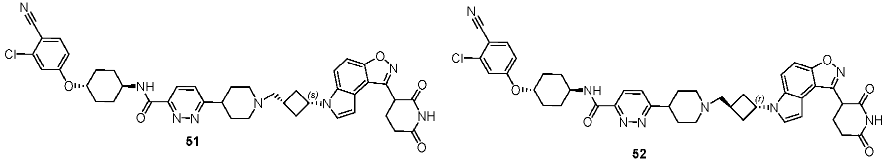

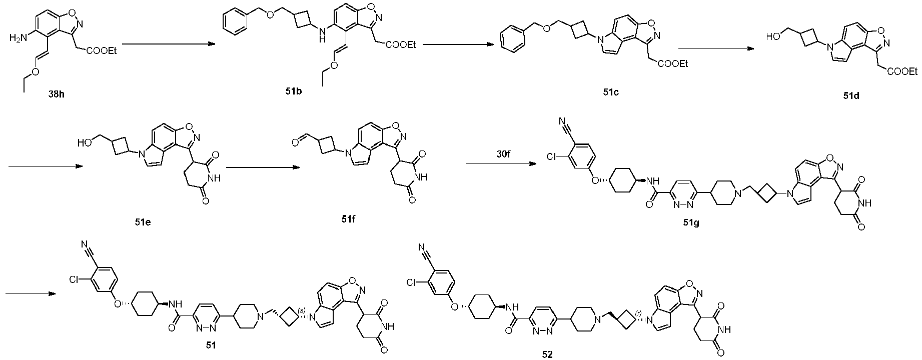

### Step 1: Preparation of intermediate 51b

Intermediate **38h** (5.0 g), 3-(benzyloxymethyl)-1-cyclobutanone (4.42 g), methanol (50 mL), and glacial acetic acid (0.465 g) were added to a reaction flask, and the mixture was stirred at room temperature for 5 min. Sodium cyanoborohydride (1.606 g) was then added, and the mixture was heated at 60 °C. After the reaction was completed, the reaction solution was added with water (300 mL) and extracted with dichloromethane (300 mL). The organic layers were combined, dried over anhydrous sodium sulfate, filtered under vacuum, and concentrated, and the residue was purified by silica gel column chromatography to give intermediate 51b (4.73 g).

MS(ESI, [M+H]+) m/z: 465.30.

### Step 2: Preparation of intermediate 51c

Intermediate **51b** (4.60 g), dichloromethane (50 mL), and hydrochloric acid (5.94 mL, 4 mol/L) were added to a reaction flask in sequence, and the mixture was reacted at room temperature for 4 h. An NaHCO₃ aqueous solution (200 mL) and dichloromethane (200 mL) were added to the reaction solution. The organic phase was separated, dried over anhydrous sodium sulfate, filtered, and concentrated, and the concentrate was separated and purified by silica gel column chromatography to give intermediate **51c** (3.56 g).

MS(ESI, [M+H]⁺) *m*/*z:* 419.33.

### Step 3: Preparation of intermediate 51d

Intermediate **51c** (3.50 g), a 10% palladium on carbon catalyst (1.75 g), MeOH (35 mL), and hydrochloric acid (2.01 mL, 4 mol/L) were added to a reaction flask in sequence, and the mixture was reacted at room temperature under H₂ atmosphere for 4 h. The reaction solution was filtered under vacuum, and the filtrate was concentrated and then separated and purified by column chromatography to give intermediate **51d** (2.05 g).

MS(ESI, [M+H]⁺) *m*/*z:* 329.32.

### Step 4: Preparation of intermediate 51e

Intermediate **51d** (2.0 g), acrylamide (0.476 g), and anhydrous tetrahydrofuran (30 mL) were added to a reaction flask in sequence. A solution of potassium *tert*-butoxide in THF (1.03 g, 9.14 mL, 9.14 mmol) was slowly added under an ice bath, and the mixture was reacted for 1 h. A saturated ammonium chloride aqueous solution (100 mL) was added to the reaction solution to quench the reaction, and the mixture was extracted with dichloromethane. The organic phase was separated, dried over anhydrous sodium sulfate, filtered, and concentrated, and the concentrate was separated and purified by silica gel column chromatography to give intermediate **51e** (1.40 g).

MS(ESI, [M+H]⁺) *m*/*z:* 354.35*.*

### Step 5: Preparation of intermediate 51f

Intermediate **51e** (1.2 g), acetonitrile (10.00 mL), and an IBX oxidant (1.43 g) were added to a reaction flask in sequence, and the mixture was reacted at 80 °C for 1 h. The reaction solution was filtered, and the filtrate was concentrated to give intermediate **51f** (1.39 g).

MS(ESI, [M+H]⁺) *m*/*z:* 352.36*.*

### Step 6: Preparation of compound 51 and compound 52

Intermediate **30f** (150 mg), intermediate **51f** (127 mg), dichloroethane (10 mL), isopropanol (4 mL), and glacial acetic acid (9.83 mg) were added to a reaction flask, and the mixture was stirred at room temperature for 5 min. Sodium triacetoxyborohydride (139 mg) was then added, and the mixture was stirred at room temperature overnight. After the reaction was completed, the reaction solution was added with water (100 mL) and extracted with dichloromethane (100 mL). The organic phases were combined, dried over anhydrous sodium sulfate, filtered under vacuum, and concentrated, and the residue was purified by silica gel column chromatography to give compound **51g** (162 mg). The compound was separated by preparative high performance liquid chromatography to give compound **51** (85 mg) and compound **52** (21 mg) in sequence. The conditions for the preparative chromatography were as follows:
Instrument and preparative column: Shimadzu LC-20AD high performance liquid chromatograph was used, and the preparative column was Uitimate XB-Phenyl (4.6×250 mm, 10 µm). Mobile phase system: acetonitrile/10 mM ammonium acetate, isocratic elution: acetonitrile/10 mM ammonium acetate = 60/40.

### Compound 51:

MS(ESI, [M+H]+) m/z: 775.34.

¹H NMR (500 MHz, DMSO-*d*₆) δ 11.19 (s, 1H), 9.02 (d, *J* = 8.2 Hz, 1H), 8.10 (d, *J =* 8.6 Hz, 1H), 7.89 - 7.81 (m, 3H), 7.79 (d, *J* = 3.2 Hz, 1H), 7.49 (d, *J* = 9.1 Hz, 1H), 7.40 (d, *J* = 2.4 Hz, 1H), 7.14 (dd, *J* = 8.7, 2.5 Hz, 1H), 6.60 (d, *J =* 3.1 Hz, 1H), 4.99 (p, *J=* 8.5 Hz, 1H), 4.70 (dd, *J =* 11.8, 5.1 Hz, 1H), 4.54 (tt, *J* = 10.2, 4.3 Hz, 1H), 3.91 (dtd, *J =* 11.6, 7.7, 4.2 Hz, 1H), 3.04 - 2.93 (m, 3H), 2.86 (ddd, *J* = 17.3, 12.1, 5.4 Hz, 1H), 2.79 - 2.70 (m, 2H), 2.64 (dt, *J* = 17.1, 4.2 Hz, 1H), 2.54 (d, *J* = 6.8 Hz, 3H), 2.44 (dp, *J* = 17.5, 6.9, 5.9 Hz, 3H), 2.23 (dq, *J* = 13.9, 4.8 Hz, 1H), 2.13 (q, *J* = 9.7, 8.6 Hz, 5H), 1.95 - 1.79 (m, 5H), 1.69 (qd, *J* = 13.3, 3.2 Hz, 2H), 1.58 - 1.46 (m, 2H).

### Compound 52:

MS(ESI, [M+H]+) m/z: 775.45.

¹H NMR (500 MHz, DMSO-*d₆*) δ 11.19 (s, 1H), 9.02 (d, *J* = 8.2 Hz, 1H), 8.10 (d, *J* = 8.6 Hz, 1H), 7.89 - 7.81 (m, 4H), 7.49 (d, *J* = 9.0 Hz, 1H), 7.40 (d, *J* = 2.4 Hz, 1H), 7.14 (dd, *J =* 8.8, 2.4 Hz, 1H), 6.61 (d, *J =* 3.1 Hz, 1H), 5.30 (p, *J =* 7.7 Hz, 1H), 4.70 (dd, *J =* 11.9, 5.1 Hz, 1H), 4.54 (tt, *J =* 10.1, 4.2 Hz, 1H), 3.91 (tdt, *J* = 11.8, 8.3, 4.0 Hz, 1H), 3.06 (d, *J =* 10.9 Hz, 2H), 3.00 (dq, *J =* 11.2, 5.6, 3.9 Hz, 1H), 2.86 (ddd, *J =* 17.2, 12.0, 5.3 Hz, 1H), 2.68 - 2.62 (m, 3H), 2.58 (dq, *J* = 15.7, 8.0, 7.6 Hz, 3H), 2.48 - 2.36 (m, 3H), 2.24 (dt, *J* = 8.9, 4.6 Hz, 1H), 2.22 - 2.12 (m, 3H), 2.11 (d, *J =* 3.9 Hz, 1H), 1.91 (td, *J =* 12.6, 11.4, 7.0 Hz, 5H), 1.85 (dd, *J =* 12.3, 8.9 Hz, 1H), 1.69 (qd, *J* = 13.2, 3.3 Hz, 2H), 1.58 - 1.46 (m, 2H).

### Example 53: Synthesis of Compound 53

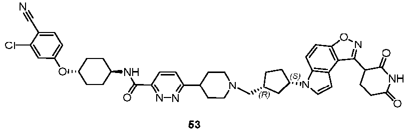

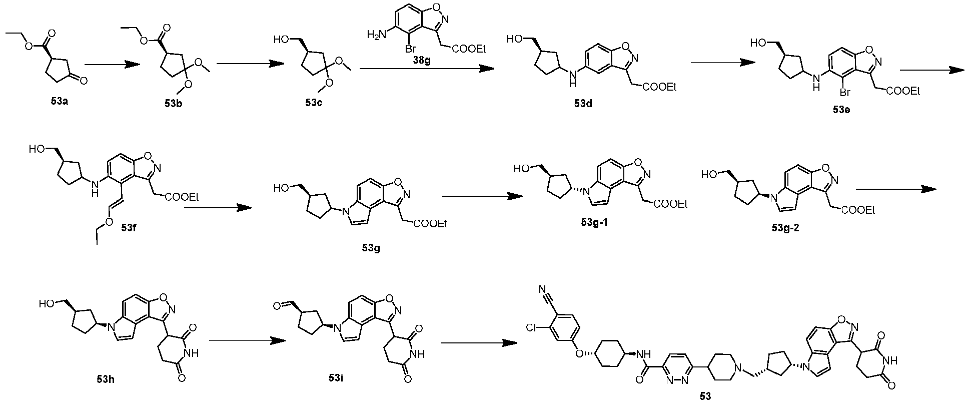

### Step 1: Preparation of intermediate 53b

Intermediate **53a** (5 g), trimethyl orthoformate (11.2 g),p-toluenesulfonic acid (0.606 g), and ethanol (50 mL) were added to a reaction flask in sequence, and the mixture was reacted at room temperature overnight. The system was added with a saturated sodium bicarbonate aqueous solution (about 100 mL) and extracted with ethyl acetate. The organic phases were combined, dried over anhydrous sodium sulfate, filtered, and concentrated, and the concentrate was separated and purified by silica gel column chromatography to give intermediate **53b** (4.5 g).

¹H NMR (500 MHz, Chloroform-d) δ 4.14 *(q, J=* 7.1 Hz, 2H), 3.21 (d, *J=* 10.6 Hz, 6H), 2.92 - 2.81 (m, 1H), 2.14 - 2.03 (m, 2H), 2.01 - 1.91 (m, 2H), 1.91 - 1.78 (m, 2H), 1.25 *(t, J=* 7.1 Hz, 3H).

### Step 2: Preparation of intermediate 53c

Intermediate **53b** (4.5 g) and THF (50 mL) were added to a reaction flask, lithium aluminum hydride (0.998 g) was slowly added under an ice bath, and the mixture was then slowly warmed to room temperature and reacted for 1 h. After the reaction was completed, a small amount of ice water was added to the reaction solution under an ice bath to quench the reaction, and DCM (200 mL) and water (200 mL) were then added. The mixture was filtered, and the organic phase of the filtrate was separated, washed with a saturated sodium chloride solution, dried over anhydrous sodium sulfate, filtered, and concentrated to give intermediate **53c** (3.9 g).

¹H NMR (500 MHz, Chloroform-*d*) δ 3.55 (dhept, *J* = 15.8, 5.4, 4.9 Hz, 2H), 3.21 (d, *J* = 3.0 Hz, 6H), 2.28 (dddd, *J =* 15.1, 8.8, 7.6, 3.9 Hz, 1H), 2.05 - 1.96 (m, 1H), 1.90 (dddd, *J* = 10.2, 8.8, 3.7, 1.5 Hz, 2H), 1.85 - 1.81 (m, 1H), 1.80 - 1.73 (m, 1H), 1.58 (ddd, *J =* 13.4, 7.3, 1.3 Hz, 1H), 1.51 - 1.40 (m, 1H).

### Step 3: Preparation of intermediate 53d

Intermediate **38g** (2.2 g), intermediate **53c** (1.3 g), triethylsilane (1.71 g), and acetonitrile (20 mL) were added to a reaction flask, iodine (1.87 g) was then added, and the mixture was warmed to 90 °C and reacted overnight. The reaction solution was cooled to room temperature and concentrated, and DCM (200 mL) and water (200 mL) were added. The organic phase was separated, washed with a saturated sodium chloride solution, dried over anhydrous sodium sulfate, filtered, and concentrated, and the concentrate was separated and purified by silica gel column chromatography to give intermediate **53d** (1.9 g).

MS (ESI, [M+H]⁺) m/z: 319.2.

### Step 4: Preparation of intermediate 53e

Intermediate **53d** (1.9 g) and DCM (30 mL) were added to a reaction flask in sequence, NBS (1.06 g) was then added, and the mixture was reacted at room temperature for 1 h. DCM (100 mL) and water (100 mL) were added to the reaction solution. The organic phase was separated, washed with a saturated sodium chloride solution, dried over anhydrous sodium sulfate, filtered, and concentrated, and the concentrate was separated and purified by silica gel column chromatography to give intermediate **53e** (0.75 g).

MS(ESI, [M+H]⁺) *m*/*z:* 397.2.

### Step 5: Preparation of intermediate 53f

Intermediate **53e** (0.7 g), (E)-1-ethoxyvinyl-2-boronic acid pinacol ester (0.419 g), [1,1'-bis(diphenylphosphino)ferrocene]palladium dichloride dichloromethane complex (0.129 g), potassium carbonate (0.731 g), 1,4-dioxane (10 mL), and water (2 mL) were added to a microwave tube in sequence, N₂ was bubbled, and the mixture was microwaved at 120 °C and reacted for 2 h. The reaction solution was cooled to room temperature, and ethyl acetate (100 mL) and water (100 mL) were added. The organic phase was separated, dried over anhydrous sodium sulfate, filtered, and concentrated, and the concentrate was separated and purified by silica gel column chromatography to give intermediate **53f** (0.51 g).

MS(ESI, [M+H]⁺) *m*/*z:* 389.2.

### Step 6: Preparation of intermediate 53g

Intermediate **53f** (0.5 g), DCM (20 mL), and hydrochloric acid (0.644 mL, 4 mol/L) were added to a reaction flask in sequence, and the mixture was reacted at room temperature for 5 h. DCM (100 mL) and a saturated sodium bicarbonate solution (100 mL) were added to the system. The organic phase was separated, and the aqueous phase was then extracted with DCM (50 mL). The organic phases were combined, dried over anhydrous sodium sulfate, filtered, and concentrated, and the concentrate was separated and purified by silica gel column chromatography to give intermediate **53g** (0.24 g).

MS(ESI, [M+H]⁺) *m*/*z:* 343.1.

### Step 7: Preparation of intermediates 53g-1 and 53g-2

Intermediate **53g** was separated by preparative high performance liquid chromatography to give intermediates 53g-**1** (0.77 g) and **53g-2** (1.51 g) in sequence. The conditions for the preparative chromatography were as follows:

Instrument and preparative column: YMC K-prep Lab100g high pressure preparative chromatograph was used, and the preparative column was YMC Sil SLG12S11-2530 (30×250 mm, 10 µm). Mobile phase system: ethanol/*n-*hexane, isocratic elution: ethanol/*n*-hexane = 60/40.

### Intermediate 53g-1:

MS(ESI, [M+H]⁺) *m*/*z*: 343.3.

¹H NMR (500 MHz, DMSO-*d*₆) δ 7.91 - 7.84 (m, 1H), 7.68 (d, *J =* 3.1 Hz, 1H), 7.47 (d, *J* = 9.1 Hz, 1H), 6.65 (d, *J =* 3.1 Hz, 1H), 5.05 (p, *J =* 7.1 Hz, 1H), 4.61 (t, *J =* 5.3 Hz, 1H), 4.26 (s, 2H), 4.14 (q, *J* = 7.1 Hz, 2H), 3.40 (ddd, *J =* 6.7, 5.2, 1.5 Hz, 2H), 2.42 - 2.31 (m, 1H), 2.22 (tdd, *J =* 13.3, 9.0, 6.0 Hz, 1H), 2.01 - 1.89 (m, 4H), 1.46 (tdd, *J =* 13.4, 7.5, 4.5 Hz, 1H), 1.18 (td, *J =* 7.1, 3.8 Hz, 3H).

### Intermediate 53g-2:

MS(ESI, [M+H]⁺) *m*/*z*: 343.2.

¹H NMR (500 MHz, DMSO-*d*₆) δ 7.90 (d, *J* = 9.0 Hz, 1H), 7.72 (d, *J =* 3.2 Hz, 1H), 7.46 (d, *J =* 9.1 Hz, 1H), 6.65 (d, *J =* 3.1 Hz, 1H), 5.02 (dq, *J =* 9.5, 7.6 Hz, 1H), 4.62 (t, *J =* 5.2 Hz, 1H), 4.26 (s, 2H), 4.16 - 4.12 (m, 2H), 3.44 (ddd, *J =* 6.5, 5.2, 1.6 Hz, 2H), 2.32 (dt, *J =* 12.4, 7.3 Hz, 1H), 2.20 (dddd, *J =* 25.0, 12.5, 8.0, 6.0 Hz, 2H), 1.97 - 1.75 (m, 2H), 1.72 - 1.59 (m, 2H), 1.18 (t, *J* = 7.1 Hz, 3H).

### Step 8: Preparation of intermediate 53h

Intermediate **53g-2** (0.35 g), acrylamide (0.078 g), and anhydrous tetrahydrofuran (20 mL) were added to a reaction flask in sequence. Potassium *tert*-butoxide (0.123 g) was slowly added under an ice bath, and the mixture was reacted for 1 h. A saturated ammonium chloride aqueous solution (100 mL) was added to the reaction solution to quench the reaction, and the mixture was extracted with dichloromethane. The organic phase was separated, dried over anhydrous sodium sulfate, filtered, and concentrated, and the concentrate was separated and purified by silica gel column chromatography to give intermediate **53h** (0.27 g).

MS(ESI, [M+H]⁺) *m*/*z*: 368.2.

¹H NMR (500 MHz, DMSO-*d*₆) δ 11.18 (s, 1H), 7.91 (d, *J* = 9.2 Hz, 1H), 7.72 (d, *J =* 3.2 Hz, 1H), 7.48 (d, *J* = 9.1 Hz, 1H), 6.57 (d, *J* = 3.1 Hz, 1H), 5.02 (p, *J =* 8.1 Hz, 1H), 4.69 (dd, *J =* 11.9, 5.1 Hz, 1H), 4.62 (t, *J =* 5.2 Hz, 1H), 3.46 - 3.41 (m, 2H), 2.85 (ddd, *J =* 17.4, 12.1, 5.4 Hz, 1H), 2.63 (dt, *J =* 17.2, 4.2 Hz, 1H), 2.44 (qd, *J =* 12.2, 4.4 Hz, 1H), 2.31 (dt, *J =* 12.2, 7.3 Hz, 1H), 2.21 (ddt, *J =* 26.5, 12.5, 5.5 Hz, 3H), 1.94 - 1.79 (m, 2H), 1.64 (tdd, *J* = 12.4, 5.9, 3.3 Hz, 2H).

### Step 3: Preparation of intermediate 53i

Intermediate **53h** (0.27 g), acetonitrile (20.00 mL), and an IBX oxidant (0.309 g) were added to a reaction flask in sequence, and the mixture was reacted at 80 °C for 1 h. The reaction solution was directly filtered, and the filtrate was concentrated to give intermediate **53i** (0.25 g).

MS(ESI, [M+H]⁺) *m*/*z*: 366.3.

### Step 4: Preparation of compound 53

Intermediate **30f** (60 mg), intermediate **53i** (59 mg), dichloroethane (10 mL), and isopropanol (2 mL) were added to a reaction flask, sodium triacetoxyborohydride (21 mg) was added, and the mixture was stirred at room temperature overnight. After the reaction was completed, the reaction solution was added with water (100 mL) and extracted with dichloromethane (100 mL). The organic phase was separated, dried over anhydrous sodium sulfate, filtered under vacuum, and concentrated, and the residue was purified by silica gel column chromatography and C₁₈ reversed-phase column chromatography to give compound **53** (31 mg).

Q-TOF(ESI, [M+H]⁺) *m*/*z:* 789.3286.

¹H NMR (500 MHz, DMSO-*d*₆) δ 11.18 (s, 1H), 9.02 (d, *J =* 8.2 Hz, 1H), 8.09 (d, *J =* 8.7 Hz, 1H), 7.91 (d, *J =* 9.2 Hz, 1H), 7.84 (dd, *J =* 17.6, 8.7 Hz, 2H), 7.75 (d, *J =* 3.2 Hz, 1H), 7.49 (d, *J =* 9.1 Hz, 1H), 7.40 (d, *J =* 2.5 Hz, 1H), 7.14 (dd, *J =* 8.8, 2.4 Hz, 1H), 6.59 (t, *J =* 2.5 Hz, 1H), 5.10 - 5.00 (m, 1H), 4.69 (dd, *J =* 11.8, 5.2 Hz, 1H), 4.54 (tt, *J =* 10.3, 4.0 Hz, 1H), 3.91 (qd, *J* = 8.4, 5.7, 4.3 Hz, 1H), 3.05 (s, 2H), 2.98 (s, 1H), 2.86 (ddd, *J =* 17.3, 12.3, 5.5 Hz, 1H), 2.63 (dt, *J =* 17.3, 4.1 Hz, 1H), 2.42 (dt, *J =* 23.6, 7.2 Hz, 5H), 2.22 (dd, *J =* 13.2, 6.1 Hz, 2H), 2.11 (d, *J =* 11.0 Hz, 4H), 1.90 (d, *J* = 13.8 Hz, 5H), 1.83 (d, *J =* 11.9 Hz, 2H), 1.74 - 1.58 (m, 4H), 1.57 - 1.47 (m, 3H).

### Example 54: Synthesis of Compound 54

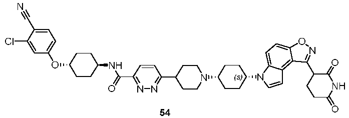

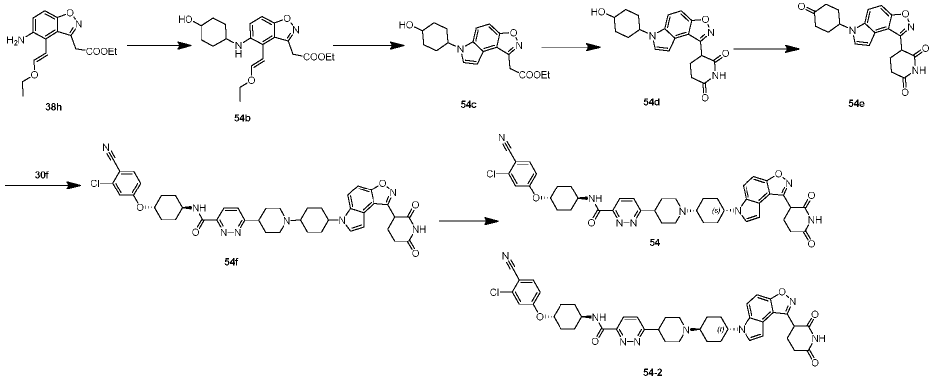

### Step 1: Preparation of intermediate 54b

Intermediate **38h** (1.0 g), 4-hydroxycyclohexanone (0.662 g), methanol (20 mL), and glacial acetic acid (0.384 g) were added to a reaction flask, and the mixture was stirred at room temperature for 5 min. Sodium cyanoborohydride (1.606 g) was then added, and the mixture was stirred at room temperature overnight. After the reaction was completed, the reaction solution was added with water (100 mL) and extracted with dichloromethane (100 mL). The organic phases were combined, dried over anhydrous sodium sulfate, filtered under vacuum, and concentrated, and the residue was purified by silica gel column chromatography to give intermediate **54b** (0.71 g).

MS(ESI, [M+H]+) m/z: 389.31.

¹H NMR (500 MHz, DMSO-*d*₆) δ 7.44 - 7.38 (m, 1H), 7.05 (d, *J* = 9.1 Hz, 1H), 6.66 (d, *J* = 13.1 Hz, 1H), 5.63 (d, *J =* 13.1 Hz, 1H), 4.47 - 4.38 (m, 1H), 4.27 (d, *J =* 8.5 Hz, 1H), 4.11 - 4.04 (m, 4H), 3.94 (q, *J =* 7.1 Hz, 2H), 3.70 (q, *J =* 4.0 Hz, 1H), 3.38 (d, *J =* 7.4 Hz, 1H), 2.37 (ddd, *J* = 14.0, 8.2, 5.9 Hz, 1H), 2.22 (ddd, *J* = 15.8, 8.5, 5.8 Hz, 1H), 1.95 - 1.86 (m, 1H), 1.77 - 1.70 (m, 1H), 1.62 (q, *J* = 6.4, 6.0 Hz, 2H), 1.54 (dt, *J =* 6.7, 3.5 Hz, 2H), 1.30 (t, *J =* 7.1 Hz, 3H), 1.19 (d, *J =* 6.9 Hz, 3H).

### Step 2: Preparation of intermediate 54c

Intermediate **54b** (0.45 g), dichloromethane (10 mL), and hydrochloric acid (0.87 mL, 4 mol/L) were added to a reaction flask in sequence, and the mixture was reacted at room temperature for 4 h. An NaHCO₃ aqueous solution (50 mL) and dichloromethane (50 mL) were added to the reaction solution. The organic phase was separated, dried over anhydrous sodium sulfate, filtered, and concentrated, and the concentrate was separated and purified by silica gel column chromatography to give intermediate **54c** (0.24 g).

MS(ESI, [M+H]⁺) *m*/*z*: 343.3.

### Step 3: Preparation of intermediate 54d

Intermediate **54c** (0.25 g), acrylamide (0.057 g), and anhydrous tetrahydrofuran (10 mL) were added to a reaction flask in sequence. Potassium *tert*-butoxide (0.09 g) was slowly added under an ice bath, and the mixture was reacted for 1 h. A saturated ammonium chloride aqueous solution (50 mL) was added to the reaction solution to quench the reaction, and the mixture was extracted with dichloromethane (50 mL). The organic phase was separated, dried over anhydrous sodium sulfate, filtered, and concentrated, and the concentrate was separated and purified by silica gel column chromatography to give intermediate **54d** (0.21 g).

MS(ESI, [M+H]⁺) *m*/*z*: 368.2.

¹H NMR (500 MHz, DMSO-*d*₆) δ 11.18 (s, 1H), 7.92 (d, *J =* 9.2 Hz, 1H), 7.70 - 7.66 (m, 1H), 7.50 - 7.46 (m, 1H), 6.56 (d, *J =* 3.1 Hz, 1H), 4.68 (dd, *J =* 11.9, 5.1 Hz, 1H), 4.57 - 4.50 (m, 2H), 3.94 (q, *J =* 3.0 Hz, 1H), 2.85 (ddd, *J* = 17.4, 12.2, 5.4 Hz, 1H), 2.63 (dt, *J =* 17.3, 4.1 Hz, 1H), 2.43 (td, *J =* 12.7, 4.6 Hz, 1H), 2.27 - 2.09 (m, 4H), 1.83 (d, *J =* 13.0 Hz, 3H), 1.78 - 1.69 (m, 2H).

### Step 4: Preparation of intermediate 54e

Intermediate **54d** (2.3 g), acetonitrile (10.00 mL), and an IBX oxidant (2.104 g) were added to a reaction flask in sequence, and the mixture was reacted at 80 °C for 1 h. The reaction solution was directly filtered, and the filtrate was concentrated to give intermediate **54e** (1.63 g).

MS(ESI, [M+H]⁺) *m*/*z*: 366.3.

¹H NMR (500 MHz, DMSO-*d*₆) δ 11.18 (s, 1H), 8.04 (d, *J =* 9.1 Hz, 1H), 7.76 - 7.71 (m, 1H), 7.54 (d, *J =* 9.1 Hz, 1H), 6.59 (dd, *J =* 8.9, 3.2 Hz, 1H), 5.15 (tt, *J =* 11.1, 4.2 Hz, 1H), 4.73 - 4.64 (m, 1H), 3.17 (d, *J* = 3.7 Hz, 1H), 2.90 - 2.75 (m, 3H), 2.63 (dt, *J =* 16.3, 3.7 Hz, 1H), 2.44 (tt, *J* = 12.3, 6.1 Hz, 1H), 2.38 - 2.29 (m, 3H), 2.24 (tt, *J* = 9.1, 3.9 Hz, 3H).

### Step 5: Preparation of compounds 54 and 54-2

Intermediate **30f** (100 mg), intermediate **54e** (183 mg), dichloroethane (20 mL), isopropanol (4 mL), and glacial acetic acid (26 mg) were added to a reaction flask, and the mixture was stirred at room temperature for 5 min. Sodium cyanoborohydride (53 mg) was then added, and the mixture was warmed to 80 °C and reacted for 12 h. After the reaction was completed, the reaction solution was added with water (100 mL) and extracted with dichloromethane. The organic phases were combined, dried over anhydrous sodium sulfate, filtered under vacuum, and concentrated. The residue was purified by silica gel column chromatography to give compound **54f** (110 mg), which was then purified by C₁₈ reversed-phase column chromatography to give compound **54** (34 mg) and compound **54-2** (41 mg) in sequence.

### Compound 54:

Q-TOF(ESI, [M+H]⁺) *m*/*z:* 789.3283.

¹H NMR (500 MHz, DMSO-*d*₆) δ 11.19 (s, 1H), 9.02 (d, *J =* 8.2 Hz, 1H), 8.14 (d, *J =* 8.7 Hz, 1H), 7.99 (d, *J =* 9.2 Hz, 1H), 7.87 (dd, *J =* 8.7, 2.7 Hz, 2H), 7.69 (s, 1H), 7.50 (d, *J =* 9.1 Hz, 1H), 7.40 (d, *J =* 2.4 Hz, 1H), 7.15 (dd, *J* = 8.8, 2.5 Hz, 1H), 6.57 (d, *J* = 3.0 Hz, 1H), 4.70 (dd, *J* = 11.8, 5.2 Hz, 2H), 4.55 (tt, *J =* 10.4, 4.4 Hz, 1H), 3.92 (dtt, *J =* 11.5, 7.8, 4.0 Hz, 1H), 3.05 (s, 1H), 2.86 (ddd, *J =* 17.3, 12.0, 5.3 Hz, 1H), 2.63 (dt, *J =* 17.1, 4.2 Hz, 1H), 2.43 (td, *J =* 12.5, 4.3 Hz, 1H), 2.23 (dq, *J =* 14.6, 5.6, 5.1 Hz, 3H), 2.16 - 2.10 (m, 5H), 2.06 - 1.97 (m, 4H), 1.96 - 1.89 (m, 4H), 1.82 (s, 3H), 1.76 - 1.64 (m, 5H), 1.58 - 1.49 (m, 2H).

### Example 55: Synthesis of Compound 55

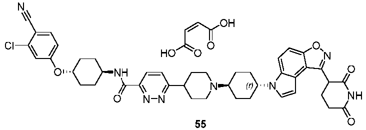

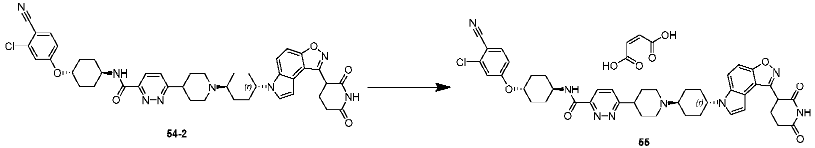

### Step 1: Preparation of compound 55

Compound **54-2** (41 mg) was dissolved in MeOH (5 mL), maleic acid (6 mg) was then added, and the mixture was stirred at room temperature for 0.5 h and then concentrated to dryness. *n*-Hexane was added, and the mixture was stirred, filtered under vacuum, and dried to give compound **55** (37 mg).

Q-TOF(ESI, [M+H]⁺) *m*/*z:* 789.3298.

¹H NMR (500 MHz, DMSO-*d*₆) δ 11.18 (s, 1H), 10.00 (s, 1H), 9.05 (d, *J* = 8.2 Hz, 1H), 8.19 (d, *J =* 8.7 Hz, 1H), 7.96 (d, *J =* 9.2 Hz, 1H), 7.85 (dd, *J =* 15.9, 8.7 Hz, 2H), 7.74 - 7.67 (m, 1H), 7.54 (d, *J =* 9.1 Hz, 1H), 7.40 (s, 1H), 7.15 (d, *J =* 8.9 Hz, 1H), 6.63 - 6.57 (m, 1H), 6.12 (s, 2H), 4.69 (dd, *J =* 11.8, 5.2 Hz, 1H), 4.64 (s, 1H), 4.56 (d, *J* = 12.1 Hz, 1H), 3.93 (s, 1H), 3.61 (d, *J =* 11.2 Hz, 2H), 2.92 - 2.81 (m, 1H), 2.64 (d, *J =* 16.5 Hz, 1H), 2.33 - 2.23 (m, 7H), 2.20 - 2.11 (m, 5H), 1.99 (d, *J =* 12.1 Hz, 3H), 1.92 (s, 5H), 1.70 (q, *J =* 12.6 Hz, 3H), 1.59 - 1.49 (m, 2H).

### Example 56: Synthesis of Compound 56

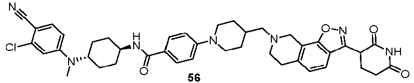

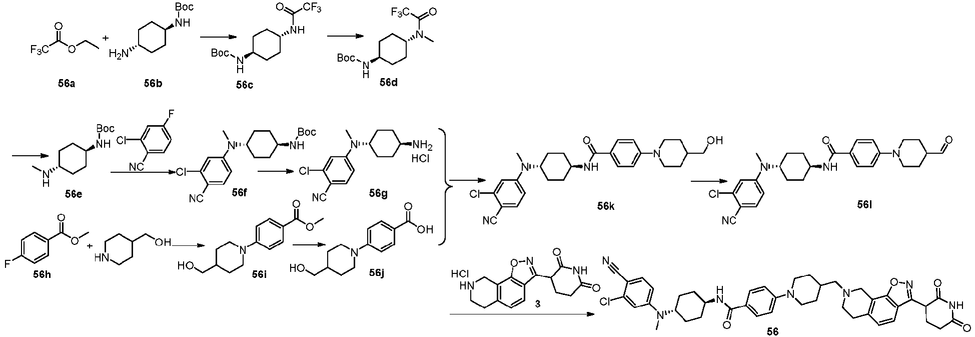

### Step 1: Preparation of intermediate 56c

Intermediate **56b** (95 g) and methanol (950 mL) were added to a reaction flask in sequence, intermediate **56a** (94 g, 79 mL) was added dropwise under an ice bath, and the mixture was reacted at room temperature overnight. After the reaction was completed, the reaction solution was filtered, and the filter cake was washed with an icy methanol solution, collected, and dried at 35 °C under reduced pressure to give intermediate **56c** (104.2 g).

¹H NMR (500 MHz, DMSO-d6) δ 9.26 (d, J = 7.4 Hz, 1H), 6.75 (d, J = 8.0 Hz, 1H), 3.54 (dtd, J = 11.5, 7.6, 3.7 Hz, 1H), 3.17 (dt, J = 14.0, 9.7 Hz, 1H), 1.77 (dt, J = 13.9, 8.5 Hz, 4H), 1.37 (s, 11H), 1.26 - 1.16 (m, 2H).

### Step 2: Preparation of intermediate 56d

60 wt% sodium hydride (6.44 g) was added to a stirred solution of intermediate **56c** (50 g) in *N*,*N-*dimethylformamide (500 mL) at 0 °C in portions, and after the addition, the mixture was stirred at 0 °C for 0.5 h. Iodomethane (20.58 g, 9.07 mL) was slowly added dropwise to the reaction solution, and the mixture was warmed to room temperature and stirred. After the reaction was completed, the reaction solution was poured into crushed ice water, a 2 N citric acid solution was added to adjust the pH to 2-3, and the mixture was filtered and collected to give intermediate **56d** (39.38 g).

¹H NMR (500 MHz, DMSO-d6) δ 6.73 (t, J = 7.5 Hz, 1H), 4.07 - 3.53 (m, 1H), 3.24 (ddp, J = 11.8, 8.1, 3.8 Hz, 1H), 2.95 (d, J = 1.7 Hz, 2H), 2.86 (s, 1H), 1.84 (d, J = 12.7 Hz, 2H), 1.78 (td, J = 12.5, 3.5 Hz, 1H), 1.65 (qd, J = 12.6, 5.2 Hz, 2H), 1.59 - 1.54 (m, 1H), 1.38 (s, 9H), 1.25 (td, J = 11.9, 3.7 Hz, 2H).

### Step 3: Preparation of intermediate 56e

Intermediate **56d** (38.5 g) and methanol (380 mL) were added to a reaction flask in sequence, and the mixture was heated to 50 °C. After the system was completely dissolved, sodium hydroxide (6.17 g) was added, and the mixture was cooled to room temperature and reacted. After the reaction was completed, the reaction solution was concentrated by evaporation under reduced pressure to remove the solvent, the residue was added with a 2 N citric acid solution to adjust the pH to 2-3, and dichloromethane/methanol (10/1) (500 mL) was added for extraction. The organic phase was separated, and the aqueous phase was adjusted to pH 11-12 with a 2 N sodium hydroxide solution and extracted with dichloromethane/methanol (9/1) (800 mL). The organic phases were combined, washed with saturated brine, dried over anhydrous sodium sulfate, and filtered to give intermediate **56e** (25.43 g).

¹H NMR (500 MHz, DMSO-d6) δ 6.67 (d, J = 8.1 Hz, 1H), 3.15 (dt, J = 7.5, 3.8 Hz, 1H), 2.23 (s, 3H), 2.11 (tt, J = 10.8, 3.8 Hz, 1H), 1.85 - 1.80 (m, 2H), 1.75 - 1.70 (m, 2H), 1.37 (s, 9H), 1.16 - 1.10 (m, 2H), 0.98 - 0.91 (m, 2H). Step 4: Preparation of intermediate **56f**

2-Chloro-4-fluorobenzonitrile (12.65 g), intermediate **56e** (20.43 g), dimethyl sulfoxide (200 mL), and *N,N-*diisopropylethylamine (21.02 g, 28.4 mL) were added to a reaction flask in sequence, and the mixture was heated to 100 °C and reacted. After the reaction was completed, ethyl acetate (500 mL) and water (1000 mL) were added for extraction, the organic phase was separated, and the aqueous phase was extracted with ethyl acetate. The organic phases were combined, washed with water and saturated brine, dried over anhydrous sodium sulfate, and filtered, and the filtrate was concentrated by evaporation under reduced pressure to remove the solvent. The residue was slurried with ethyl acetate and filtered, and the filter cake was collected to give intermediate **56f** (18.37 g).

MS(ESI, [M+H]⁺) m/z: 364.2.

¹H NMR (500 MHz, DMSO-d6) δ 7.58 (d, J = 9.0 Hz, 1H), 6.93 (d, J = 2.5 Hz, 1H), 6.83 - 6.77 (m, 2H), 3.71 (tt, J = 10.3, 5.2 Hz, 1H), 3.30 - 3.19 (m, 1H), 2.81 (s, 3H), 1.87 - 1.78 (m, 2H), 1.66 - 1.57 (m, 4H), 1.39 (s, 9H), 1.34 (t, J = 5.9 Hz, 2H).

### Step 5: Preparation of intermediate 56g

The target intermediate **56f** (19.5 g), 1,4-dioxane (150 mL), and a solution of hydrochloric acid in 1,4-dioxane (4 mol/L, 201 mL) were added to a reaction flask in sequence, and the mixture was reacted at room temperature. After the reaction was completed, the reaction solution was concentrated by evaporation under reduced pressure to remove the solvent. The residue was slurried with ethyl acetate and filtered, and the filter cake was collected to give intermediate **56g** (15.75 g).

MS(ESI, [M+H]⁺) m/z: 264.2.

¹H NMR (500 MHz, DMSO-d6) δ 8.26 (d, J = 5.4 Hz, 3H), 7.59 (d, J = 8.9 Hz, 1H), 6.97 (d, J = 2.5 Hz, 1H), 6.84 (dd, J = 9.1, 2.5 Hz, 1H), 3.77 (tt, J = 10.3, 5.3 Hz, 1H), 3.02 (dt, J = 11.9, 6.0 Hz, 1H), 2.81 (s, 3H), 2.07 - 2.00 (m, 2H), 1.68 (td, J = 10.7, 9.6, 3.2 Hz, 4H), 1.58 (tt, J = 12.0, 5.9 Hz, 2H).

### Step 6: Preparation of intermediate 56i

Intermediate **56h** (10 g), 4-hydroxymethylpiperidine (8.97 g), *N*,*N*-diisopropylethylamine (16.77 g, 22.66 mL), and dimethyl sulfoxide (100 mL) were added to a reaction flask in sequence, and the mixture was heated to 90 °C and reacted. After the reaction was completed, ethyl acetate (300 mL) and water (1000 mL) were added for extraction, the organic phase was separated, and the aqueous phase was extracted with ethyl acetate. The organic phases were combined, washed with saturated brine, dried over anhydrous sodium sulfate, and filtered, and the filtrate was concentrated by evaporation under reduced pressure to remove the solvent. The residue was slurried with petroleum ether/ethyl acetate and filtered, and the filter cake was collected to give intermediate **56i** (10.03 g).

MS(ESI, [M+H]⁺) m/z: 250.2.

¹H NMR (500 MHz, DMSO-d6) δ 7.76 (d, J = 9.1 Hz, 2H), 6.95 (d, J = 9.0 Hz, 2H), 4.49 (t, J = 5.3 Hz, 1H), 3.92 (dd, J = 12.8, 3.2 Hz, 2H), 3.76 (s, 3H), 3.29 - 3.24 (m, 2H), 2.81 (td, J = 12.6, 2.7 Hz, 2H), 1.76 - 1.69 (m, 2H), 1.61 (dddd, J = 17.8, 11.5, 6.4, 3.3 Hz, 1H), 1.22 - 1.12 (m, 2H).

### Step 7: Preparation of intermediate 56j

Intermediate **56i** (9.9 g), methanol (100 mL), sodium hydroxide (7.94 g), and water (5 mL) were added to a reaction flask in sequence, and the mixture was heated to 60 °C and reacted. After the reaction was completed, concentrated hydrochloric acid was added to adjust the pH to 2-3. The mixture was concentrated by evaporation under reduced pressure to remove the solvent, the residue was slurried with water and filtered, and the filter cake was collected to give intermediate **56j** (6.01 g).

MS(ESI, [M+H]⁺) m/z: 236.1.

¹H NMR (500 MHz, DMSO-d6) δ 7.87 (d, J = 8.4 Hz, 2H), 7.34 (s, 2H), 3.82 - 3.74 (m, 2H), 3.30 (d, J = 6.3 Hz, 2H), 3.09 (s, 2H), 1.84 - 1.77 (m, 2H), 1.68 (td, J = 9.6, 7.8, 4.0 Hz, 1H), 1.50 - 1.36 (m, 2H).

### Step 8: Preparation of intermediate 56k

Intermediate **56g** (1.13 g), intermediate **56j** (0.927 g), *N,N*-diisopropylethylamine (2.55 g, 3.44 mL), dichloromethane (50 mL), and HATU (1.948 g) were added to a reaction flask in sequence, and the mixture was reacted at room temperature. After the reaction was completed, the reaction solution was concentrated by evaporation under reduced pressure to remove the solvent, and the residue was extracted with ethyl acetate (200 mL) and water (200 mL). The organic phase was separated, and the aqueous phase was extracted with ethyl acetate. The organic phases were combined, washed with saturated brine, dried over anhydrous sodium sulfate, and filtered, and the filtrate was purified by a silica gel column to give intermediate **56k** (1.34 g).

MS(ESI, [M+H]⁺) m/z: 481.3.

¹H NMR (500 MHz, DMSO-d6) δ 7.92 (d, J = 7.6 Hz, 1H), 7.74 - 7.70 (m, 2H), 7.60 (d, J = 9.0 Hz, 1H), 6.95 - 6.91 (m, 3H), 6.83 (dd, J = 9.1, 2.6 Hz, 1H), 4.48 (t, J = 5.3 Hz, 1H), 3.85 (dd, J = 12.8, 3.4 Hz, 2H), 3.77 (ddt, J = 13.7, 10.8, 5.0 Hz, 2H), 3.27 (t, J = 5.8 Hz, 2H), 2.85 (s, 3H), 2.73 (td, J = 12.5, 2.6 Hz, 2H), 1.91 (dd, J = 13.3, 3.9 Hz, 2H), 1.70 (ddt, J = 27.1, 9.6, 4.3 Hz, 7H), 1.56 (tt, J = 12.0, 3.8 Hz, 3H).

### Step 9: Preparation of intermediate 56l

Intermediate **56k** (1 g), dichloromethane (40 mL), and Dess-Martin periodinane (1.763 g) were added to a reaction flask in sequence, and the mixture was reacted at room temperature. After the reaction was completed, dichloromethane (100 mL) and water (200 mL) were added for extraction. The organic phase was separated, and the aqueous phase was extracted with dichloromethane. The organic phases were combined, washed with saturated brine, dried over anhydrous sodium sulfate, and filtered, and the filtrate was purified by a silica gel column to give intermediate **56l** (1.05 g).

MS(ESI, [M+H]⁺) m/z: 479.3.

### Step 10: Preparation of compound 56

Intermediate **3** (100 mg), intermediate **56l** (149 mg), sodium acetate (25.5 mg), 1,2-dichloroethane (5 mL), isopropanol (1 mL), and sodium cyanoborohydride (39.1 mg) were added to a reaction flask in sequence, and the mixture was stirred at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, and filtered, and the filtrate was purified by a silica gel column to give compound **56** (81 mg).

MS(ESI, [M+H]⁺) m/z: 748.2.

¹H NMR (500 MHz, DMSO-d6) δ 11.09 (s, 1H), 7.92 (d, J = 7.6 Hz, 1H), 7.73 (d, J = 8.4 Hz, 2H), 7.60 (d, J = 8.6 Hz, 2H), 7.15 (d, J = 8.2 Hz, 1H), 6.99 - 6.88 (m, 3H), 6.83 (d, J = 9.1 Hz, 1H), 4.56 (dd, J = 11.9, 5.0 Hz, 1H), 3.90 - 3.72 (m, 6H), 2.96 (t, J = 5.5 Hz, 2H), 2.89 - 2.72 (m, 8H), 2.60 (dt, J = 17.7, 4.4 Hz, 1H), 2.49 (s, 1H), 2.44 (d, J = 7.3 Hz, 2H), 2.22 - 2.14 (m, 1H), 1.91 (d, J = 11.8 Hz, 3H), 1.83 (d, J = 12.7 Hz, 2H), 1.70 (q, J = 13.5, 12.6 Hz, 4H), 1.61 - 1.50 (m, 2H), 1.23 (q, J = 12.6, 11.9 Hz, 2H).

### Example 57: Synthesis of Compound 57

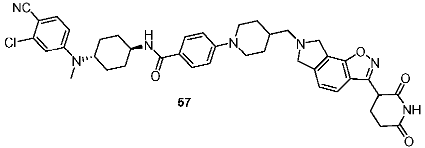

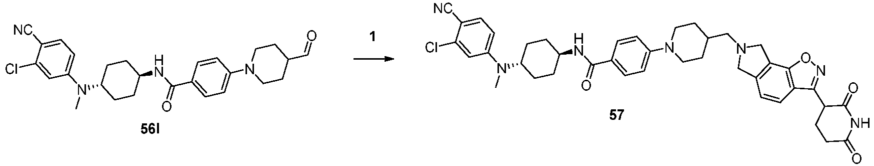

### Step 1: Preparation of compound 57

**56l** (120 mg), intermediate 1 (82 mg), sodium acetate (35 mg), and DCE/isopropanol (5:1, 30 mL) were added to a reaction flask in sequence, and the mixture was reacted at room temperature for 30 min. Sodium cyanoborohydride (33 mg) was then added, and the mixture was reacted at room temperature for 2 h. The reaction solution was concentrated by rotary evaporation to remove the solvent, and the crude product was separated and purified by silica gel column chromatography to give compound **57** (48 mg).

MS(ESI, [M+H]⁺) *m*/*z*: 734.2.

¹H NMR (500 MHz, DMSO-*d*₆) δ 11.10 (s, 1H), 7.93 (d, *J* = 7.7 Hz, 1H), 7.72 (t, *J* = 8.2 Hz, 3H), 7.60 (d, *J =* 9.0 Hz, 1H), 7.31 (d, *J* = 8.1 Hz, 1H), 6.94 (dd, *J =* 5.9, 3.1 Hz, 3H), 6.83 (dd, *J =* 9.1, 2.5 Hz, 1H), 4.60 (dd, *J* = 11.9, 5.0 Hz, 1H), 4.15 (s, 2H), 4.05 (s, 2H), 3.94 - 3.70 (m, 4H), 2.87 - 2.72 (m, 6H), 2.68 - 2.57 (m, 3H), 2.55 (d, *J =* 4.5 Hz, 1H), 2.20 (dt, *J=* 13.5, 4.8 Hz, 1H), 1.96 - 1.85 (m, 4H), 1.83 - 1.63 (m, 5H), 1.61 - 1.49 (m, 2H), 1.31 - 1.19 (m, 2H).

### Example 58: Synthesis of Compound 58

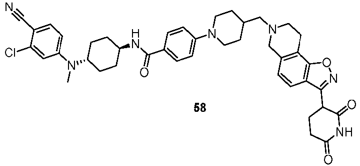

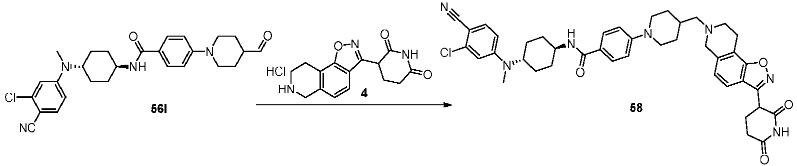

Intermediate **4** (100 mg), intermediate **56l** (149 mg), sodium acetate (25.5 mg), 1,2-dichloroethane (5 mL), isopropanol (1 mL), and sodium cyanoborohydride (39.1 mg) were added to a reaction flask in sequence, and the mixture was stirred at room temperature for 1 h. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, and filtered, and the filtrate was purified by a silica gel column to give compound **58** (71 mg).

MS(ESI, [M+H]⁺) m/z: 748.2.

¹H NMR (500 MHz, DMSO-d6) δ 11.09 (s, 1H), 7.93 (d, J = 7.6 Hz, 1H), 7.73 (d, J = 8.4 Hz, 2H), 7.59 (t, J = 8.9 Hz, 2H), 7.11 (d, J = 8.3 Hz, 1H), 6.98 - 6.90 (m, 3H), 6.86 - 6.80 (m, 1H), 4.56 (dd, J = 11.8, 5.0 Hz, 1H), 3.85 (d, J = 12.5 Hz, 2H), 3.81 - 3.74 (m, 2H), 3.71 (s, 2H), 3.00 (t, J = 5.6 Hz, 2H), 2.84 (s, 3H), 2.78 (q, J = 9.6, 7.4 Hz, 5H), 2.61 (dt, J = 17.5, 4.3 Hz, 1H), 2.45 (d, J = 4.0 Hz, 1H), 2.39 (d, J = 6.9 Hz, 2H), 2.19 (dq, J = 13.7, 4.8 Hz, 1H), 1.91 (d, J = 13.9 Hz, 3H), 1.83 (d, J = 13.4 Hz, 2H), 1.69 (q, J = 13.7, 12.7 Hz, 4H), 1.59 - 1.51 (m, 2H), 1.23 (t, J = 12.2 Hz, 2H).

### Example 59: Synthesis of Compound 59

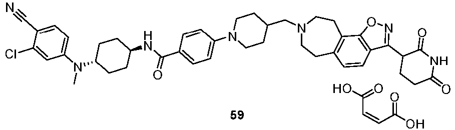

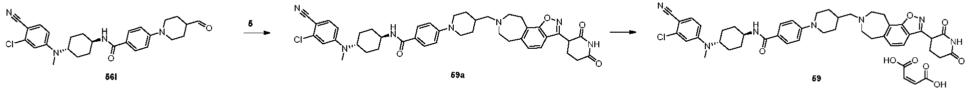

### Step 1: Preparation of intermediate 59a

**56l** (110 mg), intermediate **5** (82 mg), sodium acetate (35 mg), and DCE/isopropanol (5:1, 30 mL) were added to a reaction flask in sequence, and the mixture was reacted at room temperature for 30 min. Sodium cyanoborohydride (33 mg) was then added, and the mixture was reacted at room temperature for 2 h. The reaction solution was concentrated by rotary evaporation to remove the solvent, and the crude product was separated and purified by silica gel column chromatography to give compound **59a** (55 mg).

MS(ESI, [M+H]⁺) *m*/*z*: 762.2.

### Step 2: Preparation of compound 59

**59a** (55 mg), maleic acid (18 mg), and methanol (5 mL) were added to a reaction flask in sequence, and the mixture was reacted at room temperature for 30 min. After the reaction was completed, the reaction solution was concentrated by rotary evaporation to remove the solvent, slurried with petroleum ether, and filtered, and the filter cake was collected and dried to give compound **59** (65 mg).

¹H NMR (500 MHz, DMSO-*d*₆) δ 11.09 (s, 1H), 7.93 (d, *J* = 7.7 Hz, 1H), 7.73 (d, *J =* 8.5 Hz, 2H), 7.57 (dd, *J* = 29.4, 8.5 Hz, 2H), 7.19 (d, *J =* 8.2 Hz, 1H), 7.01 - 6.89 (m, 3H), 6.83 (dd, *J=* 9.1, 2.6 Hz, 1H), 4.55 (dd, *J* = 11.9, 5.0 Hz, 1H), 3.91 - 3.71 (m, 4H), 3.22 - 3.00 (m, 4H), 2.85 (s, 3H), 2.82 - 2.72 (m, 3H), 2.66 (s, 2H), 2.65 - 2.56 (m, 3H), 2.46 (d, *J* = 4.6 Hz, 1H), 2.34 (d, *J =* 6.5 Hz, 2H), 2.18 (dt, *J =* 13.6, 4.3 Hz, 1H), 1.96 - 1.88 (m, 2H), 1.87 - 1.63 (m, 7H), 1.61 - 1.50 (m, 2H), 1.21 (q, *J =* 11.9, 10.9 Hz, 2H).

### Example 60: Synthesis of Compound 60

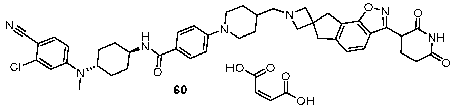

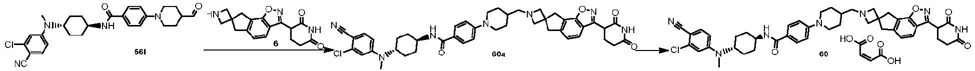

### Step 1: Preparation of intermediate 60a

Intermediate **6** (142 mg), intermediate **56l** (160 mg), 1,2-dichloroethane (5 mL), isopropanol (1 mL), and sodium cyanoborohydride (42.0 mg) were added to a reaction flask in sequence, and the mixture was stirred at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, and filtered, and the filtrate was purified by a silica gel column to give intermediate **60a** (46 mg).

MS(ESI, [M+H]⁺) m/z: 774.4.

### Step 2: Preparation of compound 60

Intermediate **60a** (46 mg), dichloromethane (10 mL), methanol (5 mL), and maleic acid (6.90 mg) were added to a reaction flask in sequence, and the mixture was reacted at room temperature for 0.5 h. After the reaction was completed, the mixture was concentrated by evaporation under reduced pressure to remove the solvent. The residue was slurried with petroleum ether and filtered, and the filter cake was collected to give compound **60** (35 mg).

MS(ESI, [M+H]⁺) m/z: 774.4.

¹H NMR (500 MHz, DMSO-d6) δ 11.09 (s, 1H), 9.76 (s, 1H), 7.94 (d, J = 7.7 Hz, 1H), 7.74 (d, J = 8.4 Hz, 2H), 7.68 (d, J = 8.1 Hz, 1H), 7.60 (d, J = 9.0 Hz, 1H), 7.36 - 7.26 (m, 1H), 6.95 (d, J = 7.5 Hz, 3H), 6.83 (d, J = 9.0 Hz, 1H), 6.08 (s, 2H), 4.58 (d, J = 6.4 Hz, 1H), 4.35 - 4.17 (m, 4H), 3.87 (d, J = 12.4 Hz, 2H), 3.77 (s, 2H), 3.53 (d, J = 44.3 Hz, 4H), 3.21 (s, 4H), 2.85 (s, 3H), 2.76 (t, J = 11.7 Hz, 3H), 2.61 (d, J = 18.3 Hz, 1H), 2.17 (d, J = 11.0 Hz, 1H), 1.91 (d, J = 11.8 Hz, 2H), 1.79 - 1.65 (m, 6H), 1.56 (t, J = 12.4 Hz, 2H), 1.28 (q, J = 15.4, 13.8 Hz, 2H).

### Example 61: Synthesis of Compound 61

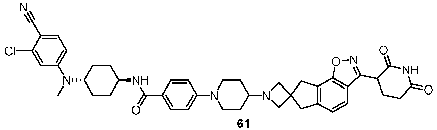

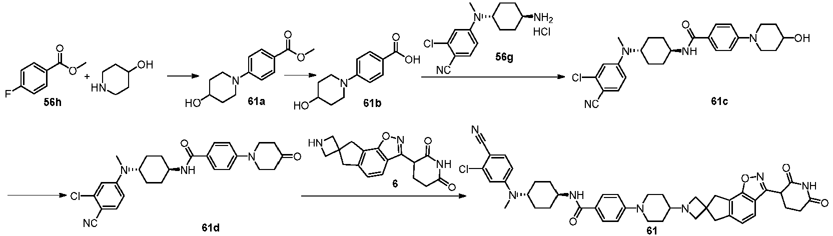

### Step 1: Preparation of intermediate 61a

Intermediate **56h** (10 g), 4-hydroxypiperidine (7.87 g), *N,N*-diisopropylethylamine (25.2 g, 34.0 mL), and dimethyl sulfoxide (100 mL) were added to a reaction flask in sequence, and the mixture was heated to 100 °C and reacted overnight. After the reaction was completed, ethyl acetate (300 mL) and water (1000 mL) were added for extraction, the organic phase was separated, and the aqueous phase was extracted with ethyl acetate. The organic phases were combined, washed with saturated brine, dried over anhydrous sodium sulfate, and filtered, and the filtrate was concentrated by evaporation under reduced pressure to remove the solvent. The residue was slurried with petroleum ether/ethyl acetate and filtered, and the filter cake was collected to give intermediate **61a** (9.47 g).

MS(ESI, [M+H]⁺) m/z: 236.1.

¹H NMR (500 MHz, DMSO-d6) δ 7.76 (d, J = 9.0 Hz, 2H), 6.96 (d, J = 9.0 Hz, 2H), 4.72 (d, J = 4.2 Hz, 1H), 3.77 (s, 3H), 3.74 - 3.67 (m, 3H), 3.04 (ddd, J = 13.1, 9.9, 3.2 Hz, 2H), 1.83 - 1.76 (m, 2H), 1.45 - 1.36 (m, 2H).

### Step 2: Preparation of intermediate 61b

Intermediate **61a** (9.47 g), methanol (200 mL), sodium hydroxide (8.05 g), and water (5 mL) were added to a reaction flask in sequence, and the mixture was heated to 60 °C and reacted. After the reaction was completed, concentrated hydrochloric acid was added to adjust the pH to 2-3. The mixture was concentrated by evaporation under reduced pressure to remove the solvent, the residue was slurried with water and filtered, and the filter cake was collected to give intermediate **61b** (10.04 g).

MS(ESI, [M+H]⁺) m/z: 222.1.

¹H NMR (500 MHz, DMSO-d6) δ 7.88 (d, J = 8.4 Hz, 2H), 7.37 (s, 2H), 3.78 (tt, J = 8.1, 3.7 Hz, 1H), 3.68 (dt, J = 11.5, 4.6 Hz, 2H), 3.22 (t, J = 10.8 Hz, 2H), 2.09 - 1.84 (m, 2H), 1.64 (q, J = 10.2 Hz, 2H).

### Step 3: Preparation of intermediate 61c

Intermediate **56g** (1.5 g), intermediate **61b** (1.258 g), *N,N*-diisopropylethylamine (3.12 g, 4.22 mL), dichloromethane (50 mL), and HATU (2.389 g) were added to a reaction flask in sequence, and the mixture was reacted at room temperature. After the reaction was completed, the reaction solution was concentrated by evaporation under reduced pressure to remove the solvent, and the residue was extracted with ethyl acetate (200 mL) and water (200 mL). The organic phase was separated, and the aqueous phase was extracted with ethyl acetate. The organic phases were combined, washed with saturated brine, dried over anhydrous sodium sulfate, and filtered, and the filtrate was purified by a silica gel column to give intermediate **61c** (1.39 g).

MS(ESI, [M+H]⁺) m/z: 467.3.

¹H NMR (500 MHz, DMSO-d6) δ 7.93 (d, J = 7.6 Hz, 1H), 7.72 (d, J = 8.6 Hz, 2H), 7.60 (d, J = 8.9 Hz, 1H), 6.96 - 6.90 (m, 3H), 6.83 (dd, J = 9.0, 2.5 Hz, 1H), 4.70 (d, J = 4.2 Hz, 1H), 3.77 (tt, J = 11.1, 5.2 Hz, 2H), 3.66 (ddt, J = 12.1, 7.9, 4.5 Hz, 3H), 2.95 (ddd, J = 13.2, 10.1, 3.1 Hz, 2H), 2.85 (s, 3H), 1.94 - 1.88 (m, 2H), 1.80 (dq, J = 12.7, 4.0 Hz, 2H), 1.69 (dtd, J = 17.4, 10.3, 8.8, 3.5 Hz, 4H), 1.55 (qd, J = 12.5, 3.7 Hz, 2H), 1.42 (dtd, J = 12.9, 9.4, 3.7 Hz, 2H).

### Step 4: Preparation of intermediate 61d

Intermediate **61c** (600 mg), dimethyl sulfoxide (10 mL), and 2-iodoxybenzoic acid (720 mg) were added to a reaction flask in sequence, and the mixture was reacted at room temperature for 1 h. After the reaction was completed, ethyl acetate (100 mL) and water (200 mL) were added for extraction, the organic phase was separated, and the aqueous phase was extracted with ethyl acetate. The organic phases were combined, washed with saturated brine, dried over anhydrous sodium sulfate, and filtered, and the filtrate was concentrated by evaporation under reduced pressure to remove the solvent, thus giving intermediate **61d** (650 mg).

MS(ESI, [M+H]⁺) m/z: 465.2.

### Step 5: Preparation of compound 61

Intermediate 6 (120 mg), intermediate **61d** (131 mg), 1,2-dichloroethane (5 mL), isopropanol (1 mL), and sodium cyanoborohydride (53.2 mg) were added to a reaction flask in sequence, and the mixture was stirred at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, and the aqueous phase was extracted with dichloromethane. The organic phases were combined, washed with saturated brine, dried over anhydrous sodium sulfate, and filtered, and the filtrate was purified by a silica gel column to give compound **61** (68 mg).

MS(ESI, [M+H]⁺) m/z: 760.2.

¹H NMR (500 MHz, DMSO-d6) δ 11.09 (s, 1H), 7.93 (d, J = 7.6 Hz, 1H), 7.73 (d, J = 8.2 Hz, 2H), 7.67 - 7.56 (m, 2H), 7.26 (d, J = 8.1 Hz, 1H), 6.93 (d, J = 10.5 Hz, 3H), 6.83 (d, J = 9.1 Hz, 1H), 4.56 (dd, J = 11.7, 5.1 Hz, 1H), 3.75 (dd, J = 32.0, 12.5 Hz, 4H), 3.21 (d, J = 28.6 Hz, 8H), 2.86 (d, J = 10.7 Hz, 5H), 2.80 - 2.72 (m, 1H), 2.60 (d, J = 17.5 Hz, 1H), 2.43 (s, 1H), 2.29 - 2.14 (m, 2H), 1.91 (d, J = 11.8 Hz, 2H), 1.79 - 1.62 (m, 6H), 1.56 (t, J = 12.6 Hz, 2H), 1.25 (q, J = 10.6, 9.9 Hz, 2H).

### Example 62: Synthesis of Compound 62

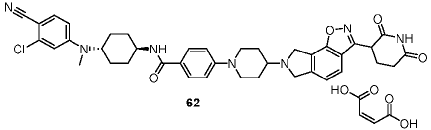

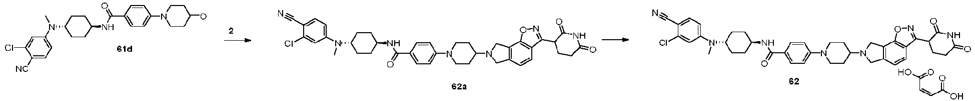

### Step 1: Preparation of intermediate 62a

**56l** (118 mg), intermediate **2** (72 mg), sodium acetate (36 mg), and DCE/isopropanol (5:1, 30 mL) were added to a reaction flask in sequence, and the mixture was reacted at room temperature for 30 min. Sodium cyanoborohydride (38 mg) was then added, and the mixture was reacted at room temperature for 2 h. The reaction solution was concentrated by rotary evaporation to remove the solvent, and the crude product was separated and purified by silica gel column chromatography to give intermediate **62a** (46 mg).

MS(ESI, [M+H]⁺) *m*/*z:* 720.4.

### Step 2: Preparation of compound 62

**62a** (46 mg), maleic acid (13 mg), and methanol (5 mL) were added to a reaction flask in sequence, the mixture was reacted at room temperature for 30 min, then concentrated by rotary evaporation to remove the solvent, slurried with petroleum ether (5 mL), and filtered, and the filter cake was collected and dried to give compound **62** (51 mg). ¹H NMR (500 MHz, DMSO-*d*₆) δ 11.13 (s, 1H), 7.97 (d, *J =* 7.7 Hz, 1H), 7.86 (d, *J =* 8.1 Hz, 1H), 7.77 (d, *J =* 8.5 Hz, 2H), 7.60 (d, *J* = 9.0 Hz, 1H), 7.41 (d, *J* = 8.2 Hz, 1H), 7.01 (d, *J =* 8.5 Hz, 2H), 6.95 (d, *J =* 2.4 Hz, 1H), 6.83 (dd, *J* = 9.1, 2.5 Hz, 1H), 6.07 (s, 2H), 4.65 (dd, *J* = 12.1, 4.9 Hz, 5H), 3.97 (s, 2H), 3.77 (dtd, *J* = 11.4, 7.7, 3.9 Hz, 2H), 2.90 (t, *J =* 12.2 Hz, 2H), 2.85 (s, 3H), 2.79 (ddd, *J =* 17.4, 12.1, 5.3 Hz, 1H), 2.63 (dt, *J =* 17.4, 4.2 Hz, 1H), 2.55 (dd, *J =* 12.6, 4.4 Hz, 2H), 2.27 - 2.11 (m, 3H), 1.96 - 1.88 (m, 2H), 1.79 - 1.62 (m, 6H), 1.55 (qd, *J =* 12.6, 3.7 Hz, 2H).

### Examples 63 and 64: Synthesis of Compounds 63 and 64

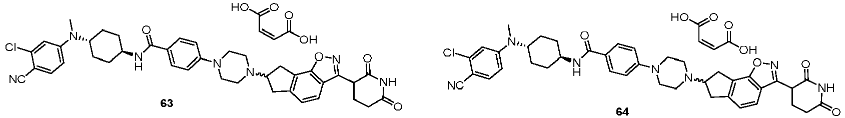

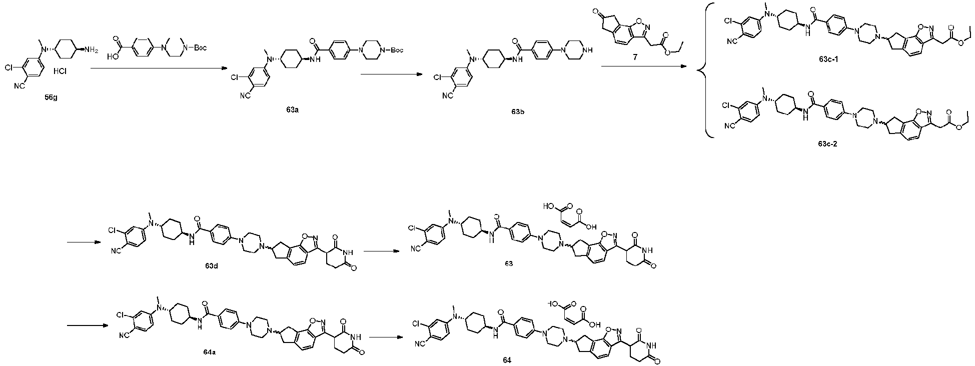

### Step 1: Preparation of intermediate 63a

56g (1.5 g), *tert*-butyl 4-(4-carboxyphenyl)piperazine-1-carboxylate (1.5 g), DCM (10 mL), HATU (2.85 g), and DIPEA (3.5 mL) were added to a reaction flask in sequence, and the mixture was reacted at room temperature. After the reaction was completed, the system was diluted with ethyl acetate, washed with a 10% citric acid aqueous solution, and then washed with a saturated sodium bicarbonate solution. The organic phase was dried over anhydrous sodium sulfate, filtered, and concentrated to give **63a** (2.66 g).

MS(ESI, [M+H]⁺) m/z: 552.51.

### Step 2: Preparation of intermediate 63b

**63a** (2.6 g), DCM (20 mL), and trifluoroacetic acid (5 mL) were added to a reaction flask in sequence, and the mixture was reacted at room temperature. After the reaction was completed, the mixture was added to a saturated sodium bicarbonate solution (200 mL) and extracted with DCM. The organic phase was dried over anhydrous sodium sulfate, filtered, and concentrated to give **63b** (1.92 g).

MS(ESI, [M+H]⁺) m/z: 452.13.

### Step 3: Preparation of intermediates 63c-1 and 63c-2

**63b** (400 mg), 1,2-dichloroethane (10 mL), isopropanol (3 mL), intermediate **7** (252 mg), one drop of acetic acid, and sodium cyanoborohydride (167 mg) were added to a reaction flask in sequence, and the mixture was reacted at room temperature overnight. After the reaction was completed, the reaction solution was added with a saturated sodium bicarbonate solution (20 mL) and water (50 mL) and then extracted with dichloromethane. The organic phase was separated, dried over anhydrous sodium sulfate, filtered, and concentrated, the residue was purified by silica gel column chromatography to give a crude product, and the crude product was separated by preparative high performance liquid chromatography to give intermediate prepeak **63c-1** (165 mg) and postpeak **63c-2** (180 mg) in sequence.

The conditions for the preparative chromatography were as follows: Instrument and preparative column: YMC high pressure preparative chromatograph was used, and the preparative column was CHIRALART Amylose-SA. Mobile phase system: ethanol-dichloromethane (10:3)/*n*-hexane, isocratic elution: ethanol-dichloromethane (10:3)/*n-*hexane = 65/35.

The data of **63c-1** were as follows:

MS(ESI, [M+H]⁺) m/z: 695.12.

¹H NMR (500 MHz, DMSO-*d*₆) δ 7.96 (d, *J =* 7.7 Hz, 1H), 7.74 (d, *J* = 8.7 Hz, 2H), 7.61 (t, *J =* 8.7 Hz, 2H), 7.29 (d, *J =* 8.1 Hz, 1H), 7.01 - 6.92 (m, 3H), 6.83 (dd, *J* = 9.0, 2.6 Hz, 1H), 4.22 - 4.09 (m, 4H), 3.77 (dq, *J =* 11.7, 6.0, 5.5 Hz, 2H), 3.43 (p, *J =* 7.7 Hz, 1H), 3.36 (d, *J =* 7.9 Hz, 1H), 3.31 - 3.19 (m, 5H), 3.03 (dt, *J =* 16.9, 9.1 Hz, 2H), 2.85 (s, 3H), 2.62 (p, *J =* 6.2, 5.3 Hz, 4H), 1.91 (d, *J =* 11.5 Hz, 2H), 1.70 (q, *J =* 12.8 Hz, 4H), 1.61 - 1.50 (m, 2H), 1.19 (t, *J =* 7.1 Hz, 3H).

### Step 4: Preparation of intermediate 63d

**63c-1** (165 mg), acrylamide (20 mg), and THF (10 mL) were added to a reaction flask in sequence, a 1 M solution of potassium *tert*-butoxide in tetrahydrofuran (0.19 mL) was added under an ice bath, and the mixture was reacted at 0 °C. After the reaction was completed, the reaction solution was added dropwise to an icy saturated ammonium chloride aqueous solution, and ethyl acetate was added for extraction. The organic phase was separated, dried over anhydrous sodium sulfate, filtered, and concentrated, and the residue was purified by silica gel column chromatography to give compound **63d** (98 mg).

MS(ESI, [M+H]⁺) m/z: 720.13.

### Step 5: Preparation of compound 63

**63d** (98 mg), maleic acid (15.7 mg), and MeOH/DCM (1/10, 5 mL) were added to a reaction flask in sequence, and after complete dissolution, the reaction solution was concentrated. The reaction solution was slurried with petroleum ether and filtered under vacuum to give compound **63** (101 mg).

MS(ESI, [M+H]⁺) m/z: 720.46.

¹H NMR (500 MHz, DMSO-*d*₆) δ 11.10 (d, *J =* 2.1 Hz, 1H), 8.01 (d, *J =* 7.6 Hz, 1H), 7.80 (d, *J =* 8.5 Hz, 2H), 7.73 (d, *J =* 8.0 Hz, 1H), 7.60 (d, *J =* 8.9 Hz, 1H), 7.34 (d, *J =* 8.1 Hz, 1H), 7.04 (d, *J =* 8.5 Hz, 2H), 6.95 (d, *J =* 2.6 Hz, 1H), 6.83 (dd, *J* = 9.0, 2.5 Hz, 1H), 6.13 (s, 3H), 4.60 (ddd, *J =* 12.0, 5.0, 2.7 Hz, 1H), 4.23 (s, 1H), 3.78 (hd, *J =* 8.0, 4.1 Hz, 3H), 3.62 (dd, *J =* 16.5, 8.0 Hz, 2H), 3.56 - 3.35 (m, 9H), 2.85 (s, 3H), 2.77 (ddd, *J =* 12.7, 5.8, 3.1 Hz, 1H), 2.66 - 2.58 (m, 1H), 2.55 (d, *J* = 4.9 Hz, 1H), 2.20 (tq, *J =* 9.0, 4.7, 3.8 Hz, 1H), 1.96 - 1.88 (m, 2H), 1.79 - 1.64 (m, 4H), 1.56 (qd, *J=* 12.4, 3.7 Hz, 2H).

### Step 6: Preparation of intermediate 64a

**63c-2** (180 mg), acrylamide (22 mg), and THF (10 mL) were added to a reaction flask in sequence, a 1 M solution of potassium *tert*-butoxide in tetrahydrofuran (0.2 mL) was added under an ice bath, and the mixture was reacted at 0 °C. After the reaction was completed, the reaction solution was added dropwise to an icy saturated ammonium chloride aqueous solution, and ethyl acetate (50 mL) was added for extraction. The organic phase was separated, dried over anhydrous sodium sulfate, filtered, and concentrated, and the residue was purified by silica gel column chromatography to give compound **64a** (105 mg).

MS(ESI, [M+H]⁺) m/z: 720.46.

### Step 7: Preparation of compound 64

**64a** (105 mg), maleic acid (16.9 mg), and MeOH/DCM (1/10, 5 mL) were added to a reaction flask in sequence, and after complete dissolution, the reaction solution was concentrated. The reaction solution was slurried with petroleum ether and filtered under vacuum to give compound **64** (117 mg).

MS(ESI, [M+H]⁺) m/z: 720.44.

¹H NMR (500 MHz, DMSO-*d*₆) δ 11.10 (d, *J =* 2.1 Hz, 1H), 8.01 (d, *J =* 7.6 Hz, 1H), 7.80 (d, *J =* 8.5 Hz, 2H), 7.73 (d, *J =* 8.0 Hz, 1H), 7.60 (d, *J =* 8.9 Hz, 1H), 7.34 (d, *J =* 8.1 Hz, 1H), 7.04 (d, *J =* 8.5 Hz, 2H), 6.95 (d, *J =* 2.6 Hz, 1H), 6.83 (dd, *J* = 9.0, 2.5 Hz, 1H), 6.13 (s, 3H), 4.60 (ddd, *J =* 12.0, 5.0, 2.7 Hz, 1H), 4.23 (s, 1H), 3.78 (hd, *J =* 8.0, 4.1 Hz, 3H), 3.62 (dd, *J =* 16.5, 8.0 Hz, 2H), 3.56 - 3.35 (m, 9H), 2.85 (s, 3H), 2.77 (ddd, *J =* 12.7, 5.8, 3.1 Hz, 1H), 2.66 - 2.58 (m, 1H), 2.55 (d, *J* = 4.9 Hz, 1H), 2.20 (tq, *J =* 9.0, 4.7, 3.8 Hz, 1H), 1.96 - 1.88 (m, 2H), 1.79 - 1.64 (m, 4H), 1.56 (qd, *J =* 12.4, 3.7 Hz, 2H).

### Examples 65 and 66: Synthesis of Compounds 65 and 66

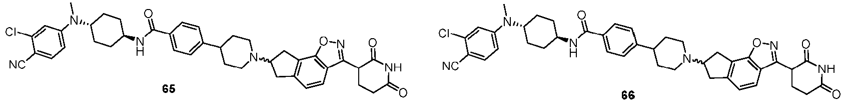

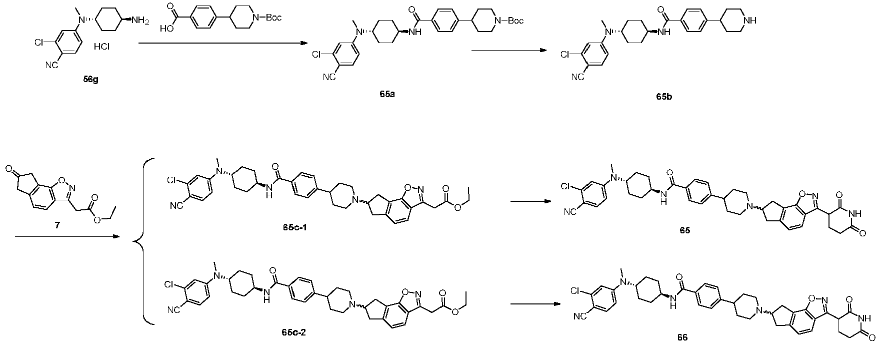

### Step 1: Preparation of intermediate 65a

**56g** (1.5 g), 1-Boc-4-(4'-carboxyphenyl)piperidine (1.5 g), DCM (10 mL), HATU (2.85 g), and DIPEA (3.5 mL) were added to a reaction flask in sequence, and the mixture was reacted at room temperature. After the reaction was completed, the system was diluted with ethyl acetate (100 mL), washed with a 10% citric acid aqueous solution (100 mL), and then washed with a saturated sodium bicarbonate solution. The organic phase was dried over anhydrous sodium sulfate, filtered, and concentrated to give **65a** (3.2 g).

MS(ESI, [M+H]⁺) m/z: 551.4.

### Step 2: Preparation of intermediate 65b

**65a** (3.2 g), DCM (20 mL), and trifluoroacetic acid (5 mL) were added to a reaction flask in sequence, and the mixture was reacted at room temperature. After the reaction was completed, the mixture was added to a saturated sodium bicarbonate solution (200 mL) and extracted with DCM. The organic phase was dried over anhydrous sodium sulfate, filtered, and concentrated to give **65b** (1.85 g).

MS(ESI, [M+H]⁺) m/z: 451.34.

### Step 3: Preparation of intermediates 65c-1 and 65c-2

**65b** (400 mg), 1,2-dichloroethane (10 mL), isopropanol (3 mL), intermediate **7** (252 mg), one drop of acetic acid, and sodium cyanoborohydride (167 mg) were added to a reaction flask in sequence, and the mixture was reacted at room temperature overnight. After the reaction was completed, the reaction solution was added with a saturated sodium bicarbonate solution (20 mL) and water (50 mL) and then extracted with dichloromethane. The organic phase was separated, dried over anhydrous sodium sulfate, filtered, and concentrated, the residue was purified by silica gel column chromatography to give a crude product, and the crude product was separated by preparative high performance liquid chromatography to give intermediate prepeak **65c-1** (150 mg) and postpeak **65c-2** (155 mg) in sequence.

The conditions for the preparative chromatography were as follows: Instrument and preparative column: YMC high pressure preparative chromatograph was used, and the preparative column was CHIRALART Amylose-SA. Mobile phase system: ethanol:dichloromethane (1:3)/n-hexane, isocratic elution: ethanol:dichloromethane (1:3)/n-hexane *=* 50/50.

The data of **65c-1** were as follows:
MS(ESI, [M+H]⁺) m/z: 694.20.

¹H NMR (500 MHz, DMSO-*d*₆) δ 8.18 (d, *J* = 7.6 Hz, 1H), 7.78 (d, *J* = 8.0 Hz, 2H), 7.61 (dd, *J* = 8.6, 6.5 Hz, 2H), 7.31 (dd, *J =* 22.0, 8.1 Hz, 3H), 6.95 (d, *J =* 2.5 Hz, 1H), 6.84 (dd, *J =* 9.1, 2.6 Hz, 1H), 4.22 - 4.06 (m, 4H), 3.89 - 3.72 (m, 2H), 3.44 (d, *J =* 7.0 Hz, 1H), 3.31 - 3.32 (m, 1H), 3.25 - 3.17 (m, 1H), 3.03 (s, 4H), 2.85 (s, 3H), 2.58 (s, 1H), 2.16 (d, *J =* 10.2 Hz, 2H), 1.92 (d, *J =* 12.1 Hz, 2H), 1.85 - 1.62 (m, 8H), 1.61 - 1.48 (m, 2H), 1.19 (t, *J =* 7.1 Hz, 3H).

### Step 4: Preparation of compound 65

**65c-1** (150 mg), acrylamide (16.9 mg), and THF (10 mL) were added to a reaction flask in sequence, a 1 M solution of potassium tert-butoxide in tetrahydrofuran (0.17 mL) was added under an ice bath, and the mixture was reacted at 0 °C. After the reaction was completed, the reaction solution was added dropwise to an icy saturated ammonium chloride aqueous solution, and ethyl acetate was added for extraction. The organic phase was separated, dried over anhydrous sodium sulfate, filtered, and concentrated, and the residue was purified by silica gel column chromatography to give compound **65** (70 mg).

MS(ESI, [M+H]⁺) m/z: 719.19.

¹H NMR (500 MHz, DMSO-*d*₆) δ 11.09 (s, 1H), 8.18 (d, *J* = 7.6 Hz, 1H), 7.78 (d, *J =* 8.2 Hz, 2H), 7.61 (dd, *J* = 15.4, 8.5 Hz, 2H), 7.34 (d, *J* = 8.0 Hz, 2H), 7.27 (d, *J =* 8.1 Hz, 1H), 6.95 (d, *J =* 2.6 Hz, 1H), 6.84 (dd, *J* = 9.1, 2.5 Hz, 1H), 4.57 (dd, *J =* 11.9, 5.0 Hz, 1H), 3.79 (dd, *J =* 11.5, 5.1 Hz, 2H), 3.44 (t, *J* = 7.5 Hz, 1H), 3.31 (s, 1H), 3.22 (dd, *J =* 16.2, 7.6 Hz, 1H), 3.11 - 2.93 (m, 4H), 2.85 (s, 3H), 2.77 (ddd, *J* = 17.2, 12.0, 5.3 Hz, 1H), 2.61 (dt, *J =* 17.0, 4.3 Hz, 2H), 2.47 (d, *J =* 8.1 Hz, 1H), 2.18 (td, *J =* 9.4, 5.4 Hz, 3H), 1.96 - 1.88 (m, 2H), 1.83 - 1.63 (m, 8H), 1.61 - 1.51 (m, 2H).

### Step 5: Preparation of compound 66

**65c-2** (165 mg), acrylamide (16.9 mg), and THF (10 mL) were added to a reaction flask in sequence, a 1 M solution of potassium *tert*-butoxide in tetrahydrofuran (0.19 mL) was added under an ice bath, and the mixture was reacted at 0 °C. After the reaction was completed, the reaction solution was added dropwise to an icy saturated ammonium chloride aqueous solution, and ethyl acetate was added for extraction. The organic phase was separated, dried over anhydrous sodium sulfate, filtered, and concentrated, and the residue was purified by silica gel column chromatography to give compound **66** (65 mg).

MS(ESI, [M+H]⁺) m/z: 719.28.

¹H NMR (500 MHz, DMSO-*d*₆) δ 11.09 (s, 1H), 8.18 (d, *J* = 7.6 Hz, 1H), 7.78 (d, *J =* 8.2 Hz, 2H), 7.61 (dd, *J* = 15.4, 8.5 Hz, 2H), 7.34 (d, *J* = 8.0 Hz, 2H), 7.27 (d, *J =* 8.1 Hz, 1H), 6.95 (d, *J =* 2.6 Hz, 1H), 6.84 (dd, *J* = 9.1, 2.5 Hz, 1H), 4.57 (dd, *J =* 11.9, 5.0 Hz, 1H), 3.79 (dd, *J =* 11.5, 5.1 Hz, 2H), 3.44 (t, *J* = 7.5 Hz, 1H), 3.31 (s, 1H), 3.22 (dd, *J =* 16.2, 7.6 Hz, 1H), 3.11 - 2.93 (m, 4H), 2.85 (s, 3H), 2.77 (ddd, *J* = 17.2, 12.0, 5.3 Hz, 1H), 2.61 (dt, *J =* 17.0, 4.3 Hz, 2H), 2.47 (d, *J =* 8.1 Hz, 1H), 2.18 (td, *J =* 9.4, 5.4 Hz, 3H), 1.96 - 1.88 (m, 2H), 1.83 - 1.63 (m, 8H), 1.61 - 1.51 (m, 2H).

### Example 67: Synthesis of Compound 67

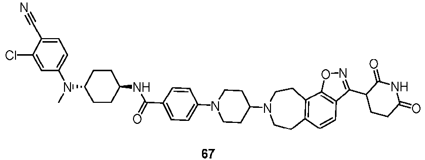

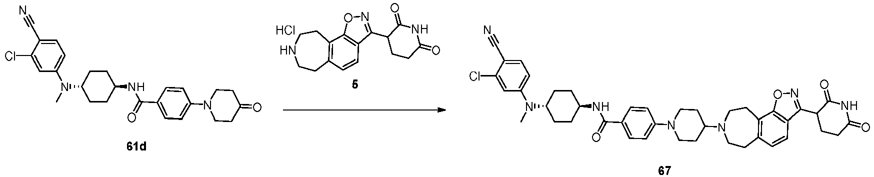

### Step 1: Preparation of compound 67

Intermediate **61d** (415 mg), DMA (10 mL), intermediate **5** (100 mg), sodium acetate (25 mg), and sodium cyanoborohydride (187 mg) were added to a reaction flask in sequence, and the mixture was reacted at 80 °C. After the reaction was completed, a saturated sodium bicarbonate solution (2 mL) was added to the reaction solution to neutralize acetic acid, and then ethyl acetate and water were added for extraction. The organic phase was separated, dried over anhydrous sodium sulfate, filtered, and concentrated, and the residue was purified by a silica gel column to give compound **67** (90 mg).

MS(ESI, [M+H]⁺) m/z: 748.46.

¹H NMR (500 MHz, DMSO-*d*₆) δ 11.08 (s, 1H), 7.94 (s, 1H), 7.72 (d, *J =* 8.6 Hz, 2H), 7.56 (dd, *J =* 32.9, 8.3 Hz, 2H), 7.18 (s, 1H), 6.89 (d, *J =* 51.9 Hz, 4H), 4.67 - 4.39 (m, 1H), 4.05 - 3.62 (m, 4H), 3.14 (s, 2H), 3.02 (s, 2H), 2.77 (t, *J =* 32.1 Hz, 13H), 2.18 (s, 1H), 1.72 (dd, *J* = 105.4, 71.9 Hz, 12H).

### Example 68: Synthesis of Compound 68

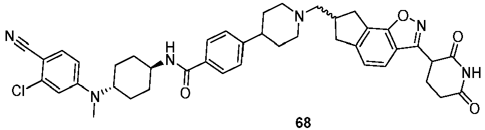

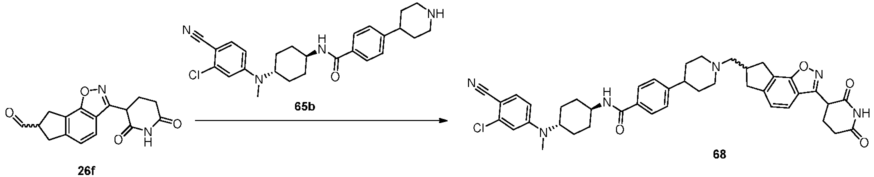

### Step 1: Preparation of compound 68

Intermediate **26f** (90 mg), intermediate **65b** (150 mg), 1,2-dichloroethane (10 mL), isopropanol (1 mL), and sodium triacetoxyborohydride (128 mg) were added to a reaction flask in sequence, and the mixture was reacted at room temperature. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography to give compound **68** (60 mg).

MS(ESI, [M+H]⁺) m/z: 733.39.

¹H NMR (500 MHz, DMSO-*d*6) δ11.09 (d, *J* = 1.3 Hz, 1H), 8.19 (d, *J* = 7.6 Hz, 1H),7.83 - 7.75 (m, 2H), 7.61 (dd, *J =* 8.6, 6.6 Hz, 2H), 7.34 (d, *J =* 8.2 Hz, 2H), 7.27 (d, *J =* 8.1 Hz, 1H), 6.95 (d, *J =* 2.6 Hz, 1H), 6.84 (dd, *J* = 9.1, 2.5 Hz, 1H), 4.57 (ddd, *J* = 11.9, 5.0, 1.7 Hz, 1H), 3.79 (dq, *J =* 11.5, 3.9 Hz, 2H), 3.20 (ddd, *J* = 25.1, 16.6, 8.2 Hz, 2H), 3.02 (d, *J* = 10.6 Hz, 2H), 2.92 (d, *J* = 14.3 Hz, 2H), 2.85 (s, 4H), 2.77 (ddd, *J* = 17.2, 11.9, 5.4 Hz, 1H), 2.68 - 2.52 (m, 3H), 2.37 (d, *J =* 7.2 Hz, 2H),2.25 - 2.13 (m, 1H), 2.04 (t, *J* = 10.9 Hz, 2H), 1.98 - 1.89 (m, 2H), 1.79 - 1.64 (m, 8H), 1.61 - 1.51 (m, 2H).

### Example 69: Synthesis of Compound 69

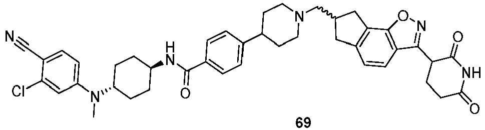

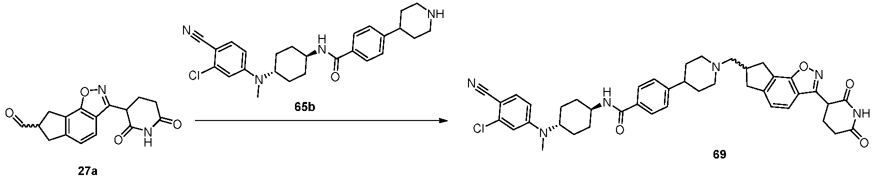

### Step 1: Preparation of compound 69

Intermediate **27a** (90 mg), intermediate **65b** (150 mg), 1,2-dichloroethane (10 mL), isopropanol (1 mL), and sodium triacetoxyborohydride (128 mg) were added to a reaction flask in sequence, and the mixture was reacted at room temperature. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography to give compound 69 (60 mg).

MS(ESI, [M+H]⁺) m/z: 733.39.

¹H NMR (500 MHz, DMSO-*d*6) δ11.09 (d, *J* = 1.3 Hz, 1H), 8.19 (d, *J =* 7.6 Hz, 1H), 7.83 - 7.75 (m, 2H), 7.61 (dd, *J =* 8.6, 6.6 Hz, 2H),7.34 (d, *J =* 8.2 Hz, 2H), 7.27 (d, *J =* 8.1 Hz, 1H), 6.95 (d, *J =* 2.6 Hz, 1H), 6.84 (dd, *J* = 9.1, 2.5 Hz, 1H), 4.57 (ddd, *J* = 11.9, 5.0, 1.7 Hz, 1H), 3.79 (dq, *J =* 11.5, 3.9 Hz, 2H), 3.20 (ddd, *J* = 25.1, 16.6, 8.2 Hz, 2H), 3.02 (d, *J* = 10.6 Hz, 2H), 2.92 (d, *J* = 14.3 Hz, 2H), 2.85 (s, 4H), 2.77 (ddd, *J* = 17.2, 11.9, 5.4 Hz, 1H), 2.68 - 2.52 (m, 3H), 2.37 (d, *J =* 7.2 Hz, 2H), 2.25 - 2.13 (m, 1H), 2.04 (t, *J* = 10.9 Hz, 2H), 1.98 - 1.89 (m, 2H), 1.79 - 1.64 (m, 8H), 1.61 - 1.51 (m, 2H).

### Example 70: Synthesis of Compound 70

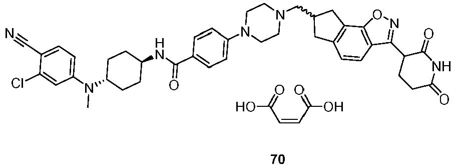

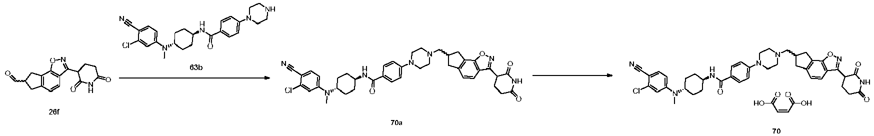

### Step 1: Preparation of intermediate 70a

Intermediate **26f** (90 mg), intermediate **63b** (150 mg), 1,2-dichloroethane (10 mL), isopropanol (1 mL), and sodium triacetoxyborohydride (128 mg) were added to a reaction flask in sequence, and the mixture was reacted at room temperature. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography to give compound **70a** (70 mg).

MS(ESI, [M+H]⁺) m/z: 734.10.

### Step 2: Preparation of compound 70

Intermediate **70a** (70 mg), dichloromethane (10 mL), methanol (1 mL), and maleic acid (11.1 mg) were added to a reaction flask in sequence, and the mixture was reacted at room temperature. After the reaction was completed, the mixture was concentrated by evaporation under reduced pressure to remove the solvent. The residue was slurried with petroleum ether and filtered, and the filter cake was collected to give compound **70** (70 mg).

MS(ESI, [M+H]⁺) m/z: 734.10.

H NMR (500 MHz, DMSO-*d*6) δ11.10 (s, 1H), 8.02 (d, *J =* 7.6 Hz, 1H), 7.80 (d, *J =* 8.4 Hz, 2H), 7.66 (d, *J =* 8.1 Hz, 1H), 7.60 (d, *J* = 9.0 Hz, 1H), 7.31 (d, *J* = 8.1 Hz, 1H), 7.04 (d, *J =* 8.5 Hz, 2H), 6.95 (d, *J =* 2.5 Hz, 1H), 6.83 (dd, *J =* 9.1, 2.5 Hz, 1H), 6.08 (s, 2H), 4.58 (ddd, *J =* 12.0, 5.1, 2.5 Hz, 1H), 3.78 (ddt, *J =* 16.1, 7.6, 4.4 Hz, 3H), 3.40 (s, 3H), 3.34 - 3.20 (m, 8H), 3.14 (d, *J =* 25.2 Hz, 4H), 3.01 (dd, *J =* 15.9, 6.4 Hz, 2H), 2.90 (dd, *J =* 16.3, 6.6 Hz, 1H), 2.78 (ddd, *J =* 17.2, 11.7, 5.1 Hz, 1H), 2.61 (dq, *J =* 17.1, 3.7 Hz, 1H), 2.19 (dp, *J =* 12.9, 4.3 Hz, 1H), 1.99 - 1.87 (m, 2H), 1.80 - 1.63 (m, 4H), 1.56 (qd, *J =* 12.3, 3.7 Hz, 2H).

### Example 71: Synthesis of Compound 71

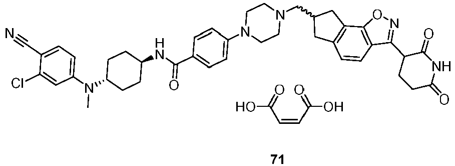

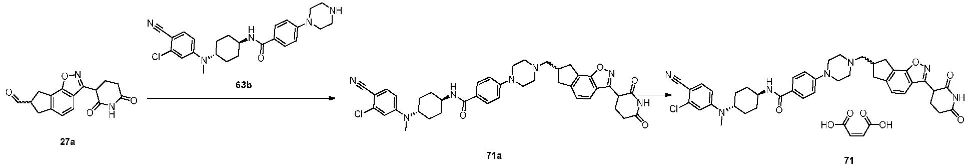

### Step 1: Preparation of intermediate 71a

Intermediate **27a** (90 mg), intermediate **63b** (150 mg), 1,2-dichloroethane (10 mL), isopropanol (1 mL), and sodium triacetoxyborohydride (128 mg) were added to a reaction flask in sequence, and the mixture was reacted at room temperature. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography to give compound **71a** (70 mg).

MS(ESI, [M+H]⁺) m/z: 734.10.

### Step 2: Preparation of compound 71

Intermediate **71a** (70 mg), dichloromethane (10 mL), methanol (1 mL), and maleic acid (11.1 mg) were added to a reaction flask in sequence, and the mixture was reacted at room temperature. After the reaction was completed, the mixture was concentrated by evaporation under reduced pressure to remove the solvent. The residue was slurried with petroleum ether and filtered, and the filter cake was collected to give compound **71** (70 mg).

MS(ESI, [M+H]⁺) m/z: 734.10.

¹H NMR (500 MHz, DMSO-*d*6) δ11.10 (s, 1H), 8.02 (d, *J* = 7.6 Hz, 1H), 7.80 (d, *J =* 8.4 Hz, 2H), 7.66 (d, *J =* 8.1 Hz, 1H), 7.60 (d, *J* = 9.0 Hz, 1H), 7.31 (d, *J* = 8.1 Hz, 1H), 7.04 (d, *J =* 8.5 Hz, 2H), 6.95 (d, *J* = 2.5 Hz, 1H), 6.83 (dd, *J =* 9.1, 2.5 Hz, 1H), 6.10 (s, 2H), 4.58 (ddd, *J =* 12.0, 5.1, 2.5 Hz, 1H), 3.78 (ddt, *J =* 16.1, 7.6, 4.4 Hz, 3H), 3.41 (dd, *J =* 15.9, 7.5 Hz, 3H), 3.37 - 3.23 (m, 8H), 3.14 (d, *J* = 24.2 Hz, 4H), 3.01 (dd, *J =* 15.9, 6.5 Hz, 2H), 2.90 (dd, *J =* 16.3, 6.6 Hz, 1H), 2.78 (ddd, *J =* 17.2, 11.7, 5.3 Hz, 1H), 2.61 (dq, *J =* 17.1, 3.7 Hz, 1H), 2.18 (dp,*J* = 12.9, 4.3 Hz, 1H), 1.96 - 1.88 (m, 2H), 1.79 - 1.64 (m, 4H), 1.56 (qd, *J =* 12.5, 3.6Hz, 2H).

### Example 72: Synthesis of Intermediate 72

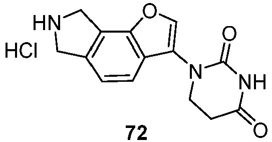

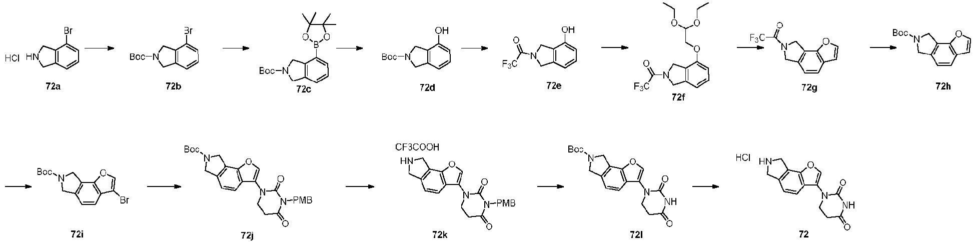

### Step 1: Preparation of intermediate 72b

**72a** (47 g), triethylamine (60.8 g), and dichloromethane (500 mL) were added to a reaction flask in sequence. The mixture was cooled to 0 °C, and Boc anhydride (48.1 g) was added dropwise. The mixture was reacted at room temperature. After the reaction was completed, the reaction solution was washed with a saturated ammonium chloride aqueous solution, the organic phase was separated, washed with saturated brine, dried over anhydrous sodium sulfate, and filtered, and the filtrate was concentrated to give intermediate **72b** (60 g).

### Step 2: Preparation of intermediate 72c

**72b** (60 g), bis(pinacolato)diboron (53.7 g), potassium acetate (38.5 g), dimethyl sulfoxide (600 mL), and 1,1'-bis(diphenylphosphino)ferrocenepalladium dichloride (8.2 g) were added to a reaction flask in sequence, and the mixture was purged three times with nitrogen, warmed to 100 °C, and reacted for 4 h. After the reaction was completed, the mixture was added with water (2.5 L) and extracted with ethyl acetate, washed with saturated brine, dried over anhydrous sodium sulfate, and filtered. The filtrate was concentrated, and the crude product was separated and purified by silica gel column chromatography to give intermediate **72c** (58 g).

MS (ESI, [M+H]⁺) *m*/*z*: 346.20.

### Step 3: Preparation of intermediate 72d

**72c** (55 g), tetrahydrofuran (500 mL), and glacial acetic acid (47.8 g) were added to a reaction flask in sequence. The mixture was cooled to 0 °C, and hydrogen peroxide (36.1 g) was added dropwise. After the dropwise addition, the mixture was reacted at room temperature for 5 h. After the reaction was completed, water (800 mL) was added, and excess hydrogen peroxide was neutralized with sodium thiosulfate. The mixture was extracted with ethyl acetate, washed with saturated brine, dried over anhydrous sodium sulfate, and filtered, and the filtrate was concentrated to give intermediate **72d** (37 g).

MS (ESI, [M-H]⁻) *m*/*z*: 234.08.

### Step 4: Preparation of intermediate 72e

**72d** (35.00 g) and a 4 M solution of hydrochloric acid in 1,4-dioxane (300 mL) were added to a reaction flask in sequence, and the mixture was reacted at room temperature for 3 h. After the reaction was completed, the reaction solution was concentrated and added with dichloromethane (300 mL) and triethylamine (60.5 g). The mixture was cooled to 0 °C, and trifluoroacetic anhydride (33.6 g) was added dropwise. The mixture was reacted at room temperature for 2 h. After the reaction was completed, the reaction solution was washed with a saturated ammonium chloride solution, the organic phase was separated, washed with saturated brine, dried over anhydrous sodium sulfate, and filtered, and the filtrate was concentrated to give intermediate **72e** (25 g).

MS (ESI, [M-H]⁻) *m*/*z*: 230.12.

### Step 5: Preparation of intermediate 72f

**72e** (25 g), bromoacetaldehyde diethyl acetal (46 g), potassium carbonate (32.3 g), and dimethylacetamide (200 mL) were added to a reaction flask in sequence, and the mixture was warmed to 150 °C and reacted. After the reaction was completed, the mixture was added with water (800 mL) and extracted with ethyl acetate, washed with saturated brine, dried over anhydrous sodium sulfate, and filtered. The filtrate was concentrated, and the crude product was separated and purified by silica gel column chromatography to give intermediate **72f** (22 g).

### Step 6: Preparation of intermediate 72g

**72f** (22 g), chlorobenzene (150 mL), and phosphoric acid (31 g) were added to a reaction flask in sequence, and the mixture was reacted at 130 °C. After the reaction was completed, the mixture was added with water (400 mL), extracted with ethyl acetate, washed with saturated brine, dried over anhydrous sodium sulfate, and filtered. The filtrate was concentrated, and the crude product was separated and purified by silica gel column chromatography to give intermediate **72g** (15.2 g).

MS (ESI, [M+H]⁺) *m*/*z*: 256.02.

### Step 7: Preparation of intermediate 72h

**72g** (12 g), sodium hydroxide (5.64 g), methanol (150 mL), and water (35 mL) were added to a reaction flask in sequence, and the mixture was reacted at room temperature for 1 h. After the reaction was completed, di-*tert*-butyl dicarbonate (12.32 g) was added, and the mixture was reacted at room temperature for 2 h. After the reaction was completed, the reaction solution was poured into water, vigorously stirred, extracted with ethyl acetate, washed with saturated brine, dried over anhydrous sodium sulfate, and filtered. The filtrate was concentrated, and the crude product was separated by silica gel column chromatography to give intermediate **72h** (10.4 g).

### Step 8: Preparation of intermediate 72i

**72h** (10.4 g) and DCM (300 mL) were added to a reaction flask in sequence, and the mixture was cooled to 0 °C. Liquid bromine (8.32 g) was added, and the mixture was reacted at room temperature for 0.5 h. After the reaction was completed, the mixture was concentrated by rotary evaporation to remove the solvent. Ethanol (80 mL) and potassium hydroxide (3.89 g) were added to the residue, and the mixture was warmed to 70 °C and reacted for 0.5 h. After the reaction was completed, the reaction solution was poured into water, vigorously stirred, extracted with ethyl acetate, washed with saturated brine, dried over anhydrous sodium sulfate, and filtered. The filtrate was concentrated, and the crude product was separated by silica gel column chromatography to give intermediate **72i** (4.61 g).

MS (ESI, [M+H]⁺) *m*/*z*: 338.01.

### Step 9: Preparation of intermediate 72j

**72i** (4.05 g), 3-(4-methoxybenzyl)dihydropyrimidine-2,4(1*H*,3*H*)-dione (3.32 g), (1*R*,2*R*)-*N*1,*N*2-dimethylcyclohexane-1,2-diamine (0.84 g), copper(I) iodide (1.13 g), cesium carbonate (11.56 g), and 1,4-dioxane (100 mL) were added to a reaction flask in sequence, and the mixture was reacted at 120 °C for 15 h. After the reaction was completed, the reaction solution was concentrated and extracted with water and ethyl acetate, followed by liquid separation. The organic phase was washed with saturated brine, dried over anhydrous sodium sulfate, and filtered. The filtrate was concentrated, and the crude product was separated by silica gel column chromatography to give intermediate **72j** (3.65 g).

MS (ESI, [M+H]⁺) *m*/*z*: 492.01.

### Step 10: Preparation of intermediate 72k

**72j** (3.65 g), dichloromethane (40 mL), and trifluoroacetic acid (20 mL) were added to a reaction flask in sequence, and the mixture was reacted at room temperature for 1 h. After the reaction was completed, the reaction solution was concentrated to give intermediate **72k** (4.68 g).

### Step 11: Preparation of intermediate 721

**72k** (3.65 g) and trifluoromethanesulfonic acid (40 mL) were added to a reaction flask in sequence, and the mixture was reacted at room temperature for 3 h. After the reaction was completed, the reaction solution was added dropwise to a solution of DIPEA (66.3 g, 90 mL) in DCM (90 mL). After the dropwise addition, the mixture was warmed to room temperature. Di-*tert*-butyl dicarbonate (2.56 g, 2.73 mL) was added, and the mixture was reacted at room temperature. After the reaction was completed, the reaction solution was concentrated by evaporation under reduced pressure to remove the solvent and extracted with ethyl acetate (200 mL) and water (500 mL). The organic phase was separated, washed with saturated brine, dried over anhydrous sodium sulfate, and filtered, and the filtrate was purified by silica gel column chromatography to give the target intermediate **721** (1.1 g).

MS (ESI, [M+H]⁺) *m*/*z*: 372.01.

### Step 12: Preparation of intermediate 72

**72l** (1.1 g) and DCM (5 mL) were added to a reaction flask in sequence, a solution of hydrochloric acid in 1,4-dioxane (4 mol/L, 16.35 mL) was added, and the mixture was reacted at room temperature. After the reaction was completed, the reaction solution was concentrated to give intermediate **72** (0.58 g).

MS (ESI, [M+H]⁺) *m*/*z*: 272.08.

¹H NMR (500 MHz, DMSO-*d*6) δ 10.56 (s, 1H), 10.18 (s, 2H), 8.21 (s, 1H), 7.63 (d, *J =* 8.1 Hz, 1H), 7.33 (d, *J* = 8.1 Hz, 1H), 4.77 *(d, J =* 4.6 Hz, 2H), 4.63 (d, *J =* 4.5 Hz, 2H), 3.84 (t, *J =* 6.7 Hz, 2H), 2.79 (t, *J =* 6.7 Hz, 2H).

### Example 73: Synthesis of Intermediate 73

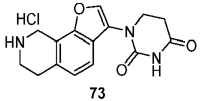

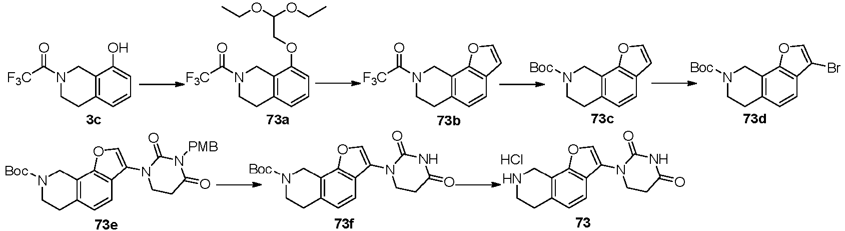

### Step 1: Preparation of intermediate 73a

Intermediate **3c** (30 g), bromoacetaldehyde diethyl acetal (48.2 g), potassium carbonate (33.8 g), and DMA (300 mL) were added to a reaction flask in sequence, and the mixture was heated to 150 °C and reacted. After the reaction was completed, the reaction solution was extracted with ethyl acetate (500 mL) and water (1000 mL). The organic phase was separated, washed with saturated brine, dried over anhydrous sodium sulfate, and filtered, and the filtrate was purified by silica gel column chromatography to give the target intermediate **73a** (31.48 g).

### Step 2: Preparation of intermediate 73b

Intermediate **73a** (32.48 g), phosphoric acid (51.8 g), and chlorobenzene (100 mL) were added to a reaction flask in sequence, and the mixture was heated to 120 °C and reacted. After the reaction was completed, the reaction solution was extracted with ethyl acetate (500 mL) and water (1000 mL). The organic phase was separated, washed with saturated brine, dried over anhydrous sodium sulfate, and filtered, and the filtrate was concentrated by evaporation under reduced pressure to remove the solvent, thus giving the target intermediate **73b** (20.58 g).

### Step 3: Preparation of intermediate 73c

Intermediate **73b** (20.58 g) and methanol (150 mL) were added to a reaction flask in sequence, a solution of sodium hydroxide (8.23 g) in water (150 mL) was added dropwise under an ice bath, and the mixture was warmed to room temperature and reacted. After the reaction was completed, the reaction solution was concentrated by evaporation under reduced pressure to remove the methanol, and 1,4-dioxane (150 mL) and di-*tert*-butyl dicarbonate (16.46 g, 17.51 mL) were added. The mixture was reacted at room temperature. After the reaction was completed, the reaction solution was extracted with ethyl acetate (500 mL) and water (800 mL), dried over anhydrous sodium sulfate, and filtered, and the filtrate was concentrated by evaporation under reduced pressure to remove the solvent, thus giving the target intermediate **73c** (26.48 g).

### Step 4: Preparation of intermediate 73d

Intermediate **73c** (26.34 g), DCM (200 mL), and liquid bromine (12.94 g, 4.15 mL) were added to a reaction flask in sequence, and the mixture was reacted at room temperature. After the reaction was completed, the reaction solution was concentrated by evaporation under reduced pressure to remove the solvent, and EtOH (200 mL) and potassium hydroxide (7.57 g) were added. The mixture was heated to 70 °C and reacted. After the reaction was completed, the reaction solution was cooled to room temperature and concentrated by evaporation under reduced pressure to remove the solvent. The residue was extracted with ethyl acetate (200 mL) and water (500 mL). The organic phase was separated, washed with saturated brine, dried over anhydrous sodium sulfate, and filtered, and the filtrate was purified by silica gel column chromatography to give the target intermediate **73d** (4.8 g).

### Step 5: Preparation of intermediate 73e

Intermediate **73d** (4.8 g), 3-(4-methoxybenzyl)dihydropyrimidine-2,4(1*H*,3*H*)-dione (4.15 g), copper(I) iodide (1.298 g), *trans*-(1*R*,2*R*)-*N*,*N*'-dimethyl-1,2-cyclohexanediamine (0.969 g, 1.089 mL), cesium carbonate (13.32 g), and 1,4-dioxane (100 mL) were added to a reaction flask in sequence, and the mixture was heated to 130 °C and reacted under N₂ atmosphere. After the reaction was completed, the reaction solution was cooled to room temperature and extracted with ethyl acetate (200 mL) and water (500 mL). The organic phase was separated, washed with saturated brine, dried over anhydrous sodium sulfate, and filtered, and the filtrate was purified by silica gel column chromatography to give the target intermediate **73e** (5.9 g).

### Step 6: Preparation of intermediate 73f

Intermediate **73e** (5.4 g) and DCM (37 mL) were added to a reaction flask in sequence, trifluoromethanesulfonic acid (67.8 g, 40 mL) was added dropwise under an ice bath, and the mixture was warmed to room temperature and reacted. After the reaction was completed, the reaction solution was added dropwise to a solution of DIPEA (66.3 g, 90 mL) in DCM (90 mL). After the dropwise addition, the mixture was warmed to room temperature. Di*-tert-*butyl dicarbonate (2.56 g, 2.73 mL) was added, and the mixture was reacted at room temperature. After the reaction was completed, the reaction solution was concentrated by evaporation under reduced pressure to remove the solvent and extracted with ethyl acetate (200 mL) and water (500 mL). The organic phase was separated, washed with saturated brine, dried over anhydrous sodium sulfate, and filtered, and the filtrate was purified by silica gel column chromatography to give the target intermediate **73f** (1.5 g).

### Step 7: Preparation of intermediate 73

Intermediate **73f** (1.5 g) and DCM (5 mL) were added to a reaction flask in sequence, a solution of hydrochloric acid in 1,4-dioxane (4 M/L, 16.35 mL) was added, and the mixture was reacted at room temperature. After the reaction was completed, the reaction solution was concentrated to give intermediate **73** (1.39 g).

MS(ESI, [M+H]⁺) m/z: 286.1.

¹H NMR (500 MHz, DMSO-d6) δ 10.54 (s, 1H), 9.77 (s, 2H), 8.16 (s, 1H), 7.51 (d, J = 8.1 Hz, 1H), 7.14 (d, J = 8.2 Hz, 1H), 4.48 (s, 2H), 3.84 (t, J = 6.7 Hz, 2H), 3.41 (t, J = 6.2 Hz, 2H), 3.13 (t, J = 6.2 Hz, 2H), 2.78 (t, J = 6.7 Hz, 2H).

### Example 74: Synthesis of Intermediate 74

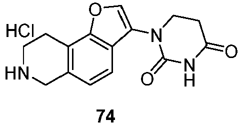

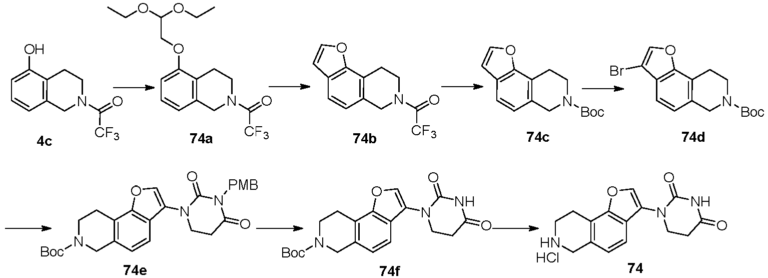

### Step 1: Preparation of intermediate 74a

Intermediate **4c** (30 g), bromoacetaldehyde diethyl acetal (34.6 g), potassium carbonate (32.4 g), and DMA (300 mL) were added to a reaction flask in sequence, and the mixture was heated to 150 °C and reacted. After the reaction was completed, the reaction solution was extracted with ethyl acetate (500 mL) and water (1000 mL). The organic phase was separated, washed with saturated brine, dried over anhydrous sodium sulfate, and filtered, and the filtrate was purified by silica gel column chromatography to give the target intermediate **74a** (15.67 g).

¹H NMR (500 MHz, DMSO-d6) δ 7.18 (t, J = 7.9 Hz, 1H), 6.87 (dt, J = 20.1, 7.9 Hz, 2H), 4.81 (td, J = 5.2, 1.5 Hz, 1H), 4.73 (d, J = 4.3 Hz, 2H), 3.96 (d, J = 5.2 Hz, 2H), 3.83 (t, J = 6.1 Hz, 2H), 3.68 (ddt, J = 9.7, 8.5, 6.4 Hz, 2H), 3.61 - 3.54 (m, 2H), 2.76 (dt, J = 15.9, 6.1 Hz, 2H), 1.14 (t, J = 7.1 Hz, 6H).

### Step 2: Preparation of intermediate 74b

Intermediate **74a** (15.67 g), phosphoric acid (25.5 g), and chlorobenzene (80 mL) were added to a reaction flask in sequence, and the mixture was heated to 120 °C and reacted. After the reaction was completed, the reaction solution was extracted with ethyl acetate (500 mL) and water (1000 mL). The organic phase was separated, washed with saturated brine, dried over anhydrous sodium sulfate, and filtered, and the filtrate was concentrated by evaporation under reduced pressure to remove the solvent, thus giving the target intermediate **74b** (11.34 g).

¹H NMR (500 MHz, DMSO-d6) δ 8.00 (dd, J = 3.1, 2.2 Hz, 1H), 7.52 (dd, J = 8.0, 2.1 Hz, 1H), 7.16 (t, J = 8.1 Hz, 1H), 6.95 (t, J = 1.8 Hz, 1H), 4.88 (d, J = 7.2 Hz, 2H), 3.96 - 3.92 (m, 2H), 3.14 - 3.08 (m, 2H)

### Step 3: Preparation of intermediate 74c

Intermediate **74b** (11.34 g) and methanol (100 mL) were added to a reaction flask in sequence, a solution of sodium hydroxide (5.05 g) in water (100 mL) was added dropwise under an ice bath, and the mixture was warmed to room temperature and reacted. After the reaction was completed, the reaction solution was concentrated by evaporation under reduced pressure to remove the methanol, and 1,4-dioxane (100 mL) and di-*tert*-butyl dicarbonate (10.11 g, 10.76 mL) were added. The mixture was reacted at room temperature. After the reaction was completed, the reaction solution was extracted with ethyl acetate (500 mL) and water (800 mL), dried over anhydrous sodium sulfate, and filtered, and the filtrate was concentrated by evaporation under reduced pressure to remove the solvent, thus giving the target intermediate **74c** (15.12 g).

MS(ESI, [M+H]⁺) m/z: 174.2.

¹H NMR (500 MHz, DMSO-d6) δ 7.96 (d, J = 2.2 Hz, 1H), 7.47 (d, J = 8.0 Hz, 1H), 7.05 (d, J = 8.0 Hz, 1H), 6.92 (d, J = 2.2 Hz, 1H), 4.61 (s, 2H), 3.66 (t, J = 6.0 Hz, 2H), 2.96 (t, J = 5.9 Hz, 2H), 1.44 (s, 9H).

### Step 4: Preparation of intermediate 74d

Intermediate **74c** (15.12 g), DCM (300 mL), and liquid bromine (7.80 g, 2.499 mL) were added to a reaction flask in sequence, and the mixture was reacted at room temperature. After the reaction was completed, the reaction solution was concentrated by evaporation under reduced pressure to remove the solvent, and EtOH (100 mL) and potassium hydroxide (4.56 g) were added. The mixture was heated to 70 °C and reacted. After the reaction was completed, the reaction solution was cooled to room temperature and concentrated by evaporation under reduced pressure to remove the solvent. The residue was extracted with ethyl acetate (200 mL) and water (500 mL). The organic phase was separated, washed with saturated brine, dried over anhydrous sodium sulfate, and filtered, and the filtrate was purified by silica gel column chromatography to give the target intermediate **74d** (5.88 g).

¹H NMR (500 MHz, DMSO-d6) δ 8.27 (s, 1H), 7.36 (d, J = 8.0 Hz, 1H), 7.21 (s, 1H), 4.64 (s, 2H), 3.67 (s, 2H), 2.96 (s, 2H), 1.44 (s, 9H).

### Step 5: Preparation of intermediate 74e

Intermediate **74d** (5.5 g), 3-(4-methoxybenzyl)dihydropyrimidine-2,4(1*H*,3*H*)-dione (3.11 g), copper(I) iodide (1.945 g), *trans*-(1*R*,2*R*)-*N*,*N*'-dimethyl-1,2-cyclohexanediamine (1.452 g, 1.632 mL), cesium carbonate (9.98 g), and 1,4-dioxane (60 mL) were added to a reaction flask in sequence, and the mixture was heated to 130 °C and reacted under N₂ atmosphere. After the reaction was completed, the reaction solution was cooled to room temperature and extracted with ethyl acetate (200 mL) and water (500 mL). The organic phase was separated, washed with saturated brine, dried over anhydrous sodium sulfate, and filtered, and the filtrate was purified by silica gel column chromatography to give the target intermediate **74e** (3.59 g).

MS(ESI, [M+H]⁺) m/z: 506.1.

¹H NMR (500 MHz, DMSO-d6) δ 8.13 (s, 1H), 7.37 (d, J = 8.1 Hz, 1H), 7.25 - 7.23 (m, 2H), 7.08 (d, J = 8.1 Hz, 1H), 6.88 - 6.86 (m, 2H), 4.84 (s, 2H), 4.61 (s, 2H), 3.85 (t, J = 6.7 Hz, 2H), 3.73 (s, 3H), 3.66 (t, J = 5.8 Hz, 2H), 2.95 (dt, J = 6.2, 2.8 Hz, 4H), 1.43 (s, 9H)

### Step 6: Preparation of intermediate 74f

Intermediate **74e** (3.59 g) and DCM (50 mL) were added to a reaction flask in sequence, trifluoromethanesulfonic acid (34.2 g, 20 mL) was added dropwise under an ice bath, and the mixture was warmed to room temperature and reacted. After the reaction was completed, the reaction solution was added dropwise to a solution of DIPEA (34.1 g, 46 mL) in DCM (50 mL). After the dropwise addition, the mixture was warmed to room temperature. Di*-tert-*butyl dicarbonate (1.585 g, 1.687 mL) was added, and the mixture was reacted at room temperature. After the reaction was completed, the reaction solution was concentrated by evaporation under reduced pressure to remove the solvent and extracted with ethyl acetate (200 mL) and water (500 mL). The organic phase was separated, washed with saturated brine, dried over anhydrous sodium sulfate, and filtered, and the filtrate was purified by silica gel column chromatography to give the target intermediate **74f** (1.09 g).

¹H NMR (500 MHz, DMSO-d6) δ 10.51 (s, 1H), 8.09 (s, 1H), 7.42 (d, J = 8.1 Hz, 1H), 7.08 (d, J = 8.2 Hz, 1H), 4.62 (s, 2H), 3.83 (t, J = 6.7 Hz, 2H), 3.66 (t, J = 6.0 Hz, 2H), 2.95 (t, J = 6.0 Hz, 2H), 2.78 (t, J = 6.7 Hz, 2H), 1.44 (s, 9H).

### Step 7: Preparation of intermediate 74

Intermediate **74f** (1.09 g) and DCM (5 mL) were added to a reaction flask in sequence, a solution of hydrochloric acid in 1,4-dioxane (4 mol/L, 9.14 mL) was added, and the mixture was reacted at room temperature. After the reaction was completed, the reaction solution was concentrated to give intermediate **74** (0.89 g).

MS(ESI, [M+H]⁺) m/z: 286.1.

¹H NMR (500 MHz, DMSO-d6) δ 10.54 (s, 1H), 9.71 (s, 2H), 8.17 (s, 1H), 7.50 (d, J = 8.1 Hz, 1H), 7.13 (d, J = 8.2 Hz, 1H), 4.36 (s, 2H), 3.84 (t, J = 6.6 Hz, 2H), 3.45 (t, J = 6.2 Hz, 2H), 3.19 (t, J = 6.5 Hz, 2H), 2.78 (t, J = 6.6 Hz, 2H).

### Example 75: Synthesis of Intermediate 75

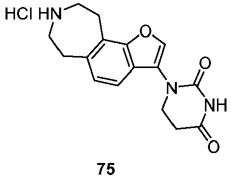

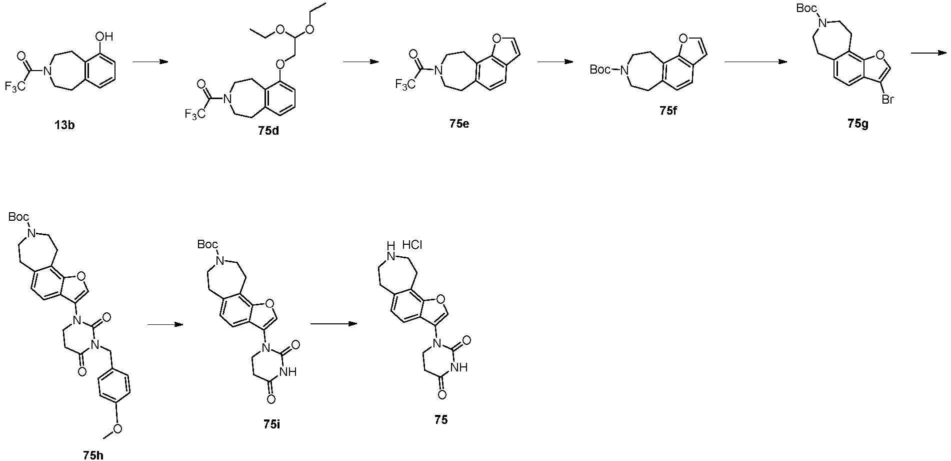

### Step 1: Preparation of intermediate 75d

The starting materials **13b** (38 g), potassium carbonate (35.3 g), bromoacetaldehyde diethyl acetal (50.4 g), and N,N-dimethylacetamide (380 mL) were added to a reaction flask in sequence, and the mixture was heated to 150 °C and reacted for 6 h. After the reaction was completed, the reaction solution was cooled to room temperature, diluted with ethyl acetate, and washed with a saturated sodium chloride solution, and the organic layer was collected, dried over anhydrous sodium sulfate, and filtered. The filtrate was concentrated, and then the residue was purified by silica gel column chromatography to give the target intermediate **75d** (23 g).

¹H NMR (500 MHz, DMSO-*d*6) δ 7.10 (td, *J* = 7.8, 3.3 Hz, 1H), 6.91 (d, *J* = 8.3 Hz, 1H), 6.80 (q, *J* = 7.2 Hz, 1H), 4.86 - 4.77 (m, 1H), 3.92 (dd, *J =* 5.3, 1.0 Hz, 2H), 3.67 (tdd, *J* = 10.1, 5.1, 2.9 Hz, 6H), 3.60 - 3.55 (m, 2H), 3.11 - 3.01 (m, 2H), 2.98 (ddd, *J* = 14.3, 8.1, 4.1 Hz, 2H), 1.16 - 1.12 (m, 6H).

### Step 2: Preparation of intermediate 75e

The starting material **75d** (23 g), phosphoric acid (28.8 g), and chlorobenzene (230 mL) were added to a reaction flask in sequence, and the mixture was heated to 130 °C and reacted for 2 h. After the reaction was completed, the reaction solution was cooled to room temperature and added with ethyl acetate and water, and the organic layer was separated. The aqueous layer was extracted twice with ethyl acetate. The organic layers were combined, dried over anhydrous sodium sulfate, and filtered. The filtrate was concentrated, and then the residue was purified by silica gel column chromatography to give the target intermediate **75e** (17 g).

¹H NMR (500 MHz, DMSO-*d*6) δ 7.96 (t, *J =* 2.2 Hz, 1H), 7.42 (dd, *J* = 7.8, 2.8 Hz, 1H), 7.09 (t, *J* = 7.6 Hz, 1H), 6.92 (d, *J =* 2.2 Hz, 1H), 3.84 - 3.76 (m, 2H), 3.76 - 3.69 (m, 2H), 3.31 - 3.28 (m, 1H), 3.28 - 3.24 (m, 1H), 3.16 - 3.11 (m, 1H), 3.11 - 3.07 (m, 1H).

### Step 3: Preparation of intermediate 75f

The starting materials **75e** (17 g), sodium hydroxide (7.2 g), methanol (135 mL), and water (45 mL) were added to a reaction flask in sequence, and the mixture was reacted at room temperature for 2 h. After the reaction was completed, di-*tert*-butyl dicarbonate (15.7 g) was added, and the mixture was reacted at room temperature for 2 h. After the reaction was completed, part of the solvent was removed by concentration, the reaction solution was added with ethyl acetate and water, and the organic layer was separated. The aqueous layer was extracted with ethyl acetate. The organic layers were combined, dried over anhydrous sodium sulfate, and filtered. The filtrate was concentrated, and then the residue was purified by silica gel column chromatography to give the target intermediate **75f** (19 g).

¹H NMR (500 MHz, DMSO-*d*6) δ 8.24 (s, 1H), 7.28 (d, *J =* 7.9 Hz, 1H), 7.19 (d, *J =* 8.0 Hz, 1H), 3.54 (d, *J =* 6.7 Hz, 2H), 3.50 (t, *J =* 5.1 Hz, 2H), 3.14 (d, *J =* 5.7 Hz, 2H), 3.05 - 2.95 (m, 2H).1.47 (s, 9H).

### Step 4: Preparation of intermediate 75g

The starting materials **75f** (17.2 g) and dichloromethane (510 mL) were added to a reaction flask in sequence, and bromine (14.4 g) was added under an ice bath. After the addition, the mixture was reacted at room temperature for 0.5 h. After the reaction was completed, a saturated aqueous sodium thiosulfate solution was added to the reaction solution to quench the reaction, and the organic layer was separated. The aqueous layer was extracted twice with dichloromethane. The organic layers were combined, dried over anhydrous sodium sulfate, and filtered, and the filtrate was concentrated to give a crude product. The crude product was dissolved in ethanol (170 mL), potassium hydroxide (10.1 g) was added, and the mixture was reacted at 80 °C for 1 h. After the reaction was completed, the mixture was cooled to room temperature and concentrated to remove part of the solvent, the reaction solution was added with ethyl acetate and water, and the organic layer was separated. The aqueous layer was extracted with ethyl acetate. The organic layers were combined, dried over anhydrous sodium sulfate, and filtered. The filtrate was concentrated, and then the residue was purified by silica gel column chromatography to give the target intermediate **75g** (14 g).

¹H NMR (500 MHz, DMSO-*d*6) δ 8.24 (s, 1H), 7.28 (d, *J =* 7.9 Hz, 1H), 7.19 (d, *J =* 8.0 Hz, 1H), 3.54 (d, *J =* 6.7 Hz, 2H), 3.50 (t, *J =* 5.1 Hz, 2H), 3.14 (d, *J =* 5.7 Hz, 2H), 3.05 - 2.95 (m, 2H).1.47 (s, 9H).

### Step 5: Preparation of intermediate 75h

The starting materials **75g** (11.8 g), 3-(4-methoxybenzyl)dihydropyrimidine-2,4(1*H*,3*H*)-dione (9.81 g), (1*S*,2*S*)-*N*1,*N*2-dimethylcyclohexane-1,2-diamine (2.29 g), copper(I) iodide (3.07 g), and cesium carbonate (31.5 g) were added to a reaction flask in sequence, and the mixture was heated to 120 °C and reacted for 12 h. After the reaction was completed, the mixture was cooled to room temperature and purified by silica gel column chromatography to give the target intermediate **75h** (14 g).

¹H NMR (500 MHz, DMSO-*d*6) δ 8.11 (s, 1H), 7.28 (d, *J* = 7.8 Hz, 1H), 7.26 - 7.21 (m, 2H), 7.08 (d, *J =* 8.0 Hz, 1H), 6.91 - 6.84 (m, 2H), 4.83 (s, 2H), 3.84 (t, *J* = 6.7 Hz, 2H), 3.72 (s, 3H), 3.52 (dt, *J* = 28.0, 5.1 Hz, 4H), 3.13 (d, *J =* 5.5 Hz, 2H), 2.97 (dt, *J* = 18.2, 5.8 Hz, 4H), 1.40 (s, 9H).

### Step 6: Preparation of intermediate 75i

The starting materials **75h** (22 g) and trifluoromethanesulfonic acid (220 mL) were added to a reaction flask in sequence, and the mixture was reacted at room temperature for 2 h. After the reaction was completed, the reaction solution was slowly added to a solution of *N*,*N*-diisopropylethylamine in dichloromethane to quench the reaction, di-*tert*-butyl dicarbonate (10.2 g) was added, and the mixture was reacted at room temperature for 2 h. After the reaction was completed, the reaction solution was added with water, and the organic layer was separated. The aqueous layer was extracted with dichloromethane. The organic layers were combined, dried over anhydrous sodium sulfate, and filtered. The filtrate was concentrated, and then the residue was purified by silica gel column chromatography to give the target intermediate **75i** (4.9 g).

MS(ESI, [M+H]⁺) m/z: 400.10.

### Step 7: Preparation of intermediate 75

**75i** (5.0 g) and 1,4-dioxane (25 mL) were added to a reaction flask in sequence, a solution of hydrochloric acid in 1,4-dioxane (25 mL, 4 M) were added, and the mixture was reacted at room temperature for 2 h. After the reaction was completed, the reaction system was added with methyl *tert*-butyl ether and filtered under vacuum, and the filter cake was washed with methyl *tert*-butyl ether, collected, and dried to give the target intermediate **75** (4.1 g).

MS(ESI, [M+H]⁺) m/z: 300.09.

¹H NMR (500 MHz, DMSO-*d*6) δ 10.52 (s, 1H), 9.33 (s, 2H), 8.13 (s, 1H), 7.40 (d, *J =* 7.9 Hz, 1H), 7.15 (d, *J* = 8.0 Hz, 1H), 3.82 (t, *J =* 6.7 Hz, 2H), 3.38 (dd, *J =* 7.1, 3.3 Hz, 2H), 3.30 - 3.18 (m, 6H), 2.78 (t, *J =* 6.7 Hz, 2H).

### Example 76: Synthesis of Intermediate 76

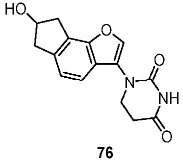

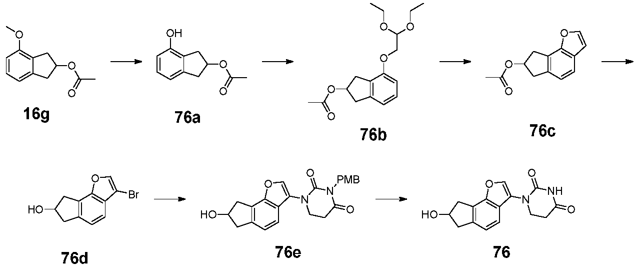

### Step 1: Preparation of intermediate 76a

The starting materials **16g** (36 g) and dichloromethane (360 mL) were added to a reaction flask in sequence, and boron tribromide (96 g) was slowly added under an ice bath. After the addition, the mixture was warmed to room temperature and reacted for 2 h. After the reaction was completed, the reaction solution was poured into ice water to quench the reaction, and the organic layer was separated. The aqueous layer was extracted with dichloromethane. The organic layers were combined, dried over anhydrous sodium sulfate, and filtered. The filtrate was concentrated, and then the residue was purified by silica gel column chromatography to give the target intermediate **76a** (31 g).

¹H NMR (500 MHz, DMSO-*d*6) δ 9.26 (s, 1H), 6.98 (t, *J =* 7.7 Hz, 1H), 6.69 (d, *J =* 7.4 Hz, 1H), 6.60 (d, *J* = 7.9 Hz, 1H), 5.39 (tt, *J =* 6.3, 2.5 Hz, 1H), 3.22 (dd, *J =* 17.0, 6.3 Hz, 1H), 3.11 (dd, *J =* 17.1, 6.3 Hz, 1H), 2.83 (ddd, *J* = 21.7, 17.0, 2.4 Hz, 2H), 1.96 (s, 3H).

### Step 2: Preparation of intermediate 76b

The starting materials **76a** (32 g), cesium carbonate (108 g), potassium iodide (5.53 g), bromoacetaldehyde diethyl acetal (82 g), and *N,N*-dimethylformamide (320 mL) were added to a reaction flask in sequence, and the mixture was heated to 90 °C and reacted for 4 h. After the reaction was completed, the reaction solution was cooled to room temperature, diluted with ethyl acetate, and washed with a saturated sodium chloride solution, the organic layer was collected, dried over anhydrous sodium sulfate, and filtered. The filtrate was concentrated, and then the residue was purified by silica gel column chromatography to give the target intermediate **76b** (46 g).

¹H NMR (500 MHz, DMSO-*d*6) δ 7.13 (t, *J =* 7.8 Hz, 1H), 6.85 (d, *J =* 7.4 Hz, 1H), 6.81 (d, *J =* 8.1 Hz, 1H), 5.41 (tt, *J =* 6.4, 2.4 Hz, 1H), 4.79 (t, *J =* 5.2 Hz, 1H), 3.95 (d, *J =* 5.2 Hz, 2H), 3.68 (dq, *J* = 9.5, 7.0 Hz, 2H), 3.57 (dqd, *J =* 9.7, 7.0, 1.2 Hz, 2H), 3.26 (dd, *J =* 17.0, 6.3 Hz, 1H), 3.15 (dd, *J* = 17.2, 6.3 Hz, 1H), 2.86 (ddd, *J =* 36.0, 17.1, 2.2 Hz, 2H), 1.96 (s, 3H), 1.14 (t, *J* = 7.0 Hz, 6H).

### Step 3: Preparation of intermediate 76c

The starting material **76b** (43 g), phosphoric acid (65.6 g), and chlorobenzene (430 mL) were added to a reaction flask in sequence, and the mixture was heated to 130 °C and reacted for 2 h. After the reaction was completed, the reaction solution was cooled to room temperature and added with ethyl acetate and water, and the organic layer was separated. The aqueous layer was extracted with ethyl acetate. The organic layers were combined, dried over anhydrous sodium sulfate, and filtered. The filtrate was concentrated, and then the residue was purified by silica gel column chromatography to give the target intermediate **76c** (31 g).

¹H NMR (500 MHz, DMSO-*d*6) δ 7.92 (d, *J =* 2.2 Hz, 1H), 7.49 (d, *J* = 7.8 Hz, 1H), 7.17 (d, *J* = 7.8 Hz, 1H), 6.94 (d, *J =* 2.2 Hz, 1H), 5.54 (tt, *J* = 6.3, 2.4 Hz, 1H), 3.51 - 3.44 (m, 1H), 3.40 (dd, *J =* 16.9, 6.3 Hz, 1H), 3.12 (dd, *J* = 17.1, 2.3 Hz, 1H), 3.02 (dd, *J =* 16.8, 2.3 Hz, 1H), 1.97 (s, 3H).

### Step 4: Preparation of intermediate 76d

The starting materials **76c** (21 g) and dichloromethane (630 mL) were added to a reaction flask in sequence, and bromine (23.3 g) was added under an ice bath. After the addition, the mixture was reacted at room temperature for 0.5 h. After the reaction was completed, a saturated sodium thiosulfate aqueous solution was added to the reaction solution to quench the reaction, and the organic layer was separated. The aqueous layer was extracted with dichloromethane. The organic layers were combined, dried over anhydrous sodium sulfate, and filtered, and the filtrate was concentrated to give a crude product. The crude product was dissolved in ethanol (210 mL), potassium hydroxide (16.4 g) was added, and the mixture was reacted at 80 °C for 1 h. After the reaction was completed, the mixture was cooled to room temperature and concentrated to remove part of the solvent, the reaction solution was added with ethyl acetate and water, and the organic layer was separated. The aqueous layer was extracted with ethyl acetate. The organic layers were combined, dried over anhydrous sodium sulfate, and filtered. The filtrate was concentrated, and then the residue was purified by silica gel column chromatography to give the target intermediate **76d** (14 g).

¹H NMR (500 MHz, DMSO-*d*6) δ 8.20 (s, 1H), 7.32 (d, *J* = 7.8 Hz, 1H), 7.25 (d, *J =* 7.8 Hz, 1H), 4.64 (tq, *J =* 6.2, 3.0 Hz, 1H), 3.26 - 3.22 (m, 1H), 2.98 - 2.91 (m, 2H), 2.88 (dd, *J* = 16.3, 3.3 Hz, 2H).

### Step 5: Preparation of intermediate 76e

The starting materials **76d** (12 g), 3-(4-methoxybenzyl)dihydropyrimidine-2,4(1*H*,3*H*)-dione (8.66 g), (1*S*,2*S*)-*N*1,*N*2-dimethylcyclohexane-1,2-diamine (2.1 g), copper(I) iodide (2.71 g), and cesium carbonate (27.8 g) were added to a reaction flask in sequence, and the mixture was heated to 120 °C and reacted for 12 h. After the reaction was completed, the mixture was cooled to room temperature and purified by silica gel column chromatography to give the target intermediate **76e** (7.0 g).

MS(ESI, [M+H]⁺) m/z: 407.10.

### Step 6: Preparation of intermediate 76

The starting materials **76e** (7 g) and trifluoromethanesulfonic acid (70 mL) were added to a reaction flask in sequence, and the mixture was reacted at room temperature for 2 h. After the reaction was completed, the reaction solution was slowly added to a saturated sodium bicarbonate solution to quench the reaction, dichloromethane was added, and the organic layer was separated. The aqueous layer was extracted with dichloromethane. The organic layers were combined, dried over anhydrous sodium sulfate, and filtered. The filtrate was concentrated, and then the residue was purified by silica gel column chromatography (methanol/dichloromethane) to give the target intermediate **76** (0.47 g).

MS(ESI, [M+H]⁺) m/z: 287.05.

¹H NMR (500 MHz, DMSO-*d*6) δ 10.49 (s, 1H), 8.03 (s, 1H), 7.38 (d, *J* = 7.9 Hz, 1H), 7.15 (d, *J =* 7.9 Hz, 1H), 4.97 (d, *J =* 4.1 Hz, 1H), 4.64 (td, *J =* 6.2, 2.9 Hz, 1H), 3.82 (t, *J =* 6.7 Hz, 2H), 3.25 (dd, *J* = 16.2, 6.1 Hz, 1H), 3.19 (dd, *J* = 16.1, 6.0 Hz, 1H), 2.94 (dd, *J* = 16.2, 3.3 Hz, 1H), 2.86 (dd, *J* = 16.0, 3.4 Hz, 1H), 2.78 (t, *J =* 6.7 Hz, 2H).

### Example 77: Preparation of Intermediate 77

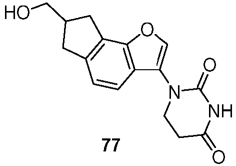

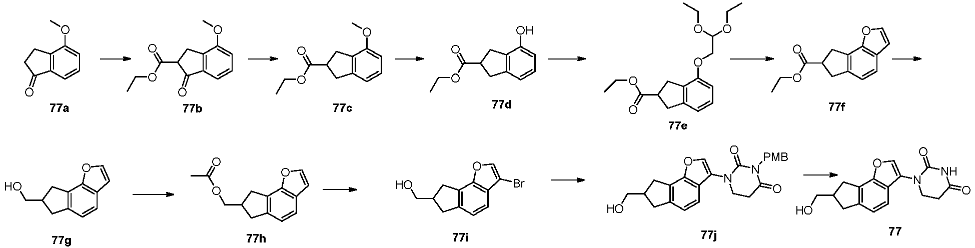

### Step 1: Preparation of intermediate 77b

**77a** (100 g), diethyl carbonate (146 g), and tetrahydrofuran (2000 mL) were added to a reaction flask in sequence, and the mixture was cooled to 0 °C. Sodium hydride (49.3 g) was added in portions, and the mixture was reacted at room temperature for 3 h. After the reaction was completed, the reaction solution was slowly poured into ice water (3 L), and ethyl acetate was added for extraction. The organic phase was separated, dried over anhydrous sodium sulfate, and purified by silica gel column chromatography to give **77b** (97 g).

MS(ESI, [M+H]⁺) *m*/*z*: 235.13.

### Step 2: Preparation of intermediate 77c

**77b** (95 g), 10% palladium on carbon (20 g), and ethanol (400 mL) were added to a reaction flask in sequence, and the mixture was purged with hydrogen and then warmed to 50 °C and reacted for 36 h. After the reaction was completed, the mixture was concentrated by rotary evaporation to remove the solvent, ethyl acetate and water were added, and the organic phase was separated, dried over anhydrous sodium sulfate, and purified by silica gel column chromatography to give **77c** (60 g).

MS(ESI, [M+H]⁺) *m*/*z*: 221.15.

### Step 3: Preparation of intermediate 77d

**77c** (60 g) and tetrahydrofuran (500 mL) were added to a reaction flask in sequence, and the mixture was cooled to 0 °C. Boron tribromide (240 mL) was slowly added dropwise, and after the dropwise addition, the mixture was reacted for 3 h with the temperature maintained. After the reaction was completed, the reaction solution was slowly poured into ice water, and ethyl acetate was added for extraction. The organic phase was separated, dried over anhydrous sodium sulfate, and concentrated by evaporation under reduced pressure to remove the solvent, thus giving **77d** (42 g).

### Step 4: Preparation of intermediate 77e

**77d** (41 g), bromoacetaldehyde diethyl acetal (82 g), cesium carbonate (135 g), potassium iodide (6.89 g), and dimethylacetamide (300 mL) were added to a reaction flask in sequence, and the mixture was warmed to 90 °C and reacted for 3 h. After the reaction was completed, the mixture was added with water (800 mL) and extracted with ethyl acetate, washed with saturated brine, dried over anhydrous sodium sulfate, and filtered. The filtrate was concentrated, and the crude product was separated and purified by silica gel column chromatography to give intermediate **77e** (55.4 g).

### Step 5: Preparation of intermediate 77f

**77e** (55 g), chlorobenzene (200 mL), and phosphoric acid (83.6 g) were added to a reaction flask in sequence, and the mixture was reacted at 130 °C for 1 h. After the reaction was completed, the mixture was extracted with water (400 mL) and ethyl acetate, washed with saturated brine, dried over anhydrous sodium sulfate, and filtered. The filtrate was concentrated, and the crude product was separated and purified by silica gel column chromatography to give intermediate **77f** (33 g).

MS(ESI, [M+H]⁺) *m*/*z*: 231.10.

### Step 6: Preparation of intermediate 77g

**77f** (33 g) and tetrahydrofuran (300 mL) were added to a reaction flask in sequence. The mixture was cooled to 0 °C, and a solution of lithium aluminum hydride in tetrahydrofuran (152 mL) was added dropwise. After the dropwise addition, the mixture was reacted for 2 h with the temperature maintained. After the reaction was completed, the reaction solution was slowly poured into ice water (600 mL), and ethyl acetate was added for extraction. The organic phase was separated, dried over anhydrous sodium sulfate, and concentrated by evaporation under reduced pressure to remove the solvent, thus giving **77g** (30 g).

### Step 7: Preparation of intermediate 77h

**77g** (30 g), dichloromethane (300 g), and DIPEA (41.2 g) were added to a reaction flask in sequence. The mixture was cooled to 0 °C, acetic anhydride (17.9 g) was slowly added dropwise, and the mixture was allowed to react at room temperature for 2 h. After the reaction was completed, water (400 mL) was added, the organic phase was separated, washed with a saturated ammonium chloride aqueous solution, dried over anhydrous sodium sulfate, and filtered, and the filtrate was concentrated to give intermediate **77h** (29 g).

### Step 7: Preparation of intermediate 77i

**77h** (30 g) and DCM (500 mL) were added to a reaction flask in sequence, and the mixture was cooled to 0 °C. Liquid bromine (21.86 g) was added, and the mixture was reacted at room temperature for 0.5 h. After the reaction was completed, the mixture was concentrated by rotary evaporation to remove the solvent. Ethanol (140 mL) and potassium hydroxide (21.93 g) were added to the residue, and the mixture was warmed to 70 °C and reacted for 0.5 h. After the reaction was completed, the reaction solution was poured into water, vigorously stirred, extracted with ethyl acetate, washed with saturated brine, dried over anhydrous sodium sulfate, and filtered. The filtrate was concentrated, and the crude product was separated by silica gel column chromatography to give intermediate **77i** (33.6 g).

### Step 8: Preparation of intermediate 77j

**77i** (4.35 g), 3-(4-methoxybenzyl)dihydropyrimidine-2,4(1*H*,3*H*)-dione (3.52 g), (1*R*,2*R*)-*N*1,*N*2-dimethylcyclohexane-1,2-diamine (0.88 g), copper(I) iodide (1.17 g), cesium carbonate (12.42 g), and 1,4-dioxane (100 mL) were added to a reaction flask in sequence, and the mixture was reacted at 120 °C for 15 h. After the reaction was completed, the reaction solution was concentrated and extracted with water and ethyl acetate, followed by liquid separation. The organic phase was washed with saturated brine, dried over anhydrous sodium sulfate, and filtered. The filtrate was concentrated, and the crude product was separated by silica gel column chromatography to give intermediate **77j** (3.82 g).

MS(ESI, [M+H]⁺) *m*/*z*: 421.11.

### Step 9: Preparation of intermediate 77

**77j** (3.82 g) and trifluoromethanesulfonic acid (40 mL) were added to a reaction flask in sequence, and the mixture was reacted at room temperature for 0.5 h. After the reaction was completed, the reaction solution was slowly added to water, and the mixture was adjusted to pH 7-8 with sodium bicarbonate and extracted with ethyl acetate. The organic phase was separated, washed with saturated brine, dried over anhydrous sodium sulfate, and filtered, and the filtrate was purified by silica gel column chromatography to give intermediate **77** (0.54 g).

¹H NMR (500 MHz, DMSO-*d*6) δ 10.49 (s, 1H), 8.02 (s, 1H), 7.36 (d, *J* = 7.9 Hz, 1H), 7.13 (d, *J* = 8.0 Hz, 1H), 4.70 (t, *J =* 5.2 Hz, 1H), 3.82 (t, *J =* 6.7 Hz, 2H), 3.41 (ddd, *J =* 6.7, 5.3, 1.3 Hz, 2H), 3.10 (ddd, *J =* 35.6, 16.1, 8.2 Hz, 2H), 2.93 - 2.74 (m, 4H), 2.70 (dqd, *J =* 8.2, 6.8, 5.5 Hz, 1H).

### Example 78: Synthesis of Compound 78

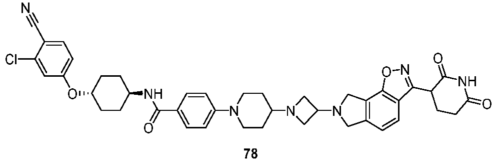

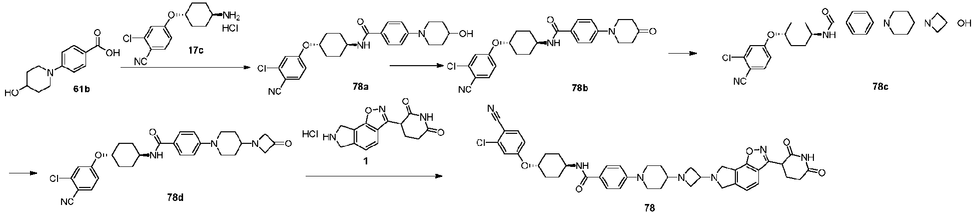

### Step 1: Preparation of intermediate 78a

Intermediate **17c** (8.25 g), intermediate **61b** (8.60 g), *N,N*-diisopropylethylamine (14.85 g, 20.07 mL), dichloromethane (150 mL), and HATU (13.11 g) were added to a reaction flask in sequence, and the mixture was reacted at room temperature. After the reaction was completed, the reaction solution was concentrated by evaporation under reduced pressure to remove the solvent, and the residue was extracted with ethyl acetate (200 mL) and water (200 mL). The organic phase was separated, washed with saturated brine, dried over anhydrous sodium sulfate, and filtered, and the filtrate was purified by silica gel column chromatography to give intermediate **78a** (9.82 g).

MS(ESI, [M+H]+) m/z: 454.1.

### Step 2: Preparation of intermediate 78b

Intermediate **78a** (3.48 g), dimethyl sulfoxide (20 mL), and 2-iodoxybenzoic acid (2.361 g) were added to a reaction flask in sequence, and the mixture was reacted at room temperature overnight. After the reaction was completed, ethyl acetate (100 mL) and water (200 mL) were added for extraction. The organic phase was separated, washed with saturated brine, dried over anhydrous sodium sulfate, and filtered, and the filtrate was concentrated by evaporation under reduced pressure to remove the solvent, thus giving intermediate **78b** (3.22 g).

MS(ESI, [M+H]+) m/z: 452.1.

### Step 3: Preparation of intermediate 78c

Intermediate **78b** (1.0 g), DMA (30 mL), 3-hydroxyazetidine hydrochloride (0.41 g), and sodium acetate (0.46 g) were added to a reaction flask in sequence, and the mixture was heated to 80 °C. Sodium cyanoborohydride (0.47 g) was added, and the mixture was reacted for 2 h with the temperature maintained. After the reaction was completed, the reaction solution was added with water and ethyl acetate. The organic phase was separated, washed with saturated brine, dried over anhydrous sodium sulfate, filtered, and concentrated, and the residue was purified by silica gel column chromatography to give intermediate **78c** (0.65 g).

MS(ESI, [M+H]+) m/z: 509.2.

### Step 4: Preparation of intermediate 78d

Intermediate **78c** (0.65 g), acetonitrile (50 mL), and 2-iodoxybenzoic acid (0.53 g) were added to a reaction flask in sequence, boron trifluoride diethyl etherate (1.81 g) was added dropwise, and the mixture was reacted at room temperature for 4 h. After the reaction was completed, ethyl acetate (100 mL) and water (200 mL) were added for extraction. The organic phase was separated, washed with saturated brine, dried over anhydrous sodium sulfate, and filtered, and the filtrate was concentrated by evaporation under reduced pressure to remove the solvent, thus giving intermediate **78d** (0.69 g).

MS(ESI, [M+H]⁺) m/z: 507.2.

### Step 4: Preparation of compound 78

Intermediate **1** (70 mg), intermediate **78d** (102 mg), sodium acetate (17.85 mg), 1,2-dichloroethane (5 mL), isopropanol (1 mL), and sodium cyanoborohydride (41.0 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give compound **78** (65 mg).

MS(ESI, [M+H]⁺) m/z: 762.2.

¹H NMR (500 MHz, DMSO-*d*6) δ 11.09 (s, 1H), 7.95 (d, *J =* 7.6 Hz, 1H), 7.86 (d, *J =* 8.8 Hz, 1H), 7.79 - 7.67 (m, 3H), 7.38 (d, *J =* 2.4 Hz, 1H), 7.31 (d, *J =* 8.1 Hz, 1H), 7.14 (dd, *J* = 8.7, 2.4 Hz, 1H), 6.93 (d, *J =* 8.7 Hz, 2H), 4.60 (dd, *J =* 11.9, 5.0 Hz, 1H), 4.53 (q, *J =* 5.4 Hz, 1H), 4.16 (s, 2H), 4.05 (t, *J =* 2.4 Hz, 2H), 3.80 (s, 1H), 3.67 (d, *J =* 12.3 Hz, 2H), 3.53 (q, *J =* 6.3 Hz, 1H), 3.43 (s, 2H), 3.34 (s, 1H), 3.04 (s, 2H), 2.91 (t, *J* = 10.8 Hz, 2H), 2.77 (ddd, *J =* 17.2, 11.9, 5.3 Hz, 1H), 2.65 - 2.57 (m, 1H), 2.27 (s, 1H), 2.24 - 2.16 (m, 1H), 2.10 (d, *J =* 10.6 Hz, 2H), 1.89 (d, *J =* 11.1 Hz, 2H), 1.73 (d, *J =* 11.7 Hz, 2H), 1.59 - 1.43 (m, 4H), 1.30 - 1.21 (m, 2H).

### Example 79: Synthesis of Compound 79

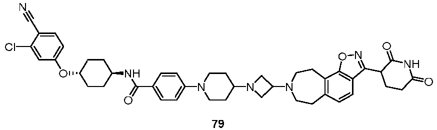

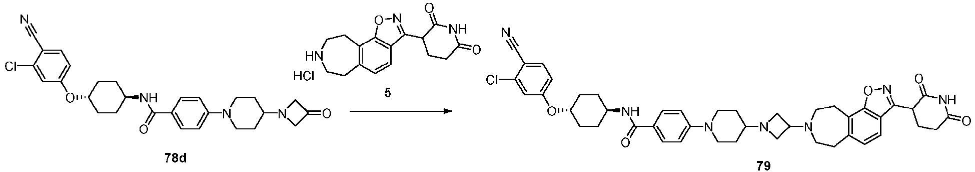

### Step 1: Preparation of compound 79

Intermediate **5** (82 mg), intermediate **78d** (105 mg), sodium acetate (16.45 mg), 1,2-dichloroethane (5 mL), isopropanol (1 mL), and sodium cyanoborohydride (44.3 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give compound **79** (65 mg).

MS(ESI, [M+H]⁺) m/z: 790.4.

¹H NMR (500 MHz, DMSO-*d*6) δ 11.07 (s, 1H), 7.96 (d, *J=* 7.6 Hz, 1H), 7.86 (d, *J =* 8.7 Hz, 1H), 7.72 (d, *J =* 8.4 Hz, 2H), 7.55 (d, *J =* 7.9 Hz, 1H), 7.38 (d, *J =* 2.4 Hz, 1H), 7.23 - 7.11 (m, 2H), 6.93 (d, *J* = 8.5 Hz, 2H), 4.54 (dt, *J =* 12.5, 6.1 Hz, 2H), 3.85 - 3.62 (m, 3H), 3.49 (s, 2H), 3.15 (d, *J* = 5.5 Hz, 2H), 3.09 - 2.97 (m, 3H), 2.89 (t, *J =* 11.4 Hz, 2H), 2.77 (ddd, *J =* 17.0, 11.8, 5.2 Hz, 2H), 2.60 (dt, *J =* 17.5, 4.5 Hz, 1H), 2.50 - 2.37 (m, 7H), 2.18 (dq, *J =* 13.2, 4.5 Hz, 1H), 2.10 (d, *J =* 10.6 Hz, 2H), 1.89 (d, *J* = 9.1 Hz, 2H), 1.75 (s, 2H), 1.52 (tt, *J* = 13.9, 7.4 Hz, 4H), 1.34 - 1.21 (m, 2H).

### Example 80: Synthesis of Compound 80

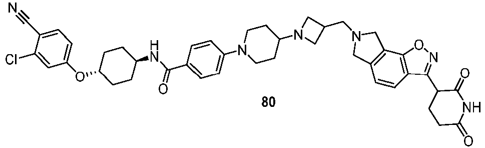

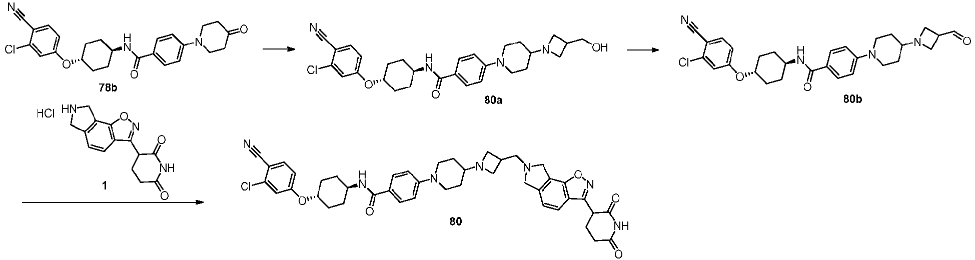

### Step 1: Preparation of intermediate 80a

Intermediate **78b** (0.95 g), DMA (30 mL), 3-hydroxymethylazacyclobutadiene hydrochloride (0.46 g), and sodium acetate (0.52 g) were added to a reaction flask in sequence, and the mixture was stirred at room temperature for 10 min. Sodium cyanoborohydride (0.55 g) was added, and the mixture was reacted at room temperature for 2 h. After the reaction was completed, the reaction solution was added with water and ethyl acetate. The organic phase was separated, washed with saturated brine, dried over anhydrous sodium sulfate, filtered, and concentrated, and the residue was purified by silica gel column chromatography to give intermediate **80a** (0.84 g).

### Step 2: Preparation of intermediate 80b

Intermediate **80a** (0.81 g), acetonitrile (50 mL), and 2-iodoxybenzoic acid (0.72 g) were added to a reaction flask in sequence, boron trifluoride diethyl etherate (2.42 g) was added dropwise, and the mixture was reacted at room temperature for 4 h. After the reaction was completed, ethyl acetate (100 mL) and water (200 mL) were added for extraction. The organic phase was separated, washed with saturated brine, dried over anhydrous sodium sulfate, and filtered, and the filtrate was concentrated by evaporation under reduced pressure to remove the solvent, thus giving intermediate **80b** (0.69 g).

### Step 3: Preparation of compound 80

Intermediate **80b** (108 mg), compound **1** (114 mg), sodium acetate (13.49 mg), 1,2-dichloroethane (5 mL), isopropanol (1 mL), and sodium cyanoborohydride (43.3 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give compound **80** (75 mg).

MS(ESI, [M+H]⁺) m/z: 776.2.

¹H NMR (500 MHz, DMSO-*d*6) δ 11.08 (s, 1H), 7.96 (d, *J =* 7.6 Hz, 1H), 7.86 (d, *J =* 8.7 Hz, 1H), 7.72 (t, *J =* 8.6 Hz, 3H), 7.38 (d, *J =* 2.4 Hz, 1H), 7.29 (d, *J =* 8.1 Hz, 1H), 7.14 (dd, *J =* 8.9, 2.4 Hz, 1H), 6.93 (d, *J* = 8.6 Hz, 2H), 4.56 (ddd, *J =* 34.2, 11.0, 5.1 Hz, 2H), 4.12 (s, 2H), 4.01 (s, 2H), 3.84 - 3.64 (m, 3H), 3.43 (s, 4H), 2.98 - 2.70 (m, 7H), 2.62 (td, *J =* 14.3, 13.0, 7.5 Hz, 2H), 2.54 (d, *J* = 4.4 Hz, 1H), 2.49 - 2.45 (m, 1H), 2.20 (dq, *J =* 13.5, 4.7 Hz, 1H), 2.15 - 2.07 (m, 2H), 1.93 - 1.86 (m, 2H), 1.73 (s, 2H), 1.58 - 1.44 (m, 4H).

### Example 81: Synthesis of Compound 81

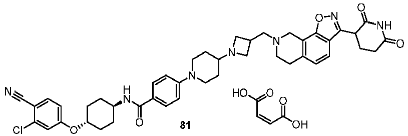

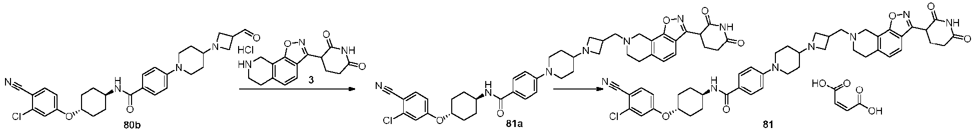

### Step 1: Preparation of intermediate 81a

Intermediate **3** (70 mg), intermediate **80b** (113 mg), sodium acetate (17.85 mg), 1,2-dichloroethane (5 mL), isopropanol (1 mL), and sodium cyanoborohydride (27.3 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give the target intermediate **81a** (47 mg).

MS(ESI, [M+H]⁺) m/z: 790.2.

### Step 2: Preparation of compound 81

Intermediate **81a** (47 mg), maleic acid (7.59 mg), and MeOH/DCM (v:v = 1/10, 5 mL) were added to a reaction flask in sequence, and after complete dissolution, the reaction solution was concentrated. The reaction solution was slurried with petroleum ether (10 mL) and filtered under vacuum to give compound **81** (38 mg).

MS(ESI, [M+H]⁺) m/z: 790.2.

¹H NMR (500 MHz, DMSO-d6) δ 11.09 (s, 1H), 10.16 (s, 1H), 7.99 (d, J = 7.6 Hz, 1H), 7.86 (d, J = 8.7 Hz, 1H), 7.75 (d, J = 8.3 Hz, 2H), 7.65 (d, J = 8.1 Hz, 1H), 7.38 (s, 1H), 7.19 (d, J = 8.3 Hz, 1H), 7.14 (d, J = 8.9 Hz, 1H), 6.98 (d, J = 8.5 Hz, 2H), 6.12 (s, 2H), 4.62 - 4.49 (m, 2H), 4.24 (d, J = 9.8 Hz, 2H), 4.05 (s, 2H), 3.97 (d, J = 12.6 Hz, 4H), 3.80 (s, 1H), 3.42 (s, 3H), 3.01 (d, J = 32.0 Hz, 6H), 2.78 (t, J = 13.3 Hz, 3H), 2.61 (d, J = 18.6 Hz, 1H), 2.23 - 2.16 (m, 1H), 2.11 (d, J = 10.6 Hz, 2H), 1.98 (d, J = 11.7 Hz, 2H), 1.89 (d, J = 11.0 Hz, 2H), 1.51 (q, J = 13.1 Hz, 4H), 1.44 - 1.35 (m, 2H).

### Example 82: Synthesis of Compound 82

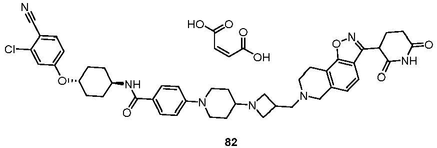

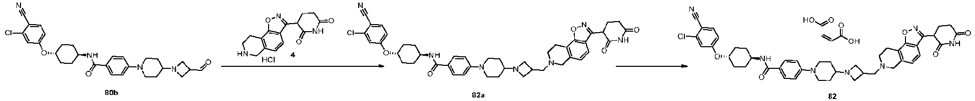

### Step 1: Preparation of intermediate 82a

Intermediate **80b** (147 mg), 1,2-dichloroethane (6 mL), isopropanol (2 mL), intermediate **4** (70 mg), sodium acetate (17 mg), and sodium triacetoxyborohydride (165 mg) were added to a reaction flask in sequence, and the mixture was stirred at room temperature. After the reaction was completed, dichloromethane (50 mL) and water (100 mL) were added for extraction. The organic phase was separated, washed with saturated brine, dried over anhydrous sodium sulfate, filtered, and concentrated, and the residue was purified by silica gel column chromatography to give compound **82a** (30 mg).

MS(ESI, [M+H]⁺) m/z: 790.62.

### Step 2: Preparation of compound 82

**82a** (30 mg), maleic acid (5 mg), and MeOH/DCM (v:v = 1/10, 5 mL) were added to a reaction flask in sequence, and after complete dissolution, the reaction solution was concentrated. The reaction solution was slurried with petroleum ether and filtered under vacuum to give compound **82** (15 mg).

MS(ESI, [M+H]⁺) m/z: 790.21.

¹H NMR (500 MHz, DMSO-*d*6) δ 11.08 (s, 1H), 9.09 (s, 1H), 7.98 (d, *J =* 7.6 Hz, 1H), 7.86 (d, *J =* 8.7 Hz, 1H), 7.75 (d, *J =* 8.3 Hz, 2H), 7.57 (d, *J* = 8.0 Hz, 1H), 7.38 (s, 1H), 7.21 (d, *J =* 8.1 Hz, 1H), 7.14 (d, *J =* 8.8 Hz, 1H), 6.97 (d, *J =* 8.5 Hz, 2H), 6.02 (s, 2H), 4.55 (dd, *J =* 12.4, 5.3 Hz, 2H), 3.97 (d, *J =* 12.1 Hz, 2H), 3.80 (s, 1H), 3.14 (d, *J =* 56.1 Hz, 6H), 3.00 - 2.56 (m, 10H), 2.22 - 2.06 (m, 3H), 2.04 - 1.78 (m, 6H), 1.76 - 1.36 (m, 8H).

### Example 83: Synthesis of Compound 83

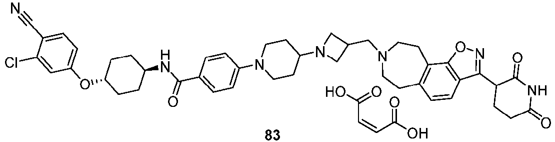

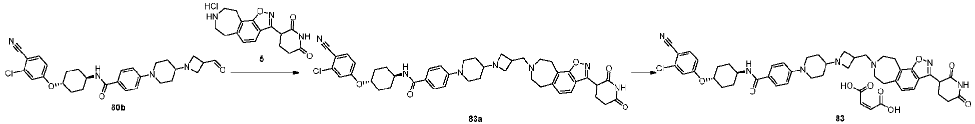

### Step 1: Preparation of intermediate 83a

Intermediate **80b** (98 mg), intermediate **5** (84 mg), sodium acetate (11.25 mg), 1,2-dichloroethane (5 mL), isopropanol (1 mL), and sodium cyanoborohydride (38.4 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give intermediate **83a** (78 mg).

MS(ESI, [M+H]⁺) m/z: 804.2.

### Step 2: Preparation of compound 83

**83a** (78 mg), dichloromethane (10 mL), and methanol (1 mL) were added to a reaction flask in sequence. After complete dissolution by stirring, maleic acid (6.4 mg) was added, and the mixture was stirred at room temperature for 10 min. The mixture was concentrated and then slurried with petroleum ether to give compound **83** (74 mg).

¹H NMR (500 MHz, DMSO-*d*6) δ 11.08 (s, 1H), 7.99 (d, *J =* 7.6 Hz, 1H), 7.86 (d, *J =* 8.7 Hz, 1H), 7.76 (d, *J =* 8.4 Hz, 2H), 7.58 (d, *J =* 8.0 Hz, 1H), 7.38 (d, *J =* 2.4 Hz, 1H), 7.22 (d, *J =* 8.1 Hz, 1H), 7.14 (dd, *J =* 8.8, 2.5 Hz, 1H), 6.98 (d, *J =* 8.5 Hz, 2H), 6.04 (s, 2H), 4.55 (td, *J* = 9.9, 7.9, 5.0 Hz, 2H), 4.17 (t, *J* = 9.1 Hz, 2H), 4.02 - 3.74 (m, 5H), 3.21 (s, 2H), 3.14 - 3.07 (m, 2H), 2.97 (s, 3H), 2.91 - 2.73 (m, 6H), 2.61 (dt, *J =* 17.3, 4.3 Hz, 1H), 2.53 (s, 1H), 2.50 - 2.44 (m, 2H), 2.22 - 2.06 (m, 3H), 1.93 (dd, *J =* 35.7, 11.5 Hz, 4H), 1.59 - 1.44 (m, 4H), 1.34 (tt, *J* = 13.0, 9.3, 7.7 Hz, 2H).

### Example 84: Synthesis of Compound 84

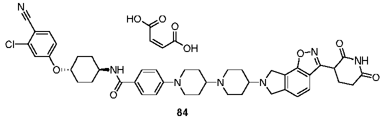

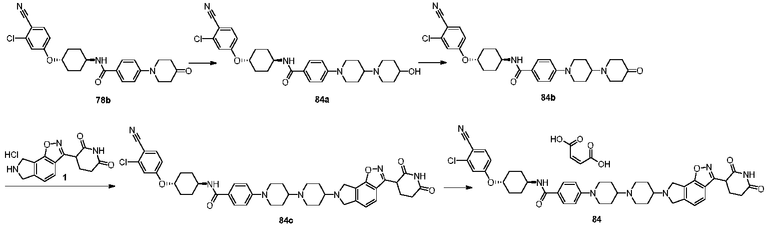

### Step 1: Preparation of intermediate 84a

Intermediate **78b** (1 g), 4-hydroxypiperidine (0.227 g), 1,2-dichloroethane (5 mL), isopropanol (1 mL), and sodium cyanoborohydride (0.352 g) were added to a reaction flask in sequence, and the mixture was heated to 80 °C and reacted. After the reaction was completed, dichloromethane (100 mL) and water (100 mL) were added for extraction. The organic phase was separated, dried over anhydrous sodium sulfate, and filtered, and the filtrate was purified by silica gel column chromatography to give the target intermediate **84a** (0.775 g).

MS(ESI, [M+H]⁺) m/z: 537.3.

¹H NMR (500 MHz, DMSO-d6) δ 8.00 (d, J = 7.6 Hz, 1H), 7.86 (d, J = 8.7 Hz, 1H), 7.74 (d, J = 8.5 Hz, 2H), 7.39 (d, J = 2.4 Hz, 1H), 7.14 (dd, J = 8.8, 2.4 Hz, 1H), 6.96 (d, J = 8.3 Hz, 2H), 4.99 (s, 1H), 4.54 (td, J = 9.9, 4.6 Hz, 1H), 4.07 - 3.73 (m, 4H), 3.57 (s, 1H), 3.17 (d, J = 3.4 Hz, 1H), 2.76 (t, J = 12.4 Hz, 2H), 2.23 - 1.38 (m, 19H).

### Step 2: Preparation of intermediate 84b

Intermediate **84a** (550 mg), 2-iodoxybenzoic acid (430 mg), acetonitrile (10 mL), and boron trifluoride diethyl etherate (1453 mg, 1.264 mL) were added to a reaction flask in sequence, and the mixture was reacted at room temperature. After the reaction was completed, ethyl acetate (200 mL) and a saturated sodium bicarbonate solution (200 mL) were added for extraction. The organic phase was dried over anhydrous sodium sulfate, filtered, and concentrated to give the target intermediate **84b** (400 mg).

MS(ESI, [M+H]⁺) m/z: 535.4.

### Step 3: Preparation of intermediate 84c

Intermediate **1** (60 mg), intermediate **84b** (100 mg), sodium acetate (15.99 mg), 1,2-dichloroethane (5 mL), isopropanol (1 mL), and sodium cyanoborohydride (36.8 mg) were added to a reaction flask in sequence, and the mixture was heated to 80 °C and reacted. After the reaction was completed, dichloromethane (50 mL) and water (100 mL) were added for extraction. The organic phase was separated, dried over anhydrous sodium sulfate, and filtered, and the filtrate was purified by silica gel column chromatography to give the target intermediate **84c** (42 mg).

MS(ESI, [M+H]⁺) m/z: 790.2.

### Step 4: Preparation of compound 84

Intermediate **84c** (42 mg), maleic acid (6.78 mg), and MeOH/DCM (v:v = 1/10, 5 mL) were added to a reaction flask in sequence, and after complete dissolution, the reaction solution was concentrated. The reaction solution was slurried with petroleum ether (10 mL) and filtered under vacuum to give compound **84** (27 mg).

MS(ESI, [M+H]⁺) m/z: 790.2.

¹H NMR (500 MHz, DMSO-d6) δ 11.11 (s, 1H), 9.46 (s, 2H), 8.00 (d, J = 7.6 Hz, 1H), 7.86 (d, J = 8.7 Hz, 1H), 7.83 - 7.67 (m, 3H), 7.38 (s, 2H), 7.14 (d, J = 8.9 Hz, 1H), 6.99 (d, J = 8.5 Hz, 2H), 6.18 (s, 2H), 4.63 (dd, J = 11.9, 4.9 Hz, 1H), 4.58 - 4.38 (m, 3H), 4.26 (dd, J = 58.3, 20.8 Hz, 2H), 4.03 (d, J = 12.4 Hz, 2H), 3.80 (s, 2H), 3.71 - 3.58 (m, 2H), 3.44 (s, 3H), 3.05 (s, 3H), 2.85 - 2.74 (m, 3H), 2.66 - 2.59 (m, 1H), 2.33 (d, J = 12.9 Hz, 1H), 2.23 - 2.18 (m, 1H), 2.11 (d, J = 10.8 Hz, 4H), 1.89 (d, J = 11.2 Hz, 3H), 1.72 (s, 2H), 1.51 (q, J = 14.4, 13.8 Hz, 4H).

### Example 85: Synthesis of Compound 85

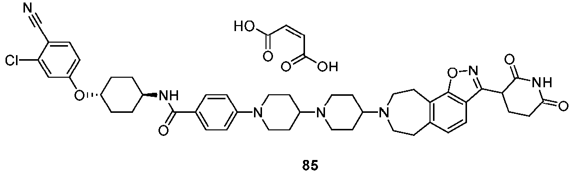

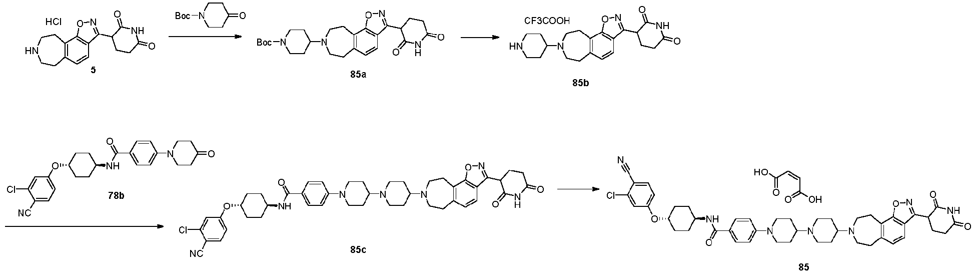

### Step 1: Preparation of intermediate 85a

Intermediate **5** (100 mg), acetonitrile (10 mL), *tert*-butyl 4-oxopiperidine-1-carboxylate (178 mg), sodium acetate (24 mg), and sodium cyanoborohydride (94 mg) were added to a reaction flask in sequence, and the mixture was stirred at 80 °C. After the reaction was completed, dichloromethane (50 mL) and water (100 mL) were added for extraction. The organic phase was separated, washed with saturated brine, dried over anhydrous sodium sulfate, filtered, and concentrated, and the residue was purified by silica gel column chromatography to give compound **85a** (110 mg).

### Step 2: Preparation of intermediate 85b

**85a** (110 mg), DCM (10 mL), and trifluoroacetic acid (2 mL) were added to a reaction flask in sequence and reacted at room temperature. After the reaction was completed, the reaction solution was concentrated to give compound **85b** (198 mg).

MS(ESI, [M+H]⁺) m/z: 383.2.

### Step 3: Preparation of intermediate 85c

Intermediate **78b** (140 mg), acetonitrile (10 mL), **85b** (189 mg), sodium acetate (76 mg), and sodium cyanoborohydride (58 mg) were added to a reaction flask in sequence, and the mixture was stirred at 80 °C. After the reaction was completed, dichloromethane (50 mL) and water (100 mL) were added for extraction. The organic phase was separated, washed with saturated brine, dried over anhydrous sodium sulfate, filtered, and concentrated, and the residue was purified by silica gel column chromatography to give compound **85c** (45 mg).

MS(ESI, [M+H]⁺) m/z: 818.2.

### Step 4: Preparation of compound 85

**85c** (45 mg), maleic acid (6 mg), and MeOH/DCM (v:v = 1/10, 5 mL) were added to a reaction flask in sequence, and after complete dissolution, the reaction solution was concentrated. The reaction solution was slurried with petroleum ether and filtered under vacuum to give compound **85** (48 mg).

MS(ESI, [M+H]⁺) m/z: 818.19.

¹H NMR (500 MHz, DMSO-*d*6) δ 11.08 (s, 1H), 9.09 (s, 1H), 7.98 (d, *J =* 7.6 Hz, 1H), 7.86 (d, *J =* 8.7 Hz, 1H), 7.75 (d, *J =* 8.3 Hz, 2H), 7.57 (d, *J* = 8.0 Hz, 1H), 7.38 (s, 1H), 7.21 (d, *J* = 8.1 Hz, 1H), 7.14 (d, *J =* 8.8 Hz, 1H), 6.97 (d, *J =* 8.5 Hz, 2H), 6.02 (s, 2H), 4.55 (dd, *J =* 12.4, 5.3 Hz, 2H), 3.97 (d, *J =* 12.1 Hz, 2H), 3.80 (s, 1H), 3.14 (d, *J =* 56.1 Hz, 6H), 3.00 - 2.56 (m, 10H), 2.22 - 2.06 (m, 3H), 2.04 - 1.78 (m, 6H), 1.76 - 1.36 (m, 8H).

### Example 86: Synthesis of Compound 86

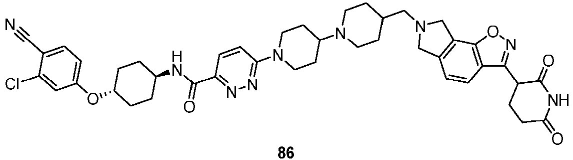

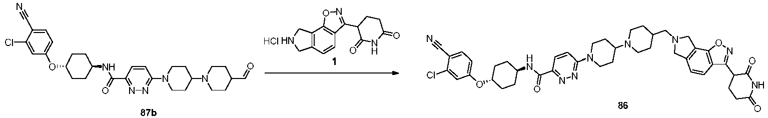

### Step 1: Preparation of compound 86

**87b** (150 mg), intermediate **1** (81 mg), sodium acetate (46 mg), 1,2-dichloroethane (10 mL), and isopropanol (2 mL) were added to a reaction flask in sequence, and the mixture was stirred at room temperature for 10 min. Sodium triacetoxyborohydride (58 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **86** (14 mg).

MS(ESI, [M+H]⁺) m/z: 806.51.

¹H NMR (500 MHz, DMSO-*d*6) δ 11.11 (s, 1H), 8.61 (d, *J =* 8.2 Hz, 1H), 7.86 (dd, *J* = 21.2, 9.1 Hz, 2H), 7.73 (d, *J =* 8.1 Hz, 1H), 7.45 - 7.35 (m, 2H), 7.32 (d, *J =* 8.1 Hz, 1H), 7.16 (dd, *J =* 8.7, 2.4 Hz, 1H), 4.68 - 4.47 (m, 4H), 4.14 (s, 2H), 4.04 (s, 2H), 3.88 (d, *J =* 10.0 Hz, 1H), 3.02 (t, *J =* 12.6 Hz, 2H), 2.91 (s, 2H), 2.80 (ddd, *J =* 17.3, 11.7, 5.3 Hz, 1H), 2.64 (dd, *J =* 16.1, 6.1 Hz, 4H), 2.30 - 2.18 (m,2H), 2.18 - 2.08 (m, 2H), 1.93 (d, *J* = 13.1 Hz, 4H), 1.81 (d, *J =* 12.3 Hz, 2H), 1.67 (q, *J =* 12.3 Hz, 2H), 1.53 (dt, *J =* 25.5, 12.0 Hz, 5H), 1.24 - 1.07 (m, 4H).

### Example 87: Synthesis of Compound 87

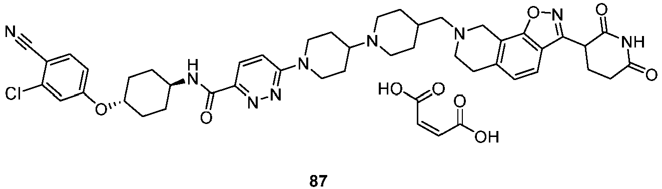

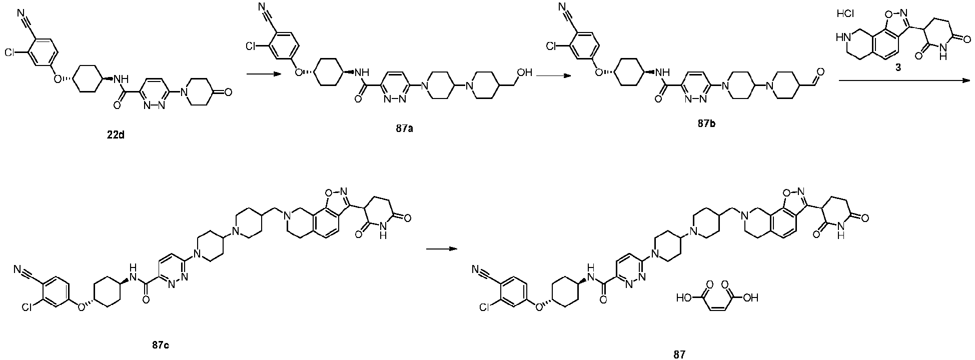

### Step 1: Preparation of intermediate 87a

**22d** (2.0 g), 4-hydroxymethylpiperidine (0.56 g), and *N,N*-dimethylacetamide (20 mL) were added to a reaction flask in sequence, sodium cyanoborohydride (6.65 g) was added in portions, and the mixture was reacted at 80 °C for 8 h. After the reaction was completed, the reaction solution was cooled to room temperature, diluted with ethyl acetate, and washed with an potassium carbonate aqueous solution and a saturated sodium chloride solution in sequence, the organic layer was collected, dried over anhydrous sodium sulfate, and filtered. The filtrate was concentrated, and then the residue was purified by silica gel column chromatography (dichloromethane/methanol) to give the target intermediate **87a** (1.2 g).

MS(ESI, [M+H]⁺) m/z: 553.55.

### Step 2: Preparation of intermediate 87b

**87a** (1.5 g), 2-iodoxybenzoic acid (1.5 g), and dimethyl sulfoxide (15 mL) were added to a reaction flask in sequence, and the mixture was reacted at 50 °C for 6 h. After the reaction was completed, the reaction solution was cooled to room temperature, quenched with a saturated sodium bicarbonate solution, and diluted with ethyl acetate. The organic layer was separated and washed with an sodium thiosulfate aqueous solution and a saturated sodium chloride solution in sequence. The organic layer was collected, dried over anhydrous sodium sulfate, and filtered. The filtrate was concentrated, and then the residue was purified by silica gel column chromatography (dichloromethane/methanol) to give the target intermediate **87b** (0.32 g).

MS(ESI, [M+H]⁺) m/z: 551.2.

### Step 3: Preparation of intermediate 87c

**87b** (70 mg), intermediate **3** (40 mg), sodium acetate (21 mg), 1,2-dichloroethane (10 mL), and isopropanol (2 mL) were added to a reaction flask in sequence, and the mixture was stirred at room temperature for 10 min. Sodium triacetoxyborohydride (24 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **87c** (30 mg).

MS(ESI, [M+H]⁺) m/z: 820.2.

### Step 4: Preparation of compound 87

Intermediate **87c** (30 mg), dichloromethane (10 mL), methanol (1 mL), and maleic acid (4.24 mg) were added to a reaction flask in sequence, and the mixture was reacted at room temperature for 1 h. After the reaction was completed, the mixture was concentrated by evaporation under reduced pressure to remove the solvent. The residue was slurried with petroleum ether and filtered, and the filter cake was collected to give compound **87** (30 mg). MS(ESI, [M+H]⁺) m/z: 820.2.

¹H NMR (500 MHz, DMSO-*d*6) δ11.09 (s, 1H), 8.98 (s, 1H), 8.61 (d, *J =* 8.3 Hz, 1H), 7.86 (t, *J =* 8.6 Hz, 2H), 7.65 (d, *J =* 8.2 Hz, 1H), 7.45 (d, *J =* 9.5 Hz, 1H), 7.38 (d, *J =* 2.4 Hz, 1H), 7.19 (d, *J =* 8.3 Hz, 1H), 7.13 (dd, *J* = 8.6, 2.5 Hz, 1H), 6.09 (s, 2H), 4.67 (d, *J =* 13.2 Hz, 2H), 4.54 (ddt, *J =* 19.2, 10.1, 4.7 Hz, 2H), 4.02 (d, *J =* 44.0 Hz, 2H), 3.86 (d, *J =* 10.0 Hz, 1H), 3.60 - 3.46 (m, 3H), 3.03 (d, *J* = 13.4 Hz, 8H), 2.77 (ddd, *J =* 17.4, 12.0, 5.4 Hz, 2H), 2.70 - 2.57 (m, 2H), 2.15 (dt, *J =* 43.5, 8.2 Hz, 6H), 2.02 (d, *J* = 13.9 Hz, 2H), 1.94 - 1.85 (m, 2H), 1.64 (q, *J* = 12.4, 11.8 Hz, 4H), 1.52 (q, *J =* 12.1 Hz,3H), 1.43 - 1.32 (m, 2H).

### Example 88: Synthesis of Compound 88

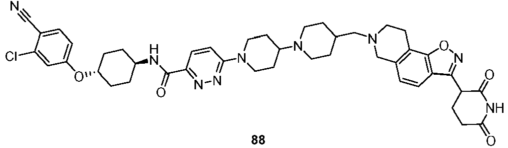

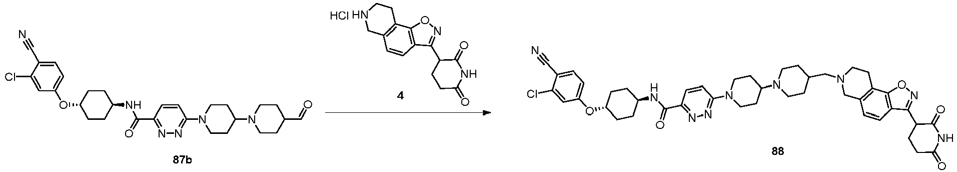

### Step 1: Preparation of compound 88

**87b** (150 mg), intermediate **4** (87 mg), sodium acetate (46 mg), 1,2-dichloroethane (10 mL), and isopropanol (2 mL) were added to a reaction flask in sequence, and the mixture was stirred at room temperature for 10 min. Sodium triacetoxyborohydride (52 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **88** (13 mg).

MS(ESI, [M+H]⁺) m/z: 820.49.

¹H NMR (500 MHz, DMSO-*d*6) δ 11.07 (s, 1H), 8.58 (d, *J =* 8.2 Hz, 1H), 7.83 (dd, *J =* 21.2, 9.1 Hz, 2H), 7.56 (d, *J =* 8.1 Hz, 1H), 7.42 - 7.31 (m, 2H), 7.16 - 7.04 (m, 2H), 4.54 (td, *J* = 11.6, 5.9 Hz, 4H), 3.86 (dtd, *J =* 11.7, 7.6, 4.1 Hz, 1H), 3.67 (s, 2H), 2.98 (q, *J =* 9.5, 6.9 Hz, 4H), 2.83 (d, *J =* 29.6 Hz, 2H), 2.76 (dt, *J =* 17.9, 6.0 Hz, 3H), 2.60 (dt, *J =* 17.3, 4.3 Hz, 2H), 2.53 (d, *J =* 8.8 Hz, 0.5H), 2.48 - 2.44 (m, 0.5H), 2.34 (d, *J =* 7.3 Hz, 2H), 2.18 (dq, *J =* 14.1, 5.2, 4.7 Hz, 2H), 2.13 - 2.06 (m, 2H), 1.94 - 1.81 (m, 4H), 1.74 (s, 2H), 1.68 - 1.38 (m, 8H), 1.13 (s, 2H). **Example 89:** Synthesis of Compound **89**

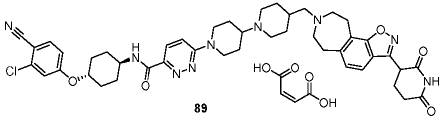

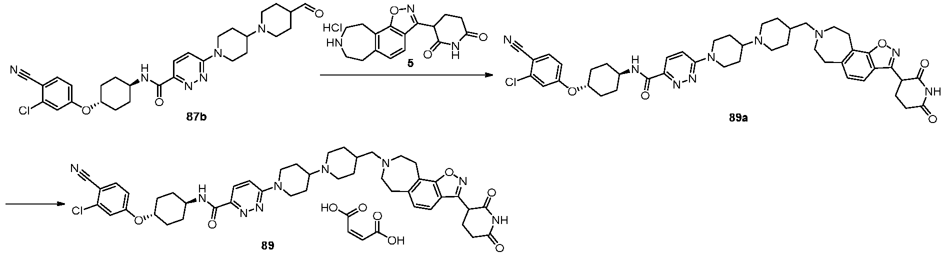

### Step 1: Preparation of intermediate 89a

Intermediate **5** (170 mg), intermediate **87b** (279 mg), sodium acetate (41.5 mg), 1,2-dichloroethane (5 mL), isopropanol (1 mL), and sodium triacetoxyborohydride (215 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give the target intermediate **89a** (43 mg).

MS(ESI, [M+H]⁺) m/z: 834.2.

### Step 2: Preparation of compound 89

Intermediate **89a** (43 mg), maleic acid (5.84 mg), and MeOH/DCM (v:v = 1/10, 5 mL) were added to a reaction flask in sequence, and after complete dissolution, the reaction solution was concentrated. The reaction solution was slurried with petroleum ether (10 mL) and filtered under vacuum to give compound **89** (48 mg).

MS(ESI, [M+H]⁺) m/z: 834.2.

¹H NMR (500 MHz, DMSO-d6) δ 11.09 (s, 1H), 10.17 (s, 1H), 8.61 (d, J = 8.2 Hz, 1H), 7.94 - 7.79 (m, 2H), 7.63 (d, J = 7.9 Hz, 1H), 7.48 - 7.34 (m, 2H), 7.26 (d, J = 7.9 Hz, 1H), 7.13 (d, J = 8.8 Hz, 1H), 6.05 (s, 2H), 4.67 (d, J = 13.1 Hz, 2H), 4.61 - 4.49 (m, 2H), 3.91 - 3.83 (m, 1H), 3.64 - 3.40 (m, 7H), 3.22 (s, 3H), 3.01 (dd, J = 30.9, 17.9 Hz, 6H), 2.87 - 2.71 (m, 2H), 2.61 (d, J = 17.8 Hz, 2H), 2.14 (dt, J = 56.0, 17.2 Hz, 8H), 1.90 (d, J = 12.2 Hz, 2H), 1.60 (ddd, J = 72.3, 25.0, 12.4 Hz, 8H).

### Example 90: Synthesis of Compound 90

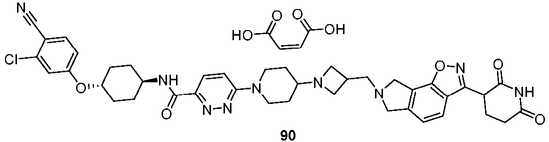

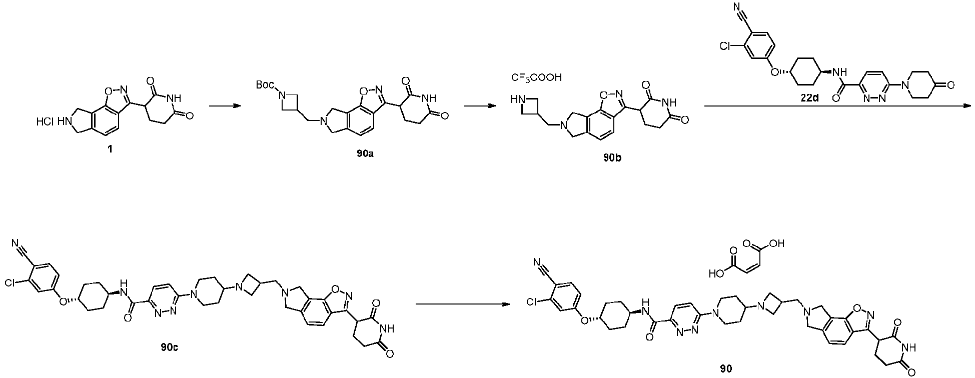

### Step 1: Preparation of intermediate 90a

Intermediate **1** (120 mg), *tert*-butyl 3-formylazetidine-1-carboxylate (164 mg), sodium acetate (73 mg), 1,2-dichloroethane (10 mL), and isopropanol (2 mL) were added to a reaction flask in sequence, and the mixture was stirred at room temperature for 10 min. Sodium triacetoxyborohydride (188 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **90a** (110 mg).

### Step 2: Preparation of intermediate 90b

**90a** (100 mg), dichloromethane (2 mL), and trifluoroacetic acid (2 mL) were added to a reaction flask in sequence, and the mixture was reacted at room temperature for 2 h. After the reaction was completed, the mixture was concentrated to remove the solvent, thus giving **90b.** The crude product was directly used in the next step. MS(ESI, [M+H]⁺) m/z: 341.2.

### Step 3: Preparation of intermediate 90c

**90b** (160 mg), compound **22d** (264 mg), sodium acetate (80 mg), and acetonitrile (20 mL) were added to a reaction flask in sequence, and the mixture was stirred at room temperature for 10 min. Sodium cyanoborohydride (61 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **90c** (45 mg).

MS(ESI, [M+H]⁺) m/z: 778.3.

### Step 4: Preparation of compound 90

Intermediate **90c** (45 mg), dichloromethane (10 mL), methanol (1 mL), and maleic acid (7.4 mg) were added to a reaction flask in sequence, and the mixture was reacted at room temperature for 1 h. After the reaction was completed, the mixture was concentrated by evaporation under reduced pressure to remove the solvent by using a rotary evaporator. The residue was slurried with petroleum ether and filtered, and the filter cake was collected to give compound **90** (30 mg).

MS(ESI, [M+H]⁺) m/z: 778.51.

¹H NMR (500 MHz, DMSO-*d*6) δ 11.10 (s, 1H), 8.61 (d, *J =* 8.2 Hz, 1H), 7.96 - 7.69 (m, 3H), 7.45 (d, *J* = 9.1 Hz, 1H), 7.38 (d, *J* = 2.4 Hz, 1H), 7.36 - 7.30 (m, 1H), 7.13 (dd, *J* = 8.7, 2.5 Hz, 1H), 6.13 (s, 2H), 4.72 - 4.47 (m, 4H), 4.40 - 4.12 (m, 5H), 3.93 (d, *J =* 58.6 Hz, 3H), 3.56 (q, *J* = 20.9, 19.8 Hz, 2H), 3.20 - 3.09 (m, 3H), 3.03 (t, *J =* 12.7 Hz, 3H), 2.78 (ddd, *J =* 17.5, 11.9, 5.4 Hz, 1H), 2.62 (d, *J* = 18.2 Hz, 1H), 2.25 - 2.16 (m, 1H), 2.15 - 1.98 (m, 4H), 1.96 - 1.84 (m, 2H), 1.58 (dq, *J =* 62.0, 12.3 Hz, 4H), 1.41 - 1.27 (m, 2H).

### Example 91: Synthesis of Compound 91

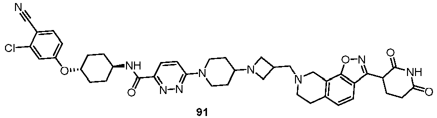

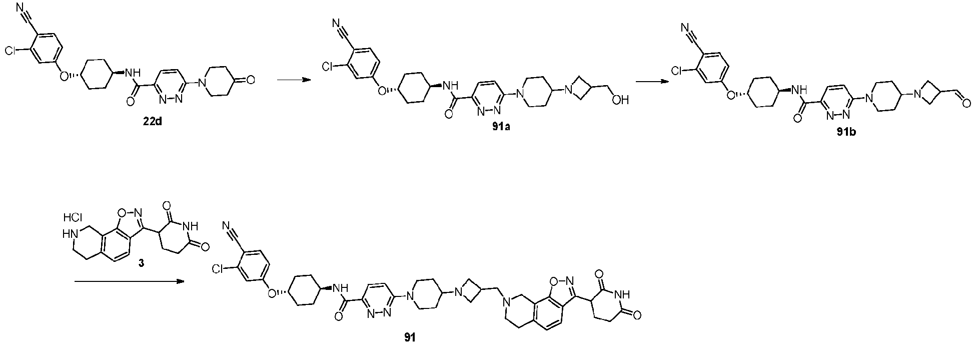

### Step 1: Preparation of intermediate 91a

Intermediate **22d** (0.87 g), DMA (30 mL), 3-hydroxymethylazacyclobutadiene hydrochloride (0.44 g), and sodium acetate (0.51 g) were added to a reaction flask in sequence. The mixture was stirred at room temperature for 10 min, sodium cyanoborohydride (0.55 g) was added, and the mixture was reacted at room temperature for 2 h. After the reaction was completed, the reaction solution was added with water and ethyl acetate. The organic phase was separated, washed with saturated brine, dried over anhydrous sodium sulfate, filtered, and concentrated, and the residue was purified by silica gel column chromatography to give intermediate **91a** (0.78 g).

### Step 2: Preparation of intermediate 91b

Intermediate **91a** (0.75 g), acetonitrile (50 mL), and 2-iodoxybenzoic acid (0.75 g) were added to a reaction flask in sequence, boron trifluoride diethyl etherate (2.32 g) was added dropwise, and the mixture was reacted at room temperature for 4 h. After the reaction was completed, ethyl acetate (100 mL) and water (200 mL) were added for extraction. The organic phase was separated, washed with saturated brine, dried over anhydrous sodium sulfate, and filtered, and the filtrate was concentrated by evaporation under reduced pressure to remove the solvent, thus giving intermediate **91b** (0.52 g).

### Step 3: Preparation of compound 91

Intermediate **91b** (98 mg), intermediate **3** (92 mg), sodium acetate (11.44 mg), 1,2-dichloroethane (5 mL), isopropanol (1 mL), and sodium cyanoborohydride (32.4 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give compound **91** (62 mg). MS(ESI, [M+H]⁺) m/z: 792.2.

¹H NMR (500 MHz, DMSO-*d*6) δ 11.08 (s, 1H), 8.58 (d, *J =* 8.2 Hz, 1H), 7.85 (d, *J* = 8.7 Hz, 1H), 7.80 (d, *J =* 9.5 Hz, 1H), 7.58 (d, *J =* 8.1 Hz, 1H), 7.38 (d, *J =* 2.4 Hz, 1H), 7.34 (d, *J =* 9.6 Hz, 1H), 7.13 (dd, *J =* 8.7, 2.7 Hz, 2H), 4.54 (ddt, *J =* 14.3, 9.8, 4.5 Hz, 2H), 4.19 (d, *J =* 12.1 Hz, 2H), 3.86 (dtd, *J =* 11.4, 7.6, 4.0 Hz, 1H), 3.78 (s, 2H), 3.44 (s, 2H), 3.23 (s, 3H), 2.99 - 2.81 (m, 4H), 2.74 (q, *J =* 5.5 Hz, 6H), 2.60 (dt, *J* = 17.2, 4.3 Hz, 1H), 2.46 (dd, *J* = 12.3, 4.3 Hz, 1H), 2.18 (dq, *J =* 13.6, 4.7 Hz, 1H), 2.15 - 2.06 (m, 2H), 1.95 - 1.85 (m, 2H), 1.74 (d, *J =* 12.3 Hz, 2H), 1.64 (qd, *J =* 13.0, 3.1 Hz, 2H), 1.57 - 1.46 (m, 2H), 1.24 - 1.15 (m, 2H).

### Example 92: Synthesis of Compound 92

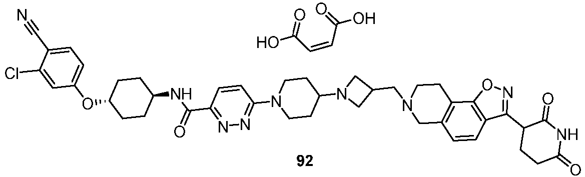

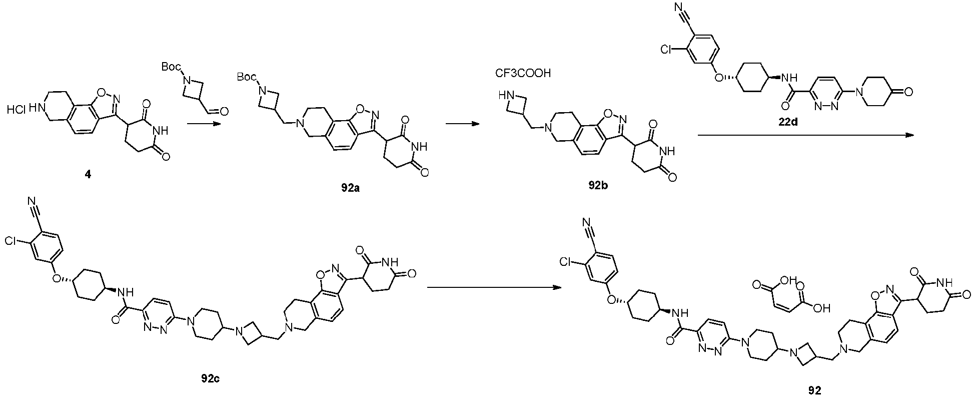

### Step 1: Preparation of intermediate 92a

Intermediate **4** (130 mg), *tert*-butyl 3-formylazetidine-1-carboxylate (200 mg), sodium acetate (80 mg), 1,2-dichloroethane (10 mL), and isopropanol (2 mL) were added to a reaction flask in sequence, and the mixture was stirred at room temperature for 10 min. Sodium triacetoxyborohydride (195 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **92a** (110 mg).

### Step 2: Preparation of intermediate 92b

**92a** (110 mg), dichloromethane (2 mL), and trifluoroacetic acid (2 mL) were added to a reaction flask in sequence, and the mixture was reacted at room temperature for 2 h. After the reaction was completed, the mixture was concentrated to remove the solvent, thus giving **92b.** The crude product was directly used in the next step. MS(ESI, [M+H]⁺) m/z: 355.5.

### Step 3: Preparation of intermediate 92c

**92b** (130 mg), compound **22d** (200 mg), sodium acetate (83 mg), and acetonitrile (20 mL) were added to a reaction flask in sequence, and the mixture was stirred at room temperature for 10 min. Sodium cyanoborohydride (65 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **92c** (50 mg).

MS(ESI, [M+H]⁺) m/z: 792.5.

### Step 4: Preparation of compound 92

Intermediate **92c** (50 mg), dichloromethane (10 mL), methanol (1 mL), and maleic acid (8.6 mg) were added to a reaction flask in sequence, and the mixture was reacted at room temperature for 1 h. After the reaction was completed, the mixture was concentrated by evaporation under reduced pressure to remove the solvent. The residue was slurried with petroleum ether and filtered, and the filter cake was collected to give compound **92** (40 mg).

MS(ESI, [M+H]⁺) m/z: 792.5.

¹H NMR (500 MHz, DMSO-*d*6) δ 11.08 (s, 1H), 8.61 (d, *J =* 8.2 Hz, 1H), 7.86 (dd, *J* = 9.2, 6.0 Hz, 2H), 7.61 (d, *J* = 8.1 Hz, 1H), 7.51 - 7.35 (m, 2H), 7.12 (t, *J =* 8.6 Hz, 2H), 6.06 (s, 2H), 4.70 - 4.47 (m, 4H), 4.22 (t, *J =* 9.4 Hz, 2H), 4.02 - 3.73 (m, 5H), 3.61 - 3.47 (m, 2H), 3.04 (d, *J* = 10.5 Hz, 4H), 2.88 (s, 4H), 2.77 (ddd, *J =* 17.3, 11.9, 5.3 Hz, 1H), 2.61 (dt, *J =* 17.3, 4.3 Hz, 1H), 2.46 (d, *J =* 8.0 Hz, 1H), 2.24 - 1.99 (m, 5H), 1.95 - 1.85 (m, 2H), 1.64 (q, *J =* 12.1 Hz, 2H), 1.58 - 1.45 (m, 2H), 1.29 (tt, *J* = 12.0, 7.1 Hz, 2H).

### Example 93: Synthesis of Compound 93

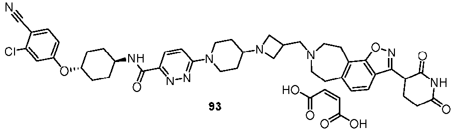

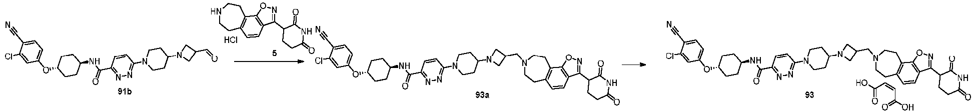

### Step 1: Preparation of intermediate 93a

Intermediate **91b** (85 mg), intermediate **5** (88 mg), sodium acetate (11.44 mg), 1,2-dichloroethane (5 mL), isopropanol (1 mL), and sodium cyanoborohydride (35.6 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give compound intermediate **93a** (56 mg).

MS(ESI, [M+H]⁺) m/z: 806.3.

### Step 2: Preparation of compound 93

**93a** (56 mg), dichloromethane (10 mL), and methanol (1 mL) were added to a reaction flask in sequence. After complete dissolution by stirring, maleic acid (17.8 mg) was added, and the mixture was stirred at room temperature for 10 min. The mixture was concentrated and then slurried with petroleum ether to give compound **93** (58 mg).

¹H NMR (500 MHz, DMSO-*d*6) δ 11.09 (s, 1H), 8.61 (d, *J =* 8.2 Hz, 1H), 7.87 (dd, *J* = 9.1, 6.4 Hz, 2H), 7.61 (d, *J* = 8.0 Hz, 1H), 7.48 - 7.37 (m, 2H), 7.24 (d, *J =* 8.1 Hz, 1H), 7.13 (dd, *J =* 8.7, 2.4 Hz, 1H), 6.07 (s, 2H), 4.55 (td, *J =* 11.3, 10.8, 4.9 Hz, 4H), 4.19 (t, *J* = 8.6 Hz, 2H), 4.00 - 3.83 (m, 3H), 3.49 (s, 3H), 3.21 - 2.92 (m, 12H), 2.78 (ddd, *J =* 17.5, 12.2, 5.5 Hz, 1H), 2.65 - 2.57 (m, 1H), 2.22 - 2.15 (m, 1H), 2.07 (dd, *J =* 41.1, 12.0 Hz, 4H), 1.95 - 1.85 (m, 2H), 1.64 (q, *J =* 12.4, 11.7 Hz, 2H), 1.52 (q, *J =* 12.0 Hz, 2H), 1.38 - 1.26 (m, 2H).

### Example 94: Synthesis of Compound 94

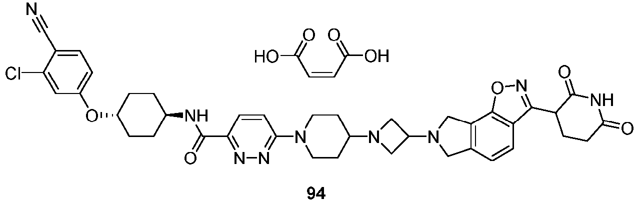

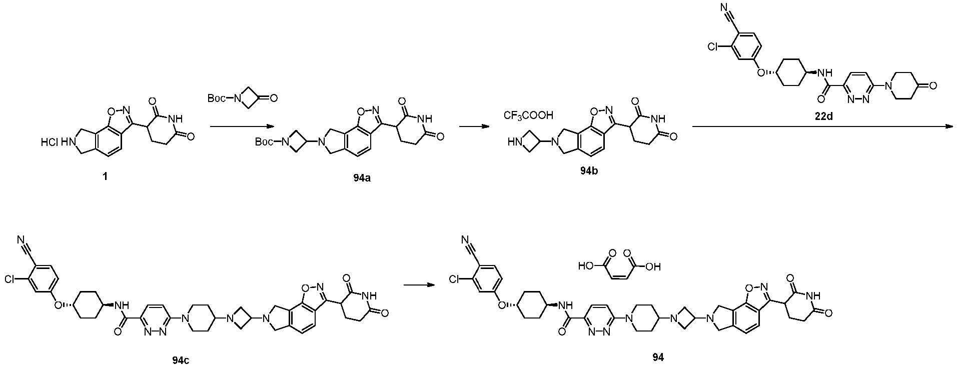

### Step 1: Preparation of intermediate 94a

Intermediate **1** (120 mg), 1-*tert*-butoxycarbonyl-3-azetidinone (151 mg), sodium acetate (73 mg), 1,2-dichloroethane (10 mL), and isopropanol (2 mL) were added to a reaction flask in sequence, and the mixture was stirred at room temperature for 10 min. Sodium triacetoxyborohydride (188 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **94a** (110 mg).

### Step 2: Preparation of intermediate 94b

**94a** (110 mg), dichloromethane (2 mL), and trifluoroacetic acid (2 mL) were added to a reaction flask in sequence, and the mixture was reacted at room temperature for 2 h. After the reaction was completed, the mixture was concentrated to remove the solvent, thus giving **94b.** The crude product was directly used in the next step. MS(ESI, [M+H]⁺) m/z: 327.2.

### Step 3: Preparation of intermediate 94c

**94b** (120 mg), compound **22d** (124 mg), sodium acetate (95 mg), and acetonitrile (20 mL) were added to a reaction flask in sequence, and the mixture was stirred at room temperature for 10 min. Sodium cyanoborohydride (31 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **94c** (80 mg).

MS(ESI, [M+H]⁺) m/z: 764.3.

### Step 4: Preparation of compound 94

Intermediate **94c** (45 mg), dichloromethane (10 mL), methanol (1 mL), and maleic acid (7.4 mg) were added to a reaction flask in sequence, and the mixture was reacted at room temperature for 1 h. After the reaction was completed, the mixture was concentrated by evaporation under reduced pressure to remove the solvent. The residue was slurried with petroleum ether and filtered, and the filter cake was collected to give compound **90** (30 mg).

MS(ESI, [M+H]⁺) m/z: 764.3.

¹H NMR (500 MHz, DMSO-*d*6) δ 11.10 (s, 1H), 8.61 (d, *J =* 8.2 Hz, 1H), 7.86 (dd, *J* = 9.2, 6.7 Hz, 2H), 7.76 (d, *J* = 8.1 Hz, 1H), 7.44 (d, *J =* 9.6 Hz, 1H), 7.40 - 7.33 (m, 2H), 7.13 (dd, *J =* 8.8, 2.5 Hz, 1H), 6.10 (s, 2H), 4.61 (dd, *J =* 11.5, 4.9 Hz, 3H), 4.53 (tt, *J =* 9.9, 4.1 Hz, 1H), 4.31 (s, 5H), 4.16 (s, 3H), 3.86 (tdt, *J =* 11.6, 7.9, 4.0 Hz, 2H), 3.65 (d, *J =* 89.7 Hz, 2H), 3.03 (s, 2H), 2.78 (ddd, *J =* 17.2, 11.9, 5.3 Hz, 1H), 2.62 (dt, *J =* 17.3, 4.1 Hz, 1H), 2.20 (dq, *J =* 13.3, 4.6 Hz, 1H), 2.11 (d, *J =* 15.1 Hz, 4H), 1.95 - 1.85 (m, 2H), 1.71 - 1.58 (m, 2H), 1.58 - 1.45 (m, 2H), 1.35 (d, *J =* 14.9 Hz, 2H).

### Example 95: Synthesis of Compound 95

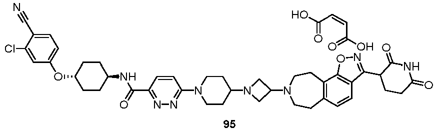

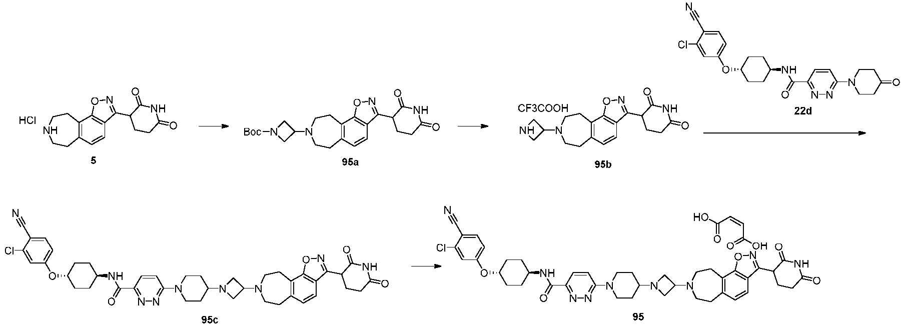

### Step 1: Preparation of intermediate 95a

Intermediate **5** (92 mg), 1-Boc-3-azetidinone (90 mg), sodium acetate (12.44 mg), 1,2-dichloroethane (5 mL), isopropanol (1 mL), and sodium cyanoborohydride (33.6 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give compound intermediate **95a** (105 mg).

### Step 2: Preparation of intermediate 95b

Intermediate **95a** (105 mg), DCM (10 mL), and trifluoroacetic acid (2 mL) were added to a reaction flask in sequence, and the mixture was reacted at room temperature. After the reaction was completed, the mixture was concentrated to remove the solvent, thus giving intermediate **95b** (142 mg).

### Step 3: Preparation of intermediate 95c

Intermediate **22d** (92 mg), intermediate **95b** (90 mg), sodium acetate (12.44 mg), 1,2-dichloroethane (5 mL), isopropanol (1 mL), and sodium cyanoborohydride (33.6 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give compound intermediate **95c** (36 mg).

MS(ESI, [M+H]⁺) m/z: 792.2.

### Step 2: Preparation of compound 95

**95c** (36 mg), dichloromethane (10 mL), and methanol (1 mL) were added to a reaction flask in sequence. After complete dissolution by stirring, maleic acid (10.56 mg) was added, and the mixture was stirred at room temperature for 10 min. The mixture was concentrated and then slurried with petroleum ether to give compound **95** (33 mg). MS(ESI, [M+H]⁺) m/z: 792.2.

¹H NMR (500 MHz, DMSO-*d*6) δ 11.08 (s, 1H), 8.61 (d, *J =* 8.2 Hz, 1H), 7.87 (dd, *J =* 9.1, 6.1 Hz, 2H), 7.58 (d, *J* = 7.9 Hz, 1H), 7.44 (d, *J =* 9.5 Hz, 1H), 7.39 (d, *J =* 2.5 Hz, 1H), 7.23 (d, *J =* 8.0 Hz, 1H), 7.13 (dd, *J* = 8.7, 2.5 Hz, 1H), 6.15 (s, 2H), 4.64 - 4.50 (m, 4H), 4.22 (t, *J =* 8.5 Hz, 2H), 4.08 (t, *J =* 8.4 Hz, 2H), 3.91 - 3.83 (m, 1H), 3.19 (d, *J =* 5.6 Hz, 3H), 3.11 - 2.99 (m, 5H), 2.78 (ddd, *J =* 17.3, 11.8, 5.2 Hz, 1H), 2.67 - 2.58 (m, 3H), 2.57 - 2.53 (m, 2H), 2.25 - 1.99 (m, 6H), 1.95 - 1.85 (m, 2H), 1.65 (q, *J =* 12.4 Hz, 2H), 1.52 (q, *J =* 12.6, 11.3 Hz, 2H), 1.39 - 1.28 (m, 2H).

### Example 96: Synthesis of Compound 96

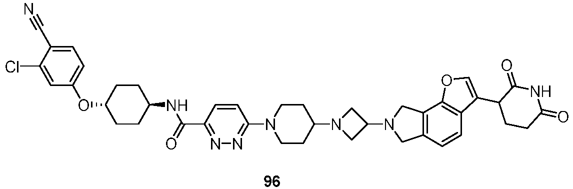

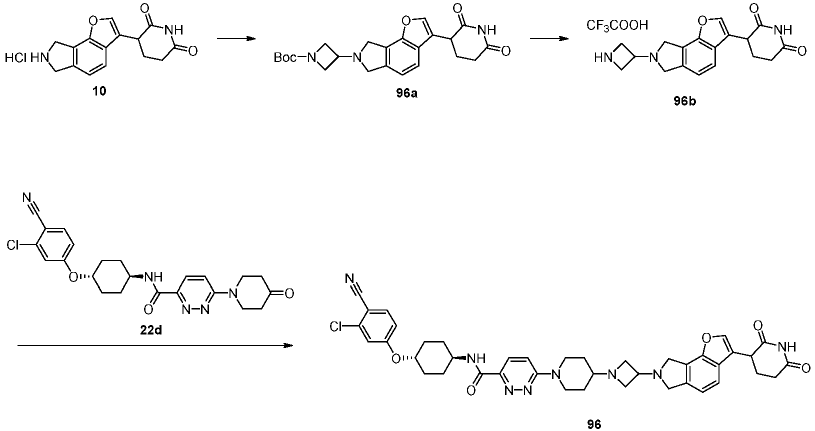

### Step 1: Preparation of intermediate 96a

Intermediate **10** (120 mg), 1-*tert*-butoxycarbonyl-3-azetidinone (151 mg), sodium acetate (73 mg), 1,2-dichloroethane (10 mL), and isopropanol (2 mL) were added to a reaction flask in sequence, and the mixture was stirred at room temperature for 10 min. Sodium triacetoxyborohydride (188 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **96a** (110 mg).

MS(ESI, [M+H]⁺) m/z: 426.2.

### Step 2: Preparation of intermediate 96b

**96a** (110 mg), dichloromethane (2 mL), and trifluoroacetic acid (2 mL) were added to a reaction flask in sequence, and the mixture was reacted at room temperature for 2 h. After the reaction was completed, the mixture was concentrated to remove the solvent, thus giving **96b.** The crude product was directly used in the next step. MS(ESI, [M+H]⁺) m/z: 326.2.

### Step 3: Preparation of compound 96

**96b** (130 mg), compound **22d** (128 mg), sodium acetate (95 mg), and acetonitrile (20 mL) were added to a reaction flask in sequence, and the mixture was stirred at room temperature for 10 min. Sodium cyanoborohydride (39 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound 96 (30 mg).

MS(ESI, [M+H]⁺) m/z: 763.3.

¹H NMR (500 MHz, DMSO-d6) δ 10.88 (s, 1H), 8.58 (d, *J=* 8.2 Hz, 1H), 7.92 - 7.75 (m, 3H), 7.48 - 7.28 (m, 3H), 7.20 - 7.08 (m, 2H), 4.59 - 4.48 (m, 1H), 4.28 - 4.05 (m, 5H), 3.99 (s, 2H), 3.83 (d, *J =* 30.3 Hz, 2H), 3.67 - 3.41 (m, 4H), 3.08 (s, 2H), 2.80 - 2.58 (m, 2.5H), 2.45 - 2.24 (m, 2.5H), 2.16 - 2.04 (m, 3H), 1.90 (d, *J =* 11.1 Hz, 2H), 1.76 (s, 2H), 1.57 (dq, *J =* 63.5, 13.0 Hz, 5H).

### Example 97: Synthesis of Compound 97

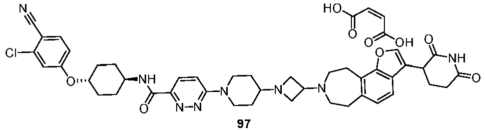

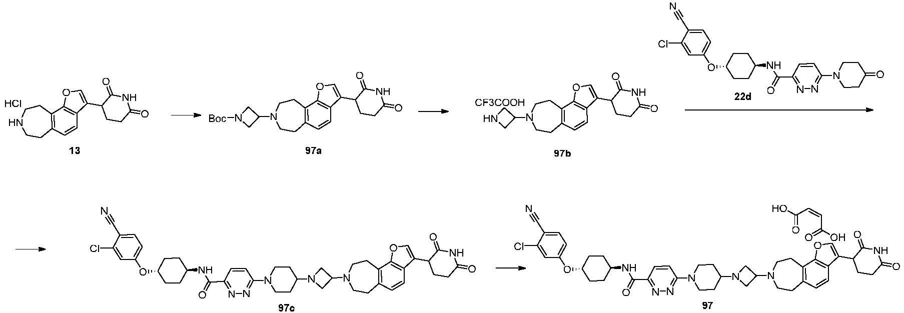

### Step 1: Preparation of intermediate 97a

Intermediate **13** (96 mg), 1-Boc-3-azetidinone (93 mg), sodium acetate (14.44 mg), 1,2-dichloroethane (5 mL), isopropanol (1 mL), and sodium cyanoborohydride (33.6 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give compound intermediate **97a** (112 mg).

### Step 2: Preparation of intermediate 97b

Intermediate **97a** (110 mg), DCM (10 mL), and trifluoroacetic acid (2 mL) were added to a reaction flask in sequence, and the mixture was reacted at room temperature. After the reaction was completed, the mixture was concentrated to remove the solvent, thus giving intermediate **97b** (142 mg).

### Step 3: Preparation of intermediate 97c

Intermediate **22d** (92 mg), intermediate **97b** (90 mg), sodium acetate (12.44 mg), 1,2-dichloroethane (5 mL), isopropanol (1 mL), and sodium cyanoborohydride (33.6 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give compound intermediate 97c (36 mg).

MS(ESI, [M+H]⁺) m/z: 791.3.

### Step 4: Preparation of compound 97

**97c** (42 mg), dichloromethane (10 mL), and methanol (1 mL) were added to a reaction flask in sequence. After complete dissolution by stirring, maleic acid (6.73 mg) was added, and the mixture was stirred at room temperature for 10 min. The mixture was concentrated and then slurried with petroleum ether to give compound **97** (33 mg). ¹H NMR (500 MHz, DMSO-d6) δ 10.88 (s, 1H), 8.61 (d, *J=* 8.2 Hz, 1H), 7.86 (q, *J=* 5.0, 4.1 Hz, 3H), 7.49 - 7.27 (m, 3H), 7.10 (dd, *J =* 34.4, 8.4 Hz, 2H), 6.16 (s, 2H), 4.66 - 4.49 (m, 3H), 4.27 - 4.01 (m, 5H), 3.92 - 3.81 (m, 1H), 3.23 - 2.99 (m, 8H), 2.74 (ddd, *J* = 17.6, 12.4, 5.4 Hz, 1H), 2.55 (s, 5H), 2.38 - 2.27 (m, 1H), 2.18 - 1.99 (m, 5H), 1.98 - 1.84 (m, 2H), 1.65 (q, *J* = 12.5 Hz, 2H), 1.52 (q, *J =* 12.5, 12.1 Hz, 2H), 1.42 - 1.28 (m, 2H).

### Example 98: Synthesis of Compound 98

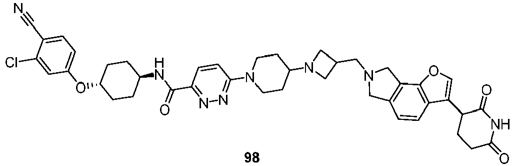

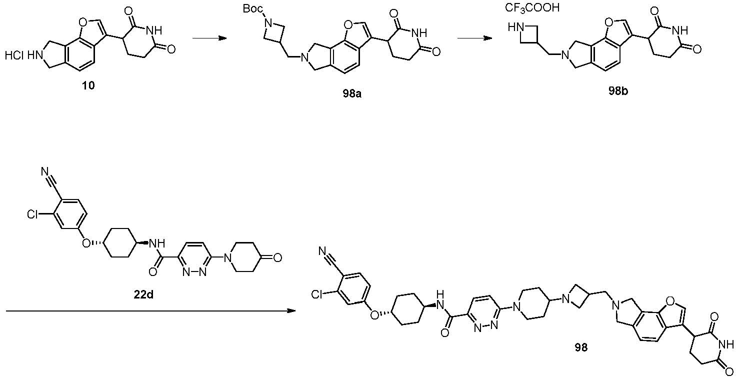

### Step 1: Preparation of intermediate 98a

Intermediate **10** (120 mg), *tert-butyl* 3-formylazetidine-1-carboxylate (188 mg), sodium acetate (73 mg), 1,2-dichloroethane (10 mL), and isopropanol (2 mL) were added to a reaction flask in sequence, and the mixture was stirred at room temperature for 10 min. Sodium triacetoxyborohydride (188 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **98a** (150 mg).

MS(ESI, [M+H]⁺) m/z: 440.2.

### Step 2: Preparation of intermediate 98b

**98a** (150 mg), dichloromethane (2 mL), and trifluoroacetic acid (2 mL) were added to a reaction flask in sequence, and the mixture was reacted at room temperature for 2 h. After the reaction was completed, the mixture was concentrated to remove the solvent, thus giving **98b.** The crude product was directly used in the next step. MS(ESI, [M+H]⁺) m/z: 340.2.

### Step 3: Preparation of intermediate 98

**98b** (165 mg), compound **22d** (265 mg), sodium acetate (80 mg), and acetonitrile (20 mL) were added to a reaction flask in sequence, and the mixture was stirred at room temperature for 10 min. Sodium cyanoborohydride (61 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered.

The filtrate was concentrated, and then the residue was purified by silica gel column chromatography (dichloromethane/methanol) to give the target compound 98 (30 mg).

MS(ESI, [M+H]⁺) m/z: 777.3.

¹H NMR (500 MHz, DMSO-d6) δ 10.88 (s, 1H), 8.58 (d, *J=* 8.2 Hz, 1H), 7.85 *(d, J=* 7.8 Hz, 2H), 7.81 (d, *J=* 9.5 Hz, 1H), 7.42 (d, *J =* 7.9 Hz, 1H), 7.40 - 7.31 (m, 2H), 7.17 - 7.09 (m, 2H), 4.53 (tt, *J =* 9.8, 4.2 Hz, 1H), 4.26 - 4.10 (m, 3H), 4.07 (s, 2H), 3.95 (s, 2H), 3.91 - 3.80 (m, 1H), 3.44 (t, *J* = 35.0 Hz, 2.5H), 3.27 (d*, J* = 11.6 Hz, 2.5H), 2.90 (d, *J=* 7.4 Hz, 3H), 2.79 - 2.53 (m, 4H), 2.31 (qd, *J=* 12.5, 4.4 Hz, 2H), 2.15 - 2.05 (m, 3H), 1.95 - 1.85 (m, 2H), 1.74 (s, 2H), 1.69 - 1.45 (m, 5H).

### Example 99: Synthesis of Compound 99

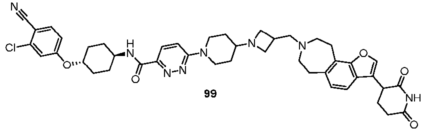

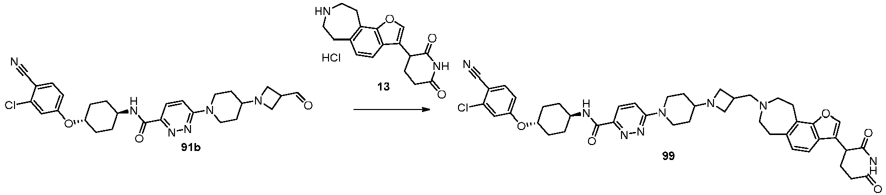

### Step 1: Preparation of compound 99

Intermediate **91b** (105 mg), intermediate **13** (92 mg), sodium acetate (16.44 mg), 1,2-dichloroethane (5 mL), isopropanol (1 mL), and sodium cyanoborohydride (40.2 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give compound **99** (42 mg).

MS (ESI, [M+H]⁺) *m*/*z:* 805.3.

¹H NMR (500 MHz, DMSO-d6) δ 10.87 (s, 1H), 8.58 (d, *J=* 8.2 Hz, 1H), 7.89 - 7.77 (m, 3H), 7.39 (d, *J* = 2.4 Hz, 1H), 7.33 (d, *J=* 9.6 Hz, 1H), 7.26 (d, *J=* 7.9 Hz, 1H), 7.13 (dd, *J* = 8.8, 2.5 Hz, 1H), 7.01 *(d, J=* 7.9 Hz, 1H), 4.53 (tt, *J=* 10.0, 4.2 Hz, 1H), 4.16 (d, *J* = 12.8 Hz, 2H), 4.08 (dd, *J=* 11.7, 5.0 Hz, 1H), 3.85 (dtt, *J=* 11.7, 8.2, 4.2 Hz, 1H), 3.25 (t, *J =* 11.0 Hz, 2H), 3.09 (dd, *J =* 6.6, 3.3 Hz, 2H), 2.94 (dd, *J =* 6.6, 3.4 Hz, 2H), 2.73 (ddd, *J =* 17.3, 12.1, 5.4 Hz, 3H), 2.69 - 2.52 (m, 8H), 2.30 (qd, *J=* 12.7, 12.3, 4.4 Hz, 2H), 2.15 - 2.05 (m, 3H), 1.95 - 1.86 (m, 2H), 1.76 - 1.59 (m, 4H), 1.57 - 1.46 (m, 2H), 1.19 (q, *J=* 9.7 Hz, 3H).

### Example 100: Synthesis of Compound 100

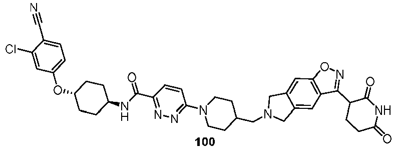

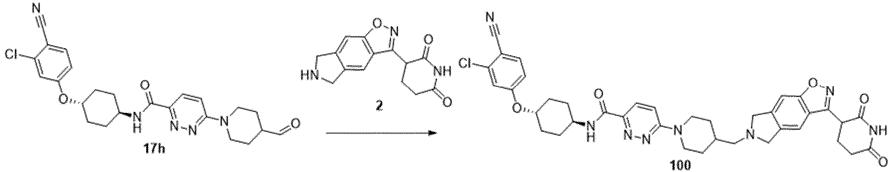

### Step 1: Preparation of compound 100

Intermediate **17h** (125 mg), intermediate **2** (102 mg), sodium acetate (22.52 mg), 1,2-dichloroethane (5 mL), isopropanol (1 mL), and sodium cyanoborohydride (44.5 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give compound **100** (78 mg).

MS (ESI, [M+H]⁺) *m*/*z:* 723.1.

¹H NMR (500 MHz, DMSO-d6) δ 11.08 (s, 1H), 8.57 *(d, J=* 8.2 Hz, 1H), 7.85 *(d, J=* 8.8 Hz, 1H), 7.80 (d, *J=* 9.5 Hz, 1H), 7.63 *(d, J=* 18.2 Hz, 2H), 7.40 - 7.32 (m, 2H), 7.13 (dd, *J=* 8.8, 2.4 Hz, 1H), 4.59 - 4.46 (m, 4H), 3.94 (s, 2H), 3.92 - 3.82 (m, 3H), 3.17 (d, *J =* 5.3 Hz, 2H), 3.06 (t, *J =* 12.4 Hz, 2H), 2.78 (ddd, *J =* 17.2, 11.9, 5.3 Hz, 1H), 2.60 (dd, *J* = 17.6, 5.3 Hz, 3H), 2.47 (dd, *J* = 12.1, 4.3 Hz, 1H), 2.20 (td, *J =* 8.7, 8.0, 3.7 Hz, 1H), 2.11 *(d, J=* 11.2 Hz, 2H), 1.91 (d, *J =* 8.9 Hz, 5H), 1.69 - 1.59 (m, 2H), 1.57 - 1.47 (m, 2H).

### Example 101: Synthesis of Compound 101

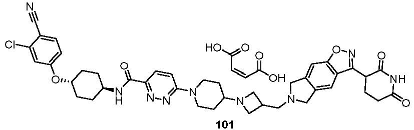

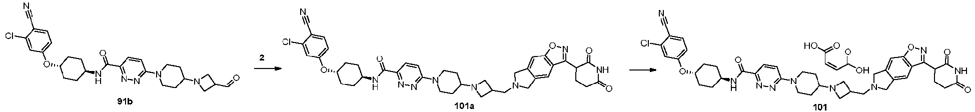

### Step 1: Preparation of intermediate 101a

Intermediate **91b** (105 mg), intermediate **2** (97 mg), sodium acetate (22.52 mg), 1,2-dichloroethane (5 mL), isopropanol (1 mL), and sodium cyanoborohydride (41.5 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give intermediate **101a** (45 mg).

MS(ESI, [M+H]⁺) *m*/*z:* 778.2*.*

### Step 2: Preparation of compound 101

**101a** (43 mg), dichloromethane (10 mL), and methanol (1 mL) were added to a reaction flask in sequence. After complete dissolution by stirring, maleic acid (6.86 mg) was added, and the mixture was stirred at room temperature for 10 min. The mixture was concentrated and then slurried with petroleum ether to give compound **101** (35 mg). MS (ESI, [M+H]⁺) *m*/*z:* 778.3.

¹H NMR (500 MHz, DMSO-d6) δ 11.08 (s, 1H), 9.78 (s, 1H), 8.61 (d, *J =* 8.2 Hz, 1H), 7.87 (dd, *J=* 9.1, 6.4 Hz, 2H), 7.65 (d, *J =* 14.8 Hz, 2H), 7.44 (d, *J* = 9.7 Hz, 1H), 7.38 (d, *J =* 2.5 Hz, 1H), 7.13 (dd, *J* = 8.8, 2.5 Hz, 1H), 6.04 (s, 2H), 4.55 (dtd, *J* = 17.7, 10.0, 4.4 Hz, 4H), 4.21 (d, *J =* 10.0 Hz, 2H), 4.04 - 3.84 (m, 7H), 3.02 (dd, *J =* 23.8, 10.9 Hz, 6H), 2.78 (ddd, *J* = 17.2, 12.0, 5.4 Hz, 1H), 2.61 (dd, *J* = 17.3, 4.2 Hz, 1H), 2.49 - 2.42 (m, 1H), 2.20 (dq, *J* = 13.6, 4.8 Hz, 1H), 2.16 - 2.00 (m, 4H), 1.97 - 1.86 (m, 2H), 1.64 (qd, *J* = 13.2, 3.2 Hz, 2H), 1.57 - 1.48 (m, 2H), 1.30 (qd, *J* = 12.1, 4.1 Hz, 2H).

### Example 102: Synthesis of Compound 102

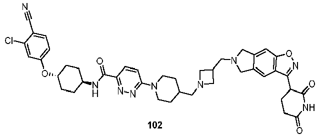

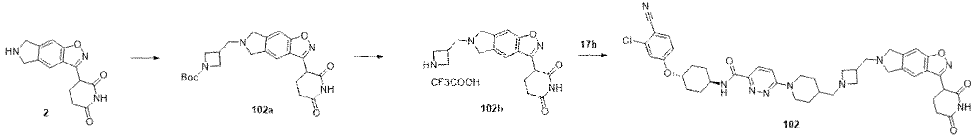

### Step 1: Preparation of intermediate 102a

Intermediate **2** (400 mg), *tert-butyl* 3-formylazetidine-1-carboxylate (360 mg), sodium acetate (213 mg), 1,2-dichloroethane (20 mL), isopropanol (4 mL), and sodium triacetoxyborohydride (520 mg) were added to a reaction flask in sequence, and the mixture was reacted at room temperature for 1 h. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give intermediate **102a** (410 mg).

MS(ESI, [M+H]⁺) *m*/*z:* 385.1*.*

### Step 2: Preparation of intermediate 102b

Intermediate **102a** (410 mg), dichloromethane (20 mL), and trifluoroacetic acid (5 mL) were added to a reaction flask in sequence, and the mixture was reacted at room temperature for 1 h. After the reaction was completed, the reaction solution was concentrated by evaporation under reduced pressure to remove the solvent, thus giving the target intermediate **102b** (642 mg).

### Step 3: Preparation of compound 102

Intermediate **102b** (225 mg), intermediate **17h** (125 mg), sodium acetate (26.52 mg), 1,2-dichloroethane (5 mL), isopropanol (1 mL), and sodium cyanoborohydride (44.5 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give compound **102** (86 mg).

MS(ESI, [M+H]⁺) *m*/*z:* 792.1.

¹H NMR (500 MHz, DMSO-d6) δ 11.07 (s, 1H), 8.56 (d, *J =* 8.2 Hz, 1H), 7.82 (dd, *J=* 29.7, 9.1 Hz, 2H), 7.61 (d, *J=* 21.5 Hz, 2H), 7.42 - 7.28 (m, 2H), 7.13 (dd, *J =* 8.8, 2.5 Hz, 1H), 4.59 - 4.41 (m, 4H), 3.87 (d, *J =* 24.2 Hz, 5H), 2.98 (t, *J* = 12.6 Hz, 2H), 2.92 - 2.71 (m, 5H), 2.68 - 2.57 (m, 2H), 2.46 (dd, *J=* 12.2, 4.4 Hz, 1H), 2.30 (s, 2H), 2.18 (dq, *J =* 13.6, 4.7 Hz, 1H), 2.15 - 2.05 (m, 2H), 1.94 - 1.85 (m, 2H), 1.76 (d, *J =* 12.8 Hz, 2H), 1.69 - 1.58 (m, 3H), 1.52 (td, *J* = 13.1, 6.3 Hz, 2H), 1.17 - 1.05 (m, 2H).

### Example 103: Synthesis of Compound 103

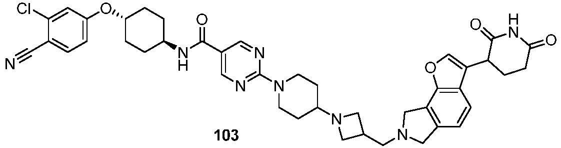

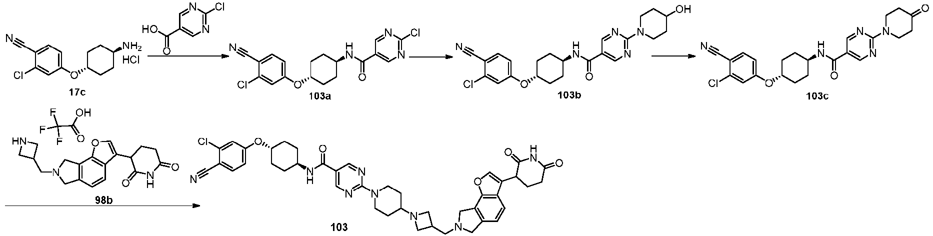

### Step 1: Preparation of intermediate 103a

Intermediate **17c** (10 g), 2-chloropyrimidine-5-carboxylic acid (6.62 g), DCM (200 mL), HATU (15.89 g), and DIPEA (18.00 g, 24.33 mL) were added to a reaction flask in sequence, and the mixture was reacted at room temperature. After the reaction was completed, the reaction solution was concentrated by evaporation under reduced pressure to remove the solvent and extracted with ethyl acetate (500 mL) and water (1000 mL). The organic phase was separated, washed with saturated brine, dried over anhydrous sodium sulfate, and filtered, and the filtrate was concentrated, slurried with EA/PE (v:v = 5/1, 50 mL), and filtered. The filter cake was collected and dried under reduced pressure to give the target intermediate **103a** (6.72 g).

### Step 2: Preparation of intermediate 103b

Intermediate **103a** (2 g), 4-hydroxypiperidine (0.620 g), DIPEA (1.982 g, 2.68 mL), and DMSO (20 mL) were added to a reaction flask in sequence, and the mixture was heated to 100 °C and reacted. After the reaction was completed, the reaction solution was extracted with ethyl acetate (100 mL) and water (200 mL). The organic phase was separated, washed with saturated brine, dried over anhydrous sodium sulfate, and filtered, and the filtrate was concentrated, slurried with ethyl acetate, and filtered. The filter cake was collected and dried under reduced pressure to give the target intermediate **103b** (2.17 g).

MS(ESI, [M+H]⁺) m/z: 456.2.

### Step 3: Preparation of intermediate 103c

Intermediate **103b** (2.07 g), DCM (30 mL), and Dess-Martin periodinane (3.85 g) were added to a reaction flask in sequence, and the mixture was reacted at room temperature. After the reaction was completed, the reaction solution was quenched with a saturated sodium thiosulfate solution (100 mL) and a sodium bicarbonate solution (100 mL) and extracted with dichloromethane (100 mL). The organic phase was separated, washed with saturated brine, dried over anhydrous sodium sulfate, and filtered, and the filtrate was purified by silica gel column chromatography to give the target intermediate **103c** (1.64 g).

MS(ESI, [M-H]⁻) m/z: 452.2.

### Step 4: Preparation of compound 103

Intermediate **98b** (332 mg), intermediate **103c** (169 mg), sodium acetate (305 mg), acetonitrile (10 mL), and sodium cyanoborohydride (46.7 mg) were added to a reaction flask in sequence, and the mixture was heated to 80 °C and reacted. After the reaction was completed, dichloromethane (50 mL) and water (100 mL) were added for extraction. The organic phase was separated, dried over anhydrous sodium sulfate, and filtered, and the filtrate was purified by silica gel column chromatography to give compound **103** (64 mg).

MS(ESI, [M+H]⁺) m/z: 777.1.

¹H NMR (500 MHz, DMSO-d6) δ 10.89 (s, 1H), 8.76 (s, 2H), 8.13 (d, J = 7.4 Hz, 1H), 7.86 (d, J = 8.1 Hz, 2H), 7.50 - 7.30 (m, 2H), 7.14 (t, J = 6.7 Hz, 2H), 4.50 (d, J = 51.1 Hz, 3H), 4.13 (dd, J = 11.8, 4.9 Hz, 1H), 4.08 (s, 2H), 3.96 (s, 2H), 3.80 (s, 1H), 3.55 (s, 2H), 3.19 (d, J = 22.7 Hz, 3H), 3.13 - 2.80 (m, 4H), 2.73 (td, J = 19.3, 13.1, 6.4 Hz, 2H), 2.57 (d, J = 18.8 Hz, 1H), 2.31 (q, J = 12.9, 11.8 Hz, 1H), 2.10 (s, 3H), 1.92 (d, J = 9.0 Hz, 2H), 1.76 (s, 2H), 1.50 (q, J = 9.3 Hz, 4H), 1.23 - 1.04 (m, 2H).

### Example 104: Synthesis of Compound 104

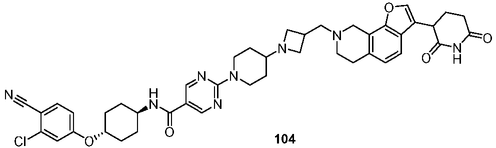

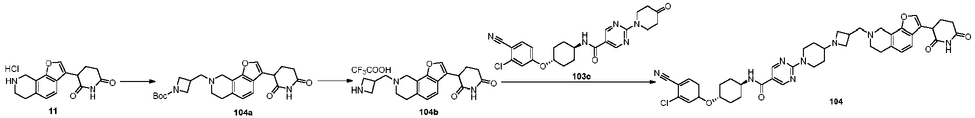

### Step 1: Preparation of intermediate 104a

Intermediate **11** (150 mg), *tert-butyl* 3-formylazetidine-1-carboxylate (173 mg), sodium acetate (38.4 mg), 1,2-dichloroethane (5 mL), isopropanol (1 mL), and sodium triacetoxyborohydride (198 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give the target intermediate **104a** (225 mg).

MS(ESI, [M+H]⁺) m/z: 454.2.

### Step 2: Preparation of intermediate 104b

Intermediate **104a** (225 mg), DCM (5 mL), and TFA (1480 mg, 1 mL) were added to a reaction flask in sequence, and the mixture was reacted at room temperature. After the reaction was completed, the reaction solution was concentrated by evaporation under reduced pressure to remove the solvent, thus giving the target intermediate **104b** (475 mg).

MS(ESI, [M+H]⁺) m/z: 354.2.

### Step 3: Preparation of compound 104

Intermediate **104b** (475 mg), intermediate **103c** (232 mg), sodium acetate (209 mg), acetonitrile (10 mL), and sodium cyanoborohydride (96 mg) were added to a reaction flask in sequence, and the mixture was heated to 80 °C and reacted. After the reaction was completed, dichloromethane (50 mL) and water (100 mL) were added for extraction. The organic phase was separated, dried over anhydrous sodium sulfate, and filtered, and the filtrate was purified by silica gel column chromatography to give compound **104** (81 mg).

MS(ESI, [M+H]⁺) m/z: 791.1.

¹H NMR (500 MHz, DMSO-d6) δ 10.87 (s, 1H), 8.76 (s, 2H), 8.13 (d, J = 7.4 Hz, 1H), 7.89 - 7.78 (m, 2H), 7.42 - 7.30 (m, 2H), 7.14 (d, J = 8.9 Hz, 1H), 6.99 (d, J = 8.1 Hz, 1H), 4.62 - 4.35 (m, 3H), 4.10 (dd, J = 11.9, 5.0 Hz, 1H), 3.78 (d, J = 19.6 Hz, 5H), 3.25 - 3.11 (m, 3H), 2.94 - 2.66 (m, 9H), 2.59 - 2.55 (m, 1H), 2.38 - 2.02 (m, 5H), 1.91 (d, J = 9.0 Hz, 2H), 1.77 (s, 2H), 1.50 (q, J = 9.4 Hz, 4H), 1.23 - 1.11 (m, 2H).

### Example 105: Synthesis of Compound 105

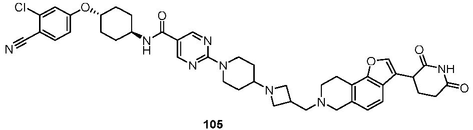

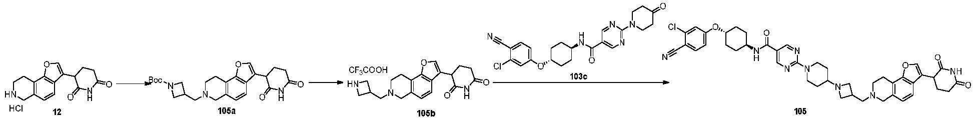

### Step 1: Preparation of intermediate 105a

Intermediate **12** (150 mg), *tert-butyl* 3-formylazetidine-1-carboxylate (173 mg), sodium acetate (38.4 mg), 1,2-dichloroethane (5 mL), isopropanol (1 mL), and sodium triacetoxyborohydride (198 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give the target intermediate **105a** (240 mg).

MS(ESI, [M+H]⁺) m/z: 454.2.

### Step 2: Preparation of intermediate 105b

Intermediate **105a** (240 mg), DCM (5 mL), and TFA (1480 mg, 1 mL) were added to a reaction flask in sequence, and the mixture was reacted at room temperature. After the reaction was completed, the reaction solution was concentrated by evaporation under reduced pressure to remove the solvent, thus giving the target intermediate **105b** (530 mg).

MS(ESI, [M+H]⁺) m/z: 354.2.

### Step 3: Preparation of compound 105

Intermediate **105b** (530 mg), intermediate **103c** (217 mg), sodium acetate (196 mg), acetonitrile (10 mL), and sodium cyanoborohydride (90 mg) were added to a reaction flask in sequence, and the mixture was heated to 80 °C and reacted. After the reaction was completed, dichloromethane (50 mL) and water (100 mL) were added for extraction. The organic phase was separated, dried over anhydrous sodium sulfate, and filtered, and the filtrate was purified by silica gel column chromatography to give compound **105** (60 mg).

MS(ESI, [M+H]⁺) m/z: 791.1.

¹H NMR (500 MHz, DMSO-d6) δ 10.87 (s, 1H), 8.74 (s, 2H), 8.11 (d, J = 7.5 Hz, 1H), 7.89 - 7.80 (m, 2H), 7.38 (d, J = 2.4 Hz, 1H), 7.32 (d, J = 7.9 Hz, 1H), 7.14 (dd, J = 8.8, 2.4 Hz, 1H), 6.94 (d, J = 8.1 Hz, 1H), 4.55 (dq, J = 9.6, 4.4 Hz, 1H), 4.37 (d, J = 12.8 Hz, 2H), 4.10 (dd, J = 11.8, 4.9 Hz, 1H), 3.83 - 3.75 (m, 1H), 3.62 (s, 2H), 3.42 (s, 2H), 3.24 (d, J = 10.9 Hz, 3H), 2.94 (t, J = 5.8 Hz, 2H), 2.86 (s, 1H), 2.77 - 2.65 (m, 6H), 2.59 - 2.54 (m, 1H), 2.31 (ddd, J = 20.4, 14.8, 10.3 Hz, 2H), 2.10 (dp, J = 9.1, 4.9, 4.5 Hz, 3H), 1.97 - 1.86 (m, 2H), 1.75 - 1.63 (m, 2H), 1.50 (dt, J = 11.0, 6.6 Hz, 4H), 1.13 (q, J = 9.5 Hz, 2H).

### Example 106: Synthesis of Compound 106

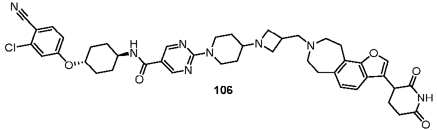

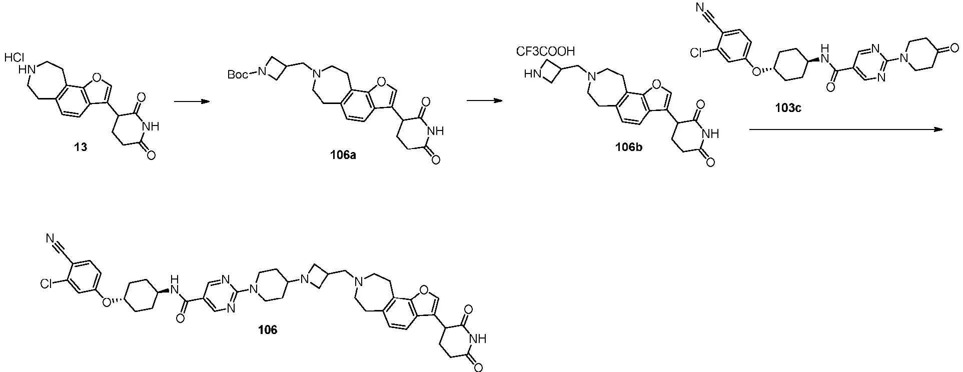

### Step 1: Preparation of intermediate 106a

Intermediate **13** (380 mg), *tert-butyl* 3-formylazetidine-1-carboxylate (360 mg), sodium acetate (213 mg), 1,2-dichloroethane (20 mL), isopropanol (4 mL), and sodium triacetoxyborohydride (520 mg) were added to a reaction flask in sequence, and the mixture was reacted at room temperature for 1 h. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give intermediate **106a** (425 mg).

### Step 2: Preparation of intermediate 106b

Intermediate **106a** (425 mg), dichloromethane (20 mL), and trifluoroacetic acid (5 mL) were added to a reaction flask in sequence, and the mixture was reacted at room temperature for 1 h. After the reaction was completed, the reaction solution was concentrated by evaporation under reduced pressure to remove the solvent, thus giving the target intermediate **106b** (672 mg).

### Step 3: Preparation of compound 106

Intermediate **106b** (210 mg), intermediate **103c** (110 mg), sodium acetate (23.52 mg), 1,2-dichloroethane (5 mL), isopropanol (1 mL), and sodium cyanoborohydride (42.5 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give compound **106** (86 mg).

MS(ESI, [M+H]⁺) *m*/*z:* 805.2.

¹H NMR (500 MHz, DMSO-d6) δ 10.87 (s, 1H), 8.74 (s, 2H), 8.10 (d, *J =* 7.5 Hz, 1H), 7.89 - 7.80 (m, 2H), 7.39 (d, *J=* 2.4 Hz, 1H), 7.26 (d, *J=* 7.8 Hz, 1H), 7.14 (dd, *J =* 8.8, 2.5 Hz, 1H), 7.01 (d, *J =* 8.0 Hz, 1H), 4.55 (dt, *J =* 9.9, 5.4 Hz, 1H), 4.35 (d, *J* = 12.7 Hz, 2H), 4.15 - 4.04 (m, 2H), 3.79 (s, 1H), 3.26 (t, *J =* 11.2 Hz, 2H), 3.09 (dd, *J* = 6.9, 3.3 Hz, 2H), 2.98 - 2.91 (m, 2H), 2.84 - 2.69 (m, 3H), 2.66 (d, *J =* 6.4 Hz, 2H), 2.63 - 2.55 (m, 5H), 2.30 (qd, *J* = 13.1, 12.3, 4.5 Hz, 2H), 2.09 (dp, *J* = 9.3, 5.3, 4.6 Hz, 3H), 1.96 - 1.87 (m, 2H), 1.67 (d, *J* = 12.4 Hz, 2H), 1.58 - 1.44 (m, 4H), 1.11 (dtd, *J=* 13.3, 9.9, 9.3, 3.7 Hz, 2H).

### Example 107: Synthesis of Compound 107

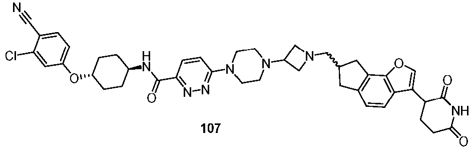

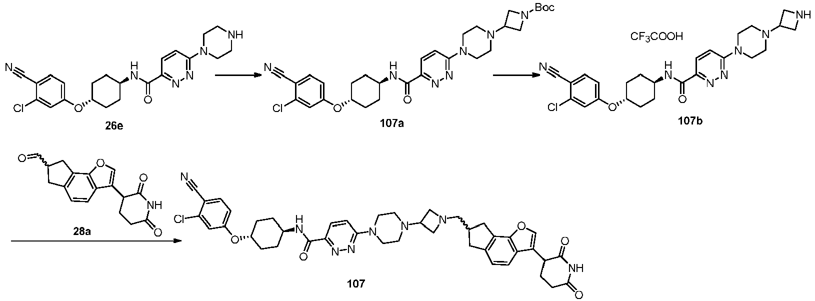

### Step 1: Preparation of intermediate 107a

Intermediate **26e** (700 mg), 1-Boc-3-azetidinone (502 mg), sodium acetate (601 mg), 1,2-dichloroethane (5 mL), isopropanol (1 mL), and sodium cyanoborohydride (184 mg) were added to a reaction flask in sequence, and the mixture was heated to 80 °C and reacted. After the reaction was completed, dichloromethane (50 mL) and water (100 mL) were added for extraction. The organic phase was separated, dried over anhydrous sodium sulfate, and filtered, and the filtrate was purified by silica gel column chromatography to give the target intermediate 107a (290 mg).

MS(ESI, [M+H]⁺) m/z: 596.5.

### Step 2: Preparation of intermediate 107b

Intermediate **107a** (290 mg), DCM (5 mL), and TFA (832 mg, 0.562 mL) were added to a reaction flask in sequence, and the mixture was reacted at room temperature. After the reaction was completed, the reaction solution was concentrated by evaporation under reduced pressure to remove the solvent, thus giving the target intermediate **107b** (487 mg).

### Step 3: Preparation of compound 107

Intermediate **28a** (70 mg), intermediate **107b** (232 mg), sodium acetate (96 mg), 1,2-dichloroethane (5 mL), isopropanol (1 mL), and sodium triacetoxyborohydride (99 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give compound **107** (72 mg).

MS(ESI, [M+H]⁺) m/z: 777.1.

¹H NMR (500 MHz, DMSO-d6) δ 10.87 (s, 1H), 8.60 (d, J = 8.2 Hz, 1H), 7.93 - 7.74 (m, 3H), 7.36 (dd, J = 18.4, 7.5 Hz, 3H), 7.11 (dd, J = 18.5, 8.4 Hz, 2H), 4.59 - 4.48 (m, 1H), 4.10 (dd, J = 11.9, 5.0 Hz, 1H), 3.86 (q, J = 11.1, 9.8 Hz, 1H), 3.77 - 3.51 (m, 6H), 3.22 - 3.13 (m, 2H), 3.12 - 2.96 (m, 3H), 2.87 - 2.68 (m, 4H), 2.65 - 2.53 (m, 3H), 2.40 (s, 4H), 2.29 (tt, J = 12.6, 6.4 Hz, 1H), 2.17 - 2.04 (m, 3H), 1.90 (d, J = 11.3 Hz, 2H), 1.64 (q, J = 12.3 Hz, 2H), 1.51 (q, J = 11.9 Hz, 2H).

### Example 108: Synthesis of Compound 108

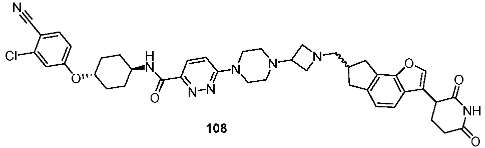

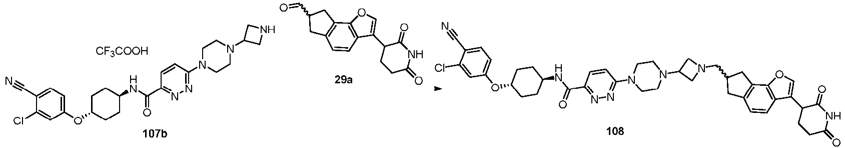

Intermediate **29a** (70 mg), intermediate **107b** (232 mg), sodium acetate (96 mg), 1,2-dichloroethane (5 mL), isopropanol (1 mL), and sodium triacetoxyborohydride (99 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give compound **108** (99 mg).

MS(ESI, [M+H]⁺) m/z: 777.1.

¹H NMR (500 MHz, DMSO-d6) δ 10.87 (s, 1H), 8.60 (d, J = 8.1 Hz, 1H), 7.94 - 7.74 (m, 3H), 7.47 - 7.29 (m, 3H), 7.12 (dd, J = 16.3, 8.4 Hz, 2H), 4.54 (d, J = 10.5 Hz, 1H), 4.11 (dd, J = 12.0, 5.0 Hz, 1H), 3.95 - 3.59 (m, 7H), 3.14 (ddd, J = 47.2, 17.3, 7.5 Hz, 5H), 2.77 (ddt, J = 29.4, 16.6, 5.8 Hz, 4H), 2.66 - 2.53 (m, 3H), 2.42 (s, 4H), 2.33 - 2.25 (m, 1H), 2.17 - 2.04 (m, 3H), 1.90 (d, J = 11.3 Hz, 2H), 1.64 (q, J = 12.3 Hz, 2H), 1.52 (q, J = 12.2 Hz, 2H).

### Example 109: Synthesis of Compound 109

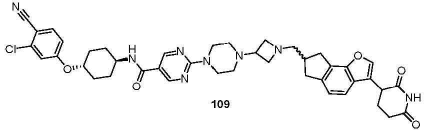

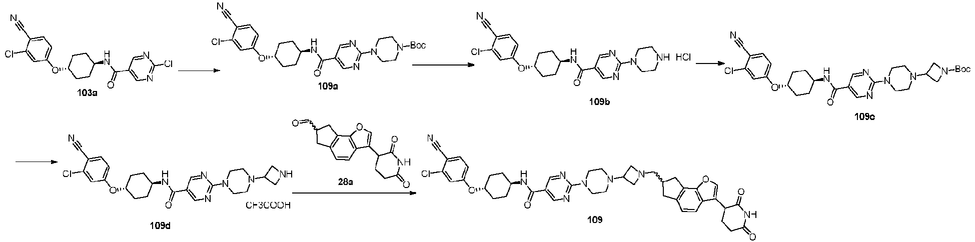

### Step 1: Preparation of intermediate 109a

Intermediate **103a** (1.7 g), 1-Boc-piperazine (1.13 g), DIPEA (1.12 mg), and DMSO (20 mL) were added to a reaction flask in sequence, and the mixture was heated to 100 °C and reacted. After the reaction was completed, ethyl acetate (50 mL) and water (100 mL) were added for extraction. The organic phase was separated, dried over anhydrous sodium sulfate, and filtered, and the filtrate was purified by silica gel column chromatography to give the target intermediate **109a** (1.72 g).

MS(ESI, [M+H]⁺) m/z: 541.2.

### Step 2: Preparation of intermediate 109b

Intermediate **109a** (1.72 g) and a 4 M solution of hydrochloric acid in 1,4-dioxane (30 mL) were added to a reaction flask in sequence, and the mixture was reacted at room temperature. After the reaction was completed, the reaction solution was concentrated by evaporation under reduced pressure to remove the solvent, thus giving the target intermediate **109b** (1.42 g).

MS(ESI, [M+H]⁺) m/z: 441.2.

### Step 3: Preparation of intermediate 109c

Intermediate **109b** (1.2 g), 1-*tert*-butoxycarbonyl-3-aminocyclobutylamine (0.86 g), 1,2-dichloroethane (30 mL), isopropanol (5 mL), sodium acetate (0.31 g), and sodium cyanoborohydride (0.41 g) were added to a reaction flask in sequence, and the mixture was heated to 80 °C and reacted. After the reaction was completed, a saturated sodium bicarbonate solution (5 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give intermediate **109c** (1.2 g).

MS(ESI, [M+H]⁺) m/z: 596.2.

### Step 4: Preparation of intermediate 109d

Intermediate **109c** (1.1 g), DCM (30 mL), and trifluoroacetic acid (10 mL) were added to a reaction flask in sequence, and the mixture was reacted at room temperature. After the reaction was completed, the reaction solution was concentrated by evaporation under reduced pressure to remove the solvent, thus giving the target intermediate **109d** (1.72 g).

### Step 5: Preparation of compound 109

Intermediate **28a** (50 mg), intermediate **109d** (110 mg), 1,2-dichloroethane (5 mL), isopropanol (1 mL), and sodium cyanoborohydride (25.3 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give compound **109** (42 mg).

MS(ESI, [M+H]⁺) *m*/*z:* 777.2*.*

¹H NMR (500 MHz, DMSO-d6) δ 10.87 (s, 1H), 8.76 (s, 2H), 8.13 (d, *J =* 7.5 Hz, 1H), 7.86 (d, *J =* 8.8 Hz, 1H), 7.80 (s, 1H), 7.39 (d, *J=* 2.4 Hz, 1H), 7.33 (d, *J=* 7.8 Hz, 1H), 7.14 (dd, *J =* 8.8, 2.5 Hz, 1H), 7.09 (d, *J =* 7.9 Hz, 1H), 4.55 (dq, *J* = 9.7, 4.5 Hz, 1H), 4.10 (dd, *J =* 11.8, 4.9 Hz, 1H), 3.87 - 3.75 (m, 5H), 3.50 (s, 2H), 3.16 - 3.10 (m, 1H), 3.07 (dd, *J=* 15.6, 6.5 Hz, 1H), 3.02 - 2.84 (m, 3H), 2.82 - 2.69 (m, 3H), 2.56 (dq, *J =* 12.5, 4.1 Hz, 3H), 2.35 - 2.27 (m, *J=* 4.3 Hz, 5H), 2.10 (dd, *J=* 8.8, 4.4 Hz, 3H), 1.91 (s, 3H), 1.50 (dt, *J=* 10.9, 6.7 Hz, 4H).

### Example 110: Synthesis of Compound 110

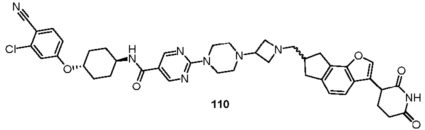

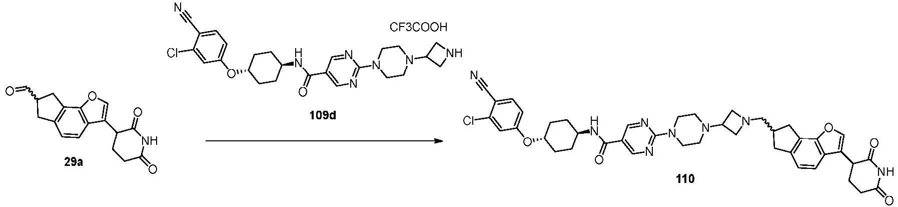

### Step 1: Preparation of compound 110

Intermediate **29a** (50 mg), intermediate **109d** (110 mg), 1,2-dichloroethane (5 mL), isopropanol (1 mL), and sodium cyanoborohydride (25.3 mg) were added to a reaction flask in sequence, and the mixture was reacted at room temperature. After the reaction was completed, a saturated sodium bicarbonate solution (2 mL) was added to the reaction solution to neutralize acetic acid, and then dichloromethane (50 mL) and water (100 mL) were added for extraction. The organic phase was separated, dried over anhydrous sodium sulfate, and filtered, and the filtrate was purified by silica gel column chromatography to give compound **110** (57 mg).

MS(ESI, [M+H]⁺) *m*/*z:* 777.2.

¹H NMR (500 MHz, DMSO-d6) δ 10.87 (s, 1H), 8.76 (s, 2H), 8.13 (d, *J =* 7.5 Hz, 1H), 7.86 (d, *J =* 8.8 Hz, 1H), 7.80 (s, 1H), 7.39 (d, *J=* 2.4 Hz, 1H), 7.33 (d, *J=* 7.8 Hz, 1H), 7.14 (dd, *J =* 8.8, 2.5 Hz, 1H), 7.09 (d, *J =* 7.9 Hz, 1H), 4.54 (dd, *J =* 9.7, 4.6 Hz, 1H), 4.12 - 4.07 (m, 1H), 3.86 - 3.75 (m, 5H), 3.46 (s, 2H), 3.16 - 3.10 (m, 1H), 3.06 (dd, *J=* 15.8, 7.3 Hz, 1H), 2.89 (d, *J* = 20.9 Hz, 3H), 2.82 - 2.69 (m, 3H), 2.56 (dt, *J =* 17.8, 4.4 Hz, 3H), 2.35 - 2.26 (m, 5H), 2.10 (dd, *J=* 8.5, 4.2 Hz, 3H), 1.96 - 1.88 (m, 2H), 1.50 (dt, *J =* 11.2, 6.7 Hz, 4H).

### Example 111: Synthesis of Compound 111

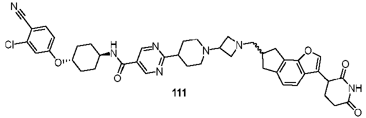

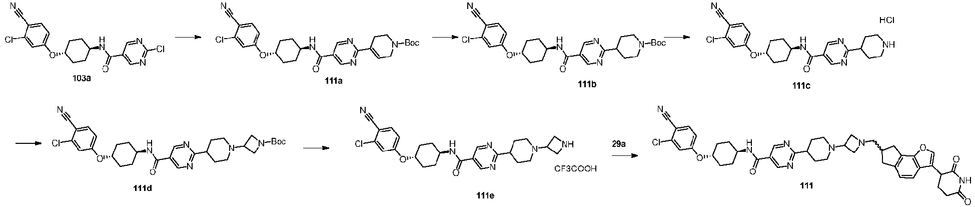

### Step 1: Preparation of intermediate 111a

**103a** (1.7 g), 4-BOC amino-1-cyclohexene boronic acid pinacol ester (1.6 g), [1,1'-bis(diphenylphosphino)ferrocene]palladium dichloride dichloromethane complex (0.31 g), potassium carbonate (1.2 g), 1,4-dioxane (50 mL), and water (5 mL) were added to a reaction flask in sequence, and the mixture was heated to 100 °C and reacted under N₂ atmosphere. After the reaction was completed, ethyl acetate (200 mL) and water (200 mL) were added for extraction. The organic phase was separated, dried over anhydrous sodium sulfate, and filtered, and the filtrate was purified by silica gel column chromatography to give the target intermediate **111a** (1.78 g).

### Step 2: Preparation of intermediate 111b

**111a** (1.78 g), 10% palladium on carbon (0.52 g), methanol (10 mL), and ethyl acetate (30 mL) were added to a reaction flask in sequence, and the mixture was purged three times with hydrogen and reacted overnight under hydrogen atmosphere. After the reaction was completed, ethyl acetate (100 mL) and water (100 mL) were added for extraction. The organic phase was separated, dried over anhydrous sodium sulfate, and filtered, and the filtrate was purified by silica gel column chromatography to give the target intermediate **111b** (1.02 g).

MS(ESI, [M+H]⁺) *m*/*z*: 540.1*.*

### Step 3: Preparation of intermediate 111c

**111b** (1.02 g) and a 4 M solution of hydrochloric acid in 1,4-dioxane (30 mL) were added to a reaction flask in sequence, and the mixture was reacted for 2 h. After the reaction was completed, the mixture was concentrated by evaporation under reduced pressure to remove the solvent, thus giving the target intermediate **111c** (1.02 g). MS(ESI, [M+H]⁺) *m*/*z*: 440.1*.*

### Step 4: Preparation of intermediate 111d

Intermediate **111c** (400 mg), 1-*tert*-butoxycarbonyl-3-aminocyclobutylamine (287 mg), sodium acetate (138 mg), 1,2-dichloroethane (25 mL), isopropanol (5 mL), and sodium cyanoborohydride (106 mg) were added to a reaction flask in sequence, and the mixture was heated to 80 °C and reacted for 2 h. After the reaction was completed, a saturated sodium bicarbonate solution (20 mL) was added to the reaction solution to neutralize acetic acid, and then dichloromethane (100 mL) and water (100 mL) were added for extraction. The organic phase was separated, dried over anhydrous sodium sulfate, and filtered, and the filtrate was purified by silica gel column chromatography to give intermediate **111d** (310 mg).

MS(ESI, [M+H]⁺) *m*/*z:* 595.2.

### Step 5: Preparation of intermediate 111e

Intermediate **111d** (300 mg), dichloromethane (20 mL), and trifluoroacetic acid (10 mL) were added to a reaction flask in sequence, and the mixture was reacted at room temperature for 1 h. After the reaction was completed, the reaction solution was concentrated by evaporation under reduced pressure to remove the solvent, thus giving the target intermediate **111e** (572 mg).

MS(ESI, [M+H]⁺) *m*/*z:* 495.2.

### Step 6: Preparation of compound 111

Intermediate **111e** (200 mg), 29a (95 mg), sodium acetate (138 mg), 1,2-dichloroethane (10 mL), isopropanol (2 mL), and sodium cyanoborohydride (56 mg) were added to a reaction flask in sequence, and the mixture was reacted at room temperature. After the reaction was completed, a saturated sodium bicarbonate solution (10 mL) was added to the reaction solution to neutralize acetic acid, and then dichloromethane (50 mL) and water (50 mL) were added for extraction. The organic phase was separated, dried over anhydrous sodium sulfate, and filtered, and the filtrate was purified by silica gel column chromatography to give compound 111 (45 mg).

MS(ESI, [M+H]⁺) *m*/*z*: 776.6.

¹H NMR (500 MHz, DMSO-d6) δ 10.87 (s, 1H), 9.08 (s, 2H), 8.58 (d, *J =* 7.5 Hz, 1H), 7.86 (d, *J =* 8.7 Hz, 1H), 7.81 (s, 1H), 7.40 (d, *J=* 2.5 Hz, 1H), 7.34 (d, *J=* 7.8 Hz, 1H), 7.15 (dd, *J =* 8.8, 2.4 Hz, 1H), 7.10 (d, *J =* 7.9 Hz, 1H), 4.57 (dq, *J=* 10.0, 4.5 Hz, 1H), 4.10 (dd, *J=* 11.9, 4.9 Hz, 1H), 3.90 - 3.79 (m, 1H), 3.51 (s, 2H), 3.16 (d, *J=* 8.6 Hz, 1H), 3.08 (dd, *J =* 15.7, 7.5 Hz, 1H), 3.02 - 2.66 (m, 9H), 2.56 (dt, *J=* 17.3, 4.2 Hz, 4H), 2.30 (qd, *J =* 12.5, 4.5 Hz, 1H), 2.10 (td, *J =* 8.9, 4.4 Hz, 3H), 1.95 (d, *J =* 13.0 Hz, 6H), 1.79 (d, *J =* 13.2 Hz, 2H), 1.60 - 1.47 (m, 4H).

### Example 112: Synthesis of Compound 112

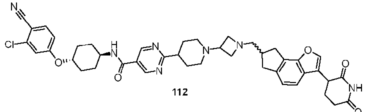

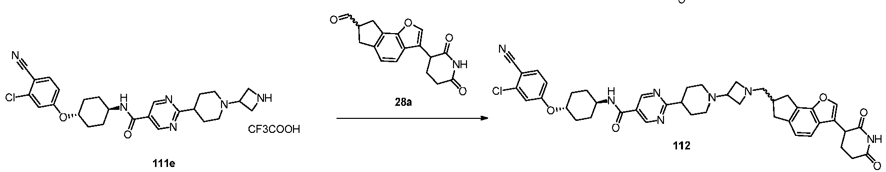

### Step 1: Preparation of compound 112

Intermediate **111e** (180 mg), **28a** (85 mg), sodium acetate (132 mg), 1,2-dichloroethane (10 mL), isopropanol (2 mL), and sodium cyanoborohydride (55 mg) were added to a reaction flask in sequence, and the mixture was reacted at room temperature. After the reaction was completed, a saturated sodium bicarbonate solution (10 mL) was added to the reaction solution to neutralize acetic acid, and then dichloromethane (50 mL) and water (50 mL) were added for extraction. The organic phase was separated, dried over anhydrous sodium sulfate, and filtered, and the filtrate was purified by silica gel column chromatography to give compound **112** (57 mg).

MS(ESI, [M+H]⁺) *m*/*z:* 776.6.

¹H NMR (500 MHz, DMSO-d6) δ 10.87 (s, 1H), 9.08 (d, *J =* 2.7 Hz, 2H), 8.57 (d, *J =* 7.3 Hz, 1H), 7.86 (dd, *J* = 8.7, 2.1 Hz, 1H), 7.80 *(d, J=* 2.4 Hz, 1H), 7.45 - 7.38 (m, 1H), 7.33 (d, *J* = 7.7 Hz, 1H), 7.20 - 7.13 (m, 1H), 7.09 (d, *J=* 8.0 Hz, 1H), 4.57 (td, *J=* 9.5, 4.6 Hz, 1H), 4.10 (dd, *J=* 11.7, 5.0 Hz, 1H), 3.92 - 3.79 (m, 1H), 3.50 (s, 2H), 3.15 (dd, *J=* 14.4, 6.2 Hz, 1H), 3.07 (dd, *J =* 15.7, 7.0 Hz, 1H), 2.98 - 2.68 (m, 9H), 2.63 - 2.52 (m, 4H), 2.38 - 2.25 (m, 1H), 2.19 - 2.06 (m, 3H), 1.93 (d, *J=* 14.9 Hz, 6H), 1.78 (q, *J =* 12.0 Hz, 2H), 1.61 - 1.47 (m, 4H).

### Example 113: Synthesis of Compound 113

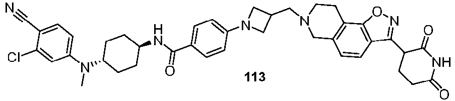

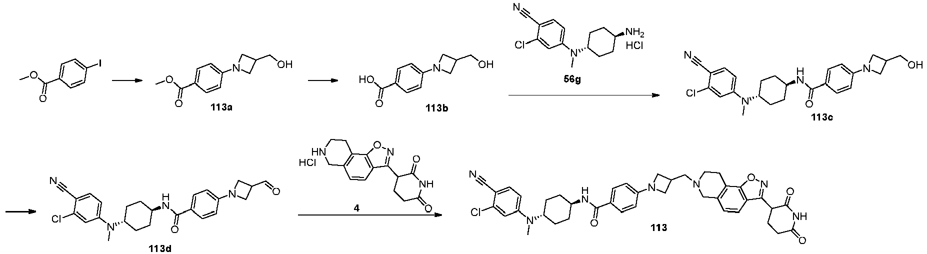

### Step 1: Preparation of intermediate 113a

Methyl 4-iodobenzoate (5.0 g), 3-azetidinemethanol hydrochloride (2.83 g), L-proline (0.88 g), copper(I) iodide (0.73 g), potassium carbonate (13.18 g), and dimethyl sulfoxide (50 mL) were added to a reaction flask in sequence, and the mixture was reacted at 100 °C for 3 h. After the reaction was completed, the reaction solution was extracted with water and ethyl acetate, followed by liquid separation. The organic phase was washed with saturated brine, dried over anhydrous sodium sulfate, and filtered, and the filtrate was concentrated. The crude product was separated by silica gel column chromatography to give intermediate **113a** (3.32 g).

MS(ESI, [M+H]⁺) *m*/*z:* 222.2*.*

### Step 2: Preparation of intermediate 113b

**113a** (3.4 g), methanol (50 mL), water (10 mL), and sodium hydroxide (1.84 g) were added to a reaction flask in sequence, and the mixture was reacted at room temperature for 2 h. After the reaction was completed, the reaction solution was added with water, and the pH was adjusted to about 3 with hydrochloric acid. The mixture was filtered under vacuum, and the filter cake was dried by air blasting to give intermediate **113b** (2.32 g).

MS(ESI, [M+H]⁺) *m*/*z:* 208.1*.*

### Step 3: Preparation of intermediate 113c

**113b** (0.5 g), **56g** (0.72 g), HATU (1.3 g), DIPEA (1.2 g), and DMF (20 mL) were added to a reaction flask in sequence, and the mixture was reacted at room temperature. After the reaction was completed, ethyl acetate (70 mL) and water (150 mL) were added. The organic phase was separated, washed with a saturated sodium chloride solution, dried over anhydrous sodium sulfate, and filtered, and the concentrate was separated and purified by silica gel column chromatography to give intermediate **113c** (0.75 g).

MS(ESI, [M+H]⁺) *m*/*z:* 453.2*.*

### Step 4: Preparation of intermediate 113d

**113c** (0.3 g), Dess-Martin periodinane (0.84 g), and dichloromethane (30 mL) were added to a reaction flask in sequence, and the mixture was reacted at room temperature for 1 h. After the reaction was completed, dichloromethane (70 mL) and water (100 mL) were added. The organic phase was separated, washed with a saturated sodium chloride solution, dried over anhydrous sodium sulfate, filtered, and concentrated to give intermediate **113d** (0.4 g).

### Step 5: Preparation of compound 113

Intermediate **113d** (112 mg), compound **4** (80 mg), sodium acetate (40.8 mg), 1,2-dichloroethane (10 mL), isopropanol (2 mL), and sodium cyanoborohydride (31 mg) were added to a reaction flask in sequence, and the mixture was reacted at room temperature. After the reaction was completed, a saturated sodium bicarbonate solution (10 mL) was added to the reaction solution to neutralize acetic acid, and then dichloromethane (50 mL) and water (50 mL) were added for extraction. The organic phase was separated, dried over anhydrous sodium sulfate, and filtered, and the filtrate was purified by silica gel column chromatography to give compound 113 (65 mg).

MS(ESI, [M+H]⁺) *m*/*z:* 720.2*.*

¹H NMR (500 MHz, DMSO-*d*₆) δ 11.08 (s, 1H), 7.87 (d, *J =* 7.6 Hz, 1H), 7.76 - 7.69 (m, 2H), 7.59 (dd, *J =* 8.6, 5.8 Hz, 2H), 7.10 (d, *J=* 8.3 Hz, 1H), 6.94 (d, *J=* 2.6 Hz, 1H), 6.83 (dd, *J =* 9.1, 2.6 Hz, 1H), 6.44 - 6.38 (m, 2H), 4.56 (dd, *J=* 11.9, 5.0 Hz, 1H), 4.02 (t, *J =* 7.6 Hz, 2H), 3.76 (d, *J =* 17.9 Hz, 4H), 3.59 (dd, *J =* 7.5, 5.4 Hz, 2H), 3.09 (q, *J=* 6.8 Hz, 1H), 3.00 (t, *J =* 5.9 Hz, 2H), 2.87 - 2.73 (m, 8H), 2.61 (dt, *J=* 17.2, 4.2 Hz, 1H), 2.47 (dd, *J =* 12.1, 4.4 Hz, 1H), 2.19 (dq, *J=* 13.5, 4.8 Hz, 1H), 1.95 - 1.87 (m, 2H), 1.75 - 1.64 (m, 4H), 1.55 (td, *J=* 12.2, 3.6 Hz, 2H).

### Example 114: Synthesis of Compound 114

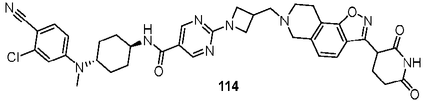

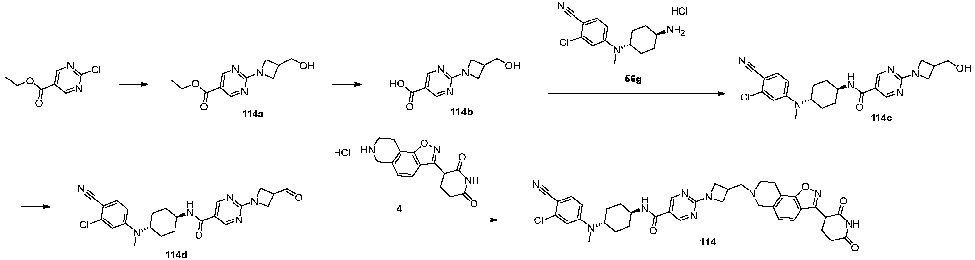

### Step 1: Preparation of intermediate 114a

Ethyl 2-chloropyrimidine-5-carboxylate (9.06 g), 3-azetidinylmethanol hydrochloride (5.02 g), potassium carbonate (16.78 g), and DMF (50 mL) were added to a reaction flask in sequence, and the mixture was reacted at room temperature for 2 h. After the reaction was completed, ethyl acetate (100 mL) and water (150 mL) were added. The organic phase was separated, washed with a saturated sodium chloride solution, dried over anhydrous sodium sulfate, filtered, and concentrated to give intermediate **114a** (7.4 g).

MS(ESI, [M+H]⁺) *m*/*z:* 238.2*.*

### Step 2: Preparation of intermediate 114b

**114a** (7.4 g), methanol (70 mL), water (7 mL), and sodium hydroxide (2.36 g) were added to a reaction flask in sequence, and the mixture was reacted at room temperature for 2 h. After the reaction was completed, the reaction solution was added with water, and the pH was adjusted to about 3 with hydrochloric acid. The mixture was filtered under vacuum, and the filter cake was dried by air blasting to give intermediate **114b** (5.3 g).

### Step 3: Preparation of intermediate 114c

**114b** (0.38 g), intermediate **56g** (0.50 g), HATU (0.95 g), DIPEA (0.86 g), and DMF (20 mL) were added to a reaction flask in sequence, and the mixture was reacted at room temperature. After the reaction was completed, ethyl acetate (70 mL) and water (150 mL) were added. The organic phase was separated, washed with a saturated sodium chloride solution, dried over anhydrous sodium sulfate, and filtered, and the concentrate was separated and purified by silica gel column chromatography to give intermediate **114c** (0.55 g).

MS(ESI, [M+H]⁺) *m*/*z:* 453.1.

### Step 4: Preparation of intermediate 114d

**114c** (0.3 g), Dess-Martin periodinane (0.84 g), and dichloromethane (30 mL) were added to a reaction flask in sequence, and the mixture was reacted at room temperature for 1 h. After the reaction was completed, dichloromethane (70 mL) and water (100 mL) were added. The organic phase was separated, washed with a saturated sodium chloride solution, dried over anhydrous sodium sulfate, filtered, and concentrated to give intermediate **114d** (0.4 g).

### Step 5: Preparation of compound 114

Intermediate **114d** (98 mg), intermediate **4** (76 mg), sodium acetate (37.8 mg), 1,2-dichloroethane (10 mL), isopropanol (2 mL), and sodium cyanoborohydride (31 mg) were added to a reaction flask in sequence, and the mixture was reacted at room temperature. After the reaction was completed, a saturated sodium bicarbonate solution (10 mL) was added to the reaction solution to neutralize acetic acid, and then dichloromethane (50 mL) and water (50 mL) were added for extraction. The organic phase was separated, dried over anhydrous sodium sulfate, and filtered, and the filtrate was purified by silica gel column chromatography to give compound 114 (55 mg).

MS(ESI, [M+H]⁺) *m*/*z:* 722.1.

¹H NMR (500 MHz, DMSO-d6) δ 11.07 (s, 1H), 8.74 (s, 2H), 8.12 (d, *J =* 7.5 Hz, 1H), 7.59 (dd, *J =* 8.6, 5.6 Hz, 2H), 7.11 (d, *J=* 8.3 Hz, 1H), 6.95 (d, *J=* 2.6 Hz, 1H), 6.84 (dd, *J=* 9.1, 2.5 Hz, 1H), 4.56 (dd, *J=* 11.9, 5.0 Hz, 1H), 4.23 (t, *J=* 8.6 Hz, 2H), 3.86 - 3.72 (m, 6H), 3.07 (dt, *J =* 14.2, 6.9 Hz, 1H), 3.00 (t, *J =* 5.9 Hz, 2H), 2.84 (d, *J=* 5.6 Hz, 7H), 2.79 - 2.72 (m, 1H), 2.61 (dt, *J=* 17.3, 4.3 Hz, 1H), 2.47 (dd, *J =* 12.2, 4.4 Hz, 1H), 2.19 (dq, *J =* 13.4, 4.9 Hz, 1H), 1.93 (d, *J =* 11.7 Hz, 2H), 1.78 - 1.63 (m, 4H), 1.59 - 1.49 (m, 2H).

### Example 115: Synthesis of Compound 115

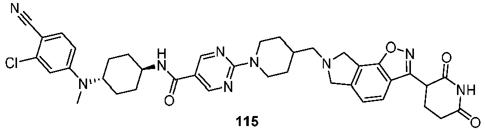

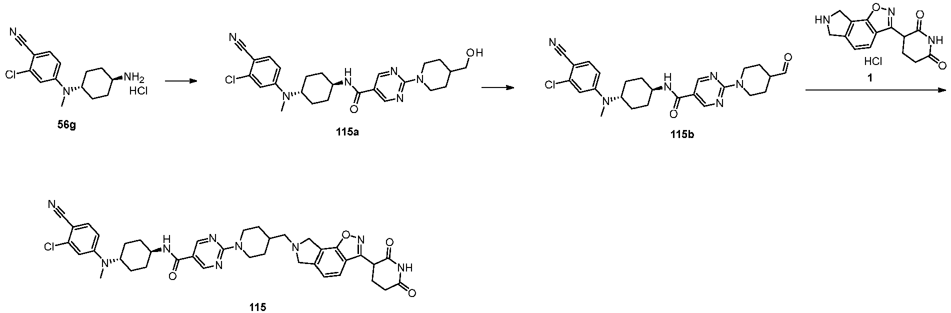

### Step 1: Preparation of intermediate 115a

2-(4-Hydroxymethylpiperidin-1-yl)pyrimidine-5-carboxylic acid (1.3 g), intermediate 56g (1.50 g), HATU (2.95 g), DIPEA (2.58 g), and DMF (20 mL) were added to a reaction flask in sequence, and the mixture was reacted at room temperature. After the reaction was completed, ethyl acetate (70 mL) and water (150 mL) were added. The organic phase was separated, washed with a saturated sodium chloride solution, dried over anhydrous sodium sulfate, and filtered, and the concentrate was separated and purified by silica gel column chromatography to give intermediate 115a (2.2 g).

MS(ESI, [M+H]⁺) *m*/*z:* 483.2*.*

### Step 2: Preparation of intermediate 115b

115a (0.35 g), Dess-Martin periodinane (0.94 g), and dichloromethane (30 mL) were added to a reaction flask in sequence, and the mixture was reacted at room temperature for 1 h. After the reaction was completed, dichloromethane (70 mL) and water (100 mL) were added. The organic phase was separated, washed with a saturated sodium chloride solution, dried over anhydrous sodium sulfate, filtered, and concentrated to give intermediate **115b** (0.4 g).

### Step 3: Preparation of compound 115

**115b** (172 mg), compound **1** (100 mg), sodium acetate (82 mg), and DCE/isopropanol (5:1, 20 mL) were added to a reaction flask in sequence, and the mixture was reacted at room temperature for 30 min. Sodium cyanoborohydride (63 mg) was then added, and the mixture was reacted at room temperature. After the reaction was completed, the reaction solution was concentrated by rotary evaporation to remove the solvent, and the crude product was separated and purified by silica gel column chromatography to give compound **115** (95 mg).

MS(ESI, [M+H]⁺) *m*/*z:* 736.2.

¹H NMR (500 MHz, DMSO-d6) δ 11.09 (s, 1H), 8.75 (s, 2H), 8.08 (d, *J =* 7.5 Hz, 1H), 7.72 (d, *J =* 8.0 Hz, 1H), 7.60 (d, *J=* 9.0 Hz, 1H), 7.31 (d, *J =* 8.1 Hz, 1H), 6.95 (d, *J=* 2.5 Hz, 1H), 6.84 (dd, *J* = 9.0, 2.5 Hz, 1H), 4.75 (d, *J =* 13.0 Hz, 2H), 4.60 (dd, *J =* 11.9, 5.0 Hz, 1H), 4.16 (s, 2H), 4.08 - 4.02 (m, 2H), 3.78 (ddp, *J =* 12.1, 8.3, 4.5, 4.0 Hz, 2H), 3.01 (t, *J* = 12.5 Hz, 2H), 2.84 (s, 3H), 2.77 (ddd, *J=* 17.1, 11.9, 5.3 Hz, 1H), 2.68 - 2.58 (m, 3H), 2.54 (d, *J=* 4.4 Hz, 1H), 2.20 (dq, *J =* 13.6, 4.8 Hz, 1H), 1.99 - 1.85 (m, 5H), 1.76 - 1.63 (m, 4H), 1.53 (qd, *J =* 12.4, 3.5 Hz, 2H), 1.17 - 1.06 (m, 2H).

### Example 116: Synthesis of Compound 116

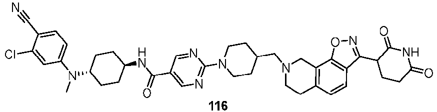

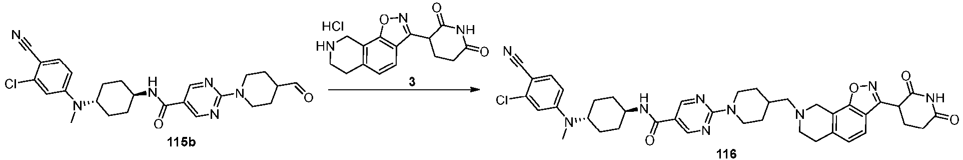

### Step 1: Preparation of compound 116

**115b** (142 mg), intermediate **3** (85 mg), sodium acetate (78 mg), and DCE/isopropanol (5:1, 20 mL) were added to a reaction flask in sequence, and the mixture was reacted at room temperature for 30 min. Sodium cyanoborohydride (54 mg) was then added, and the mixture was reacted at room temperature. After the reaction was completed, the reaction solution was concentrated by rotary evaporation to remove the solvent, and the crude product was separated and purified by silica gel column chromatography to give compound **116** (75 mg).

MS(ESI, [M+H]⁺) *m*/*z:* 750.2*.*

¹H NMR (500 MHz, DMSO-d6) δ 11.08 (s, 1H), 8.74 (s, 2H), 8.08 (d, *J =* 7.5 Hz, 1H), 7.59 (d, *J =* 9.0 Hz, 2H), 7.15 (d, *J=* 8.2 Hz, 1H), 6.95 (d, *J=* 2.5 Hz, 1H), 6.84 (dd, *J =* 9.1, 2.6 Hz, 1H), 4.73 (d, *J =* 13.2 Hz, 2H), 4.56 (dd, *J=* 11.9, 5.0 Hz, 1H), 3.80 (d, *J =* 12.6 Hz, 4H), 3.05 - 2.94 (m, 4H), 2.84 (s, 3H), 2.77 (dp, *J =* 12.2, 5.7 Hz, 3H), 2.60 (dt, *J=* 17.4, 4.2 Hz, 1H), 2.48 (d, *J=* 4.3 Hz, 1H), 2.44 (d, *J =* 7.3 Hz, 2H), 2.18 (dq, *J =* 13.7, 4.7 Hz, 1H), 2.03 (dd, *J=* 8.8, 5.2 Hz, 1H), 1.98 - 1.90 (m, 2H), 1.85 (d, *J =* 12.9 Hz, 2H), 1.77 - 1.65 (m, 4H), 1.53 (qd,*J* = 12.3, 3.6 Hz, 2H), 1.09 (qd, *J=* 12.4, 4.2 Hz, 2H).

### Example 117: Synthesis of Compound 117

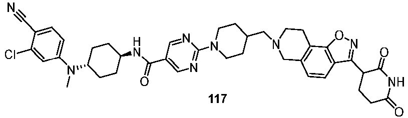

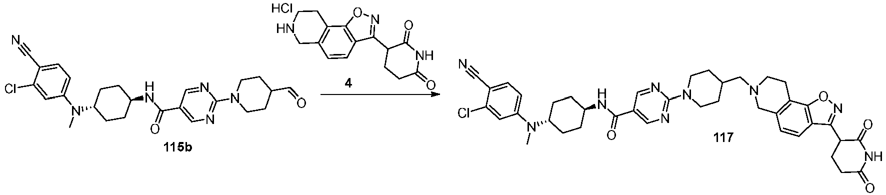

### Step 1: Preparation of compound 117

**115b** (145 mg), intermediate **4** (84 mg), sodium acetate (73 mg), and DCE/isopropanol (5:1, 20 mL) were added to a reaction flask in sequence, and the mixture was reacted at room temperature for 30 min. Sodium cyanoborohydride (57 mg) was then added, and the mixture was reacted at room temperature. After the reaction was completed, the reaction solution was concentrated by rotary evaporation to remove the solvent, and the crude product was separated and purified by silica gel column chromatography to give compound **117** (86 mg).

MS(ESI, [M+H]⁺) *m*/*z:* 750.2*.*

¹H NMR (500 MHz, DMSO-d6) δ 11.07 (s, 1H), 8.74 (s, 2H), 8.08 (d, *J =* 7.5 Hz, 1H), 7.59 (t, *J =* 8.7 Hz, 2H), 7.10 (d, *J=* 8.3 Hz, 1H), 6.95 (d, *J=* 2.5 Hz, 1H), 6.84 (dd, *J =* 9.1, 2.5 Hz, 1H), 4.73 (d, *J =* 13.0 Hz, 2H), 4.56 (dd, *J=* 11.9, 5.0 Hz, 1H), 3.82 - 3.75 (m, 2H), 3.71 (s, 2H), 2.99 (dt, *J =* 13.7, 7.7 Hz, 3H), 2.84 (s, 3H), 2.76 (dt, *J=* 14.7, 5.4 Hz, 3H), 2.61 (dt, *J=* 17.3, 4.1 Hz, 1H), 2.46 (dd, *J=* 12.1, 4.4 Hz, 1H), 2.39 (d, *J=* 7.2 Hz, 2H), 2.19 (dq, *J=* 8.7, 4.6 Hz, 1H), 2.07 (s, 1H), 2.00 (s, 1H), 1.93 (d, *J =* 12.0 Hz, 2H), 1.89 - 1.81 (m, 2H), 1.77 - 1.63 (m, 4H), 1.59 - 1.48 (m, 2H), 1.09 (t, *J =* 11.5 Hz, 2H).

### Example 118: Synthesis of Compound 118

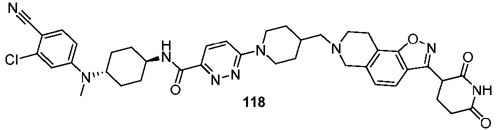

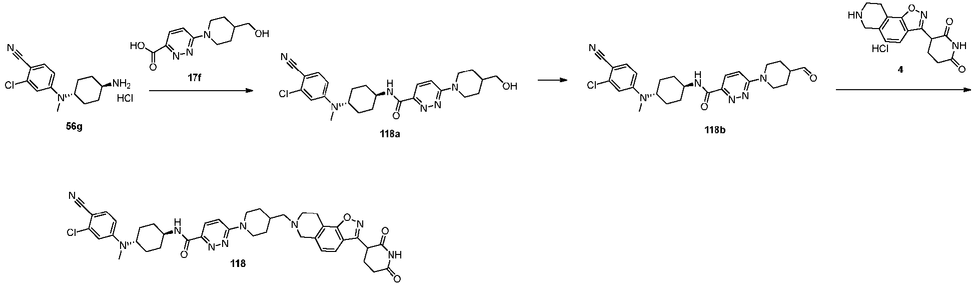

### Step 1: Preparation of intermediate 118a

Intermediate **17f** (1.23 g), intermediate **56g** (1.55 g), HATU (2.75 g), DIPEA (2.68 g), and DMF (20 mL) were added to a reaction flask in sequence, and the mixture was reacted at room temperature. After the reaction was completed, ethyl acetate (70 mL) and water (150 mL) were added. The organic phase was separated, washed with a saturated sodium chloride solution, dried over anhydrous sodium sulfate, and filtered, and the concentrate was separated and purified by silica gel column chromatography to give intermediate **118a** (2.6 g).

MS(ESI, [M+H]⁺) *m*/*z:* 483.2*.*

### Step 2: Preparation of intermediate 118b

**118a** (0.55 g), Dess-Martin periodinane (1.24 g), and dichloromethane (30 mL) were added to a reaction flask in sequence, and the mixture was reacted at room temperature for 1 h. After the reaction was completed, dichloromethane (70 mL) and water (100 mL) were added. The organic phase was separated, washed with a saturated sodium chloride solution, dried over anhydrous sodium sulfate, filtered, and concentrated to give intermediate **118b** (0.62 g).

### Step 3: Preparation of compound 118

Intermediate **118b** (125 mg), intermediate **4** (74 mg), sodium acetate (82 mg), and DCE/isopropanol (5:1, 20 mL) were added to a reaction flask in sequence, and the mixture was reacted at room temperature for 30 min. Sodium cyanoborohydride (62 mg) was then added, and the mixture was reacted at room temperature. After the reaction was completed, the reaction solution was concentrated by rotary evaporation to remove the solvent, and the crude product was separated and purified by silica gel column chromatography to give compound 118 (45 mg).

MS(ESI, [M+H]⁺) *m*/*z:* 750.2*.*

¹H NMR (500 MHz, DMSO-d6) δ 11.07 (s, 1H), 8.47 (d, *J=* 8.3 Hz, 1H), 7.80 (d, *J =* 9.6 Hz, 1H), 7.59 (dd, *J* = 11.8, 8.5 Hz, 2H), 7.34 (d, *J=* 9.6 Hz, 1H), 7.10 (d, *J=* 8.3 Hz, 1H), 6.94 (d, *J=* 2.5 Hz, 1H), 6.82 (dd, *J=* 9.1, 2.5 Hz, 1H), 4.56 (dd, *J=* 11.9, 5.0 Hz, 1H), 4.49 (d, *J=* 13.1 Hz, 2H), 3.81 (ddt, *J=* 36.6, 11.2, 5.5 Hz, 2H), 3.71 (s, 2H), 3.11 - 2.96 (m, 4H), 2.85 (s, 3H), 2.82 - 2.73 (m, 3H), 2.61 (dt, *J=* 17.3, 4.3 Hz, 1H), 2.47 (dd, *J =* 12.3, 4.4 Hz, 1H), 2.39 (d, *J=* 7.2 Hz, 2H), 2.19 (dt, *J=* 13.2, 4.6 Hz, 1H), 2.03 (s, 1H), 1.89 (dd, *J =* 28.6, 11.8 Hz, 4H), 1.81 - 1.61 (m, 6H), 1.21 - 1.11 (m, 2H).

### Example 119: Synthesis of Compound 119

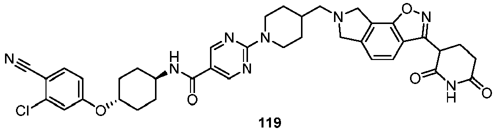

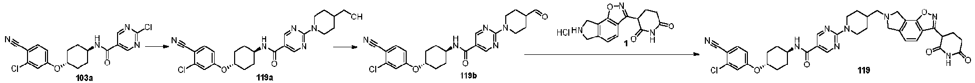

### Step 1: Preparation of intermediate 119a

Intermediate **103a** (2.35 g), 4-hydroxymethylpiperidine (0.830 g), DIPEA (2.329 g, 3.15 mL), and DMSO (20 mL) were added to a reaction flask in sequence, and the mixture was heated to 100 °C and reacted. After the reaction was completed, the reaction solution was extracted with ethyl acetate (100 mL) and water (200 mL). The organic phase was separated, washed with saturated brine, dried over anhydrous sodium sulfate, and filtered, and the filtrate was concentrated, slurried with ethyl acetate (20 mL), and filtered. The filter cake was collected and dried under reduced pressure to give the target intermediate **119a** (1.91 g).

### Step 2: Preparation of intermediate 119b

Intermediate **119a** (3.1 g), 2-iodoxybenzoic acid (3.69 g), and DMSO (30 mL) were added to a reaction flask in sequence, and the mixture was reacted at room temperature. After the reaction was completed, ethyl acetate (200 mL) and a saturated sodium bicarbonate solution (200 mL) were added for extraction. The organic phase was dried over anhydrous sodium sulfate and filtered, and the filtrate was purified by silica gel column chromatography to give compound **119b** (1.77 g).

### Step 3: Preparation of compound 119

Intermediate **119b** (134 mg), 1,2-dichloroethane (6 mL), isopropanol (2 mL), intermediate **1** (80 mg), sodium acetate (23 mg), and sodium triacetoxyborohydride (165 mg) were added to a reaction flask in sequence, and the mixture was stirred at room temperature. After the reaction was completed, dichloromethane (50 mL) and water (100 mL) were added for extraction. The organic phase was separated, washed with saturated brine, dried over anhydrous sodium sulfate, filtered, and concentrated, and the residue was purified by silica gel column chromatography to give compound **119** (110 mg).

MS(ESI, [M+H]⁺) m/z: 723.1.

¹H NMR (500 MHz, DMSO-d6) δ 11.09 (s, 1H), 8.75 (s, 2H), 8.10 (d, *J* = 7.4 Hz, 1H), 7.86 (d, *J =* 8.7 Hz, 1H), 7.71 (d, *J* = 8.0 Hz, 1H), 7.39 (d, *J* = 2.4 Hz, 1H), 7.31 (d, *J* = 8.2 Hz, 1H), 7.14 (dd, *J* = 8.7, 2.4 Hz, 1H), 4.75 (d, *J* = 13.0 Hz, 2H), 4.65 - 4.50 (m, 2H), 4.16 (s, 2H), 4.05 (t, *J* = 2.4 Hz, 2H), 3.80 (s, 1H), 3.01 (t, *J* = 12.3 Hz, 2H), 2.77 (ddd, *J* = 17.1, 11.9, 5.3 Hz, 1H), 2.69 - 2.58 (m, 3H), 2.56 - 2.52 (m, 1H), 2.24 - 2.16 (m, 1H), 2.11 (s, 2H), 1.90 (d, *J* = 13.8 Hz, 5H), 1.59 - 1.44 (m, 4H), 1.18 - 1.04 (m, 2H).

### Example 120: Synthesis of Compound 120

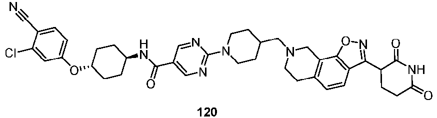

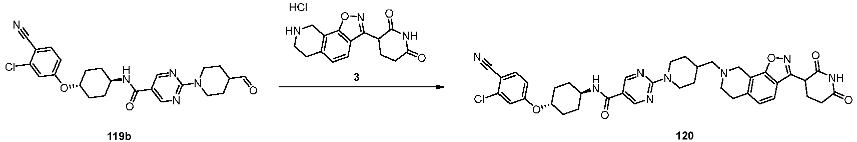

### Step 1: Preparation of compound 120

**119b** (130 mg), intermediate **3** (95 mg), sodium acetate (46 mg), and acetonitrile (20 mL) were added to a reaction flask in sequence, and the mixture was stirred at room temperature for 10 min. Sodium triacetoxyborohydride (115 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **120** (80 mg).

MS(ESI, [M+H]⁺) m/z: 737.1.

¹H NMR (500 MHz, DMSO-d6) δ 11.07 (s, 1H) , 8.74 (s, 2H), 8.09 (d, *J =* 7.5 Hz, 1H), 7.85 (d, *J =* 8.8 Hz, 1H), 7.59 (d, *J =* 8.1 Hz, 1H), 7.38 (d, *J =* 2.4 Hz, 1H), 7.19 - 7.10 (m, 2H), 4.73 (d, *J =* 13.0 Hz, 2H), 4.55 (dt, *J =* 11.7, 5.8 Hz, 2H), 3.81 (s, 3H), 3.07 - 2.92 (m, 4H), 2.77 (dp, *J =* 12.0, 5.5 Hz, 3H), 2.60 (dt, *J =* 17.4, 4.2 Hz, 1H), 2.52 (d, *J =* 4.4 Hz, 0.5H), 2.47 (d, *J =* 4.2 Hz, 0.5H), 2.43 (d, *J =* 7.3 Hz, 2H), 2.18 (dq, *J =* 13.7, 4.7 Hz, 1H), 2.14 - 1.99 (m, 3H), 1.91 (d, *J =* 7.6 Hz, 2H), 1.85 (d, *J =* 13.0 Hz, 2H), 1.58 - 1.43 (m, 4H), 1.07 (dd, *J =* 12.3, 8.9 Hz, 2H).

### Example 121: Synthesis of Compound 121

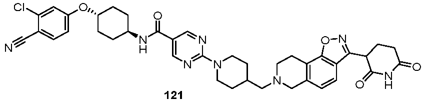

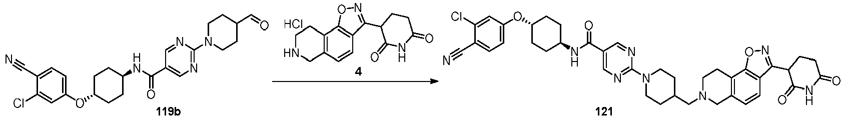

Intermediate **4** (80 mg), intermediate **119b** (116 mg), sodium acetate (20.40 mg), 1,2-dichloroethane (5 mL), isopropanol (1 mL), and sodium triacetoxyborohydride (105 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give compound 121 (133 mg). MS(ESI, [M+H]⁺) m/z: 737.1.

¹H NMR (500 MHz, DMSO-d6) δ 11.08 (s, 1H), 8.74 (s, 2H), 8.09 (d, *J =* 7.5 Hz, 1H), 7.86 (d, *J =* 8.8 Hz, 1H), 7.58 (d, *J=* 8.1 Hz, 1H), 7.38 (d, *J=* 2.4 Hz, 1H), 7.14 (dd, *J =* 8.8, 2.4 Hz, 1H), 7.10 (d, *J =* 8.3 Hz, 1H), 4.76 - 4.71 (m, 2H), 4.56 (dd, *J =* 11.8, 5.0 Hz, 2H), 3.79 (d, *J =* 9.7 Hz, 1H), 3.71 (s, 2H), 3.02 - 2.95 (m, 4H), 2.81 - 2.73 (m, 3H), 2.64 - 2.58 (m, 1H), 2.50 - 2.44 (m, 1H), 2.39 (d, *J =* 7.2 Hz, 2H), 2.19 (dq, *J =* 13.6, 4.7 Hz, 1H), 2.12 - 2.07 (m, 2H), 2.00 (ddd, *J =* 11.6, 7.6, 3.8 Hz, 1H), 1.95 - 1.89 (m, 2H), 1.87 - 1.81 (m, 2H), 1.50 (dt, *J =* 10.6, 6.7 Hz, 4H), 1.08 (qd, *J =* 12.6, 4.2 Hz, 2H).

### Example 122: Synthesis of Compound 122

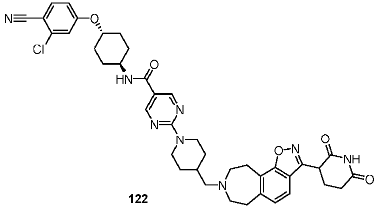

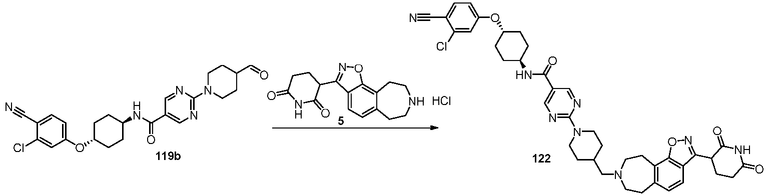

Intermediate **5** (80 mg), intermediate **119b** (111 mg), sodium acetate (19.54 mg), 1,2-dichloroethane (5 mL), isopropanol (1 mL), and sodium triacetoxyborohydride (101 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give compound **122** (77 mg).

MS(ESI, [M+H]⁺) m/z: 751.2.

¹H NMR (500 MHz, DMSO-d6) δ 11.08 (s, 1H), 8.75 (s, 2H), 8.10 (d, J = 7.5 Hz, 1H), 7.86 (d, J = 8.8 Hz, 1H), 7.54 (d, J = 8.0 Hz, 1H), 7.39 (d, J = 2.4 Hz, 1H), 7.18 (d, J = 8.1 Hz, 1H), 7.14 (dd, J = 8.8, 2.5 Hz, 1H), 4.74 (d, J = 12.9 Hz, 2H), 4.54 (dd, J = 11.9, 5.1 Hz, 2H), 3.80 (s, 1H), 3.18 - 3.13 (m, 2H), 3.04 (d, J = 7.2 Hz, 2H), 2.98 (t, J = 12.5 Hz, 2H), 2.80 - 2.73 (m, 1H), 2.69 - 2.65 (m, 2H), 2.64 - 2.58 (m, 3H), 2.49 - 2.44 (m, 1H), 2.34 (d, J = 6.8 Hz, 2H), 2.18 (dq, J = 13.9, 4.8 Hz, 1H), 2.13 - 2.07 (m, 2H), 1.92 (d, J = 8.6 Hz, 3H), 1.85 (d, J = 13.8 Hz, 2H), 1.54 - 1.47 (m, 4H), 1.11 - 1.03 (m, 2H).

### Example 123: Synthesis of Compound 123

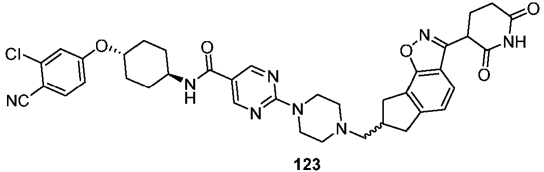

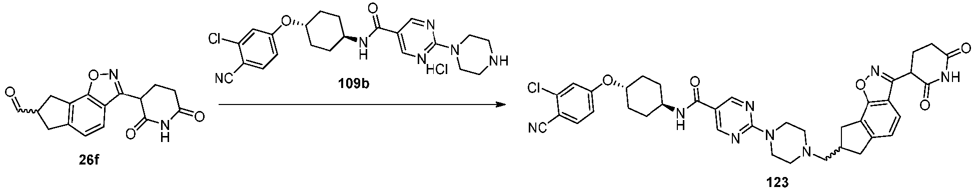

### Step 1: Preparation of compound 123

Intermediate **26f** (100 mg), intermediate **109b** (103 mg), 1,2-dichloroethane (10 mL), isopropanol (2 mL), and sodium triacetoxyborohydride (141 mg) were added to a reaction flask in sequence, and the mixture was reacted at room temperature. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography to give compound **123** (70 mg).

MS(ESI, [M+H]⁺) m/z: 723.11.

¹H NMR (500 MHz, DMSO-*d*6) δ 11.08 (s, 1H), 8.77 (s, 2H), 8.13 (d, *J =* 7.4 Hz, 1H), 7.86 (d, *J =* 8.8 Hz, 1H), 7.62 (d, *J=* 8.0 Hz, 1H), 7.39 (d, *J=* 2.4 Hz, 1H), 7.27 (d, *J=* 8.1 Hz, 1H), 7.14 (dd, *J= 8.8,* 2.4 Hz, 1H), 4.57 (ddd, *J=* 11.8, 5.0, 1.7 Hz, 2H), 3.92 - 3.71 (m, 5H), 3.29 - 3.15 (m, 2H), 2.98 - 2.71 (m, 4H), 2.65 - 2.57 (m, 1H), 2.48 (d, *J=* 5.4 Hz, 5H), 2.39 (d, *J=* 7.3 Hz, 2H), 2.19 (dd, *J =* 8.8, 4.3 Hz, 1H), 2.14 - 2.05 (m, 2H), 1.92 (s, 2H), 1.57 - 1.42 (m, 4H).

### Example 124: Synthesis of Compound 124

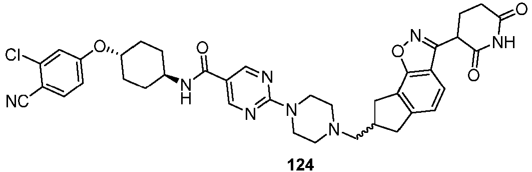

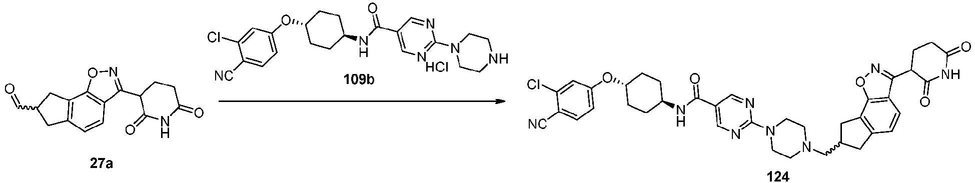

### Step 1: Preparation of compound 124

Intermediate **27a** (100 mg), intermediate **109b** (100 mg), 1,2-dichloroethane (10 mL), isopropanol (2 mL), and sodium triacetoxyborohydride (140 mg) were added to a reaction flask in sequence, and the mixture was reacted at room temperature. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography to give compound **124** (63 mg).

MS(ESI, [M+H]⁺) m/z: 723.31.

¹H NMR (500 MHz, DMSO-d6) δ 11.08 (s, 1H), 8.77 (s, 2H), 8.13 (d, *J =* 7.4 Hz, 1H), 7.86 (d, *J =* 8.8 Hz, 1H), 7.62 (d, *J =* 8.0 Hz, 1H), 7.39 (d, *J =* 2.4 Hz, 1H), 7.27 (d, *J =* 8.1 Hz, 1H), 7.14 (dd, *J* = 8.8, 2.4 Hz, 1H), 4.57 (ddd, *J =* 11.8, 5.0, 1.7 Hz, 2H), 3.92 - 3.71 (m, 5H), 3.29 - 3.15 (m, 2H), 2.98 - 2.71 (m, 4H), 2.65 - 2.57 (m, 1H), 2.48 (d, *J =* 5.4 Hz, 5H), 2.39 (d, *J =* 7.3 Hz, 2H), 2.19 (dd, *J =* 8.8, 4.3 Hz, 1H), 2.14 - 2.05 (m, 2H), 1.92 (s, 2H), 1.57 - 1.42 (m, 4H).

### Example 125: Synthesis of Compound 125

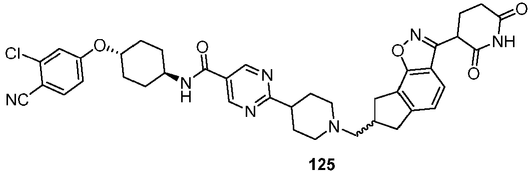

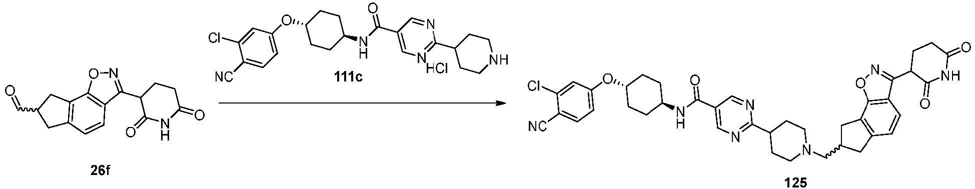

### Step 1: Preparation of compound 125

Intermediate **26f** (100 mg), intermediate **111c** (105 mg), 1,2-dichloroethane (10 mL), isopropanol (2 mL), and sodium triacetoxyborohydride (140 mg) were added to a reaction flask in sequence, and the mixture was reacted at room temperature. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography to give compound **125** (66 mg).

MS(ESI, [M+H]⁺) m/z: 722.14.

¹H NMR (500 MHz, DMSO-d6) δ 11.08 (s, 1H), 9.08 (s, 2H), 8.57 (d, *J =* 7.5 Hz, 1H), 7.86 (d, *J =* 8.8 Hz, 1H), 7.61 (d, *J =* 8.0 Hz, 1H), 7.40 (d, *J =* 2.4 Hz, 1H), 7.27 (d, *J =* 8.1 Hz, 1H), 7.15 (dd, *J =* 8.8, 2.4 Hz, 1H), 4.68 - 4.48 (m, 2H), 3.84 (d, *J =* 8.5 Hz, 1H), 3.29 - 3.11 (m, 2H), 3.04 - 2.71 (m, 7H), 2.66 - 2.56 (m, 1H), 2.47 (dd, *J =* 12.1, 4.4 Hz, 1H), 2.37 (d, *J =* 7.3 Hz, 2H), 2.26 - 2.02 (m, 5H), 2.02 - 1.77 (m, 6H), 1.54 (p, *J=* 10.8, 9.2 Hz, 4H). **Example 126:** Synthesis of Compound **126**

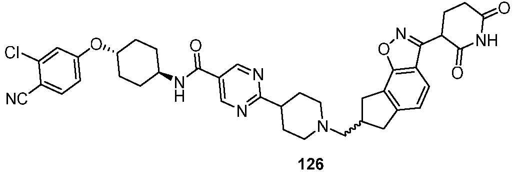

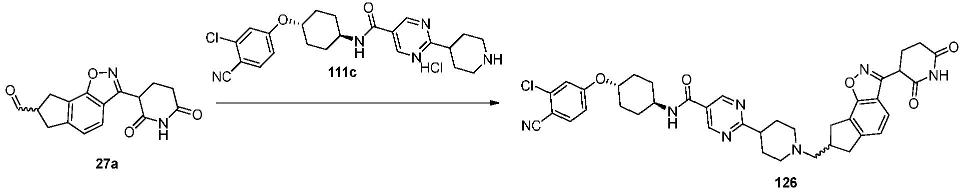

### Step 1: Preparation of compound 126

Intermediate **27a** (100 mg), intermediate **111c** (103 mg), 1,2-dichloroethane (10 mL), isopropanol (2 mL), and sodium triacetoxyborohydride (140 mg) were added to a reaction flask in sequence, and the mixture was reacted at room temperature. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography to give compound **126** (73 mg).

MS(ESI, [M+H]⁺) m/z: 722.21.

¹H NMR (500 MHz, DMSO-d6) δ 11.08 (s, 1H), 9.08 (s, 2H), 8.57 (d, *J =* 7.5 Hz, 1H), 7.86 (d, *J =* 8.8 Hz, 1H), 7.61 (d, *J =* 8.0 Hz, 1H), 7.40 (d, *J =* 2.4 Hz, 1H), 7.27 (d, *J =* 8.1 Hz, 1H), 7.15 (dd, *J =* 8.8, 2.4 Hz, 1H), 4.68 - 4.48 (m, 2H), 3.84 (d, *J =* 8.5 Hz, 1H), 3.29 - 3.11 (m, 2H), 3.04 - 2.71 (m, 7H), 2.66 - 2.56 (m, 1H), 2.47 (dd, *J =* 12.1, 4.4 Hz, 1H), 2.37 (d, *J =* 7.3 Hz, 2H), 2.26 - 2.02 (m, 5H), 2.02 - 1.77 (m, 6H), 1.54 (p, *J =* 10.8, 9.2 Hz, 4H).

### Example 127: Synthesis of Compound 127

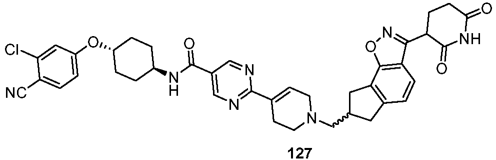

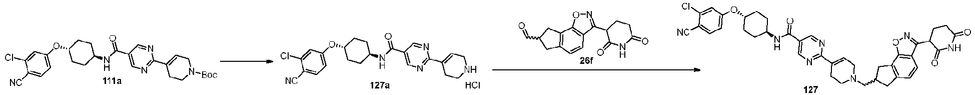

### Step 1: Preparation of intermediate 127a

Intermediate **111a** (300 mg), ethyl acetate (10 mL), and a solution of hydrochloric acid in 1,4-dioxane (4 M, 2 mL) were added to a reaction flask in sequence, and the mixture was reacted at room temperature. After the reaction was completed, the reaction system was filtered under vacuum and dried to give compound **127a** (265 mg).

### Step 2: Preparation of compound 127

Intermediate **26f** (100 mg), intermediate **127a** (103 mg), 1,2-dichloroethane (10 mL), isopropanol (2 mL), and sodium triacetoxyborohydride (141 mg) were added to a reaction flask in sequence, and the mixture was reacted at room temperature. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography to give compound **127** (93 mg).

MS(ESI, [M+H]⁺) m/z: 720.1.

¹H NMR (500 MHz, DMSO-d6) δ 11.08 (s, 1H), 9.10 (s, 2H), 8.58 (d, *J =* 7.4 Hz, 1H), 7.86 (d, *J =* 8.8 Hz, 1H), 7.61 (d, *J=* 8.1 Hz, 1H), 7.40 (d, *J* = 2.4 Hz, 1H), 7.36 - 7.24 (m, 2H), 7.15 (dd, *J* = 8.8, 2.4 Hz, 1H), 4.63 - 4.48 (m, 2H), 3.85 (s, 1H), 3.29 - 3.11 (m, 4H), 3.02 - 2.56 (m, 9H), 2.49 - 2.42 (m, 1H), 2.26 - 2.06 (m, 3H), 2.02 - 1.87 (m, 2H), 1.54 (q, *J* = 10.1, 9.5 Hz, 4H).

### Example 128: Synthesis of Compound 128

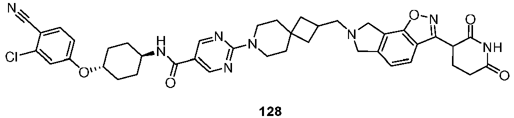

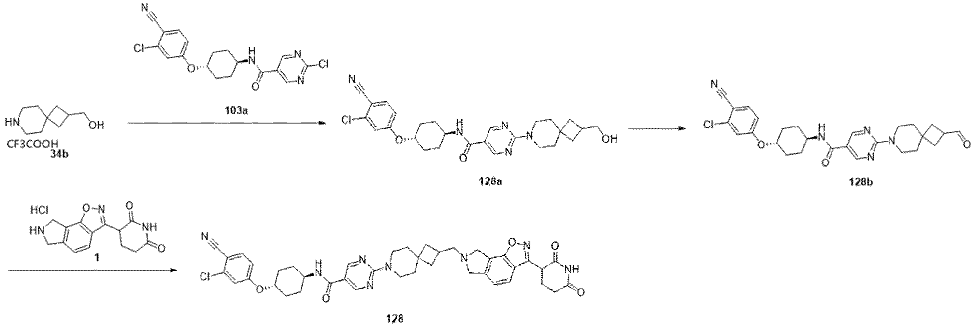

### Step 1: Preparation of compound 128a

**34b** (0.56 g), compound **103a** (1.0 g), *N*,*N*-diisopropylethylamine (1.8 g), and dimethyl sulfoxide (10 mL) were added to a reaction flask in sequence, and the mixture was reacted at 80 °C for 2 h. After the reaction was completed, the reaction solution was cooled to room temperature, diluted with ethyl acetate, and washed with a saturated sodium chloride solution, and the organic layer was collected, dried over anhydrous sodium sulfate, and filtered. The filtrate was concentrated, and then the residue was purified by silica gel column chromatography (dichloromethane/methanol) to give the target intermediate **128a** (1.0 g).

### Step 2: Preparation of intermediate 128b

**128a** (1.5 g), Dess-Martin periodinane (1.7 g), and dichloromethane (15 mL) were added to a reaction flask in sequence, and the mixture was reacted at room temperature for 3 h. After the reaction was completed, the reaction solution was quenched with a saturated sodium bicarbonate solution. The organic layer was separated and washed with a saturated sodium chloride solution. The organic layer was collected, dried over anhydrous sodium sulfate, and filtered. The filtrate was concentrated, and then the residue was purified by silica gel column chromatography (dichloromethane/methanol) to give the target intermediate **128b** (1.3 g).

MS(ESI, [M+H]⁺) m/z: 508.2.

### Step 3: Preparation of compound 128

**128b** (140 mg), intermediate **1** (95 mg), sodium acetate (46 mg), and acetonitrile (20 mL) were added to a reaction flask in sequence, and the mixture was stirred at room temperature for 10 min. Sodium triacetoxyborohydride (120 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography

### (dichloromethane/methanol) to give the target compound 128 (50 mg).

MS(ESI, [M+H]⁺) m/z: 763.3.

¹H NMR (500 MHz, DMSO-d6) δ 11.08 (s, 1H), 8.73 (s, 2H), 8.09 (d, *J =* 7.4 Hz, 1H), 7.85 (d, *J =* 8.7 Hz, 1H), 7.70 (d, *J =* 8.0 Hz, 1H), 7.38 (d, *J =* 2.4 Hz, 1H), 7.29 (d, *J =* 8.1 Hz, 1H), 7.14 (dd, *J =* 8.8, 2.4 Hz, 1H), 4.69 - 4.48 (m, 2H), 4.12 (s, 2H), 4.01 (s, 2H), 3.77 (dt, *J =* 45.1, 5.4 Hz, 5H), 2.91 - 2.71 (m, 3H), 2.59 (ddd, *J =* 22.4, 12.5, 6.3 Hz, 3H), 2.20 (dq, *J =* 14.3, 4.7 Hz, 1H), 2.05 (dd, *J* = 24.9, 14.8 Hz, 4H), 1.98 - 1.84 (m, 2H), 1.71 - 1.42 (m, 10H).

### Example 129: Synthesis of Compound 129

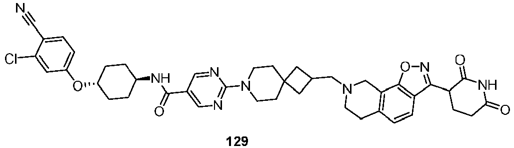

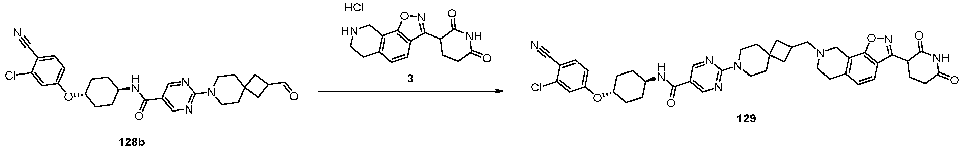

### Step 1: Preparation of compound 129

**128b** (120 mg), intermediate **3** (90 mg), sodium acetate (40 mg), and acetonitrile (20 mL) were added to a reaction flask in sequence, and the mixture was stirred at room temperature for 10 min. Sodium triacetoxyborohydride (110 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **129** (60 mg).

MS(ESI, [M+H]⁺) m/z: 777.1.

¹H NMR (500 MHz, DMSO-d6) δ 11.07 (s, 1H) , 8.73 (s, 2H), 8.09 (d, *J =* 7.5 Hz, 1H), 7.85 (d, *J =* 8.7 Hz, 1H), 7.58 (d, *J =* 8.1 Hz, 1H), 7.38 (d, *J =* 2.5 Hz, 1H), 7.18 - 7.10 (m, 2H), 4.62 - 4.49 (m, 2H), 3.89 - 3.75 (m, 5H), 3.71 (t, *J =* 5.6 Hz, 2H), 2.93 (q, *J =* 5.6 Hz, 2H), 2.81 - 2.70 (m, 3H), 2.69 - 2.57 (m, 4H), 2.53 (s, 0.5H), 2.46 (dd, *J =* 12.2, 4.5 Hz, 0.5H), 2.18 (dq, *J =* 13.4, 4.8 Hz, 1H), 2.14 - 1.99 (m, 4H), 1.97 - 1.86 (m, 2H).1.63 (t, *J =* 5.6 Hz, 2H), 1.59 - 1.43 (m, 8H).

### Example 130: Synthesis of Compound 130

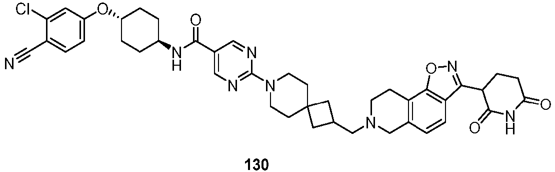

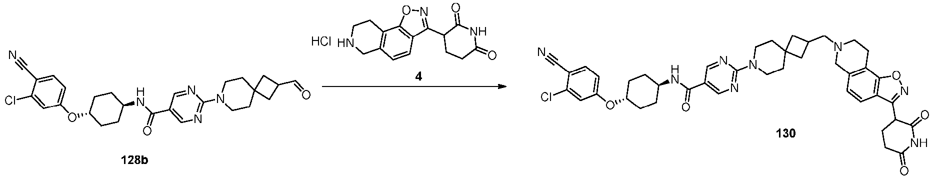

### Step 1: Preparation of compound 130

**128b** (100 mg), intermediate **4** (80 mg), sodium acetate (35 mg), and acetonitrile (20 mL) were added to a reaction flask in sequence, and the mixture was stirred at room temperature for 10 min. Sodium triacetoxyborohydride (101 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **130** (55 mg).

MS(ESI, [M+H]⁺) m/z: 777.5.

¹H NMR (500 MHz, DMSO-*d*6) δ 11.07 (s, 1H), 8.73 (s, 2H), 8.09 (d, *J =* 7.4 Hz, 1H), 7.86 (d, *J =* 8.7 Hz, 1H), 7.57 (d, *J =* 8.2 Hz, 1H), 7.38 (d, *J =* 2.4 Hz, 1H), 7.14 (dd, *J =* 8.7, 2.5 Hz, 1H), 7.09 (d, *J =* 8.3 Hz, 1H), 4.55 (dt, *J =* 10.1, 5.0 Hz, 2H), 3.88 - 3.75 (m, 3H), 3.74 - 3.62 (m, 4H), 2.98 (t, *J* = 5.9 Hz, 2H), 2.76 (qd, *J =* 8.7, 8.3, 6.0 Hz, 3H), 2.69 - 2.55 (m, 4H), 2.52 (d, *J =* 4.2 Hz, 0.3H), 2.46 (dd, *J =* 12.1, 4.4 Hz, 0.7H), 2.18 (dq, *J =* 13.5, 4.8 Hz, 1H), 2.09 (dd, *J =* 9.6, 4.4 Hz, 2H), 2.06 - 1.98 (m, 2H), 1.92 (dd, *J =* 9.4, 4.3 Hz, 2H), 1.62 (t, *J =* 5.5 Hz, 2H), 1.57 - 1.43 (m, 8H).

### Example 131: Synthesis of Compound 131

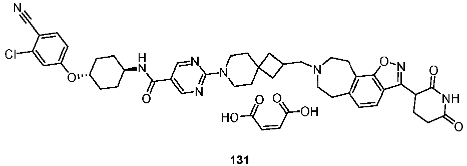

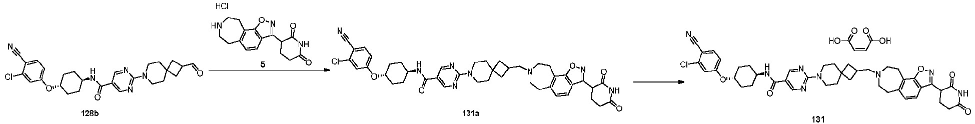

### Step 1: Preparation of intermediate 131a

**128b** (105 mg), intermediate **5** (88 mg), sodium acetate (40 mg), and acetonitrile (20 mL) were added to a reaction flask in sequence, and the mixture was stirred at room temperature for 10 min. Sodium triacetoxyborohydride (109 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **131a** (50 mg).

MS(ESI, [M+H]⁺) m/z: 791.1.

### Step 2: Preparation of compound 131

Intermediate **131a** (50 mg), dichloromethane (10 mL), methanol (1 mL), and maleic acid (10 mg) were added to a reaction flask in sequence, and the mixture was reacted at room temperature for 1 h. After the reaction was completed, the reaction solution was concentrated by evaporation under reduced pressure to remove the solvent. The residue was slurried with petroleum ether and filtered, and the filter cake was collected to give compound **131** (46 mg).

MS(ESI, [M+H]⁺) m/z: 791.5.

¹H NMR (500 MHz, DMSO-d6) δ 11.09 (s, 1H), 9.67 (s, 1H), 8.74 (s, 2H), 8.10 (d, *J =* 7.4 Hz, 1H), 7.86 (d, *J* = 8.9 Hz, 1H), 7.67 (d, *J =* 8.1 Hz, 1H), 7.38 (d, *J =* 2.4 Hz, 1H), 7.29 (d, *J =* 8.1 Hz, 1H), 7.14 (dd, *J =* 8.8, 2.4 Hz, 1H), 6.09 (s, 2H), 4.56 (ddd, *J =* 20.7, 11.1, 5.1 Hz, 2H), 3.80 (q, *J =* 10.2, 7.8 Hz, 3H), 3.71 (t, *J =* 5.6 Hz, 3H), 3.29 (s, 9H), 2.79 (ddd, *J =* 17.3, 12.2, 5.8 Hz, 2H), 2.61 (dt, *J =* 17.3, 4.1 Hz, 1H), 2.54 (d, *J* = 12.6 Hz, 0.6H), 2.47 (s, 0.4H), 2.22 - 2.03 (m, 5H), 1.91 (dd, *J =* 9.7, 4.4 Hz, 2H), 1.64 (dd, *J =* 12.2, 8.1 Hz, 4H), 1.57 - 1.42 (m, 6H).

### Example 132: Synthesis of Compound 132

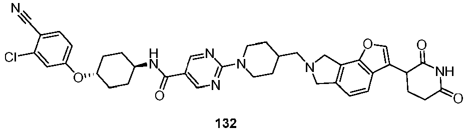

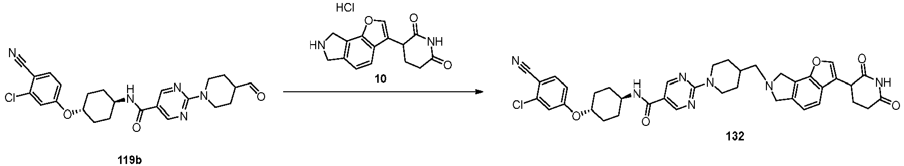

### Step 1: Preparation of compound 132

**119b** (100 mg), intermediate **10** (80 mg), sodium acetate (43 mg), and acetonitrile (20 mL) were added to a reaction flask in sequence, and the mixture was stirred at room temperature for 10 min. Sodium triacetoxyborohydride (108 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **132** (32 mg).

MS(ESI, [M+H]⁺) m/z: 722.1.

¹H NMR (500 MHz, DMSO-d6) δ 10.88 (s, 1H), 8.75 (s, 2H), 8.10 (d, *J =* 7.4 Hz, 1H), 7.86 (d, *J =* 7.4 Hz, 2H), 7.41 (dd, *J* = 22.9, 5.2 Hz, 2H), 7.14 (d, *J =* 8.2 Hz, 2H), 4.74 (d, *J* = 13.0 Hz, 2H), 4.62 - 4.48 (m, 1H), 4.22 - 4.05 (m, 3H), 3.98 (s, 2H), 3.80 (s, 1H), 3.00 (t, *J* = 12.5 Hz, 2H), 2.74 (ddd, *J =* 17.4, 12.2, 5.3 Hz, 1H), 2.68 - 2.54 (m, 3H), 2.39 - 2.25 (m, 1H), 2.12 (dt, *J =* 15.3, 5.2 Hz, 3H), 1.89 (d, *J =* 14.0 Hz, 5H), 1.59 - 1.43 (m, 4H), 1.11 (q, *J* = 13.2, 12.5 Hz, 2H).

### Example 133: Synthesis of Compound 133

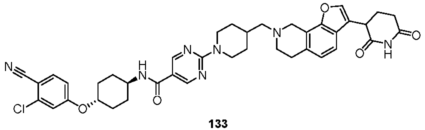

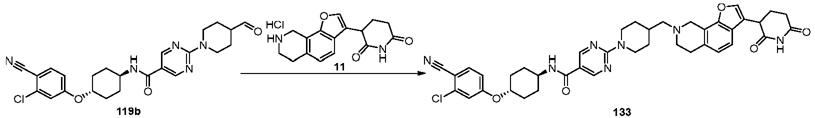

Intermediate **11** (80 mg), intermediate **119b** (117 mg), sodium acetate (40.9 mg), 1,2-dichloroethane (5 mL), isopropanol (1 mL), and sodium triacetoxyborohydride (106 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give compound **133** (130 mg).

MS(ESI, [M+H]⁺) m/z: 736.4.

¹H NMR (500 MHz, DMSO-d6) δ 10.87 (s, 1H), 8.74 (s, 2H), 8.09 (d, J = 7.5 Hz, 1H), 7.86 (d, J = 8.8 Hz, 1H), 7.82 (s, 1H), 7.38 (d, J = 2.5 Hz, 1H), 7.35 (d, J = 8.0 Hz, 1H), 7.14 (dd, J = 8.8, 2.4 Hz, 1H), 7.00 (d, J = 8.1 Hz, 1H), 4.76 - 4.70 (m, 2H), 4.58 - 4.51 (m, 1H), 4.10 (dd, J = 11.8, 4.9 Hz, 1H), 3.79 (d, J = 12.3 Hz, 3H), 2.99 (td, J = 13.0, 2.6 Hz, 2H), 2.91 (t, J = 5.8 Hz, 2H), 2.72 (p, J = 5.9, 5.5 Hz, 3H), 2.59 - 2.55 (m, 1H), 2.41 (d, J = 7.2 Hz, 2H), 2.34 - 2.26 (m, 1H), 2.10 (dp, J = 8.6, 4.3 Hz, 3H), 2.02 (td, J = 11.4, 10.5, 5.7 Hz, 1H), 1.95 - 1.89 (m, 2H), 1.88 - 1.81 (m, 2H), 1.54 - 1.46 (m, 4H), 1.08 (qd, J = 12.1, 3.8 Hz, 2H).

### Example 134: Synthesis of Compound 134

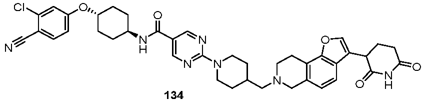

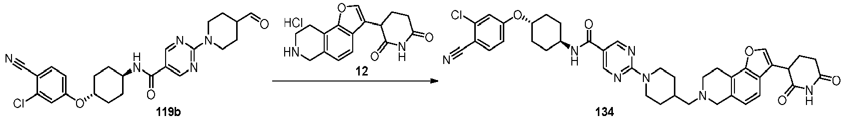

Intermediate **12** (80 mg), intermediate **119b** (117 mg), sodium acetate (40.9 mg), 1,2-dichloroethane (5 mL), isopropanol (1 mL), and sodium triacetoxyborohydride (106 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give compound **134** (107 mg).

MS(ESI, [M+H]⁺) m/z: 736.4.

¹H NMR (500 MHz, DMSO-d6) δ 10.87 (s, 1H), 8.74 (s, 2H), 8.09 (d, J = 7.5 Hz, 1H), 7.88 - 7.82 (m, 2H), 7.38 (d, J = 2.4 Hz, 1H), 7.33 (d, J = 8.0 Hz, 1H), 7.14 (dd, J = 8.8, 2.4 Hz, 1H), 6.95 (d, J = 8.2 Hz, 1H), 4.73 (dt, J = 13.1, 3.3 Hz, 2H), 4.58 - 4.51 (m, 1H), 4.10 (dd, J = 11.8, 4.9 Hz, 1H), 3.79 (s, 1H), 3.65 (s, 2H), 3.02 - 2.94 (m, 4H), 2.78 - 2.69 (m, 3H), 2.59 - 2.54 (m, 1H), 2.36 (d, J = 7.1 Hz, 2H), 2.29 (td, J = 12.2, 4.1 Hz, 1H), 2.14 - 2.06 (m, 3H), 2.03 - 1.96 (m, 1H), 1.94 - 1.88 (m, 2H), 1.87 - 1.80 (m, 2H), 1.50 (dt, J = 10.7, 6.2 Hz, 4H), 1.08 (qd, J = 12.5, 4.1 Hz, 2H).

### Example 135: Synthesis of Compound 135

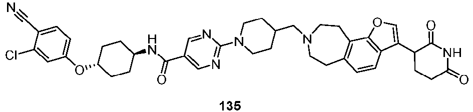

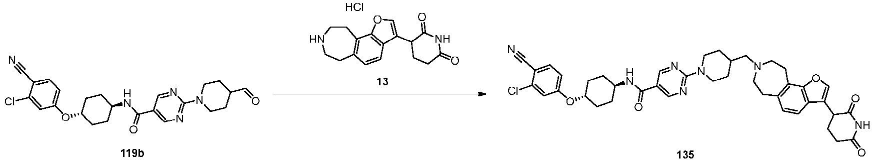

### Step 1: Preparation of compound 135

**119b** (105 mg), intermediate **13** (86 mg), sodium acetate (45 mg), and acetonitrile (20 mL) were added to a reaction flask in sequence, and the mixture was stirred at room temperature for 10 min. Sodium triacetoxyborohydride (115 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography (dichloromethane/methanol) to give the target compound **135** (60 mg).

MS(ESI, [M+H]⁺) m/z: 750.1.

¹H NMR (500 MHz, DMSO-d6) δ 10.87 (s, 1H), 8.75 (s, 2H), 8.10 (d, *J =* 7.5 Hz, 1H), 7.95 - 7.78 (m, 2H), 7.39 (d, *J* = 2.4 Hz, 1H), 7.26 (d, *J* = 7.8 Hz, 1H), 7.14 (dd, *J* = 8.8, 2.4 Hz, 1H), 7.01 (d, *J* = 8.0 Hz, 1H), 4.74 (d, *J =* 13.0 Hz, 2H), 4.55 (dd, *J =* 9.5, 5.2 Hz, 1H), 4.09 (dd, *J =* 11.9, 4.9 Hz, 1H), 3.80 (s, 1H), 3.12 (d, *J =* 6.9 Hz, 2H), 3.06 - 2.91 (m, 4H), 2.73 (ddd, *J =* 17.3, 12.1, 5.4 Hz, 1H), 2.68 - 2.53 (m, 5H), 2.39 - 2.25 (m, 3H), 2.10 (dt, *J* = 14.9, 5.4 Hz, 3H), 1.99 - 1.80 (m, 5H), 1.50 (dt, *J =* 10.9, 6.9 Hz, 4H), 1.07 (q, *J =* 11.1 Hz, 2H).

### Example 136: Synthesis of Compound 136

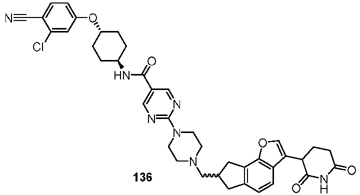

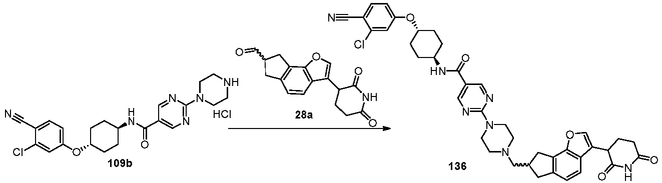

Intermediate **28a** (70 mg), intermediate **109b** (103 mg), 1,2-dichloroethane (5 mL), isopropanol (1 mL), and sodium triacetoxyborohydride (99 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give compound 136 (90 mg).

MS(ESI, [M+H]⁺) m/z: 722.1.

¹H NMR (500 MHz, DMSO-d6) δ 10.88 (s, 1H), 8.77 (s, 2H), 8.13 (d, J = 7.4 Hz, 1H), 7.98 - 7.66 (m, 2H), 7.51 - 7.25 (m, 2H), 7.13 (dd, J = 13.1, 8.3 Hz, 2H), 4.55 (s, 1H), 4.11 (dd, J = 12.1, 4.8 Hz, 1H), 3.82 (d, J = 23.6 Hz, 5H), 3.24 - 3.08 (m, 2H), 2.93 - 2.66 (m, 4H), 2.57 (d, J = 18.4 Hz, 1H), 2.47 (s, 4H), 2.34 (dd, J = 33.1, 9.9 Hz, 3H), 2.10 (d, J = 10.3 Hz, 3H), 1.92 (d, J = 9.8 Hz, 2H), 1.67 - 1.35 (m, 4H).

### Example 137: Synthesis of Compound 137

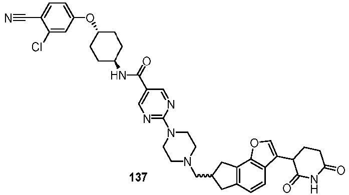

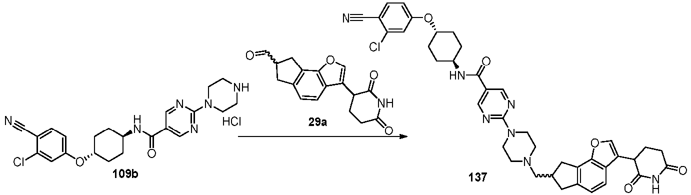

Intermediate **29a** (70 mg), intermediate **109b** (103 mg), 1,2-dichloroethane (5 mL), isopropanol (1 mL), and sodium triacetoxyborohydride (99 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give compound **137** (80 mg).

MS(ESI, [M+H]⁺) m/z: 722.1.

¹H NMR (500 MHz, DMSO-d6) δ 10.88 (s, 1H), 8.77 (s, 2H), 8.13 (d, J = 7.5 Hz, 1H), 7.94 - 7.74 (m, 2H), 7.43 - 7.28 (m, 2H), 7.13 (dd, J = 13.2, 8.4 Hz, 2H), 4.61 - 4.49 (m, 1H), 4.11 (dd, J = 11.9, 4.9 Hz, 1H), 3.95 - 3.71 (m, 5H), 3.16 (ddd, J = 30.6, 15.8, 7.6 Hz, 2H), 2.88 (q, J = 10.3, 7.8 Hz, 2H), 2.75 (ddd, J = 31.3, 13.9, 5.2 Hz, 2H), 2.57 (d, J = 18.8 Hz, 1H), 2.49 - 2.43 (m, 4H), 2.37 (d, J = 7.3 Hz, 2H), 2.29 (td, J = 12.2, 4.1 Hz, 1H), 2.10 (d, J = 10.1 Hz, 3H), 1.92 (d, J = 9.9 Hz, 2H), 1.61 - 1.41 (m, 4H).

### Example 138: Synthesis of Compound 138

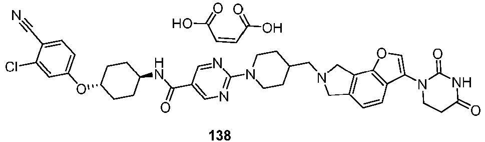

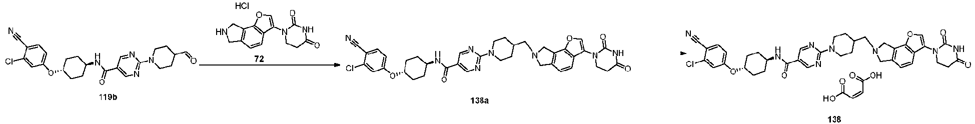

### Step 1: Preparation of intermediate 138a

**119b** (110 mg), intermediate **72** (92 mg), sodium acetate (45 mg), and acetonitrile (20 mL) were added to a reaction flask in sequence, and the mixture was stirred at room temperature for 10 min. Sodium triacetoxyborohydride (112 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography to give the target compound **138a** (40 mg).

MS(ESI, [M+H]⁺) m/z: 723.1.

### Step 2: Preparation of compound 138

Intermediate **138a** (40 mg), dichloromethane (10 mL), methanol (1 mL), and maleic acid (8 mg) were added to a reaction flask in sequence, and the mixture was reacted at room temperature for 1 h. After the reaction was completed, the reaction solution was concentrated by evaporation under reduced pressure to remove the solvent. The residue was slurried with petroleum ether and filtered, and the filter cake was collected to give compound **138** (36 mg).

MS(ESI, [M+H]⁺) m/z: 723.1.

¹H NMR (500 MHz, DMSO-d6) δ 10.57 (s, 1H), 8.77 (s, 2H), 8.22 (s, 1H), 8.13 (d, *J =* 7.4 Hz, 1H), 7.86 (d, *J* = 8.7 Hz, 1H), 7.64 (d, *J =* 8.1 Hz, 1H), 7.39 (d, *J =* 2.4 Hz, 1H), 7.32 (d, *J =* 8.1 Hz, 1H), 7.14 (dd, *J =* 8.8, 2.4 Hz, 1H), 6.10 (s, 2H), 4.78 (d, *J =* 13.3 Hz, 3H), 4.56 (q, *J =* 7.2, 5.8 Hz, 1H), 3.90 - 3.74 (m, 3H), 3.31 - 3.26 (m, 4H), 3.03 (td, *J =* 13.2, 2.7 Hz, 2H), 2.80 (t, *J =* 6.7 Hz, 2H), 2.20 (s, 1H), 2.15 - 2.05 (m, 2H), 1.92 (t, *J =* 7.4 Hz, 4H), 1.51 (p, *J =* 6.3, 5.8 Hz, 4H), 1.30 - 1.14 (m, 3H).

### Example 139: Synthesis of Compound 139

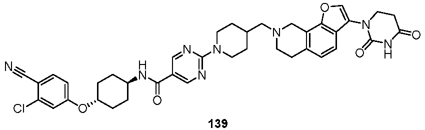

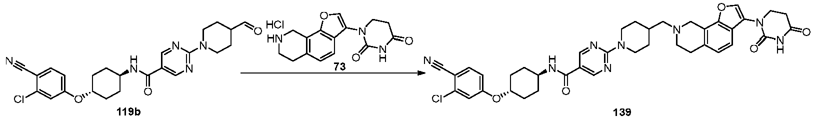

Intermediate **73** (80 mg), intermediate **119b** (116 mg), sodium acetate (40.8 mg), 1,2-dichloroethane (5 mL), isopropanol (1 mL), and sodium triacetoxyborohydride (105 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give compound **139** (119 mg).

MS(ESI, [M+H]⁺) m/z: 737.1.

¹H NMR (500 MHz, DMSO-d6) δ 10.51 (s, 1H), 8.74 (s, 2H), 8.09 (d, J = 7.5 Hz, 1H), 8.05 (s, 1H), 7.86 (d, J = 8.8 Hz, 1H), 7.40 - 7.36 (m, 2H), 7.14 (dd, J = 8.8, 2.5 Hz, 1H), 7.04 (d, J = 8.2 Hz, 1H), 4.73 (d, J = 13.2 Hz, 2H), 4.55 (dt, J = 9.8, 5.6 Hz, 1H), 3.83 (t, J = 6.6 Hz, 2H), 3.78 (s, 3H), 3.03 - 2.97 (m, 2H), 2.92 (t, J = 5.8 Hz, 2H), 2.78 (t, J = 6.7 Hz, 2H), 2.75 - 2.71 (m, 2H), 2.42 (d, J = 7.2 Hz, 2H), 2.13 - 2.08 (m, 2H), 2.02 (s, 1H), 1.91 (t, J = 5.7 Hz, 2H), 1.87 - 1.82 (m, 2H), 1.53 - 1.47 (m, 4H), 1.13 - 1.05 (m, 2H).

### Example 140: Synthesis of Compound 140

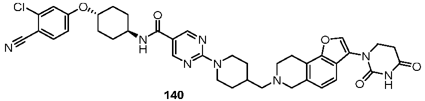

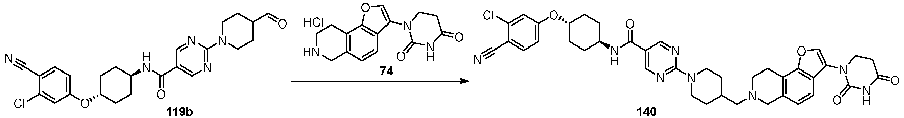

Intermediate **74** (80 mg), intermediate **119b** (116 mg), sodium acetate (40.8 mg), 1,2-dichloroethane (5 mL), isopropanol (1 mL), and sodium triacetoxyborohydride (105 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give compound **140** (40 mg).

MS(ESI, [M+H]⁺) m/z: 737.1.

¹H NMR (500 MHz, DMSO-d6) δ 10.51 (s, 1H), 8.74 (s, 2H), 8.14 - 8.03 (m, 2H), 7.86 (d, J = 8.8 Hz, 1H), 7.42 - 7.33 (m, 2H), 7.14 (dd, J = 8.8, 2.5 Hz, 1H), 6.99 (d, J = 8.2 Hz, 1H), 4.73 (dt, J = 13.0, 3.4 Hz, 2H), 4.59 - 4.50 (m, 1H), 3.83 (t, J = 6.7 Hz, 3H), 3.66 (s, 2H), 3.04 - 2.91 (m, 4H), 2.77 (dt, J = 13.3, 6.4 Hz, 4H), 2.37 (d, J = 7.1 Hz, 2H), 2.15 - 2.06 (m, 2H), 1.99 (d, J = 8.8 Hz, 1H), 1.96 - 1.88 (m, 2H), 1.88 - 1.80 (m, 2H), 1.56 - 1.44 (m, 4H), 1.08 (qd, J = 12.4, 4.1 Hz, 2H).

### Example 141: Synthesis of Compound 141

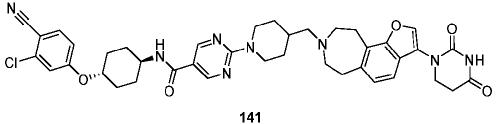

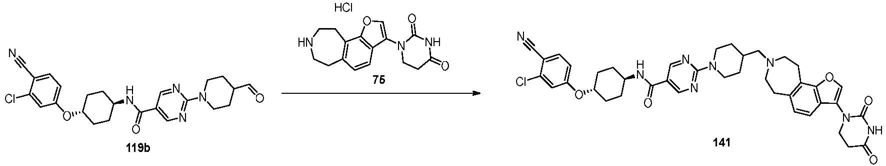

### Step 1: Preparation of compound 141

**119b** (110 mg), intermediate **75** (93 mg), sodium acetate (49 mg), and acetonitrile (20 mL) were added to a reaction flask in sequence, and the mixture was stirred at room temperature for 10 min. Sodium triacetoxyborohydride (110 mg) was then added, and the mixture was reacted at room temperature for 2 h. After the reaction was completed, a saturated sodium bicarbonate solution was added to the reaction system to quench the reaction, and the mixture was extracted with dichloromethane. The organic layer was collected, dried over anhydrous sodium sulfate, and filtered. The filtrate was concentrated, and then the residue was purified by silica gel column chromatography to give the target compound 141 (70 mg).

MS(ESI, [M+H]⁺) m/z: 751.1.

¹H NMR (500 MHz, DMSO-d6) δ 10.50 (s, 1H), 8.75 (s, 2H), 8.10 (d, *J =* 7.5 Hz, 1H), 8.05 (s, 1H), 7.86 (d, *J* = 8.8 Hz, 1H), 7.39 (d, *J* = 2.5 Hz, 1H), 7.29 (d, *J* = 7.8 Hz, 1H), 7.14 (dd, *J* = 8.8, 2.5 Hz, 1H), 7.06 (d, *J =* 8.0 Hz, 1H), 4.74 (d, *J* = 13.1 Hz, 2H), 4.55 (dt, *J* = 9.7, 5.8 Hz, 1H), 3.82 (t, *J* = 6.7 Hz, 3H), 3.12 (d, *J* = 6.8 Hz, 2H), 3.05 - 2.93 (m, 4H), 2.77 (t, *J* = 6.7 Hz, 2H), 2.70 - 2.56 (m, 4H), 2.32 (d, *J* = 6.7 Hz, 2H), 2.16 - 2.06 (m, 2H), 1.95 - 1.89 (m, 3H), 1.85 (d, *J* = 13.1 Hz, 2H), 1.50 (p, *J* = 6.4, 5.8 Hz, 4H), 1.13 - 1.01 (m, 2H).

### Example 142: Synthesis of Compound 142

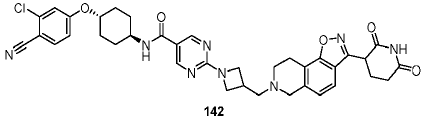

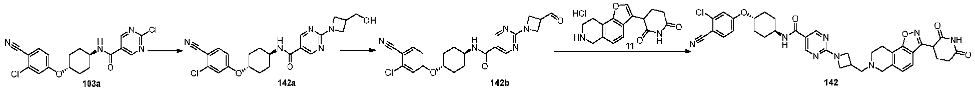

### Step 1: Preparation of intermediate 142a

Intermediate **103a** (500 mg), 3-azetidinemethanol hydrochloride (174 mg), DIPEA (496 mg, 0.670 mL), and DMSO (5 mL) were added to a reaction flask in sequence, and the mixture was heated to 100 °C and reacted. After the reaction was completed, the reaction solution was extracted with ethyl acetate (100 mL) and water (200 mL). The organic phase was separated, washed with saturated brine, dried over anhydrous sodium sulfate, and filtered, and the filtrate was concentrated, slurried with ethyl acetate, and filtered. The filter cake was collected and dried under reduced pressure to give the target intermediate **142a** (352 mg).

MS(ESI, [M+H]⁺) m/z: 442.2.

### Step 2: Preparation of intermediate 142b

Intermediate **142a** (150 mg), DCM (10 mL), and Dess-Martin periodinane (288 mg) were added to a reaction flask in sequence, and the mixture was reacted at room temperature. After the reaction was completed, the reaction solution was quenched with a saturated sodium thiosulfate solution (100 mL) and a sodium bicarbonate solution (100 mL) and extracted with dichloromethane (100 mL). The organic phase was separated, washed with saturated brine, dried over anhydrous sodium sulfate, and filtered, and the filtrate was purified by silica gel column chromatography to give the target intermediate **142b** (157 mg).

### Step 3: Preparation of compound 142

Intermediate **11** (110 mg), intermediate **142b** (150 mg), sodium acetate (56.1 mg), 1,2-dichloroethane (5 mL), isopropanol (1 mL), and sodium triacetoxyborohydride (145 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give compound **142** (160 mg).

MS(ESI, [M+H]⁺) m/z: 709.1.

¹H NMR (500 MHz, DMSO-d6) δ 11.08 (s, 1H), 8.74 (s, 2H), 8.24 - 8.03 (m, 1H), 7.86 (d, J = 8.9 Hz, 1H), 7.58 (d, J = 8.3 Hz, 1H), 7.38 (s, 1H), 7.12 (dd, J = 16.9, 8.5 Hz, 2H), 4.56 (d, J = 12.2 Hz, 2H), 4.38 - 4.12 (m, 2H), 3.78 (d, J = 34.0 Hz, 5H), 3.17 (s, 1H), 3.04 (d, J = 37.3 Hz, 3H), 2.81 (t, J = 17.4 Hz, 5H), 2.61 (d, J = 17.5 Hz, 1H), 2.15 (d, J = 44.3 Hz, 3H), 1.92 (s, 2H), 1.50 (s, 4H).

### Example 143: Synthesis of Compound 143

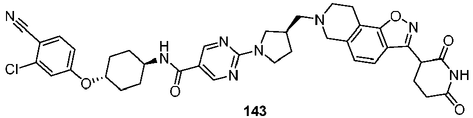

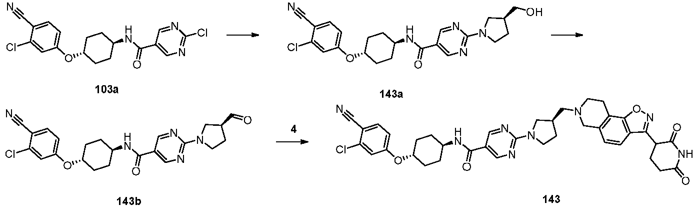

### Step 1: Preparation of intermediate 143a

Intermediate **103a** (300 mg), (R)-pyrrolidine-3-methanol hydrochloride (158 mg), DIPEA (396 mg), and DMSO (15 mL) were added to a reaction flask in sequence, and the mixture was warmed to 70 °C and reacted for 1 h. After the reaction was completed, the reaction solution was extracted with water (50 mL) and ethyl acetate (50 mL). The organic phases were combined, washed with a saturated sodium chloride solution, dried over anhydrous sodium sulfate, and filtered, and the filtrate was concentrated by evaporation under reduced pressure to remove the solvent, thus giving the target intermediate **143a** (300 mg).

MS(ESI, [M+H]⁺) m/z: 456.1.

### Step 2: Preparation of intermediate 143b

143a (0.3 g), Dess-Martin periodinane (0.78 g), and dichloromethane (30 mL) were added to a reaction flask in sequence, and the mixture was reacted at room temperature for 1 h. After the reaction was completed, dichloromethane (70 mL) and water (100 mL) were added. The organic phase was separated, washed with a saturated sodium chloride solution, dried over anhydrous sodium sulfate, filtered, and concentrated to give intermediate **143b** (0.37 g).

### Step 3: Preparation of compound 143

**143b** (105 mg), intermediate **4** (78 mg), sodium acetate (65 mg), and DCE/isopropanol (5:1, 20 mL) were added to a reaction flask in sequence, and the mixture was reacted at room temperature for 30 min. Sodium cyanoborohydride (55 mg) was then added, and the mixture was reacted at room temperature. After the reaction was completed, the reaction solution was concentrated by rotary evaporation to remove the solvent, and the crude product was separated and purified by silica gel column chromatography to give compound **143** (68 mg).

MS(ESI, [M+H]⁺) m/z: 723.4.

¹H NMR (500 MHz, DMSO-d6) δ 11.08 (s, 1H), 8.74 (d, *J =* 6.0 Hz, 2H), 8.10 (d, *J =* 7.5 Hz, 1H), 7.86 (d, *J =* 8.7 Hz, 1H), 7.58 (d, *J =* 8.1 Hz, 1H), 7.38 (d, *J =* 2.4 Hz, 1H), 7.16 - 7.07 (m, 2H), 4.56 (dt, *J =* 13.4, 6.7 Hz, 2H), 3.85 - 3.65 (m, 5H), 3.51 (dt, *J =* 11.4, 7.6 Hz, 1H), 3.28 (dd, *J =* 11.5, 7.2 Hz, 1H), 3.01 (t, *J =* 5.9 Hz, 2H), 2.89 - 2.67 (m, 4H), 2.62 (t, *J =* 4.3 Hz, 1H), 2.59 (d, *J = 7.0* Hz, 2H), 2.47 (dd, *J =* 12.1, 4.5 Hz, 1H), 2.19 (dq, *J =* 13.5, 4.8 Hz, 1H), 2.12 (dt, *J =* 18.2, 4.1 Hz, 3H), 1.98 - 1.87 (m, 2H), 1.75 (dq, *J =* 12.2, 8.1 Hz, 1H), 1.56 - 1.45 (m, 4H).

### Example 144: Synthesis of Compound 144

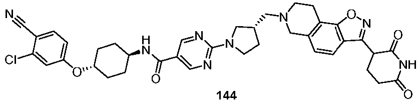

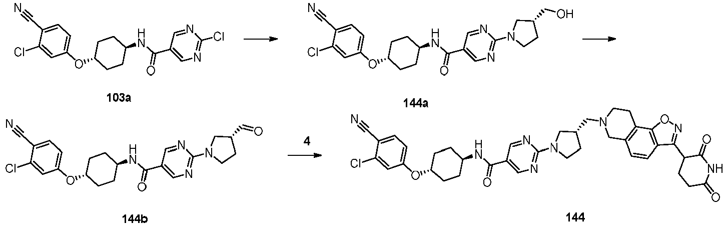

### Step 1: Preparation of intermediate 144a

Intermediate **103a** (300 mg), (S)-pyrrolidine-3-methanol hydrochloride (158 mg), DIPEA (396 mg), and DMSO (15 mL) were added to a reaction flask in sequence, and the mixture was warmed to 70 °C and reacted for 1 h. After the reaction was completed, the reaction solution was extracted with water (50 mL) and ethyl acetate (50 mL). The organic phases were combined, washed with a saturated sodium chloride solution, dried over anhydrous sodium sulfate, and filtered, and the filtrate was concentrated by evaporation under reduced pressure to remove the solvent, thus giving the target intermediate **144a** (310 mg).

MS(ESI, [M+H]⁺) m/z: 456.1.

### Step 2: Preparation of intermediate 144b

**144a** (0.3 g), Dess-Martin periodinane (0.78 g), and dichloromethane (30 mL) were added to a reaction flask in sequence, and the mixture was reacted at room temperature for 1 h. After the reaction was completed, dichloromethane (70 mL) and water (100 mL) were added. The organic phase was separated, washed with a saturated sodium chloride solution, dried over anhydrous sodium sulfate, filtered, and concentrated to give intermediate **144b** (0.32 g).

### Step 3: Preparation of compound 144

**144b** (105 mg), intermediate **4** (78 mg), sodium acetate (65 mg), and DCE/isopropanol (5:1, 20 mL) were added to a reaction flask in sequence, and the mixture was reacted at room temperature for 30 min. Sodium cyanoborohydride (55 mg) was then added, and the mixture was reacted at room temperature. After the reaction was completed, the reaction solution was concentrated by rotary evaporation to remove the solvent, and the crude product was separated and purified by silica gel column chromatography to give compound **144** (78 mg).

MS(ESI, [M+H]⁺) m/z: 723.4.

¹H NMR (500 MHz, DMSO-d6) δ 11.08 (s, 1H), 8.74 (d, *J =* 6.0 Hz, 2H), 8.10 (d, *J =* 7.5 Hz, 1H), 7.86 (d, *J =* 8.8 Hz, 1H), 7.58 (d, *J =* 8.1 Hz, 1H), 7.38 (d, *J =* 2.4 Hz, 1H), 7.18 - 7.07 (m, 2H), 4.56 (dd, *J =* 11.9, 5.0 Hz, 2H), 3.86 - 3.65 (m, 5H), 3.51 (dt, *J =* 11.3, 7.6 Hz, 1H), 3.28 (dd, *J =* 11.6, 7.1 Hz, 1H), 3.01 (t, *J =* 5.9 Hz, 2H), 2.77 (dddd, *J =* 39.4, 27.7, 14.9, 6.5 Hz, 4H), 2.60 (dd, *J =* 17.2, 5.7 Hz, 3H), 2.47 (dd, *J =* 12.1, 4.4 Hz, 1H), 2.19 (dq, *J =* 13.2, 4.6 Hz, 1H), 2.12 (d, *J =* 13.9 Hz, 3H), 1.91 (s, 2H), 1.75 (dq, *J =* 12.2, 8.1 Hz, 1H), 1.57 - 1.44 (m, 4H). **Example 145:** Synthesis of Compound **145**

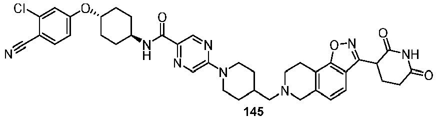

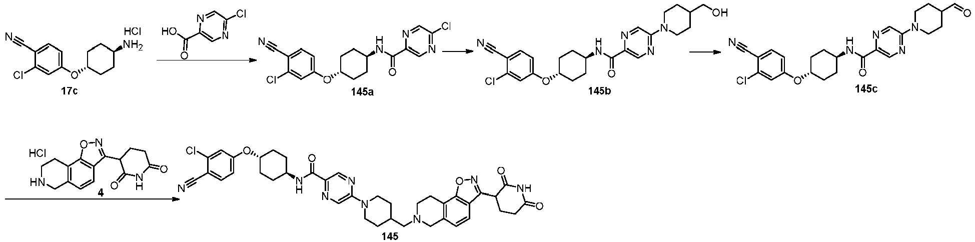

### Step 1: Preparation of intermediate 145a

Intermediate **17c** (2 g), 5-chloropyrazine-2-carboxylic acid (1.14 g), DCM (20 mL), HATU (3.18 g), and DIPEA (3.64 mL) were added to a reaction flask in sequence, and the mixture was reacted at room temperature. After the reaction was completed, the reaction solution was concentrated by evaporation under reduced pressure to remove the solvent and extracted with ethyl acetate and water. The organic phase was separated, washed with saturated brine, dried over anhydrous sodium sulfate, and filtered, the filtrate was concentrated, and the residue was purified by silica gel column chromatography to give the target intermediate **145a** (2.5 g).

### Step 2: Preparation of intermediate 145b

Intermediate **145a** (2 g), 4-hydroxymethylpiperidine (0.648 g), DIPEA (2.67 mL), and DMSO (20 mL) were added to a reaction flask in sequence, and the mixture was heated to 100 °C and reacted. After the reaction was completed, the reaction solution was extracted with ethyl acetate and water. The organic phase was separated, washed with saturated brine, dried over anhydrous sodium sulfate, and filtered, the filtrate was concentrated, and the residue was purified by silica gel column chromatography to give the target intermediate **145b** (2.04 g).

MS (ESI, [M+H]⁺) m/z: 470.28.

### Step 3: Preparation of intermediate 145c

Intermediate **145b** (0.6 g), 2-iodoxybenzoic acid (0.71 g), and DMSO (10 mL) were added to a reaction flask in sequence, and the mixture was reacted at room temperature. After the reaction was completed, ethyl acetate and a saturated sodium bicarbonate solution were added for extraction. The organic phase was dried over anhydrous sodium sulfate and filtered, and the filtrate was purified by silica gel column chromatography to give compound **145c** (0.56 g).

### Step 4: Preparation of compound 145

Intermediate **4** (85 mg), intermediate **145c** (124 mg), sodium acetate (43.3 mg), 1,2-dichloroethane (5 mL), isopropanol (1 mL), and sodium triacetoxyborohydride (112 mg) were added to a reaction flask in sequence, and the mixture was reacted at room temperature. After the reaction was completed, dichloromethane (50 mL) and water (100 mL) were added for extraction. The organic phase was separated, dried over anhydrous sodium sulfate, and filtered, and the filtrate was purified by silica gel column chromatography to give compound **145** (128 mg).

MS(ESI, [M+H]⁺) m/z: 737.2.

¹H NMR (500 MHz, DMSO-d6) δ 11.08 (s, 1H), 8.59 (d, J = 1.3 Hz, 1H), 8.25 (d, J = 1.4 Hz, 1H), 8.06 (d, J = 8.3 Hz, 1H), 7.86 (d, J = 8.8 Hz, 1H), 7.58 (d, J = 8.2 Hz, 1H), 7.37 (d, J = 2.4 Hz, 1H), 7.15 - 7.08 (m, 2H), 4.56 (dd, J = 11.8, 5.0 Hz, 1H), 4.54 - 4.42 (m, 3H), 3.87 - 3.80 (m, 1H), 3.71 (s, 2H), 3.07 - 2.96 (m, 4H), 2.77 (td, J = 11.9, 9.9, 5.8 Hz, 3H), 2.61 (dt, J = 17.3, 4.3 Hz, 1H), 2.47 (dd, J = 12.2, 4.4 Hz, 1H), 2.39 (d, J = 7.2 Hz, 2H), 2.19 (dq, J = 13.5, 4.8 Hz, 1H), 2.09 (dd, J = 12.4, 4.1 Hz, 2H), 2.02 (t, J = 3.6 Hz, 1H), 1.92 - 1.82 (m, 4H), 1.65 - 1.56 (m, 2H), 1.51 (td, J = 12.9, 6.3 Hz, 2H), 1.20 - 1.11 (m, 2H).

### Example 146: Synthesis of Compound 146

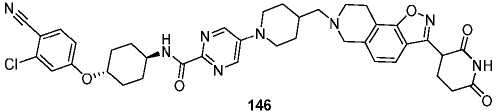

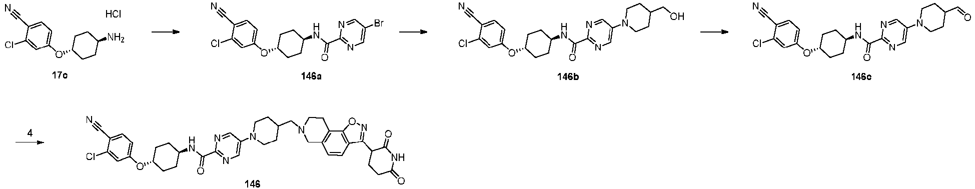

### Step 1: Preparation of intermediate 146a

Intermediate **17c** (2.83 g), 5-bromopyrimidine-2-carboxylic acid (2.0 g), HATU (4.50 g), DIPEA (6.37 g), and DMF (30 mL) were added to a reaction flask in sequence, and the mixture was reacted at room temperature for 1 h. After the reaction was completed, the reaction solution was extracted with water (50 mL) and ethyl acetate (50 mL). The organic phases were combined, washed with a saturated sodium chloride solution, dried over anhydrous sodium sulfate, and filtered, and the filtrate was concentrated by evaporation under reduced pressure to remove the solvent. The crude product was purified by silica gel column chromatography to give the target intermediate **146a** (1.1 g). Step 2: Preparation of intermediate **146b**

**146a** (800 mg), 4-hydroxymethylpiperidine (254 mg), BINAP (114 mg), palladium acetate (45 mg), cesium carbonate (1.7 g), and 1,4-dioxane (50 mL) were added to a reaction flask in sequence, and the mixture was heated to 100 °C and reacted under N₂ atmosphere. After the reaction was completed, ethyl acetate (50 mL) and water (50 mL) were added for extraction. The organic phase was separated, dried over anhydrous sodium sulfate, and filtered, and the filtrate was purified by silica gel column chromatography to give the target intermediate **146b** (0.52 g).

MS(ESI, [M+H]⁺) m/z: 470.1.

### Step 3: Preparation of intermediate 146c

**146b** (0.32 g), Dess-Martin periodinane (0.65 g), and dichloromethane (30 mL) were added to a reaction flask in sequence, and the mixture was reacted at room temperature for 1 h. After the reaction was completed, dichloromethane (70 mL) and water (100 mL) were added. The organic phase was separated, washed with a saturated sodium chloride solution, dried over anhydrous sodium sulfate, filtered, and concentrated to give intermediate **146c** (0.32 g).

### Step 4: Preparation of compound 146

**146c** (132 mg), intermediate **4** (85 mg), sodium acetate (68 mg), and DCE/isopropanol (5:1, 20 mL) were added to a reaction flask in sequence, and the mixture was reacted at room temperature for 30 min. Sodium cyanoborohydride (62 mg) was then added, and the mixture was reacted at room temperature. After the reaction was completed, the reaction solution was concentrated by rotary evaporation to remove the solvent, and the crude product was separated and purified by silica gel column chromatography to give compound **146** (92 mg).

MS(ESI, [M+H]⁺) m/z: 737.4.

¹H NMR (500 MHz, DMSO-d6) δ 11.08 (s, 1H), 8.52 (s, 2H), 8.29 (d, *J =* 8.2 Hz, 1H), 7.86 (d, *J =* 8.7 Hz, 1H), 7.58 (d, *J=* 8.2 Hz, 1H), 7.37 (d, *J=* 2.5 Hz, 1H), 7.16 - 7.08 (m, 2H), 4.61 - 4.49 (m, 2H), 3.99 *(d, J=* 12.9 Hz, 2H), 3.86 - 3.77 (m, 1H), 3.72 (s, 2H), 3.00 (t, *J =* 5.9 Hz, 2H), 2.91 (td, *J =* 12.7, 12.3, 2.7 Hz, 2H), 2.83 - 2.74 (m, 3H), 2.61 (dt, *J=* 17.3, 4.4 Hz, 1H), 2.47 (dd, *J=* 12.1, 4.4 Hz, 1H), 2.40 *(d, J=* 7.1 Hz, 2H), 2.23 - 2.15 (m, 1H), 2.13 - 2.06 (m, 2H), 1.98 - 1.83 (m, 5H), 1.66 - 1.47 (m, 4H), 1.24 (q, *J=* 11.7 Hz, 2H).

### Test Example 1: Effect of Compounds on AR Degradation in Cells

### 1.1 Assay on degradation activity for AR in VCaP cells

The detection was carried out using the HTRF HUMAN ANDROGEN RECEPTOR DETECTION KIT (cisbio, 64ANDRPEG), 4× Supplemented Lysis buffer was prepared using the Blocking reagent stock solution (100×) and Lysis buffer stock solution (4×), and 1× Supplemented Lysis buffer was prepared using the 4× Supplemented Lysis buffer and ddH₂O. Premixed antibody solutions were prepared using Detection buffer, Androgen Receptor Eu Cryptate antibody, and Androgen Receptor d2 antibody.

VCaP cells in a good growth state were taken, washed with PBS, digested with pancreatin, and added with 2% CSS-FBS phenol red-free DMEM complete medium to terminate the digestion. The cells were collected by using a centrifuge tube, adjusted to a cell density of 1 × 10⁶ cells/mL, and seeded in a 96-well plate (100 µL/well). The cells were incubated overnight in a cell incubator. Compounds were added using a nanoliter pipettor such that the final concentrations of the compounds were 1000 nM-0.977 nM. Two duplicate wells were set for each concentration, and meanwhile, a control was set. After another 24 h of incubation in the cell incubator, the medium was pipetted out, the 1× Supplemented Lysis buffer was added at 40 µL/well to lyse the cells. After the cells were oscillated for 40 min at room temperature, 16 µL of the cell lysate was added to a 384-well plate, the Premixed antibody solutions were added at 4 µL/well, and the mixture was left to stand at 25 °C for 2 h. Fluorescence values were detected at 665 nm/620 nm using an Envision microplate reader, the four-parameter analysis was performed, the dose-response curves were fit, and DC₅₀ (the concentration of the drug when the degradation rate reaches 50%) and Dmax (the maximum degradation rate) were calculated. The results are shown in Table 1.

**Table 1**

| Compound | Degradation activity for AR in VCaP cells | Compound | Degradation activity for AR in VCaP cells | Compound | Degradation activity for AR in VCaP cells |
|---|---|---|---|---|---|
| | (DC50, nM) | | (DC50, nM) | | (DC50, nM) |
| 17 | ≤1 | 82 | ≤1 | 98 | ≤1 |
| 26 | ≤1 | 86 | ≤1 | 102 | ≤1 |
| 27 | ≤1 | 87 | ≤1 | 107 | ≤1 |
| 34 | ≤1 | 88 | ≤1 | 108 | ≤1 |
| 36 | ≤1 | 90 | ≤1 | 109 | ≤1 |
| 37 | ≤1 | 91 | ≤1 | 110 | ≤1 |
| 48 | ≤1 | 92 | ≤1 | 113 | ≤1 |
| 58 | ≤1 | 94 | ≤1 | 117 | ≤1 |
| 71 | ≤1 | 95 | ≤1 | 118 | ≤1 |
| 80 | ≤1 | 96 | ≤1 | 145 | ≤1 |
| 81 | ≤1 | 97 | ≤1 | | |

### 1.2 Assay on degradation activity for AR in LNCaP cells

The detection was carried out using the HTRF HUMAN ANDROGEN RECEPTOR DETECTION KIT (cisbio, 64ANDRPEG), 4× Supplemented Lysis buffer was prepared using the Blocking reagent stock solution (100×) and Lysis buffer stock solution (4×), and 1× Supplemented Lysis buffer was prepared using the 4× Supplemented Lysis buffer and ddH₂O. Premixed antibody solutions were prepared using Detection buffer, Androgen Receptor Eu Cryptate antibody, and Androgen Receptor d2 antibody.

LNCaP cells in a good growth state were taken, washed with PBS, digested with pancreatin, and added with 2% CSS-FBS phenol red-free 1640 complete medium to terminate the digestion. The cells were collected by using a centrifuge tube, adjusted to a cell density of 1 × 10⁶ cells/mL, and seeded in a 96-well plate (100 µL/well). The cells were incubated overnight in a cell incubator. Compounds were added using a nanoliter pipettor such that the final concentrations of the compounds were 500 nM-0.488 nM. Two duplicate wells were set for each concentration, and meanwhile, a control was set. After another 24 h of incubation in the cell incubator, the medium was pipetted out, the 1× Supplemented Lysis buffer was added at 40 µL/well to lyse the cells. After the cells were oscillated for 40 min at room temperature, 16 µL of the cell lysate was added to a 384-well plate, the Premixed antibody solutions were added at 4 µL/well, and the mixture was left to stand at 25 °C for 2 h. Fluorescence values were detected at 665 nm/620 nm using an Envision microplate reader, the four-parameter analysis was performed, the dose-response curves were fit, and DC₅₀ (the concentration of the drug when the degradation rate reaches 50%) and Dmax (the maximum degradation rate) were calculated. The results are shown in Table 2.

**Table 2**

| Compound | Degradation activity for AR in LNCaP cells | | Compound | Degradation activity for AR in LNCaP cells | |
|---|---|---|---|---|---|
| | (DC50, nM) | (Dmax,%) | | (DC50, nM) | (Dmax,%) |
| 18 | ≤20 | >80% | 97 | ≤20 | >80% |
| 19 | ≤20 | >80% | 98 | ≤20 | >80% |
| 20 | ≤20 | >80% | 102 | ≤20 | >80% |
| 34 | ≤20 | >80% | 107 | ≤20 | >80% |
| 35 | ≤20 | >80% | 109 | ≤20 | >80% |
| 36 | ≤20 | >80% | 110 | ≤20 | >80% |
| 37 | ≤20 | >80% | 117 | ≤20 | >80% |
| 47 | ≤20 | >80% | 118 | ≤20 | >80% |
| 48 | ≤20 | >80% | 122 | ≤20 | >80% |
| 90 | ≤20 | >80% | 145 | ≤20 | >80% |
| 91 | ≤20 | >80% | | | |

The test results show that the compounds of the present application exhibit degradation activity against the ARs in VCaP cells and LNCaP cells.

### Test Example 2: Inhibitory Activity for In Vitro Cell Proliferation

### 2.1 Assay on inhibitory activity for VCaP cell proliferation

VCaP cells in a good growth state were taken, washed with PBS, digested with pancreatin, and added with 5% CSS-FBS phenol red-free DMEM complete medium to terminate the digestion. The cells were collected by using a centrifuge tube, adjusted to a cell density of 1 × 10⁵ cells/mL, and seeded in a 96-well plate (100 µL/well). Meanwhile, compounds were added using a nanoliter pipettor such that the final concentrations of the compounds were 10000 nM-0.61 nM. Two duplicate wells were set for each concentration, and meanwhile, a control was set. After another 168 h of incubation in the cell incubator, the detection reagent CCK-8 (manufacturer: Dojindo, 10 µL/well) was added. After 2.0 h of incubation in the cell incubator, absorbance values were detected at 450 nm using a PerkinElmer Envision microplate reader, the four-parameter analysis was performed, the dose-response curves were fit, and IC₅₀ was calculated. The results are shown in Table 3.

**Table 3**

| Compound | Inhibitory activity for VCaP cell proliferation (IC₅₀, nM) | Compound | Inhibitory activity for VCaP cell proliferation (IC₅₀, nM) | Compound | Inhibitory activity for VCaP cell proliferation (IC₅₀, nM) |
|---|---|---|---|---|---|
| 17 | <1000 | 97 | <10 | 122 | <100 |
| 26 | <100 | 98 | <100 | 123 | <1000 |
| 27 | <100 | 99 | <100 | 124 | <1000 |
| 28 | <100 | 101 | <10 | 125 | <1000 |
| 34 | <100 | 102 | <10 | 127 | <100 |
| 35 | <100 | 103 | <10 | 128 | <100 |
| 36 | <10 | 104 | <10 | 129 | <100 |
| 37 | <100 | 105 | <10 | 130 | <100 |
| 41 | <10 | 106 | <100 | 131 | <100 |
| 42 | <10 | 107 | <10 | 132 | <1000 |
| 46 | <10 | 108 | <10 | 133 | <1000 |
| 47 | <10 | 109 | <100 | 134 | <100 |
| 48 | <100 | 110 | <100 | 136 | <1000 |
| 58 | <10 | 111 | <100 | 137 | <1000 |
| 59 | <10 | 112 | <1000 | 139 | <1000 |
| 68 | <10 | 113 | <100 | 142 | <1000 |
| 90 | <10 | 114 | <100 | 145 | <100 |
| 91 | <10 | 115 | <1000 | 146 | <1000 |
| 92 | <10 | 117 | <100 | | |
| 93 | <10 | 118 | <100 | | |
| 94 | <100 | 119 | <100 | | |
| 95 | <100 | 120 | <1000 | | |
| 96 | <10 | 121 | <10 | | |

### 2.2 Assay on inhibitory activity for LNCaP cell proliferation

LNCaP cells in a good growth state were taken, washed with PBS, digested with pancreatin, and added with 2% CSS-FBS phenol red-free 1640 complete medium to terminate the digestion. The cells were collected by using a centrifuge tube, adjusted to a cell density of 1 × 10⁵ cells/mL, and seeded in a 96-well plate (100 µL/well). Meanwhile, compounds were added using a nanoliter pipettor such that the final concentrations of the compounds were 10000 nM-0.61 nM. Two duplicate wells were set for each concentration, and meanwhile, a control was set. After another 168 h of incubation in the cell incubator, the detection reagent CCK-8 (manufacturer: Dojindo, 10 µL/well) was added. After 2.0 h of incubation in the cell incubator, absorbance values were detected at 450 nm using a PerkinElmer Envision microplate reader, the four-parameter analysis was performed, the dose-response curves were fit, and IC₅₀ was calculated. The results are shown in Table 4.

**Table 4**

| Compound | Inhibitory activity for LNCaP cell proliferation (IC₅₀, nM) | Compound | Inhibitory activity for LNCaP cell proliferation (IC₅₀, nM) | Compound | Inhibitory activity for LNCaP cell proliferation (IC₅₀, nM) |
|---|---|---|---|---|---|
| 17 | <1000 | 88 | <1000 | 120 | <1000 |
| 18 | <1000 | 91 | <1000 | 121 | <1000 |
| 19 | <1000 | 92 | <1000 | 122 | <1000 |
| 20 | <1000 | 94 | <1000 | 123 | <1000 |
| 21 | <1000 | 95 | <1000 | 128 | <1000 |
| 23 | <1000 | 96 | <1000 | 129 | <1000 |
| 24 | <1000 | 97 | <1000 | 130 | <1000 |
| 25 | <1000 | 98 | <1000 | 131 | <1000 |
| 26 | <1000 | 99 | <1000 | 132 | <1000 |
| 27 | <1000 | 100 | <1000 | 133 | <1000 |
| 28 | <1000 | 102 | <1000 | 134 | <1000 |
| 29 | <1000 | 107 | <1000 | 135 | <1000 |
| 30 | <1000 | 108 | <1000 | 136 | <1000 |
| 31 | <1000 | 109 | <1000 | 137 | <1000 |
| 32 | <1000 | 110 | <1000 | 138 | <1000 |
| 33 | <1000 | 111 | <1000 | 139 | <1000 |
| 34 | <1000 | 39 | <1000 | 44 | <1000 |
| 35 | <1000 | 40 | <1000 | 46 | <1000 |
| 36 | <1000 | 41 | <1000 | 48 | <1000 |
| 37 | <1000 | 43 | <1000 | 93 | <1000 |
| 38 | <1000 | 112 | <1000 | 140 | <1000 |
| 49 | <1000 | 113 | <1000 | 141 | <1000 |
| 50 | <1000 | 114 | <1000 | 142 | <1000 |
| 51 | <1000 | 116 | <1000 | 143 | <1000 |
| 52 | <1000 | 117 | <1000 | 144 | <1000 |
| 54 | <1000 | 118 | <1000 | 145 | <1000 |
| 55 | <1000 | 119 | <1000 | 146 | <1000 |
| 58 | <1000 | | | | |
| 82 | <1000 | | | | |

The compounds of the present application exhibit inhibitory activity for the proliferation of LNCaP cells and VCaP cells.

**Test Example *3. In Vitro* Stability in Liver Microsome**

Liver microsome incubation samples (species: human, monkey, rat, and mouse) were prepared by mixing a PBS buffer (pH = 7.4), a liver microsome solution (0.5 mg/mL), a test compound, and an NADPH + MgCl₂ solution and incubated at 37 °C and 300 rpm for 1 h. Zero-hour samples were prepared by mixing a PBS buffer (pH = 7.4), a liver microsome solution (0.5 mg/mL), and a test compound. An acetonitrile solution containing an internal standard was added to the samples, and supernatants were prepared by protein precipitation, diluted, and then assayed by LC/MS/MS. The results are shown in Table 5.

**Table 5**

| **Compound** | Human liver microsome | **Compound** | Human liver microsome |
|---|---|---|---|
| | Residuals at 60 min (%) | | Residuals at 60 min (%) |
| 17 | >85% | 27 | >85% |
| 18 | >85% | 34 | >85% |
| 21 | >85% | 47 | >85% |
| 48 | >85% | 48 | >85% |
| 24 | >85% | 94 | >85% |
| 25 | >85% | 90 | >85% |
| 26 | >85% | 119 | >85% |

The compounds of the present application exhibit *in vitro* metabolic stability in liver microsomes.

### Test Example 4: Mouse Pharmacokinetics

ICR mice weighing 18-22 g were randomized into groups of 9 after 3-5 days of acclimatization, and then intragastrically given the test compound solution at a dose of 10 mg/kg and intravenously injected with the test compound solution at a dose of 1 mg/kg.

Blood sampling time points for the intragastrical administration group were 15 min, 0.5 h, 1 h, 2 h, 3 h, 4 h, 6 h, 8 h, 10 h, and 24 h, and blood sampling time points for the intravenous injection administration group were 0.083 min, 15 min, 0.5 h, 1 h, 2 h, 4 h, 6 h, 8 h, 10 h, and 24 h; blood was collected from the orbit to prepare plasma samples to be tested. 30 µL of each of the plasma samples to be tested and a standard curve sample were taken, and an acetonitrile solution containing an internal standard was added. Supernatants were obtained by protein precipitation, diluted, and then assayed by LC/MS/MS. Pharmacokinetic parameters were fitted using a non-compartmental model (see Table 6).

**Table 6**

| Compound | Mouse i.v. 1 mpk | | | Mouse i.g. 10 mpk | | | |
|---|---|---|---|---|---|---|---|
| | AUC (ng*h/mL) | t_{1/2} (h) | Cl (L/hr/kg) | AUC (ng*h/mL) | t_{1/2} (h) | Cmax (ng/mL) | F(%) |
| 17 | 3164 | 7.28 | 0.283 | 14571 | 6.82 | 1161 | 46.05% |
| 18 | 5930 | | 0.162 | 18370 | 5.52 | 1197 | 30.98% |
| 19 | 2601 | 7.4 | 0.342 | 10821 | 49.9 | 562 | 41.61% |
| 26 | 5354 | 7.58 | 0.167 | 13249 | 11 | 886 | 24.75% |
| 27 | 5599 | 7.6 | 0.158 | 20072 | 5.47 | 1503 | 35.85% |
| 119 | 2717 | 11.5 | 0.348 | 10603 | 8.6 | 559 | 39.02% |

The compounds of the present application have good *in vivo* pharmacokinetic parameters and exhibit advantages in AUC, t_{1/2}, Cmax, or F (%).

All patents, patent applications, and other publications are explicitly incorporated herein by reference for the purpose of description and disclosure. These publications are provided solely because they were disclosed prior to the filing date of the present application. All statements as to the dates of these documents or descriptions as to the content of these documents are based on the information available to the applicant and do not constitute any admission as to the correctness of the dates or the content of these documents. Moreover, in any country or region, any reference to these publications herein shall not be construed as an admission that the publications form part of the commonly recognized knowledge in the art.

Those skilled in the art will recognize that the scope of the present application is not limited to the embodiments and examples described above. Instead, various modifications, substitutions, or recombinations may be made without departing from the spirit of the present application, and the embodiments resulting from these adjustments all fall within the protection scope of the present application.

## Claims

1. A compound of formula I-AA, a stereoisomer thereof, or a pharmaceutically acceptable salt thereof, 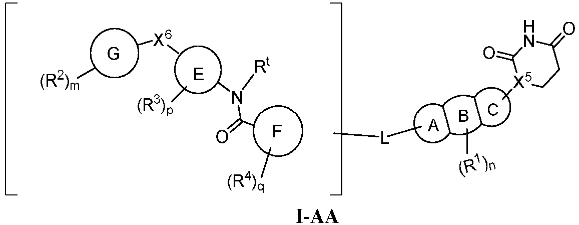 wherein,
ring A is absent or selected from the group consisting of C₅₋₁₅ cycloalkenyl, 5- to 15-membered heterocycloalkenyl, phenyl, and 5- to 6-membered heteroaryl;
ring B is selected from the group consisting of phenyl and 5- to 6-membered heteroaryl;
ring C is selected from the group consisting of 5- to 6-membered heteroaryl;
each R¹ is independently selected from the group consisting of halogen, -OH, -NH₂, -CN, C₁₋₁₀ alkyl, C₁₋₁₀ alkoxy, and halogenated C₁₋₁₀ alkyl, wherein the -OH, -NH₂, C₁₋₁₀ alkyl, C₁₋₁₀ alkoxy, or halogenated C₁₋₁₀ alkyl is optionally substituted with one or more substituents;
n is selected from the group consisting of 0, 1, 2, and 3;
L is selected from a connecting group;
X⁵ is selected from the group consisting of CH and N;
X⁶ is selected from the group consisting of -O-, -NH-, and -N(C₁₋₆ alkyl)-, wherein the -NH- or -N(C₁₋₆ alkyl)- is optionally substituted with one or more substituents;
each R², R³, and R⁴ is independently selected from the group consisting of halogen, -OH, -NH₂, -CN, C₁₋₁₀ alkyl, C₁₋₁₀ alkoxy, and halogenated C₁₋₁₀ alkyl, wherein the -OH, -NH₂, C₁₋₁₀ alkyl, C₁₋₁₀ alkoxy, or halogenated C₁₋₁₀ alkyl is optionally substituted with one or more substituents;
m, p, and q are each independently selected from the group consisting of 0, 1, 2, 3, and 4;
ring G is selected from the group consisting of C₆₋₁₀ aryl and 5- to 10-membered heteroaryl;
ring E is selected from the group consisting of C₃₋₁₀ cycloalkyl and 3- to 10-membered heterocycloalkyl;
ring F is selected from the group consisting of C₆₋₁₀ aryl and 5- to 10-membered heteroaryl;
R^{t} is selected from the group consisting of hydrogen, -OH, C₁₋₆ alkyl, C₃₋₁₀ cycloalkyl, and 3- to 10-membered heterocycloalkyl, wherein the C₁₋₆ alkyl, C₃₋₁₀ cycloalkyl, or 3- to 10-membered heterocycloalkyl is optionally substituted with one or more substituents.

2. The compound, the stereoisomer thereof, or the pharmaceutically acceptable salt thereof according to claim 1, wherein the compound of formula I-AA is selected from a compound of formula I-1, 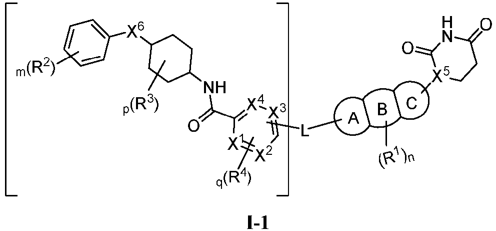 wherein,
ring A is absent or selected from the group consisting of C₅₋₁₀ cycloalkenyl, 5- to 10-membered heterocycloalkenyl, phenyl, and 5- to 6-membered heteroaryl;
ring B is selected from phenyl;
ring C is selected from the group consisting of isoxazolyl and furanyl;
each R¹ is independently selected from the group consisting of halogen, -OH, -NH₂, -CN, C₁₋₄ alkyl, C₁₋₄ alkoxy, and halogenated C₁₋₄ alkyl;
n is selected from the group consisting of 0, 1, 2, and 3;
L is selected from a connecting group;
X¹, X², X³, and X⁴ are each independently selected from the group consisting of N and CH;
X⁵ is selected from the group consisting of CH and N;
X⁶ is selected from the group consisting of -O-, -NH-, and -N(C₁₋₆ alkyl)-;
each R², R³, and R⁴ is independently selected from the group consisting of halogen, -OH, -NH₂, -CN, C₁₋₄ alkyl, C₁₋₄ alkoxy, and halogenated C₁₋₄ alkyl;
m, p, and q are each independently selected from the group consisting of 0, 1, 2, 3, and 4.

3. The compound, the stereoisomer thereof, or the pharmaceutically acceptable salt thereof according to claim 1 or 2, wherein ring A is absent or selected from the group consisting of C₅₋₇ membered cycloalkenyl, 5- to 10-membered heterocycloalkenyl, phenyl, and 5- to 6-membered heteroaryl; or
ring A is absent or selected from the group consisting of C₅₋₆ membered cycloalkenyl, 5- to 9-membered heterocycloalkenyl, phenyl, and 5- to 6-membered heteroaryl; or
ring A is absent or selected from the group consisting of cyclopentenyl, monocyclohexenyl, bicyclohexenyl, dihydropyrrolyl, tetrahydropyridinyl, tetrahydroazepinyl, azaspirooctenyl, azaspirononenyl, phenyl, pyrrolyl, pyrazolyl, furanyl, oxazolyl, and dihydrooxazinyl.

4. The compound, the stereoisomer thereof, or the pharmaceutically acceptable salt thereof according to any one of claims 1 to 3, wherein the structural fragment 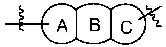 is selected from the group consisting of 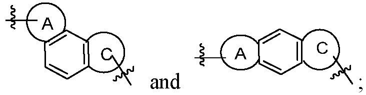 or
the structural fragment 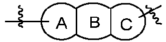
is selected from the group consisting of 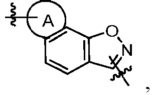 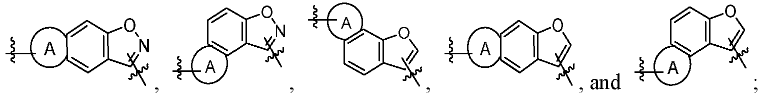 or
the structural fragment 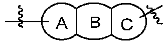
is selected from the group consisting of 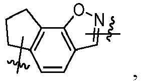 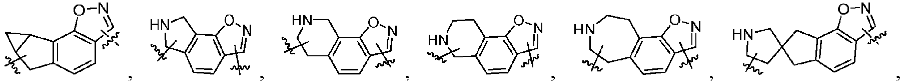 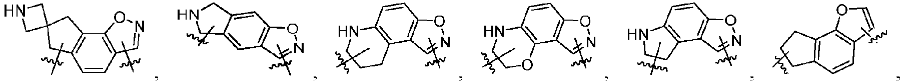 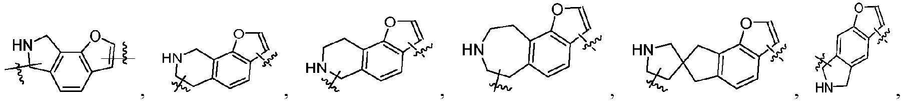 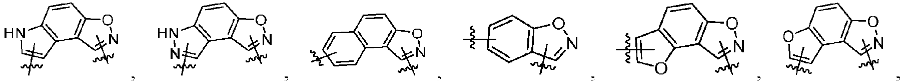 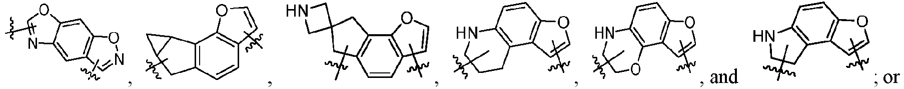
the structural fragment 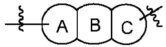
is selected from the group consisting of 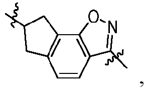 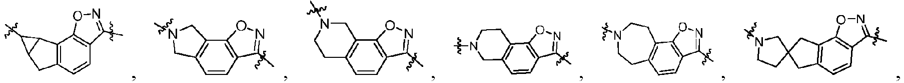 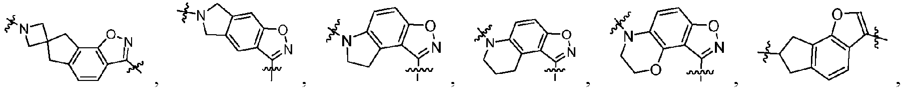 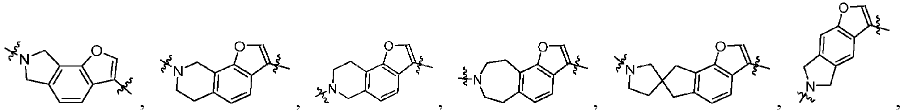 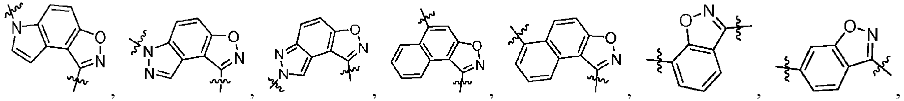 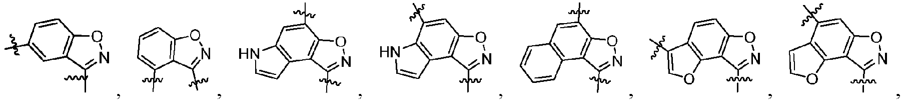 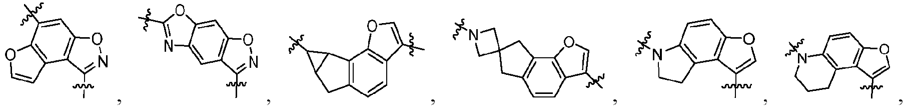
and

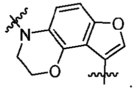

5. The compound, the stereoisomer thereof, or the pharmaceutically acceptable salt thereof according to any one of claims 1 to 4, wherein each R¹ is independently selected from the group consisting of halogen, -OH, -NH₂, -CN, C₁₋₃ alkyl, C₁₋₃ alkoxy, and halogenated C₁₋₃ alkyl; or
each R¹ is independently selected from the group consisting of fluorine, chlorine, bromine, -OH, -NH₂, and -CN.

6. The compound, the stereoisomer thereof, or the pharmaceutically acceptable salt thereof according to any one of claims 1 to 5, wherein L is selected from the group consisting of C₁₋₃₀ alkylene, C₂₋₃₀ alkenylene, and C₂₋₃₀ alkynylene, wherein one or more -CH₂- of the C₁₋₃₀ alkylene, C₂₋₃₀ alkenylene, or C₂₋₃₀ alkynylene are optionally substituted with -O-, C₃₋₁₂ cycloalkyl, 3- to 12-membered heterocycloalkyl, 4- to 12-membered heterocycloalkenyl, C₆₋₁₂ aryl, 5- to 12-membered heteroaryl, -NH-, -N(C₁₋₆ alkyl)-, or -S-, and the C₁₋₃₀ alkylene, C₂₋₃₀ alkenylene, or C₂₋₃₀ alkynylene is optionally substituted with one or more substituents; or
L is selected from the group consisting of -LNK¹-Cy¹-LNK-Cy²-LNK²-, -Cy¹-LNK-Cy²-LNK²-, -LNK¹-Cy¹-Cy²-LNK²-, -Cy¹-LNK-Cy²-, -Cy¹-Cy²-LNK²-, -LNK-Cy²-LNK²-, -Cy¹-LNK-, -Cy¹-Cy²-, and -Cy²-, wherein
Cy¹ is selected from the group consisting of a bond and the following groups optionally substituted with one or more R^{a}: C₃₋₁₂ cycloalkyl, 4- to 12-membered heterocycloalkyl, and 4- to 12-membered heterocycloalkenyl;
LNK, LNK¹, and LNK² are each independently selected from the group consisting of a bond, C₁₋₁₂ alkylene, and C₁₋₁₂ heteroalkylene;
Cy² is selected from the group consisting of a bond and the following groups optionally substituted with one or more R^{b}: C₃₋₁₂ cycloalkyl, 4- to 12-membered heterocycloalkyl, and 4- to 12-membered heterocycloalkenyl;
each R^{a} and R^{b} is independently selected from the group consisting of halogen, -OH, -NH₂, -CN, C₁₋₄ alkyl, C₁₋₄ alkoxy, halogenated C₁₋₄ alkyl, C₁₋₄ alkylamino, di-C₁₋₄ alkylamino, C₃₋₁₂ cycloalkyl, and 4- to 12-membered heterocycloalkyl.

7. The compound, the stereoisomer thereof, or the pharmaceutically acceptable salt thereof according to claim 6, wherein Cy¹ is selected from the group consisting of a bond and the following groups optionally substituted with one or more R^{a}: C₄₋₁₁ cycloalkyl, 4- to 11-membered heterocycloalkyl, and 4- to 11-membered heterocycloalkenyl; or
Cy¹ is selected from the group consisting of the following groups optionally substituted with one or more R^{a}: piperidinyl, diazaspirononanyl, piperazinyl, monoazaspirononanyl, cyclohexyl, spirononanyl, azetidinyl, octahydrocyclopentapyrrolyl, azabicyclononanyl, monoazaspiroundecanyl, diazaspiroundecanyl, pyrrolidinyl, and tetrahydropyridinyl; or
Cy¹ is selected from the group consisting of the following groups optionally substituted with one or more R^{a}:

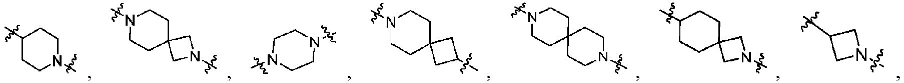

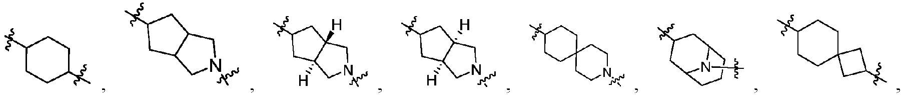

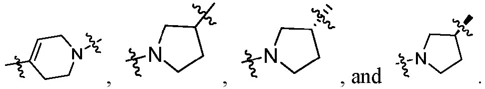

8. The compound, the stereoisomer thereof, or the pharmaceutically acceptable salt thereof according to claim 6, wherein Cy² is selected from the group consisting of a bond and the following groups optionally substituted with one or more R^{b}: C₄₋₁₁ cycloalkyl and 4- to 11-membered heterocycloalkyl; or
Cy² is selected from the group consisting of a bond and the following groups optionally substituted with one or more R^{b}: cyclobutyl, cyclopentyl, cyclohexyl, azetidinyl, pyrrolidinyl, and piperidinyl; or
Cy² is selected from the group consisting of a bond,

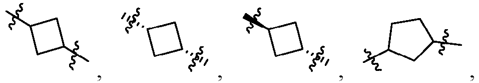

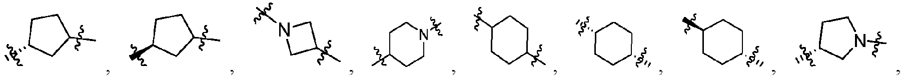

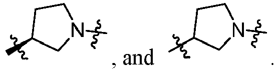

9. The compound, the stereoisomer thereof, or the pharmaceutically acceptable salt thereof according to claim 6, wherein the structural fragment -L- or -LNK¹-Cy¹-LNK-Cy²-LNK²- is selected from the group consisting of

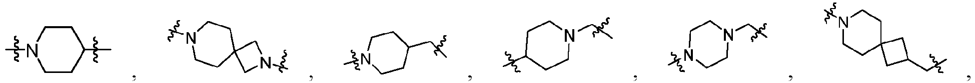

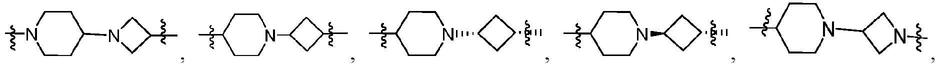

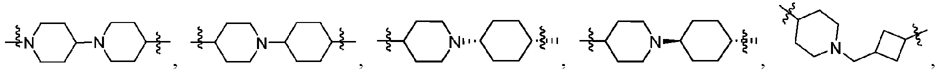

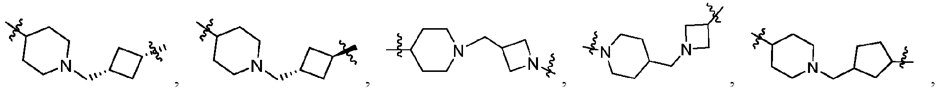

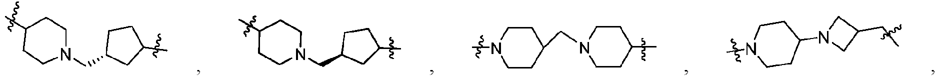

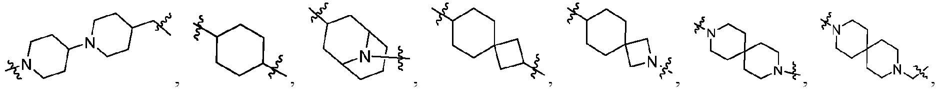

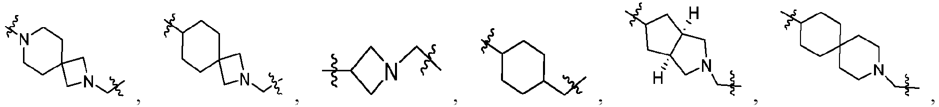

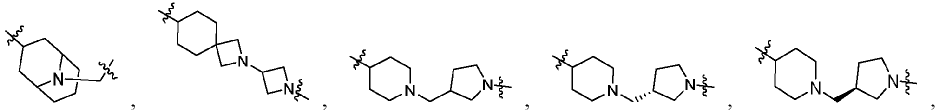

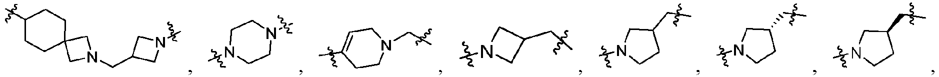

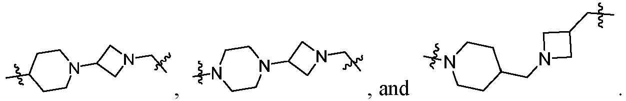

10. The compound, the stereoisomer thereof, or the pharmaceutically acceptable salt thereof according to any one of claims 1 to 9, wherein the structural fragment 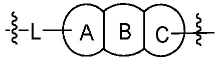 is selected from the group consisting of

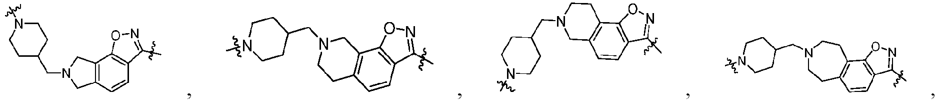

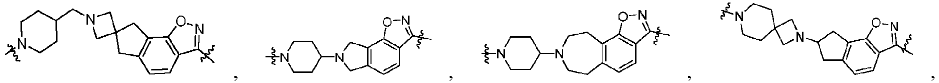

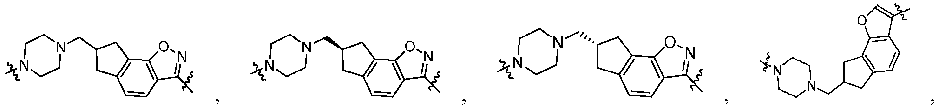

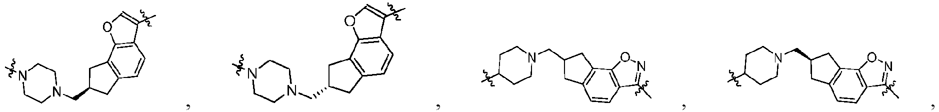

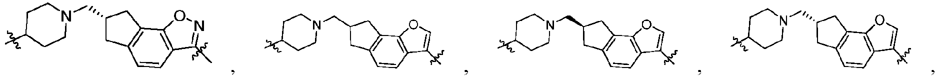

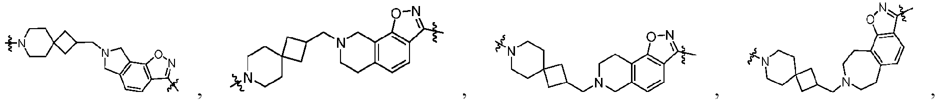

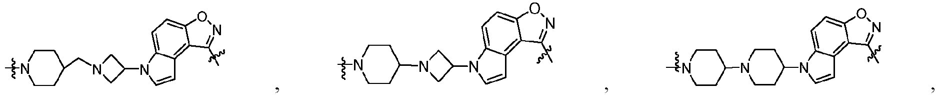

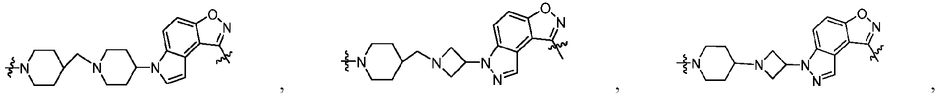

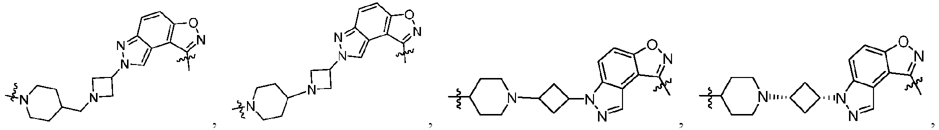

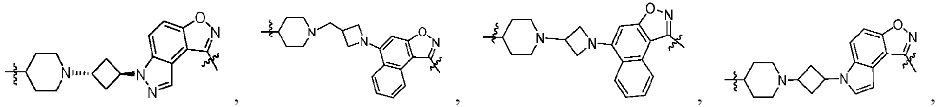

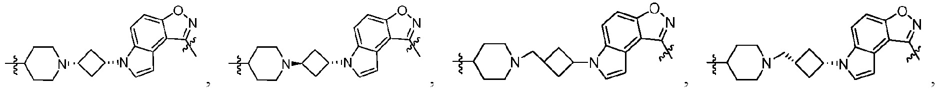

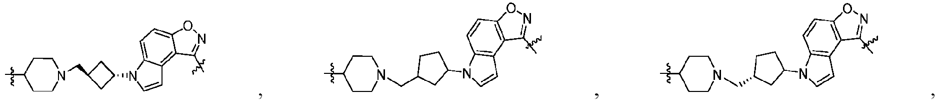

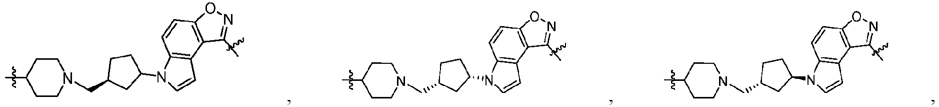

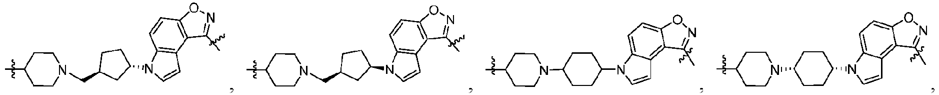

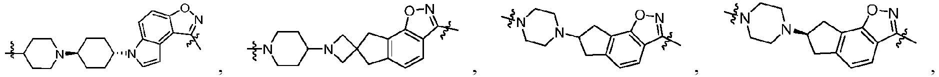

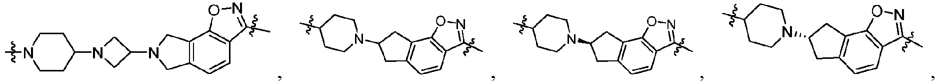

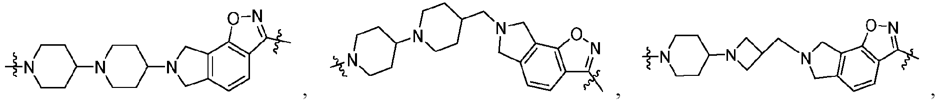

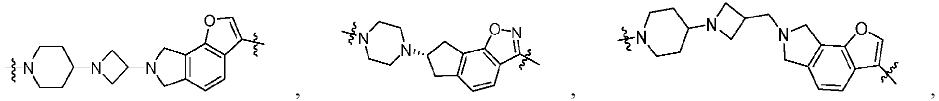

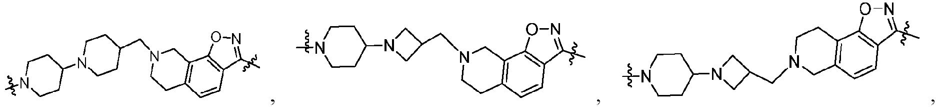

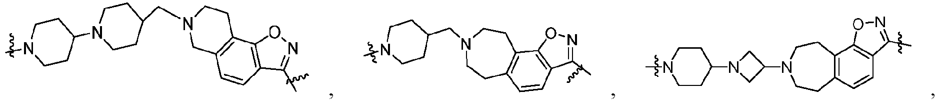

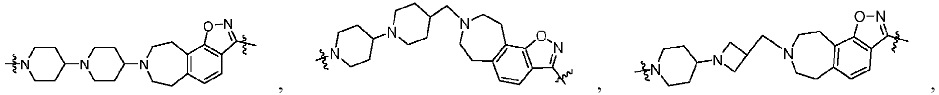

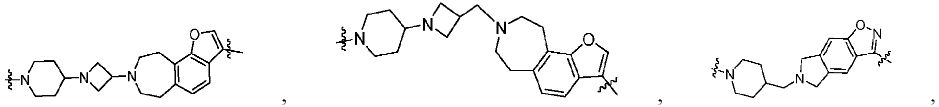

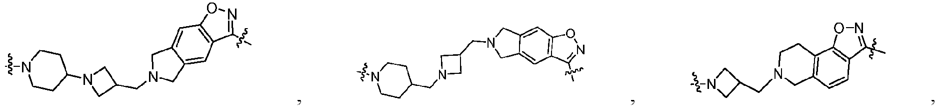

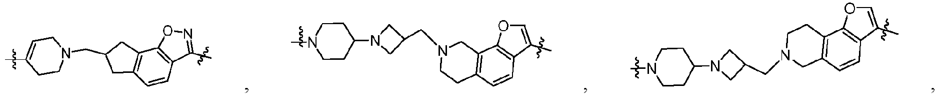

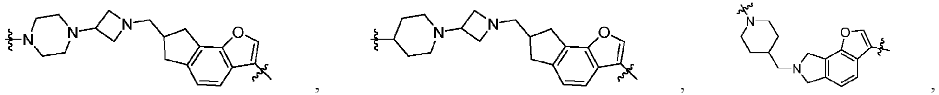

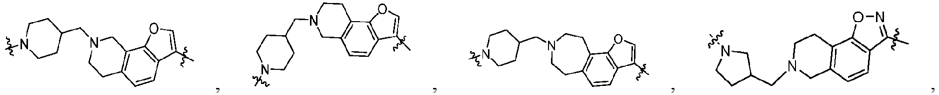

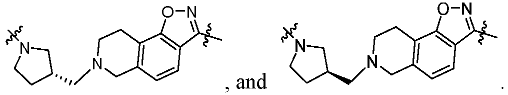

11. The compound, the stereoisomer thereof, or the pharmaceutically acceptable salt thereof according to claim 1 or 2, wherein the structural fragment 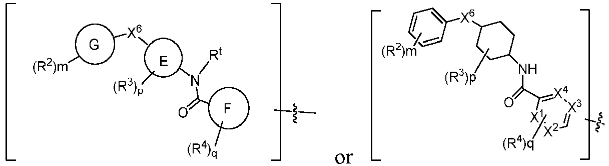 is selected from the group consisting of

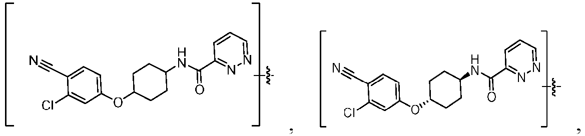

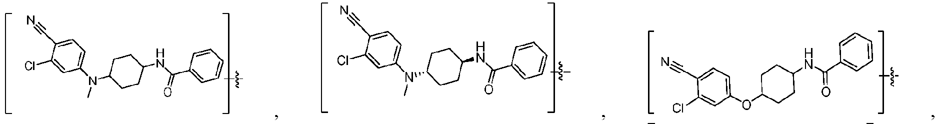

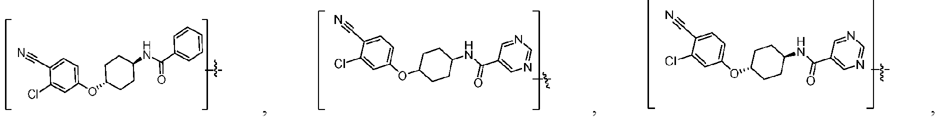

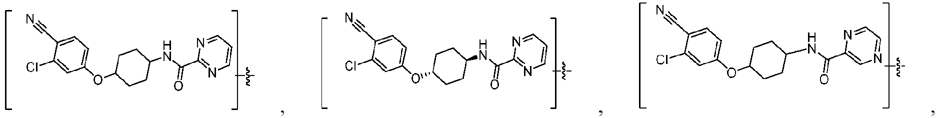

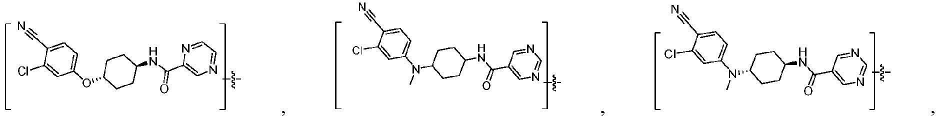

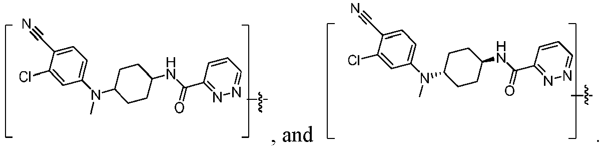

12. The compound, the stereoisomer thereof, or the pharmaceutically acceptable salt thereof according to any one of claims 1 to 11, wherein the compound of formula I-AA or the compound of formula I-1 is selected from the group consisting of compounds of formula I, formula I-A1, and formula I-A, 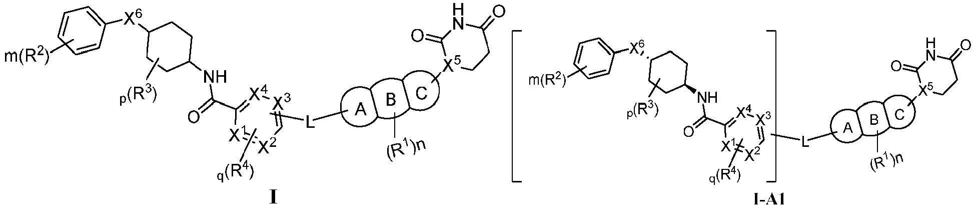 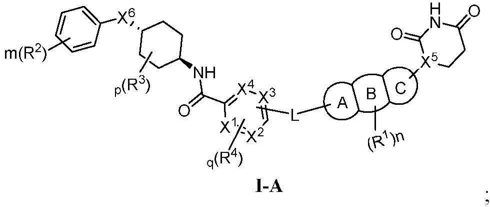 or
the compound of formula I-AA or the compound of formula I-1 is selected from the group consisting of compounds of formula I-1A, formula I-2A, formula I-3A, formula I-4A, formula I-5A, formula I-6A, formula I-7A, formula I-8A, formula I-9A, formula I-10A, formula I-11A, formula I-12A, formula I-13A, formula I-14A, formula I-15A, formula I-16A, and formula I-17A, wherein 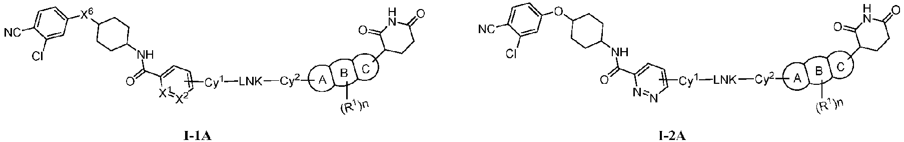 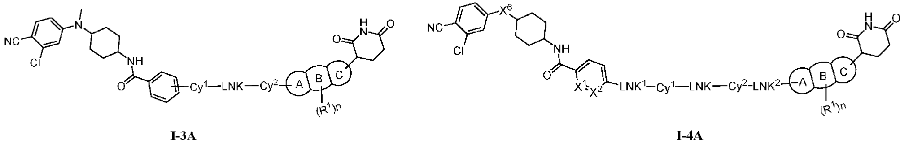 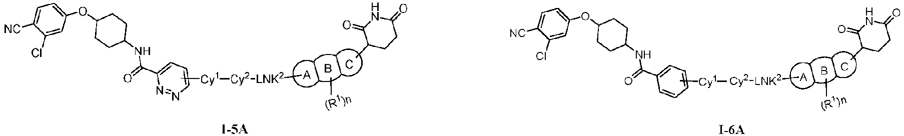 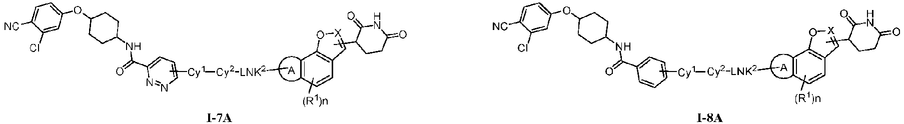 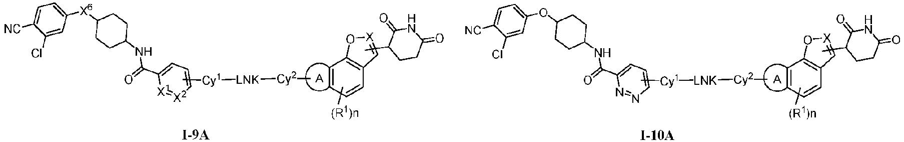 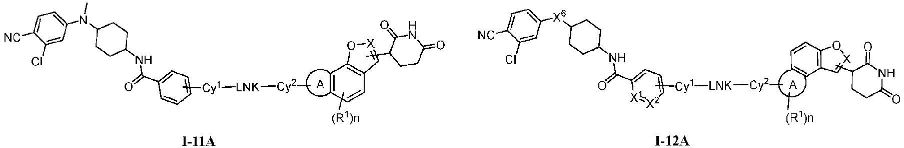 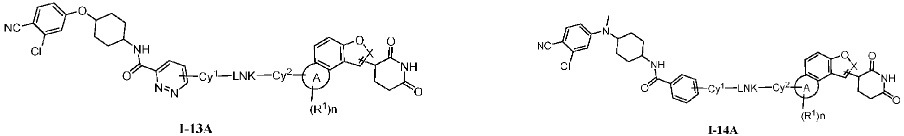 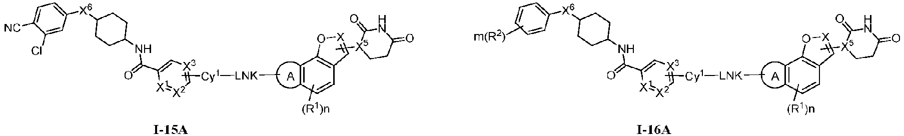 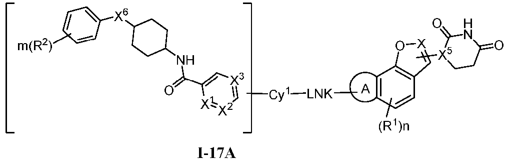
X is selected from the group consisting of CH and N; or
the compound of formula I-AA or the compound of formula I-1 is selected from the group consisting of compounds of formula I-1A-1, formula I-2A-1, formula I-3A-1, formula I-4A-1, formula I-5A-1, formula I-6A-1, formula I-7A-1, formula I-8A-1, formula I-9A-1, formula I-10A-1, formula I-11A-1, formula I-12A-1, formula I-13A-1, formula I-14A-1, formula I-15A-1, formula I-16A-1, and formula I-17A-1, wherein 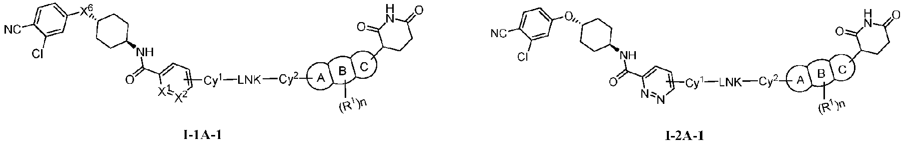 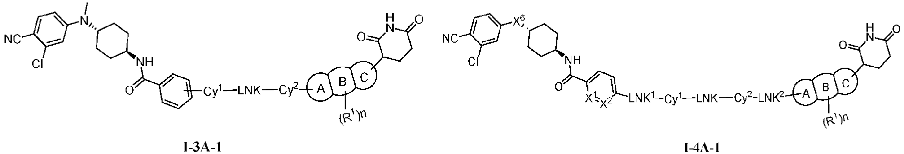 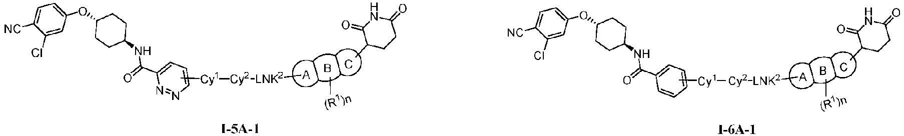 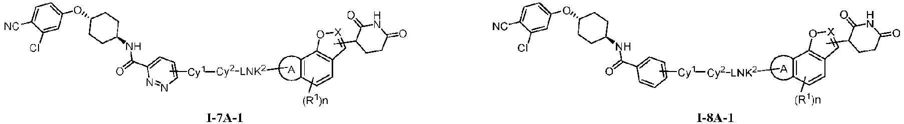 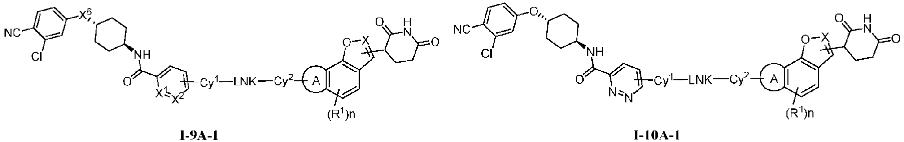 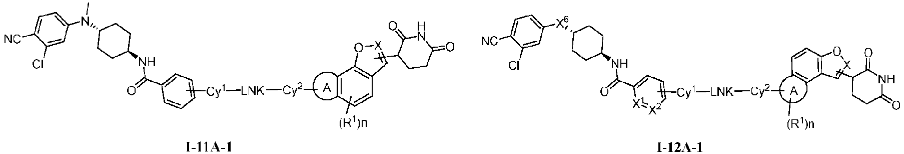 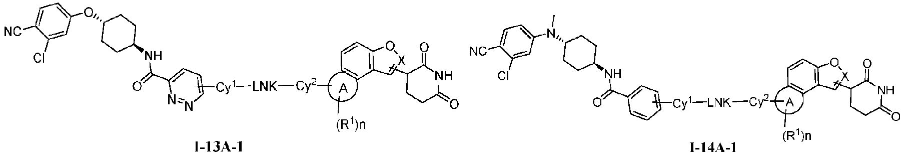 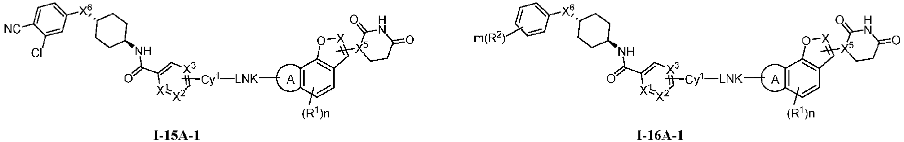 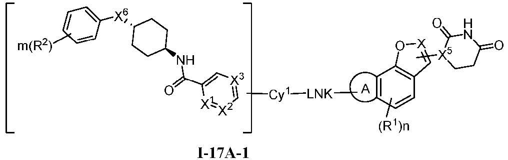
X is selected from the group consisting of CH and N.

13. The compound, the stereoisomer thereof, or the pharmaceutically acceptable salt thereof according to claim 1 or 2, wherein
ring A is selected from the group consisting of C₅₋₆ cycloalkenyl, 5- to 8-membered heterocycloalkenyl containing 1-3 heteroatoms selected from the group consisting of N, O, and S (e.g., 1-2 heteroatoms selected from the group consisting of N and O), phenyl, and 5- to 6-membered heteroaryl containing 1-3 heteroatoms selected from the group consisting of N, O, and S (e.g., 1-2 heteroatoms selected from the group consisting of N and O);
ring B is phenyl;
ring C is selected from the group consisting of isoxazolyl and furanyl;
each R¹ is independently selected from the group consisting of halogen, -OH, -NH₂, -CN, and C₁₋₃ alkyl (e.g., methyl, ethyl, or propyl);
n is selected from the group consisting of 0 and 1;
L is selected from LNK¹-Cy¹-LNK-Cy²-LNK²-, wherein LNK, LNK¹, and LNK² are each independently selected from the group consisting of a bond and C₁₋₃ alkylene, Cy¹ is selected from the group consisting of a bond, C₃₋₇ cycloalkyl, 4- to 7-membered heterocycloalkyl, and 5- to 7-membered heterocycloalkenyl, Cy² is selected from the group consisting of a bond, C₃₋₇ cycloalkyl, 4- to 7-membered heterocycloalkyl, and 5- to 7-membered heterocycloalkenyl, and Cy¹ and Cy² are not bonds at the same time;
X¹, X², X³, and X⁴ are each independently selected from the group consisting of N and CH;
X⁵ is selected from the group consisting of CH and N;
X⁶ is selected from the group consisting of -O-, -NH-, and -N(C₁₋₆ alkyl)-;
each R², R³, and R⁴ is independently selected from the group consisting of halogen, -OH, -NH₂, -CN, and C₁₋₃ alkyl (e.g., methyl, ethyl, or propyl);
m is selected from the group consisting of 1 and 2;
p and q are each independently selected from the group consisting of 0 and 1.

14. The compound, the stereoisomer thereof, or the pharmaceutically acceptable salt thereof according to any one of claims 1 to 13, wherein the compound is selected from the group consisting of the following compounds: 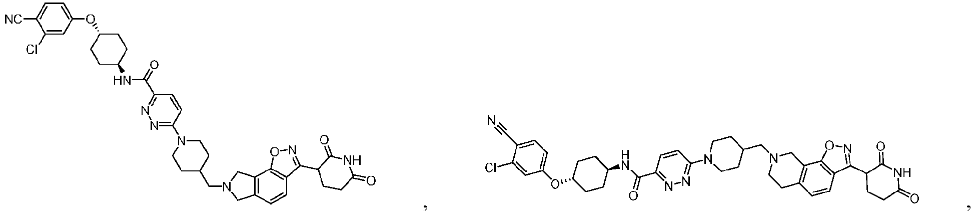 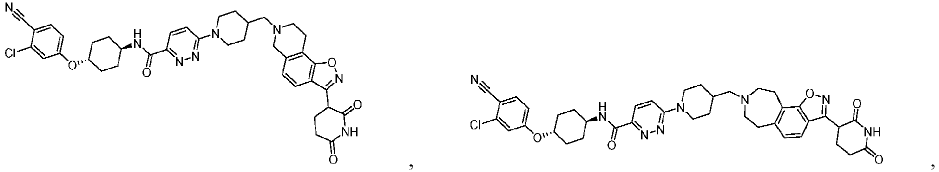 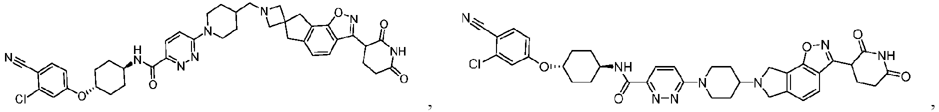 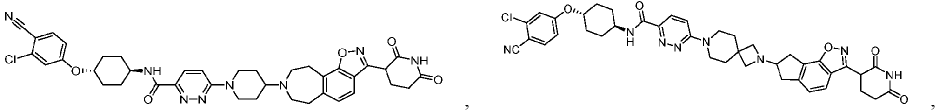 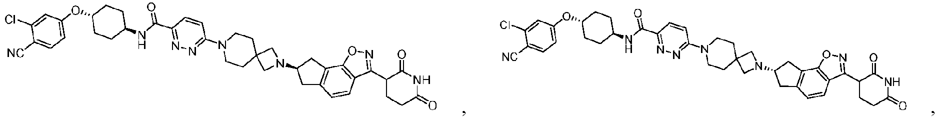 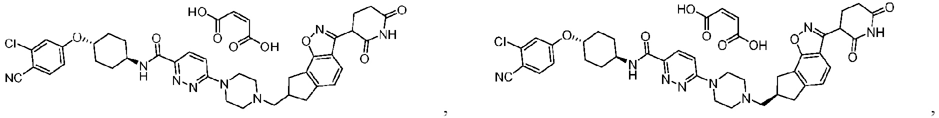 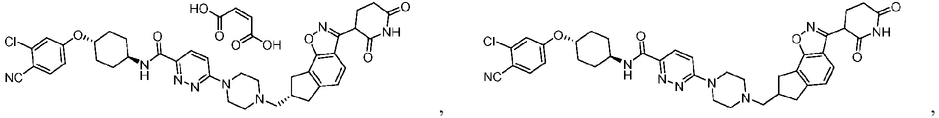 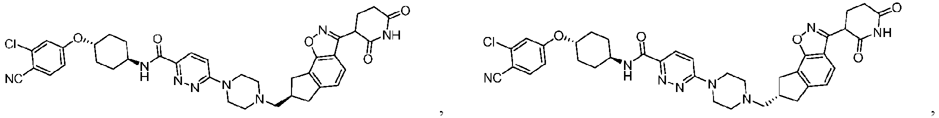 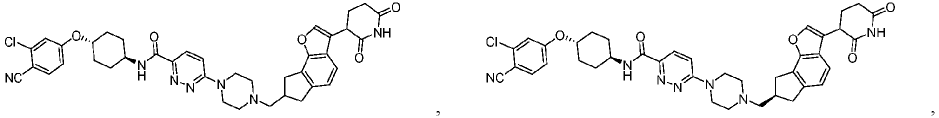 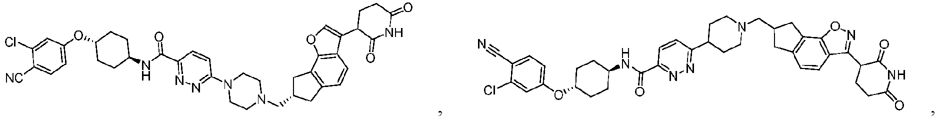 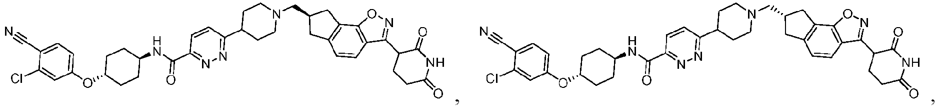 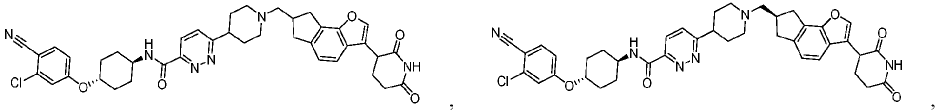 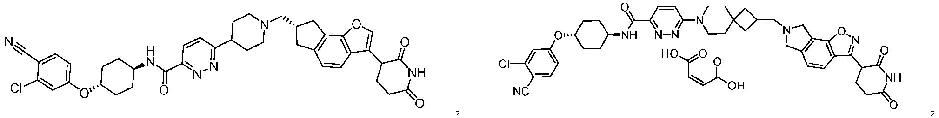 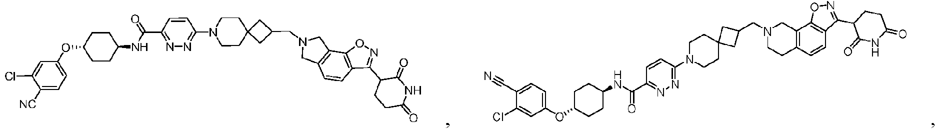 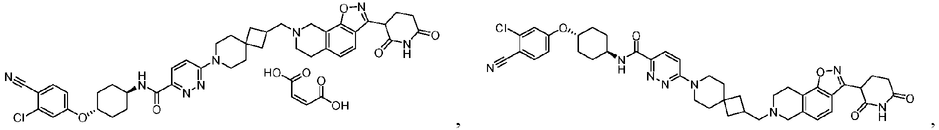 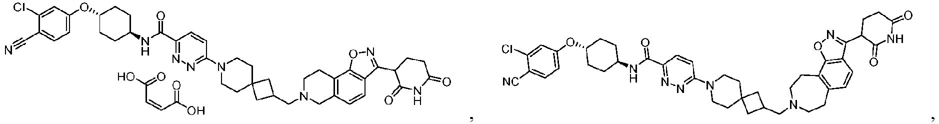 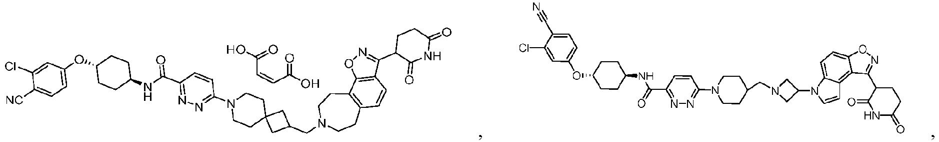 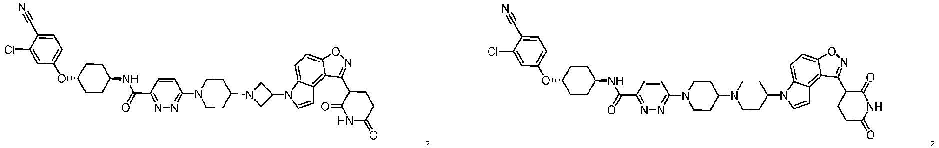 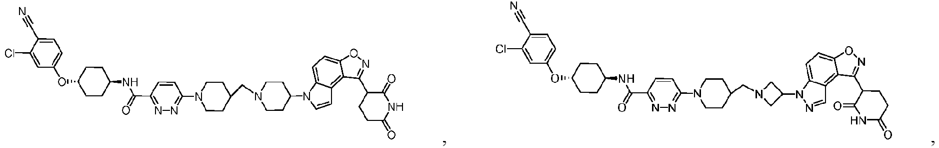 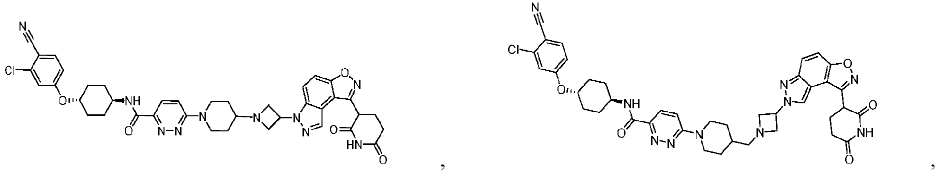 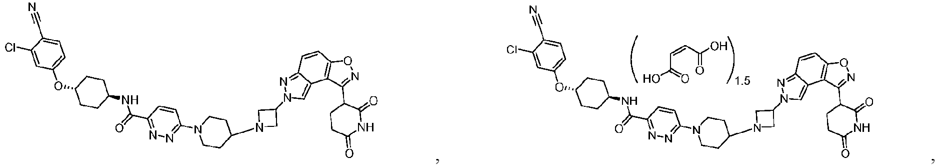 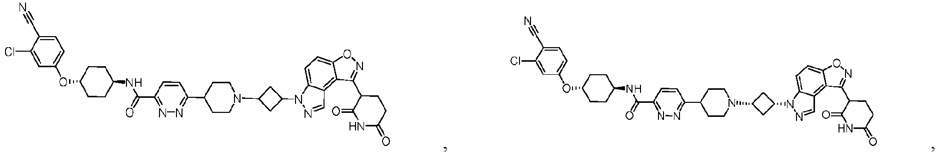 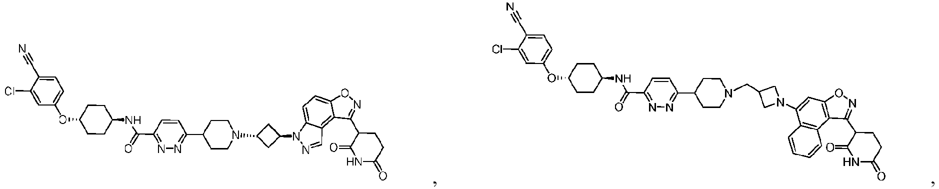 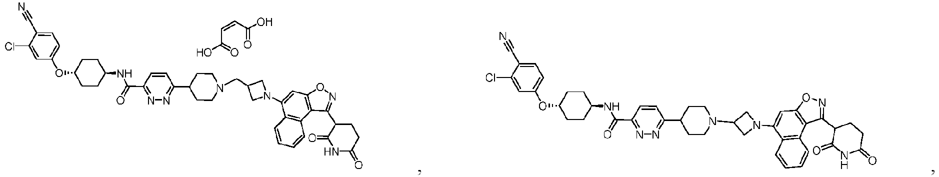 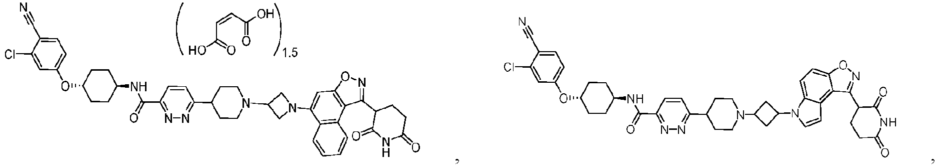 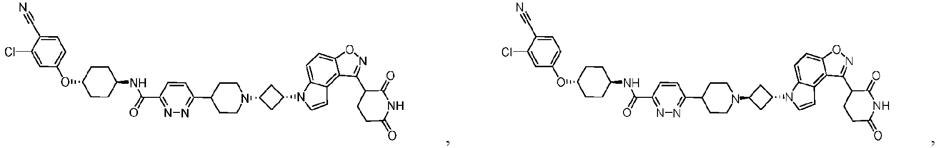 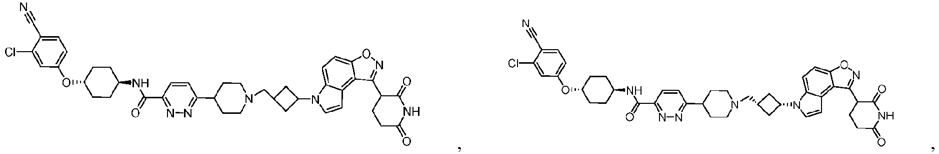 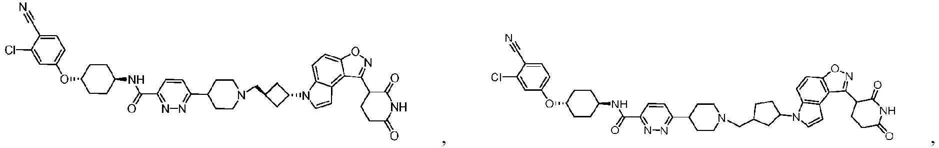 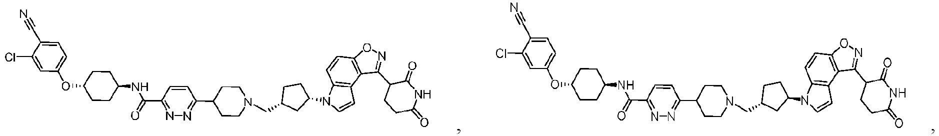 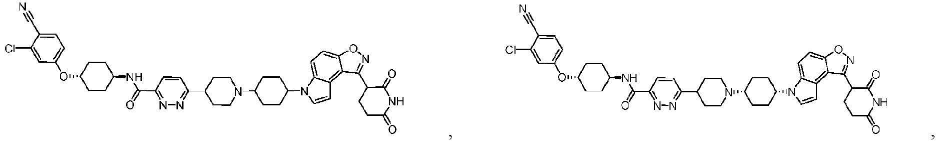 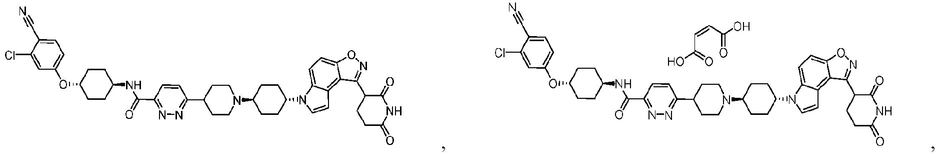 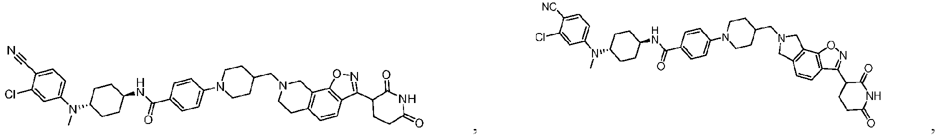 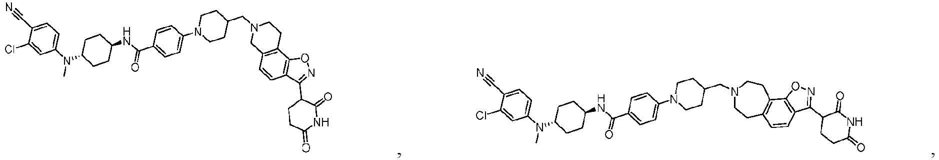 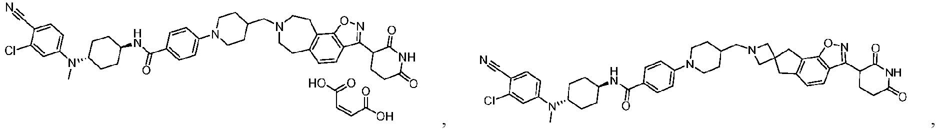 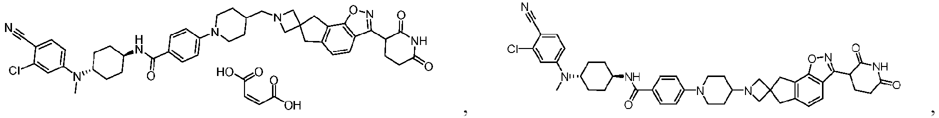 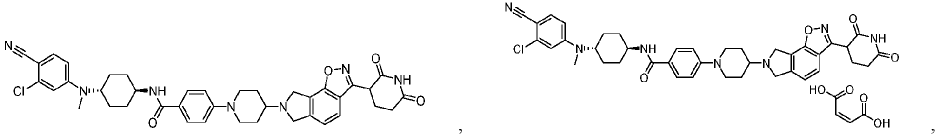 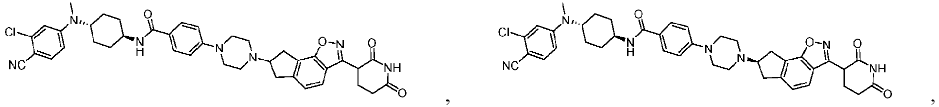 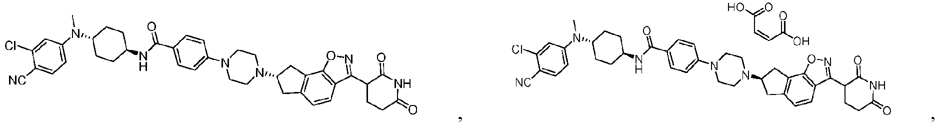 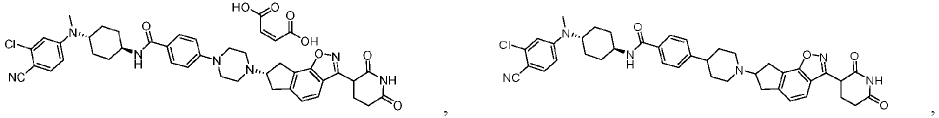 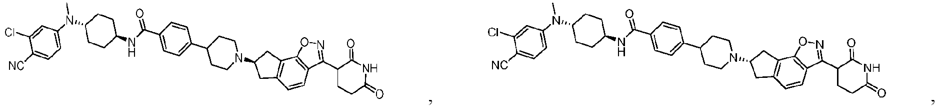 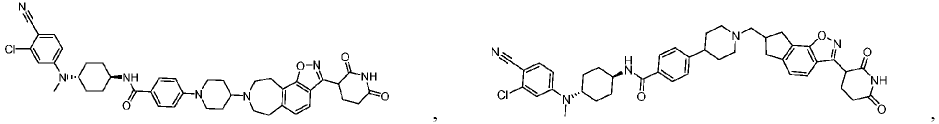 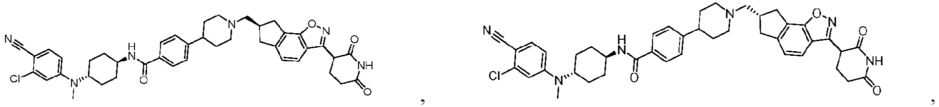 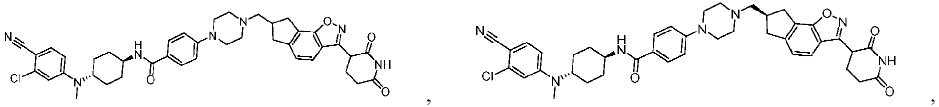 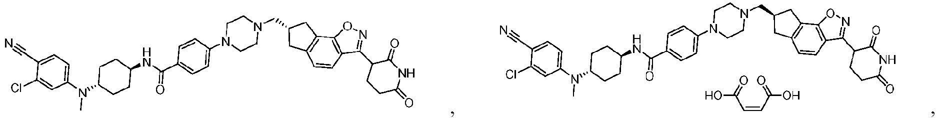 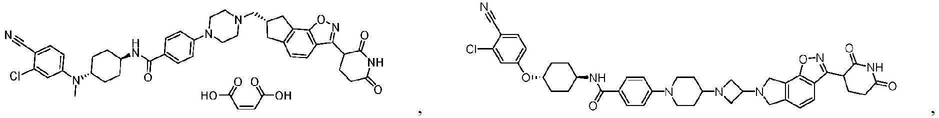 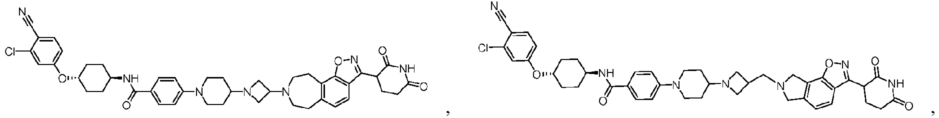 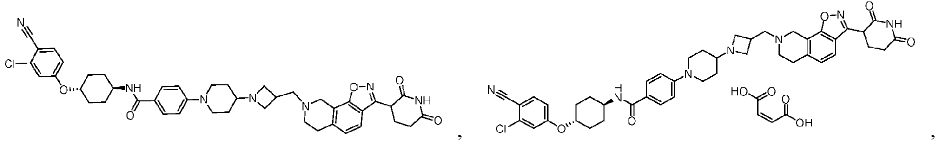 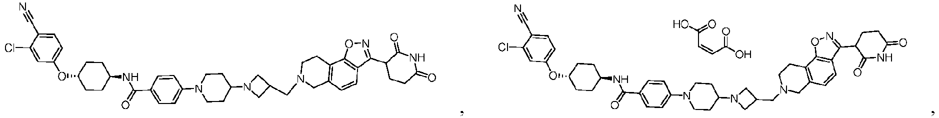 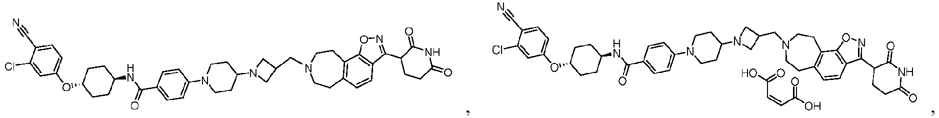 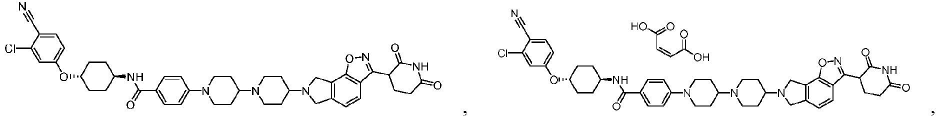 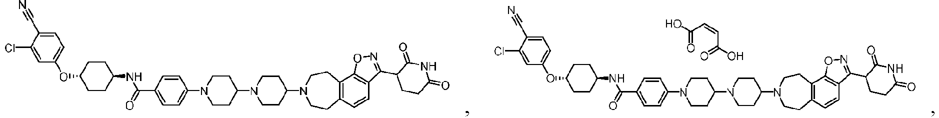 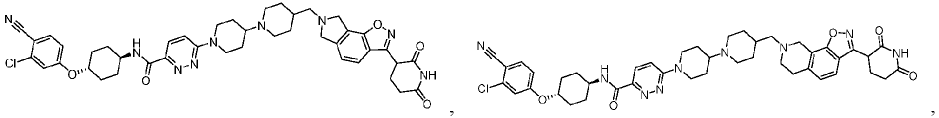 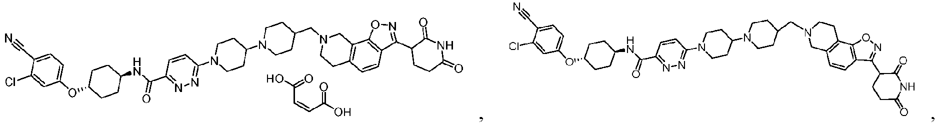 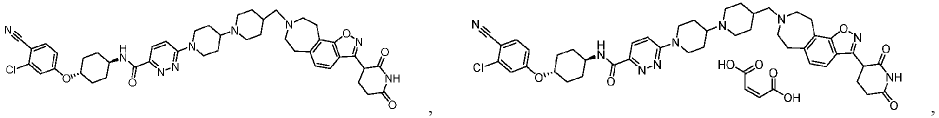 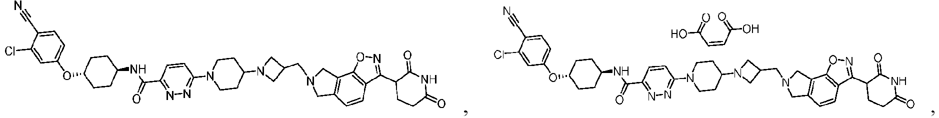 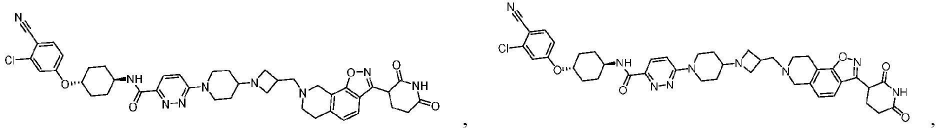 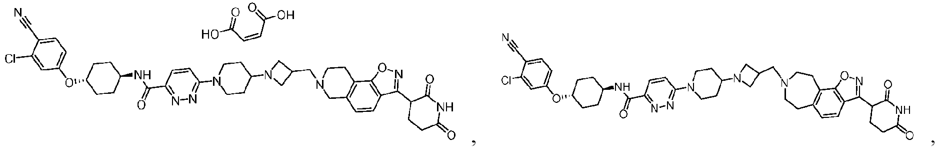 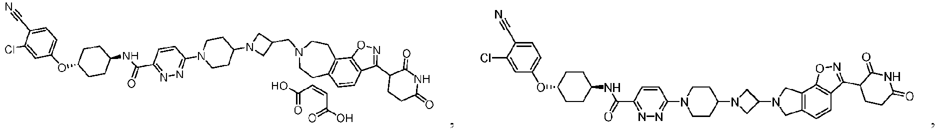 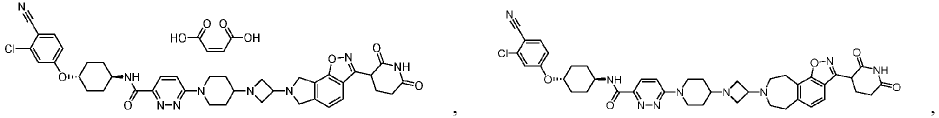 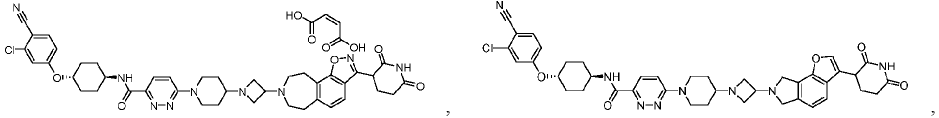 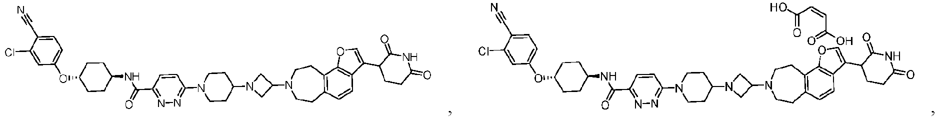 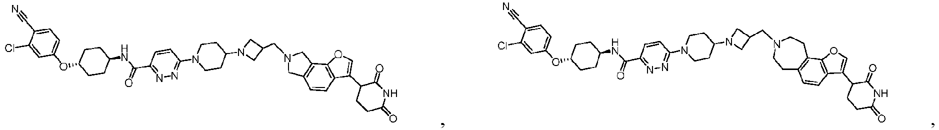 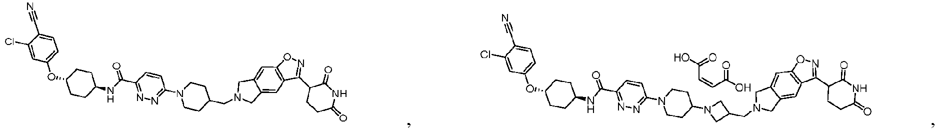 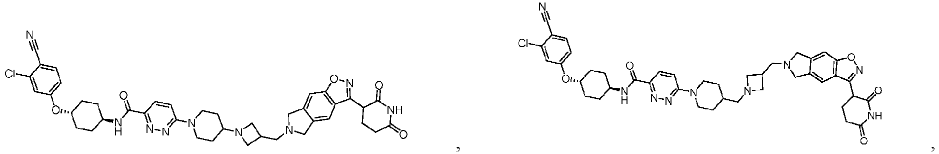 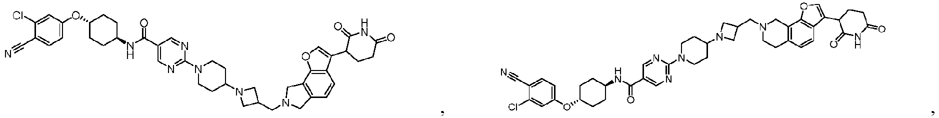 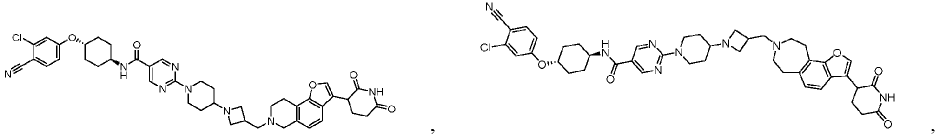 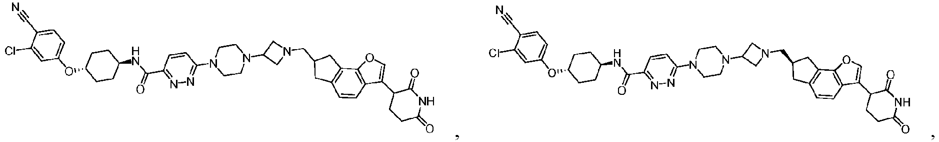 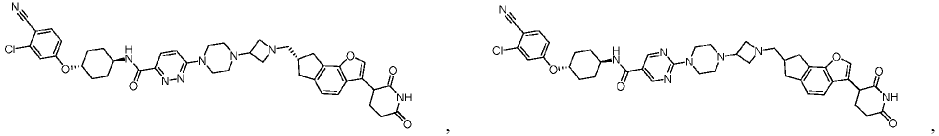 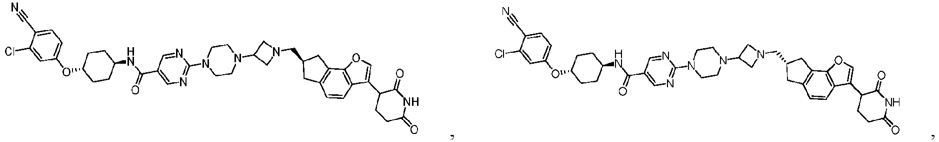 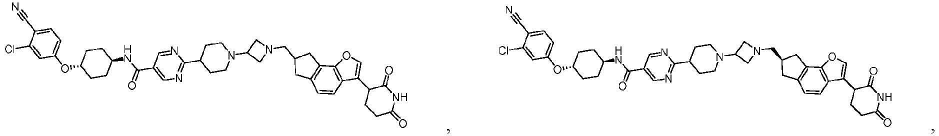 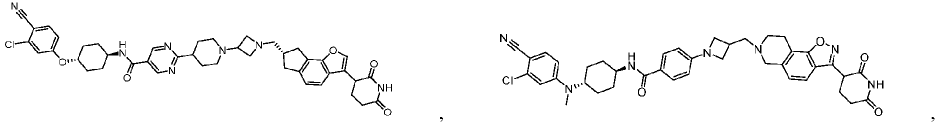 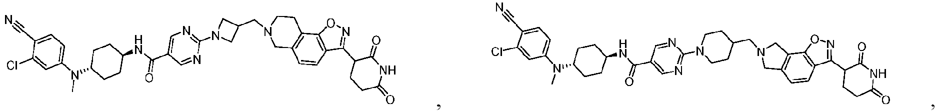 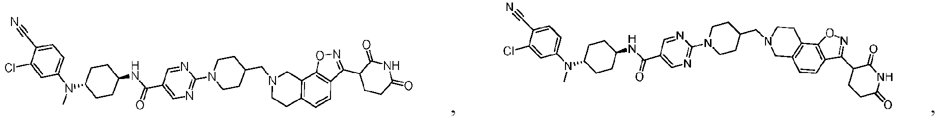 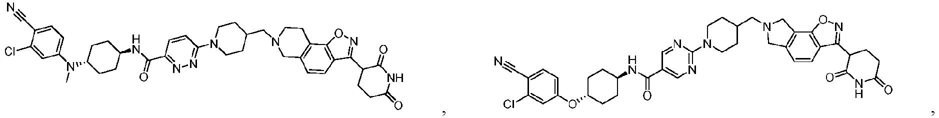 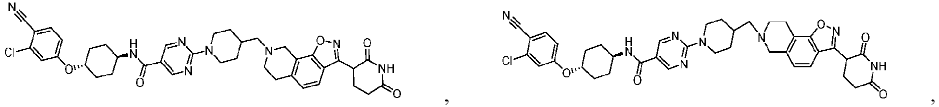 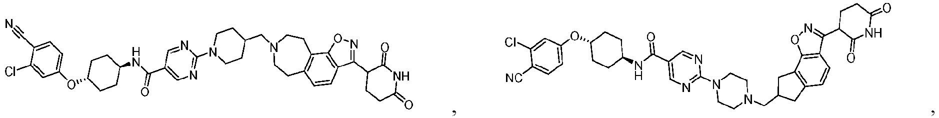 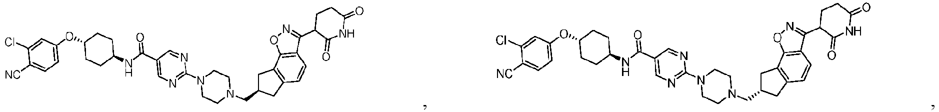 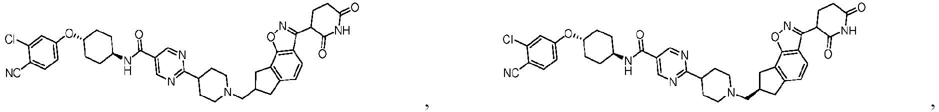 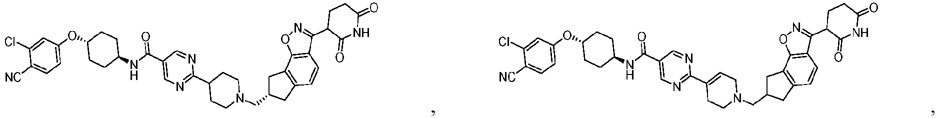 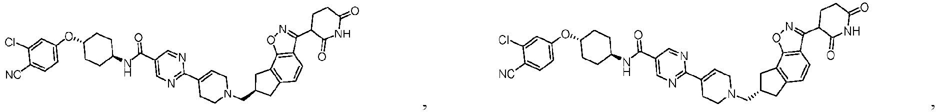 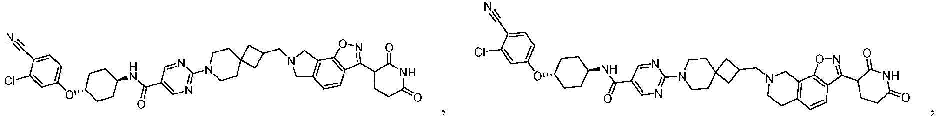 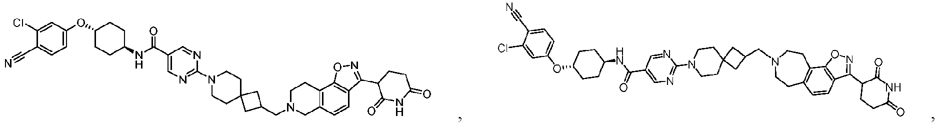 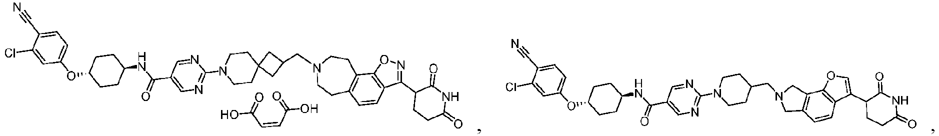 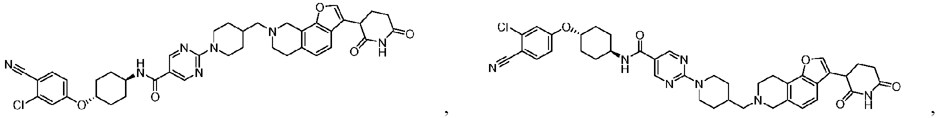 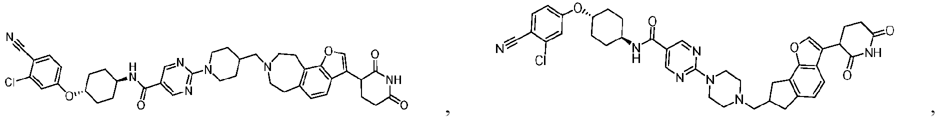 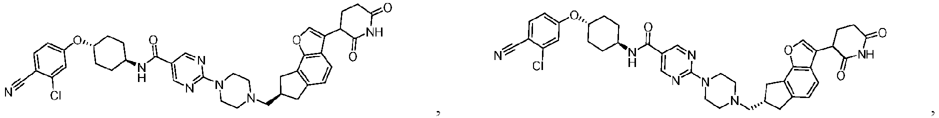 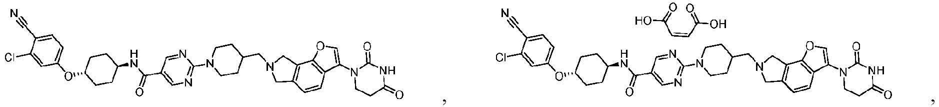 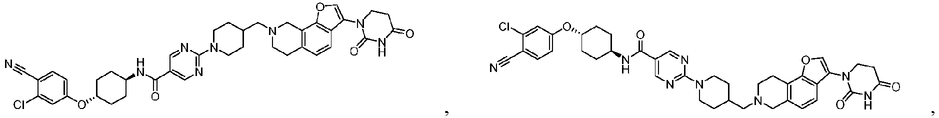 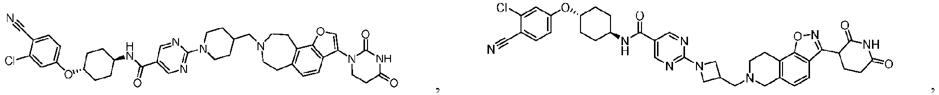 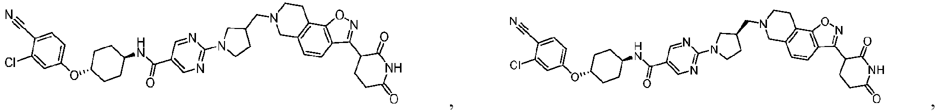 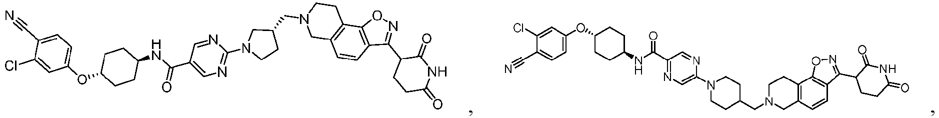 and

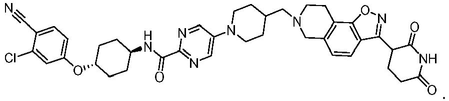

15. A compound of formula I' or formula I", a moiety, a stereoisomer thereof, a derivative, or a pharmaceutically acceptable salt thereof, wherein 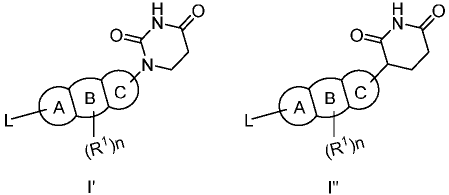
ring A is absent or selected from the group consisting of C₅₋₁₀ membered cycloalkenyl, 5- to 10-membered heterocycloalkenyl, phenyl, and 5- to 6-membered heteroaryl;
ring B is selected from phenyl;
ring C is selected from the group consisting of isoxazolyl and furanyl;
L is selected from a connecting group.

16. A compound of formula I'-a or formula I"-a, a moiety, a stereoisomer thereof, a derivative, or a pharmaceutically acceptable salt thereof, wherein 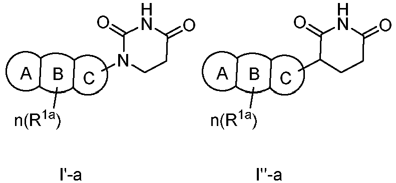
ring A is absent or selected from the group consisting of C₅₋₁₀ membered cycloalkenyl, 5- to 10-membered heterocycloalkenyl, phenyl, and 5- to 6-membered heteroaryl;
ring B is selected from phenyl;
ring C is selected from the group consisting of isoxazolyl and furanyl;
each R^{1a} is independently selected from the group consisting of halogen, -OH, -NH₂, -CN, =O, -CHO, C₁₋₄ alkyl, C₁₋₄ alkoxy, C₁₋₆ alkyl OC(O)-, C₃₋₁₂ cycloalkyl, and 4- to 12-membered heterocycloalkyl, wherein the C₁₋₄ alkyl, C₁₋₄ alkoxy, C₃₋₁₂ cycloalkyl, or 4- to 12-membered heterocycloalkyl is optionally substituted with one or more of the following groups: halogen, =O, -OH, -NH₂, -CN, CHO, COOH, -C₁₋₄ alkyl-OH, C₁₋₆ alkyl OC(O)-, and 4- to 10-membered heterocycloalkyl optionally substituted with C₁₋₆ alkyl COC(O)-;
n is selected from the group consisting of 0, 1, 2, and 3.

17. The compound, the moiety, the stereoisomer thereof, the derivative, or the pharmaceutically acceptable salt thereof according to claim 15 or 16, wherein the compound is selected from the group consisting of:

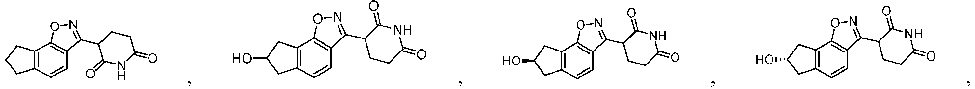

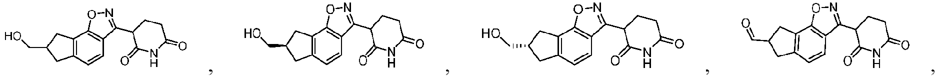

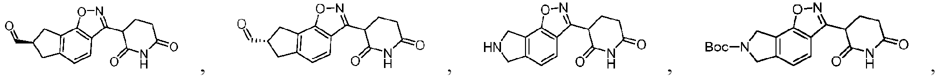

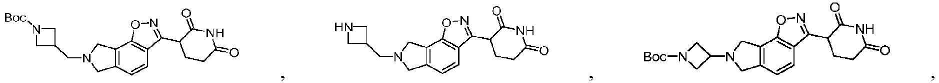

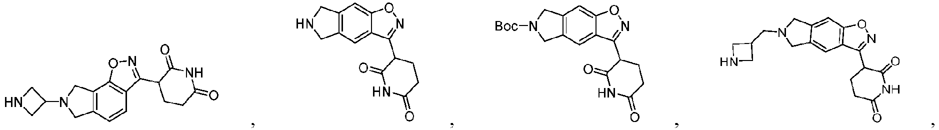

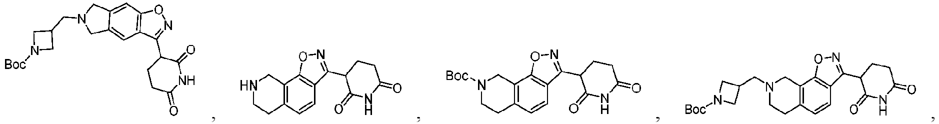

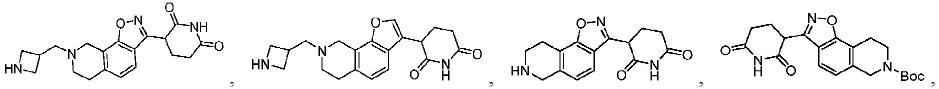

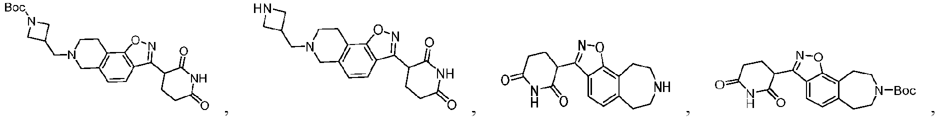

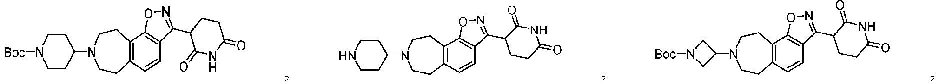

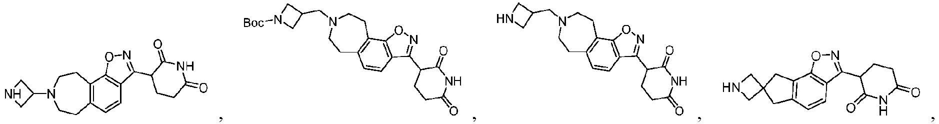

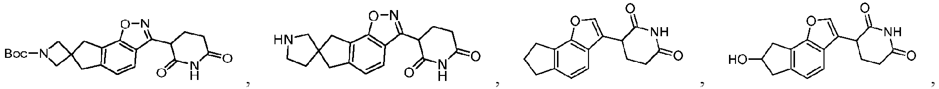

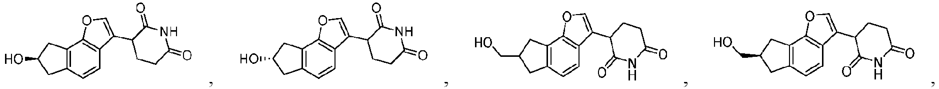

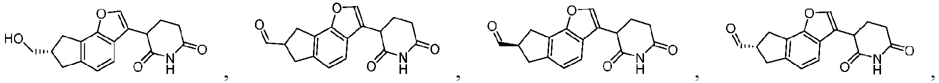

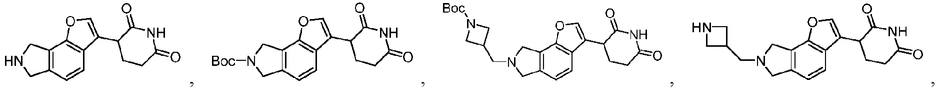

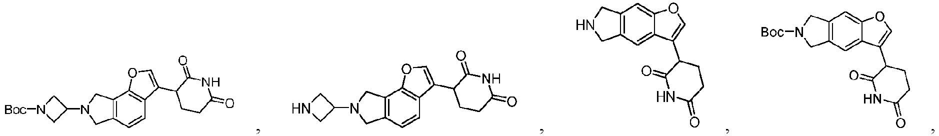

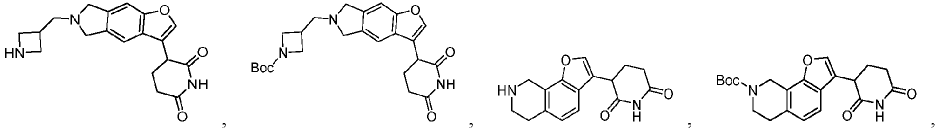

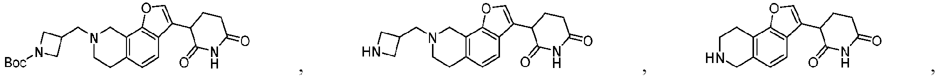

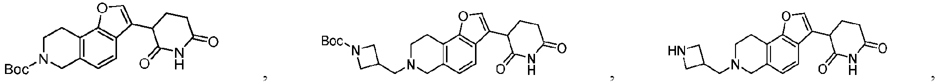

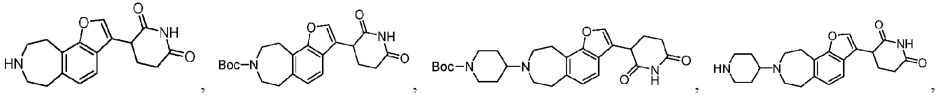

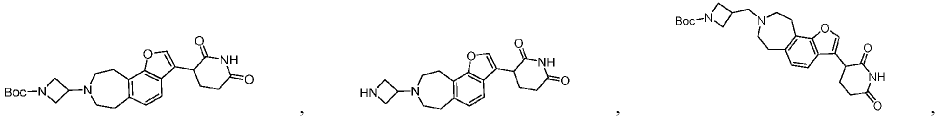

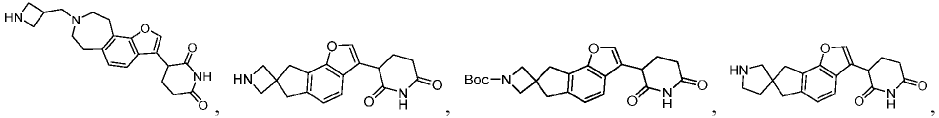

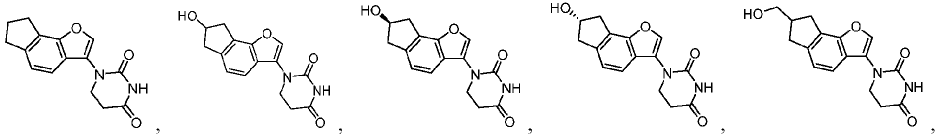

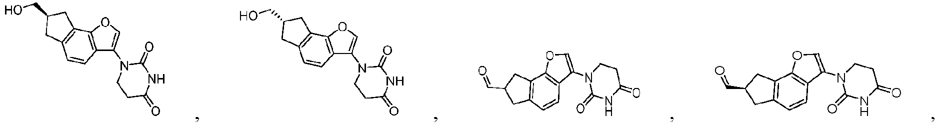

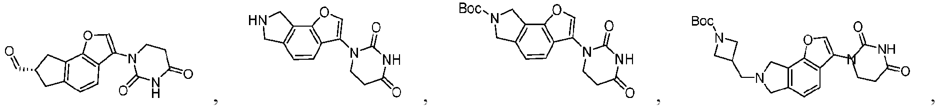

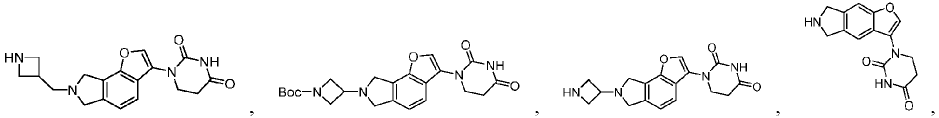

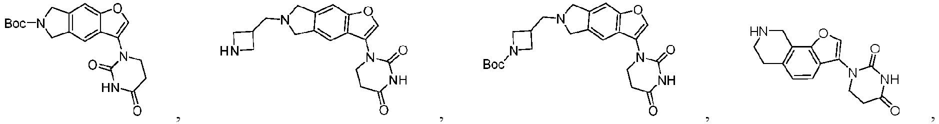

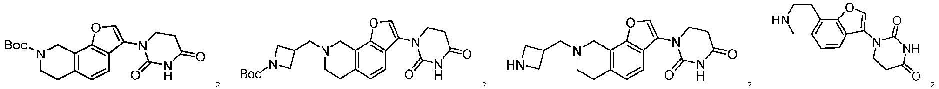

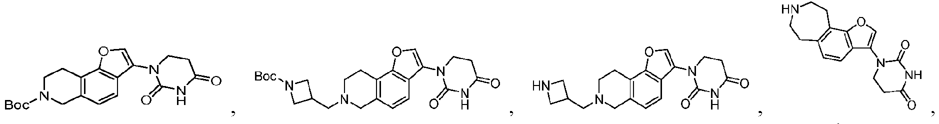

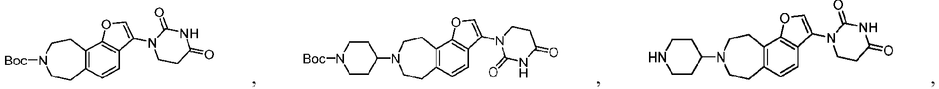

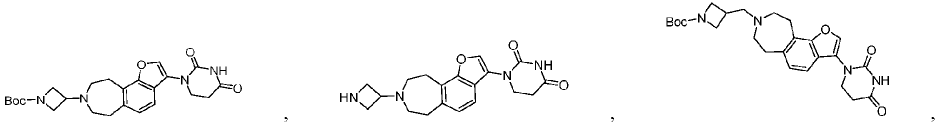

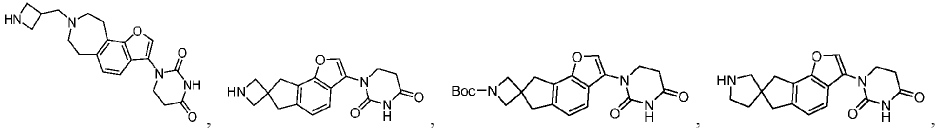

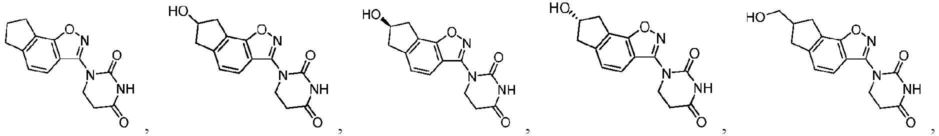

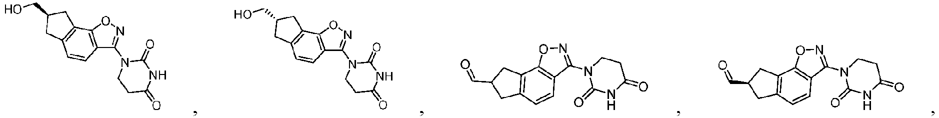

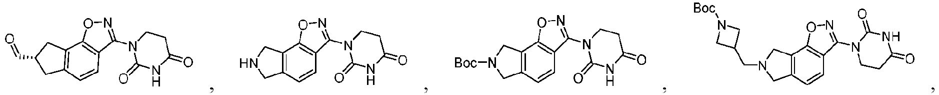

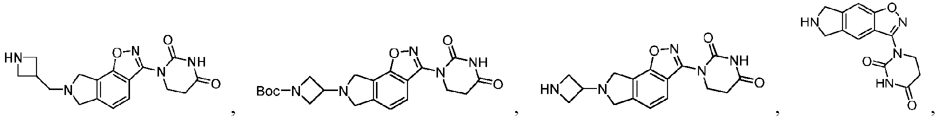

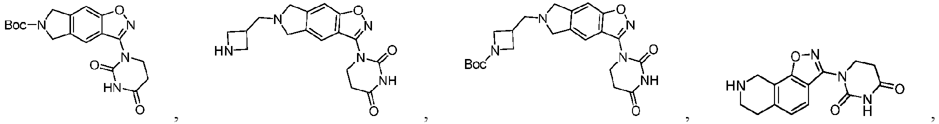

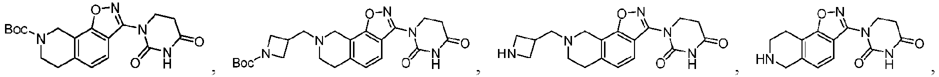

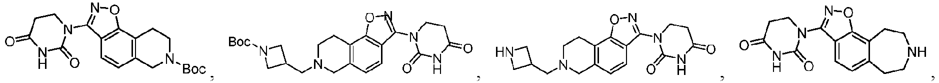

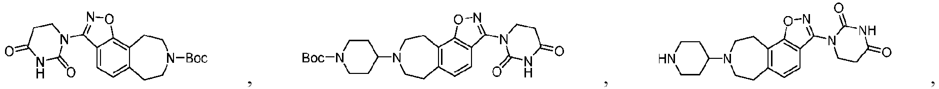

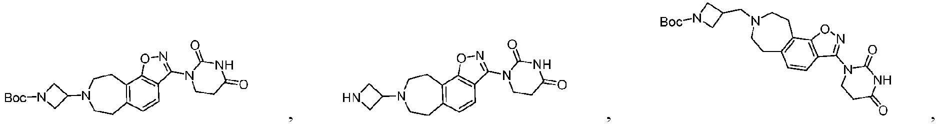

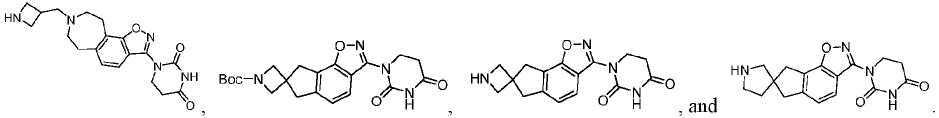

18. Use of the compound, the moiety, the isomer thereof, the derivative thereof, or the pharmaceutically acceptable salt thereof according to any one of claims 15 to 17 in a Protac molecule, or use thereof for constituting part of a Protac molecule, or use thereof for degrading an androgen receptor (AR), wherein the compound is present in the form of a Protac molecule.

19. A pharmaceutical composition, comprising the compound, the stereoisomer thereof, or the pharmaceutically acceptable salt thereof according to any one of claims 1 to 14, or the compound, the moiety, the isomer thereof, the derivative thereof, or the pharmaceutically acceptable salt thereof according to any one of claims 15 to 17.

20. Use of the compound, the stereoisomer thereof, or the pharmaceutically acceptable salt thereof according to any one of claims 1 to 14, the compound, the moiety, the isomer thereof, the derivative thereof, or the pharmaceutically acceptable salt thereof according to any one of claims 15 to 17, or the pharmaceutical composition according to claim 19 for preparing a medicament for preventing or treating a disorder treated by degrading a target protein that binds to a targeting ligand.
